# EUROPEAN PATENT APPLICATION

(11) **EP 4 385 988 A1**
(43) Date of publication of application: **19.06.2024**
(21) Application number: 22855501.7
(22) Date of filing: 11.08.2022
(51) Int. Cl.: C07D 403/14, C07D 413/14, C07D 401/14, A61K 31/501, A61P 25/00

(54) **SUBSTITUTED TRIAZOLE DERIVATIVE, PREPARATION METHOD THEREFOR, PHARMACEUTICAL COMPOSITION THEREOF, AND USE THEREOF**

(30) Priority: 12.08.2021 CN 202110927008
(71) Applicant: Shanghai Simr Biotechnology Co., Ltd., Shanghai 201318 (CN); Shanghai Simrd Biotechnology Co., Ltd, Shanghai 201399 (CN)
(72) Inventor: LIU, Yang, Shanghai 201318 (CN); JIN, Yun, Shanghai 201318 (CN); YANG, Fei, Shanghai 201318 (CN); WANG, Fei, Shanghai 201318 (CN)
(74) Representative: Murgitroyd & Company
(86) International application number: PCT/CN2022/111829
(87) International publication number: WO 2023/016521

(57) **Abstract**

Disclosed are a substituted triazole derivative, a preparation method therefor, a pharmaceutical composition thereof, and a use thereof; specifically, provided are a compound represented by general formula (I), cis-trans isomers thereof, enantiomers thereof, diastereoisomers thereof, racemates thereof, solvates thereof, hydrates thereof, or pharmaceutically acceptable salts or prodrugs thereof, a preparation method therefor, a pharmaceutical composition containing the compound, and a use of the compound as an α5-GABA_{A} receptor inverse modulator.

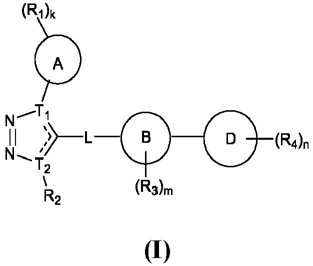

## Description

The present application claims the right of the priority of Chinese patent application 2021109270082 filed on August 12, 2021. The contents of the Chinese patent application are incorporated herein by reference in its entirety.

### TECHNICAL FIELD

The present disclosure relates to substituted triazole derivatives with a modulatory function on an α5-GABA_{A} receptor, a preparation method therefor, a pharmaceutical composition containing the same, and a use thereof as a medicament.

### BACKGROUND

γ-Aminobutyric acid (GABA) is an important inhibitory neurotransmitter in the mammalian central nervous system. There are two types of GABA receptors in nature. One is GABA_{A} receptor, which is a member of the ligand-gated ion channel superfamily, and the other is GABA_{B} receptor, which is a member of the G protein-coupled receptor superfamily. It is found that there are several GABA_{A} receptor subunits in mammals, including α1-6, β1-4, γ1-3, δ, ε, θ, and ρ1-3, among which α subunit, β subunit, and γ subunit are essential for the formation of a complete and functional GABA_{A} receptor, and α subunit is crucial for the binding of benzodiazepine to the GABA_{A} receptor.

The α5-containing GABA_{A} receptor (α5-GABA_{A} receptor) accounts for less than 5% of the GABA_{A} receptors in the mammalian brain and is expressed at a very low level in the cerebral cortex, but accounts for more than 20% of the GABA_{A} receptors in the hippocampus tissue of the brain and is hardly expressed in other brain regions. Considering the specific distribution and functional research of the α5-GABA_{A} receptors in the hippocampus tissue of the brain, many pharmaceutical companies including Roche and Merck are engaged in the research of α5-GABA_{A} receptor ligands, and a large number of compounds have been gradually synthesized, particularly inverse agonists targeting the α5-GABA_{A} receptors in the hippocampus tissue of the brain, among which α5IA and MRK-016 have shown good therapeutic effects for the treatment of cognitive diseases in animal disease models. It is widely recognized that inverse agonists of the α5-GABA_{A} receptor can be used for the treatment of cognitive diseases, especially for Alzheimer's disease. The patent application US20110224278 discloses that inverse agonists of the α5-GABA_{A} receptor can be used for the treatment of multi-infarct dementia and stoke related diseases.

Research in the last decade has demonstrated (Zlokovic, B. V. et al. Nat Rev Neurosci. 12(12), 723-738) that the blood-brain barrier is damaged in many disease states, especially neurodegenerative diseases, Alzheimer's disease, and stroke. As a result, even substances that normally cannot enter the brain can also exert a corresponding pharmacological effect. Therefore, inverse agonists of the α5-GABA_{A} receptor that normally cannot cross the blood-brain barrier can also be used to treat Alzheimer's disease and stroke.

In 2002, Xu Zhang's laboratory reported that the α5-GABA_{A} receptor is also predominantly expressed in small neurons with up-regulation in the nerve axotomy model (Xiao HS et al., Proc Natl Acad Sci USA. 2002, 99(12), 8360-8365). The patent application CN103239720 discloses that the α5-GABA_{A} receptor is expressed in the peripheral nervous system and its expression level is significantly elevated in the partial nerve injury model. Inverse agonists of the α5-GABA_{A} receptor exert the analgesic effect of various types of pain by selectively binding to the α5-GABA_{A} receptor in the peripheral nervous system. The animal experimental model data show that the stronger the inverse agonism of the inverse agonist, the better the analgesic effect is.

A lot of research has been conducted to detect whether a compound is an inverse agonist or an antagonist of the α5-GABA_{A} receptor. For example, in the international patent applications WO1992022652 and WO1994013799, the combination of α5, β3, and γ2 of the GABA_{A} receptor is used to detect whether a compound binds to the receptor; in the process of drug screening, the method described by Goeders *et al.* (Goeders, N. E. and Kuhar, M. J. Life Sci. 1985, 37(4), 345-355) is commonly used. There are also many studies on detecting whether a ligand binding to the α5-GABA_{A} receptor is an antagonist, an agonist, or an inverse agonist, which can be referred to the method described by Wafford *et al.* (Wafford, K. A., Whiting, P. J. and Kemp, J. A. Mol. Pharmacol. 1993, 43, 240-244).

There are many methods for screening whether drugs cross the blood-brain barrier. It has been reported in the literature (Jones et al., BioorgMed Chem Lett. 2006, 16(4), 872-875) that compounds can be detected for inhibition of (³H)RO-15-1788 (a labeled specific inverse agonist of the α5-GABA_{A} receptor) binding in the brain, and that MRK016 can effectively inhibit (³H)RO-15-1788 binding in the central nervous system, while MRK016-M3 is barely able to significantly inhibit (³H)RO-15-1788 binding in the central nervous system. It can also be detected by testing the drug in different tissues, for example, by testing the distribution ratio of the drug in the brain and plasma to determine whether the drug can effectively cross the blood-brain barrier.

Previous studies have shown that inhibiting or reducing the α5-GABA_{A} receptor-mediated extrasynaptic inhibitory effect by drugs or genetic methods can improve cognitive and learning abilities but also lead to mild anxiety-like behaviors (Brickley, S.G. & Mody, I. Neuron. 2012, 73, 23-34; Harris, D. et al. J. Med. Chem. 2008, 51, 3788-3803; Savić, M. M. *et al. Brain Res.* **2008,** 1208, 150-159; Clément, Y. et al. Behav. Brain Res. 2012, 233, 474-482). There are also studies showing that fear and anxiety traits are associated with reduced Gabra5 mRNA (Heldt, S. A. & Ressler, K. J. Eur. J. Neurosci. 2007, 26, 3631-3644; Tasan, R.O. et al. Neuroscience. 2011, 183, 71-80). Paolo Botta *et al.* have disclosed the mechanism by which the α5-GABA_{A} receptor is involved in anxiety and fear. Specific knockout of α5-GABA_{A} receptor in brain regions leads to fear and anxiety behaviors in animals. As a result, α5-GABA_{A} inverse agonists can cause side effects of fear and anxiety when entering the brain, thus affecting their druggability.

### CONTENT OF THE PRESENT INVENTION

The present disclosure provides a substituted triazole derivative, a preparation method therefor, a pharmaceutical composition thereof, and a use thereof. This class of compounds have pharmaceutical properties such as good selective inverse agonistic activity for α5-GABA_{A} and bioavailability as well as low central exposure.

The present disclosure provides a compound of formula I, a *cis-trans* isomer thereof, an enantiomer thereof, a diastereomer thereof, a racemate thereof, a solvate thereof, a hydrate thereof, a pharmaceutically acceptable salt thereof, or a prodrug thereof, 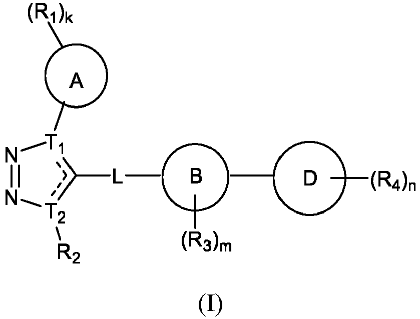 wherein
ring A is selected from a benzene ring or a 5- to 6-membered heteroaryl ring;
each R₁ is independently selected from hydrogen, halogen, cyano, C₁₋₆ alkyl, C₁₋₆ alkoxy, C₁₋₆ alkylamino, C₃₋₆ cycloalkyl, or 3- to 6-membered heterocycloalkyl, and C₁₋₆ alkyl, C₁₋₆ alkoxy, C₁₋₆ alkylamino, C₃₋₆ cycloalkyl, and/or 3- to 6-membered heterocycloalkyl are optionally substituted by 1 to 3 R';
each R' is independently selected from hydrogen, halogen, hydroxyl, amino, cyano, methyl, cyclopropyl, or methoxy;
k is 0, 1, 2, or 3;
T₁ and T₂ are each independently selected from a carbon atom or a nitrogen atom;
R₂ is selected from hydrogen, halogen, cyano, C₁₋₃ alkyl, C₁₋₃ alkoxy, C₁₋₆ alkylamino, C₃₋₆ cycloalkyl, 3- to 6-membered heterocycloalkyl, or 5- to 6-membered heteroaryl, each of which is optionally substituted by 1 to 3 R';
L is selected from -CHz-O-, -CH=CH-, or -CH₂-NH-;
ring B is selected from a benzene ring or a 5- to 10-membered heteroaryl ring;
each R₃ is independently selected from hydrogen, halogen, cyano, C₁₋₆ alkyl, C₁₋₆ alkoxy, C₁₋₆ alkylamino, C₃₋₆ cycloalkyl, or 3- to 6-membered heterocycloalkyl, each of which is optionally substituted by 1 to 3 R';
m is 0, 1, 2, or 3;
ring D is selected from a 4- to 14-membered heterocycloalkyl ring;
each R₄ is independently selected from hydrogen, halogen, cyano, =O, -R₅, -OR₆, - COOR₆, -C(O)R₅, -NR₆R₇, -NR₆COR₅, -NR₆SO₂R₅, -CH₂-C(O)NR₆R₇, -C(O)NR₆R₇, -SO₂R₆, or -SO₂NR₆R₇;
R₅ is selected from C₁₋₆ alkyl, C₃₋₆ cycloalkyl, 3- to 6-membered heterocycloalkyl, 6-to 10-membered aryl, 6- to 10-membered aryl(C₁₋₃)alkyl, 5- to 10-membered heteroaryl, or 5-to 10-membered heteroaryl(C₁₋₃)alkyl, each of which is optionally substituted by 1 to 3 R;
R₆ and R₇ are each independently selected from hydrogen, hydroxyl, amino, cyano, C₁₋₆ alkyl, C₁₋₆ alkoxy, C₁₋₆ alkylamino, C₃₋₆ cycloalkyl, 3- to 6-membered heterocycloalkyl, 6-to 10-membered aryl, 6- to 10-membered aryl(C₁₋₆)alkyl, 5- to 10-membered heteroaryl, or 5-to 10-membered heteroaryl(C₁₋₆)alkyl, each of which is optionally substituted by 1 to 3 R;
when R₄ is selected from -NR₆R₇, -C(O)NR₆R₇, or -SO₂NR₆R₇, R₆ and R₇ together with the N atom to which they are attached form a 4- to 7-membered heterocycle, and the 4- to 7-membered heterocycle is optionally substituted by 1 to 3 R;
each R is independently selected from hydrogen, halogen, cyano, hydroxyl, amino, - COOH, C₁₋₆ alkyl, C₁₋₆ alkoxy, C₁₋₆ alkylamino, C₃₋₆ cycloalkyl, or 3- to 6-membered heterocycloalkyl, and the C₁₋₆ alkyl, C₁₋₆ alkoxy, C₁₋₆ alkylamino, C₃₋₆ cycloalkyl, or 3- to 6-membered heterocycloalkyl is optionally substituted by 1 to 3 R';
n is 0, 1, 2, 3, 4, 5, or 6.

In certain preferred embodiments of the present disclosure, certain groups in the compound of formula I, the *cis-trans* isomer thereof, the enantiomer thereof, the diastereomer thereof, the racemate thereof, the solvate thereof, the hydrate thereof, the pharmaceutically acceptable salt thereof, or the prodrug thereof are defined as follows, and unmentioned groups are as described in any one of the embodiments of the present disclosure (referred to as "in a certain embodiment of the present disclosure"),
ring A is selected from a benzene ring or a 5- to 6-membered heteroaryl ring;
each R₁ is independently selected from hydrogen, halogen, cyano, C₁₋₆ alkyl, C₁₋₆ alkoxy, C₁₋₆ alkylamino, C₃₋₆ cycloalkyl, or 3- to 6-membered heterocycloalkyl, and the C₁₋₆ alkyl, C₁₋₆ alkoxy, C₁₋₆ alkylamino, C₃₋₆ cycloalkyl, and/or 3- to 6-membered heterocycloalkyl are optionally substituted by 1 to 3 R';
each R' is independently selected from hydrogen, halogen, hydroxyl, amino, cyano, methyl, cyclopropyl, or methoxy;
k is 0, 1, 2, or 3;
T₁ and/or T₂ are each independently selected from a carbon atom or a nitrogen atom;
R₂ is selected from hydrogen, halogen, cyano, C₁₋₃ alkyl, C₁₋₃ alkoxy, C₁₋₆ alkylamino, C₃₋₆ cycloalkyl, or 3- to 6-membered heterocycloalkyl, each of which is optionally substituted by 1 to 3 R';
L is selected from -CHz-O-, -CH=CH-, or -CH₂-NH-;
ring B is selected from a benzene ring or a 5- to 10-membered heteroaryl ring;
each R₃ is independently selected from hydrogen, halogen, cyano, C₁₋₆ alkyl, C₁₋₆ alkoxy, C₁₋₆ alkylamino, C₃₋₆ cycloalkyl, or 3- to 6-membered heterocycloalkyl, each of which is optionally substituted by 1 to 3 R';
m is 0, 1, 2, or 3;
ring D is selected from a 4- to 14-membered heterocycloalkyl ring;
ring D can be substituted by one or more than one independent R₄, and each R₄ is independently selected from hydrogen, halogen, cyano, =O, -R₅, -OR₆, -COOR₆, -C(O)R₅, - NR₆R₇, -NR₆COR₅, -NR₆SO₂R₅, -C(O)NR₆R₇, -SO₂R₆, or -SO₂NR₆R₇;
R₅ is selected from C₁₋₆ alkyl, C₃₋₆ cycloalkyl, 3- to 6-membered heterocycloalkyl, 6-to 10-membered aryl, 6- to 10-membered aryl(C₁₋₃)alkyl, 5- to 10-membered heteroaryl, or 5-to 10-membered heteroaryl(C₁₋₃)alkyl, each of which is optionally substituted by 1 to 3 R;
R₆ and/or R₇ are each independently selected from hydrogen, hydroxyl, amino, cyano, C₁₋₆ alkyl, C₁₋₆ alkoxy, C₁₋₆ alkylamino, C₃₋₆ cycloalkyl, 3- to 6-membered heterocycloalkyl, 6-to 10-membered aryl, 6- to 10-membered aryl(C₁₋₆)alkyl, 5- to 10-membered heteroaryl, or 5-to 10-membered heteroaryl(C₁₋₆)alkyl, each of which is optionally substituted by 1 to 3 R;
when R₄ is selected from -NR₆R₇, -C(O)NR₆R₇, or -SO₂NR₆R₇, R₆ and R₇ together with the N atom to which they are attached can form a 4- to 7-membered heterocycle, and the 4- to 7-membered heterocycle is optionally substituted by 1 to 3 R;
each R is independently selected from hydrogen, halogen, cyano, hydroxyl, amino, C₁₋₆ alkyl, C₁₋₆ alkoxy, C₁₋₆ alkylamino, C₃₋₆ cycloalkyl, or 3- to 6-membered heterocycloalkyl, and the C₁₋₆ alkyl, C₁₋₆ alkoxy, C₁₋₆ alkylamino, C₃₋₆ cycloalkyl, or 3- to 6-membered heterocycloalkyl is optionally substituted by 1 to 3 R';
n is 0, 1, 2, 3, 4, 5, or 6.

In a certain embodiment of the present disclosure, R₂ is 5- to 6-membered heteroaryl.

In a certain embodiment of the present disclosure, each R₄ is independently -CH₂-C(O)NR₆R₇.

In a certain embodiment of the present disclosure, each R is independently COOH.

In a certain embodiment of the present disclosure, in ring A and R₂, the heteroatom in the 5- to 6-membered heteroaryl is selected from 1, 2, or 3 types of N, O, and S, and the number of the heteroatom is 1, 2, or 3; preferably pyridyl or isoxazolyl.

In a certain embodiment of the present disclosure, in ring B, R₅, R₆, and R₇, the heteroatom in the 5- to 10-membered heteroaryl is selected from 1, 2, or 3 types of N, O, and S, and the number of the heteroatom is 1, 2, or 3; preferably pyridyl, pyridazinyl, pyrazinyl, or pyridopyridazinyl.

In a certain embodiment of the present disclosure, in R₁, R', R₂, R₃, R₄, and R, the halogen is independently fluorine, chlorine, bromine, or iodine; preferably fluorine or chlorine.

In a certain embodiment of the present disclosure, in R₁, R₃, R₅, R₆, R₇, and R, the C_{1- 6} alkyl is independently methyl, ethyl, *n*-propyl, isopropyl, *n*-butyl, isobutyl, *sec-*butyl, or *tert-*butyl.

In a certain embodiment of the present disclosure, in R₂, the C₁₋₃ alkyl is independently methyl, ethyl, *n*-propyl, or isopropyl; preferably methyl.

In a certain embodiment of the present disclosure, in R₁, R₃, R₆, R₇, and R, the C₁₋₆ alkoxy is independently methoxy, ethoxy, *n*-propoxy, isopropoxy, *n*-butoxy, isobutoxy, *sec-*butoxy, or *tert*-butoxy; preferably methoxy.

In a certain embodiment of the present disclosure, in R₂, the C₁₋₃ alkoxy is independently methoxy, ethoxy, *n*-propoxy, or isopropoxy.

In a certain embodiment of the present disclosure, in R₁, R₂, R₃, R₆, R₇, and R, the C₁₋₆ alkylamino is independently -NHCH₃, -N(CH₃)₂, -NHCH₂CH₃, -N(CH₃)CH₂CH₃, - N(CH₂CH₃)₂, -NHCH₂CH₂CH₃, -NHCH(CH₃)₂, or -NHCH₂CH₂CH₂CH₃, preferably -NMe₂.

In a certain embodiment of the present disclosure, in R₁, R₂, R₃, R₆, R₇, and R, the C₃₋₆ cycloalkyl is independently cyclopropyl, cyclobutyl, cyclopentyl, or cyclohexyl; preferably cyclopropyl.

In a certain embodiment of the present disclosure, in R₁, R₂, R₃, R₆, R₇, and R, the heteroatom in the 3- to 6-membered heterocycloalkyl is selected from 1, 2, or 3 types of N, O, and S, and the number of the heteroatom is 1, 2, or 3; preferably oxetanyl, pyrrolidinyl, tetrahydrofuranyl, morpholinyl, tetrahydropyranyl, or piperidinyl.

In a certain embodiment of the present disclosure, in ring D, the 4- to 14-membered heterocycloalkyl is a saturated or semi-saturated monocyclic, bicyclic, or tricyclic group having 1, 2, 3, or 4 heteroatoms selected from 1, 2, or 3 types of N, O, and S, wherein the ring directly attached to ring B is not aromatic; preferably 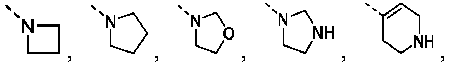 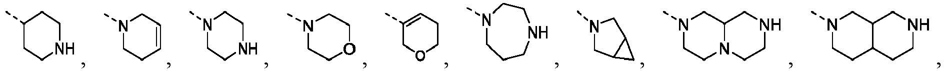 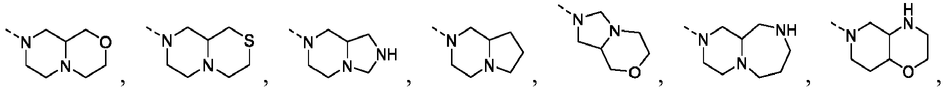 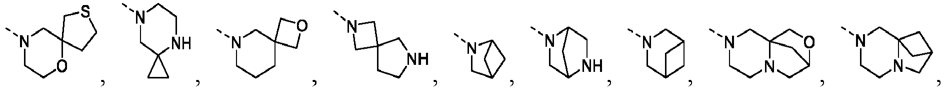 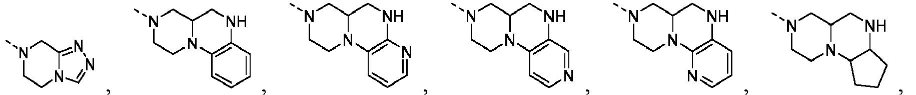 or

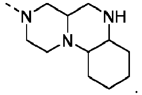

In a certain embodiment of the present disclosure, in ring D, the 4- to 14-membered heterocycloalkyl is a saturated monocyclic, bicyclic, or tricyclic group having 1, 2, 3, or 4 heteroatoms selected from 1, 2, or 3 types of N, O, and S.

In a certain embodiment of the present disclosure, when R₆ and R₇ together with the N atom to which they are attached can form a 4- to 7-membered heterocycle, the 4- to 7-membered heterocycle is a saturated monocyclic or bicyclic group having 1, 2, or 3 heteroatoms selected from 1, 2, or 3 types of N, O, and S; preferably

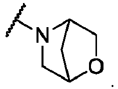

In a certain embodiment of the present disclosure, in R₅, R₆, and R₇, the 6- to 10-membered aryl is independently phenyl or naphthyl, preferably phenyl.

In a certain embodiment of the present disclosure, each R₁ is independently hydrogen, halogen, cyano, C₁₋₃ alkyl, C₁₋₃ alkoxy, or C₃₋₆ cycloalkyl, and the C₁₋₃ alkyl, C₁₋₃ alkoxy, or C₃₋₆ cycloalkyl is optionally substituted by 1 to 3 R'; preferably, each R₁ is independently halogen, cyano, C₁₋₃ alkyl, or C₁₋₃ alkoxy, and the C₁₋₃ alkyl and/or C₁₋₃ alkoxy are optionally substituted by 1 to 3 R'; each R' is independently hydrogen, halogen, hydroxyl, cyano, or methoxy; for example, each R' is independently hydrogen, halogen, or hydroxyl.

In a certain embodiment of the present disclosure, k is 0, 1, or 2; for example, k is 1.

In a certain embodiment of the present disclosure, R₂ is selected from hydrogen, halogen, cyano, C₁₋₃ alkyl, C₃₋₆ cycloalkyl, or 5- to 6-membered heteroaryl, each of which is optionally substituted by 1 to 3 R'; for example, R₂ is selected from hydrogen, halogen, cyano, C₁₋₃ alkyl, or C₃₋₆ cycloalkyl, each of which is optionally substituted by 1 to 3 R'.

In a certain embodiment of the present disclosure, when T₁ is a nitrogen atom, T₂ is a carbon atom, 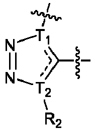 is 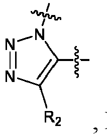 R₂ is selected from hydrogen, halogen, cyano, C₁₋₃ alkyl, C₁₋₃ alkoxy, C₁₋₆ alkylamino, C₃₋₆ cycloalkyl, or 5- to 6-membered heteroaryl, each of which is optionally substituted by 1 to 3 R', and the R' is as defined in any one of the embodiments of the present disclosure. Preferably, R₂ is selected from hydrogen, halogen, cyano, C₁₋₃ alkyl, C₁₋₃ alkyl substituted by cyclopropyl or hydroxyl, or 5- to 6-membered heteroaryl substituted by C₁₋₃ alkyl.

In a certain embodiment of the present disclosure, when T₁ is a nitrogen atom, T₂ is a carbon atom, and R₂ is selected from H, F, Cl, CN, Me, CHF₂, CF₃, -CH₂OH, -CH₂OCH₃, 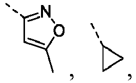 or 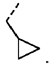 Preferably, R₂ is selected from H, Cl, Me, CH₂OH, CN, CHF₂, CF₃, 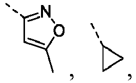 or

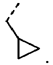

In a certain embodiment of the present disclosure, when T₁ is a carbon atom, T₂ is a nitrogen atom, 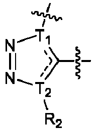 is 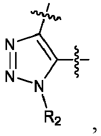 R₂ is selected from C₁₋₃ alkyl, C₃₋₆ cycloalkyl, or 5- to 6-membered heteroaryl, each of which is optionally substituted by 1 to 3 R', and the R' is as defined in any one of the embodiments of the present disclosure. Preferably, R₂ is selected from Me or CHF₂. Most preferably, R₂ is Me.

In a certain embodiment of the present disclosure, when T₁ is a nitrogen atom, T₂ is a carbon atom, 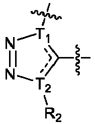 is 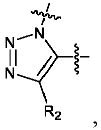 R₂ is selected from hydrogen, halogen, cyano, C₁₋₃ alkyl, C₁₋₃ alkoxy, C₁₋₆ alkylamino, C₃₋₆ cycloalkyl, or 3- to 6-membered heterocycloalkyl, each of which is optionally substituted by 1 to 3 R', and the R' is as defined in any one of the embodiments of the present disclosure. Preferably, R₂ is selected from hydrogen, halogen, cyano, C₁₋₃ alkyl, C₁₋₃ alkyl substituted by hydroxyl, or C₃₋₆ cycloalkyl.

In a certain embodiment of the present disclosure, when T₁ is a carbon atom, T₂ is a nitrogen atom, 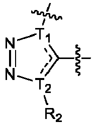 is 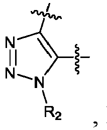 R₂ is selected from C₁₋₃ alkyl, C₃₋₆ cycloalkyl, or 3- to 6-membered heterocycloalkyl, each of which is optionally substituted by 1 to 3 R', and the R' is as defined in any one of the embodiments of the present disclosure. Preferably, R₂ is selected from hydrogen, halogen, cyano, C₁₋₃ alkyl, C₁₋₃ alkyl substituted by hydroxyl, or C₃₋₆ cycloalkyl. More preferably, R₂ is selected from Me or CHF₂. Most preferably, R₂ is Me.

In a certain embodiment of the present disclosure, each R₃ is independently hydrogen, halogen, cyano, C₁₋₃ alkyl, or C₁₋₃ alkoxy, each of which is optionally substituted by 1 to 3 R'.

In a certain embodiment of the present disclosure, m is 0 or 1, for example, m is 0.

In a certain embodiment of the present disclosure, each R₄ is independently selected from hydrogen, halogen, cyano, =O, C₁₋₃ alkyl, C₁₋₃ alkoxy, C₁₋₆ alkylamino, C₃₋₆ cycloalkyl, 3- to 6-membered heterocycloalkyl, phenyl, phenyl(C₁₋₃)alkyl, 5- to 10-membered heteroaryl, 5- to 10-membered heteroaryl(C₁₋₃)alkyl, -COOH, -CH₂-C(O)NR₆R₇, -C(O)NR₆R₇, -SO₂R₆, or -SO₂NR₆R₇; the C₁₋₃ alkyl, C₁₋₃ alkoxy, C₁₋₆ alkylamino, C₃₋₆ cycloalkyl, 3- to 6-membered heterocycloalkyl, phenyl, phenyl(C₁₋₃)alkyl, 5- to 10-membered heteroaryl, or 5- to 10-membered heteroaryl(C₁₋₃)alkyl is optionally substituted by 1 to 3 R; when R₄ is selected from -C(O)NR₆R₇ or -SO₂NR₆R₇, R₆ and R₇ together with the N atom to which they are attached can form a 4- to 7-membered heterocycle, and the 4- to 7-membered heterocycle is optionally substituted by 1 to 3 R;
preferably, each R₄ is independently selected from the following substituents: hydrogen, cyano, =O, C₁₋₃ alkyl, C₁₋₃ alkoxy, C₁₋₆ alkylamino, C₃₋₆ cycloalkyl, 3- to 6-membered heterocycloalkyl, phenyl, phenyl(C₁₋₃)alkyl, 5- to 10-membered heteroaryl, 5- to 10-membered heteroaryl(C₁₋₃)alkyl, -COOH, -C(O)NR₆R₇, -SO₂R₆, or -SO₂NR₆R₇; the C₁₋₃ alkyl, C₁₋₃ alkoxy, C₁₋₆ alkylamino, C₃₋₆ cycloalkyl, 3- to 6-membered heterocycloalkyl, phenyl, phenyl(C₁₋₃)alkyl, 5- to 10-membered heteroaryl, or 5- to 10-membered heteroaryl(C₁₋₃)alkyl is optionally substituted by 1 to 3 R; when R₄ is selected from -C(O)NR₆R₇ or -SO₂NR₆R₇, R₆ and R₇ together with the N atom to which they are attached can form a 4- to 7-membered heterocycle, and the 4- to 7-membered heterocycle is optionally substituted by 1 to 3 R.

In a certain embodiment of the present disclosure, R₆ and R₇ are each independently hydrogen, C₁₋₆ alkyl, C₃₋₆ cycloalkyl, 3- to 6-membered heterocycloalkyl, phenyl, phenyl(C₁₋₃)alkyl, 5- to 10-membered heteroaryl, or 5- to 10-membered heteroaryl(C₁₋₃)alkyl, each of which is optionally substituted by 1 to 3 R.

In a certain embodiment of the present disclosure, each R is independently selected from hydrogen, halogen, cyano, hydroxyl, amino, -COOH, C₁₋₃ alkyl, C₁₋₃ alkoxy, C₁₋₆ alkylamino, C₃₋₆ cycloalkyl, or 3- to 6-membered heterocycloalkyl, and the C₁₋₃ alkyl, C₁₋₃ alkoxy, C₁₋₆ alkylamino, C₃₋₆ cycloalkyl, or 3- to 6-membered heterocycloalkyl is optionally substituted by 1 to 3 R'; for example, each R is independently selected from hydrogen, halogen, cyano, hydroxyl, amino, C₁₋₃ alkyl, C₁₋₃ alkoxy, C₁₋₆ alkylamino, C₃₋₆ cycloalkyl, or 3- to 6-membered heterocycloalkyl, and the C₁₋₃ alkyl, C₁₋₃ alkoxy, C₁₋₆ alkylamino, C₃₋₆ cycloalkyl, or 3- to 6-membered heterocycloalkyl is optionally substituted by 1 to 3 R'.

In a certain embodiment of the present disclosure, n is 0, 1, 2, 3, or 4, for example, n is 1 or 2.

In a certain embodiment of the present disclosure, the structural moiety 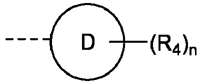 is structural moiety (II) 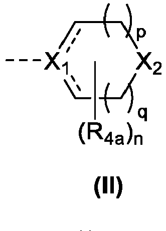 wherein
X₁ is selected from N or C; 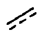
is 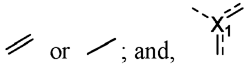 is selected from 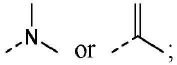
X₂ is a carbon atom, -NR_{4c}-, -O-, or -S(O)_{w}-, the carbon atom is substituted by one or two independent R_{4b}, and each R_{4b} is independently selected from hydrogen, -OR₆, -NR₆R₇, - NR₆COR₅, -C(O)NR₆R₇, or -SO₂NR₆R₇;
R₅ is C₁₋₃ alkyl, C₃₋₆ cycloalkyl, 3- to 6-membered heterocycloalkyl, or 5- to 10-membered heteroaryl, each of which is optionally substituted by 1 to 3 R';
R₆ and R₇ are each independently hydrogen, C₁₋₃ alkyl, C₃₋₆ cycloalkyl, 3- to 6-membered heterocycloalkyl, phenyl, phenyl(C₁₋₃)alkyl, 5- to 10-membered heteroaryl, or 5- to 10-membered heteroaryl(C₁₋₃)alkyl, each of which is optionally substituted by 1 to 3 R;
when R_{4b} is -NR₆R₇, -C(O)NR₆R₇, or -SO₂NR₆R₇, R₆ and R₇ together with the N atom to which they are attached can form a 4- to 7-membered heterocycle, the heterocycle can contain 1 to 3 heteroatoms as ring atoms, the heteroatoms can be independently selected from N, O, and S atoms, and the 4- to 7-membered heterocycle is optionally substituted by 1 to 3 R;
each R_{4c} is independently hydrogen, C₁₋₃ alkyl, C₃₋₆ cycloalkyl, 3- to 6-membered heterocycloalkyl, phenyl, phenyl(C₁₋₃)alkyl, 5- to 10-membered heteroaryl, or 5- to 10-membered heteroaryl(C₁₋₃)alkyl, each of which is optionally substituted by 1 to 3 R;
each R is independently hydrogen, halogen, cyano, hydroxyl, amino, C₁₋₃ alkyl, C₁₋₃ alkoxy, C₁₋₆ alkylamino, C₃₋₆ cycloalkyl, or 3- to 6-membered heterocycloalkyl, and the C₁₋₃ alkyl, C₁₋₃ alkoxy, C₁₋₆ alkylamino, C₃₋₆ cycloalkyl, or 3- to 6-membered heterocycloalkyl is optionally substituted by 1 to 3 R';
each R₄ₐ is independently hydrogen, oxo, cyano, C₁₋₃ alkyl, -COOR₆, -CH₂-C(O)NR₆R₇, -C(O)NR₆R₇, C₃₋₆ cycloalkyl, 3- to 6-membered heterocycloalkyl, or 5- to 6-membered heteroaryl; the C₁₋₃ alkyl, C₃₋₆ cycloalkyl, 3- to 6-membered heterocycloalkyl, or 5-to 6-membered heteroaryl is optionally substituted by 1 to 3 R', and the R' is as defined in any one of the embodiments of the present disclosure;
or two R₄ₐ attached to the same carbon atom together form a 3- to 6-membered saturated heterocycle, the heterocycle can contain 1 to 3 heteroatoms as ring atoms, the heteroatoms can be independently selected from N, O, and S atoms, and the 3- to 6-membered heterocycle is optionally substituted by 1 to 3 R;
n is 0, 1, 2, 3, or 4;
p and q are each independently 0, 1, or 2, and p and q are not both 2;
w is 0, 1, or 2.

In a certain embodiment of the present disclosure, the structural moiety 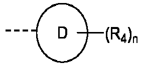 is selected from structural moiety (II) 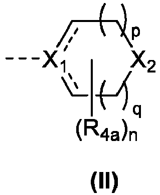 wherein
X₁ is selected from N or C; 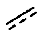
is 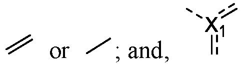 is selected from 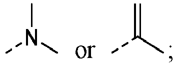
X₂ is selected from a carbon atom, -NR_{4c}-, -O-, or -S(O)_{w}-, the carbon atom is substituted by one or two independent R_{4b}, and each R_{4b} is independently selected from hydrogen, -OR₆, -NR₆R₇, -NR₆COR₅, -NR₆SO₂R₅, -C(O)NR₆R₇, -SO₂R₅, or -SO₂NR₆R₇;
R₅ is selected from C₁₋₃ alkyl, C₃₋₆ cycloalkyl, 3- to 6-membered heterocycloalkyl, or 5- to 10-membered heteroaryl, each of which is optionally substituted by 1 to 3 R';
R₆ and/or R₇ are each independently selected from hydrogen, C₁₋₃ alkyl, C₃₋₆ cycloalkyl, 3- to 6-membered heterocycloalkyl, phenyl, phenyl(C₁₋₃)alkyl, 5- to 10-membered heteroaryl, or 5- to 10-membered heteroaryl(C₁₋₃)alkyl, each of which is optionally substituted by 1 to 3 R;
when R_{4b} is selected from -NR₆R₇, -C(O)NR₆R₇, or -SO₂NR₆R₇, R₆ and R₇ together with the N atom to which they are attached can form a 4- to 7-membered heterocycle, the heterocycle can contain 1 to 3 heteroatoms as ring atoms, the heteroatoms can be independently selected from N, O, and S atoms, and the 4- to 7-membered heterocycle is optionally substituted by 1 to 3 R;
each R_{4c} is independently selected from hydrogen, C₁₋₃ alkyl, C₃₋₆ cycloalkyl, 3- to 6-membered heterocycloalkyl, phenyl, phenyl(C₁₋₃)alkyl, 5- to 10-membered heteroaryl, or 5- to 10-membered heteroaryl(C₁₋₃)alkyl, each of which is optionally substituted by 1 to 3 R;
each R is independently selected from hydrogen, halogen, cyano, hydroxyl, amino, C₁₋₃ alkyl, C₁₋₃ alkoxy, C₁₋₆ alkylamino, C₃₋₆ cycloalkyl, or 3- to 6-membered heterocycloalkyl, and the C₁₋₃ alkyl, C₁₋₃ alkoxy, C₁₋₆ alkylamino, C₃₋₆ cycloalkyl, or 3- to 6-membered heterocycloalkyl is optionally substituted by 1 to 3 R';
each R₄ₐ is independently selected from hydrogen, oxo, cyano, C₁₋₃ alkyl, C₃₋₆ cycloalkyl, 3- to 6-membered heterocycloalkyl, or 5- to 6-membered heteroaryl; the C₁₋₃ alkyl, C₃₋₆ cycloalkyl, 3- to 6-membered heterocycloalkyl, or 5- to 6-membered heteroaryl is optionally substituted by 1 to 3 R', and the R' is as defined in any one of the embodiments of the present disclosure;
n is selected from 0, 1, 2, 3, or 4;
p and q are each independently selected from 0, 1, or 2, and p and q are not both 2.

In a certain embodiment of the present disclosure, the structural moiety (II)

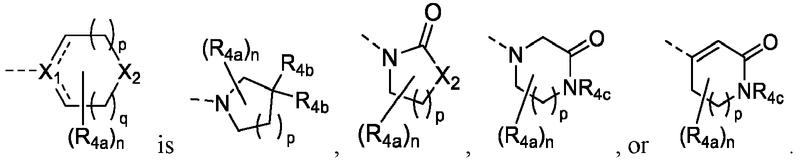

In a certain embodiment of the present disclosure, the structural moiety (II)

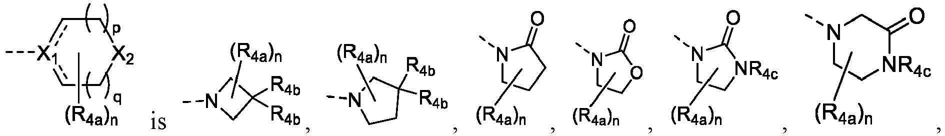

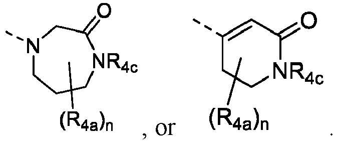

In a certain embodiment of the present disclosure, the structural moiety 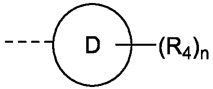 is structural moiety (III) 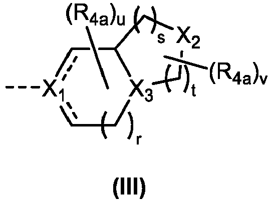 wherein
X₁ is N or C; 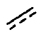
is 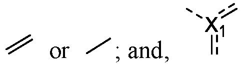 is 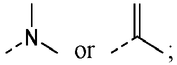
X₂ is a carbon atom substituted by one or two independent R_{4b}, -NR_{4c}-, -O-, or -S(O)_{w}-, and the R_{4b} is hydrogen, -OR₆, -NR₆R₇, -NR₆COR₅, -NR₆SO₂R₅, -C(O)NR₆R₇, -SO₂R₅, or - SO₂NR₆R₇;
R₅ is C₁₋₃ alkyl, C₃₋₆ cycloalkyl, 3- to 6-membered heterocycloalkyl, or 5- to 10-membered heteroaryl, each of which is optionally substituted by 1 to 3 R';
R₆ and R₇ are each independently hydrogen, C₁₋₃ alkyl, C₃₋₆ cycloalkyl, 3- to 6-membered heterocycloalkyl, phenyl, phenyl(C₁₋₃)alkyl, 5- to 10-membered heteroaryl, or 5- to 10-membered heteroaryl(C₁₋₃)alkyl, each of which is optionally substituted by 1 to 3 R;
when R_{4b} is -NR₆R₇, -C(O)NR₆R₇, or -SO₂NR₆R₇, R₆ and R₇ together with the N atom to which they are attached can form a 4- to 7-membered heterocycle, the heterocycle can contain 1 to 3 heteroatoms as ring atoms, the heteroatoms can be independently selected from N, O, and S atoms, and the 4- to 7-membered heterocycle is optionally substituted by 1 to 3 R;
the R_{4c} is hydrogen, C₁₋₃ alkyl, C₃₋₆ cycloalkyl, 3- to 6-membered heterocycloalkyl, phenyl, phenyl(C₁₋₃)alkyl, 5- to 10-membered heteroaryl, or 5- to 10-membered heteroaryl(C₁₋₃)alkyl, each of which is optionally substituted by 1 to 3 R;
each R is independently hydrogen, halogen, cyano, hydroxyl, amino, C₁₋₃ alkyl, C₁₋₃ alkoxy, C₁₋₆ alkylamino, C₃₋₆ cycloalkyl, or 3- to 6-membered heterocycloalkyl, and the C₁₋₃ alkyl, C₁₋₃ alkoxy, C₁₋₆ alkylamino, C₃₋₆ cycloalkyl, or 3- to 6-membered heterocycloalkyl is optionally substituted by 1 to 3 R';
X₃ is CR₄ₐ or N;
each R₄ₐ is independently hydrogen, oxo, cyano, C₁₋₃ alkyl, C₃₋₆ cycloalkyl, 3- to 6-membered heterocycloalkyl, phenyl, or 5- to 6-membered heteroaryl; the C₁₋₃ alkyl, C₃₋₆ cycloalkyl, 3- to 6-membered heterocycloalkyl, phenyl, or 5- to 6-membered heteroaryl is optionally substituted by 1 to 3 R', and the R' is as defined in any one of the embodiments of the present disclosure;
u and v are each independently 0, 1, 2, 3, or 4;
r, s, and t are each independently 0, 1, or 2;
w is 0, 1, or 2.

In a certain embodiment of the present disclosure, in the structural moiety (III), each R₄ₐ is independently hydrogen, cyano, C₁₋₃ alkyl, C₃₋₆ cycloalkyl, 3- to 6-membered heterocycloalkyl, or 5- to 6-membered heteroaryl; the C₁₋₃ alkyl, C₃₋₆ cycloalkyl, 3- to 6-membered heterocycloalkyl, or 5- to 6-membered heteroaryl is optionally substituted by 1 to 3 R', and the R' is as defined in any one of the embodiments of the present disclosure.

In a certain embodiment of the present disclosure, the structural moiety (III) 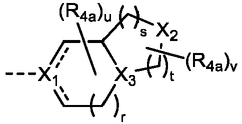 is 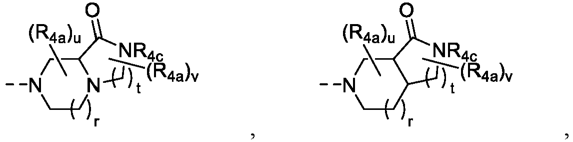 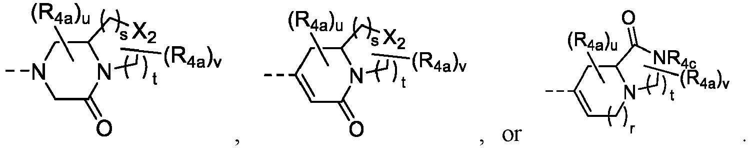 Preferably, the structural moiety (III) 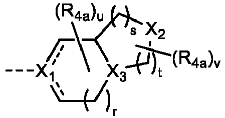 is

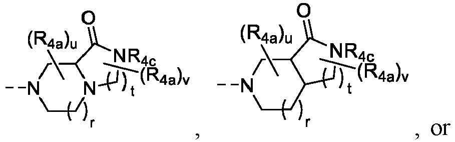

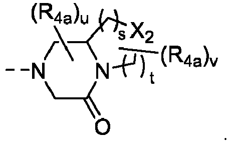

In a certain embodiment of the present disclosure, the structural moiety (III) 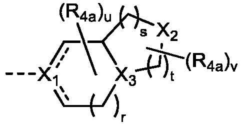 is 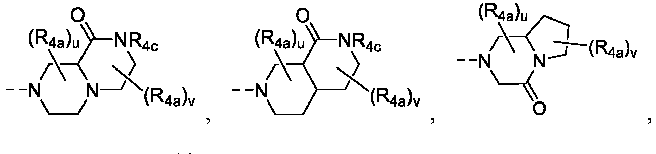 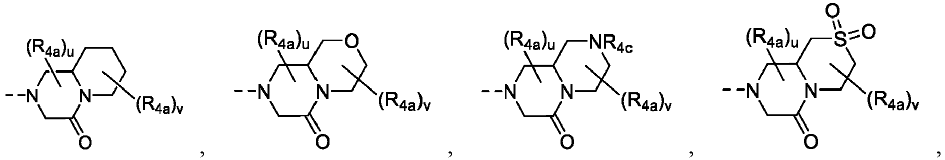 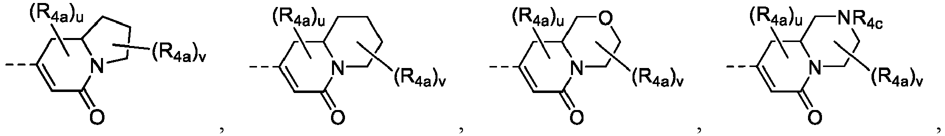 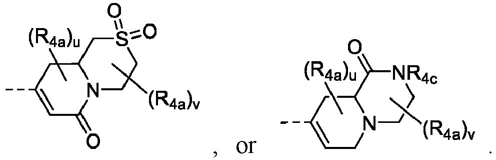 Preferably, the structural moiety (III)

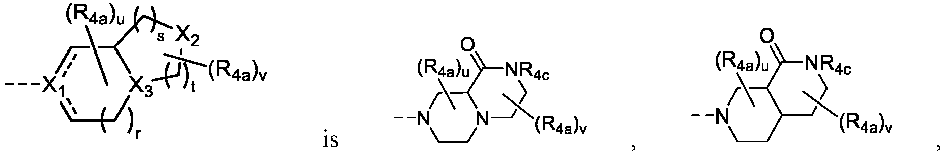

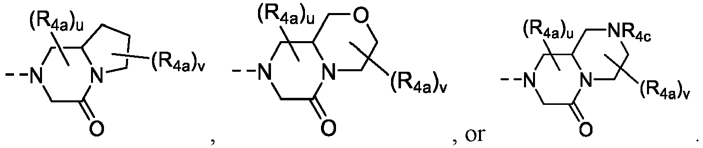

In a certain embodiment of the present disclosure, the structural moiety 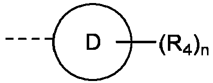 is structural moiety (IV) 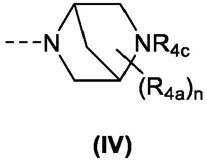 wherein
R_{4c} is hydrogen, C₁₋₃ alkyl, C₃₋₆ cycloalkyl, 3- to 6-membered heterocycloalkyl, phenyl, phenyl(C₁₋₃)alkyl, 5- to 10-membered heteroaryl, or 5- to 10-membered heteroaryl(C₁₋₃)alkyl, each of which is optionally substituted by 1 to 3 R;
each R is independently hydrogen, halogen, cyano, hydroxyl, amino, C₁₋₃ alkyl, C₁₋₃ alkoxy, C₁₋₆ alkylamino, C₃₋₆ cycloalkyl, or 3- to 6-membered heterocycloalkyl, and the C₁₋₃ alkyl, C₁₋₃ alkoxy, C₁₋₆ alkylamino, C₃₋₆ cycloalkyl, or 3- to 6-membered heterocycloalkyl is optionally substituted by 1 to 3 R';
each R₄ₐ is independently hydrogen, cyano, oxo, C₁₋₃ alkyl, C₃₋₆ cycloalkyl, 3- to 6-membered heterocycloalkyl, or 5- to 6-membered heteroaryl; the C₁₋₃ alkyl, C₃₋₆ cycloalkyl, 3-to 6-membered heterocycloalkyl, or 5- to 6-membered heteroaryl is optionally substituted by 1 to 3 R', and the R' is as defined in any one of the embodiments of the present disclosure;
n is selected from 0, 1, 2, 3, or 4.

In a certain embodiment of the present disclosure, the structural moiety 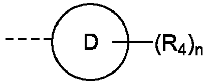 is structural moiety (V) 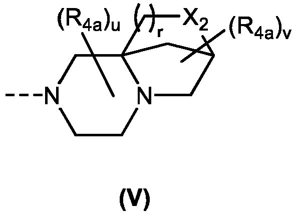 wherein
X₂ is a carbon atom substituted by one or two independent R₄ₐ or -O-,
each R₄ₐ is independently hydrogen, cyano, C₁₋₃ alkyl, C₃₋₆ cycloalkyl, 3- to 6-membered heterocycloalkyl, or 5- to 6-membered heteroaryl, or two R₄ₐ attached to the same carbon atom together form an oxo(C=O) group; the C₁₋₃ alkyl, C₃₋₆ cycloalkyl, 3- to 6-membered heterocycloalkyl, or 5- to 6-membered heteroaryl is optionally substituted by 1 to 3 R', and the R' is as defined in any one of the embodiments of the present disclosure;
u and v are each independently selected from 0, 1, 2, 3, or 4;
r is selected from 0 or 1.

In a certain embodiment of the present disclosure, the structural moiety (V) 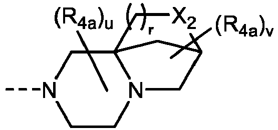 is

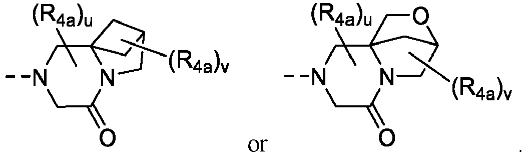

In a certain embodiment of the present disclosure, the structural moiety 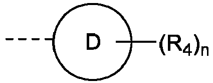 is structural moiety (VI) 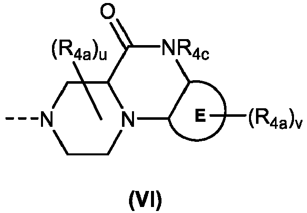 wherein
ring E is a 5- to 6-membered saturated cycloalkyl ring, a benzene ring, or a 5- to 6-membered heteroaryl ring;
each R₄ₐ is independently hydrogen, oxo, halogen, cyano, C₁₋₃ alkyl, C₃₋₆ cycloalkyl, 3- to 6-membered heterocycloalkyl, or 5- to 6-membered heteroaryl; the C₁₋₃ alkyl, C₃₋₆ cycloalkyl, 3- to 6-membered heterocycloalkyl, or 5- to 6-membered heteroaryl is optionally substituted by 1 to 3 R', and the R' is as defined in any one of the embodiments of the present disclosure;
R_{4c} is hydrogen, C₁₋₃ alkyl, C₃₋₆ cycloalkyl, or 3- to 6-membered heterocycloalkyl; preferably, R_{4c} is hydrogen;
u and v are each independently 0, 1, 2, 3, or 4.

In a certain embodiment of the present disclosure, the structural moiety (VI) 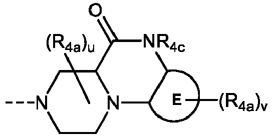 is

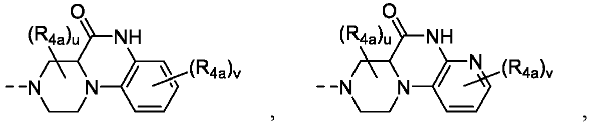

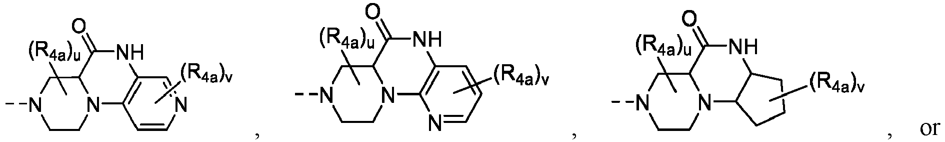

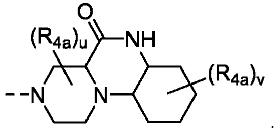

In a certain embodiment of the present disclosure, each R₄ₐ is independently H, =O, - F, -Cl, -Me, -*i*-Pr, -*t*-Bu, -CH₂CH(CH₃)₂, -COOH, -CN, -CH₂CN, -CH₂OH, -(CH₂)₂OH, - CH₂OCH₃, -CONH₂, -CONHCH₃, -CON(CH₃)₂, -CONHCH₂CH₃, -CH₂CONHCH₂CH₃, 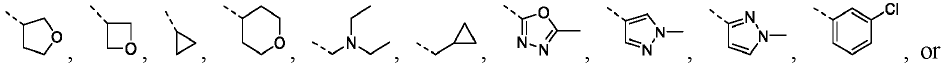 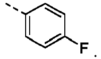 Preferably, each R₄ₐ is independently H, =O, -F, -Me, -*i*-Pr, -*t*-Bu, -CH₂CH(CH₃)₂, -COOH, -CN, -CH₂OH, -CONH₂, -CONHCH₂CH₃, -CON(CH₃)₂, -CH₂CONHCH₂CH₃,

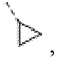

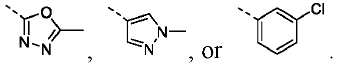

In a certain embodiment of the present disclosure, each R_{4b} is independently H, =O, - OCH₃, -NMe₂, -NHCOCH₃, -NHSO₂CH₃, -SO₂Me, -COOH, -CONH₂, -CONHCH₃, - CON(CH₃)₂, -CONHCH₂CH₃, -SO₂NHCH₃, 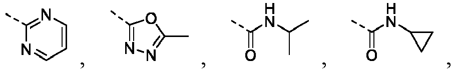 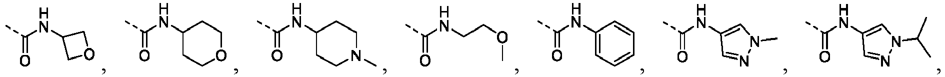 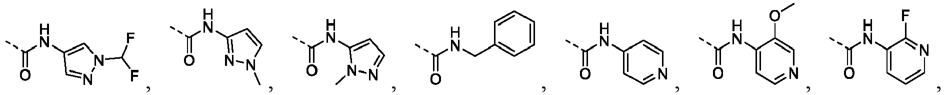 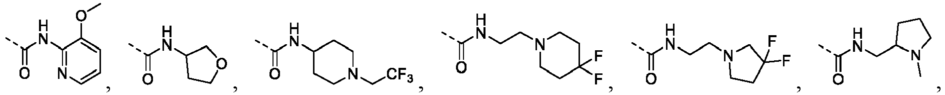 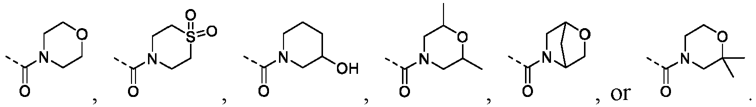 Preferably, each R_{4b} is independently H, -OCH₃, -NMe₂, -NHCOCH₃, -COOH, -CONHCH₂CH₃, -SO₂NHCH₃,

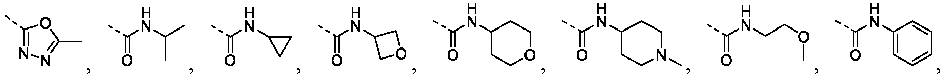

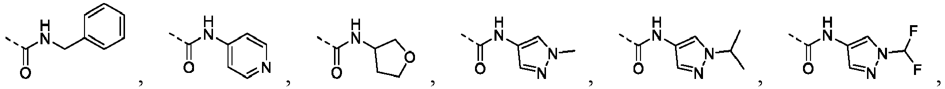

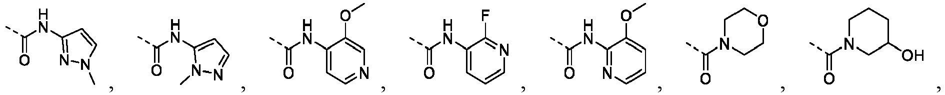

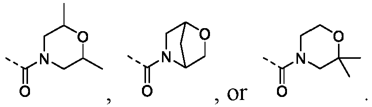

In a certain embodiment of the present disclosure, each R_{4c} is independently H, -Me, -Et, -i-Pr, -CF₃, -CHF₂, -CH₂CF₃, -CH₂CF₂H, -CH₂CN, -(CH₂)₂CN, -(CH₂)₃CN, -(CH₂)₂NH₂, -(CH₂)₂OH, -(CH₂)₂OCH₃, -(CH₂)₃OCH₃, -COCH₃, -COCH(CH₃)₂, -SO₂NHCH₃, -SO₂CH₃, 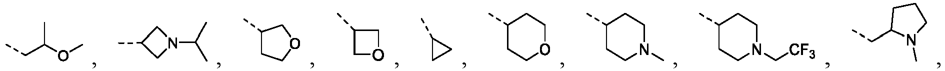 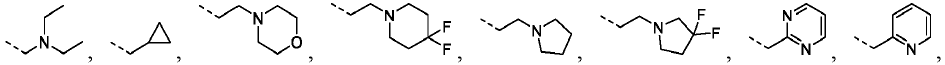 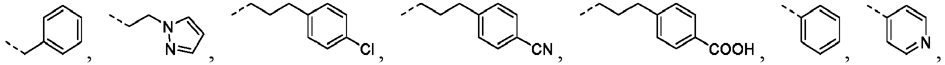 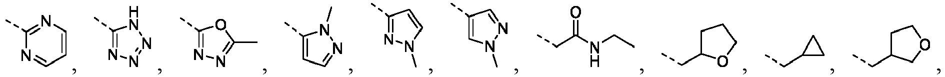 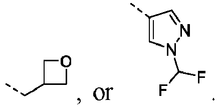 Preferably, each R_{4c} is independently H, -Me, -CH₂CF₃, -CH₂CN, - (CH₂)₃CN, -(CH₂)₂NH₂, -(CH₂)₂OH, -(CH₂)₂OCH₃, -(CH₂)₃OCH₃, -SO₂CH₃, -COCH₃,

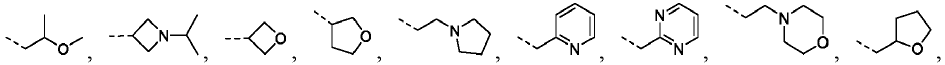

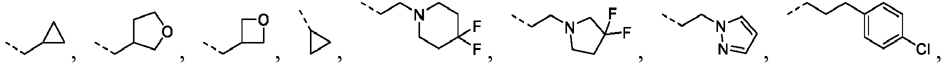

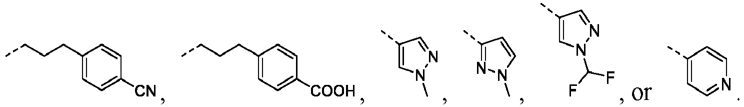

In a certain embodiment of the present disclosure, each R₁ is independently H, F, Cl, I Me, CN, -CH₂F, -CF₂H, CF₃, -OCF₂H, -CH(CN)₂, -CH₂OH, -CH₂OCH₃, or 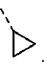 . Preferably, each R₁ is independently F, Cl, Me, -CH₂F, -CF₂H, CF₃, CN, -CH(CN)₂, -CHzOH, -CH₂OCH₃, or -OCF₂H.

In a certain embodiment of the present disclosure, ring A is a benzene ring, a pyridine ring, or a pyrimidine ring; preferably, ring A is a benzene ring or a pyridine ring.

In a certain embodiment of the present disclosure, the structural moiety 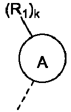 is selected from 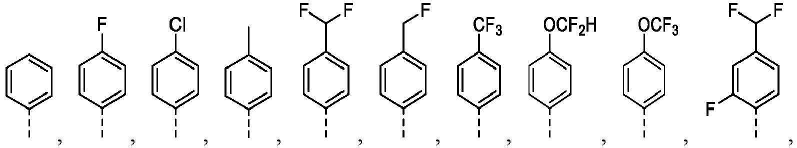 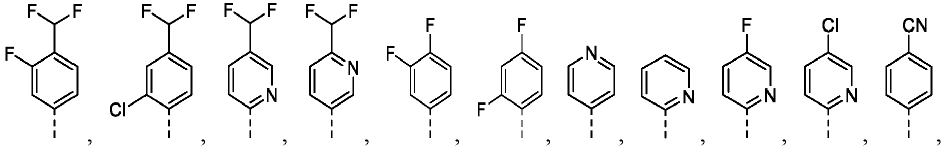 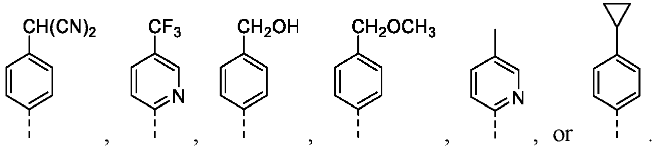 Preferably, the structural moiety 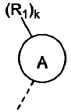 is selected from

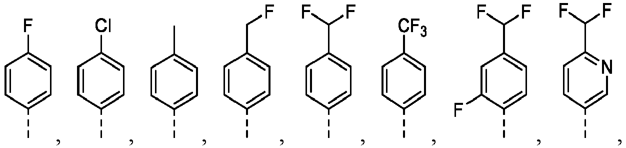

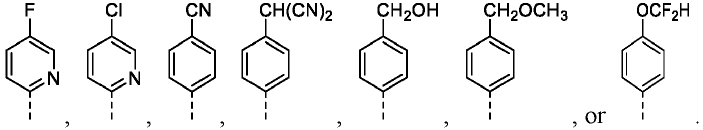

In a certain embodiment of the present disclosure, the structural moiety 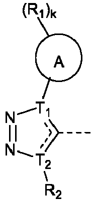 is selected from 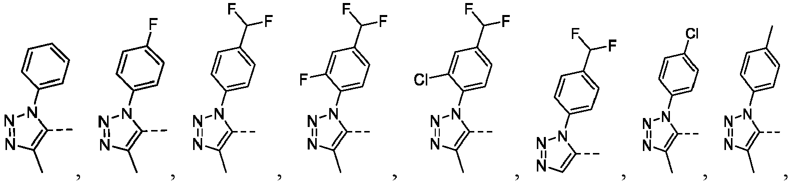 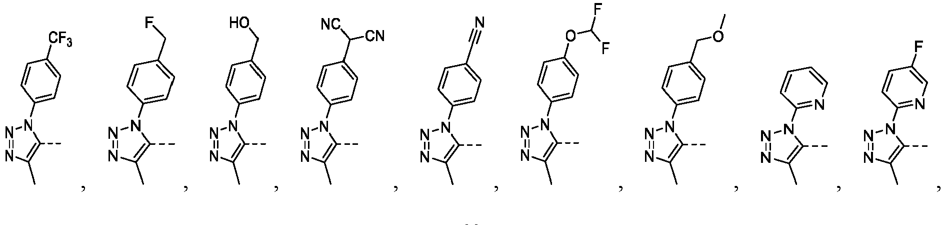 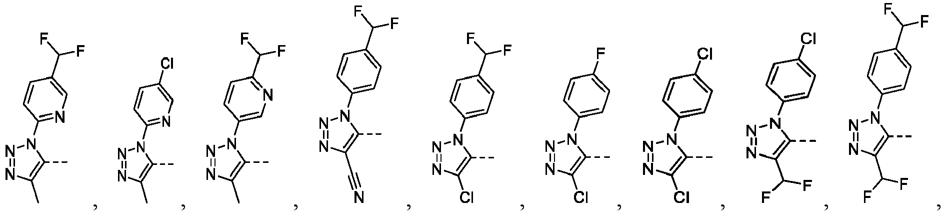 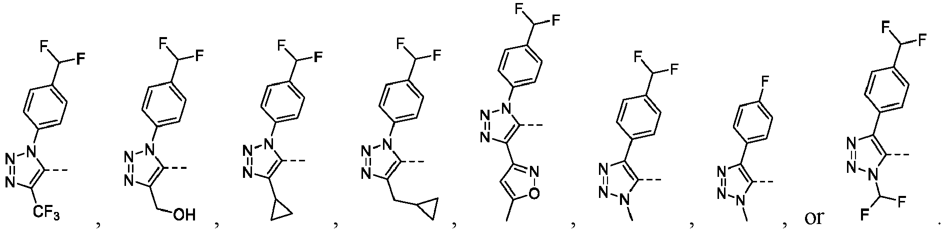 Preferably, the structural moiety 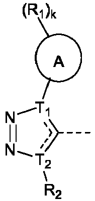 is selected from

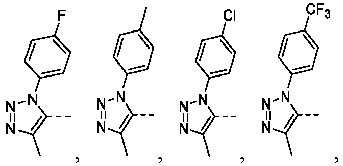

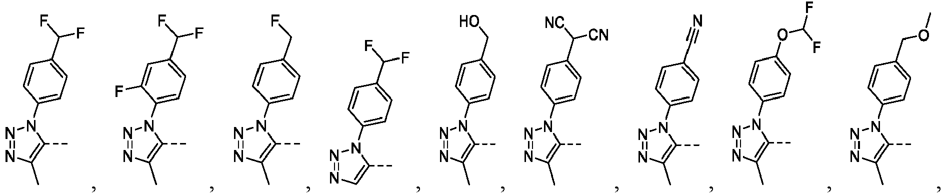

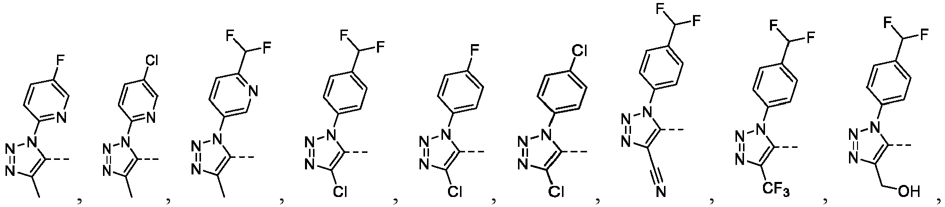

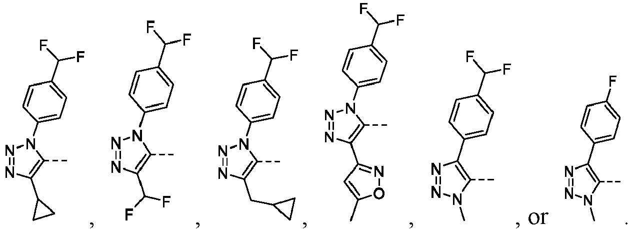

In a certain embodiment of the present disclosure, L is -CH₂-O-.

In a certain embodiment of the present disclosure, ring B is a benzene ring, a pyridine ring, a pyridazine ring, a pyrazine ring, or a pyridopyridazine ring; preferably, ring B is a pyridine ring, a pyridazine ring, a pyrazine ring, or a pyridopyridazine ring; for example, ring B is a pyridine ring, a pyridazine ring, or a pyridopyridazine ring.

In a certain embodiment of the present disclosure, each R₃ is independently H, F, Cl, CN, Me, or -OCH₃; more preferably, each R₃ is independently H, CN, Me, or -OCH₃.

In a certain embodiment of the present disclosure, the structural moiety 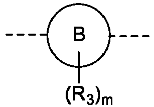 is 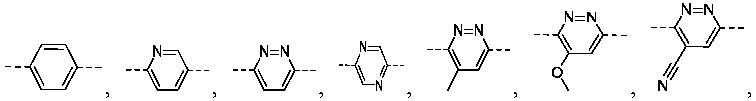 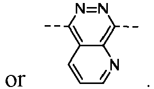 Preferably, the structural moiety

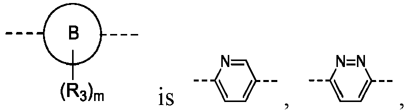

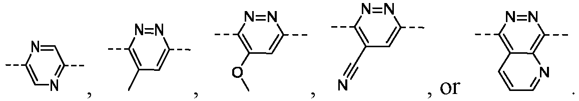

In a certain embodiment of the present disclosure, ring D is 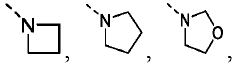 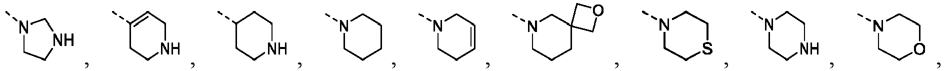 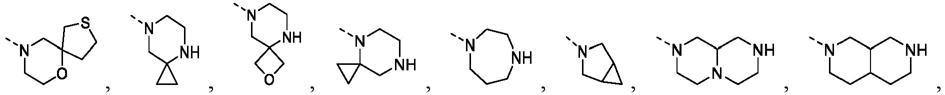 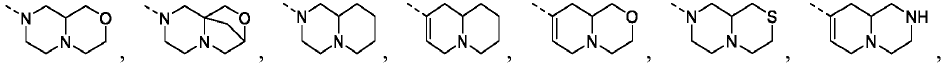 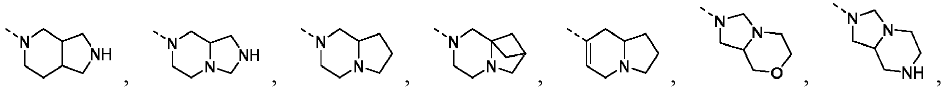 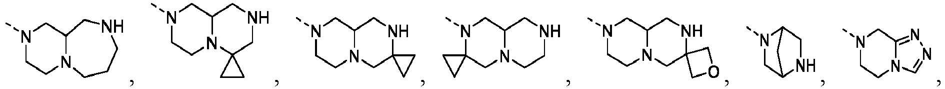 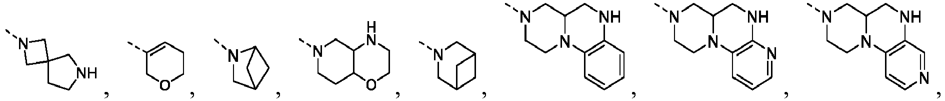 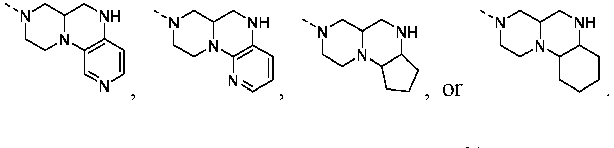 Preferably, ring D is

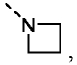

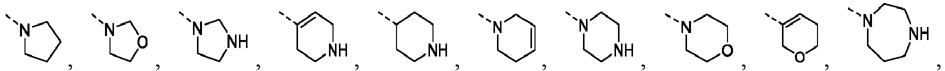

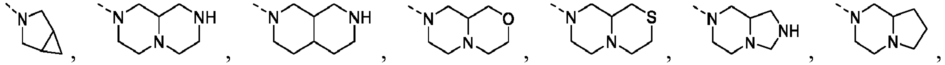

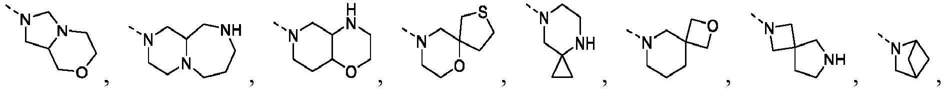

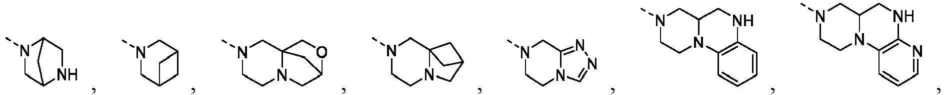

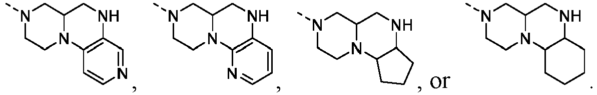

In a certain embodiment of the present disclosure, each R₄ is independently H, F, Cl, =O, -Me, -COOH, -Et, -i-Pr, -t-Bu, -CH₂CH(CH₃)₂, -CF₃, -CHF₂, -CH₂CF₃, -CH₂CF₂, -CN, - CH₂CN, -(CH₂)₂CN, -(CH₂)₃CN, -CH₂OH, -(CH₂)₂OH, -CH₂OCH₃, -OCH₃, -NMe₂, - NHCOCH₃, -NHSO₂CH₃, -SO₂Me, -CONH₂, -CONHCH₃, -CON(CH₃)₂, -CONHCH₂CH₃, - SO₂NHCH₃, -COCH(CH₃)₂, -CONHCH₂CH₃, -CH₂CONHCH₂CH₃, -(CH₂)₂NH₂, - (CH₂)₂OCH₃, -(CH₂)₃OCH₃, -COCH₃, 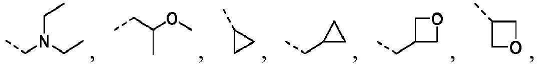 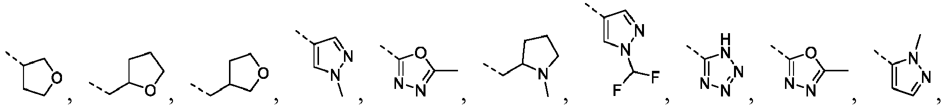 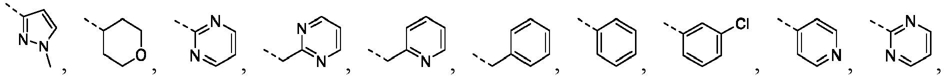 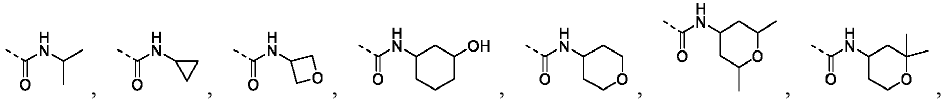 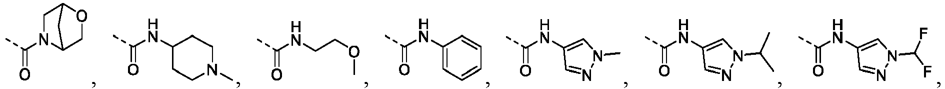 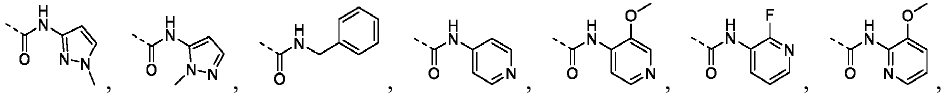 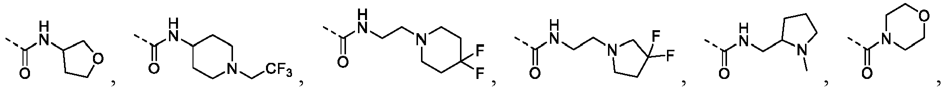 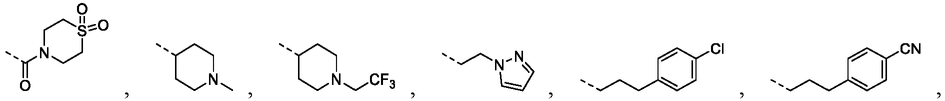 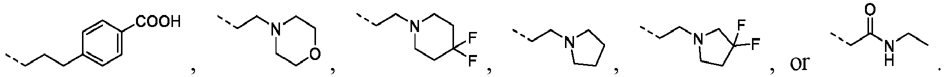 Preferably, each R₄ is independently H, F, =O, -Me, -i-Pr, -t-Bu, -CH₂CH(CH₃)₂, -OCH₃, - (CH₂)₂OCH₃, -(CH₂)₃OCH₃, -NMe₂, -COOH, -CN, -CH₂CN, -CH₂OH, -CONH₂, -CONHCH₃, -CONHCH₂CH₃, -CON(CH₃)₂, -CH₂CONHCH₂CH₃, -NHCOCH₃, -CH₂CF₃, -(CH₂)₂NH₂, - (CH₂)₂OH, -SO₂CH₃, -SO₂NHCH₃, -(CH₂)₃CN, -COCH₃,

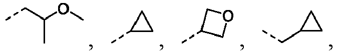

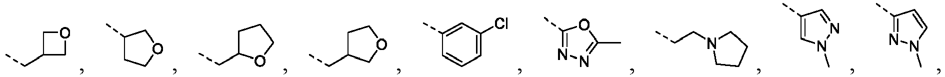

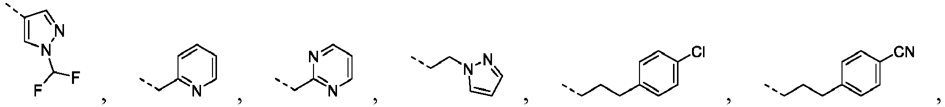

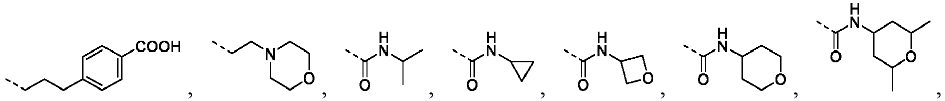

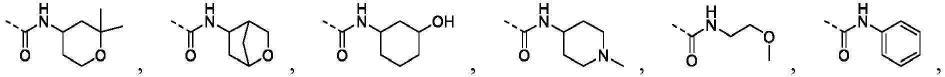

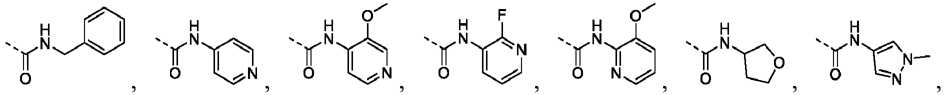

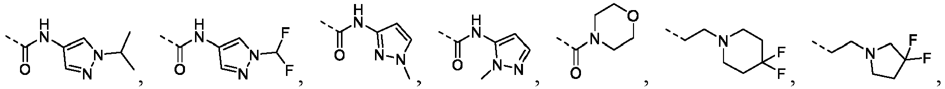

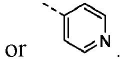

In a certain embodiment of the present disclosure, the structural moiety 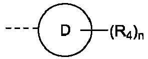 is the following substituents: 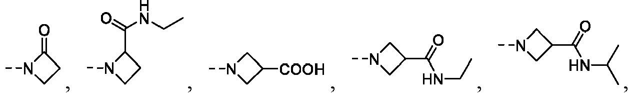 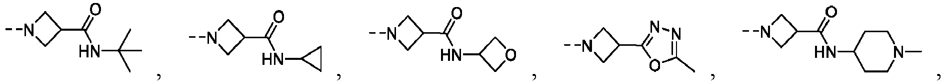 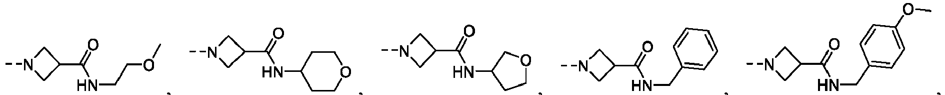 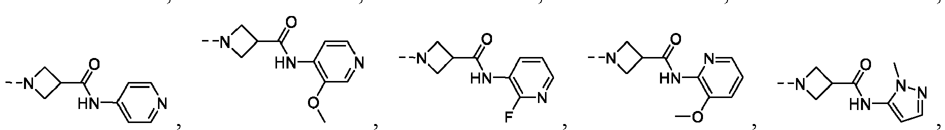 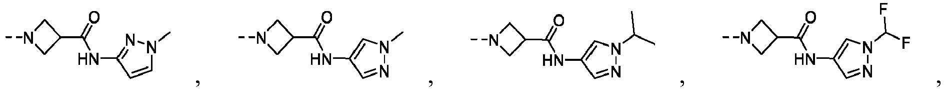 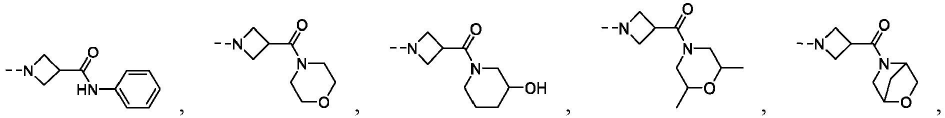 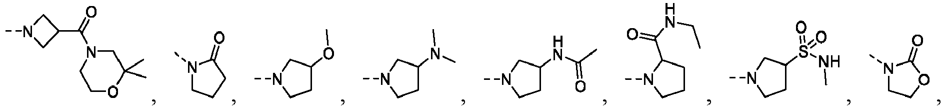 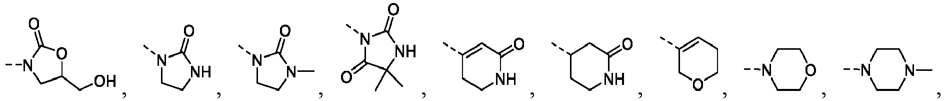 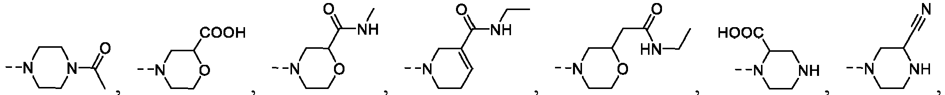 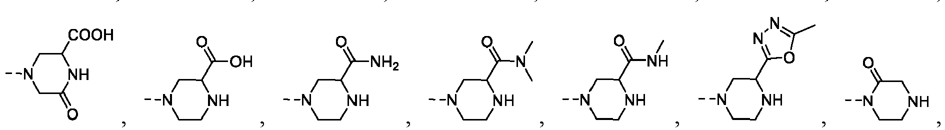 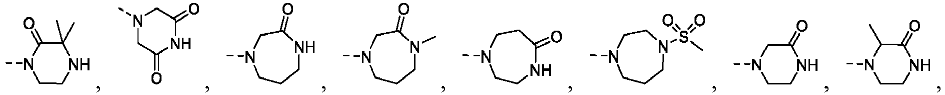 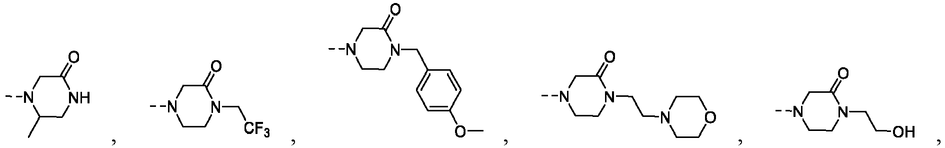 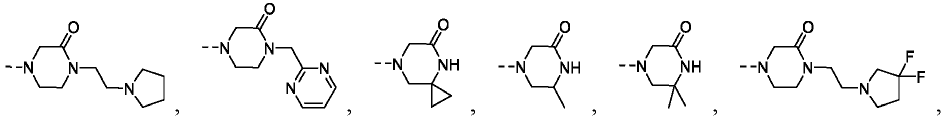 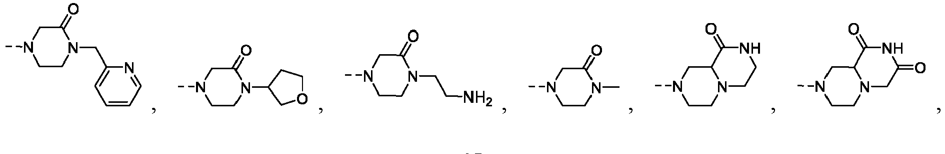 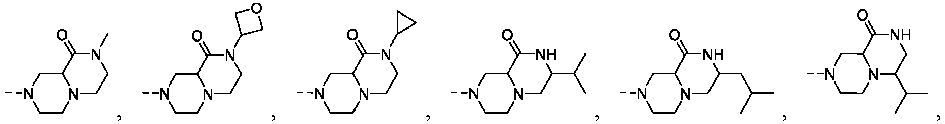 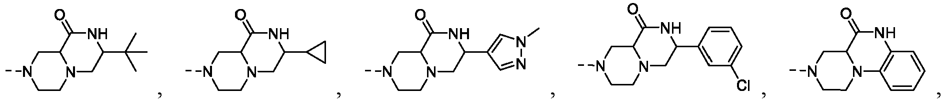 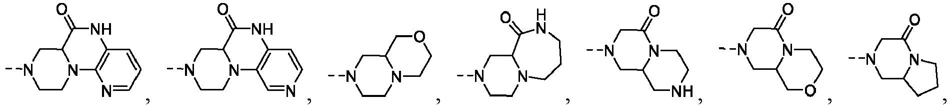 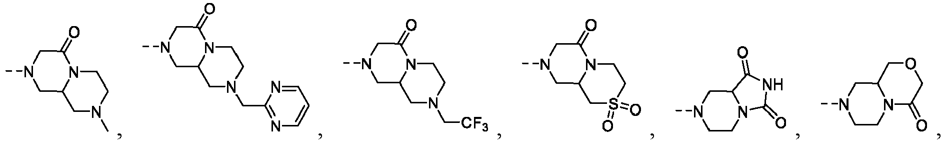 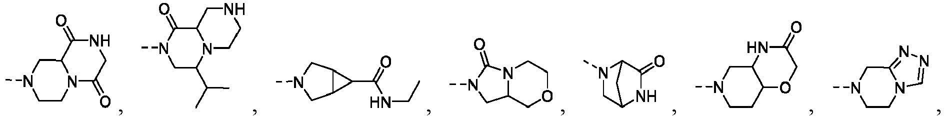 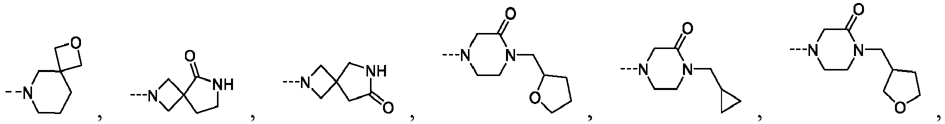 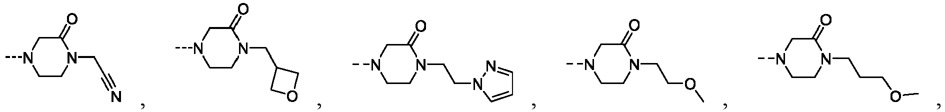 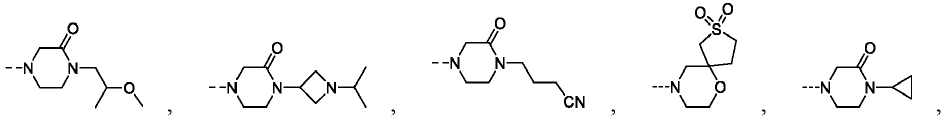 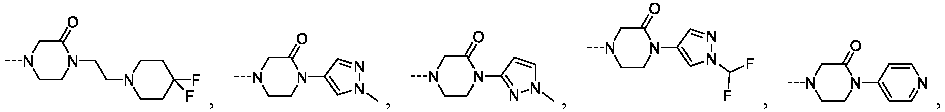 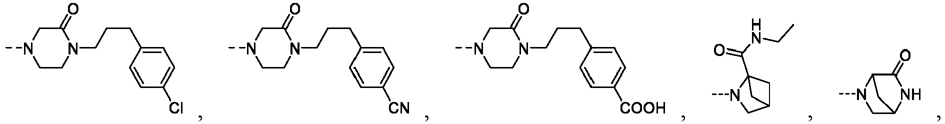 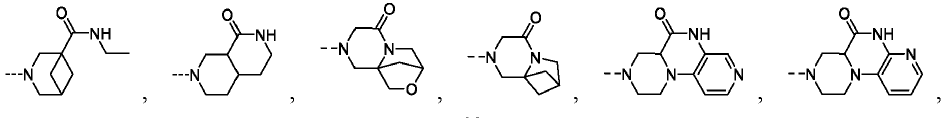 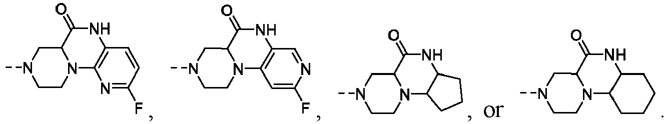 Preferably, the structural moiety 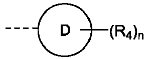 is selected from the following substituents:

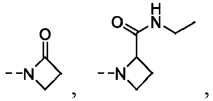

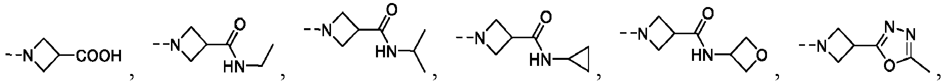

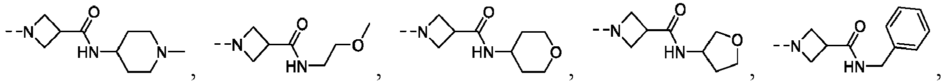

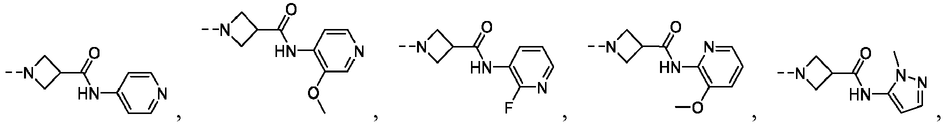

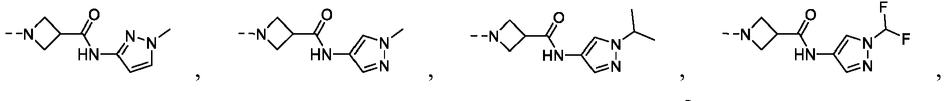

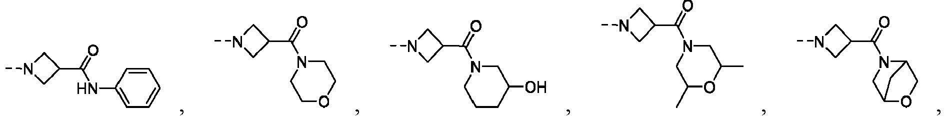

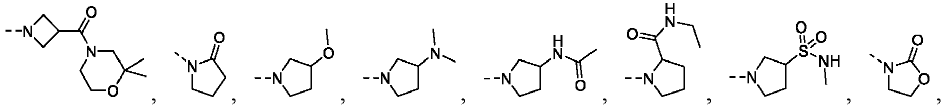

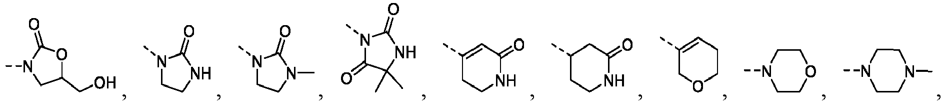

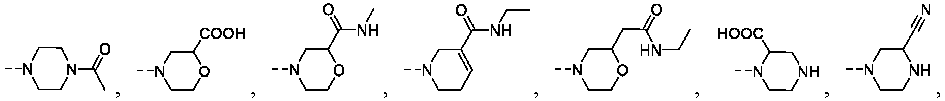

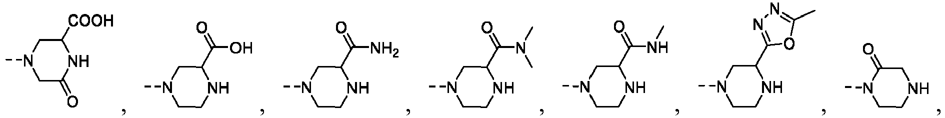

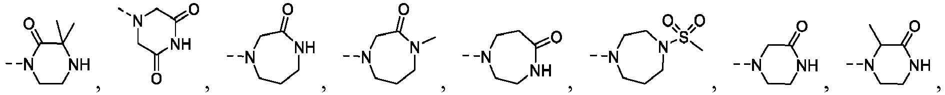

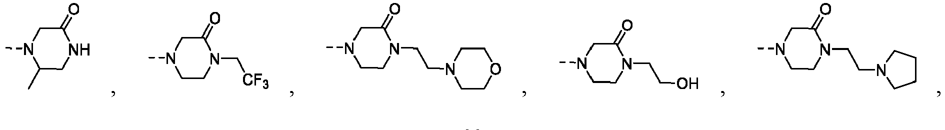

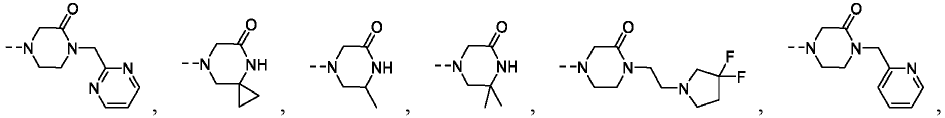

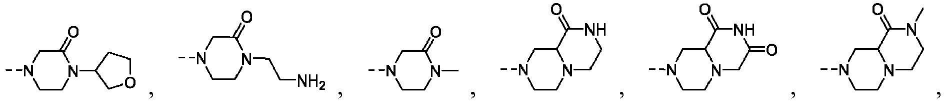

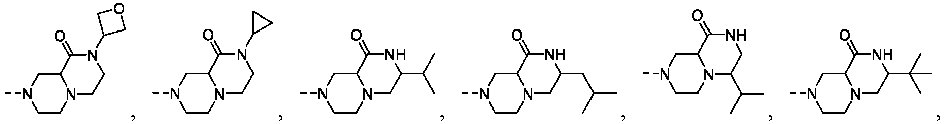

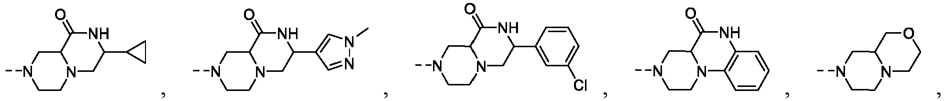

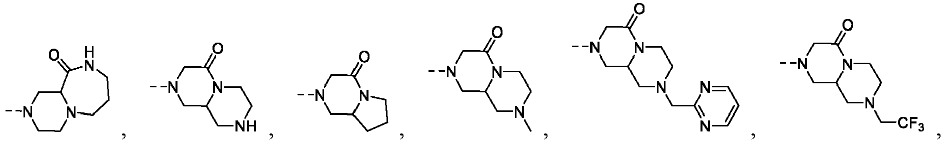

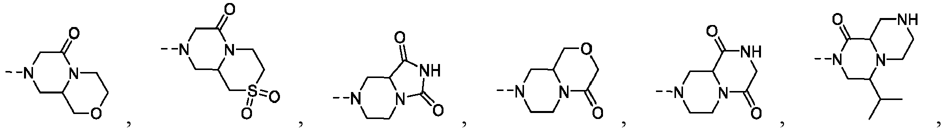

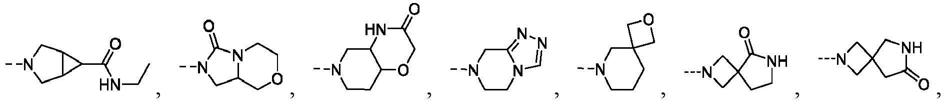

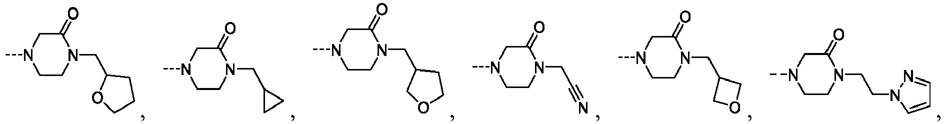

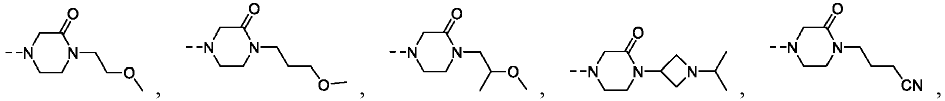

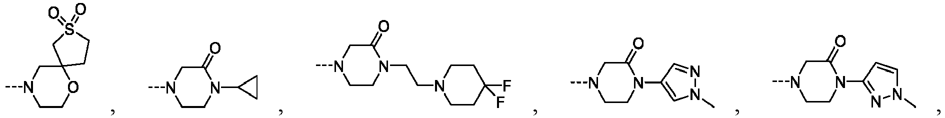

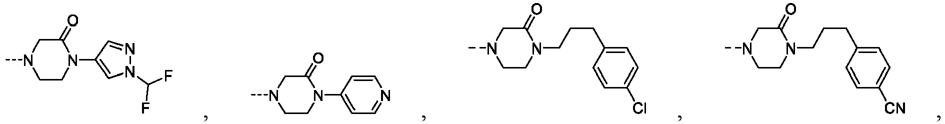

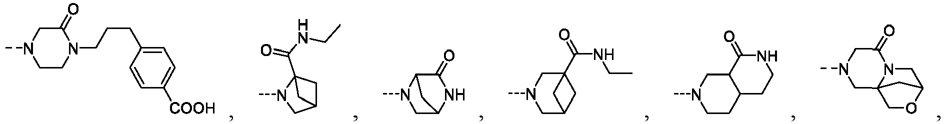

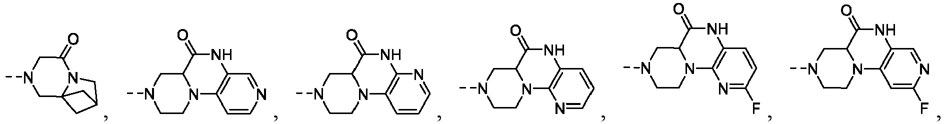

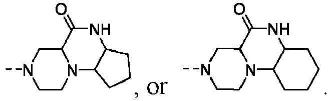

In a certain embodiment of the present disclosure, the compound of formula I is a compound of formula I-A: 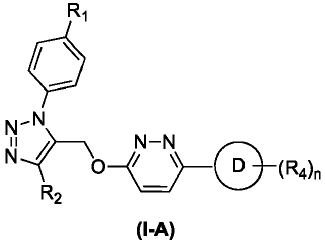 wherein

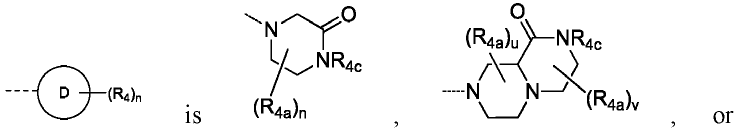

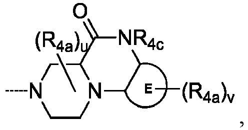

R₁, R₂, R₄ₐ, R_{4c}, u, v, n, and ring E are as defined in any embodiment of the present disclosure.

In a certain embodiment of the present disclosure, in 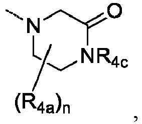 R_{4c} is hydrogen, C₁₋₃ alkyl, C₃₋₆ cycloalkyl, 5- to 10-membered heteroaryl, 5- to 10-membered heteroaryl(C₁₋₃)alkyl, or 3- to 6-membered heterocycloalkyl;
in R_{4c}, the C₁₋₃ alkyl is optionally substituted by 1 to 3 independently selected substituents: halogen, cyano, hydroxyl, amino, C₁₋₃ alkyl, C₁₋₃ alkoxy, C₁₋₆ alkylamino, C₃₋₆ cycloalkyl, 3- to 6-membered heterocycloalkyl, or 3- to 6-membered heterocycloalkyl substituted by 1 to 3 halogens;
in R_{4c}, the 3- to 6-membered heterocycloalkyl is optionally substituted by 1 to 3 C₁₋₃ alkyl groups;
in R_{4c}, the 5- to 10-membered heteroaryl is optionally substituted by 1 to 3 independently selected substituents: C₁₋₃ alkyl or C₁₋₃ haloalkyl;
in R_{4c}, the 5- to 10-membered heteroaryl(C₁₋₃)alkyl is optionally substituted by 1 to 3 independently selected substituents: halogen, cyano, or C₁₋₃ alkoxy.

In a certain embodiment of the present disclosure, in 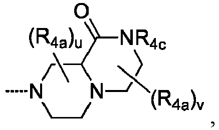 R4C is hydrogen, C₁₋₃ alkyl, C₃₋₆ cycloalkyl, or 3- to 6-membered heterocycloalkyl.

In a certain embodiment of the present disclosure, in 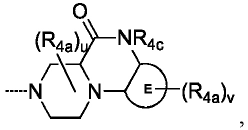 R_{4c} is hydrogen.

In a certain embodiment of the present disclosure, R_{4c} is hydrogen.

In a certain embodiment of the present disclosure, in 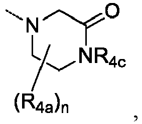 each R₄ₐ is independently hydrogen, oxo, C₁₋₃ alkyl, or COOH, and n is 0, 1, or 2. Preferably, n is 0.

In a certain embodiment of the present disclosure, in 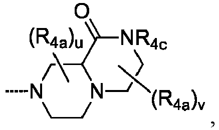 each R₄ₐ is independently hydrogen, oxo, C₁₋₃ alkyl, C₃₋₆ cycloalkyl, phenyl, or 5- to 6-membered heteroaryl;
in R₄ₐ, the C₁₋₃ alkyl is optionally substituted by 1 to 3 C₁₋₃ alkyl groups;
in R₄ₐ, the 5- to 6-membered heteroaryl is optionally substituted by 1 to 3 C₁₋₃ alkyl groups;
in R₄ₐ, the phenyl is optionally substituted by 1 to 3 halogens;
u is 0;
v is 0 or 1. Preferably, R₄ₐ is hydrogen or C₁₋₃ alkyl, and the C₁₋₃ alkyl is optionally substituted by 1 to 3 C₁₋₃ alkyl groups.

In a certain embodiment of the present disclosure, in 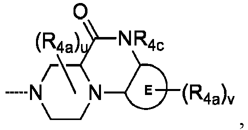 each R₄ₐ is independently hydrogen or halogen; u is 0, and v is 0 or 1. Preferably, u is 0, and v is 0.

In a certain embodiment of the present disclosure, the compound of formula I is selected from the following compounds:

| **Example** | **Structure** | **Example** | **Structure** |
|---|---|---|---|
| 1 | 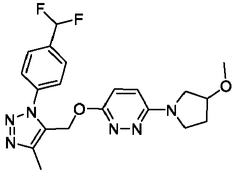 | 109 | 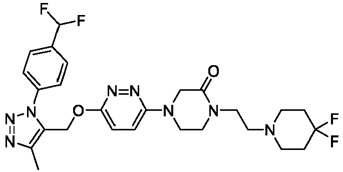 |
| 2 | 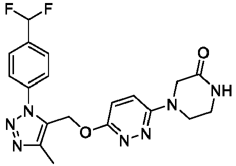 | 110 | 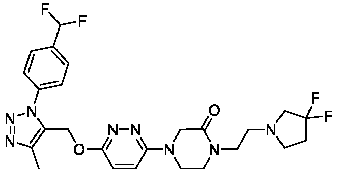 |
| 3 | 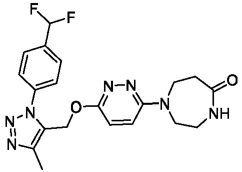 | 111 | 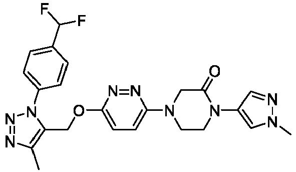 |
| 4 | 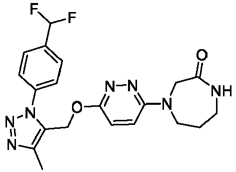 | 112 | 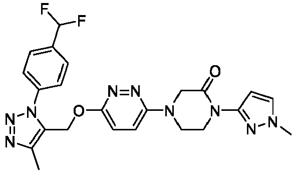 |
| 5 | 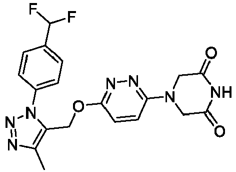 | 113 | 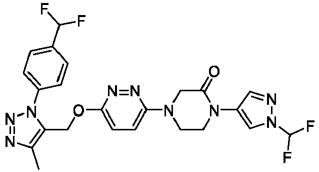 |
| 6 | 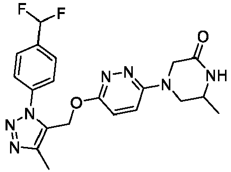 | 114 | 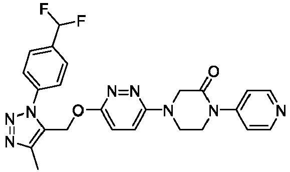 |
| 7 | 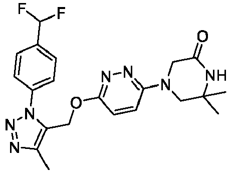 | 115 | 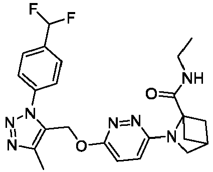 |
| 8 | 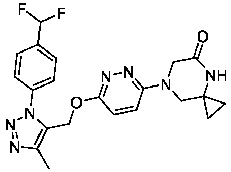 | 116 | 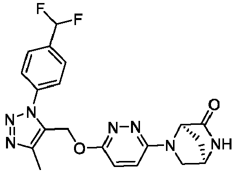 |
| 9 | 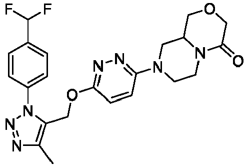 | 117 | 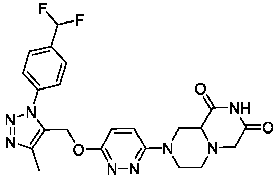 |
| 10 | 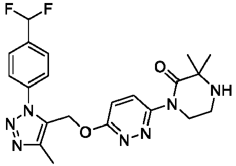 | 118 | 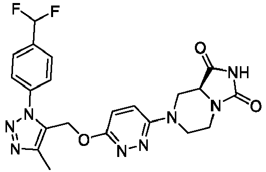 |
| 11 | 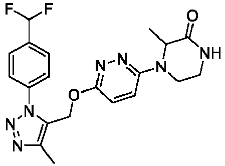 | 119 | 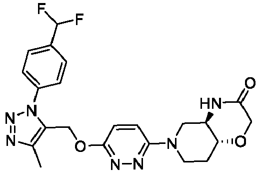 |
| 12 | 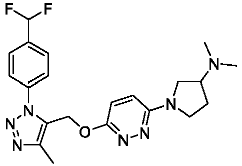 | 120 | 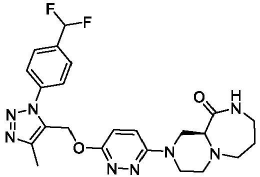 |
| 13 | 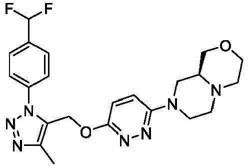 | 121 | 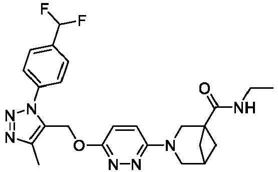 |
| 14 | 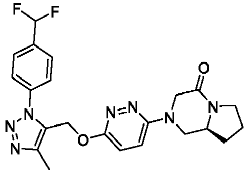 | 122 | 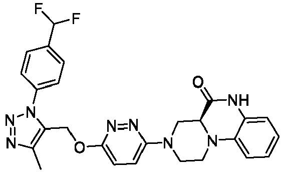 |
| 15 | 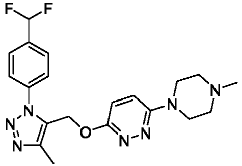 | 123 | 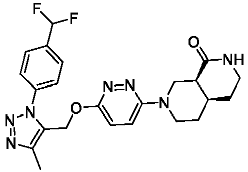 |
| 16 | 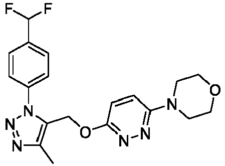 | 124 | 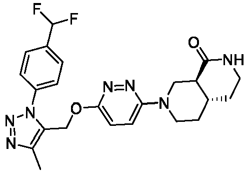 |
| 17 | 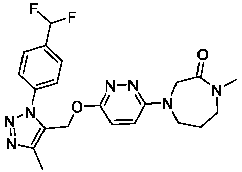 | 125 | 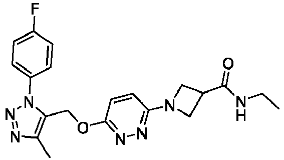 |
| 18 | 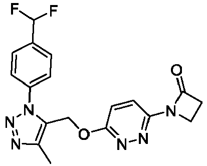 | 126 | 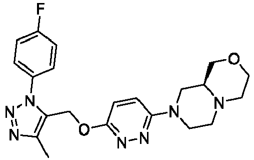 |
| 19 | 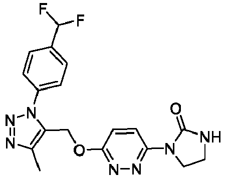 | 127 | 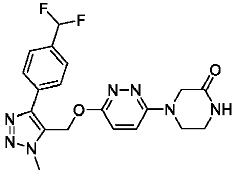 |
| 20 | 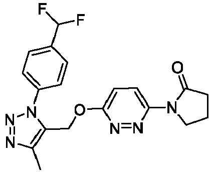 | 128 | 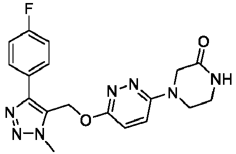 |
| 21 | 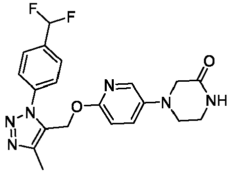 | 129 | 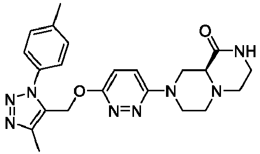 |
| 22 | 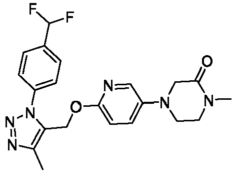 | 130 | 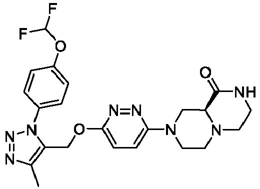 |
| 23 | 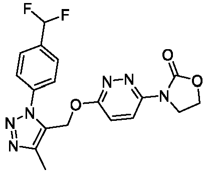 | 131 | 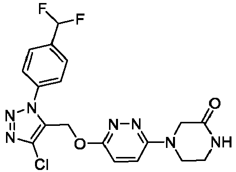 |
| 24 | 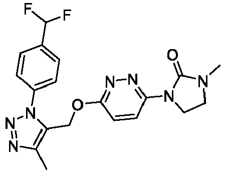 | 132 | 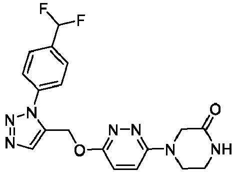 |
| 25 | 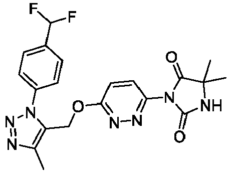 | 133 | 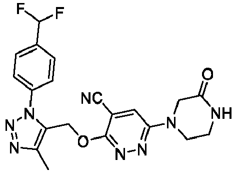 |
| 26 | 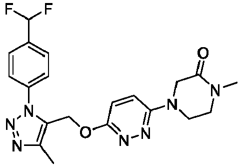 | 134 | 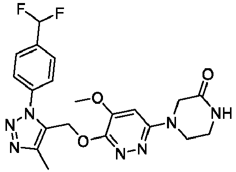 |
| 27 | 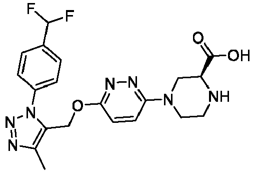 | 135 | 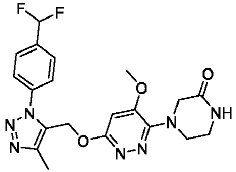 |
| 28 | 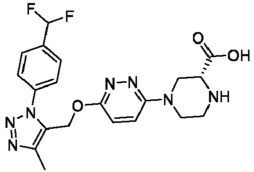 | 136 | 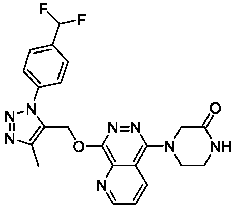 |
| 29 | 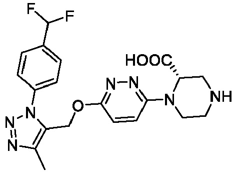 | 137 | 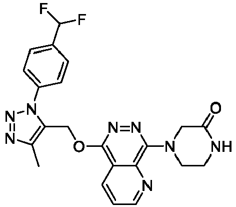 |
| 30 | 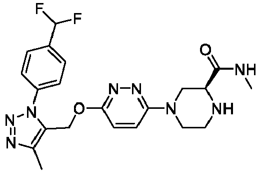 | 138 | 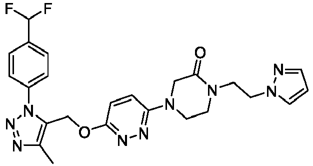 |
| 31 | 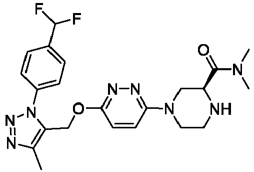 | 139 | 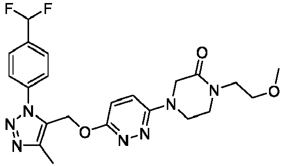 |
| 32 | 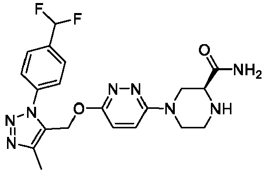 | 140 | 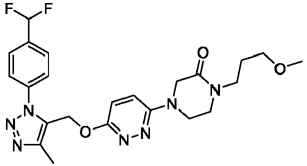 |
| 33 | 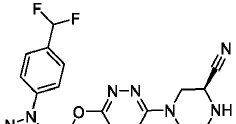 | 141 | 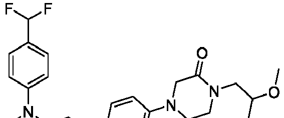 |
| 34 | 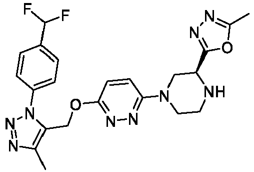 | 142 | 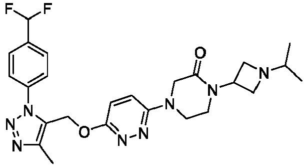 |
| 35 | 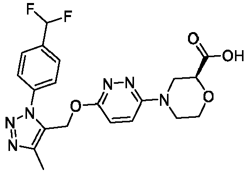 | 143 | 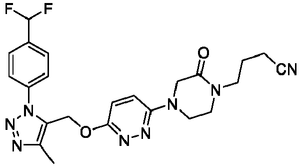 |
| 36 | 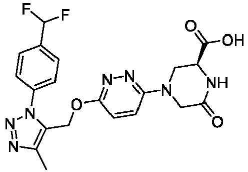 | 144 | 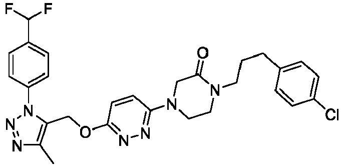 |
| 37 | 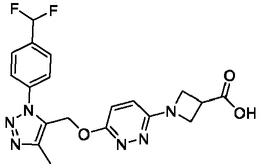 | 145 | 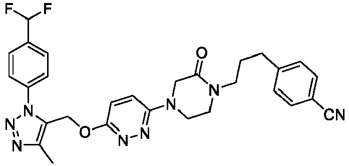 |
| 38 | 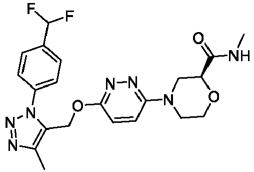 | 146 | 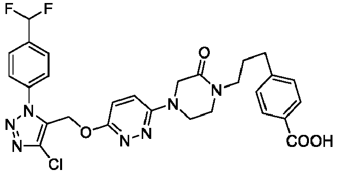 |
| 39 | 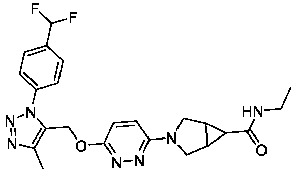 | 147 | 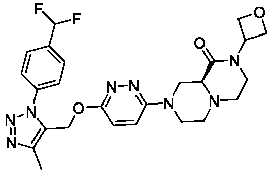 |
| 40 | 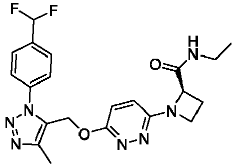 | 148 | 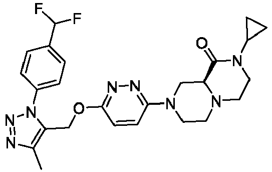 |
| 41 | 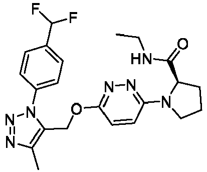 | 149 | 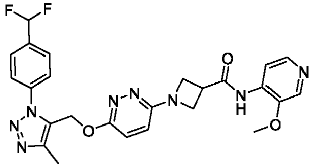 |
| 42 | 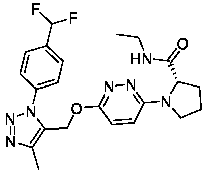 | 150 | 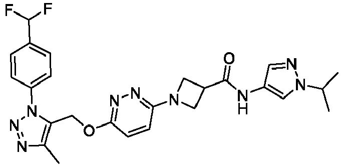 |
| 43 | 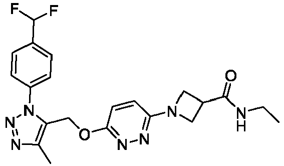 | 151 | 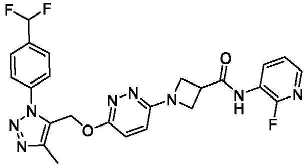 |
| 44 | 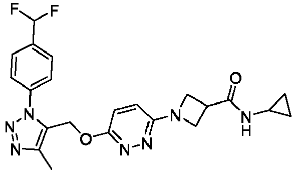 | 152 | 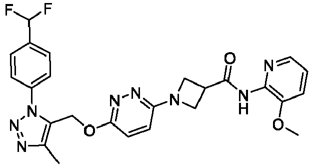 |
| 45 | 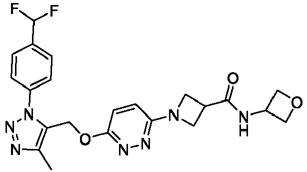 | 153 | 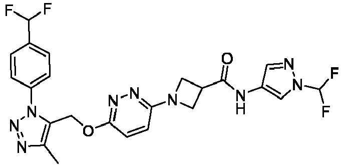 |
| 46 | 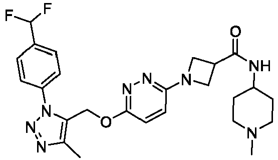 | 154 | 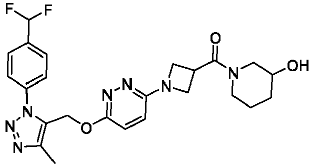 |
| 47 | 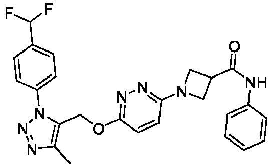 | 155 | 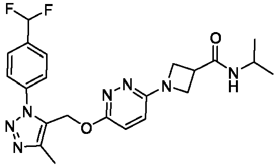 |
| 48 | 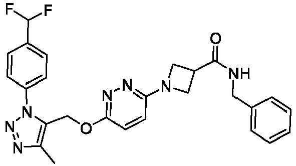 | 156 | 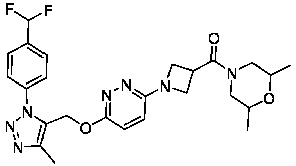 |
| 49 | 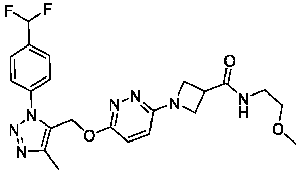 | 157 | 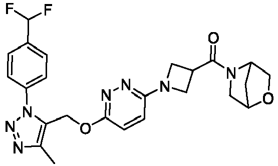 |
| 50 | 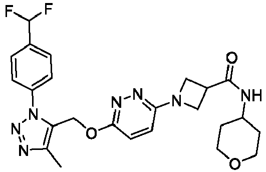 | 158 | 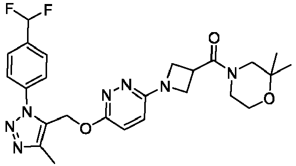 |
| 51 | 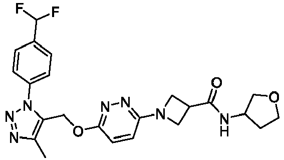 | 159 | 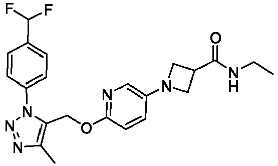 |
| 52 | 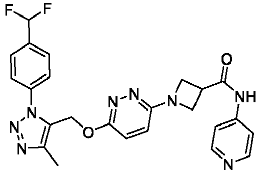 | 160 | 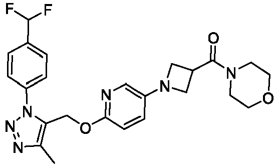 |
| 53 | 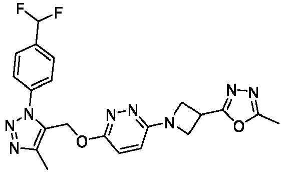 | 161 | 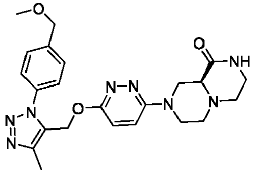 |
| 54 | 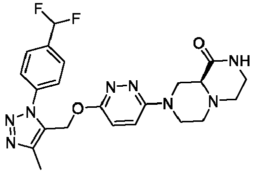 | 162 | 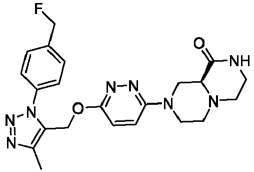 |
| 55 | 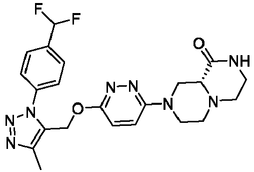 | 163 | 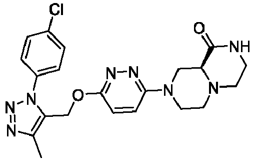 |
| 56 | 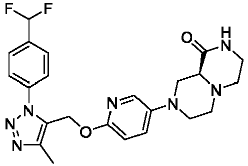 | 164 | 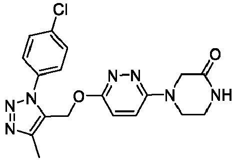 |
| 57 | 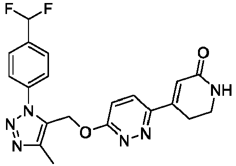 | 165 | 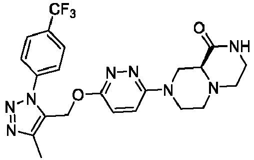 |
| 58 | 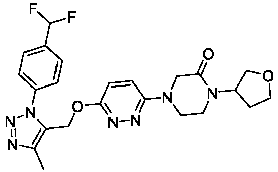 | 166 | 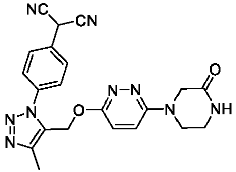 |
| 59 | 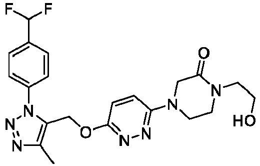 | 167 | 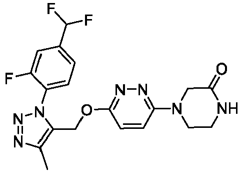 |
| 60 | 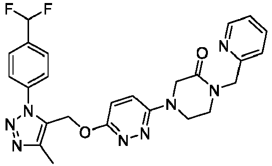 | 168 | 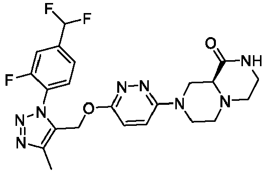 |
| 61 | 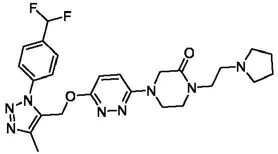 | 169 | 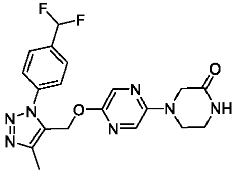 |
| 62 | 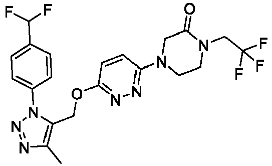 | 170 | 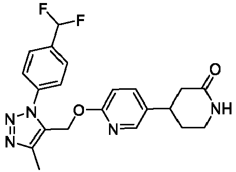 |
| 63 | 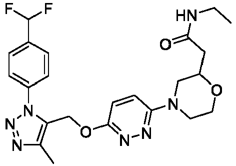 | 171 | 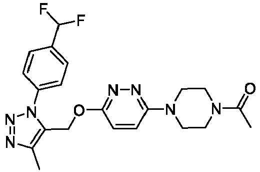 |
| 64 | 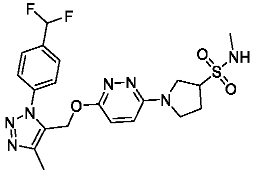 | 172 | 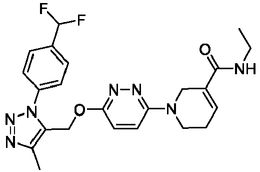 |
| 65 | 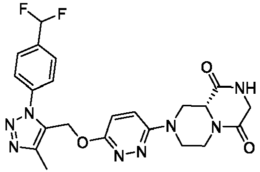 | 173 | 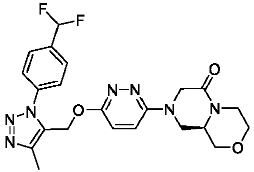 |
| 66 | 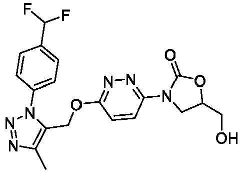 | 174 | 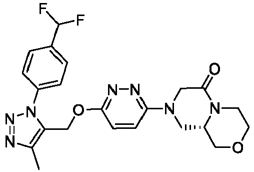 |
| 67 | 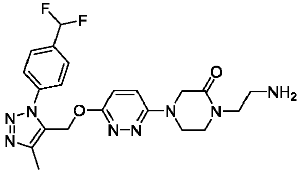 | 175 | 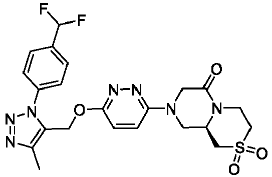 |
| 68 | 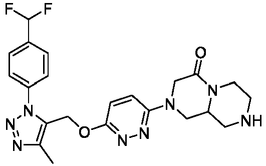 | 176 | 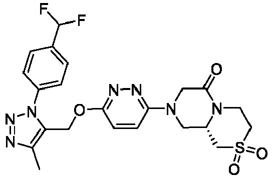 |
| 69 | 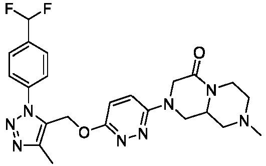 | 177 | 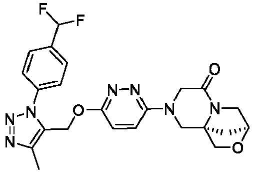 |
| 70 | 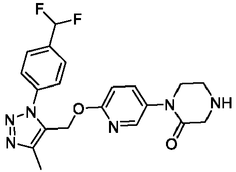 | 178 | 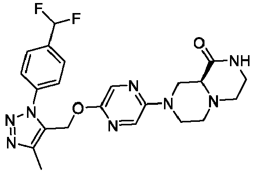 |
| 71 | 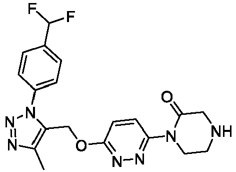 | 179 | 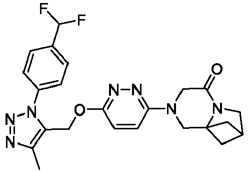 |
| 72 | 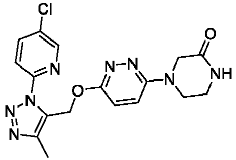 | 180 | 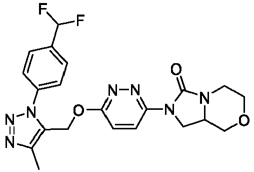 |
| 73 | 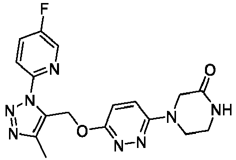 | 181 | 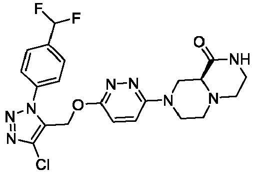 |
| 74 | 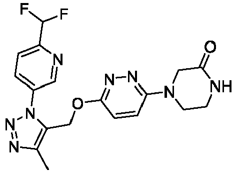 | 182 | 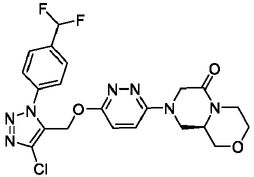 |
| 75 | 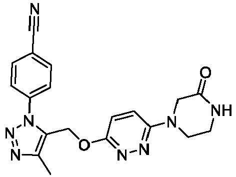 | 183 | 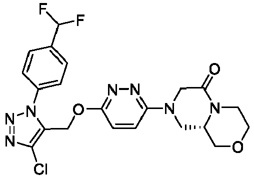 |
| 76 | 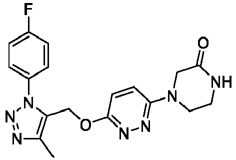 | 184 | 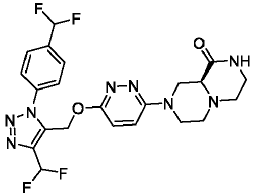 |
| 77 | 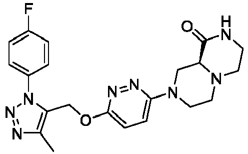 | 185 | 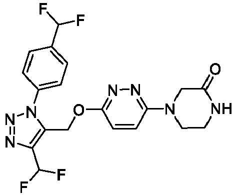 |
| 78 | 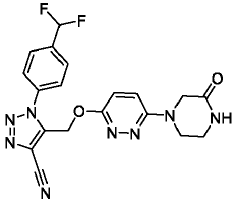 | 186 | 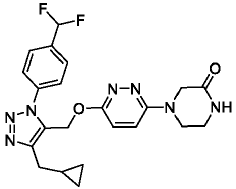 |
| 79 | 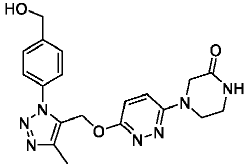 | 187 | 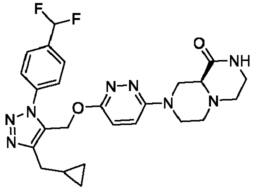 |
| 80 | 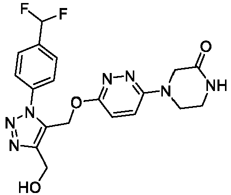 | 188 | 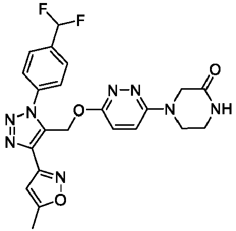 |
| 81 | 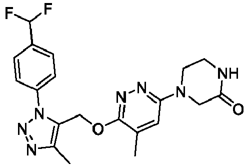 | 189 | 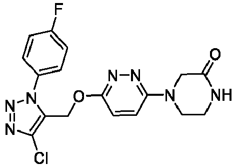 |
| 82 | 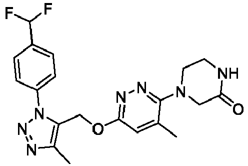 | 190 | 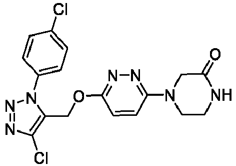 |
| 83 | 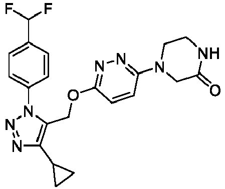 | 191 | 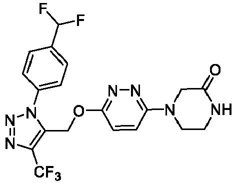 |
| 84 | 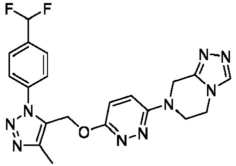 | 192 | 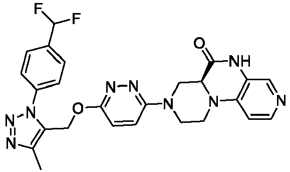 |
| 85 | 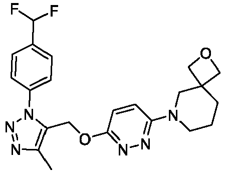 | 193 | 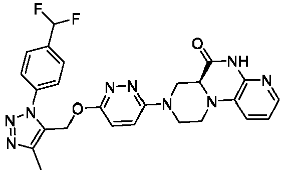 |
| 86 | 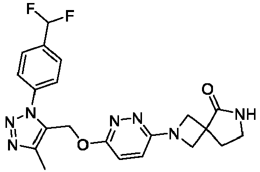 | 194 | 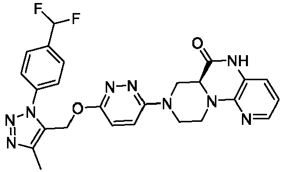 |
| 87 | 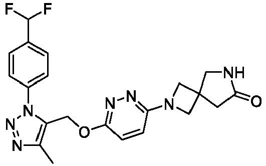 | 195 | 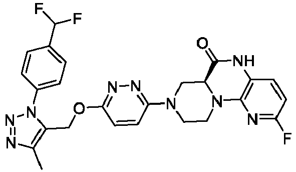 |
| 88 | 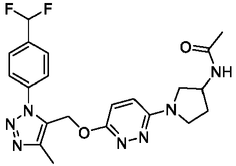 | 196 | 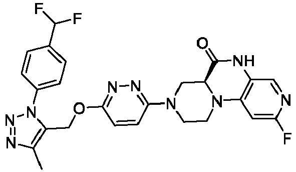 |
| 89 | 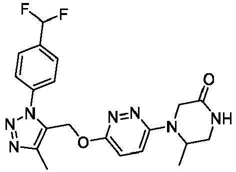 | 197 | 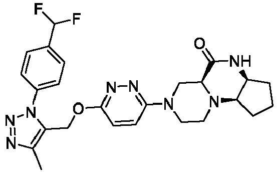 |
| 90 | 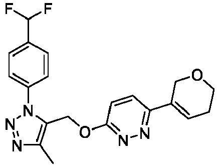 | 198 | 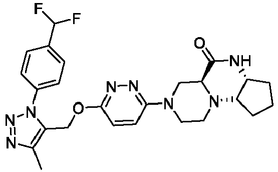 |
| 91 | 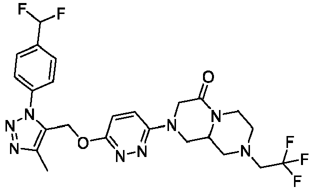 | 199 | 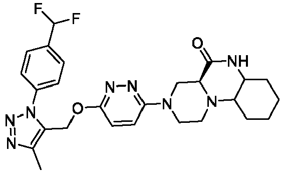 |
| 92 | 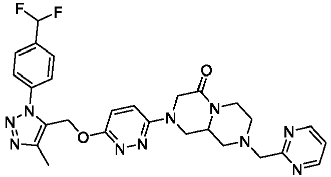 | 200 | 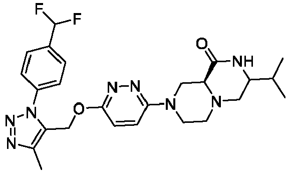 |
| 93 | 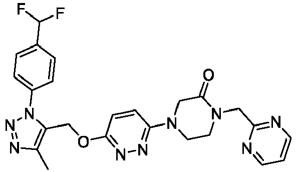 | 201 | 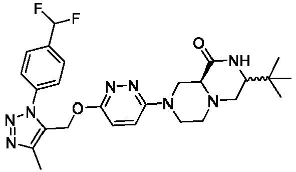 |
| 94 | 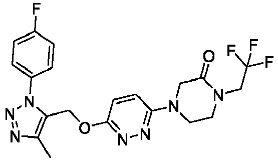 | 202 | 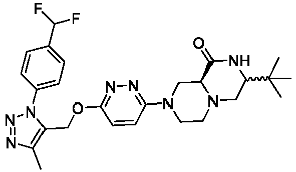 |
| 95 | 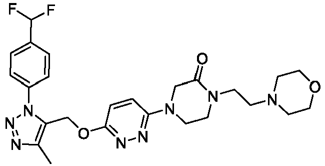 | 203 | 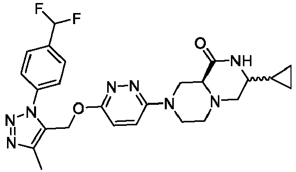 |
| 96 | 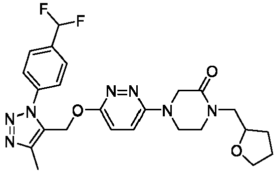 | 204 | 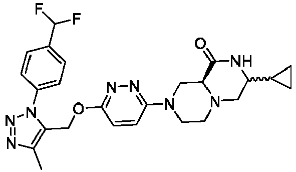 |
| 97 | 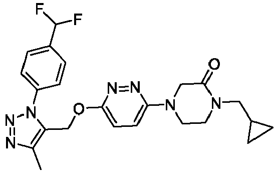 | 205 | 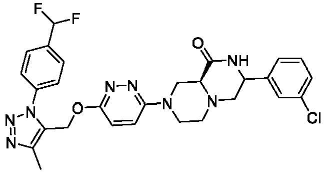 |
| 98 | 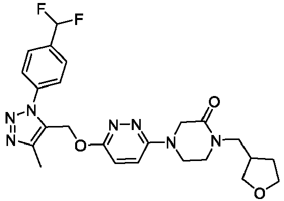 | 206 | 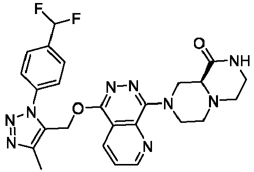 |
| 99 | 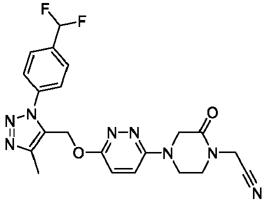 | 207 | 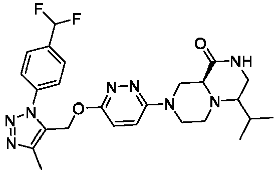 |
| 100 | 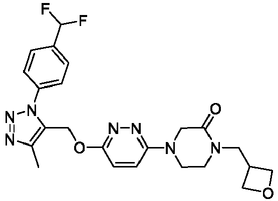 | 208 | 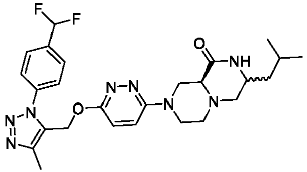 |
| 101 | 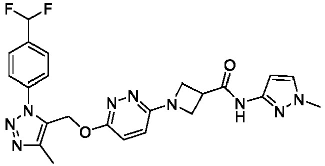 | 209 | 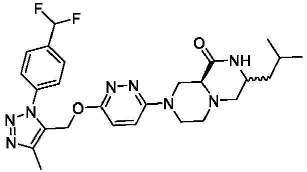 |
| 102 | 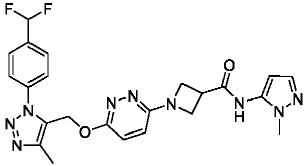 | 210 | 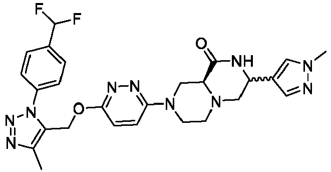 |
| 103 | 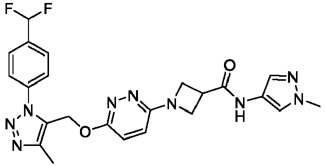 | 211 | 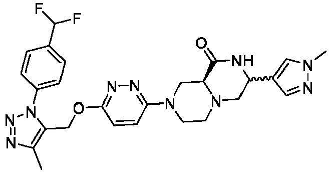 |
| 104 | 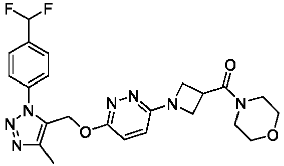 | 212 | 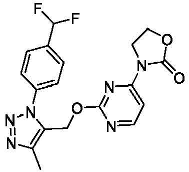 |
| 105 | 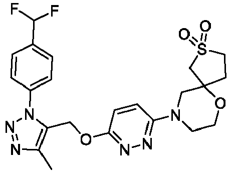 | 213 | 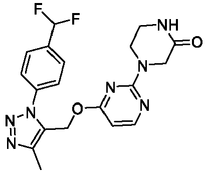 |
| 106 | 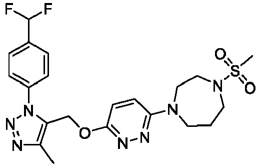 | 214 | 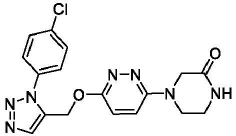 |
| 107 | 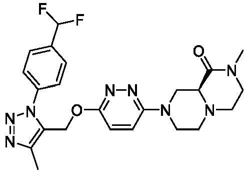 | 215 | 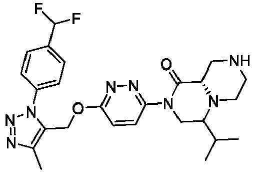 |
| 108 | 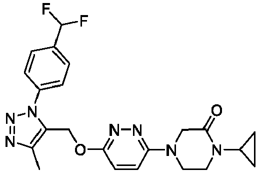 | | |

.

The present disclosure also provides a pharmaceutical composition, comprising the compound of formula I, the *cis-trans* isomer thereof, the enantiomer thereof, the diastereomer thereof, the racemate thereof, the solvate thereof, the hydrate thereof, the pharmaceutically acceptable salt thereof, or the prodrug thereof as described in any one of the above embodiments.

In some embodiments of the present disclosure, the pharmaceutical composition further comprises one or more than one pharmaceutically acceptable carrier, diluent, or excipient.

In another aspect of the present disclosure, the present disclosure also provides a use of the compound of formula I, the *cis-trans* isomer thereof, the enantiomer thereof, the diastereomer thereof, the racemate thereof, the solvate thereof, the hydrate thereof, the pharmaceutically acceptable salt thereof, or the prodrug thereof as described in any one of the above embodiments, or the pharmaceutical composition as previously described in the manufacture of an α5-GABA_{A} receptor modulator.

In yet another aspect of the present disclosure, the present disclosure also provides a use of the compound of formula I, the *cis-trans* isomer thereof, the enantiomer thereof, the diastereomer thereof, the racemate thereof, the solvate thereof, the hydrate thereof, the pharmaceutically acceptable salt thereof, or the prodrug thereof as described in any one of the above embodiments, or the pharmaceutical composition as previously described in the manufacture of a medicament for treating or preventing a disease related to an α5-GABA_{A} receptor.

The present disclosure also provides a method for treating or preventing a disease related to an α5-GABA_{A} receptor, comprising administering to a patient an effective dose of the compound of formula I, the *cis-trans* isomer thereof, the enantiomer thereof, the diastereomer thereof, the racemate thereof, the solvate thereof, the hydrate thereof, the pharmaceutically acceptable salt thereof, or the prodrug thereof, or the pharmaceutical composition as described in any one of the above embodiments.

The present disclosure also provides a use of the compound of formula I, the *cis-trans* isomer thereof, the enantiomer thereof, the diastereomer thereof, the racemate thereof, the solvate thereof, the hydrate thereof, the pharmaceutically acceptable salt thereof, or the prodrug thereof, or the pharmaceutical composition as described in any one of the above embodiments in the manufacture of a medicament for treating or preventing the following diseases: pain, Alzheimer's disease, multi-infarct dementia, and stroke.

The present disclosure also provides a method for treating or preventing pain, Alzheimer's disease, multi-infarct dementia, and stroke, characterized by administering to a patient an effective dose of the compound of formula I, the *cis-trans* isomer thereof, the enantiomer thereof, the diastereomer thereof, the racemate thereof, the solvate thereof, the hydrate thereof, the pharmaceutically acceptable salt thereof, or the prodrug thereof, or the pharmaceutical composition as described in any one of the above embodiments.

In a preferred embodiment, the pain is neuropathic pain, inflammatory pain, and cancer pain.

In a preferred embodiment, the pain is selected from: headache, facial pain, neck pain, shoulder pain, back pain, chest pain, abdominal pain, back pain, lower back pain, lower limb pain, musculoskeletal pain, vascular pain, gout, arthritis pain, visceral pain, pain caused by infectious diseases (*e.g*., AIDS and postherpetic neuralgia), polyostotic pain, sickle cell anemia associated pain, autoimmune disease associated pain, multiple sclerosis associated pain, or inflammation associated pain, chronic pain caused by injury or surgery, nociceptive pain, diabetic peripheral neuropathy pain, trigeminal neuralgia, lumbar or cervical radiculopathy, glossopharyngeal neuralgia, autonomic nerve reflex pain, reflex sympathetic dystrophy associated pain, nerve root avulsion associated pain, cancer associated pain, chemical injury associated pain, toxin associated pain, nutritional deficiency associated pain, or degenerative osteoarthropathy associated pain.

### Definition

Unless otherwise specified, the following terms and phrases when used herein have the following definitions. A specific term or phrase should not be considered indefinite or unclear in the absence of a particular definition, but should be understood in the ordinary sense. When a trading name appears herein, it is intended to refer to its corresponding commodity or active ingredient.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, an allergic reaction or other problems or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by bringing the neutral form of the compound into contact with a sufficient amount of base in a pure solution or a suitable inert solvent. The pharmaceutically acceptable base addition salt includes a salt of sodium, potassium, calcium, ammonium, organic amine, magnesium, or similar salts. When the compound of the present disclosure contains a relatively basic functional group, a base addition salt can be obtained by bringing the neutral form of the compound into contact with a sufficient amount of acid in a pure solution or a suitable inert solvent. Examples of the pharmaceutically acceptable acid addition salt include an inorganic acid salt, wherein the inorganic acid includes, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid; and an organic acid salt, wherein the organic acid includes, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid; and salts of amino acid (such as arginine), and a salt of an organic acid such as glucuronic acid. Certain specific compounds of the present disclosure contain both basic and acidic functional groups, thus can be converted to any base or acid addition salt.

The pharmaceutically acceptable salt of the present disclosure can be prepared from the parent compound that contains an acidic or basic moiety by conventional chemical method. Generally, such salt can be prepared by reacting the free acid or base form of the compound with a stoichiometric amount of an appropriate base or acid in water or an organic solvent or a mixture thereof.

The compounds of the present disclosure may exist in specific geometric or stereoisomeric forms. The present disclosure contemplates all such compounds, including *cis* and *trans* isomers, (-)- and (+)-enantiomers, (*R*)- and (*S*)-enantiomers, diastereoisomers, (*D*)-isomers, (*L*)-isomers, racemic mixtures, and other mixtures thereof, such as enantiomer or diastereomer enriched mixtures, all of which are within the scope of the present disclosure. Additional asymmetric carbon atoms may be present in substituents such as alkyl. All these isomers and their mixtures are included within the scope of the present disclosure.

Unless otherwise specified, the term "enantiomer" or "optical isomer" refers to stereoisomers that are mirror images of each other.

Unless otherwise specified, the term *"cis-trans* isomer" or "geometric isomer" is caused by the inability to rotate freely of double bonds or single bonds of ring-forming carbon atoms.

Unless otherwise specified, the term "diastereomer" refers to a stereoisomer in which a molecule has two or more chiral centers and the relationship between the molecules is not mirror images.

Unless otherwise specified, "(*D*)" or "(+)" refers to dextrorotation, "(*L*)" or "(-)" refers to levorotation, and "(*DL*)" or "(±)" refers to racemic.

Unless otherwise specified, the absolute configuration of a stereogenic center is represented by a wedged solid bond ( 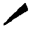 ) and a wedged dashed bond ( 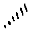 ), and the relative configuration of a stereogenic center is represented by a straight solid bond ( 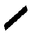 ) and a straight dashed bond ( 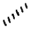 ), a wave line ( 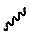 ) is used to represent a wedged solid bond ( 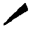 ) or a wedged dashed bond ( 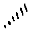 ), or the wave line ( 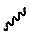 ) is used to represent a straight solid bond ( 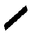 ) and a straight dashed bond ( 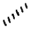 ).

The compounds of the present disclosure may exist in specific forms. Unless otherwise specified, the term "tautomer" or "tautomeric form" means that at room temperature, the isomers of different functional groups are in dynamic equilibrium and can be transformed into each other quickly. If tautomers possibly exist (such as in solution), the chemical equilibrium of tautomers may be reached. For example, proton tautomer (also called prototropic tautomer) includes interconversion through proton migration, such as keto-enol isomerization and imine-enamine isomerization. Valence tautomer includes some recombination of bonding electrons for mutual transformation. A specific example of keto-enol tautomerization is the tautomerism between two tautomers of pentane-2,4-dione and 4-hydroxy-3-en-2-one.

The term "prodrug" generally refers to functional group derivatization of a compound of general formula (I), and its derivative can be easily converted into the compound of general formula (I) *in vivo.* Selection and preparation of suitable prodrugs can be found, for example, as described in Design of Prodrug, ed. H. Bundgaard, Elsevier, 1985.

The compound of the present disclosure may contain an unnatural proportion of atomic isotopes on one or more than one atom constituting the compound, the isotopes have the same atomic number, but their atomic mass or mass number is different from those that predominantly exist in nature. For example, the compound can be labeled with a radioactive isotope, such as deuterium (²H), tritium (³H), iodine-125 (¹²⁵I), or C-14 (¹⁴C). All isotopic variations of the compound of the present disclosure, whether radioactive or not, are encompassed within the scope of the present disclosure. Isotopic variants may enhance certain therapeutic advantages, for example, deuterated drugs can be formed by replacing hydrogen with deuterium, the bond formed by deuterium and carbon is stronger than that of ordinary hydrogen and carbon, compared with non-deuterated drugs, deuterated drugs have the advantages of reduced toxic and side effects, increased drug stability, enhanced efficacy, and extended biological half-life of drugs, or they can provide a standardized compound that can be used for characterization of a biological sample. Isotope-enriched compounds within the general formula (I) can be prepared by conventional techniques well known to those skilled in the art, or by methods similar to those described in the routes and examples of the present disclosure, using appropriate isotope-enriched reagents and/or intermediates without redundant experimentation.

"Optional" or "optionally" means that the subsequently described event or circumstance may, but does not necessarily, occur, and the description includes instances where the event or circumstance occurs and instances where it does not.

The nomenclature used in the present disclosure is based on the IUPAC systematic nomenclature generated by ChemDraw software. The presence of any open valence bond on a carbon, oxygen, sulfur, or nitrogen atom in the structures provided in the present disclosure indicates the presence of a hydrogen atom.

The term "substituted" means one or more than one hydrogen atom on a specific atom is substituted by the substituent, including deuterium and hydrogen variables, as long as the valence of the specific atom is normal and the substituted compound is stable. When the substituent is oxo (*i.e.,* =O), it means that two hydrogen atoms are substituted and oxo substitution does not occur on aryl. The term "optionally substituted" means an atom can be substituted by a substituent or not, unless otherwise specified, the type and number of the substituent may be arbitrary as long as being chemically achievable.

When any variable (such as R) occurs in the constitution or structure of the compound more than once, the definition of the variable at each occurrence is independent. Thus, for example, if a group is substituted by 0 to 2 R, the group can be optionally substituted by up to two R, wherein the definition of R at each occurrence is independent. Moreover, a combination of the substituent and/or the variant thereof is allowed only when the combination results in a stable compound.

When the number of a linking group is 0, such as -(CRR)₀-, it means that the linking group is a single bond.

When one of the variables is selected from a single bond, it means that the two groups linked by the single bond are connected directly. For example, when L in A-L-Z represents a single bond, the structure of A-L-Z is actually A-Z.

When the enumerative linking group does not indicate the direction for linking, the direction for linking is arbitrary, for example, the linking group L contained in 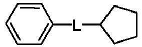 is 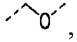 then 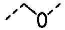 can link a benzene ring and cyclopentyl to form 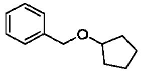 in the direction same as left-to-right reading order, and form 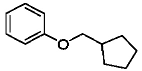 in the direction contrary to left-to-right reading order. A combination of the linking groups, substituents and/or variables thereof is allowed only when such combination can result in a stable compound.

Unless otherwise specified, the number of atoms in a ring is usually defined as the number of ring members, for example, "3- to 7-membered ring" refers to a "ring" in which 3 to 7 atoms are arranged around.

Unless otherwise specified, the term "halogen" refers to fluorine, chlorine, bromine, and iodine.

Unless otherwise specified, the term "C₁₋₆ alkyl" refers to a linear or branched saturated hydrocarbon group consisting of 1 to 6 carbon atoms. The C₁₋₆ alkyl includes Ci-s, C₁₋₄, C₁₋₃, C₁₋₂, C₂₋₆, C₂₋₄, C₆, C₅ alkyl, *etc.;* it can be monovalent (such as methyl), divalent (such as methylene), or multivalent (such as methine). Examples of C₁₋₆ alkyl include, but are not limited to, methyl (Me), ethyl (Et), propyl (including *n*-propyl and isopropyl), butyl (including *n*-butyl, isobutyl, *s*-butyl, and *t*-butyl), pentyl (including *n*-pentyl, isopentyl, and neopentyl), hexyl, *etc.*

Unless otherwise specified, the term "C₁₋₃ alkyl" refers to a linear or branched saturated hydrocarbon group consisting of 1 to 3 carbon atoms. The C₁₋₃ alkyl includes C₁₋₂, C₂₋₃ alkyl, *etc.;* it can be monovalent (such as methyl), divalent (such as methylene), or multivalent (such as methine). Examples of C₁₋₃ alkyl include, but are not limited to, methyl (Me), ethyl (Et), propyl (including *n*-propyl and isopropyl), *etc.*

Unless otherwise specified, the term "C₁₋₆ alkoxy" refers to an alkyl group containing 1 to 6 carbon atoms that are connected to the rest of the molecule through an oxygen atom. The C₁₋₆ alkyl includes C₁₋₄, C₁₋₃, C₁₋₂, C₂₋₆, C₂₋₄, C₆, C₅, C₄, C₃ alkoxy, *etc.;* examples of C₁₋₆ alkoxy include, but are not limited to, methoxy, ethoxy, propoxy (including n-propoxy and isopropoxy), butoxy (including *n*-butoxy, isobutoxy, *s*-butoxy, and *t*-butoxy), pentyloxy (including *n*-pentyloxy, isopentyloxy, and neopentyloxy), hexyloxy, *etc.*

Unless otherwise specified, the term "C₁₋₃ alkoxy" refers to an alkyl group containing 1 to 3 carbon atoms that are connected to the rest of the molecule through an oxygen atom. The C₁₋₆ alkyl includes C₁₋₂, C₂₋₃ alkoxy, *etc.;* examples of C₁₋₃ alkoxy include, but are not limited to, methoxy, ethoxy, propoxy (including *n*-propoxy and isopropoxy), *etc.*

Unless otherwise specified, the term "C₁₋₆ alkylamino" refers to an alkyl group containing 1 to 6 carbon atoms that are connected to the rest of the molecule through an amino group. The C₁₋₆ alkyl includes C₁₋₄, C₁₋₃, C₁₋₂, C₂₋₆, C₂₋₄, C₆, C₅, C₄, C₃, C₂ alkylamino, *etc.;* examples of C₁₋₆ alkylamino include, but are not limited to, -NHCH₃, -N(CH₃)₂, -NHCH₂CH₃, -N(CH₃)CH₂CH₃, -N(CH₂CH₃)₂, -NHCH₂CH₂CH₃, -NHCH(CH₃)₂, -NHCH₂CH₂CH₂CH₃, *etc.*

Unless otherwise specified, the term "C₃₋₆ cycloalkyl" refers to a saturated cyclic hydrocarbon group consisting of 3 to 6 carbon atoms, which is a monocyclic and bicyclic system, and the C₃₋₆ cycloalkyl includes C₃₋₅, C₄₋₅, C₅₋₆ cycloalkyl, *etc.*; it can be monovalent, divalent, or multivalent. Examples of C₃₋₆ cycloalkyl include, but are not limited to, cyclopropyl, cyclobutyl, cyclopentyl, cyclohexyl, *etc.*

Unless otherwise specified, the term "3- to 6-membered heterocycloalkyl" by itself or in combination with other terms refers to a saturated monocyclic group consisting of 3 to 6 ring atoms, wherein 1, 2, 3, or 4 ring atoms are heteroatoms independently selected from O, S, and N, and the rest are carbon atoms, wherein nitrogen atoms are optionally quaternized, and nitrogen and sulfur heteroatoms can be optionally oxidized (*i.e.,* NO and S(O)_{z}, z is 1 or 2). In addition, with regard to the "3- to 6-membered heterocycloalkyl", a heteroatom may occupy the connection position of the heterocycloalkyl with the rest of the molecule. The 3- to 6-membered heterocycloalkyl includes 4- to 6-membered, 5- to 6-membered, 4-membered, 5-membered, 6-membered heterocycloalkyl, *etc.*; examples of 3- to 6-membered heterocycloalkyl include, but are not limited to, azetidinyl, oxetanyl, thietanyl, pyrrolidinyl, pyrazolidinyl, imidazolidinyl, tetrahydrothiophenyl, tetrahydrofuranyl (including tetrahydrofuran-2-yl), piperidinyl, tetrahydropyranyl, tetrahydrothiopyranyl, piperazinyl, morpholinyl, thiomorpholinyl, *etc.*

Unless otherwise specified, the term "4- to 14-membered heterocycloalkyl" by itself or in combination with other terms refers to a saturated or semi-saturated cyclic group consisting of 4 to 14 ring atoms, wherein 1, 2, 3, 4, 5, or 6 ring atoms are heteroatoms independently selected from O, S, and N, and the rest are carbon atoms, wherein nitrogen atoms are optionally quaternized, and nitrogen and sulfur heteroatoms can be optionally oxidized (i.e., NO and S(O)_{z}, z is 1 or 2). It includes monocyclic, bicyclic, and tricyclic systems, wherein the bicyclic or tricyclic system can be a fused ring system, a spiro ring system, and a bridged ring system; in addition, with regard to the "4- to 14-membered heterocycloalkyl", a heteroatom may occupy the connection position of the heterocycloalkyl with the rest of the molecule. Some of the ring systems in the bicyclic or tricyclic system can be aromatic, and the ring connected to the rest of the molecule is not aromatic. Examples of 4- to 14-membered heterocycloalkyl include, but are not limited to, cyclopropyl, azetidinyl, oxetanyl, thietanyl, pyrrolidinyl, pyrazolidinyl, imidazolidinyl, tetrahydrothiophenyl, tetrahydrofuranyl, piperidinyl, tetrahydropyranyl, tetrahydrothiopyranyl, piperazinyl, morpholinyl, thiomorpholinyl, homopiperazinyl, 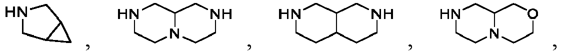 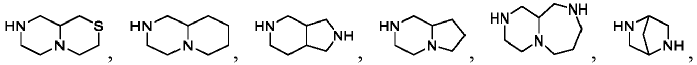 *etc.*

Unless otherwise specified, the terms "6- to 10-membered aromatic ring" and "6- to 10-membered aryl" can be used interchangeably, and the term "6- to 10-membered aryl" refers to a monovalent aromatic carbocyclic ring system containing 6 to 10 carbon atoms and having at least one aromatic ring or multiple fused rings in which at least one ring is aromatic. Examples of aryl include, but are not limited to, phenyl, naphthyl, biphenyl, or indanyl.

Unless otherwise specified, the terms "5- to 10-membered heteroaromatic ring" and "5- to 10-membered heteroaryl" in the present disclosure can be used interchangeably, and the term "5- to 10-membered heteroaryl" refers to a cyclic group consisting of 5 to 10 ring atoms with a conjugated π-electron system, wherein 1, 2, 3, or 4 ring atoms are heteroatoms independently selected from O, S, and N, and the rest are carbon atoms. It can be a monocyclic and fused bicyclic system, wherein each ring is aromatic. Herein, nitrogen atoms are optionally quaternized, and nitrogen and sulfur heteroatoms can be optionally oxidized (*i.e.*, NO and S(O)_{z}, z is 1 or 2). 5- to 10-membered heteroaryl can be linked to the rest of the molecule through a heteroatom or a carbon atom, and the 5- to 10-membered heteroaryl includes 5- to 8-membered, 5- to 7-membered, 5- to 6-membered, 5-membered, 6-membered heteroaryl, *etc.* Examples of the 5- to 10-membered heteroaryl include, but are not limited to, pyrrolyl (including *N*-pyrrolyl, 2-pyrrolyl, 3-pyrrolyl, *etc.*), pyrazolyl (including 2-pyrazolyl, 3-pyrrolyl, *etc.*), imidazolyl (including *N*-imidazolyl, 2-imidazolyl, 4-imidazolyl, 5-imidazolyl, *etc.*)*,* oxazolyl (including 2-oxazolyl, 4-oxazolyl, 5-oxazolyl, *etc*.), triazolyl (1*H*-1,2,3-triazolyl, 2*H*-1,2,3-triazolyl, 1*H*-1,2,4-triazolyl, and 4*H-*1,2,4-triazolyl), tetrazolyl, isoxazolyl (including 3-isoxazolyl, 4-isoxazolyl, 5-isoxazolyl, *etc*.), thiazolyl (including 2-thiazolyl, 4-thiazolyl, 5-thiazolyl, *etc.*)*,* furyl (including 2-furyl, 3-furyl, *etc.*), thienyl (including 2-thienyl, 3-thienyl, *etc.*), pyridyl (including 2-pyridyl, 3-pyridyl, 4-pyridyl, *etc*.), pyrazinyl, pyrimidinyl (including 2-pyrimidinyl, 4-pyrimidinyl, *etc*.), benzothiazolyl (including 2-benzothiazolyl, *etc.*), purinyl, benzimidazolyl (including 2-benzimidazolyl, *etc*.), benzoxazolyl, indazolyl (including 5-indazolyl, *etc*.), isoquinolyl (including 1-isoquinolyl, 5-isoquinolinyl, *etc.*), quinoxalinyl (including 2-quinoxalinyl, 5-quinoxalinyl, *etc*.), quinolinyl (including 3-quinolinyl, 6-quinolinyl, *etc.*), *etc.*

Unless otherwise specified, the terms "5- to 6-membered heteroaromatic ring" and "5-to 6- membered heteroaryl" in the present disclosure can be used interchangeably, and the term "5- to 6- membered heteroaryl" refers to a cyclic group consisting of 5 to 6 ring atoms with a conjugated *π*-electron system, wherein 1, 2, 3, or 4 ring atoms are heteroatoms independently selected from O, S, and N, and the rest are carbon atoms. Herein, nitrogen atoms are optionally quaternized, and nitrogen and sulfur heteroatoms can be optionally oxidized (*i.e*., NO and S(O)_{z}, z is 1 or 2). 5- to 6-membered heteroaryl can be linked to the rest of the molecule through a heteroatom or a carbon atom, and the 5- to 6-membered heteroaryl includes 5-membered, 6-membered heteroaryl, *etc.* Examples of the 5- to 6-membered heteroaryl include, but are not limited to, pyrrolyl (including *N*-pyrrolyl, 2-pyrrolyl, 3-pyrrolyl, *etc.*)*,* pyrazolyl (including 2-pyrazolyl, 3-pyrazolyl, *etc*.), imidazolyl (including *N*-imidazolyl, 2-imidazolyl, 4-imidazolyl, 5-imidazolyl, *etc.*), oxazolyl (including 2-oxazolyl, 4-oxazolyl, 5-oxazolyl, *etc.*), triazolyl (1*H*-1,2,3-triazolyl, 2*H*-1,2,3-triazolyl, 1*H*-1,2,4-triazolyl, 4*H*-1,2,4-triazolyl), tetrazolyl, isoxazolyl (including 3-isoxazolyl, 4-isoxazolyl, 5-isoxazolyl, *etc.*), thiazolyl (including 2-thiazolyl, 4-thiazolyl, 5-thiazolyl, *etc.*), furyl (including 2-furyl, 3-furyl, *etc.*), thienyl (including 2-thienyl, 3-thienyl, *etc.*), pyridyl (including 2-pyridyl, 3-pyridyl, 4-pyridyl, *etc.*), pyrazinyl, pyrimidinyl (including 2-pyrimidinyl, 4-pyrimidinyl, *etc.*), *etc.*

Unless otherwise specified, Cₙ₋ₙ₊ₘ or Cₙ-Cₙ₊ₘ includes any specific case of n to n+m carbons, for example, C₁₋₇ includes C₁, C₂, C₃, C₄, C₅, C₆, and C₇, and any range from n to n+m is also included, for example, C₁₋₇ includes C₁₋₃, C₁₋₆, C₃₋₆, C₄₋₇, C₅₋₇, *etc.*; similarly, n-membered to n+m-membered means that the number of atoms on the ring is from n to n+m, for example, 3- to 7-membered ring includes 3-membered ring, 4-membered ring, 5-membered ring, 6-membered ring, and 7-membered ring, and any range from n to n+m is also included, for example, 3- to 7-membered ring includes 3- to 6-membered ring, 4- to 7-membered ring, 5- to 7-membered ring, 6- to 7-membered ring, *etc.*

The term "leaving group" refers to a functional group or atom which can be replaced by another functional group or atom through a substitution reaction (such as nucleophilic substitution reaction). For example, representative leaving groups include triflate; chlorine, bromine, and iodine; sulfonate group, such as mesylate, tosylate, *p*-bromobenzenesulfonate, *p-*toluenesulfonate; acyloxy, such as acetoxy, trifluoroacetoxy.

The term "protecting group" includes, but is not limited to, "amino protecting group", "hydroxyl protecting group", or "mercapto protecting group". The term "amino protecting group" refers to a protecting group suitable for preventing the side reactions occurring at the nitrogen of an amino. Representative amino protecting groups include, but are not limited to: formyl; acyl, such as alkanoyl (*e.g*., acetyl, trichloroacetyl, or trifluoroacetyl); alkoxycarbonyl, such as *tert*-butoxycarbonyl (Boc); arylmethoxycarbonyl such as benzyloxycarbonyl (Cbz) and 9-fluorenylmethoxycarbonyl (Fmoc); arylmethyl, such as benzyl (Bn), trityl (Tr), 1,1-bis-(4'-methoxyphenyl)methyl; silyl, such as trimethylsilyl (TMS) and *tert*-butyldimethylsilyl (TBS). The term "hydroxyl protecting group" refers to a protecting group suitable for blocking the side reaction on hydroxyl. Representative hydroxyl protecting groups include, but are not limited to: alkyl, such as methyl, ethyl, and *tert-*butyl; acyl, such as alkanoyl (*e.g.*, acetyl); arylmethyl, such as benzyl (Bn), *p*-methoxybenzyl (PMB), 9-fluorenylmethyl (Fm), and diphenylmethyl (benzhydryl, DPM); silyl, such as trimethylsilyl (TMS) and *tert*-butyl dimethyl silyl (TBS).

The term "treatment" as used in the present disclosure refers to the administration of one or more than one pharmaceutical substance, in particular the compound of formula (I) and/or the pharmaceutically acceptable salt thereof according to the present disclosure, to an individual suffering from a disease or having symptoms of the disease, for the purpose of curing, alleviating, mitigating, altering, healing, improving, ameliorating, or affecting the disease or symptoms of the disease. The term "prevention" as used in the present disclosure refers to the administration of one or more than one pharmaceutical substance, in particular the compound of formula (I) and/or the pharmaceutically acceptable salt thereof according to the present disclosure, to an individual susceptible to the disease, for the purpose of preventing the individual from developing the disease. When referring to a chemical reaction, the terms "treating", "contacting", and "reacting" refer to the addition or mixing of two or more reagents under appropriate conditions to produce the indicated and/or desired products. It should be understood that the reaction that produces the indicated and/or desired products may not necessarily result directly from the combination of the two reagents initially added, *i.e.*, there may be one or more than one intermediate generated in the mixture that ultimately leads to the formation of the indicated and/or desired products.

As used in the present disclosure, a "patient" is defined as any warm-blooded animal, for example, including, but not limited to, a mouse, guinea pig, dog, horse, or human; preferably, the patient is a human.

The term "effective dose" as used in the present disclosure refers to an amount that is generally sufficient to produce a beneficial effect on an individual. The effective dose of the compound of the present disclosure can be determined by conventional methods (*e.g.*, modeling, dose-escalation studies, or clinical trials) in conjunction with conventional influencing factors (*e.g*., mode of administration, pharmacokinetics of the compound, severity and course of the disease, medical history of the individual, health of the individual, response of the individual to drugs, *etc.*)*.*

As mentioned above, the new compound and the pharmaceutically acceptable salt thereof and the prodrug thereof of the present disclosure have important pharmacological properties and are α5-GABA_{A} receptor inverse agonists. Therefore, the compound of the present disclosure can be used alone or in combination with other medicaments for treating or preventing diseases mediated by GABA_{A} receptor ligands containing α5 subunits. These diseases include, but are not limited to, pain, Alzheimer's disease, multi-infarct dementia, and stroke.

Therefore, the present disclosure also relates to a pharmaceutical composition comprising the compound as defined above and a pharmaceutically acceptable carrier and/or adjuvant.

Similarly, the present disclosure also provides the compound as described above for use in the manufacture of a medicament for treating or preventing diseases related to the α5-GABA_{A} receptor, especially for treating or preventing the following diseases: pain, Alzheimer's disease, multi-infarct dementia, and stroke.

It is preferred to treat or prevent pain.

It is particularly preferred to treat or prevent neuropathic pain, inflammatory pain, and cancer pain.

As used in the present disclosure, "cancer pain" refers to the pain that occurs during the development process of a malignant tumor. Currently, it is thought that there are three mechanisms of cancer pain, namely, pain caused directly by the development of the cancer, pain caused by the treatment of the cancer with chemotherapeutic agents, and pain disorders complicating the cancer patient.

As used in the present disclosure, "neuropathic pain" refers to the pain triggered or caused by the primary damage and dysfunction of the nervous system.

As used in the present disclosure, "inflammatory pain" refers to the pain caused by local acute inflammation or chronic inflammation that stimulates nerves.

As used in the present disclosure, "acute pain" is defined as the pain caused by the injury of skin, body structure or internal organs and/or noxious stimulation of the disease, or the pain caused by the abnormal function of muscle or internal organs without actual tissue damage.

As used in the present disclosure, "chronic pain" is defined as the pain that persists beyond the usual course of an acute disease or a reasonable period of time for injury healing, or that is associated with a chronic pathological process that causes persistent pain, or that recurs at regular intervals of months or years. The pain that persists after the disease should have been cured or beyond the usual course of treatment can be regarded as chronic pain. The length of time that the pain lasts depends on the nature of pain and the course of treatment associated with pain. The pain that lasts longer than the usual course of treatment is chronic pain.

The medicaments disclosed in the present disclosure can efficiently treat the chronic pain as defined above, and the medicaments disclosed in the present disclosure can be used to treat hyperalgia accompanied with other diseases, including hyperalgesia, allodynia, enhanced algesia, and enhanced pain memory, for which the present disclosure will improve the treatment of pain.

As used in the present disclosure, "headache" can be divided into primary headache, including tension headache, migraine headache, and cluster headache, and secondary headache, which is caused by other diseases. Headache can be caused when pain-sensitive tissues of the head and face are diseased or stimulated. These pain-sensitive tissues are distributed in the scalp, face, mouth, and throat. Since they are mainly muscles or blood vessels in head with abundant nerve fibers and sensitive to pain, headache can be caused when these tissues are injured.

As used in the present disclosure, "facial pain" includes, but is not limited to, trigeminal neuralgia, atypical facial pain, facial palsy, and facial spasm.

As used in the present disclosure, "trigeminal neuralgia" is a unique chronic painful disease, also known as tic douloureux, which refers to transient, paroxysmal, and recurring electric shock-like severe pain in the distribution area of the trigeminal nerve, or accompanied with ipsilateral facial spasm. Trigeminal neuralgia is divided into primary trigeminal neuralgia, which means that no neurological sign is found clinically and no organic disease is detected; and secondary trigeminal neuralgia, which means that neurological signs are found clinically and organic diseases such as tumor and inflammation are detected.

As used in the present disclosure, "atypical facial pain" refers to the pain caused by various etiologies, appearing as persistent burning pain that is non-intermittent and independent of particular action or stimulation. The pain is mostly bilateral and often extends beyond the distribution range of the trigeminal nerve to even cervical skin. The etiology can be the stimulation of nasosinusitis, malignant tumor, jaw and skull base infection, or pain caused by injured trigeminal nerve.

As used in the present disclosure, "neck pain, back pain, shoulder pain" refers to the pain caused by acute or chronic muscle strain and bone joint degeneration and injury, *etc.* The common diseases that cause neck, shoulder, and upper limb pain include cervicoshoulder myofascitis, nuchal ligamentitis, cervical spondylosis, scapulohumeral periarthritis, thoracic outlet syndrome, lateral epicondylitis, *etc.* Alternatively, the pain caused by autoimmune diseases is common in rheumatoid arthritis, ankylosing spondylitis, rheumatic arthritis, *etc.* Other diseases that may cause neck pain, back pain, and shoulder pain include neck and shoulder tumors, neuritis, arteriovenous disease, and various infections as well as referred pain caused by chest and abdominal organ lesions.

As used in the present disclosure, "chest, abdominal, and back pain" refers to the pain caused by diseases of chest and abdominal viscera and chest and abdominal wall tissues.

As used in the present disclosure, "lower back and lower limb pain" refers to lower back, lumbosacral, sacroiliac, hip, buttock, and lower limb pain.

As used in the present disclosure, "musculoskeletal pain" includes, but is not limited to, myofascial pain, trauma-induced pain, and chronic regional pain syndrome.

As used in the present disclosure, "diabetic peripheral neuropathy pain" refers to the pain caused by nerve injury complicated by diabetes, and the nerve injury in diabetes is at least partially caused by reduced blood flow and hyperglycemia.

As used in the present disclosure, "visceral pain" includes, but is not limited to, the pain of irritable bowel syndrome (IBS), with or without chronic fatigue syndrome (CFS), inflammatory bowel disease (IBD), and interstitial cystitis.

As used in the present disclosure, "vascular pain" refers to the pain resulting from one or more than one of the following factors. Firstly, improper perfusion of tissue, resulting in temporary or continuous ischemia, such as the ischemia in limb muscles during exercise; secondly, delayed change, such as ulcer or gangrene in skin or abdominal viscera; thirdly, sudden or accelerated change in macrovascular caliber, such as the change in aneurysm, fourthly, aortic rupture, resulting in overflow of blood and stimulation of nociceptive fibers in peritoneal or pleura parietal layers; fifthly, strong spasm caused by severe stimulation of artery endothelium by intra-arterial injection; sixthly, impairment of venous return, leading to massive edema of rapidly expanded fascial compartment (Bonica et al., The Management of Pain, Volume 1 (2nd edition), Philadelphia; Leas & Feboger, 1990).

As used in the present disclosure, "autonomic nerve reflex pain" refers to the pain caused by "reflex sympathetic dystrophy syndrome". Reflex sympathetic dystrophy syndrome refers to a condition in which the body suffers an acute or chronic injury with severe spontaneous pain and hypersensitivity to touch and pain.

As used in the present disclosure, "postoperative pain" refers to a complex physiological response of the body to the disease itself and the tissue injury caused by surgery, which is manifested as an unpleasant psychological and behavioral experience.

As used in the present disclosure, "arthritis pain" includes, but is not limited to, pain resulting from osteoarthritis, rheumatoid arthritis, ankylosing spondylitis, psoriatic arthritis, gout, pseudogout, infectious arthritis, tendinitis, bursitis, bone damage, joint soft tissue inflammation, *etc.*

As used in the present disclosure, "postherpetic neuralgia" refers to severe pain that persists in the subcutaneous area of the original rash area after the shingles rash has healed.

As used in the present disclosure, "nociceptive pain" refers to the pain caused by stimulation of afferent tissue damaging process by nociceptors, or the pain caused by prolonged excitation of nociceptors.

The technical and scientific terms not specifically defined used in the present disclosure have the meanings commonly understood by those skilled in the art to which the present disclosure belongs.

The solvent used in the present disclosure is commercially available. The following abbreviations are used in the present disclosure: Ac stands for acetyl; ACN stands for acetonitrile; B₂pin₂ stands for bis(pinacolato)diboron; CbzCl stands for benzyl chloroformate; DAST stands for diethylaminosulfur trifluoride; DCDMH stands for 1,3-dichloro-5,5-dimethylhydantoin; DCM stands for dichloromethane; DEAD stands for diethyl azodicarboxylate; DIBAL-H stands for diisobutylaluminum hydride; DIEA stands for diisopropylethylamine; DMAP stands for 4-dimethylaminopyridine; DMB stands for 2,4-dimethoxybenzyl; DMF stands for *N*,*N*-dimethylformamide; DMSO stands for dimethyl sulfoxide; EDCI stands for 1-ethyl-(3-dimethylaminopropyl)carbodiimide hydrochloride; HATU stands for 2-(7-azabenzotriazol-1-yl)-*N*,*N*,*N',N*'-tetramethyluronium hexafluorophosphate; HOBt stands for 1-hydroxybenzotriazole; HPLC stands for high performance liquid chromatography; KHMDS stands for potassium bis(trimethylsilyl)amide; LAH stands for lithium aluminum hydride; *m*-CPBA stands for *m*-chloroperoxybenzoic acid; MsCl stands for methanesulfonyl chloride; M.W. stands for microwave heating; NCS stands for *N*-chlorosuccinimide; Pd(dppf)Cl₂ stands for [1,1'-bis(diphenylphosphino)ferrocene]dichloropalladium(II); Pd₂(dba)₃ stands for tris(dibenzylideneacetone)dipalladium(0); PhNTf₂ stands for *N-*phenylbis(trifluoromethanesulfonimide); PMBCl stands for *p*-methoxybenzyl chloride; pyr. stands for pyridine; Ruphos stands for 2-dicyclohexylphosphino-2',6'-di-i-propoxy-1,1'-biphenyl; Ruphos-Pd-G3 stands for methanesulfonato(2-dicyclohexylphosphino-2',6'-di-i-propoxy-1,1'-biphenyl)(2'-amino-1,1'-biphenyl-2-yl)palladium(II); SFC stands for supercritical fluid chromatography; TBAF stands for tetrabutylammonium fluoride; TBSCl stands for *tert*-butyldimethylsilyl chloride; *t*-BuXphos stands for 2-di-*tert*-butylphosphino-2',4',6'-triisopropylbiphenyl; THF stands for tetrahydrofuran; TEA stands for triethylamine; TEAF stands for triethylamine trihydrofluoride; Tf stands for trifluoromethanesulfonyl; TFA stands for trifluoroacetic acid; TFAA stands for trifluoroacetic anhydride; TLC stands for thin-layer chromatography; TMS stands for trimethylsilyl; Tol. stands for toluene; Ts stands for *p-*toluenesulfonyl; Xantphos stands for 4,5-bis(diphenylphosphino)-9,9-dimethylxanthene; LCMS stands for liquid chromatography-mass spectrometry; h stands for hour; min stands for minute.

### Preparation method

The present disclosure also relates to a method for producing the compound of general formula (I) as defined above, and the synthesis methods for the compound are shown as follows:

### Synthesis method (I):

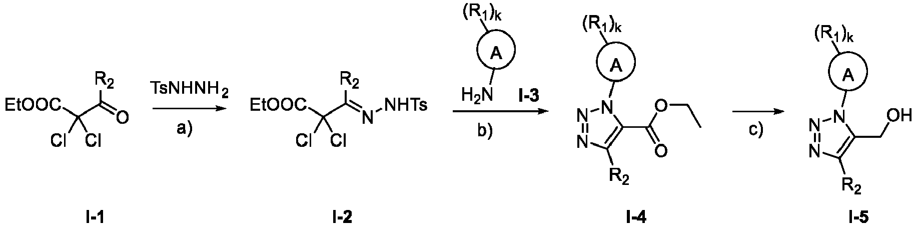

When T₁ is a nitrogen atom and T₂ is a carbon atom:
Compound **I-1** and compound **I-3** are generally provided from commercially available raw materials. In an alcohol or ether solvent, such as ethanol or tetrahydrofuran, compound **I-1** is reacted with *p*-toluenesulfonyl hydrazide to form hydrazone compound **I-2.** As a nucleophile, aromatic amine **I-3** attacks on hydrazone compound **I-2,** and then undergoes intramolecular cyclization and aromatization to form triazole compound **I-4.** Reaction step b) is generally carried out in an alcohol or ether solvent, and the alcohol or ether solvent includes, but is not limited to, ethanol, isopropanol, tetrahydrofuran, *etc.* Compound **I-5** can be obtained by a reduction reaction of compound **I-4,** and the reduction reaction includes, but is not limited to, a reaction using a reducing agent such as sodium borohydride, lithium borohydride, lithium aluminum hydride, or DIBAL-H in an ether or alcohol solvent such as tetrahydrofuran or methanol.

### Synthesis method (II):

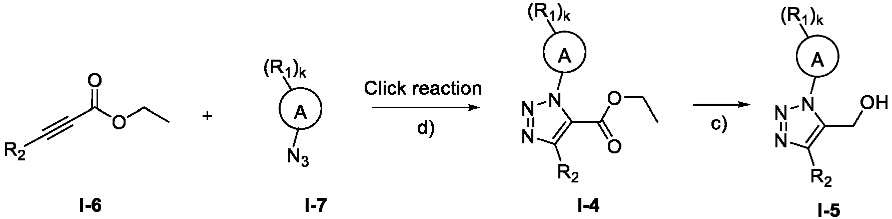

Compound **I-5** can also be synthesized through a Click reaction of alkyne compound **I-6** and azide compound **I-7,** as shown in step d). The reaction is completed under copper salt-catalyzed conditions, and the copper salt includes, but is not limited to, copper sulfate, cuprous iodide, copper acetate, *etc.* Compound **I-6** in which R₂ is an aryl structure can be synthesized through a Sonagashira coupling of the corresponding haloaryl compound and ethyl propiolate; whereas compound **I-7** can be synthesized through a reaction of the corresponding amino compound **I-3** and a nitrite salt or nitrite ester, the nitrite salt includes, but is not limited to, sodium nitrite, potassium nitrite, *etc.*, and the nitrite ester includes, but is not limited to, *tert*-butyl nitrite, isoamyl nitrite, *etc.* For step d), different regioisomers may be produced in the reaction, and compound **I-4** needs to be obtained through isomer separation.

### Synthesis method (III):

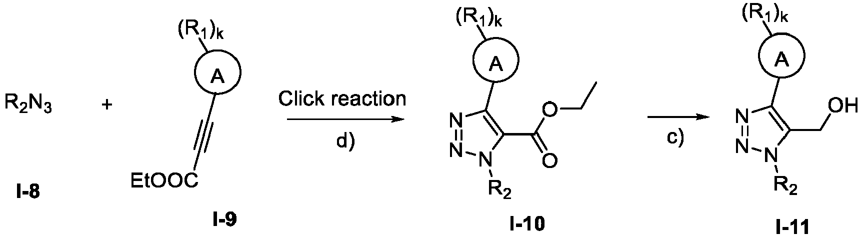

When T₁ is a carbon atom and T₂ is a nitrogen atom:
Compound **I-11** can also be synthesized through a Click reaction of alkynoate compound **I-9** with azide compound **I-8,** as shown in step d). The reaction is completed under copper salt-catalyzed conditions, and the copper salt includes, but is not limited to, copper sulfate, cuprous iodide, copper acetate, *etc.* Alkynoate compound **I-9** can be synthesized through a Sonagashira coupling of the corresponding haloaryl compound and ethyl propiolate; for step d), different regioisomers may be produced in the reaction, and compound **I-11** needs to be obtained through isomer separation.

### Synthesis method (IV):

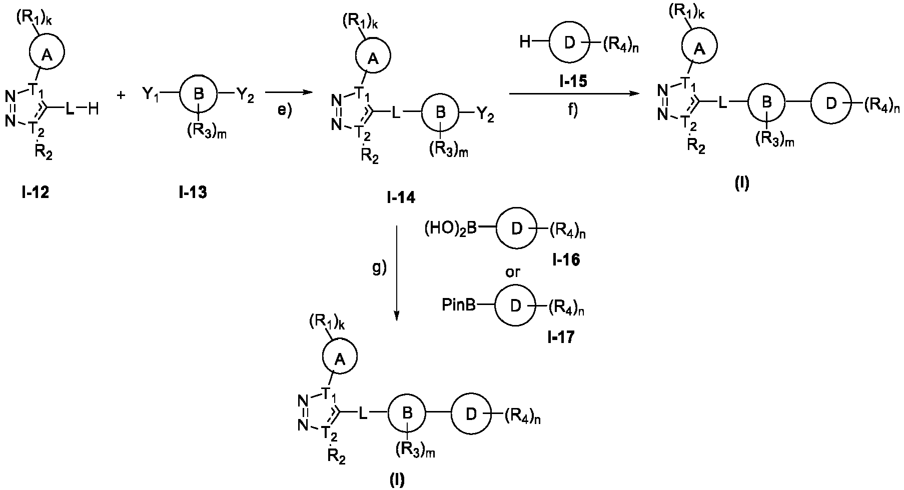

In this synthetic method, compounds **I-5** and **I-11** are represented by general formula compound **I-12,** and compound **I-14** is synthesized through a direct substitution reaction or coupling reaction of compound **I-12** and compound **I-13** in step e). The direct substitution reaction is a nucleophilic substitution reaction with alkaline conditions as the corresponding reaction conditions, for example, a reaction using sodium hydride, cesium carbonate, or potassium phosphate as a base in various solvents such as DMF, acetonitrile, or tetrahydrofuran; the coupling reaction is a metal-catalyzed coupling reaction for the formation of a carbon-oxygen or carbon-nitrogen bond, including, but not limited to, a palladium-catalyzed Buchwald reaction, a copper-catalyzed Ullmann reaction, *etc.* When ring D is linked to ring B through a nitrogen atom, compound **(I)** can be generated through a reaction of compound **I-14** and compound **I-15** in step f), which corresponds to alkaline direct nucleophilic substitution conditions including, but not limited to, a reaction using sodium hydride, cesium carbonate, or potassium phosphate as a base in various solvents such as DMF, acetonitrile, or tetrahydrofuran. Alternatively, step f) represents a metal-catalyzed coupling reaction, such as a palladium-catalyzed Buchwald reaction, a copper-catalyzed Ullmann reaction, *etc.* When ring D is linked to ring B through a carbon atom, compound **(I)** can be generated through a reaction of compound **I-14** and boric acid compound **I-16** or borate ester compound **I-17** in step g), which corresponds to a palladium-catalyzed Suzuki reaction, *etc.*, and the palladium catalysts used include, but are not limited to, Ruphos-Pd-G3, Pd(dppf)Cl₂, Pd₂(dba)₃, *etc.* In the structural moiety, Y₁ and Y₂ represent leaving groups, such as triflate, *p*-toluenesulfonate, chlorine, bromine, iodine, *etc.*

### Synthesis method (V):

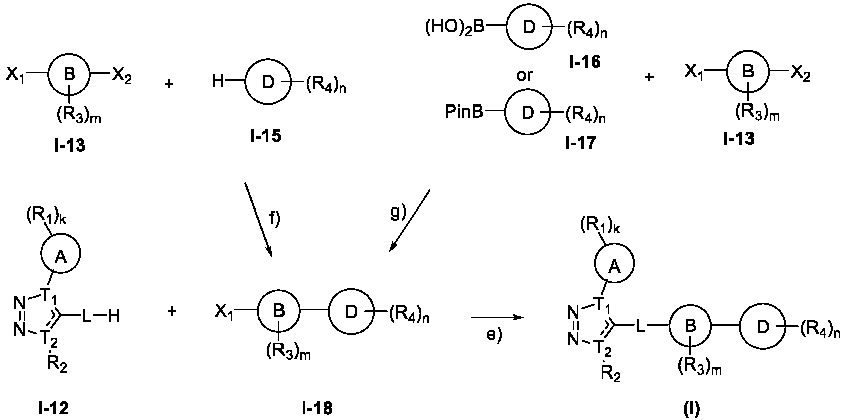

In another embodiment provided by the present disclosure, compound **(I)** can be synthesized through a direct substitution reaction or coupling reaction of compound **I-12** and compound **I-18** in step e). When ring D is linked to ring B through a nitrogen atom, compound **I-18** can be generated through a reaction of compound **I-13** and compound **I-15** in step f); when ring D is linked to ring B through a carbon atom, compound **I-18** can be generated through a reaction of compound **I-13** and boric acid compound **I-16** or borate ester compound **I-17** in step g); the synthesis steps e), f), and g) are as described in synthesis method (IV).

### Synthesis method (VI):

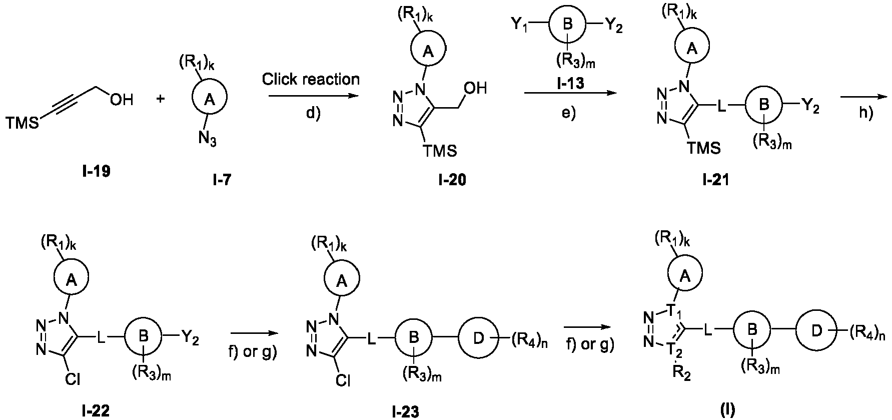

In another embodiment provided by the present disclosure, **I-20** is obtained as a product of the Click reaction of compound **I-19** and compound **I-7** in step d), and after the introduction of ring B in step e), trimethylsilyl is converted into chlorine in the molecule of compound **I-21** in step h), which corresponds to the conditions using NCS, DCDMH, *etc.* as the source of chlorine and KF, TBAF, *etc.* as the base. Chlorotriazole compound **I-23** is obtained through a direct substitution reaction or coupling reaction of compound **I-22** with the introduction of ring D, and an R₂ group can be introduced into the molecule of compound **I-23** through a metal-catalyzed coupling reaction, which is suitable for the synthesis of compounds with different R₂ groups. The synthesis steps d), e), f), and g) are as described in synthesis method (IV).

The present disclosure also relates to the compound of general formula **(I)** as described above, which is prepared by the method as described above. If the preparation method is not described in the examples, the compound of general formula **(I)** and the intermediate product thereof can be prepared according to a similar method or the method as described above. The raw materials known in the art can be commercially available, or can be prepared according to methods known in the art or a similar method based on the known methods.

It is understandable that the compound of general formula **(I)** of the present disclosure can be derivatized on the functional group to obtain the derivative which can be converted into the parent compound *in vivo.*

### Pharmaceutical composition

The present disclosure provides a use of a pharmaceutical composition comprising a therapeutically effective dose of an α5-GABA_{A} inverse agonist. Although the α5-GABA_{A} inverse agonist for use in the treatment of the present disclosure may be administered in the form of a raw material compound, it is preferred that the active ingredient, optionally in the form of a physiologically acceptable salt, is mixed into a pharmaceutical composition together with one or more than one additive, excipient, carrier, buffer, diluent, and/or other conventional pharmaceutical auxiliary material.

In a preferred embodiment, the present disclosure provides a pharmaceutical composition comprising an α5-GABA_{A} inverse agonist, wherein the α5-GABA_{A} inverse agonist is mixed with one or more than one pharmaceutically acceptable carrier, and optionally with other therapeutic and/or prophylactic components known or used in the art. The carrier must be "acceptable" in the sense of being compatible with the other ingredients of the preparation and not harmful to the recipient thereof.

The pharmaceutical compositions for use in the present disclosure may be those suitable for oral, rectal, bronchial, nasal, pulmonary, topical (including buccal and sublingual), transdermal, vaginal, or parenteral (including cutaneous, subcutaneous, intramuscular, intraperitoneal, intravenous, intraarterial, intracerebral, intraocular injection or infusion) administration, or those in a form suitable for administration by inhalation or spray, including powder and liquid aerosol administration, or sustained release system administration. Suitable examples of sustained release systems include a semipermeable matrix of a solid hydrophobic polymer containing the compound of the present disclosure, wherein the matrix may be in the form of a shaped article, such as a film or microcapsule.

The compound for use in the present disclosure, together with a conventional additive or diluent, may thus be formulated into pharmaceutical compositions and unit dose forms thereof. Such forms include solids (especially in the form of tablets, filled capsules, powders, and pills), and liquids (especially aqueous or non-aqueous solutions, suspensions, emulsions, and elixirs), and capsules filled with the above forms, all forms for oral administration, suppositories for rectal administration, and sterile injectable solutions for parenteral administration. Such pharmaceutical compositions and unit dose forms thereof may comprise conventional ingredients in conventional proportions, with or without additional active compounds or ingredients, and such unit dose forms may contain any suitable effective dose of the active ingredient commensurate with the desired daily dose range to be employed.

The compound for use in the present disclosure can be administered in a variety of oral and parenteral dosage forms. For those skilled in the art, the following dosage forms may comprise the compound or the pharmaceutically acceptable salt thereof of the present disclosure as the active ingredient.

For preparing a pharmaceutical composition from the compound for use in the present disclosure, the pharmaceutically acceptable carrier may be a solid or liquid. Solid preparations include powders, tablets, pills, capsules, cachets, suppositories, and dispersible granules. A solid carrier may be one or more than one substance which also functions as a diluent, flavoring agent, solubilizer, lubricant, suspending agent, binder, preservative, tablet disintegrating agent, or encapsulating material.

In powders, the carrier is a finely divided solid, which is mixed with the finely divided active ingredient.

In tablets, the active ingredient is mixed with the carrier having the necessary binding capacity in appropriate proportions and compressed into the desired shape and size.

The powders and tablets preferably contain 5% or 10% to about 70% of the active compound. Suitable carriers are magnesium carbonate, magnesium stearate, talc, sugar, lactose, pectin, dextrin, starch, gelatin, tragacanth, methylcellulose, sodium carboxymethylcellulose, low melting waxes, cocoa butter, *etc.* The term "preparation" includes an active compound formulated with an encapsulating material as the carrier providing a capsule in which the active component, with or without carriers, is surrounded by the carrier so as to be associated therewith. Similarly, the preparations include cachets and lozenges. Tablets, powders, capsules, pills, cachets, and lozenges can be used as solid forms suitable for oral administration.

For preparing suppositories, a low melting wax, such as a mixture of fatty acid glyceride or cocoa butter, is first melted and then the active ingredient is homogeneously dispersed therein by stirring. The molten homogeneous mixture is then poured into appropriately sized molds, allowed to cool, and thereby to solidify.

Compositions suitable for vaginal administration may be in the form of vaginal suppositories, tampons, creams, gels, pastes, foams, or sprays, and the compositions contain, in addition to the active ingredient, suitable carriers known in the art.

Liquid preparations include solutions, suspensions, and emulsions, for example, aqueous solutions or water-propylene glycol solutions. For example, liquid preparations for parenteral injection can be formulated as water-polyethylene glycol solutions.

The compound for use in the present disclosure may thus be formulated for parenteral administration (*e.g*., injection, such as bolus injection or continuous infusion) and may be present in unit dose form in ampoules, pre-filled syringes, small volume infusion bags, or multi-dose containers with an added preservative. The compositions may take the form of suspensions, solutions, or emulsions in oily or aqueous carriers, and may contain preparation ingredients such as suspending agents, stabilizers, and/or dispersants. Alternatively, the active ingredient may be in powder form, which may be obtained by aseptic isolation from a sterile solid or by lyophilization from a solution for constitution with a suitable carrier, such as sterile, pyrogen-free water, before use.

Aqueous solutions suitable for oral administration can be prepared by dissolving the active ingredient in water and adding desired colorants, flavoring agents, stabilizers, and thickeners.

Aqueous suspensions suitable for oral administration can be prepared by dispersing the finely divided active ingredient in water containing a viscous substance, such as natural or synthetic gums, resins, methylcellulose, sodium carboxymethylcellulose, or other well-known suspending agents.

Also included are solid preparations designed for conversion shortly before use to liquid preparations for oral administration. Such liquid preparations include solutions, suspensions, and emulsions. In addition to the active ingredient, such preparations may include colorants, flavoring agents, stabilizers, buffers, artificial and natural sweeteners, dispersants, thickeners, solubilizers, *etc.*

For topical application to the epidermis, the compound of the present disclosure may be formulated as an ointment, cream, or lotion, or transdermal patch. For example, ointments and creams may be formulated with an aqueous or oily matrix plus suitable thickeners and/or gelling agents. Lotions may be formulated with an aqueous or oily matrix and in general also contain one or more than one emulsifier, stabilizer, dispersant, suspending agent, thickener, or colorant.

Compositions suitable for oral or topical administration include lozenges containing the active ingredient in a flavored matrix, typically sucrose and arabic gum or tragacanth; pastilles containing the active ingredient in an inert matrix such as gelatin and glycerol or sucrose and arabic gum; and mouthwashes containing the active ingredient in a suitable liquid carrier.

Solutions or suspensions may be applied directly to the nasal cavity by conventional methods, for example with a dropper, pipette, or nebulizer. The composition may be provided in single-dose or multi-dose form.

Respiratory administration can also be achieved by means of an aerosol in which the active ingredient is provided in a pressurized pack with a suitable propellant including chlorofluorocarbon (CFC) such as dichlorodifluoromethane, trichlorofluoromethane, or dichlorotetrafluoroethane, carbon dioxide, or other suitable gas. The aerosol may also contain a surfactant such as lecithin, as appropriate. The dose of the medicament can be controlled by a metered valve.

In addition, the active ingredient may be in the form of a dry powder, for example, a powder mixture of the compound with a suitable powder matrix such as lactose, starch, or starch derivatives such as hydroxypropyl methylcellulose and polyvinylpyrrolidone (PVP). The powder carrier allows for easy gel formation in the nasal cavity. The powder composition may be present in unit dose form, for example, in capsules or cartridges (*e.g*., gelatin capsules or cartridges), or in blister packs in which the powder can be administered by means of an inhaler.

In compositions for respiratory administration (including intranasal compositions), the compound generally has a small particle size, for example, a particle size of 5 microns or less. Such a particle size can be obtained by methods known in the art, for example by micronization.

If desired, compositions suitable for sustained release of the active ingredient can be applied.

The pharmaceutical preparation is preferably in unit dose form. In such form, the preparation is subdivided into unit doses containing the appropriate amount of the active ingredient. The unit dose form can be an encapsulated preparation where the sealed package contains a large number of separated preparations, such as encapsulated tablets, capsules, and powders in vials or ampoules. In addition, the unit dose form can be a capsule, tablet, cachet, or lozenge itself, or it can be the appropriate amount of any of the above capsules and tablets in encapsulated form.

Tablets or capsules for oral administration and liquids for intravenous administration and continuous infusion are preferred compositions. More detailed information on preparation and administration techniques can be found in the latest edition of Remington's Pharmaceutical Sciences (Maack Publishing Co., Easton, PA).

The amount of active ingredient in a unit dose preparation can vary depending on the specific application and the efficacy of the active ingredient, and can be adjusted from 0.1 mg to about 1 g. For example, in pharmaceutical use, the medicament can be administrated in capsules of 0.1 to about 400 mg one to three times daily, and the composition may also contain other compatible therapeutic agents if necessary.

### DETAILED DESCRIPTION OF THE PREFERRED EMBODIMENT

The present disclosure is described in detail by the examples below, but it does not mean that there are any adverse restrictions on the present disclosure. The present disclosure has been described in detail herein, and its specific examples have also been disclosed; for one skilled in the art, it is obvious to make various modifications and improvements to the specific examples of the present disclosure without departing from the spirit and scope of the present disclosure.

The experimental materials and reagents used in the following examples can be obtained from commercially available sources unless otherwise specified.

### Preparation of intermediates:

### Synthesis of intermediate A8:

### 3-Chloro-6-((1-(4-(difluoromethyl)phenyl)-4-methyl-1H-1,2,3-triazol-5-yl)methoxy)pyridazine (A8)

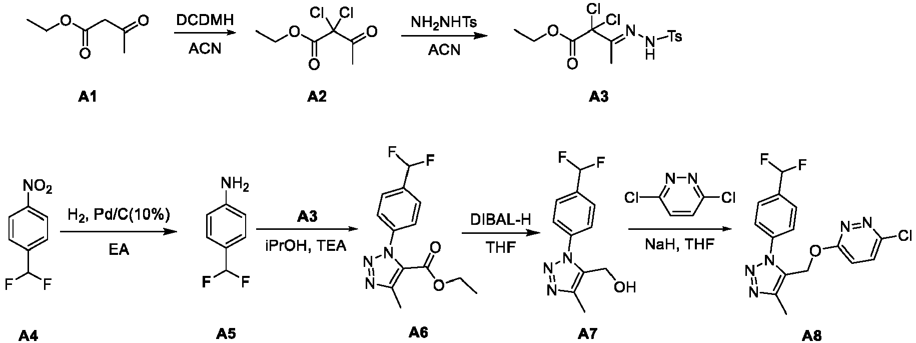

### Step 1: Synthesis of ethyl 2,2-dichloro-3-oxobutanoate (A2)

**A1** (20 g, 153.7 mmol) and ammonium chloride (4.11 g, 76.84 mmol) were dissolved in acetonitrile (300 mL), then 1,3-dichloro-5,5-dimethylhydantoin (45.42 g, 230.5 mmol) was added thereto in batches, and the reaction was stirred at room temperature for 18 hours under an argon atmosphere. The completion of the reaction was monitored by TLC. The reaction mixture was concentrated under reduced pressure, and the residue was dissolved in petroleum ether (300 mL), washed with saturated brine (200 mL × 3), dried over anhydrous sodium sulfate, filtered, and the filtrate was concentrated under reduced pressure to obtain the title product (30 g, colorless liquid).

### Step 2: Synthesis of ethyl (3E)-2,2-dichloro-3-[(4-methylbenzenesulfonamido)imino]butanoate (A3)

*p*-Toluenesulfonylhydrazide (13 g, 69.81 mmol) was dissolved in isopropanol (80 mL) at room temperature, then **A2** (14.59 g, 73.30 mmol) was added thereto, and the reaction was stirred at room temperature for 18 hours. The reaction mixture was concentrated under reduced pressure to remove half of the solvent, and the residue was left at 0°C for 2 hours. The residue was filtered, and the filter cake was collected and dried under reduced pressure to obtain the title product (14 g, yellow solid).

### Step 3: Synthesis of 4-(difluoromethyl)aniline hydrochloride (A5)

**A4** (3.6 g, 20.79 mmol) was dissolved in ethyl acetate (70 mL), cooled to 0°C, then wet palladium on carbon (10%, 0.36 g) was added thereto. The system was replaced with hydrogen (hydrogen balloon), and the reaction was stirred at 0°C for 18 hours. The completion of the reaction was monitored by TLC. The reaction mixture was filtered at 0°C, and hydrochloric acid/ethyl acetate solution (4 mL, 4.0 M) was added dropwise to the filtrate while stirring. The mixture was stirred at 0°C for 30 minutes, filtered, and the filter cake was collected and dried under reduced pressure to obtain the title product (3.1 g, yellow solid).

### Step 4: Synthesis of ethyl 1-(4-(difluoromethyl)phenyl)-4-methyl-1H-1,2,3-triazole-5-carboxylate (A6)

**A5** (2.3 g, 16.07 mmol) and **A3** (5.9 g, 16.07 mmol) were mixed in isopropanol (70 mL), cooled to 0°C, and triethylamine (0.36 g, 64.28 mmol) was added dropwise thereto. The mixture was slowly warmed to room temperature, and the reaction was stirred for 18 hours. The completion of the reaction was monitored by TLC. The reaction mixture was diluted with isopropanol (100 mL), filtered, and the filtrate was concentrated under reduced pressure. The residue was purified by flash silica gel column chromatography (ethyl acetate: petroleum ether = 0:1 to 1:3) to obtain the title product (3.0 g, yellow solid).

MS (ESI) m/z [M+H]⁺ = 282.1.

### Step 5: Synthesis of 1-(4-(difluoromethyl)phenyl)-4-methyl-1H-1,2,3-triazol-5-yl)methanol (A7)

**A6** (3.0 g, 10.67 mmol) was dissolved in tetrahydrofuran (25 mL) under an argon atmosphere, cooled to 0°C, and diisobutylaluminum hydride (1.5 M toluene solution, 28.45 mL, 42.68 mmol) was added dropwise thereto. The reaction mixture was slowly warmed to room temperature, and the reaction was stirred for 3 hours. The completion of the reaction was monitored by TLC. The reaction mixture was slowly poured into 1 M hydrochloric acid (100 mL) at 0°C to quench. The mixture was extracted with ethyl acetate (150 mL × 2), washed with water (200 mL) and saturated brine (100 mL), dried over anhydrous sodium sulfate, and the filtrate was concentrated under reduced pressure to obtain the title product (1.4 g, yellow solid).

MS (ESI) m/z [M+H]⁺ = 240.1.

### Step 6: Synthesis of 3-chloro-6-((1-(4-(difluoromethyl)phenyl)-4-methyl-1H-1,2,3-triazol-5-yl)methoxy)pyridazine (A8)

**A7** (1.4 g, 5.85 mmol) was dissolved in tetrahydrofuran (25 mL) under an argon atmosphere, cooled to 0°C, and sodium hydride (0.35 g, 8.77 mmol, 60%) was added thereto. The reaction was stirred at 0°C for 0.5 hours, and then 3,6-dichloropyridazine (1.31 g, 8.77 mmol) was added thereto. The reaction mixture was slowly warmed to room temperature and stirred for 1 hour. The completion of the reaction was monitored by TLC. The reaction mixture was slowly poured into ice water (100 mL) at 0°C to quench. The mixture was extracted with ethyl acetate (100 mL × 2), washed with water (100 mL) and saturated brine (100 mL), dried over anhydrous sodium sulfate, and the filtrate was concentrated under reduced pressure. The residue was purified by flash silica gel column chromatography (ethyl acetate: petroleum ether = 0: 1 to 2:1) to obtain the title product (1.8 g, yellow solid).

MS (ESI) m/z [M+H]⁺ = 352.1.

### Synthesis of intermediate A9:

### 3-((1-(4-(Difluoromethyl)phenyl)-4-methyl-1H-1,2,3-triazol-5-yl)methoxy)-6-iodopyridazine (A9)

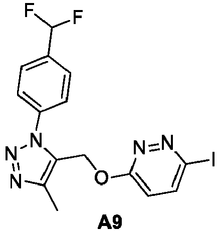

The experimental operation was as described in the synthesis of intermediate **A8,** using **A7** and 3-chloro-6-iodopyridazine as reactants to obtain the title product.

MS (ESI) m/z [M+H]⁺ = 443.8.

¹H NMR (400 MHz, CDsOD) δ 7.91 (d, *J* = 9.2 Hz, 1H), 7.79-7.74 (m, 4H), 7.02-6.75 (m, 2H), 5.59-5.64 (m, 2H), 2.48-2.51 (m, 3H).

### Synthesis of intermediate A10:

### 5-Bromo-2-((1-(4-(difluoromethyl)phenyl)-4-methyl-1H-1,2,3-triazol-5-yl)methoxy)pyridine (A10)

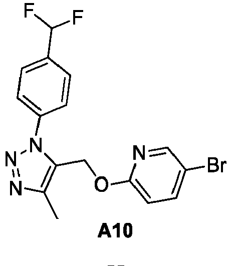

The experimental operation was as described in the synthesis of intermediate **A8,** using **A7** and 2-fluoro-5-bromopyridine as reactants to obtain the title product.

MS (ESI) m/z [M+H]⁺ = 395.2, 397.2.

### Synthesis of intermediate A11:

### 2-((1-(4-(Difluoromethyl)phenyl)-4-methyl-1H-1,2,3-triazol-5-yl)methoxy)-5-iodopyridine (A11)

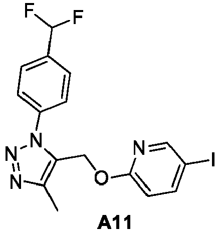

The experimental operation was as described in the synthesis of intermediate **A8,** using **A7** and 2-fluoro-5-iodopyridine as reactants to obtain the title product.

MS (ESI) m/z [M+H]⁺ = 443.0.

### Synthesis of intermediate A12:

### 6-Chloro-3-((1-(4-(difluoromethyl)phenyl)-4-methyl-1H-1,2,3-triazol-5-yl)methoxy)pyridazine-4-carbonitrile (A12)

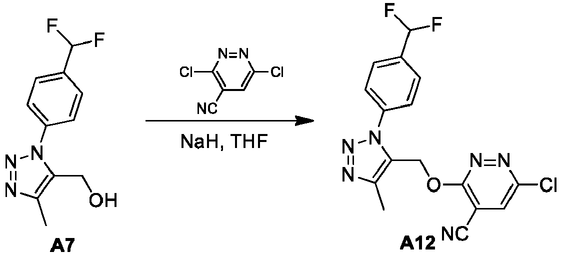

The experimental operation was as described in the synthesis of intermediate **A8,** using **A7** and 3,6-dichloropyridazine-4-carbonitrile as reactants to obtain the title product.

MS (ESI) m/z [M+H]⁺ = 377.1.

### Synthesis of intermediate B4:

### 3-Chloro-6-((1-(5-chloropyridin-2-yl)-4-methyl-1H-1,2,3-triazol-5-yl)methoxy)pyridazine (B4)

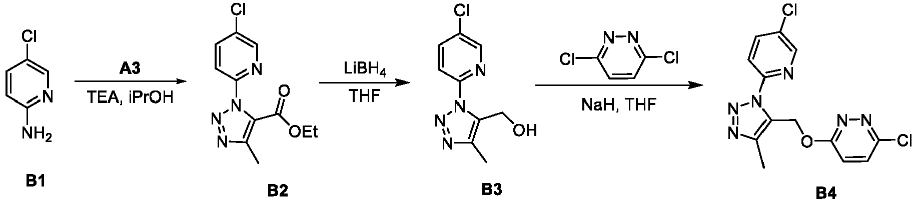

### Step 1: Synthesis of ethyl 1-(5-chloropyridin-2-yl)-4-methyl-1H-1,2,3-triazole-5-carboxylate (B2)

The experimental operation was as described in step 4 in the synthesis of intermediate **A8,** using intermediates **B1** and **A3** as reactants to obtain the title product.

MS (ESI) m/z [M+H]⁺ = 267.1.

### Step 2: Synthesis of (1-(5-chloropyridin-2-yl)-4-methyl-1H-1,2,3-triazol-5-yl)methanol (B3)

Compound **B1** (200 mg, 0.75 mmol) was dissolved in tetrahydrofuran (20 mL) under an argon atmosphere, then lithium borohydride (0.16 g, 7.50 mmol) was added thereto, and the reaction was stirred at 30°C for 16 hours. The completion of the reaction was monitored by TLC. The reaction mixture was quenched by adding water (30 mL) and extracted with ethyl acetate (30 mL × 3). The organic phases were combined, washed with saturated brine (50 mL), dried over anhydrous sodium sulfate, filtered, and the filtrate was concentrated under reduced pressure. The residue was purified by flash silica gel column chromatography (ethyl acetate: petroleum ether = 0: 1 to 1:2) to obtain the title product (0.12 g, white solid).

MS (ESI) m/z [M+H]⁺ = 225.1.

### Step 3: Synthesis of 3-chloro-6-((1-(5-chloropyridin-2-yl)-4-methyl-1H-1,2,3-triazol-5-yl)methoxy)pyridazine (B4)

The experimental operation was as described in the synthesis of intermediate **A8,** using intermediate **B3** and 3,6-dichloropyridazine as reactants to obtain the title product.

MS (ESI) m/z [M+H]⁺ = 337.0.

### Synthesis of intermediate C4:

### 3-Chloro-6-((1-(5-fluoropyridin-2-yl)-4-methyl-1H-1,2,3-triazol-5-yl)methoxy)pyridazine (C4)

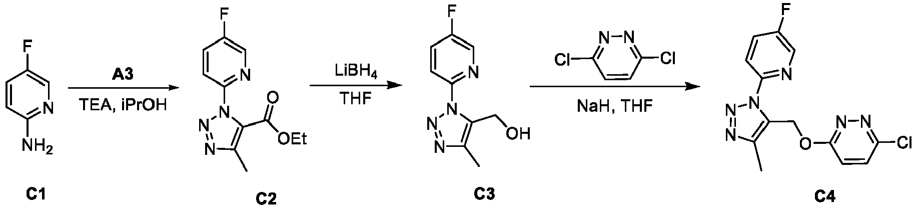

The experimental operation was as described in the synthesis of intermediate **B4,** using C1 as the starting material to obtain the title product.

MS (ESI) m/z [M+H]⁺ = 321.1

### Synthesis of intermediate D4:

### 3-Chloro-6-((1-6-(difluoromethyl)pyridin-3-yl)-4-methyl-1H-1,2,3-triazol-5-yl)methoxy)pyridazine (D4)

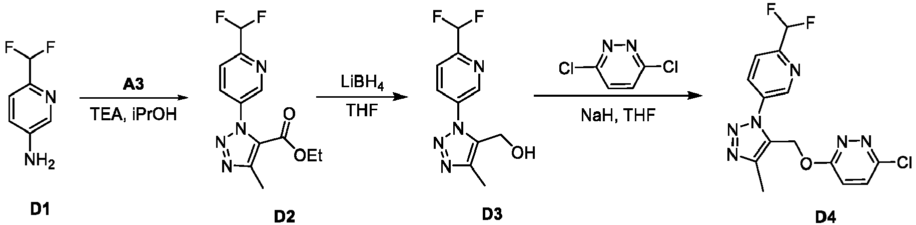

The synthesis method was as described in the synthesis of intermediate **B4,** using **D1** as the starting material to obtain the title product.

MS (ESI) m/z [M+H]⁺ = 353.1.

### Synthesis of intermediate E4:

### 3-Chloro-6-((1-(4-cyanophenyl)-4-methyl-1H-1,2,3-triazol-5-yl)methoxy)pyridazine (E4)

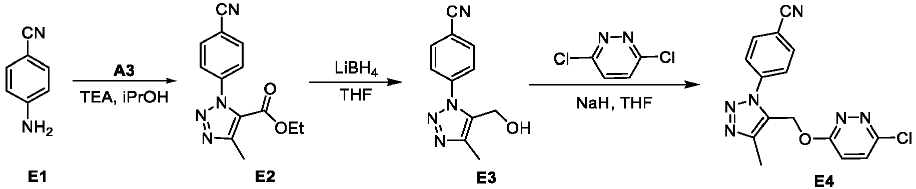

The synthesis method was as described in the synthesis of intermediate **B4,** using **E1** as the starting material to obtain the title product.

MS (ESI) m/z [M+H]⁺ = 327.0.

### Synthesis of intermediate F4:

### 3-Chloro-6-((1-(4-fluorophenyl)-4-methyl-1H-1,2,3-triazol-5-yl)methoxy)pyridazine (F4)

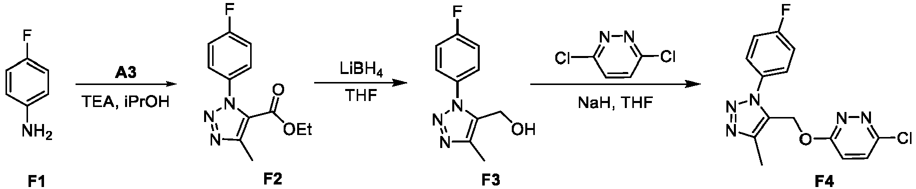

The synthesis method was as described in the synthesis of intermediate **B4,** using **F1** as the starting material to obtain the title product.

### MS (ESI) m/z [M+H]⁺ = 319.8.

### Synthesis of intermediate G5:

3-((1-(4-(((*tert*-Butyldimethylsilyl)oxy)methyl)phenyl)-4-methyl-1*H*-1,2,3-triazol-5-yl)methoxy)-6-chloropyridazine (**G5**)

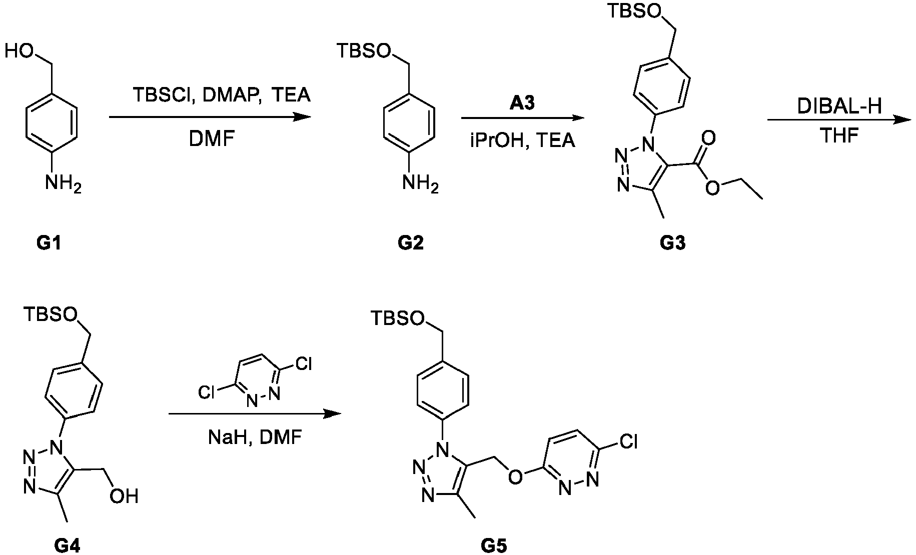

### Step 1: Synthesis of 4-(((tert-butyldimethylsilyl)oxy)methyl)aniline (G2)

Compound **G1** (3.0 g, 24.4 mmol), *tert*-butyldimethylsilyl chloride (4.04 g, 26.8 mmol), DMAP (0.98 g, 8.04 mmol), and triethylamine (2.71 g, 26.8 mmol) were sequentially added to DMF (40.0 mL) at room temperature, and the reaction mixture was reacted at 20°C for 16 hours. The reaction mixture was diluted with water (200 mL) and extracted with dichloromethane (100 mL × 3). The organic phases were combined, dried over anhydrous sodium sulfate, filtered, and the filtrate was concentrated under reduced pressure to obtain the title compound (5.70 g, red liquid).

¹H NMR (400 MHz, DMSO-*d₆*) *δ* 6.94 (d, *J* = 8.4 Hz, 2H), 6.51 (d, *J* = 8.4 Hz, 2H), 4.96 (s, 2H), 4.49 (s, 2H), 0.86 (s, 9H), 0.03 (s, 6H).

### Step 2: Synthesis of ethyl 1-(4-(((tert-butyldimethylsilyl)oxy)methyl)phenyl)-4-methyl-1H-1,2,3-triazole-5-carboxylate (G3)

The experimental operation was as described in step 4 in the synthesis of intermediate **A8,** using intermediates **A3** and **G2** as reactants to obtain the title product.

¹H NMR (400 MHz, CDCl₃) *δ* 7.52-7.43 (m, 2H), 7.42-7.35 (m, 2H), 4.83 (s, 2H), 4.27 (q, *J* = 6.8 Hz, 2H), 2.64 (s, 3H), 1.24 (t, *J* = 7.2 Hz, 3H), 0.97 (s, 9H), 0.13 (s, 6H).

### Step 3: Synthesis of (1-(4-(((tert-butyldimethylsilyl)oxy)methyl)phenyl)-4-methyl-1H-1,2,3-triazol-5-yl)methanol (G4)

The experimental operation was as described in step 5 in the synthesis of intermediate **A8,** using intermediate **G3** as the reactant to obtain the title product.

¹H NMR (400 MHz, CDCl₃) *δ* 7.68-7.57 (m, 2H), 7.53-7.46 (m, 2H), 4.83 (s, 2H), 4.70-4.65 (m, 2H), 2.49-2.40 (m, 3H), 0.97 (s, 9H), 0.13 (s, 6H).

### Step 4: Synthesis of 3-((1-(4-(((tert-butyldimethylsilyl)oxy)methyl)phenyl)-4-methyl-1H-1,2,3-triazol-5-yl)methoxy)-6-chloropyridazine (G5)

The experimental operation was as described in step 6 in the synthesis of intermediate **A8,** using intermediate **G4** and 3,6-dichloropyridazine as reactants to obtain the title product.

MS (ESI) m/z [M+H]⁺ = 446.2.

¹H NMR (400 MHz, CDCl₃) *δ* 7.55-7.45 (m, 4H), 7.43 (d, *J* = 9.2 Hz, 1H), 6.98 (d, *J* = 9.2 Hz, 1H), 5.54 (s, 2H), 4.81 (s, 2H), 2.51 (s, 3H), 0.96 (s, 9H), 0.13 (s, 6H).

### Synthesis of intermediate H5:

### 3-Chloro-6-((4-cyclopropyl-1-(4-(difluoromethyl)phenyl)-1H-1,2,3-triazol-5-yl)methoxy)pyridazine (H5)

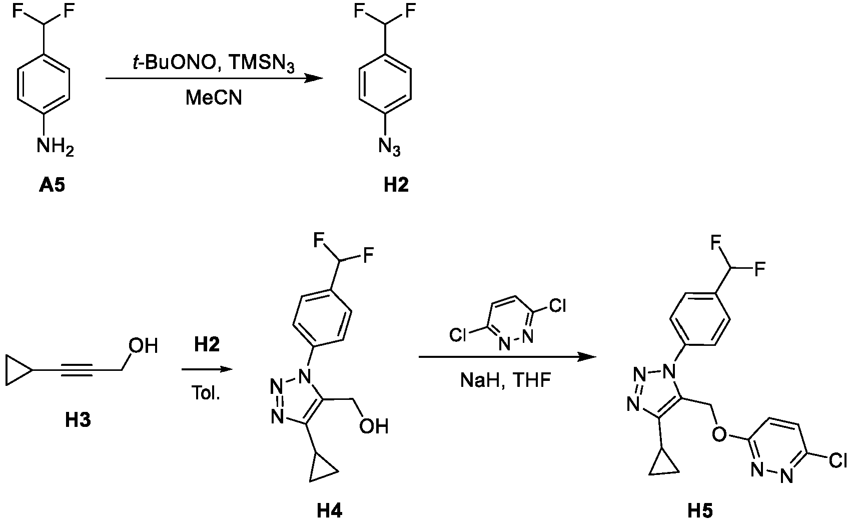

### Step 1: Synthesis of 1-azido-4-(difluoromethyl)benzene (H2)

**A5** (1.0 g, 5.59 mmol) was slowly added to a mixed solution of sulfuric acid (1 mL) and trifluoroacetic acid (5 mL) at 0°C, then sodium nitrite (501 mg, 7.26 mmol) aqueous solution (5 mL) was slowly added dropwise to the above mixture, and the reaction was carried out at 0°C for 0.5 hours. At this temperature, sodium azide (700 mg, 10.8 mmol) aqueous solution (2 mL) was slowly added dropwise to the above mixture, and the reaction mixture was warmed to 16°C and reacted for 2 hours. TLC showed that the reaction was completed. The reaction mixture was added with 15% sodium hydroxide aqueous solution to adjust the pH to 9, stirred for 10 minutes, and then extracted with ethyl acetate (50 mL × 3). The organic phase was dried over anhydrous sodium sulfate and filtered, and the filtrate was concentrated under reduced pressure to obtain the title product (1.03 g, brown liquid).

### Step 2: Synthesis of (4-cyclopropyl-1-(4-(difluoromethyl)phenyl)-1H-1,2,3-triazol-5-yl)methanol (H4)

**H3** (500 mg, 5.2 mmol) and **H2** (2.64 g, 15.6 mmol) were added to toluene (15 mL), and the reaction mixture was stirred at 120°C for 12 hours under a nitrogen atmosphere. Product generation was detected by LCMS. The reaction mixture was concentrated under reduced pressure to obtain a crude product. The crude product was separated by high performance liquid chromatography (chromatographic column: Phenomenex C18 150 * 40 mm * 5 µm; mobile phase: water (0.225% trifluoroacetic acid) - acetonitrile; gradient: 25% to 45%/10 min; flow rate: 60 mL/min) to obtain the title product (270 mg, yellow solid).

MS (ESI) m/z [M+H]⁺ = 266.1.

¹H NMR (400 MHz, DMSO-*d*₆) δ 7.86-7.79 (m, 4H), 7.16 (t, *J* = 55.6 Hz, 1H), 5.59 (t, *J* = 5.2 Hz, 1H), 4.82 (d, *J* = 5.6 Hz, 2H), 2.10-2.00 (m, 1H), 0.99-0.96 (m, 2H), 0.92-0.91 (m, 2H).

### Step 3: Synthesis of 3-chloro-6-((4-cyclopropyl-1-(4-(difluoromethyl)phenyl)-1H-1,2,3-triazol-5-yl)methoxy)pyridazine (H5)

The experimental operation was as described in the synthesis of intermediate **A8,** using **H4** and 3,6-dichloropyridazine as reactants to obtain the title product.

MS (ESI) m/z [M+H]⁺ = 378.0.

¹H NMR (400 MHz, CDCl₃) δ 7.71-7.66 (m, 4H), 7.44 (d, *J=* 9.2 Hz, 1 H), 6.99 (d, *J* = 9.2 Hz, 1 H), 6.72 (t, *J=* 56.4 Hz, 1H), 5.68 (s, 2 H), 2.07-2.03 (m, 1 H), 1.06-1.05 (m, 2H), 1.04-1.03 (m, 2H).

### Synthesis of intermediate J8:

### 5-(((6-Chloropyridazin-3-yl)oxy)methyl)-1-(4-(difluoromethyl)phenyl)-1H-1,2,3-triazole-4-carbonitrile (J8)

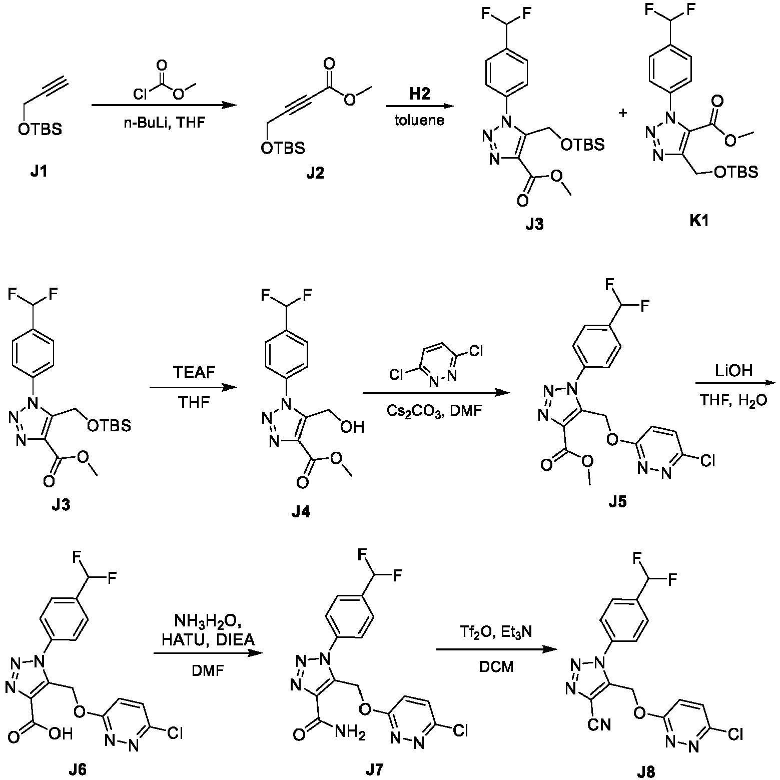

### Step 1: Synthesis of methyl 4-((tert-butyldimethylsilyl)oxy)but-2-ynoate (J2)

**J1** (5.0 g, 29.4 mmol) was dissolved in anhydrous tetrahydrofuran (50 mL), cooled to -78°C, and a solution of *n*-butyllithium in tetrahydrofuran (14.1 mL, 2.5 M, 35.3 mmol) was slowly added dropwise thereto under a nitrogen atmosphere. The reaction mixture was stirred for 30 minutes. Methyl chloroformate (3.18 g, 33.8 mmol) was slowly added to the above reaction mixture. The reaction mixture was warmed to room temperature and reacted for 12 hours. TLC showed that all raw materials were consumed. The reaction mixture was quenched with saturated ammonium chloride aqueous solution (50 mL) and extracted with ethyl acetate (50 mL × 3). The organic phase was dried over anhydrous sodium sulfate and then concentrated under reduced pressure. The crude product was purified by flash silica gel column chromatography (ethyl acetate: petroleum ether = 0: 1 to 1:3) to obtain the title product (4.36 g, yellow liquid).

¹H NMR (400 MHz, CDCl₃) δ 4.43 (s, 2H), 3.78 (s, 3H), 0.91 (s, 9H), 0.13 (s, 6H).

### Step 2: Synthesis of methyl 5-(((tert-butyldimethylsilyl)oxy)methyl)-1-(4-(difluoromethyl)phenyl)-1H-1,2,3-triazole-4-carboxylate (J3)

Compound **J2** (135 mg, 0.59 mmol) and compound **H2** (100 mg, 0.59 mmol) were dissolved in anhydrous toluene (5.0 mL) at room temperature. The reaction mixture was heated to 100°C, stirred, and reacted for 12 hours. The reaction endpoint was monitored by TLC. The reaction mixture was concentrated under reduced pressure, and the crude product was purified by silica gel column chromatography (petroleum ether: ethyl acetate = 100: 1 to 3:1) to obtain the title product **J3** (27 mg, light yellow solid) and the isomer compound **K1** (27 mg, yellow oil) of **J3**.

### Compound J3:

¹H NMR (400 MHz, CDsOD) δ 7.86-7.81 (m, 2H), 7.80-7.75 (m, 2H), 6.89 (t, *J* = 56.0 Hz, 1H), 5.08 (s, 2H), 3.95 (s, 3H), 0.76 (s, 9H), 0.00 (s, 6H)

### Compound K1:

¹H NMR (400 MHz, CDsOD) δ 7.75 (d, *J* = 8.0 Hz, 2H), 7.65 (d, *J* = 8.8 Hz, 2H), 6.91 (t, *J=* 56.0 Hz, 1H), 5.05 (s, 2H), 3.82 (s, 3H), 0.93 (s, 9H), 0.16 (s, 6H)

### Step 3: Synthesis of methyl 1-(4-(difluoromethyl)phenyl)-5-(hydroxymethyl)-1H-1,2,3-triazole-4-carboxylate (J4)

**J3** (200 mg, 0.504 mmol) and TEAF (378 mg, 2.52 mmol) were sequentially added to anhydrous tetrahydrofuran (5 mL) at room temperature, and the system was replaced with nitrogen three times. The reaction mixture was reacted at 40°C for 1 hour. TLC showed that all raw materials were consumed. The reaction mixture was concentrated under reduced pressure, and the crude product was purified by flash silica gel column chromatography (ethyl acetate: petroleum ether = 0: 1 to 1:3) to obtain the title product (105 mg, white solid).

¹H NMR (400 MHz, CDCl₃) δ 7.80-7.75 (m, 2H), 7.72-7.67 (m, 2H), 6.77 (t, *J* = 56.4 Hz, 1H), 4.87 (d, *J=* 7.6 Hz, 2H), 4.08 (s, 3H), 3.82 (t, *J* = 7.2 Hz, 1H)

### Step 4: Synthesis of methyl 5-(((6-chloropyridazin-3-yl)oxy)methyl)-1-(4-(difluoromethyl)phenyl)-1H-1,2,3-triazole-4-carboxylate (J5)

Compound **J4** (65 mg, 0.23 mmol), 3,6-dichloropyridazine (51 mg, 0.34 mmol), and cesium carbonate (225 mg, 0.69 mmol) were sequentially added to anhydrous DMF (4.0 mL) at room temperature. The system was replaced with nitrogen three times, and the reaction was carried out for 14 hours. TLC showed that all raw materials were consumed. The reaction mixture was diluted with ethyl acetate (50 mL) and washed with saturated brine (5 mL × 3). The organic phase was dried over anhydrous sodium sulfate and then concentrated under reduced pressure. The crude product was purified by flash silica gel column chromatography (methanol: dichloromethane = 0:1 to 1:10) to obtain the title product (95 mg, yellow oil).

¹H NMR (400 MHz, CDsOD) δ 7.77 (s, 4H), 7.63 (d, *J* = 9.2 Hz, 1H), 7.08 (d, *J* = 9.2 Hz, 1H), 6.89 (t, *J=* 55.6 Hz, 1H), 5.92 (s, 2H), 3.97 (s, 3H)

### Step 5: Synthesis of 5-(((6-chloropyridazin-3-yl)oxy)methyl)-1-(4-(difluoromethyl)phenyl)-1H-1,2,3-triazole-4-carboxylic acid (J6)

**J5** (95.0 mg, 0.25 mmol) was dissolved in anhydrous tetrahydrofuran (4 mL) and water (2 mL) at room temperature, then lithium hydroxide monohydrate (33 mg, 0.79 mmol) was added thereto, and the reaction was carried out for 2 hours. TLC showed that all raw materials were consumed. The reaction mixture was added with 1.0 M dilute hydrochloric acid to adjust the pH to 4 and extracted with ethyl acetate (50 mL × 3). The organic phase was dried over anhydrous sodium sulfate, filtered, and then concentrated under reduced pressure to obtain the title product (95 mg, yellow solid).

¹H NMR (400 MHz, CDsOD) δ 7.77 (s, 4H), 7.63 (d, *J* = 9.2 Hz, 1H), 7.09 (d, *J* = 9.2 Hz, 1H), 6.89 (t, *J* = 55.6 Hz, 1H), 5.92 (s, 2H)

### Step 6: Synthesis of 5-(((6-chloropyridazin-3-yl)oxy)methyl)-1-(4-(difluoromethyl)phenyl)-1H-1,2,3-triazole-4-carboxamide (J7)

**J6** (95.0 mg, 0.25 mmol), HATU (142 mg, 0.37 mmol), and DIEA(96 mg, 0.75 mmol) were sequentially added to anhydrous DMF (2 mL) at room temperature. The system was replaced with nitrogen three times, and the reaction was carried out for 1 hour. Ammonia hydroxide (21.0 mg, 30%, 0.37 mmol) was then added thereto, and the reaction was carried out at room temperature for another 13 hours. LCMS showed that the reaction was completed. The reaction mixture was diluted with ethyl acetate (50 mL). The organic phase was sequentially washed with saturated citric acid aqueous solution (5 mL × 3), saturated sodium bicarbonate aqueous solution (5 mL × 3), and saturated brine (5 mL × 3), dried over anhydrous sodium sulfate, and filtered, and the filtrate was concentrated under reduced pressure to obtain the title product (200 mg, yellow oil).

MS (ESI) m/z [M+H]⁺ = 380.1.

¹H NMR (400 MHz, CDsOD) δ 7.76 (s, 4H), 7.62 (d, *J=* 9.2 Hz, 1H), 7.10-7.05 (m, 1H), 6.88 (t, *J* = 55.6 Hz, 1H), 5.94 (s, 2H)

### Step 7: Synthesis of 5-(((6-chloropyridazin-3-yl)oxy)methyl)-1-(4-(difluoromethyl)phenyl)-1H-1,2,3-triazole-4-carbonitrile (J8)

**J7** (95.0 mg, 0.25 mmol), triethylamine (63 mg, 0.62 mmol), and trifluoroacetic anhydride (176 mg, 0.62 mmol) were sequentially added to anhydrous dichloromethane (2 mL) at room temperature. The system was replaced with nitrogen three times, and the reaction was carried out for 15 hours. LCMS showed that the reaction was completed. The reaction mixture was diluted with dichloromethane (50 mL) and washed with saturated brine (10 mL × 3). The organic phase was dried over anhydrous sodium sulfate and filtered. The filtrate was concentrated under reduced pressure, and the crude product was purified by flash silica gel column chromatography (methanol: dichloromethane = 0:1 to 1:10) to obtain (40 mg, yellow oily liquid).

MS (ESI) m/z [M+H]⁺ = 362.8.

¹H NMR (400 MHz, CDsOD) δ 7.84 (s, 4H), 7.71 (d, *J* = 9.6 Hz, 1H), 7.28 (d, *J* = 9.2 Hz, 1H), 6.93 (t, *J=* 56.0 Hz, 1H), 5.81 (s, 2H)

### Synthesis of intermediate K3:

### 3-((4-(((tert-Butyldimethylsilyl)oxy)methyl)-1-(4-(difluoromethyl)phenyl)-1H-1,2,3-triazol-5-yl)methoxy)-6-chloropyridazine (K3)

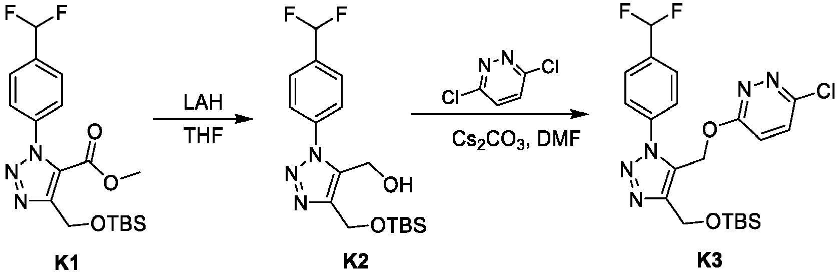

### Step 1: Synthesis of (4-(((tert-butyldimethylsilyl)oxy)methyl)-1-(4-(difluoromethyl)phenyl)-1H-1,2,3-triazol-5-yl)methanol (K2)

Compound **K1** (500 mg, 1.26 mmol) was dissolved in anhydrous tetrahydrofuran (3 mL) at room temperature, cooled to 0°C, then LiAlH₄ (48.0 mg, 1.26 mmol) was added thereto, and the system was replaced with nitrogen three times. The reaction mixture was stirred at 0°C for 10 minutes. TLC showed that the reaction was completed. Water (0.5 mL), sodium hydroxide solution (15%, 0.5 mL), and water (1.5 mL) were sequentially added to the mixture. Ethyl acetate (100 mL) was then added thereto to dilute. After the mixture was stirred at room temperature for 15 minutes, anhydrous sodium sulfate solid was added thereto, and the resulting mixture was stirred for another 15 minutes. The organic phase was filtered and concentrated under reduced pressure to obtain the title product (250 mg, yellow solid).

¹H NMR (400 MHz, CDsOD) δ 7.87 (d, *J* = 8.4 Hz, 2H), 7.80 (d, *J* = 8.4 Hz, 2H), 6.91 (t, *J=* 56.0 Hz, 1H), 4.94 (s, 2H), 4.72 (s, 2H), 0.94 (s, 9H), 0.18 (s, 6H)

### Step 3: Synthesis of 3-((4-(((tert-butyldimethylsilyl)oxy)methyl)-1-(4-(difluoromethyl)phenyl)-1H-1,2,3-triazol-5-yl)methoxy)-6-chloropyridazine (K3)

Compound **K2** (250 mg, 0.675 mmol) and 3,6-dichloropyridazine (143 mg, 1.012 mmol) were dissolved in anhydrous *N*,*N*-dimethylformamide (5 mL) at room temperature, then cesium carbonate (441 mg, 1.35 mmol) was added thereto, and the system was replaced with nitrogen three times. The reaction mixture was stirred at 19°C for 12 hours. TLC showed that the reaction was completed. The reaction mixture was diluted with water (15 mL) and extracted with ethyl acetate (30 mL × 3). The organic phase was dried over anhydrous sodium sulfate and then concentrated under reduced pressure to obtain the title product (100 mg, yellow solid).

MS (ESI) m/z [M+H]⁺ = 482.0.

¹H NMR (400 MHz, CDsOD) δ 7.79 (s, 4H), 7.66 (d, *J=* 9.2 Hz, 1H), 7.18 (d, *J=* 9.2 Hz, 1H), 6.89 (t, *J* = 55.6 Hz, 1H), 5.71 (s, 2H), 5.02 (s, 2H), 0.88 (s, 9H), 0.12 (s, 6H).

### Synthesis of intermediate L4:

### 3-Chloro-6-((4-methyl-1-(p-tolyl)-1H-1,2,3-triazol-5-yl)methoxy)pyridazine (L4)

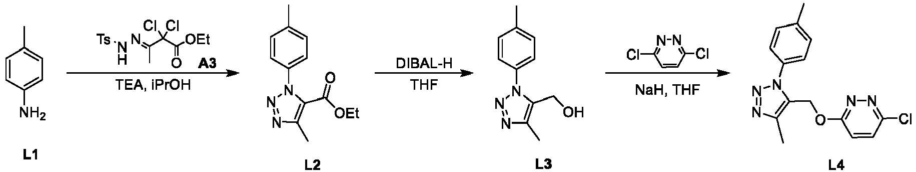

The synthesis method was as described in steps 4 to 6 in the synthesis of intermediate **A8,** using **L1** as the starting material to obtain the title product.

MS (ESI) m/z [M+H]⁺ = 301.1.

### Synthesis of intermediate M4:

### 3-Chloro-6-((1-(4-(difluoromethoxy)phenyl)-4-methyl-1H-1,2,3-triazol-5-yl)methoxy)pyridazine (M4)

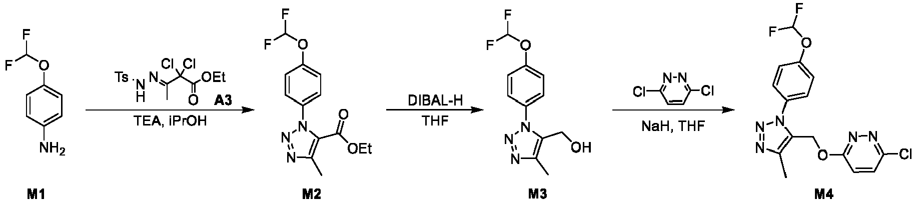

The synthesis method was as described in steps 4 to 6 in the synthesis of intermediate **A8,** using **M1** as the starting material to obtain the title product.

MS (ESI) m/z [M+H]⁺ = 337.1.

### Synthesis of intermediate N7:

### 3-Chloro-6-((4-(4-(difluoromethyl)phenyl)-1-methyl-1H-1,2,3-triazol-5-yl)methoxy)pyridazine (N7)

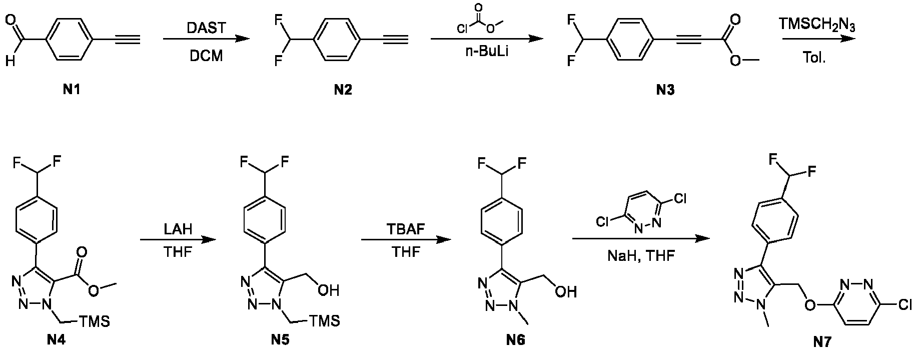

### Step 1: Synthesis of 1-(difluoromethyl)-4-ethynylbenzene (N2)

DAST (7.43 g, 46.1 mmol) was added dropwise to a solution of compound **N1** (4 g, 30.74 mmol) in dichloromethane (50 mL) at 0°C. The reaction mixture was stirred at 16°C for 12 hours. The reaction endpoint was monitored by TLC. The reaction mixture was quenched with saturated sodium bicarbonate aqueous solution (15 mL) and extracted with dichloromethane (50 mL × 3). The organic phase was washed with saturated brine (20 mL), dried over anhydrous sodium sulfate, filtered, and the filtrate was concentrated under reduced pressure to obtain the title product (4.5 g, yellow solid).

¹H NMR (400 MHz, CDCl₃) δ 7.59-7.56 (m, 2H), 7.51-7.46 (m, 2H), 6.65 (t, *J* = 56.0 Hz, 1H), 3.20 (s, 3H)

### Step 2: Synthesis of methyl 3-(4-(difluoromethyl)phenyl)propiolate (N3)

*n*-Butyllithium (5.8 mL, 14.5 mmol, 2.5 M) was slowly added dropwise to a solution of compound **N2** (1 g, 6.6 mmol) in tetrahydrofuran (20 mL) at -78°C, and the reaction mixture was stirred at -78°C for 0.5 hours. Methyl chloroformate (745 mg, 7.89 mmol) was slowly added dropwise thereto, and the reaction mixture was stirred at this temperature for another 2 hours. The reaction endpoint was monitored by TLC. Saturated ammonium chloride aqueous solution (20 mL) was slowly added dropwise thereto at -78°C to quench the reaction, and the reaction mixture was extracted with ethyl acetate (30 mL × 2). The organic phases were combined, washed with saturated brine (10 mL), dried over anhydrous sodium sulfate, filtered, and the filtrate was concentrated. The crude product was purified by silica gel column chromatography (petroleum ether: ethyl acetate = 1:0 to 95:5) to obtain the title product (500 mg, yellow solid).

¹H NMR (400 MHz, CDCl₃) δ7.68 (d, *J=* 8.4 Hz, 2H), 7.54 (d, *J=* 8.4 Hz, 2H), 6.67 (t, *J=* 56.0 Hz, 1H), 3.87 (s, 3H).

### Step 3: Synthesis of methyl 4-(4-(difluoromethyl)phenyl)-1-((trimethylsilyl)methyl)-1H-1,2,3-triazole-5-carboxylate (N4)

Trimethylsilylmethyl azide (3.44 g, 26.6 mmol) was added to a solution of compound **N3** (1.4 g, 6.66 mmol) in anhydrous toluene (30 mL) under a nitrogen atmosphere, and the reaction mixture was heated to 100°C and stirred for 12 hours. Product generation was monitored by LCMS. The reaction mixture was concentrated under reduced pressure, and the residue was purified by high performance liquid chromatography (chromatographic column: Xtimate C18 150 * 40 mm * 5 µm; mobile phase: water (trifluoroacetic acid) - acetonitrile; gradient: 45% to 75%; flow rate: 60 mL/min) to obtain the title product (660 mg, white solid).

MS (ESI) m/z [M+H]⁺ = 340.1.

¹H NMR (400 MHz, DMSO-*d*₆) δ7.83 (d, *J=* 8.4 Hz, 2H), 7.66 (d, *J=* 8.4 Hz, 2H), 7.10 (t, *J=* 56.0 Hz, 1H), 4.33 (s, 2H), 3.83 (s, 3H), 0.11 (s, 9H)

### Step 4: Synthesis of (4-(4-(difluoromethyl)phenyl)-1-((trimethylsilyl)methyl)-1H-1,2,3-triazol-5-yl)methanol (N5)

Lithium aluminum hydride (106 mg, 2.8 mmol) was added to a solution of compound **N4** (660 mg, 1.87 mmol) in tetrahydrofuran (10 mL) at 0°C in batches. The reaction mixture was stirred at 0°C for 1 hour. The reaction endpoint was monitored by TLC. The reaction mixture was quenched by sequentially adding water (2 mL), sodium hydroxide aqueous solution (2 mL, 15%), and water (6 mL). The reaction mixture was extracted with ethyl acetate (10 mL × 3). The organic phases were combined, dried over anhydrous sodium sulfate, filtered, and the filtrate was concentrated under reduced pressure to obtain the title compound (550 mg, yellow solid).

¹H NMR (400 MHz, CDsOD) δ 7.84 (d, *J* = 8.4 Hz, 2H), 7.66 (d, *J* = 8.4 Hz, 2H), 6.82 (t, *J=* 56.0 Hz, 1H), 4.77 (s, 2H), 3.99 (s, 2H), 0.23 (s, 9H)

### Step 5: Synthesis of (4-(4-(difluoromethyl)phenyl)-1-methyl-1H-1,2,3-triazol-5-yl)methanol (N6)

Tetrabutylammonium fluoride (791 mg, 5.3 mmol) was added to a solution of compound **N5** (550 mg, 1.77 mmol) in tetrahydrofuran (10 mL), and stirred at 22°C for 3 hours. The reaction endpoint was monitored by LCMS. The reaction mixture was concentrated under reduced pressure, and the residue was purified by preparative thin-layer chromatography (dichloromethane: methanol = 10:1) to obtain the title product (340 mg, white solid).

MS (ESI) m/z [M+H]⁺ = 240.0.

¹H NMR (400 MHz, CDsOD) δ7.84 (d, *J=* 8.4 Hz, 2H), 7.66 (d, *J=* 8.4 Hz, 2H), 6.83 (t, *J=* 56.0 Hz, 1H), 4.81 (s, 2H), 4.18 (s, 3H)

### Step 6: Synthesis of 3-chloro-6-((4-(4-(difluoromethyl)phenyl)-1-methyl-1H-1,2,3-triazol-5-yl)methoxy)pyridazine (N7)

Compound **N6** (100 mg, 0.28 mmol) was dissolved in tetrahydrofuran (10 mL) at 0°C. Sodium hydride (50 mg, 1.25 mmol, content: 60%) was added thereto, then the reaction mixture was stirred for half an hour, and 3,6-dichloropyridazine (187 mg, 1.25 mmol) in tetrahydrofuran (5 mL) was slowly added dropwise thereto. The reaction mixture was warmed to 19°C, and stirred for another 2 hours. The reaction endpoint was monitored by LCMS. The reaction was quenched by adding water (2 mL), and the reaction mixture was extracted with ethyl acetate (10 mL × 3). The organic phases were combined, dried over sodium sulfate, filtered, and the filtrate was concentrated under reduced pressure. The residue was purified by preparative thin-layer chromatography (dichloromethane: methanol = 10: 1) to obtain the title product (230 mg, yellow solid).

MS (ESI) m/z [M+H]⁺ = 353.0.

¹H NMR (400 MHz, CDsOD) δ 7.88 (d, *J=* 8.0 Hz, 2H), 7.70-7.64 (m, 3H), 7.28 (d, *J=* 9.2 Hz, 1 H), 6.81 (t, *J=* 56.0 Hz, 1H), 5.80 (s, 2 H), 4.28 (s, 3 H)

### Synthesis of intermediate P6:

### 3-Chloro-6-((4-(4-fluorophenyl)-1-methyl-1H-1,2,3-triazol-5-yl)methoxy)pyridazine (P6)

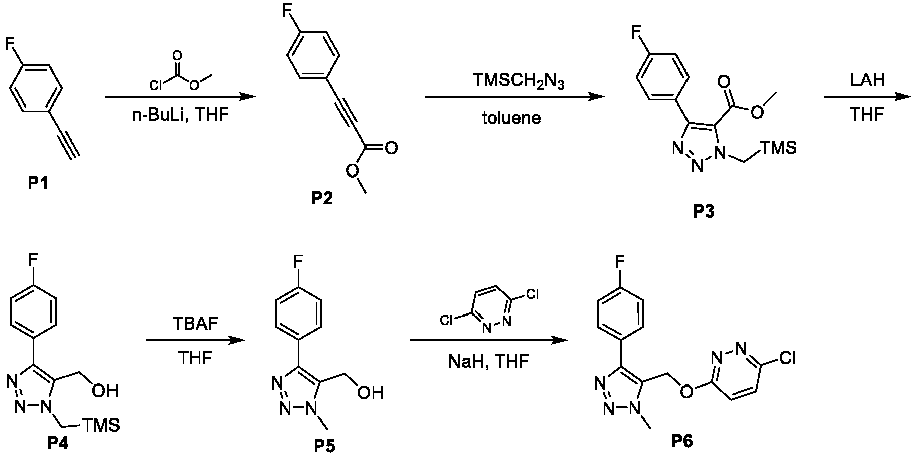

The synthesis method was as described in steps 2 to 6 in the synthesis of intermediate **N7,** using **P1** as the starting material to obtain the title product.

MS (ESI) m/z [M+H]⁺ = 320.1.

### Synthesis of intermediate Q3:

### 3-Chloro-6-((4-chloro-1-(4-(difluoromethyl)phenyl)-1H-1,2,3-triazol-5-yl)methoxy)pyridazine (Q3)

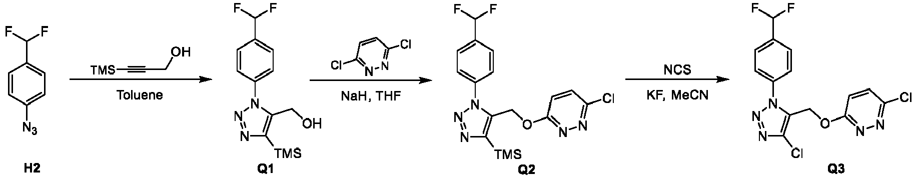

### Step 1: Synthesis of (1-(4-(difluoromethyl)phenyl)-4-(trimethylsilyl)-1H-1,2,3-triazol-5-yl)methanol (Q1)

The experimental operation was as described in step 2 in the synthesis of intermediate **H5,** using intermediate **H2** and 3-(trimethylsilyl)prop-2-yn-1-ol as reactants to obtain the title product.

MS (ESI) m/z [M+H]⁺ = 298.1.

¹H NMR (400 MHz, CDCl₃) δ 7.80-7.68 (m, 4H), 6.73 (t, *J=* 56.0 Hz, 1H), 4.73 (d, *J* = 4.4 Hz, 2H), 2.10-2.04 (m, 1H), 0.43 (s, 9H).

### Step 2: Synthesis of 3-chloro-6-((1-(4-(difluoromethyl)phenyl)-4-(trimethylsilyl)-1H-1,2,3-triazol-5-yl)methoxy)pyridazine (Q2)

The experimental operation was as described in step 3 in the synthesis of intermediate **H5,** using intermediate **Q1** and 3,6-dichloropyridazine as reactants to obtain the title product.

MS (ESI) m/z [M+H]⁺ = 410.1.

¹H NMR (400 MHz, CDCl₃) δ 7.68-7.63 (m, 4H), 7.44 (d, *J* = 9.2 Hz, 1H), 6.97 (d, *J* = 9.2 Hz, 1H), 6.71 (t, *J=* 56.0 Hz, 1H), 5.55 (s, 2H), 0.40 (s, 9H).

### Step 3: Synthesis of 3-chloro-6-((4-chloro-1-(4-(difluoromethyl)phenyl)-1H-1,2,3-triazol-5-yl)methoxy)pyridazine (Q3)

Compound **Q2** (40.0 mg, 0.098 mmol) was dissolved in acetonitrile (2.0 mL) at room temperature, then potassium fluoride (34.8 mg, 0.58 mmol) and *N*-chlorosuccinimide (130 mg, 0.97 mmol) were sequentially added thereto, and the reaction was heated to 80°C and stirred for 16 hours. The reaction endpoint was monitored by TLC. The reaction mixture was filtered, and the filter cake was washed with acetonitrile (2.0 mL). The filtrates were combined, and the solvent was removed under reduced pressure. The residue was purified by preparative thin-layer chromatography (petroleum ether: ethyl acetate = 4:1) to obtain the title product (100 mg, white solid).

MS (ESI) m/z [M+H]⁺ = 372.0.

¹H NMR (400 MHz, CDCl₃) δ 7.70 (s, 4H), 7.44 (d, *J=* 9.2 Hz, 1H), 6.99 (d, *J=* 9.2 Hz, 1H), 6.72 (t, *J=* 56.0 Hz, 1H), 5.58 (s, 2H).

### Example 1:

### 3-((1-(4-(Difluoromethyl)phenyl)-4-methyl-1H-1,2,3-triazol-5-yl)methoxy)-6-(3-methoxypyrrolidin-1-yl)pyridazine (1)

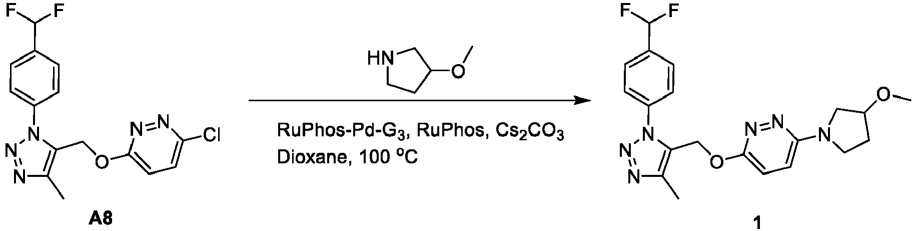

**A8** (30 mg, 0.085 mmol) and 3-methoxypyrrolidine (8.6 mg, 0.085 mmol) were dissolved in dioxane (3.0 mL) under an argon atmosphere, then 2-dicyclohexylphosphino-2',6'-di-i-propoxy-1,1'-biphenyl (4 mg, 0.0085 mmol), cesium carbonate (28 mg, 0.085 mmol), and methanesulfonato(2-dicyclohexylphosphino-2',6'-di-*i*-propoxy-1,1'-biphenyl)(2'-amino-1,1'-biphenyl-2-yl)palladium(II) (7 mg, 0.0085 mmol) were sequentially added thereto, and the reaction mixture was stirred at 100°C for 16 hours. The completion of the reaction was monitored by LCMS. The reaction mixture was diluted with dichloromethane (50 mL) and filtered. The filtrate was concentrated under reduced pressure. The residue was purified by flash silica gel column chromatography.

MS (ESI) m/z [M+H]⁺ = 417.2.

¹H NMR (400 MHz, DMSO-*d*₆) δ 7.79 (s, 4H), 7.14 (t, *J=* 55.6 Hz, 1H), 7.04 - 6.93 (m, 2H), 5.43 (s, 2H), 4.08-4.05 (m, 1H), 3.52-3.43 (m, 3H), 3.38-3.35 (m, 1H), 3.25 (s, 3H), 2.40 (s, 3H), 2.04-2.03 (m, 2H).

### Example 2:

### 4-(6-((1-(4-(Difluoromethyl)phenyl)-4-methyl-1H-1,2,3-triazol-5-yl)methoxy)pyridazin-3-yl)piperazin-2-one (2)

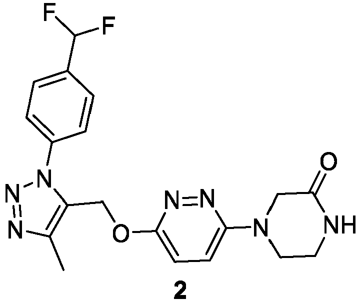

The experimental operation was as described in Example 1, using **A8** and 2-piperazinone as reactants to obtain the title product.

MS (ESI) m/z [M+H]⁺ = 416.2.

¹H NMR (400 MHz, DMSO-*d*₆) δ 8.08 (s, 1H), 7.78 (s, 4H), 7.40 (d, *J* = 9.6 Hz, 1H), 7.14 (t, *J* = 55.6 Hz, 1H), 7.04 (d, *J* = 9.6 Hz, 1H), 5.45 (s, 2H), 3.97 (s, 2H), 3.66 (t, *J* = 5.2 Hz, 2H), 3.27 (t, *J* = 5.2 Hz, 2H), 2.39 (s, 3H).

### Example 3:

### 1-(6-((1-(4-(Difluoromethyl)phenyl)-4-methyl-1H-1,2,3-triazol-5-yl)methoxy)pyridazin-3-yl)-1,4-diazepan-5-one (3)

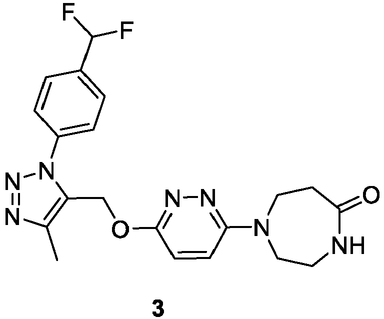

The experimental operation was as described in Example 1, using **A8** and 1,4-diazepan-5-one hydrochloride as reactants to obtain the title product.

MS (ESI) m/z [M+H]⁺ = 430.2.

¹H NMR (400 MHz, DMSO-*d*₆) δ 7.79 (s, 4H), 7.61 (br s, 1H), 7.37 (d, *J* = 9.8 Hz, 1H), 7.14 (t, *J=* 55.6 Hz, 1H), 7.00 (d, *J=* 9.8 Hz, 1H), 5.44 (s, 2H), 3.73-3.69 (m, 4H), 3.15-3.12 (m, 2H), 2.46-2.42 (m, 2H), 2.39 (s, 3H).

### Example 4:

### 4-(6-((1-(4-(Difluoromethyl)phenyl)-4-methyl-1H-1,2,3-triazol-5-yl)methoxy)pyridazin-3-yl)-1,4-diazepan-2-one (4)

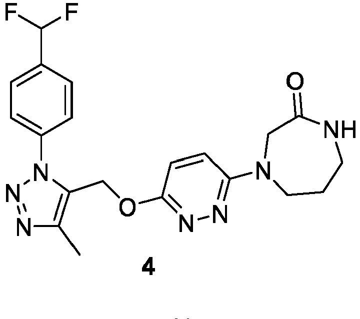

The experimental operation was as described in Example 1, using **A8** and 1,4-diazepan-2-one hydrochloride as reactants to obtain the title product.

MS (ESI) m/z [M+H]⁺ = 430.2.

¹H NMR (400 MHz, DMSO-*d*₆) δ 7.78 (s, 4H), 7.45 (br s, 1H), 7.23 (d, *J* = 9.6 Hz, 1H), 7.12 (t, *J* =55.6 Hz, 1H), 7.00 (d, *J* = 9.6 Hz, 1H), 5.41 (s, 2H), 4.15 (s, 2H), 3.87-3.85 (m, 2H), 3.20-3.17 (m, 2H), 2.38 (s, 3H), 1.67-1.65 (m, 2H).

### Example 5:

### 4-(6-((1-(4-(Difluoromethyl)phenyl)-4-methyl-1H-1,2,3-triazol-5-yl)methoxy)pyridazin-3-yl)piperazine-2,6-dione (5)

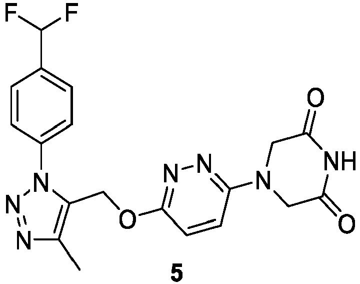

The experimental operation was as described in Example 1, using **A8** and piperazine-2,6-dione as reactants to obtain the title product.

MS (ESI) m/z [M+H]⁺ = 430.1.

¹H NMR (400 MHz, DMSO-*d*₆) δ 11.23 (s, 1H), 7.77 (s, 4H), 7.62 (d, *J* = 9.6 Hz, 1H), 7.12 (t, *J* = 55.6 Hz, 1H), 7.11 (d, *J* = 9.6 Hz, 1H), 5.45 (s, 2H), 4.32 (s, 4H), 2.38 (s, 3H).

### Example 6:

### 4-(6-((1-(4-(Difluoromethyl)phenyl)-4-methyl-1H-1,2,3-triazol-5-yl)methoxy)pyridazin-3-yl)-6-methylpiperazin-2-one (6)

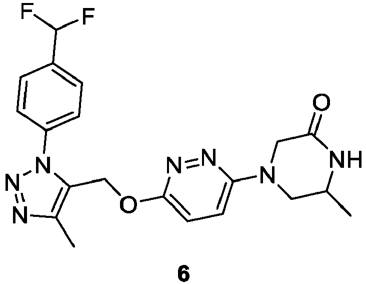

The experimental operation was as described in Example 1, using **A8** and compound 6-methylpiperazin-2-one as reactants to obtain the title product.

MS (ESI) m/z [M+H]⁺= 430.1.

¹H NMR (400 MHz, CDsOD) *δ* 7.76 (s, 4H), 7.35 (d, *J* = 9.6 Hz, 1H), 7.00 (d, *J* = 9.6 Hz, 1H), 6.88 (t, *J* =55.6 Hz, 1H), 5.50 (s, 2H), 4.28-4.18 (m, 1H), 4.04-3.93 (m, 2H), 3.76-3.65 (m, 1H), 3.28-3.20 (m, 1H), 2.48 (s, 3H), 1.26 (d, *J=* 6.4 Hz, 3H)_{∘}

### Example 7:

### 4-(6-((1-(4-(Difluoromethyl)phenyl)-4-methyl-1H-1,2,3-triazol-5-yl)methoxy)pyridazin-3-yl)-6,6-dimethylpiperazin-2-one (7)

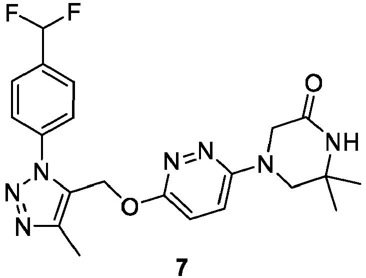

The experimental operation was as described in Example 1, using **A8** and 6,6-dimethylpiperazin-2-one as reactants to obtain the title product.

MS (ESI) m/z [M+H]⁺= 443.9.

¹H NMR (400 MHz, CDsOD) *δ* 7.77 (s, 4H), 7.35 (d, *J* = 9.6 Hz, 1H), 7.00 (d, *J* = 9.6 Hz, 1H), 6.88 (t, *J* = 55.6 Hz, 1H), 5.50 (s, 2H), 4.08 (s, 2H), 3.58 (s, 2H), 2.48 (s, 3H), 1.32 (s, 6H).

### Example 8:

### 7-(6-((1-(4-(Difluoromethyl)phenyl)-4-methyl-1H-1,2,3-triazol-5-yl)methoxy)pyridazin-3-yl)-4,7-diazaspiro[2.5]octan-5-one (8)

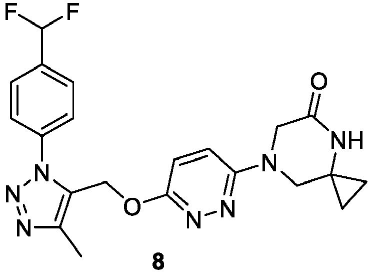

The experimental operation was as described in Example 1, using **A8** and 4,7-diazaspiro[2.5]octan-5-one as reactants to obtain the title product.

MS (ESI) m/z [M+H]⁺= 442.1.

¹H NMR (400 MHz, CDsOD) *δ* 7.77 (s, 4H), 7.31 (d, *J* = 9.6 Hz, 1H), 6.72-7.06 (m, 2H), 5.50 (s, 2H), 4.22 (s, 2H), 3.64 (s, 2H), 2.48 (s, 3H), 0.90-0.82 (m, 4H).

### Example 9:

### (S)-8-(6-((1-(4-(Difluoromethyl)phenyl)-4-methyl-1H-1,2,3-triazol-5-yl)methoxy)pyridazin-3-yl)hexahydropyrazino[2,1-c][1,4]oxazin-4(3H)-one (9)

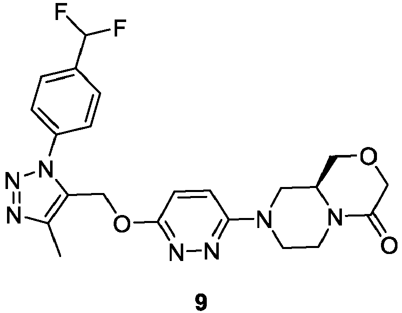

The experimental operation was as described in Example 1, using **A8** and (S)-hexahydropyrazino[2,1-*c*][1,4]oxazin-4(3*H*)-one as reactants to obtain the title product.

MS (ESI) m/z [M+H]⁺ = 472.2.

¹H NMR (400MHz, DMSO-*d*₆) δ 7.78 (s, 4H), 7.43(d, *J* = 9.4 Hz, 1H), 7.13 (t, *J* = 55.6 Hz, 1H), 7.03 (d, *J* = 9.4 Hz, 1H), 5.46 (s, 2H), 4.41-4.38 (m, 1H), 4.19-4.15 (m, 2H), 4.05-4.02 (m, 3H), 3.61-3.58 (m, 1H), 2.80-2.77 (m, 2H), 2.69-2.66 (m, 2H), 2.39 (s, 3H).

### Example 10:

### 1-(6-((1-(4-(Difluoromethyl)phenyl)-4-methyl-1H-1,2,3-triazol-5-yl)methoxy)pyridazin-3-yl)-3,3-dimethylpiperazin-2-one (10)

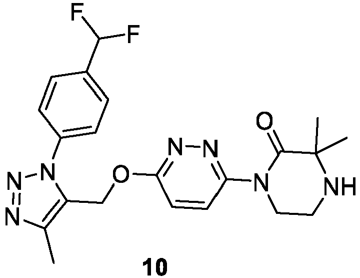

The experimental operation was as described in Example 1, using **A8** and 3,3-dimethylpiperazin-2-one as reactants to obtain the title product.

MS (ESI) m/z [M+H]⁺ = 444.2.

¹H NMR (400MHz, DMSO-*d*₆) δ 7.80-7.78 (m, 5H), 7.17 (d, *J* = 9.4 Hz, 1H), 7.13 (t, *J* = 55.6 Hz, 1H), 5.57 (s, 2H), 3.85 (t, *J* = 5.2 Hz, 2H), 3.05 (t, *J* = 5.2 Hz, 2H), 2.41 (s, 3H), 1.30 (s, 6H).

### Example 11:

### 4-(6-((1-(4-(Difluoromethyl)phenyl)-4-methyl-1H-1,2,3-triazol-5-yl)methoxy)pyridazin-3-yl)-3-methylpiperazin-2-one (11)

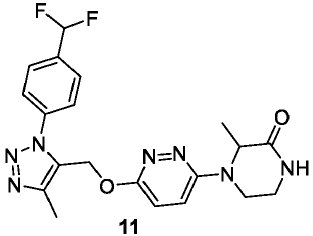

The experimental operation was as described in Example 1, using **A8** and 3-methylpiperazin-2-one as reactants to obtain the title product.

MS (ESI) m/z [M+H]⁺ = 430.2.

¹H NMR (400 MHz, DMSO-*d*₆) δ 7.99 (s, 1H), 7.78 (s, 4H), 7.35 (d, *J* = 9.4 Hz, 1H), 7.13 (t, *J=* 55.6 Hz, 1H), 7.00 (d, *J* = 9.4 Hz, 1H), 5.44 (s, 2H), 4.65-4.62 (m, 1H), 4.12-4.09 (m, 1H), 3.27-3.22 (m, 3H), 2.39 (s, 3H), 1.28 (d, *J=* 6.8 Hz, 3H).

### Example 12:

### 1-(6-((1-(4-(Difluoromethyl)phenyl)-4-methyl-1H-1,2,3-triazol-5-yl)methoxy)pyridazin-3-yl)-N,N-dimethylpyrrolidin-3-amine (12)

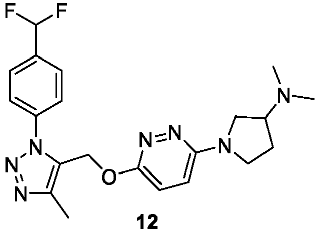

The experimental operation was as described in Example 1, using **A8** and *N,N-*dimethylpyrrolidin-3-amine hydrochloride as reactants to obtain the title product.

MS (ESI) m/z [M+H]⁺ = 430.1.

¹H NMR (400 MHz, DMSO-*d*₆) δ 7.79 (s, 4H), 7.14 (t, *J* = 55.6 Hz, 1H), 6.99 (d, *J* = 9.4 Hz, 1H), 6.95 (d, *J=* 9.4 Hz, 1H), 5.43 (s, 2H), 3.66-3.62 (m, 1H), 3.57-3.53 (m, 1H), 3.33-3.31 (m, 1H), 3.15-3.10 (m, 1H), 2.82-2.80 (m, 1H), 2.39 (s, 3H), 2.21 (s, 6H), 2.17-2.14 (m, 1H), 1.84-1.74 (m, 1H).

### Example 13:

### (S)-8-(6-((1-(4-(Difluoromethyl)phenyl)-4-methyl-1H-1,2,3-triazol-5-yl)methoxy)pyridazin-3-yl)octahydropyrazino[2,1-c][1,4]oxazine (13)

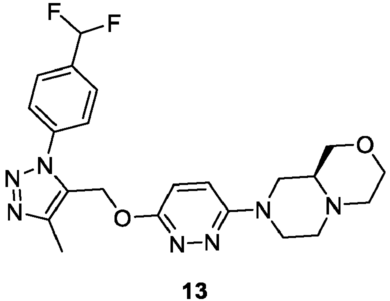

The experimental operation was as described in Example 1, using **A8** and (*S*)-octahydropyrazino[2,1-*c*][1,4]oxazine hydrochloride as reactants to obtain the title product.

MS (ESI) m/z [M+H]⁺ = 458.2.

¹H NMR (400 MHz, DMSO-*d*₆) δ 7.78 (s, 4H), 7.37 (d, *J* = 9.4 Hz, 1H), 7.13 (t, *J* = 55.6 Hz, 1H), 6.99 (d, *J* = 9.4 Hz, 1H), 5.44 (s, 2H), 4.06-3.92 (m, 2H), 3.76-3.69 (m, 2H), 3.54-3.51 (m, 2H), 3.16-3.11 (m, 1H), 2.90-2.84 (m, 1H), 2.79-2.76 (m, 1H), 2.66-2.63 (m, 1H), 2.39 (s, 3H), 2.22-2.14 (m, 3H).

### Example 14:

### (S)-2-(6-((1-(4-(difluoromethyl)phenyl)-4-methyl-1H-1,2,3-triazol-5-yl)methoxy)pyridazin-3-yl)hexahydropyrrolo[1,2-a]pyrazin-4(1H)-one (14)

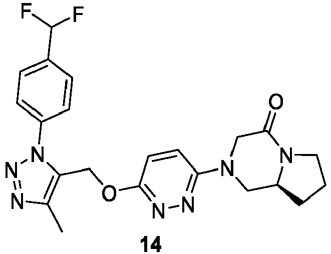

The experimental operation was as described in Example 1, using **A8** and (*S*)-hexahydropyrrolo[1,2-*a*]pyrazin-4(1*H*)-one hydrochloride as reactants to obtain the title product.

MS (ESI) m/z [M+H]⁺ = 456.2.

¹H NMR (400 MHz, DMSO-*d*₆) δ 7.78 (s, 4H), 7.37 (d, *J* = 9.4 Hz, 1H), 7.13 (t, *J* = 55.6 Hz, 1H), 7.00 (d, *J* = 9.4 Hz, 1H), 5.44 (s, 2H), 4.56-4.53 (m, 1H), 4.36-4.31 (m, 1H), 3.66-3.62 (m, 2H), 3.45-3.42 (m, 2H), 2.81-2.76 (m, 1H), 2.39 (s, 3H), 2.08-2.06 (m, 1H), 1.92-1.90 (m, 1H), 1.54-1.51 (m, 1H), 1.50-1.46 (m, 1H).

### Example 15:

### 3-((1-(4-(Difluoromethyl)phenyl)-4-methyl-1H-1,2,3-triazol-5-yl)methoxy)-6-(4-methylpiperazin-1-yl)pyridazine (15)

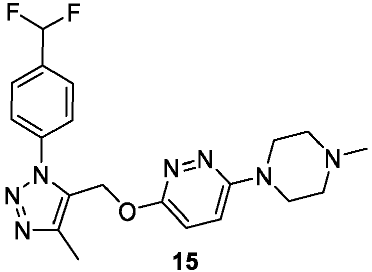

The experimental operation was as described in Example 1, using **A8** and 1-methylpiperazine as reactants to obtain the title product.

MS (ESI) m/z [M+H]⁺ = 416.2.

¹H NMR (400 MHz, CDCl₃) δ 7.75-7.55 (m, 4H), 7.08 (d, *J=* 9.4 Hz, 1H), 6.86 (d, *J* = 9.4 Hz, 1H), 6.62 (t, *J=* 55.6 Hz, 1H), 5.46 (s, 2H), 3.64 (br. s, 4H), 2.65 (br. s, 4H), 2.49 (s, 3H), 2.43 (s, 3H).

### Example 16:

### 4-(6-((1-(4-(Difluoromethyl)phenyl)-4-methyl-1H-1,2,3-triazol-5-yl)methoxy)pyridazin-3-yl)morpholine (16)

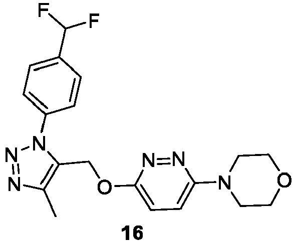

The experimental operation was as described in Example 1, using **A8** and morpholine as reactants to obtain the title product.

MS (ESI) m/z [M+H]⁺ = 403.1.

¹H NMR (400 MHz, CDCl₃) δ 7.72-7.65 (m, 4H), 7.04 (d, *J* = 9.4 Hz, 1H), 6.88 (d, *J* = 9.4 Hz, 1H), 6.72 (t, *J* = 55.6 Hz, 1H), 5.47 (s, 2H), 3.87-3.85 (m, 4H), 3.53-3.50 (m, 4H), 2.50 (s, 3H).

### Example 17:

### 4-(6-((1-(4-(Difluoromethyl)phenyl)-4-methyl-1H-1,2,3-triazol-5-yl)methoxy)pyridin-3-yl)-1-methyl-1,4-diazepan-2-one (17)

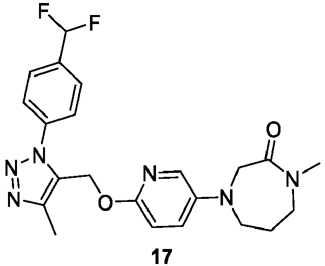

The experimental operation was as described in Example 1, using **A8** and 1-methyl-1,4-diazepan-2-one hydrochloride as reactants to obtain the title product.

MS (ESI) m/z [M+H]⁺ = 443.2.

¹H NMR (400 MHz, DMSO-*d*₆) δ 7.79 (s, 4H), 7.40 (d, *J=* 9.4 Hz, 1H), 7.17 (t, *J=* 55.6 Hz, 1H), 7.01 (d, *J* = 9.4 Hz, 1H), 5.45 (s, 2H), 3.74-3.68 (m, 4H), 3.49-3.46 (m, 2H), 2.85 (s, 3H), 2.60-2.54 (m, 2H), 2.39 (s, 3H).

### Example 18:

### 1-(6-((1-(4-(Difluoromethyl)phenyl)-4-methyl-1H-1,2,3-triazol-5-yl)methoxy)pyridazin-3-yl)azetidin-2-one (18)

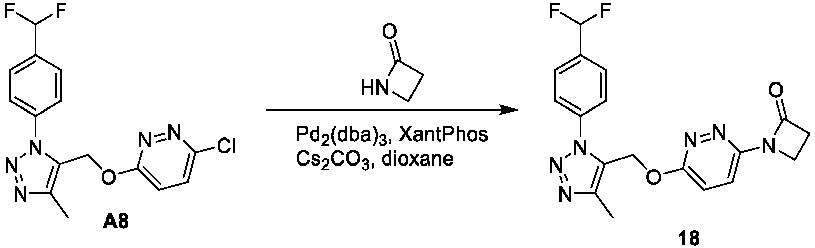

**A8** (42.2 mg, 0.12 mmol) and 2-azetidinone (12.8 mg, 0.18 mmol) were dissolved in toluene (2 mL) under an argon atmosphere, then 4,5-bis(diphenylphosphino)-9,9-dimethylxanthene (6.94 mg, 0.012 mmol), cesium carbonate (78.20 mg, 0.24 mmol), and tris(dibenzylideneacetone)dipalladium (10.99 mg, 0.012 mmol) were sequentially added thereto, and the reaction mixture was stirred at 100°C for 16 hours. The completion of the reaction was monitored by TLC. The reaction mixture was diluted with dichloromethane (50 mL) and filtered. The filtrate was concentrated under reduced pressure. The residue was purified by flash silica gel column chromatography (petroleum ether: ethyl acetate = 0:1) to obtain the title product (25 mg, white solid).

MS (ESI) m/z [M+H]⁺ = 387.1.

¹H NMR (400 MHz, DMSO-*d*₆) δ 7.84 (d, *J=* 9.4 Hz, 1H), 7.79 (s, 4H), 7.24 (d, *J=* 9.4 Hz, 1H), 7.14 (t, *J=* 55.6 Hz, 1H), 5.55 (s, 2H), 3.75 (t, *J=* 4.6 Hz, 2H), 3.17 (t, *J=* 4.6 Hz, 2H), 2.41 (s, 3H).

### Example 19:

### 1-(6-((1-(4-(Difluoromethyl)phenyl)-4-methyl-1H-1,2,3-triazol-5-yl)methoxy)pyridazin-3-yl)imidazolidin-2-one (19)

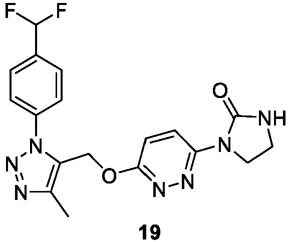

The experimental operation was as described in Example **18,** using **A8** and 2-imidazolidinone as reactants to obtain the title product.

MS (ESI) m/z [M+H]⁺ = 402.1.

¹H NMR (400 MHz, DMSO-*d*₆) δ 8.41 (d, *J* = 9.2 Hz, 1H), 7.77 (s, 4H), 7.38 (s, 1H), 7.13 (d, *J=* 9.2 Hz, 1H), 7.11 (t, *J=* 56.0 Hz, 1H), 5.51 (s, 2H), 3.98 (t, *J=* 8.4 Hz, 2H), 3.41 (t, *J=* 8.4 Hz, 2H), 2.39 (s, 3H).

### Example 20:

### 1-(6-((1-(4-(Difluoromethyl)phenyl)-4-methyl-1H-1,2,3-triazol-5-yl)methoxy)pyridazin-3-yl)pyrrolidin-2-one (20)

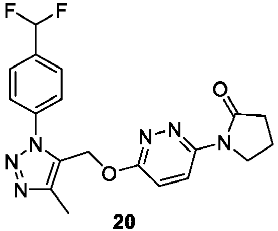

The experimental operation was as described in Example **18,** using **A8** and pyrrolidin-2-one as reactants to obtain the title product.

MS (ESI) m/z [M+H]⁺ = 401.1.

¹H NMR (400 MHz, DMSO-*d*₆) δ 8.46 (d, *J=* 9.6 Hz, 1H), 7.77 (s, 4H), 7.22 (d, *J* = 9.6 Hz, 1H), 7.12 (t, *J=* 55.6 Hz,1H), 5.55 (s, 2H), 3.98 (t, *J=* 7.2 Hz, 2H), 2.55 (t, *J=* 8.0 Hz, 2H), 2.40 (s, 3H), 2.10-1.99 (m, 2H).

### Example 21:

### 4-(6-[(1-(4-(Difluoromethyl)phenyl)-4-methyl-1H-1,2,3-triazol-5-yl)methoxy]pyridin-3-yl)piperazin-2-one (21)

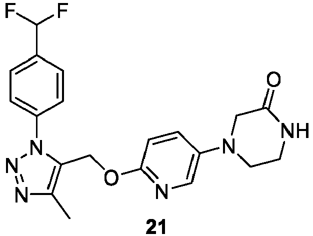

The experimental operation was as described in Example **18,** using **A10** and 2-piperazinone as reactants to obtain the title product.

MS (ESI) m/z [M+H]⁺ = 415.2.

¹H NMR (400 MHz, DMSO-*d*₆) δ 8.02 (s, 1H), 7.79 (s, 4H), 7.71 (d, *J* = 2.6 Hz, 1H), 7.46 (dd, *J=* 8.8, 2.6 Hz, 1H), 7.15 (t, *J=* 55.6 Hz, 1H), 6.70 (d, *J=* 8.8 Hz, 1H), 5.33 (s, 2H), 3.63 (s, 2H), 3.28 (br. s, 4H), 2.38 (s, 3H).

### Example 22:

### 4-(6-((1-(4-(Difluoromethyl)phenyl)-4-methyl-1H-1,2,3-triazol-5-yl)methoxy)pyridin-3-yl)-1-methylpiperazin-2-one (22)

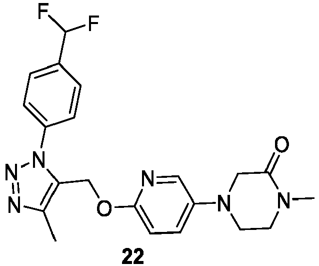

The experimental operation was as described in Example **18,** using **A10** and 1-methyl-2-piperazinone as reactants to obtain the title product.

MS (ESI) m/z [M+H]⁺ = 429.2.

¹H NMR (400 MHz, CDCl₃) δ 7.75-7.65 (m, 5H), 7.30-7.27 (m, 1H), 6.85-6.58 (m, 2H), 5.31 (s, 2H), 3.78 (s, 2H), 3.48-3.46 (m, 2H), 3.40-3.38 (m, 2H), 3.04 (s, 3H), 2.49 (s, 3H).

### Example 23:

### 3-(6-((1-(4-(Difluoromethyl)phenyl)-4-methyl-1H-1,2,3-triazol-5-yl)methoxy)pyridazin-3-yl)oxazolidin-2-one (23)

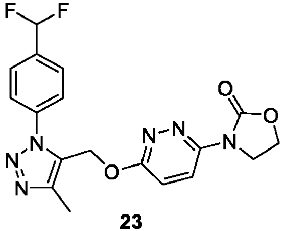

The experimental operation was as described in Example **18,** using **A8** and oxazolidin-2-one as reactants to obtain the title product.

MS (ESI) m/z [M+H]⁺ = 403.1.

¹H NMR (400 MHz, CDCl₃) δ 8.54 (d, *J* = 9.4 Hz, 1H), 7.70-7.67 (m, 4H), 7.06 (d, *J* = 9.4 Hz, 1H), 6.70 (t, *J* = 55.6 Hz, 1H), 5.53 (s, 2H), 4.59-4.56 (m, 2H), 4.39-4.37 (m, 2H), 2.51 (s, 3H).

### Example 24:

### 1-(6-((1-(4-(Difluoromethyl)phenyl)-4-methyl-1H-1,2,3-triazol-5-yl)methoxy)pyridazin-3-yl)-3-methylimidazolidin-2-one (24)

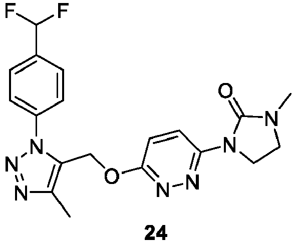

The experimental operation was as described in Example **18,** using **A8** and 1-methylimidazolidin-2-one as reactants to obtain the title product.

MS (ESI) m/z [M+H]⁺ = 416.1.

¹H NMR (400 MHz, CDCl₃) δ 8.63 (d, *J* = 9.4 Hz, 1H), 7.68-7.66 (m, 4H), 6.96 (d, *J* = 9.4 Hz, 1H), 6.70 (t, *J* = 55.6 Hz, 1H), 5.48 (s, 2H), 4.11 (br. s, 2H), 3.54 (br. s, 2H), 2.93(s, 3H), 2.49 (s, 3H).

### Example 25:

### 3-(6-((1-(4-(Difluoromethyl)phenyl)-4-methyl-1H-1,2,3-triazol-5-yl)methoxy)pyridazin-3-yl)-5,5-dimethylimidazolidine-2,4-dione (25)

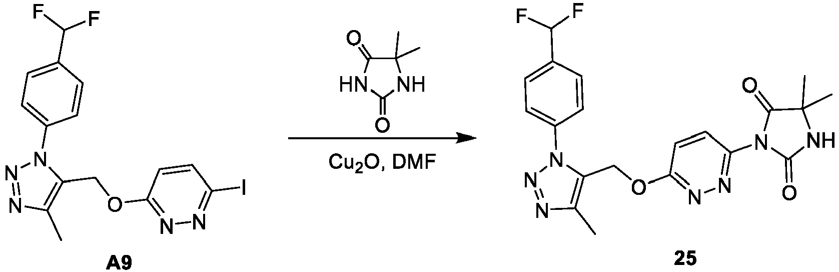

Compound **A9** (350 mg, 0.79 mmol), 5,5-dimethylimidazolidine-2,4-dione (350.0 mg, 0.79 mmol), and cuprous oxide (112 mg, 0.79 mmol) were sequentially added to anhydrous *N*,*N*-dimethylformamide (10.0 mL), and the system was replaced with nitrogen three times. The reaction mixture was reacted at 180°C for 2.5 hours. Product generation was shown by LCMS. The reaction mixture was cooled to room temperature and diluted with ethyl acetate (150 mL). The organic phase was washed with saturated brine (30 mL × 3), dried over anhydrous sodium sulfate, and then concentrated under reduced pressure. The crude product was purified by high performance liquid chromatography (separation conditions: chromatographic column: Phenomenex C18 75 * 30 mm * 3 µm; mobile phase: water (0.225% trifluoroacetic acid solution) - acetonitrile; gradient: B%: 30% to 50%; flow rate: 30 mL/min) to obtain the title product (63.0 mg, white solid).

MS (ESI) m/z [M+H]⁺= 444.2.

¹H NMR (400 MHz, CDsOD) δ 7.77 (s, 4H), 7.71 (d, *J* = 9.2 Hz, 1H), 7.31 (d, *J* = 9.2 Hz, 1H), 6.88 (t, *J* = 56.0 Hz, 1H), 5.70 (s, 2H), 2.51 (s, 3H), 1.53 (s, 6H).

### Example 26:

### 4-(6-((1-(4-(Difluoromethyl)phenyl)-4-methyl-1H-1,2,3-triazol-5-yl)methoxy)pyridazin-3-yl)-1-methylpiperazin-2-one (26)

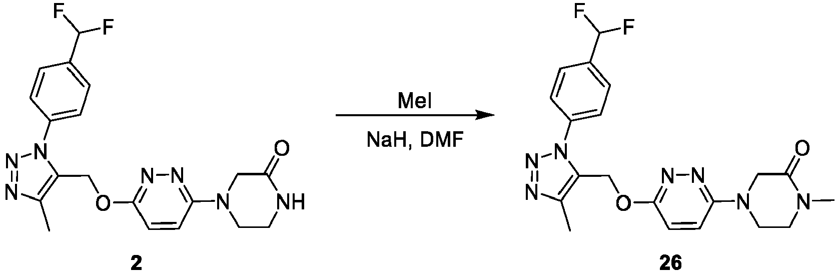

Example **2** (40 mg, 0.096 mmol) was dissolved in *N*,*N-*dimethylformamide (4 mL) under an argon atmosphere, cooled to 0°C, and then sodium hydride (5.3 mg, 60%, 0.12 mmol) was added thereto. The mixture was stirred for 30 minutes, and then methyl iodide (16 mg, 0.11 mmol) was added dropwise thereto. The reaction mixture was stirred at room temperature for 2 hours. The completion of the reaction was monitored by TLC. The reaction mixture was quenched by pouring into water (30 mL) and extracted with ethyl acetate (30 mL × 2). The organic phases were combined, washed with saturated brine (50 mL), dried over anhydrous sodium sulfate, and concentrated under reduced pressure. The residue was purified sequentially by flash silica gel column chromatography (petroleum ether: ethyl acetate = 0:1) and reversed-phase C18 flash preparative chromatography (acetonitrile: water = 0 to 40%) to obtain the title product (7 mg, white solid).

MS (ESI) m/z [M+H]⁺ = 430.2.

¹H NMR (400 MHz, DMSO-*d*₆) δ 7.78 (s, 4H), 7.43 (d, *J* = 9.6 Hz, 1H), 7.14 (t, *J* = 55.6 Hz, 1H), 7.04 (d, *J* = 9.6 Hz, 1H), 5.45 (s, 2H), 4.02 (s, 2H), 3.74 (t, *J* = 5.4 Hz, 2H), 3.39 (t, *J* = 5.4 Hz, 2H), 2.87 (s, 3H), 2.39 (s, 3H).

### Example 27:

### (S)-4-(6-((1-(4-(Difluoromethyl)phenyl)-4-methyl-1H-1,2,3-triazol-5-yl)methoxy)pyridazin-3-yl)piperazine-2-carboxylic acid (27)

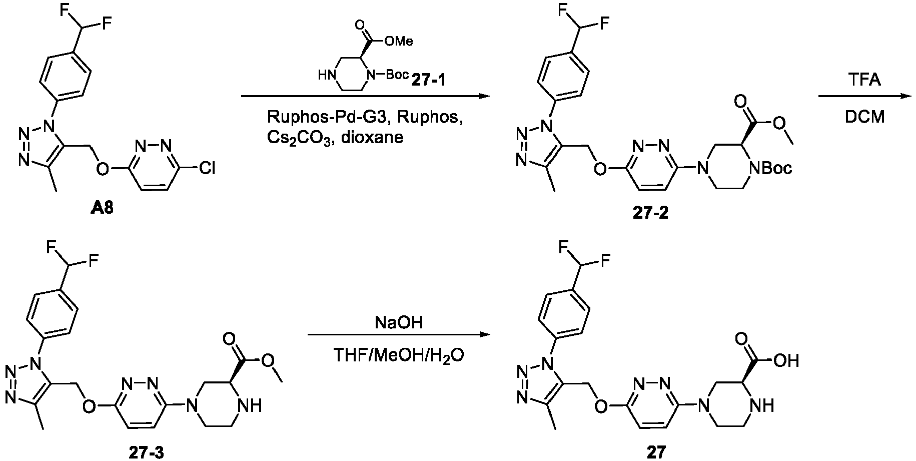

### Step 1: Synthesis of 1-(tert-butyl) 2-methyl (S)-4-(6-((1-(4-(difluoromethyl)phenyl)-4-methyl-1H-1,2,3-triazol-5-yl)methoxy)pyridazin-3-yl)piperazine-1,2-dicarboxylate (27-2)

The experimental operation was as described in Example 1, using **A8** and **27-1** as reactants to obtain the title product.

MS (ESI) m/z [M+H]⁺ = 560.2.

### Step 2: Synthesis of methyl (S)-4-(6-((1-(4-(difluoromethyl)phenyl)-4-methyl-1H-1,2,3-triazol-5-yl)methoxy)pyridazin-3-yl)piperazine-2-carboxylate (27-3)

**27-2** (130 mg, 0.23 mmol) was dissolved in dichloromethane (10 mL), then trifluoroacetic acid (1 mL) was added thereto, and the mixture was stirred at room temperature for 2 hours. The completion of the reaction was monitored by LCMS. The reaction mixture was concentrated under reduced pressure to obtain the title product **27-3** (130 mg, yellow oil), and the crude product was directly used in the next reaction step without further purification.

MS (ESI) m/z [M+H]⁺ = 460.

### Step 3: Synthesis of (S)-4-(6-((1-(4-(difluoromethyl)phenyl)-4-methyl-1H-1,2,3-triazol-5-yl)methoxy)pyridazin-3-yl)piperazine-2-carboxylic acid (27)

**27-3** (130 mg, 0.23 mmol) was dissolved in a mixed solvent of tetrahydrofuran (2 mL), methanol (2 mL), and water (2 mL), then sodium hydroxide (46 mg, 1.15 mmol) was added thereto, and the mixture was stirred at room temperature for 2 hours. The completion of the reaction was monitored by LCMS. The reaction mixture was added with 1.0 M dilute hydrochloric acid to adjust the pH to 3, and extracted with dichloromethane (10 mL × 3). The organic phase was dried over anhydrous sodium sulfate and filtered, and the filtrate was concentrated under reduced pressure. The residue was purified by reversed-phase C18 flash preparative chromatography (acetonitrile: water = 0 to 35%) to obtain the title product (30 mg, white solid).

MS (ESI) m/z [M+H]⁺ = 446.2.

¹H NMR (400 MHz, DMSO-*d*₆) δ 7.78 (s, 4H), 7.44 (d, *J* = 9.6 Hz, 1H), 7.14 (t, *J* = 55.6 Hz, 1H), 7.05 (d, *J* = 9.6 Hz, 1H), 5.46 (s, 2H), 4.31-4.27 (m, 1H), 3.99-3.96 (m, 1H), 3.56 (br. s, 1H), 3.20-3.14 (m, 3H), 2.99-2.94 (m, 1H), 2.39 (s, 3H).

### Example 28:

### (R)-4-(6-((1-(4-(Difluoromethyl)phenyl)-4-methyl-1H-1,2,3-triazol-5-yl)methoxy)pyridazin-3-yl)piperazine-2-carboxylic acid (28)

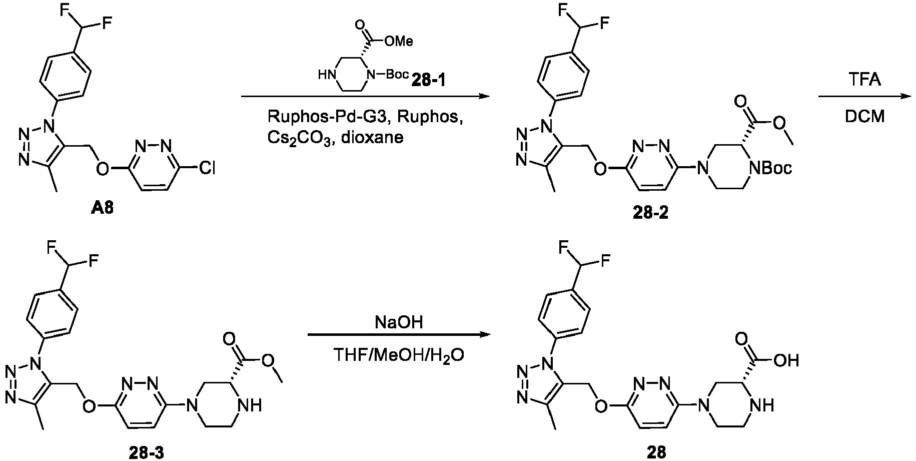

The experimental operation was as described in Example **27,** using **A8** and *(R)*-1-tert-butyl 3-methylpiperazine-1,3-dicarboxylate as reactants, and undergoing the steps of coupling, deprotection, and hydrolysis reactions to obtain the title product.

MS (ESI) m/z [M+H]⁺ = 446.2.

¹H NMR (400 MHz, DMSO-*d*₆) δ 7.78 (s, 4H), 7.44 (d, *J* = 9.6 Hz, 1H), 7.14 (t, *J* = 55.6 Hz, 1H), 7.05 (d, *J* = 9.6 Hz, 1H), 5.46 (s, 2H), 4.28-4.25 (m, 1H), 3.99-3.95 (m, 1H), 3.56 (br. s, 1H), 3.20-3.14 (m, 3H), 2.99-2.93 (m, 1H), 2.39 (s, 3H).

### Example 29:

### (S)-1-(6-((1-(4-(Difluoromethyl)phenyl)-4-methyl-1H-1,2,3-triazol-5-yl)methoxy)pyridazin-3-yl)piperazine-2-carboxylic acid (29)

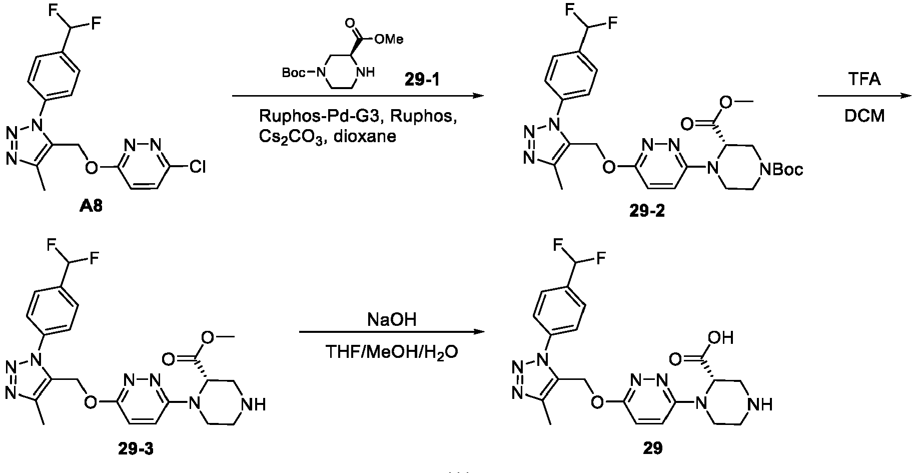

The experimental operation was as described in Example **27,** using **A8** and (*S*)*-*1*-tert-*butyl 3-methylpiperazine-1,3-dicarboxylate as reactants, and undergoing the steps of coupling, deprotection, and hydrolysis reactions to obtain the title product.

MS (ESI) m/z [M+H]⁺ = 446.2.

¹H NMR (400 MHz, DMSO-*d*₆) δ 7.77 (s, 4H), 7.28 (d, *J* = 8.8 Hz, 1H), 7.12 (t, *J* = 56.4 Hz, 1H), 6.96 (d, *J* = 8.8 Hz, 1H), 5.41 (s, 2H), 4.66 (s, 1H), 3.92-3.90 (m, 1H), 3.52-3.49 (m, 1H), 3.07-3.04 (m, 1H), 2.92-2.89 (m, 1H), 2.74-2.64 (m, 2H), 2.37 (s, 3H).

### Example 30:

### (S)-4-(6-((1-(4-(Difluoromethyl)phenyl)-4-methyl-1H-1,2,3-triazol-5-yl)methoxy)pyridazin-3-yl)-N-methylpiperazine-2-carboxamide (30)

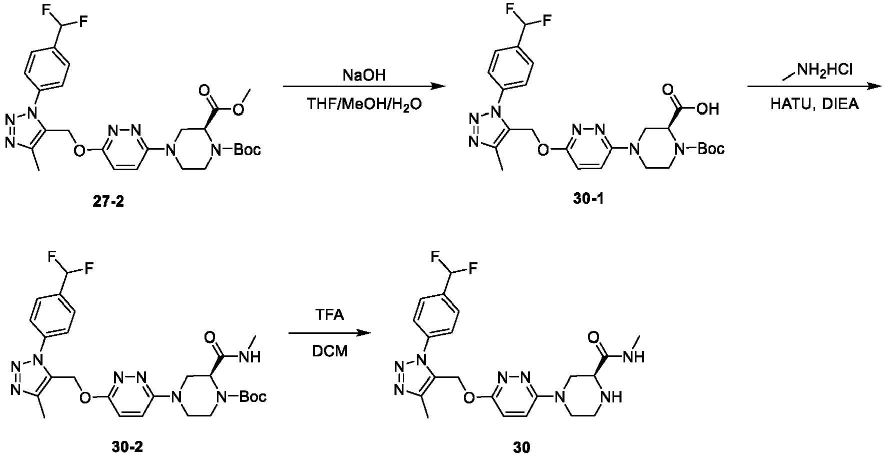

### Step 1: Synthesis of (S)-1-(tert-butoxycarbonyl)-4-(6-((1-(4-(difluoromethyl)phenyl)-4-methyl-1H-1,2,3-triazol-5-yl)methoxy)pyridazin-3-yl)piperazine-2-carboxylic acid (30-1)

**27-2** (200 mg, 0.36 mmol) was dissolved in a mixed solution of tetrahydrofuran (2 mL), methanol (2 mL), and water (2 mL), then sodium hydroxide (14 mg, 0.36 mmol) was added thereto, and the mixture was stirred at room temperature for 3 hours. The completion of the reaction was monitored by LCMS. The reaction mixture was added with 1.0 M dilute hydrochloric acid to adjust the pH to 3, and extracted with dichloromethane (10 mL × 3). The organic phase was dried over anhydrous sodium sulfate and filtered, and the filtrate was concentrated under reduced pressure to obtain the title product (150 mg, yellow solid).

MS (ESI) m/z [M+H]⁺ = 546.2.

### Step 2: tert-Butyl (S)-4-(6-((1-(4-(difluoromethyl)phenyl)-4-methyl-1H-1,2,3-triazol-5-yl)methoxy)pyridazin-3-yl)-2-(methylcarbamoyl)piperazine-1-carboxylate (30-2)

**30-1** (50 mg, 0.092 mmol) and methylamine hydrochloride (7 mg, 0.10 mmol) were dissolved in *N*,*N-*dimethylformamide (3 mL), then HATU (38 mg, 0.10 mmol) and *N,N-*diisopropylethylamine (36 mg, 0.28 mmol) were added thereto, and the mixture was stirred at room temperature for 16 hours. The completion of the reaction was monitored by LCMS. The reaction mixture was added with saturated brine (20 mL) and extracted with dichloromethane (5 mL × 3). The organic phase was dried over anhydrous sodium sulfate and filtered, and the filtrate was concentrated under reduced pressure to obtain the title product (50 mg, yellow solid).

MS (ESI) m/z [M+H]⁺ = 559.3.

### Step 3: (S)-4-(6-((1-(4-(Difluoromethyl)phenyl)-4-methyl-1H-1,2,3-triazol-5-yl)methoxy)pyridazin-3-yl)-N-methylpiperazine-2-carboxamide (30)

**30-2** (50 mg, 0.09 mmol) was dissolved in dichloromethane (5 mL), then trifluoroacetic acid (0.5 mL) was added thereto, and the mixture was stirred at room temperature for 3 hours. The completion of the reaction was monitored by LCMS. The reaction mixture was concentrated under reduced pressure, and the residue was purified by reversed-phase C18 flash preparative chromatography (acetonitrile: water = 0 to 35%) to obtain the title product (20 mg, white solid).

MS (ESI) m/z [M+H]⁺ = 459.2.

¹H NMR (400 MHz, DMSO-*d*₆) δ 8.18 (s, 1H), 7.86-7.85 (m, 1H), 7.78 (s, 4H), 7.35 (d, *J* = 9.6 Hz, 1H), 7.13 (t, *J* = 55.6 Hz, 1H), 6.99 (d, *J* = 9.6 Hz, 1H), 5.44 (s, 2H), 3.97-3.95 (m, 1H), 3.73-3.70 (m, 1H), 3.29-3.27 (m, 1H), 2.97-2.91 (m, 3H), 2.73-2.71 (m, 1H), 2.60 (d, *J* = 4.4 Hz, 3H), 2.39 (s, 3H).

### Example 31:

### (S)-4-(6-((1-(4-(Difluoromethyl)phenyl)-4-methyl-1H-1,2,3-triazol-5-yl)methoxy)pyridazin-3-yl)-N,N-dimethylpiperazine-2-carboxamide (31)

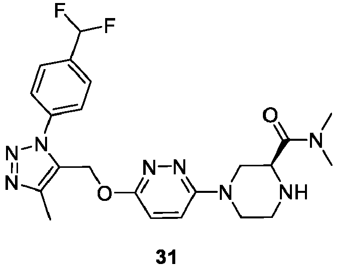

The experimental operation was as described in steps 2 and 3 in Example **30,** using intermediate **30-1** and dimethylamine hydrochloride as reactants, and undergoing the steps of condensation and deprotection reactions to obtain the title product.

MS (ESI) m/z [M+H]⁺= 473.2.

¹H NMR (400 MHz, DMSO-*d*₆) δ 9.14 (br. s, 1H), 7.80 (s, 4H), 7.54 (d, *J* = 9.6 Hz, 1H), 7.14 (t, *J* = 55.6 Hz, 1H), 7.13 (d, *J* = 9.6 Hz, 1H), 5.46 (s, 2H), 4.59-4.56 (m, 2H), 4.20-4.16 (m, 1H), 3.33-3.32 (m, 1H), 3.24 (s, 1H), 3.15 (s, 3H), 3.09-2.99 (m, 2H), 2.91 (s, 3H), 2.40 (s, 3H).

### Example 32:

### (S)-4-(6-((1-(4-(Difluoromethyl)phenyl)-4-methyl-1H-1,2,3-triazol-5-yl)methoxy)pyridazin-3-yl)piperazine-2-amide (32)

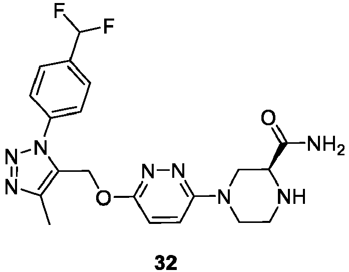

The experimental operation was as described in steps 2 and 3 in Example **30,** using intermediate **30-1** and ammonium chloride as reactants, and undergoing the steps of condensation and deprotection reactions to obtain the title product.

MS (ESI) m/z [M+H]⁺ = 445.2.

¹H NMR(400 MHz, DMSO-*d*₆) δ 7.78 (s, 4H), 7.38-7.36 (m, 2H), 7.18 (br, 1H), 7.13 (t, *J* = 55.6 Hz, 1H), 6.99 (d, *J* = 9.6 Hz, 1H), 5.44 (s, 2H), 4.00-3.98 (m, 1H), 3.75-3.72 (m, 1H), 3.28-3.27 (m, 1H), 3.00-2.90 (m, 3H), 2.74-2.71 (m, 1H), 2.39 (s, 3H).

### Example 33:

### (S)-4-(6-((1-(4-(Difluoromethyl)phenyl)-4-methyl-1H-1,2,3-triazol-5-yl)methoxy)pyridazin-3-yl)piperazine-2-carbonitrile (33)

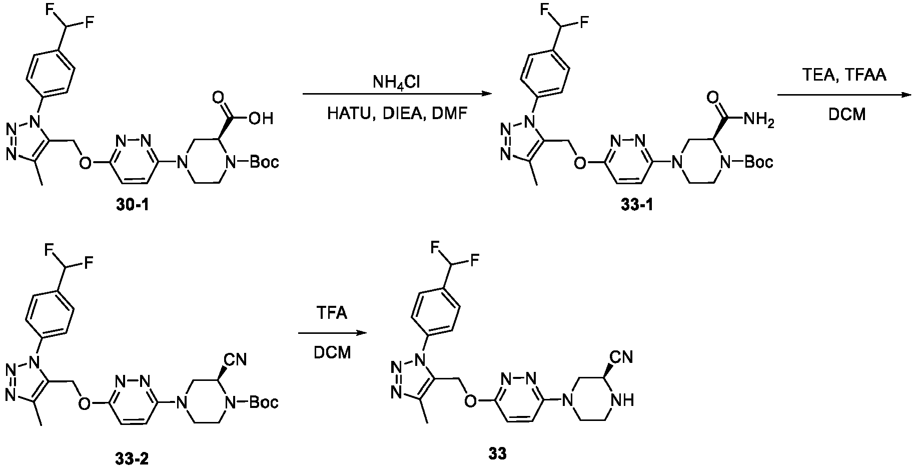

### Step 1: Synthesis of (S)-tert-butyl tert-butyl 2-carbamoyl-4-(6-((1-(4-(difluoromethyl)phenyl)-4-methyl-1H-1,2,3-triazol-5-yl)methoxy)pyridazin-3-yl)piperazine-1-carboxylate (33-1)

The experimental operation was as described in step 2 in Example **30,** using intermediate **30-1** and ammonium chloride as reactants to obtain the title product (33-1).

### Step 2: Synthesis of tert-butyl (S)-2-cyano-4-(6-((1-(4-(difluoromethyl)phenyl)-4-methyl-1H-1,2,3-triazol-5-yl)methoxy)pyridazin-3-yl)piperazine-1-carboxylate (33-2)

**33-1** (100 mg, 0.18 mmol) was dissolved in dichloromethane (8 mL), then triethylamine (73 mg, 0.72 mmol) was added thereto, and the mixture was cooled to 0°C. Trifluoroacetic anhydride (76 mg, 0.36 mmol) was slowly added thereto, and the reaction mixture was returned to room temperature and stirred for 1 hour. The completion of the reaction was monitored by LCMS. The reaction mixture was added with saturated sodium bicarbonate aqueous solution to adjust the pH to 7.0, and extracted with dichloromethane (10 mL × 2). The organic phase was dried over anhydrous sodium sulfate and filtered, and the filtrate was concentrated under reduced pressure. The residue was purified by flash silica gel column chromatography (ethyl acetate: petroleum ether = 0:1 to 3:1) to obtain the title product (45 mg, white solid).

MS (ESI) m/z [M+H]⁺ = 527.2.

### Step 3: Synthesis of (S)-4-(6-((1-(4-(difluoromethyl)phenyl)-4-methyl-1H-1,2,3-triazol-5-yl)methoxy)pyridazin-3-yl)piperazine-2-carbonitrile (33)

**33-2** (45 mg, 0.085 mmol) was dissolved in dichloromethane (5 mL), then trifluoroacetic acid (0.8 mL, 10.73 mmol) was added thereto, and the mixture was stirred at room temperature for 2 hours. The completion of the reaction was monitored by LCMS. The reaction mixture was added with saturated sodium bicarbonate aqueous solution to adjust the pH to 7.0, and extracted with dichloromethane (10 mL × 2). The organic phase was dried over anhydrous sodium sulfate and filtered, and the filtrate was concentrated under reduced pressure. The residue was purified by reversed-phase C18 flash preparative chromatography (acetonitrile: water = 0 to 35%) to obtain the title product (17 mg, light yellow solid).

MS (ESI) m/z [M+H]⁺ = 427.2.

¹H NMR (400 MHz, DMSO-*d*₆) δ 7.77 (s, 4H), 7.43 (d, *J* = 9.6 Hz, 1H), 7.12 (t, *J* = 56.0 Hz, 1H), 7.02 (d, *J* = 9.6 Hz, 1H), 5.44 (s, 2H), 4.23 (s, 1H), 4.12-4.09 (m, 1H), 3.88-3.86 (m, 1H), 3.28-3.17 (m, 1H), 3.10-3.06 (m, 1H), 2.93-2.80 (m, 3H), 2.38 (s, 3H).

### Example 34:

### (S)-2-(4-(6-((1-(4-(Difluoromethyl)phenyl)-4-methyl-1H-1,2,3-triazol-5-yl)methoxy)pyridazin-3-yl)piperazin-2-yl)-5-methyl-1,3,4-oxadiazole (34)

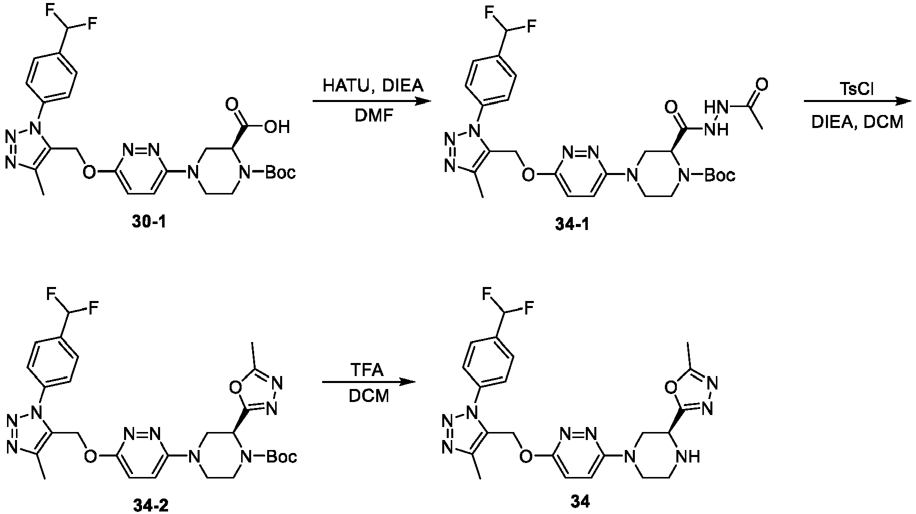

### Step 1: Synthesis of tert-butyl (S)-2-(2-acetylhydrazine-1-carbonyl)-4-(6-((1-(4-(difluoromethyl)phenyl)-4-methyl-1H-1,2,3-triazol)-5-yl)methoxy)pyridazin-3-ylpiperazine-1-carboxylate (34-1)

The experimental operation was as described in step 2 in Example **30,** using intermediate **30-1** and acetylhydrazine as reactants to obtain the title product **(34-1).**

MS (ESI) m/z [M+H]⁺ = 602.3.

### Step 2: Synthesis of tert-butyl (S)-4-(6-((1-(4-(difluoromethyl)phenyl)-4-methyl-1H-1,2,3-triazol-5-yl)methoxy)pyridazin-3-yl)-2-(5-methyl-1,3,4-oxadiazol-2-yl)piperazine-1-carboxylate (34-2)

**34-1** (100 mg, 0.17 mmol) and *N*,*N-*diisopropylethylamine (220 mg, 1.70 mmol) were dissolved in dichloromethane (15 mL), then *p*-toluenesulfonyl chloride (320 mg, 1.70 mmol) was added thereto, and the mixture was stirred at room temperature for 16 hours. The completion of the reaction was monitored by LCMS. The reaction mixture was added with saturated brine (20 mL) and extracted with dichloromethane (10 mL × 3). The organic phase was dried over anhydrous sodium sulfate and filtered, and the filtrate was concentrated under reduced pressure. The residue was purified by preparative thin-layer chromatography (dichloromethane: methanol = 20:1) to obtain the title product (90 mg, yellow solid).

MS (ESI) m/z [M+H]⁺ = 584.2.

### Step 3: Synthesis of (S)-2-(4-(6-((1-(4-(difluoromethyl)phenyl)-4-methyl-1H-1,2,3-triazol-5-yl)methoxy)pyridazin-3-yl)piperazin-2-yl)-5-methyl-1,3,4-oxadiazole (34)

**34-2** (90 mg, 0.15 mmol) was dissolved in dichloromethane (5 mL), then trifluoroacetic acid (1 mL) was added thereto, and the mixture was stirred at room temperature for 2 hours. The completion of the reaction was monitored by LCMS. The reaction mixture was concentrated under reduced pressure, and the residue was purified by reversed-phase C18 flash preparative chromatography (acetonitrile: water = 0 to 35%) to obtain the title product (31 mg, white solid).

MS (ESI) m/z [M+H]⁺ = 484.2.

¹H NMR (400 MHz, DMSO-*d*₆) δ 7.78 (s, 4H), 7.41 (d, *J* = 9.6 Hz, 1H), 7.14 (t, *J* = 56.0 Hz, 1H), 7.01 (d, *J* = 9.6 Hz, 1H), 5.45 (s, 2H), 4.12-4.19 (m, 1H), 4.09-4.06 (m, 1H), 3.70-3.67 (m, 1H), 3.34-3.31 (m, 1H), 3.11-3.16 (m, 1H), 2.99-2.96 (m, 1H), 2.81-2.75 (m, 1H), 2.47 (s, 3H), 2.39 (s, 3H).

### Example 35:

### (S)-4-(6-((1-(4-(Difluoromethyl)phenyl)-4-methyl-1H-1,2,3-triazol-5-yl)methoxy)pyridazin-3-yl)morpholine-2-carboxylic acid (35)

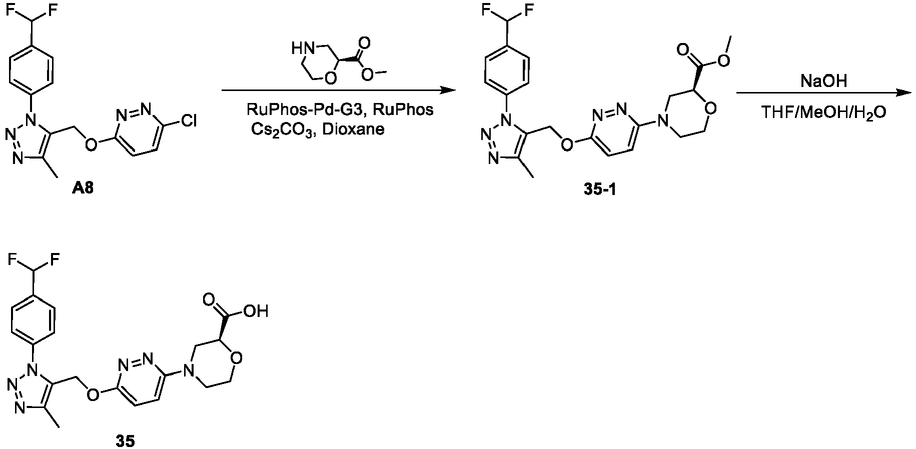

### Step 1: Synthesis of methyl (S)-4-(6-((1-(4-(difluoromethyl)phenyl)-4-methyl-1H-1,2,3-triazol-5-yl)methoxy)pyridazin-3-yl)morpholine-2-carboxylate (35-1)

The experimental operation was as described in Example **1,** using **A8** and methyl (*S*)-morpholine-2-carboxylate as reactants to obtain the title product.

MS (ESI) m/z [M+H]⁺ = 461.2.

### Step 2: Synthesis of (S)-4-(6-((1-(4-(difluoromethyl)phenyl)-4-methyl-1H-1,2,3-triazol-5-yl)methoxy)pyridazin-3-yl)morpholine-2-carboxylic acid (35)

**35-1** (50 mg, 0.11 mmol) was dissolved in a mixed solvent of tetrahydrofuran (2 mL), methanol (2 mL), and water (2 mL), then sodium hydroxide (46 mg, 1.15 mmol) was added thereto, and the mixture was stirred at room temperature for 2 hours. The completion of the reaction was monitored by LCMS. The reaction mixture was added with 1.0 M dilute hydrochloric acid to adjust the pH to 3, and extracted with dichloromethane (10 mL × 3). The organic phase was dried over anhydrous sodium sulfate and filtered, and the filtrate was concentrated under reduced pressure. The residue was purified by reversed-phase C18 flash preparative chromatography (acetonitrile: water = 0 to 35%) to obtain the title product (18 mg, white solid).

MS (ESI) m/z [M+H]⁺ = 447.3.

¹H NMR (400 MHz, DMSO-*d*₆) δ 7.79 (s, 4H), 7.42 (d, *J* = 9.6 Hz, 1H), 7.14 (t, *J* = 55.6 Hz, 1H), 7.04 (d, *J* = 9.6 Hz, 1H), 5.47 (s, 2H), 4.22-4.20 (m, 1H), 4.02-3.91 (m, 2H), 3.66-3.62 (m, 2H), 3.25-3.10 (m, 2H), 2.40 (s, 3H).

### Example 36:

### (S)-4-(6-((1-(4-(Difluoromethyl)phenyl)-4-methyl-1H-1,2,3-triazol-5-yl)methoxy)pyridazin-3-yl)-6-oxopiperazine-2-carboxylic acid (36)

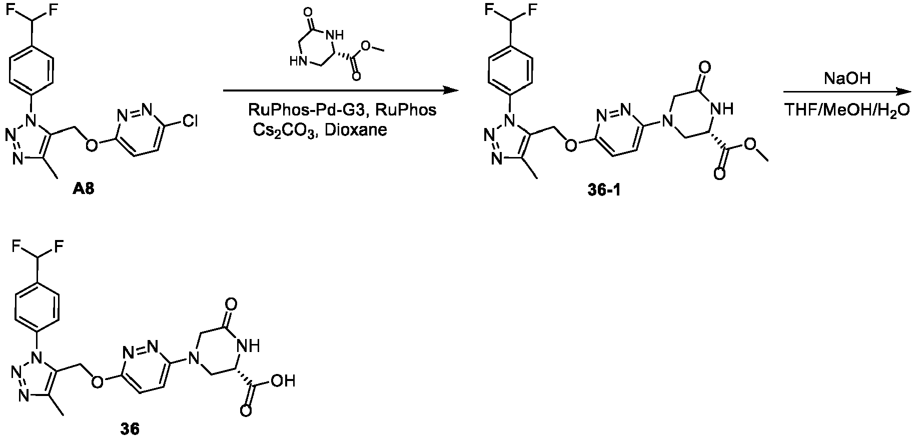

The experimental operation was as described in Example **35,** using **A8** and methyl (*S*)-6-oxopiperazine-2-carboxylate as reactants and through the coupling and hydrolysis to obtain the title product.

MS (ESI) m/z [M+H]⁺ = 460.1.

¹H NMR (400 MHz, DMSO-*d*₆) δ 8.13 (br. s, 1H), 7.78 (s, 4H), 7.35 (d, *J* = 9.4 Hz, 1H), 7.13 (t, *J* = 55.6 Hz, 1H), 7.03 (d, *J* = 9.4 Hz, 1H), 5.44 (s, 2H), 4.17-4.05 (m, 3H), 3.84-3.80 (m, 1H), 3.66-3.63 (m, 1H), 2.39 (s, 3H).

### Example 37:

### 1-(6-((1-(4-(Difluoromethyl)phenyl)-4-methyl-1H-1,2,3-triazol-5-yl)methoxy)pyridazin-3-yl)azetidine-3-carboxylic acid (37)

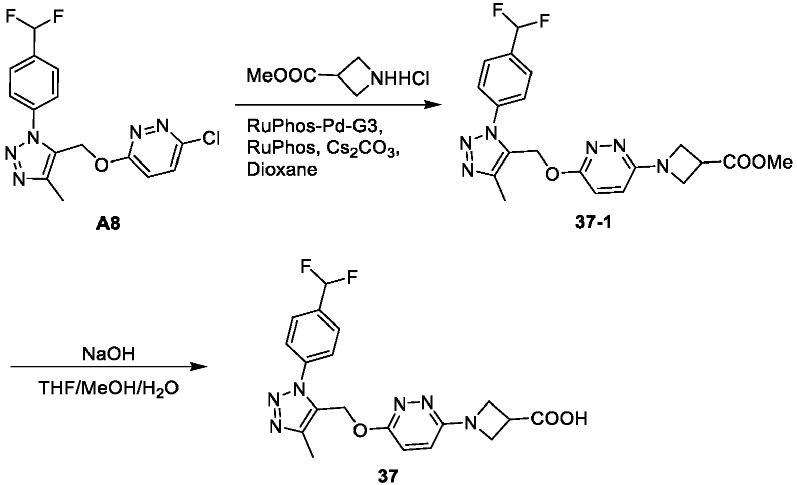

The experimental operation was as described in Example **35,** using **A8** and methyl azetidine-3-carboxylate hydrochloride as reactants and through the coupling and hydrolysis to obtain the title product.

MS (ESI) m/z [M+H]⁺ = 417.1.

¹H NMR (400MHz, DMSO-*d*₆) δ 7.79 (s, 4H), 7.15 (t, *J* = 55.6 Hz, 1H), 7.01 (s, 1H), 6.99 (d, *J* = 9.4 Hz, 1H), 6.92 (d, *J* = 9.4 Hz, 1H), 5.44 (s, 2H), 4.14-4.09 (m, 2H), 4.05-3.96 (m, 2H), 3.49-3.45 (m, 1H), 2.39 (s, 3H).

### Example 38:

### (S)-4-(6-((1-(4-(Difluoromethyl)phenyl)-4-methyl-1H-1,2,3-triazol-5-yl)methoxy)pyridazin-3-yl-N-methylmorpholine-2-carboxamide (38)

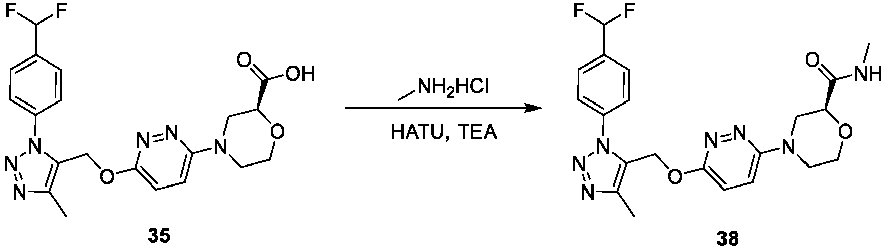

Example **35** (60 mg, 0.13 mmol), methylamine hydrochloride (17.55 mg, 0.26 mmol), and HATU (152.95 mg, 0.26 mmol) were dissolved in dichloromethane (5 mL) at room temperature, then triethylamine (37.02 mg, 0.39 mmol) was added thereto, and the reaction mixture was stirred at room temperature for 1 hour. The completion of the reaction was monitored by LCMS. The reaction mixture was diluted with water (10 mL) and extracted with ethyl acetate (15 mL × 3). The phases were separated, and the organic phase was concentrated under reduced pressure. The residue was purified by reversed-phase C18 flash preparative chromatography (acetonitrile: water = 0 to 40%) to obtain the title product (10 mg, white solid).

MS (ESI) m/z [M+H]⁺ = 460.2.

¹H NMR (400 MHz, DMSO-*d*₆) δ 7.86 (br. s, 1H), 7.79 (s, 4H), 7.43 (d, *J* = 9.6 Hz, 1H), 7.15 (t, *J* = 55.6 Hz,1H), 7.03 (d, *J* = 9.6 Hz, 1H), 5.47 (s, 2H), 4.20-4.18 (m, 1H), 4.02-3.99 (m, 2H), 3.90-3.86 (m, 1H), 3.69-3.64 (m, 1H), 2.99-2.90 (m, 1H), 2.83-2.74 (m, 1H), 2.62 (d, *J* = 4.6 Hz, 3H), 2.40 (s, 3H).

### Example 39:

### 3-(6-((1-(4-(Difluoromethyl)phenyl)-4-methyl-1H-1,2,3-triazol-5-yl)methoxy)pyridazin-3-yl)-N-ethyl-3-azabicyclo[3.1.0]hexane-6-carboxamide (39)

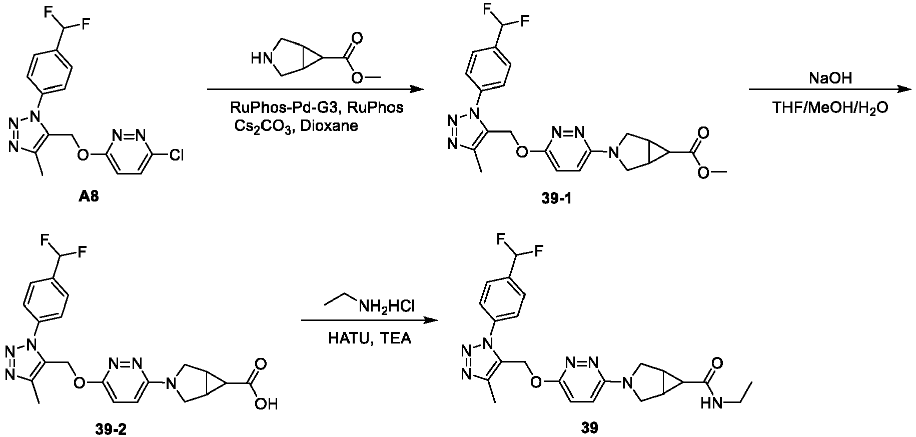

The experimental operation was as described in Example **35** and Example **38,** using intermediate **A8** and methyl 3-azabicyclo[3.1.0]hexane-6-carboxylate as reactants, and undergoing the steps of coupling, hydrolysis, and condensation reactions to obtain the title product.

MS (ESI) m/z [M+H]⁺ = 470.3.

¹H NMR (400 MHz, DMSO-*d*₆) δ 7.96 (t, *J* = 5.6 Hz, 1H), 7.77 (s, 4H), 7.12 (t, *J* = 55.6 Hz, 1H), 7.01-6.95 (m, 2H), 5.40 (s, 2H), 3.67 (d, *J* = 10.4 Hz, 2H), 3.36 (d, *J* = 10.4 Hz, 2H), 3.10-2.99 (m, 2H), 2.37 (s, 3H), 1.99-1.97 (s, 2H), 1.42 (s, 1H), 0.98 (t, *J=* 7.2 Hz, 3H).

### Example 40:

### (R)-1-(6-((1-(4-(Difluoromethyl)phenyl)-4-methyl-1H-1,2,3-triazol-5-yl)methoxy)pyridazin-3-yl)-azetidine-2-carboxamide (40)

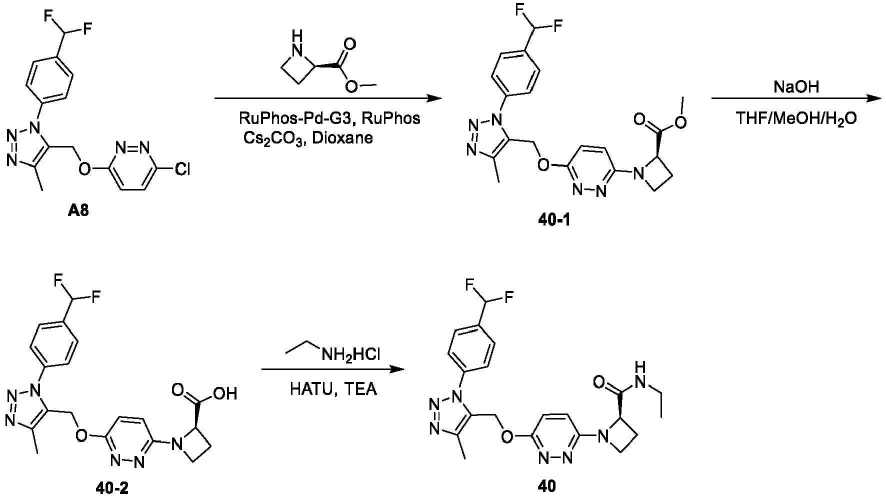

The experimental operation was as described in Example **35** and Example **38,** using intermediate **A8** and methyl azetidine-2-carboxylate as reactants, and undergoing the steps of coupling, hydrolysis, and condensation reactions to obtain the title product.

MS (ESI) m/z [M+H]⁺ = 444.2.

¹H NMR (400 MHz, DMSO-*d*₆) δ 8.04-8.03 (m, 1H), 7.77 (s, 4H), 7.13 (t, *J* = 55.6 Hz, 1H), 7.00 (d, *J* = 9.4 Hz, 1H), 6.83 (d, *J* = 9.4 Hz, 1H), 5.42 (s, 2H), 4.55-4.49 (m, 1H), 3.91-3.88 (m, 1H), 3.86-3.80 (m, 1H), 3.33-3.07 (m, 2H), 2.38 (s, 3H), 2.32-2.25 (m, 2H), 1.00 (t, *J* = 7.2 Hz, 3H).

### Example 41:

### (R)-1-(6-((1-(4-(Difluoromethyl)phenyl)-4-methyl-1H-1,2,3-triazol-5-yl)methoxy)pyridazin-3-yl)-N-ethylpyrrolidine-2-carboxamide (41)

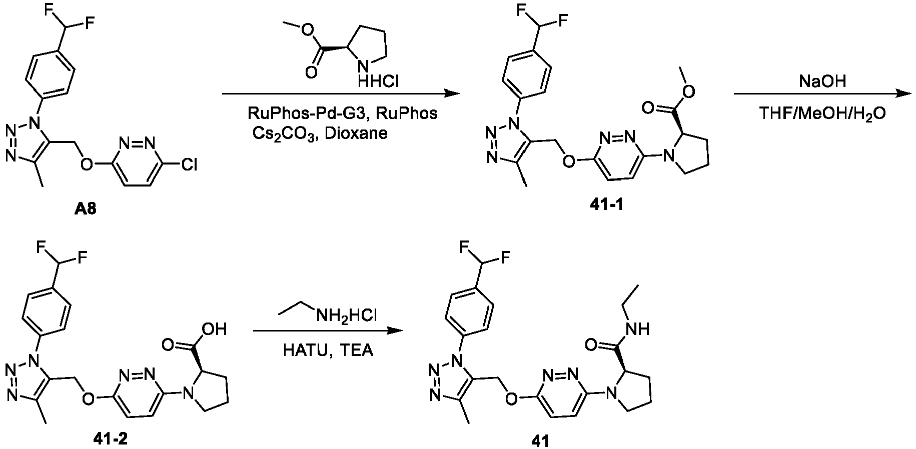

The experimental operation was as described in Example **35** and Example **38,** using intermediate **A8** and (*R*)-proline methyl ester hydrochloride as reactants, and undergoing the steps of coupling, hydrolysis, and condensation reactions to obtain the title product.

MS (ESI) m/z [M+H]⁺ = 444.2.

¹H NMR (400 MHz, DMSO-*d*₆) δ 7.82 (br. s, 1H), 7.79 (s, 4H), 7.15 (t, *J* = 55.6 Hz, 1H), 6.99 (d, *J* = 9.4 Hz, 1H), 6.89 (d, *J* = 9.4 Hz, 1H), 5.43 (s, 2H), 4.28-4.25 (m, 1H), 3.68-3.62 (m, 1H), 3.30-3.25 (m, 1H), 3.05-3.01 (m, 2H), 2.39 (s, 3H), 1.94-1.89 (m, 2H), 1.29-1.23 (m, 2H), 0.95 (t, *J* = 7.0 Hz, 3H).

### Example 42:

### (S)-1-(6-((1-(4-(Difluoromethyl)phenyl)-4-methyl-1H-1,2,3-triazol-5-yl)methoxy)pyridazin-3-yl)-N-ethylpyrrolidine-2-carboxamide (42)

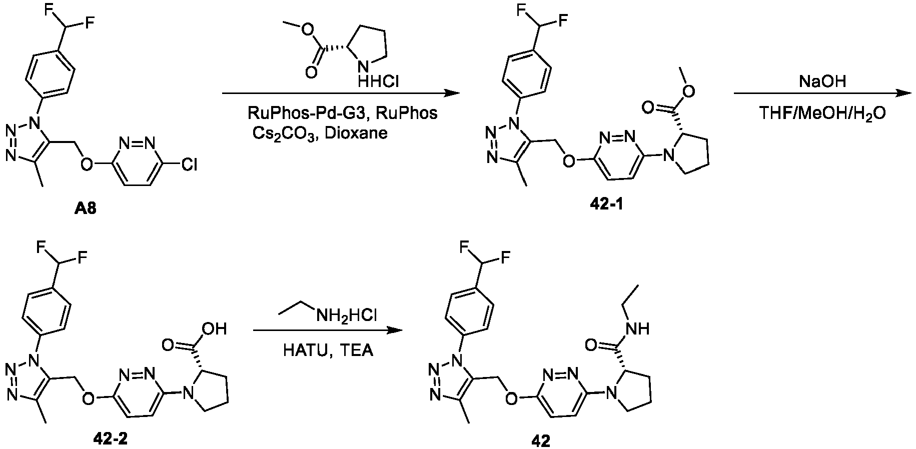

The experimental operation was as described in Example **35** and Example **38,** using intermediate **A8** and (*S*)-proline methyl ester hydrochloride as reactants, and undergoing the steps of coupling, hydrolysis, and condensation reactions to obtain the title product.

MS (ESI) m/z [M+H]⁺ = 444.2.

¹H NMR (400 MHz, DMSO-*d*₆) δ 7.82 (br. s, 1H), 7.79 (s, 4H), 7.15 (t, *J* = 55.6 Hz, 1H), 6.99 (d, *J* = 9.4 Hz, 1H), 6.89 (d, *J* = 9.4 Hz, 1H), 5.43 (s, 2H), 4.28-4.25 (m, 1H), 3.67-3.60 (m, 1H), 3.30-3.25 (m, 1H), 3.04-3.01 (m, 2H), 2.39 (s, 3H), 1.94-1.89 (m, 2H), 1.29-1.23 (m, 2H), 0.95 (t, *J* = 7.0 Hz, 3H).

### Example 43:

### 1-(6-[(1-(4-(Difluoromethyl)phenyl)-4-methyl-1H-1,2,3-triazol-5-yl)methoxy]pyridazin-3-yl)-N-ethylazetidine-3-carboxamide (43)

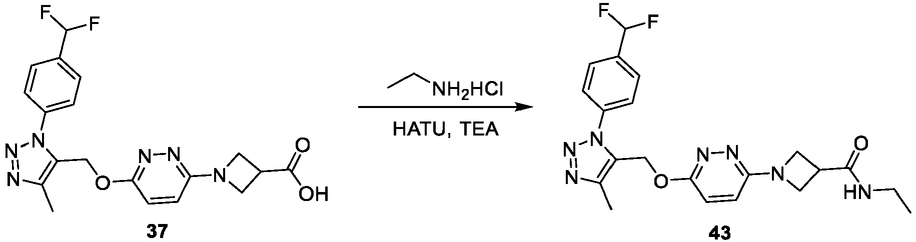

The experimental operation was as described in Example **38,** using compound **37** and ethylamine hydrochloride as reactants to obtain the title product.

MS (ESI) m/z [M+H]⁺ = 444.2.

¹H NMR (400 MHz, DMSO-*d*₆) δ 8.02 (t, *J* = 5.2 Hz, 1H), 7.79 (s, 4H), 7.22 (t, *J* = 55.6 Hz, 1H), 7.00 (d, *J* = 9.4 Hz, 1H), 6.91 (d, *J* = 9.4 Hz, 1H), 5.44 (s, 2H), 4.08-4.03 (m, 2H), 3.97-3.94 (m, 2H), 3.44-3.34 (m, 1H), 3.03-3.10 (m, 2H), 2.40 (s, 3H), 1.01 (t, *J* = 7.2 Hz, 3H).

### Example 44:

### N-Cyclopropyl-1-(6-((1-(4-(difluoromethyl)phenyl)-4-methyl-1H-1,2,3-triazol-5-yl)methoxy)pyridazin-3-yl)azetidine-3-carboxamide (44)

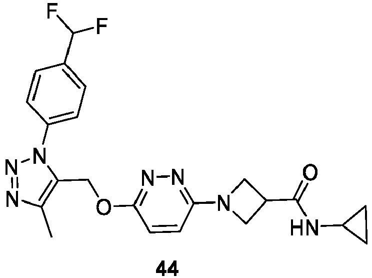

The experimental operation was as described in Example **38,** using compound 37 and cyclopropylamine as reactants to obtain the title product.

MS (ESI) m/z [M+H]⁺ = 456.2.

¹H NMR (400 MHz, DMSO-*d*₆) δ 8.08 (br. s, 1H), 7.79 (s, 4H), 7.22 (t, *J* = 55.6 Hz, 1H), 6.99 (d, *J* = 9.4 Hz, 1H), 6.91 (d, *J* = 9.4 Hz, 1H), 5.43 (s, 2H), 4.06-4.02 (m, 2H), 3.96-3.92 (m, 2H), 3.43-3.37 (m, 1H), 2.66-2.62 (m, 1H), 2.39 (s, 3H), 0.65-0.56 (m, 2H), 0.43-0.40 (m, 2H).

### Example 45:

### 1-(6-((1-(4-(Difluoromethyl)phenyl)-4-methyl-1H-1,2,3-triazol-5-yl)methoxy)pyridazin-3-yl)-N-(oxetan-pyridin-3-yl)azetidine-3-carboxamide (45)

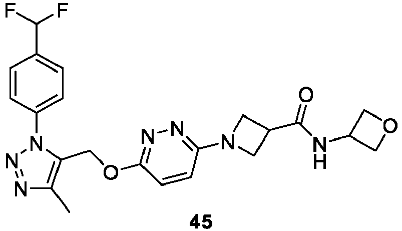

The experimental operation was as described in Example **38,** using compound **37** and 3-oxetanamine as reactants to obtain the title product.

MS (ESI) m/z [M+H]⁺ = 472.2.

¹H NMR (400 MHz, DMSO-*d*₆) δ 8.77 (d, *J* = 8.0 Hz, 1H), 7.78 (s, 4H), 7.13 (t, *J* = 55.6 Hz, 1H), 6.98 (d, *J* = 9.4 Hz, 1H), 6.90 (d, *J* = 9.4 Hz, 1H), 5.43 (s, 2H), 4.81-4.77 (m, 1H), 4.71-4.68 (m, 2H), 4.44-4.40 (m, 2H), 4.09-4.05 (m, 2H), 3.98-3.94 (m, 2H), 3.49-3.45 (m, 1H), 2.38 (s, 3H).

### Example 46:

### 1-(6-((1-(4-(Difluoromethyl)phenyl)-4-methyl-1H-1,2,3-triazol-5-yl)methoxy)pyridazin-3-yl)-N-(1-methylpiperidin-4-yl)azetidine-3-carboxamide (46)

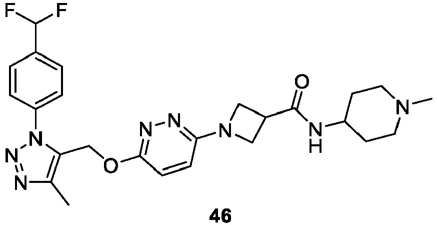

The experimental operation was as described in Example **38,** using compound **37** and 1-methyl-4-aminopiperidine as reactants to obtain the title product.

MS (ESI) m/z [M+H]⁺ = 513.2.

¹H NMR (400 MHz, DMSO-*d*₆) δ 7.90 (d, *J* = 8.0 Hz, 1H), 7.78 (s, 4H), 7.13 (t, *J* = 55.6 Hz, 1H), 6.98 (d, *J* = 9.4 Hz, 1H), 6.89 (d, *J* = 9.4 Hz, 1H), 5.42 (s, 2H), 4.06-4.02 (m, 2H), 3.95-3.92 (m, 2H), 3.45-3.32 (m, 2H), 2.69-2.64 (m, 2H), 2.38 (s, 3H), 2.12 (s, 3H), 1.92-1.87 (m, 2H), 1.70-1.65 (m, 2H), 1.40-1.33 (m, 2H).

### Example 47:

### 1-(6-((1-(4-(Difluoromethyl)phenyl)-4-methyl-1H-1,2,3-triazol-5-yl)methoxy)pyridazin-3-yl)-N-phenylazetidine-3-carboxamide (47)

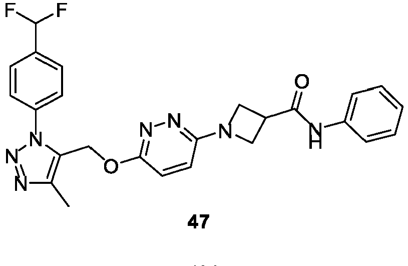

The experimental operation was as described in Example **38,** using compound **37** and aniline as reactants to obtain the title product.

MS (ESI) m/z [M+H]⁺ = 492.2.

¹H NMR (400 MHz, DMSO-*d*₆) δ 11.21 (br. s, 1H), 7.79 (s, 4H), 7.61 (d, *J* = 8.0 Hz, 1H), 7.33-7.15 (m, 4H), 7.07-7.01 (m, 2H), 5.42 (s, 2H), 4.35-4.28 (m, 4H), 3.74-3.76 (m, 1H), 2.41 (s, 3H).

### Example 48:

### N-Benzyl-1-(6-((1-(4-(difluoromethyl)phenyl)-4-methyl-1H-1,2,3-triazol-5-yl)methoxy)pyridazin-3-yl)azetidine-3-carboxamide (48)

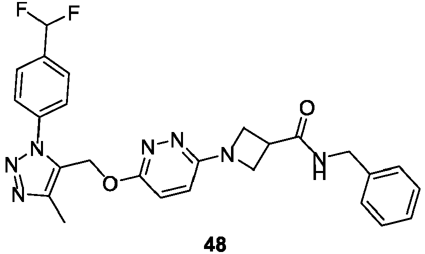

The experimental operation was as described in Example **38,** using compound **37** and benzylamine as reactants to obtain the title product.

MS (ESI) m/z [M+H]⁺ = 506.2.

¹H NMR (400 MHz, DMSO-*d*₆) δ 8.52 (br. s, 1H), 7.78 (s, 4H), 7.33-7.13 (m, 6H), 6.98 (d, *J* = 9.4 Hz, 1H), 6.91 (d, *J* = 9.4 Hz, 1H), 5.43 (s, 2H), 4.32-4.29 (m, 2H), 4.11-4.07 (m, 2H), 4.02-3.98 (m, 2H), 3.55-3.53 (m, 1H), 2.39 (s, 3H).

### Example 49:

### 1-(6-((1-(4-(Difluoromethyl)phenyl)-4-methyl-1H-1,2,3-triazol-5-yl)methoxy)pyridazin-3-yl)-N-(2-methoxyethyl)azetidine-3-carboxamide (49)

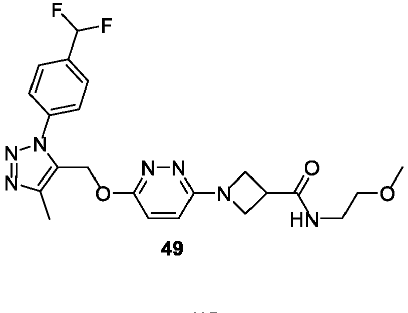

The experimental operation was as described in Example **38,** using compound **37** and 2-methoxyethylamine as reactants to obtain the title product.

MS (ESI) m/z [M+H]⁺ = 474.2.

¹H NMR (400 MHz, DMSO-*d*₆) δ 8.14 (br. s, 1H), 7.78 (s, 4H), 7.28-7.00 (m, 3H), 5.42 (s, 2H), 4.17-4.02 (m, 2H), 4.05-4.02 (m, 2H), 3.52-3.48 (m, 1H), 3.26-3.23 (m, 7H), 2.39 (s, 3H).

### Example 50:

### 1-(6-((1-(4-(Difluoromethyl)phenyl)-4-methyl-1H-1,2,3-triazol-5-yl)methoxy)pyridazin-3-yl)-N-(tetrahydro-2H-pyran-4-yl)azetidine-3-carboxamide (50)

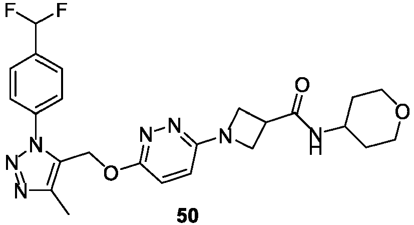

The experimental operation was as described in Example 38, using compound 37 and 4-amino-tetrahydropyran as reactants to obtain the title product.

MS (ESI) m/z [M+H]⁺ = 500.2.

¹H NMR (400 MHz, DMSO-*d*₆) δ 7.98 (d, *J* = 8.0 Hz, 1H), 7.78 (s, 4H), 7.13 (t, *J* = 55.6 Hz, 1H), 6.98 (d, *J* = 9.4 Hz, 1H), 6.89 (d, *J* = 9.4 Hz, 1H), 5.42 (s, 2H), 4.05 (t, *J* = 5.2 Hz, 2H), 3.95 (t, *J* = 5.2 Hz, 2H), 3.46-3.42 (m, 1H), 3.35-3.30 (m, 2H), 2.38 (s, 3H), 1.71-1.66 (m, 2H), 1.40-1.33 (m, 2H).

### Example 51:

### 1-(6-((1-(4-(Difluoromethyl)phenyl)-4-methyl-1H-1,2,3-triazol-5-yl)methoxy)pyridazin-3-yl)-N-(tetrahydrofuran-3-yl)azetidine-3-carboxamide (51)

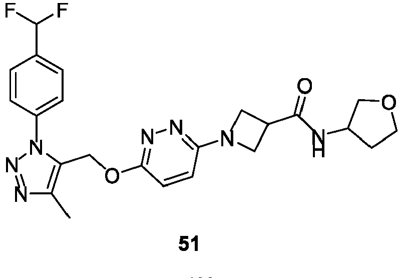

The experimental operation was as described in Example 38, using compound 37 and 3-aminotetrahydrofuran as reactants to obtain the title product.

MS (ESI) m/z [M+H]⁺ = 486.2.

¹H NMR (400 MHz, DMSO-*d*₆) δ 8.23 (br. s, 1H), 7.78 (s, 4H), 7.13 (t, *J* = 55.6 Hz, 1H), 6.98 (d, *J* = 9.4 Hz, 1H), 6.90 (d, *J* = 9.4 Hz, 1H), 5.44 (s, 2H), 4.25-4.22 (m, 1H), 4.08-4.03 (m, 2H), 3.97-3.94 (m, 2H), 3.78-3.71 (m, 2H), 3.68-3.64 (m, 1H), 3.47-3.44 (m, 2H), 2.39 (s, 3H), 2.21-2.05 (m, 1H), 1.73-1.68 (m, 1H).

### Example 52:

### 1-(6-((1-(4-(Difluoromethyl)phenyl)-4-methyl-1H-1,2,3-triazol-5-yl)methoxy)pyridazin-3-yl)-N-(pyridin-4-yl)azetidine-3-carboxamide (52)

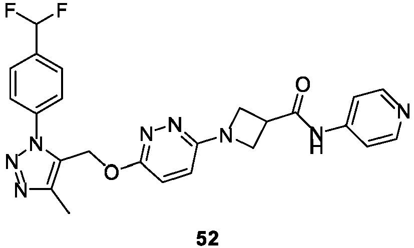

The experimental operation was as described in Example **38,** using compound **37** and 4-aminopyridine as reactants to obtain the title product.

MS (ESI) m/z [M+H]⁺ = 493.2.

¹H NMR (400 MHz, DMSO-*d*₆) δ 11.44 (s, 1H), 8.42 (d, *J* = 7.6 Hz, 2H), 7.57 (d, *J* = 7.6 Hz, 2H), 7.14 (t, *J* = 55.6 Hz, 1H), 7.01 (d, *J* = 9.4 Hz, 1H), 6.95 (d, *J* = 9.4 Hz, 1H), 5.44 (s, 2H), 4.18-4.14 (m, 2H), 3.75-3.69 (m, 2H), 2.39 (s, 3H).

### Example 53:

### 2-(1-(6-((1-(4-(Difluoromethyl)phenyl)-4-methyl-1H-1,2,3-triazol-5-yl)methoxy)pyridazin-3-yl)azetidin-3-yl)-5-methyl-1,3,4-oxadiazole (53)

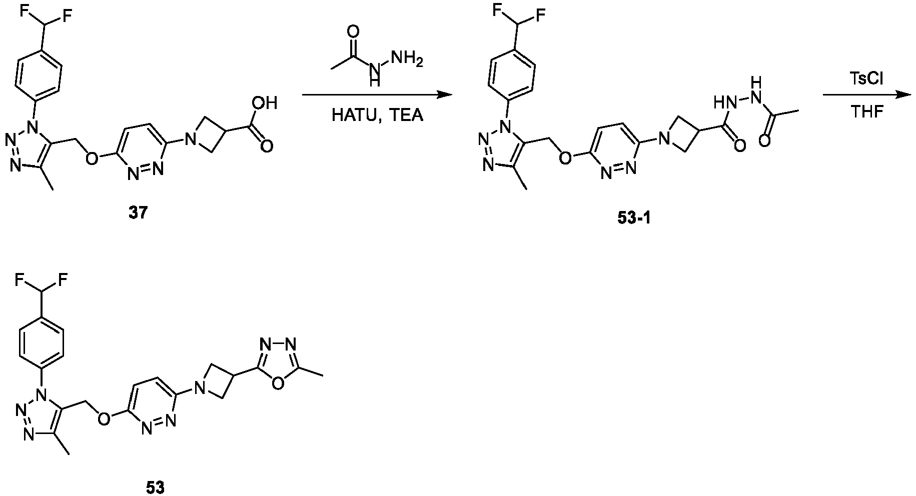

### Step 1: Synthesis of N'-acetyl-1-(6-((1-(4-(difluoromethyl)phenyl)-4-methyl-1H-1,2,3-triazol-5-yl)methoxy)pyridazin-3-yl)azetidine-3-carbohydrazide (53-1)

The experimental operation was as described in Example **38,** using **37** and acetylhydrazine as reactants to obtain the title product.

### Step 2: Synthesis of 2-(1-(6-((1-(4-(difluoromethyl)phenyl)-4-methyl-1H-1,2,3-triazol-5-yl)methoxy)pyridazin-3-yl)azetidin-3-yl)-5-methyl-1,3,4-oxadiazole (53)

53-1 (150 mg, 0.22 mmol) and *N*,*N-*diisopropylethylamine (220 mg, 1.70 mmol) were dissolved in dichloromethane (15 mL), then *p*-toluenesulfonyl chloride (320 mg, 1.70 mmol) was added thereto, and the mixture was stirred at room temperature for 16 hours. The completion of the reaction was monitored by LCMS. The reaction mixture was added with saturated brine (20 mL) and extracted with dichloromethane (10 mL × 3). The organic phase was dried over anhydrous sodium sulfate and filtered, and the filtrate was concentrated under reduced pressure. The residue was purified by preparative thin-layer chromatography (dichloromethane: methanol = 20:1) to obtain the title product (63 mg, white solid).

MS (ESI) m/z [M+H]⁺ = 455.2.

¹H NMR (400 MHz, DMSO-*d*₆) δ 7.79 (s, 4H), 7.14 (t, *J* = 55.6 Hz, 1H), 7.04-6.97 (m, 2H), 5.45 (s, 2H), 4.39-4.35 (m, 2H), 4.25-4.22 (m, 1H), 4.17-4.13 (m, 2H), 2.39 (s, 3H), 2.47 (s, 3H).

### Example 54:

### (S)-8-(6-((1-(4-(Difluoromethyl)phenyl)-4-methyl-1H-1,2,3-triazol-5-yl)methoxy)pyridazin-3-yl)hexahydro-2H-pyrazino[1,2-a]pyrazin-1(6H)-one (54)

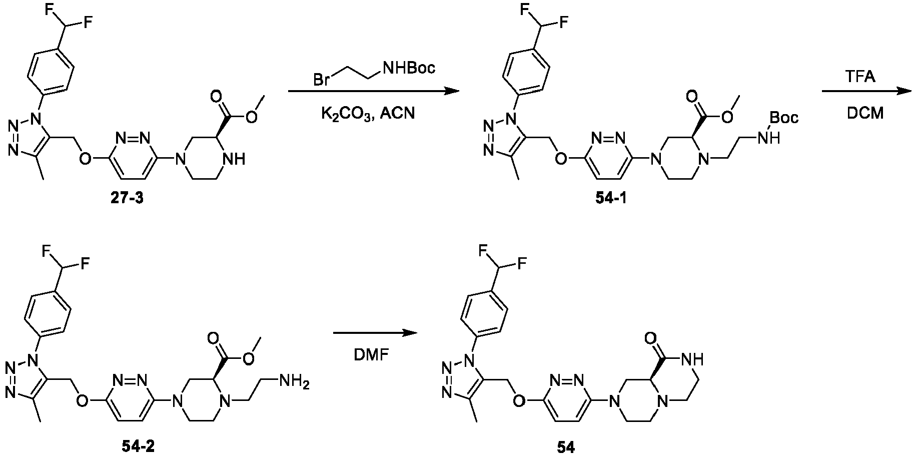

### Step 1: Synthesis of methyl (S)-1-(2-((tert-butoxycarbonyl)amino)ethyl)-4-(6-((1-(4-(difluoromethyl)phenyl)-4-methyl-1H-1,2,3-triazol-5-yl)methoxy)pyridazin-3-ylpiperazine-2-carboxylate (54-1)

**27-3** (110 mg, 0.24 mmol) was dissolved in acetonitrile (15 mL), then potassium carbonate (66 mg, 0.48 mmol) and *tert*-butyl *N*-(2-bromoethyl)carbamate (81 mg, 0.36 mmol) were sequentially added thereto. The mixture was stirred at 60°C for 16 hours. The completion of the reaction was monitored by LCMS. The reaction mixture was added with saturated brine (15 mL) and extracted with dichloromethane (10 mL × 3). The organic phase was dried over anhydrous sodium sulfate and filtered, and the filtrate was concentrated under reduced pressure. The residue was purified by flash silica gel column chromatography (ethyl acetate: petroleum ether = 1:1 to 1:0) to obtain the title product (80 mg, colorless oil).

MS (ESI) m/z [M+H]⁺ = 603.3.

### Step 2: Synthesis of methyl (S)-1-(6-((1-(4-(difluoromethyl)phenyl)-4-methyl-1H-1,2,3-triazol-5-yl)methoxy)pyridazin-3-yl)piperazine-2-carboxylate (54-2)

**54-1** (78 mg, 0.13 mmol) was dissolved in dichloromethane (5 mL), then trifluoroacetic acid (1 mL) was added thereto, and the mixture was stirred at room temperature for 2 hours. The completion of the reaction was monitored by LCMS, and the pH of the reaction mixture was adjusted to neutral with saturated sodium bicarbonate aqueous solution. The reaction mixture was extracted with dichloromethane (10 mL × 3). The organic phase was dried over anhydrous sodium sulfate and filtered, and the filtrate was concentrated under reduced pressure to obtain the title product (65 mg, yellow solid).

MS (ESI) m/z [M+H]⁺ = 503.2.

### Step 3: Synthesis of (S)-8-(6-((1-(4-(difluoromethyl)phenyl)-4-methyl-1H-1,2,3-triazol-5-yl)methoxy)pyridazin-3-yl)hexahydro-2H-pyrazino[1,2-a]pyrazin-1(6H)-one (54)

**54-2** (50 mg, 0.099 mmol) was dissolved in *N*,*N-*dimethylformamide (5 mL), and the reaction mixture was stirred at 60°C for 16 hours. The completion of the reaction was monitored by LCMS, and the reaction mixture was added with saturated brine (30 mL) and extracted with ethyl acetate (10 mL × 3). The organic phase was dried over anhydrous sodium sulfate and filtered, and the filtrate was concentrated under reduced pressure. The residue was purified by reversed-phase C18 flash preparative chromatography (acetonitrile: water = 0 to 35%) to obtain the title product (12 mg, white solid).

MS (ESI) m/z [M+H]⁺ = 471.2.

¹H NMR (400 MHz, DMSO-*d*₆) δ 7.79 (s, 5H), 7.40 (d, *J* = 9.6 Hz, 1H), 7.13 (t, *J* = 56.0 Hz, 1H), 6.99 (d, *J* = 9.6 Hz, 1H), 5.45 (s, 2H), 4.45-4.43 (m, 1H), 4.02-3.99 (m, 1H), 3.37-3.35 (m, 1H), 3.09-3.05 (m, 1H), 2.95-2.85 (m, 3H), 2.65-2.61 (m, 2H), 2.39 (s, 3H), 2.33-2.25 (m, 2H).

### Example 55:

### (R)-8-(6-((1-(4-(Difluoromethyl)phenyl)-4-methyl-1H-1,2,3-triazol-5-yl)methoxy)pyridazin-3-yl)hexahydro-2H-pyrazino[1,2-a]pyrazin-1(6H)-one (55)

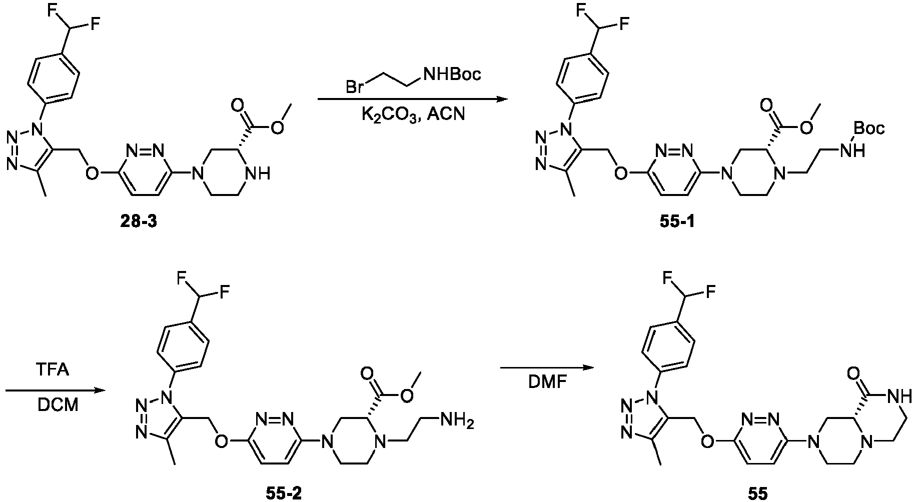

The experimental operation was as described in Example **54,** using intermediate **28-3** as the reactant, and undergoing the steps of alkylation, deprotection, and cyclization reactions to obtain the title product.

MS (ESI) m/z [M+H]⁺ = 471.2.

¹H NMR (400 MHz, DMSO-*d*₆) δ 7.79 (s, 5H), 7.40 (d, *J* = 9.6 Hz, 1H), 7.13 (t, *J* = 56.0 Hz, 1H), 6.99 (d, *J* = 9.6 Hz, 1H), 5.45 (s, 2H), 4.45-4.43 (m, 1H), 4.02-3.99 (m, 1H), 3.37-3.35 (m, 1H), 3.08-3.05 (m, 1H), 2.93-2.85 (m, 3H), 2.64-2.61 (m, 2H), 2.39 (s, 3H), 2.33-2.24 (m, 2H).

### Example 56:

### (S)-8-(6-((1-(4-(Difluoromethyl)phenyl)-4-methyl-1H-1,2,3-triazol-5-yl)methoxy)pyridin-3-yl)octahydro-1H-pyrazino[1,2-a]pyrazin-1-one (56)

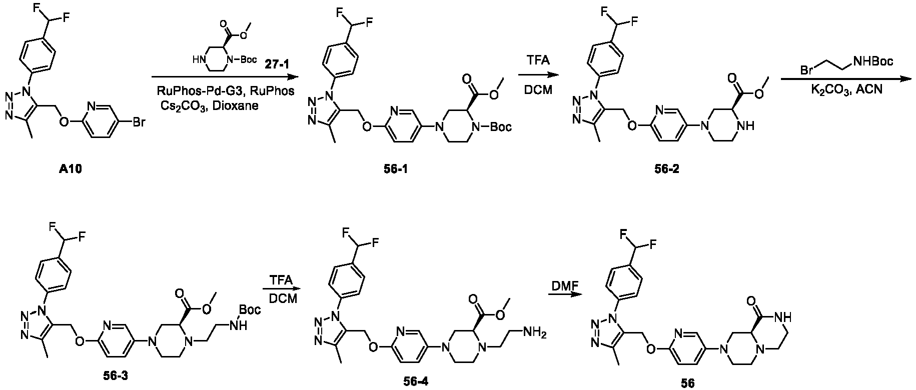

### Step 1: Synthesis of 1-(tert-butyl) 2-methyl (S)-4-(6-((1-(4-(difluoromethyl)phenyl)-4-methyl-1H-1,2,3-triazol-5-yl)methoxy)pyridin-3-yl)piperazine-1,2-dicarboxylate (56-1)

The experimental operation was as described in Example **1,** using intermediates **A10** and **27-1** as reactants to obtain the title product.

MS (ESI) m/z [M+H]⁺ = 559.2.

### Step 2: Synthesis of methyl (S)-4-(6-((1-(4-(difluoromethyl)phenyl)-4-methyl-1H-1,2,3-triazol-5-yl)methoxy)pyridin-3-yl)piperazine-2-carboxylate (56-2)

The experimental operation was as described in step 2 in Example **27,** using intermediate **56-1** as the reactant to obtain the title product.

MS (ESI) m/z [M+H]⁺ = 459.2.

### Step 3: Synthesis of methyl (S)-1-(2-((tert-butoxycarbonyl)amino)ethyl)-4-(6-((1-(4-(difluoromethyl)phenyl)-4-methyl-1H-1,2,3-triazol-5-yl)methoxy)pyridin-3-yl)piperazine-2-carboxylate (56-3)

The experimental operation was as described in step 1 in Example **54,** using intermediate **56-2** and *tert*-butyl (2-bromoethyl)carbamate as reactants to obtain the title product.

MS (ESI) m/z [M+H]⁺ = 602.2.

### Step 4: Synthesis of methyl (S)-1-(2-aminoethyl)-4-(6-((1-(4-(difluoromethyl)phenyl)-4-methyl-1H-1,2,3-triazol-5-yl)methoxy)yl)pyridin-3-yl)piperazine-2-carboxylate (56-4)

The experimental operation was as described in step 2 in Example **54,** using intermediate **56-3** as the reactant to obtain the title product.

MS (ESI) m/z [M+H]⁺ = 502.2.

### Step 5: Synthesis of (S)-8-(6-((1-(4-(difluoromethyl)phenyl)-4-methyl-1H-1,2,3-triazol-5-yl)methoxy)pyridin-3-yl)octahydro-1H-pyrazino[1,2-a]pyrazin-1-one (56)

The experimental operation was as described in step 3 in Example **54,** using intermediate **56-4** as the reactant to obtain the title product.

MS (ESI) m/z [M+H]⁺ = 470.2.

¹H NMR (400 MHz, DMSO-*d*₆) δ 7.78 (s, 4H), 7.69 (s, 1H), 7.43 (d, *J* = 9.4 Hz, 1H), 7.14 (t, *J* = 55.6 Hz, 1H), 6.98 (d, *J* = 9.4 Hz, 1H), 5.32 (s, 2H), 3.77-3.71 (m, 1H), 3.38-3.35 (m, 1H), 3.09-3.05 (m, 1H), 2.95-2.91 (m, 2H), 2.71-2.66 (m, 2H), 2.43-2.39 (m, 2H), 2.38 (s, 3H), 2.35-2.31 (m, 2H).

### Example 57:

### 4-(6-((1-(4-(Difluoromethyl)phenyl)-4-methyl-1H-1,2,3-triazol-5-yl)methoxy)pyridazin-3-yl)-5,6-dihydropyridin-2(1H)-one (57)

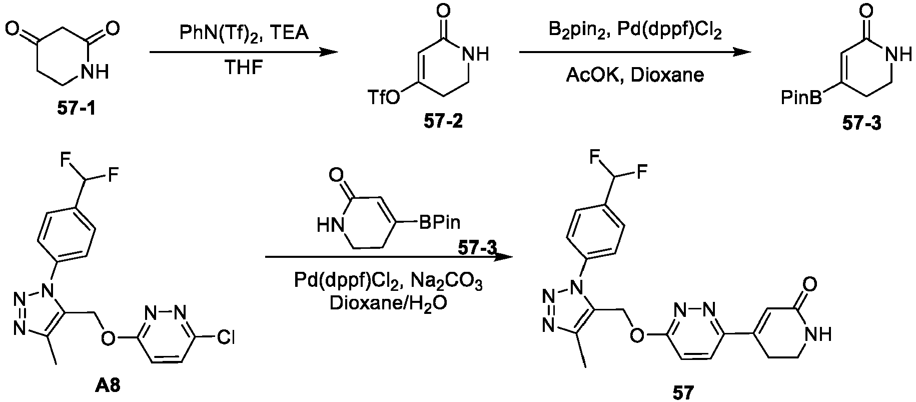

### Step 1: Synthesis of 6-oxo-1,2,3,6-tetrahydropyridin-4-yl trifluoromethanesulfonate (57-2)

Piperidine-2,4-dione (1 g, 8.8 mmol) and triethylamine (1.8 g, 17.7 mmol) were dissolved in tetrahydrofuran (30 mL) under an argon atmosphere, then 1,1,1-trifluoro-*N-*phenyl-*N*-((trifluoromethyl)sulfonyl)methanesulfonamide (3.8 g, 10.6 mmol) was added thereto, and the reaction mixture was stirred at room temperature for 16 hours. The completion of the reaction was monitored by TLC. The reaction mixture was diluted with water (50 mL) and extracted with ethyl acetate (50 mL × 3). The organic phases were combined, washed with saturated brine (100 mL), dried over anhydrous sodium sulfate, and concentrated under reduced pressure. The residue was purified by flash silica gel column chromatography (petroleum ether: ethyl acetate = 3:1) to obtain the title product (1.8 g, white solid).

MS (ESI) m/z [M+H]⁺ = 245.9.

### Step 2: Synthesis of 4-(4,4,5,5-tetramethyl-1,3,2-dioxaborolan-2-yl)-5,6-dihydropyridin-2-2(1H)-one (57-3)

6-Oxo-1,2,3,6-tetrahydropyridin-4-yl trifluoromethanesulfonate (500 mg, 2.04 mmol) and bis(pinacolato)diboron (620 mg, 2.45 mmol) were dissolved in dioxane (20 mL) under an argon atmosphere, then [1,1'-bis(diphenylphosphino)ferrocene]dichloropalladium(II) (150 mg, 0.20 mmol) and potassium acetate (400 mg, 4.08 mmol) were sequentially added thereto, and the reaction mixture was stirred at 70°C for 1.5 hours. The completion of the reaction was monitored by TLC. The reaction mixture was concentrated under reduced pressure to obtain the crude product **57-3** (500 mg). The crude product was used directly in the next step without further purification.

MS (ESI) m/z [M+H]⁺ = 224.0.

### Step 3: Synthesis of 4-(6-((1-(4-(difluoromethyl)phenyl)-4-methyl-1H-1,2,3-triazol-5-yl)methoxy)pyridazin-3-yl)-5,6-dihydropyridin-2(1H)-one (57)

3-Chloro-6-(((1-(4-(difluoromethyl)phenyl)-4-methyl-1*H-*1,2,3-triazol-5-yl)methoxy)pyridazine (100 mg, 0.28 mmol) and 4-(4,4,5,5-tetramethyl-1,3,2-dioxaborolan-2-yl)-5,6-dihydropyridin-2-2(1*H*)-one (69 mg, 0.31 mmol) were dissolved in a mixed solvent of dioxane (4 mL) and water (4 mL) under an argon atmosphere, then [1,1'-bis(diphenylphosphino)ferrocene]dichloropalladium(II) (21 mg, 0.028 mmol) and sodium carbonate (74 mg, 0.70 mmol) were sequentially added thereto, and the reaction mixture was stirred at 95°C for 16 hours. The completion of the reaction was monitored by TLC. The reaction mixture was quenched by pouring into water (20 mL) and extracted with ethyl acetate (30 mL × 2). The organic phases were combined, washed with saturated brine (50 mL), dried over anhydrous sodium sulfate, and concentrated under reduced pressure. The residue was purified sequentially by preparative thin-layer chromatography (petroleum ether: ethyl acetate = 0:1) and reversed-phase C18 flash preparative chromatography (acetonitrile: water = 0 to 40%) to obtain the title product (20 mg, white solid).

MS (ESI) m/z [M+H]⁺ = 413.1.

¹H NMR (400 MHz, DMSO-*d*₆) δ 8.16 (d, *J* = 9.4 Hz, 1H), 7.79 (s, 4H), 7.67 (s, 1H), 7.25 (d, *J* = 9.4 Hz, 1H), 7.13 (t, *J* = 55.6 Hz, 1H), 6.52 (s, 1H), 5.65 (s, 2H), 3.39-3.34 (m, 2H), 2.88-2.85 (m, 2H), 2.42 (s, 3H).

### Example 58:

### 4-(6-((1-(4-(Difluoromethyl)phenyl)-4-methyl-1H-1,2,3-triazol-5-yl)methoxy)pyridazin-3-yl)-1-(tetrahydrofuran-3-yl)piperazin-2-one (58)

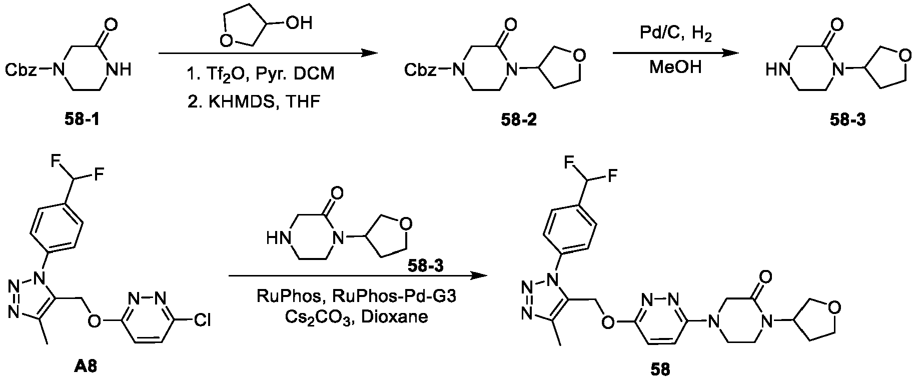

### Step 1: Synthesis of benzyl 3-oxo-4-(tetrahydrofuran-3-yl)piperazine-1-carboxylate (58-2)

Tetrahydrofuran-3-ol (1 g, 11.35 mmol) and pyridine (1.18 g, 14.87 mmol) were dissolved in dichloromethane (30 mL) under an argon atmosphere, cooled to -10°C in an ice bath, and then trifluoromethanesulfonic anhydride (2.3 mL) was added dropwise thereto. The reaction mixture was stirred at -10°C for 30 minutes, then slowly warmed to room temperature, and stirred for another 3 hours to obtain a 3-trifluoromethanesulfonate-tetrahydrofuran solution. Benzyl 3-oxopiperazine-1-carboxylate (200 mg, 0.85 mmol) was dissolved in a mixed solvent of tetrahydrofuran (13 mL) and *N,N-*dimethylformamide (0.5 mL) under an argon atmosphere, and cooled to -60°C. Potassium bis(trimethylsilyl)amide (1.2 mL, 1.2 mmol) was added dropwise thereto, and the resulting mixture was kept at -60°C and stirred for 30 minutes before warmed to -10°C. After stirring for another 1 hour, the 3-trifluoromethanesulfonate-tetrahydrofuran solution (220 mg, 1.02 mmol) prepared above was added thereto, and the reaction was stirred at 20°C for another 16 hours. The completion of the reaction was monitored by TLC. The reaction mixture was quenched by adding pure water (30 mL), diluted by adding ethyl acetate (50 mL), and the phases were separated. The aqueous phase was extracted with ethyl acetate (20 mL × 3). The organic phases were combined, washed with saturated brine (50 mL), dried over anhydrous sodium sulfate, filtered, and the filtrate was concentrated under reduced pressure. The residue was purified by flash silica gel column chromatography (petroleum ether: ethyl acetate = 2:3) to obtain the title product (120 mg, colorless oil).

MS (ESI) m/z [M+H]⁺ = 305.1.

### Step 2: Synthesis of 1-(tetrahydrofuran-3-yl)piperazin-2-one (58-3)

Benzyl 3-oxo-4-(tetrahydrofuran-3-yl)piperazine-1-carboxylate (120 mg, 0.39 mmol) was dissolved in methanol (20 mL) and palladium/carbon (20 mg, 10%) was added to the reaction mixture. After the reaction system was replaced with hydrogen three times, the reaction mixture was stirred at room temperature for 16 hours under a hydrogen atmosphere. The completion of the reaction was monitored by TLC. The reaction mixture was filtered to remove the catalyst and concentrated under reduced pressure to obtain the title product **58-3** (60 mg, colorless oil).

MS (ESI) m/z [M+H]⁺ = 171.1.

### Step 3: 4-(6-((1-(4-(Difluoromethyl)phenyl)-4-methyl-1H-1,2,3-triazol-5-yl)methoxy)pyridazin-3-yl)-1-(tetrahydrofuran-3-yl)piperazin-2-one (58)

The experimental operation was as described in Example **1,** using **A8** and intermediate 58-3 as reactants to obtain the title product.

MS (ESI) m/z [M+H]⁺ = 486.2.

¹H NMR (400 MHz, DMSO-*d*₆) δ 7.78 (s, 4H), 7.37 (d, *J* = 9.6 Hz, 1H), 7.13 (t, *J* = 55.6 Hz, 1H), 7.04 (d, *J* = 9.6 Hz, 1H), 5.45 (s, 2H), 5.12-5.08 (m, 1H), 4.05 (s, 2H), 3.91-3.89 (m, 1H), 3.68-3.72 (m, 3H), 3.67-3.53 (m, 2H), 3.40-3.36 (m, 2H), 2.39 (s, 3H), 2.18-2.07 (m, 1H), 1.87-1.83 (m, 1H).

### Example 59:

### 4-(6-((1-(4-(Difluoromethyl)phenyl)-4-methyl-1H-1,2,3-triazol-5-yl)methoxy)pyridazin-3-yl)-1-(2-hydroxyethyl)piperazin-2-one (59)

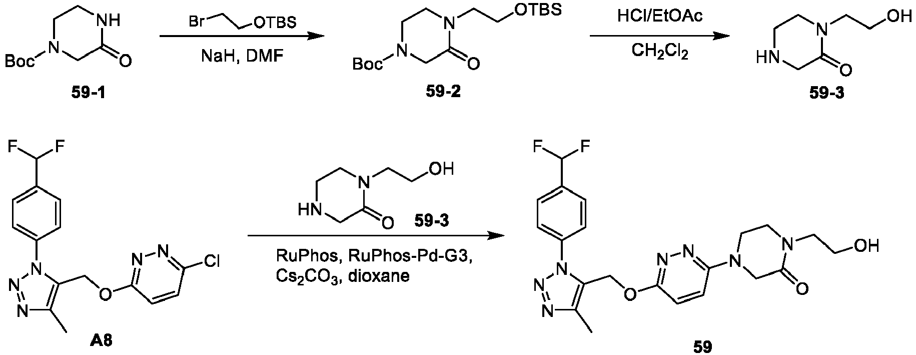

### Step 1: Synthesis of tert-butyl 4-(2-((tert-butyldimethylsilyl)oxy)ethyl)-3-oxopiperazine-1-carboxylate (59-2)

Compound **59-1** (1.0 g, 4.99 mmol) was dissolved in *N*,*N-*dimethylformamide (20.0 mL) under a nitrogen atmosphere, then sodium hydride (399 mg, 9.99 mmol, 60%) was added thereto in batches, and the mixture was stirred and reacted for half an hour. Compound (2-bromoethoxy)-*tert*-butyldimethylsilane (2.99 g, 12.5 mmol) was added thereto, and the reaction mixture was stirred and reacted for another 15 hours. The reaction mixture was quenched with water (10 mL), concentrated under reduced pressure to remove the solvent, diluted with water (100 mL), and extracted with dichloromethane (50 mL × 3). The organic phases were combined, dried over anhydrous sodium sulfate, filtered, and concentrated under reduced pressure. The crude product was purified by silica gel column chromatography (petroleum ether: ethyl acetate = 5:1 to 1:1) to obtain the title product (1.43 g, colorless oily liquid).

¹H NMR (400 MHz, CDCl₃) δ 4.08 (s, 2H), 3.81 (t, *J* = 5.2 Hz, 2H), 3.68-3.58 (m, 2H), 3.55-3.47 (m, 4H), 1.48 (s, 9H), 0.89 (s, 9H), 0.05 (s, 6H).

### Step 2: Synthesis of 1-(2-hydroxyethyl)piperazin-2-one (59-3)

Compound **59-2** (630 mg, 1.68 mmol) was dissolved in dichloromethane (5 mL) at room temperature, then a solution of hydrogen chloride in ethyl acetate (5 mL, 4.0 M) was added thereto, and the reaction was stirred for half an hour. The reaction mixture was concentrated under reduced pressure to obtain the title product (250 mg, yellow solid), which was directly used in the next reaction step without further purification.

¹H NMR (400 MHz, CDsOD) δ 3.85 (s, 2H), 3.79-3.73 (m, 4H), 3.50-3.29 (m, 4H)

### Step 3: Synthesis of 4-(6-((1-(4-(difluoromethyl)phenyl)-4-methyl-1H-1,2,3-triazol-5-yl)methoxy)pyridazin-3-yl)-1-(2-hydroxyethyl)piperazin-2-one (59)

The experimental operation was as described in Example **1,** using **A8** and intermediate 59-3 as reactants to obtain the title product.

MS (ESI) m/z [M+H]⁺ = 460.2.

¹H NMR (400 MHz, CDsOD) δ 7.77 (s, 4H), 7.34 (d, *J* = 9.6 Hz, 1H), 7.01 (d, *J* = 9.6 Hz, 1H), 6.89 (t, *J* = 55.6 Hz, 1H), 5.50 (s, 2H), 4.60 (s, 2H), 3.83-3.78 (m, 2H), 3.77-3.72 (m, 2H), 3.67-3.62 (m, 2H), 3.60-3.55 (m, 2H), 2.47 (s, 3H).

### Example 60:

### 4-(6-((1-(4-(Difluoromethyl)phenyl)-4-methyl-1H-1,2,3-triazol-5-yl)methoxy)pyridazin-3-yl)-1-(pyridin-2-ylmethyl)piperazin-2-one (60)

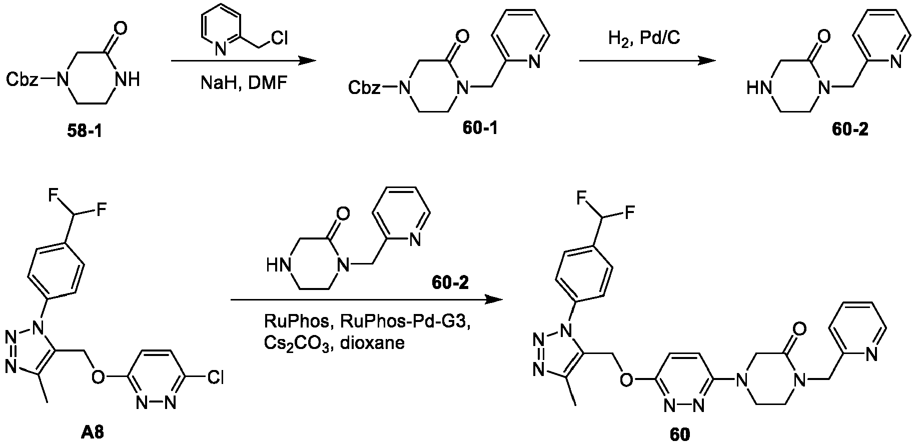

The experimental operation was as described in step 1 of Example **59** and steps 2 to 3 of Example **58,** using benzyl 3-oxopiperazine-1-carboxylate and 2-(chloromethyl)pyridine as starting materials, and undergoing the steps of alkylation, deprotection, and coupling reactions to obtain the title product.

MS (ESI) m/z [M+H]⁺ = 507.2.

¹H NMR (400MHz, DMSO-*d*₆) δ 8.51 (d, *J* = 8.0 Hz, 1H), 7.78 (s, 4H), 7.74 (dd, *J* = 7.6, 8.0Hz, 1H), 7.43 (d, *J* = 9.4 Hz, 1H), 7.28-7.24 (m, 2H), 7.13 (t, *J* = 55.6 Hz, 1H), 7.05 (d, *J* = 9.4 Hz, 1H), 5.46 (s, 2H), 4.65 (s, 2H), 4.14 (s, 2H), 3.78 (t, *J* = 5.2 Hz, 2H), 3.47 (t, *J* = 5.2 Hz, 2H), 2.39 (s, 3H).

### Example 61:

### 4-(6-((1-(4-(Difluoromethyl)phenyl)-4-methyl-1H-1,2,3-triazol-5-yl)methoxy)pyridazin-3-yl)-1-(2-(pyrrolidin-1-yl)ethyl)piperazin-2-one (61)

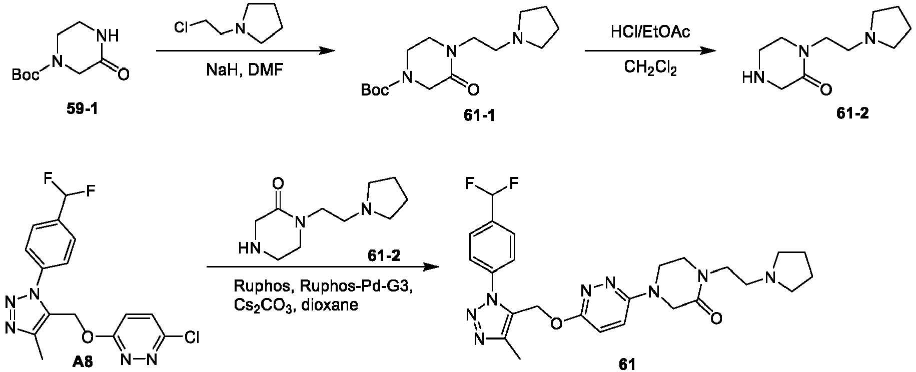

The experimental operation was as described in Example **59,** using *tert*-butyl *3-*oxopiperazine-1-carboxylate and 1-(2-chloroethyl)pyrrolidine as starting materials, and undergoing the steps of alkylation, deprotection, and coupling reactions to obtain the title product.

MS (ESI) m/z [M+H]⁺= 513.2.

¹H NMR (400 MHz, CDsOD) δ 7.76 (s, 4H), 7.34 (d, *J* = 9.6 Hz, 1H), 7.01 (d, *J*= 9.6 Hz, 1H), 6.90 (t, *J* = 55.6 Hz, 1H), 5.50 (s, 2H), 4.17 (s, 2H), 3.83 (t, *J* = 5.2 Hz, 2H), 3.69 (t, *J* = 6.4 Hz, 2H), 3.60-3.56 (m, 2H), 2.99-2.91 (m, 2H), 2.91-2.81 (m, 4H), 2.47 (s, 2H), 2.52-2.43 (m, 1H), 1.95-1.85 (m, 4H)

### Example 62:

### 4-(6-((1-(4-(Difluoromethyl)phenyl)-4-methyl-1H-1,2,3-triazol-5-yl)methoxy)pyridazin-3-yl)-1-(2,2,2-trifluoroethyl)piperazin-2-one (62)

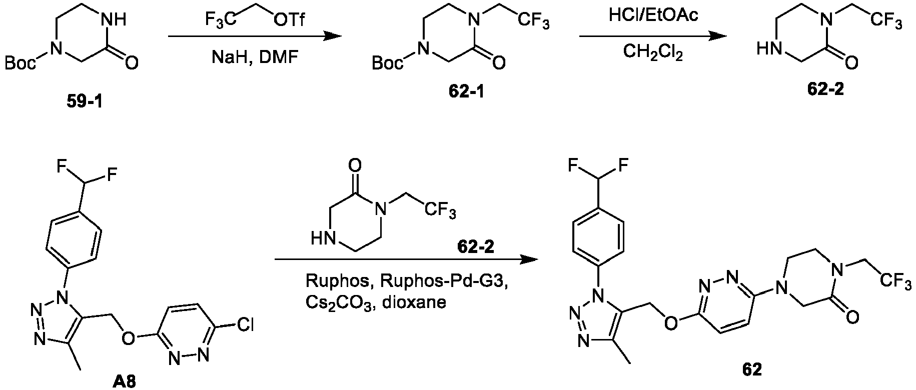

The experimental operation was as described in Example **59,** using *tert*-butyl 3-oxopiperazine-1-carboxylate and 2,2,2-trifluoroethyl trifluoromethanesulfonate as starting materials, and undergoing the steps of alkylation, deprotection, and coupling reactions to obtain the title product.

MS (ESI) m/z [M+H]⁺ = 498.1.

¹H NMR (400 MHz, CDsOD) δ 7.76 (s, 4H), 7.35 (d, *J* = 9.4 Hz, 1H), 7.03 (d, *J* = 9.4 Hz, 1H), 6.88 (t, *J* = 55.6 Hz, 1H), 5.51 (s, 2H), 4.26 (s, 2H), 4.24-4.19 (m, 4H), 3.85-3.82 (m, 2H), 3.70-3.67 (m, 2H), 2.48 (s, 3H).

### Example 63:

### 2-(4-(6-((1-(4-(Difluoromethyl)phenyl)-4-methyl-1H-1,2,3-triazol-5-yl)methoxy)pyridazin-3-yl)morpholin-2-yl)-N-ethylacetamide (63)

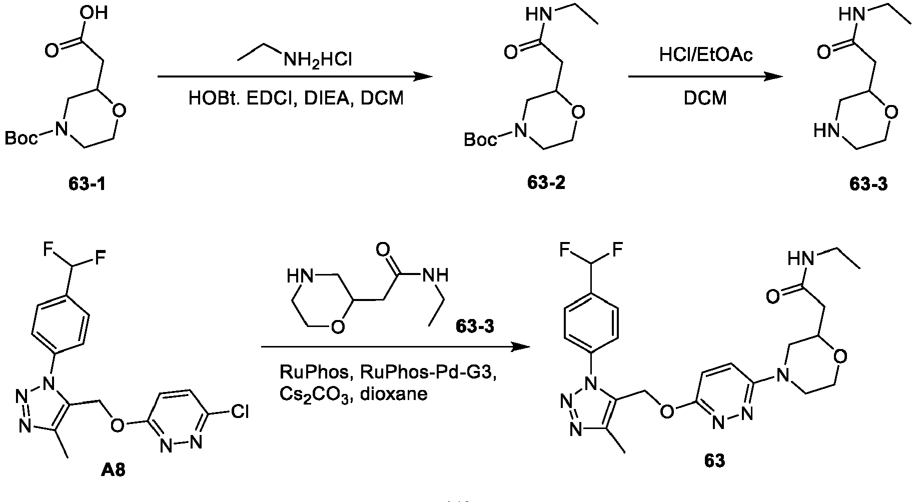

### Step 1: Synthesis of tert-butyl 2-(2-(ethylamino)-2-oxoethyl)morpholine-4-carboxylate (63-2)

Compound **63-1** (250 mg, 1.01 mmol), ethylamine hydrochloride (98.0 mg, 1.22 mmol), HOBt (179 mg, 1.32 mmol), EDCI (254 mg, 1.32 mmol), and diisopropylethylamine (395 mg, 3.05 mmol) were sequentially added to anhydrous dichloromethane (10.0 mL) at room temperature, and the system was replaced with nitrogen three times. The reaction mixture was stirred at room temperature for 12 hours. TLC showed that the reaction was completed. Dichloromethane (100 mL) was added to the reaction mixture, and the organic phase was washed with brine (15.0 mL × 3), dried over anhydrous sodium sulfate, filtered, and concentrated under reduced pressure. The crude product was purified by flash silica gel column chromatography (dichloromethane: methanol = 10:1) to obtain (260 mg, yellow oil).

¹H NMR (400 MHz, CDsOD) δ 3.92 (d, *J* = 12.8 Hz, 1H), 3.83-3.80 (m, 3H), 3.51-3.48 (m 1H), 3.22-3.18 (m, 2H), 2.39-2.27 (m, 2H), 1.46 (s, 9H), 1.11 (t, *J* = 7.2 Hz, 3H)

### Step 2: Synthesis of N-ethyl-2-(morpholin-2-yl)acetamide (63-3)

Compound **63-2** (260 mg, 0.955 mmol) was dissolved in anhydrous dichloromethane (4.0 mL) at room temperature, then hydrogen chloride/ethyl acetate (2 mL, 4.0 M) was added to the mixture, and the system was replaced with nitrogen three times. The reaction mixture was reacted at 16°C for 2 hours. TLC showed the starting material was completely reacted. The reaction mixture was concentrated under reduced pressure to obtain (160 mg, white solid, crude product).

¹H NMR (400 MHz, CDsOD) δ 4.16-4.01 (m, 2H), 3.84-3.81 (m, 1H), 3.36 (d, *J* = 12.4 Hz, 1H), 3.29-3.08 (m, 4H), 2.97 (t, *J=* 12.0 Hz, 1H), 2.47-2.37 (m, 2H), 1.12 (t, *J* = 7.2 Hz, 3H)

### Step 3: Synthesis of 2-(4-(6-((1-(4-(difluoromethyl)phenyl)-4-methyl-1H-1,2,3-triazol-5-yl)methoxy)pyridazin-3-yl)morpholin-2-yl)-N-ethylacetamide (63)

The experimental operation was as described in Example **1,** using **A8** and intermediate 63-3 as reactants to obtain the title product.

MS (ESI) m/z [M+H]⁺= 488.0.

¹H NMR (400 MHz, CDsOD) δ 7.76 (s, 4H), 7.34 (d, *J* = 9.6 Hz, 1H), 7.04-6.73 (m, 2H), 5.50 (s, 2H), 4.04-3.92 (m, 3H), 3.88 (d, *J* = 13.2 Hz, 1H), 3.72-3.70 (m, 1H), 3.27-3.18 (m, 2H), 2.99-2.97 (m, 1H), 2.73-2.71 (m, 1H), 2.47 (s, 3H), 2.45-2.35 (m, 2H), 1.13 (t, *J=* 7.2 Hz, 3H)

### Example 64:

### 1-(6-((1-(4-(Difluoromethyl)phenyl)-4-methyl-1H-1,2,3-triazol-5-yl)methoxy)pyridazin-3-yl)-N-methylpyrrolidine-3-sulfonamide (64)

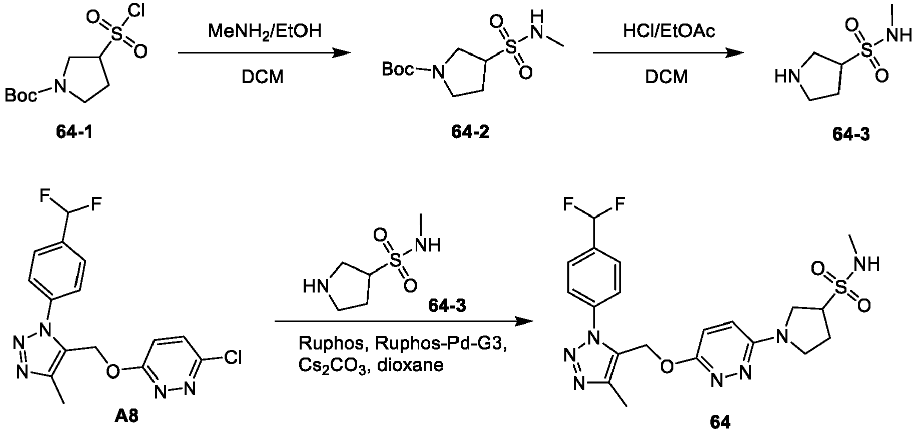

### Step 1: Synthesis of tert-butyl 3-(N-methylsulfamoyl)pyrrolidine-1-carboxylate (64-2)

Compound **64-1** (125 mg, 0.46 mmol) was dissolved in anhydrous dichloromethane (5.0 mL) at room temperature, then a solution of methylamine in ethanol (247 mg, 2.32 mmol, 30%) was added thereto, and the reaction was stirred for 1 hour. The reaction endpoint was monitored by TLC. The reaction mixture was concentrated under reduced pressure to obtain the title product (150 mg, yellow oily liquid).

¹H NMR (400 MHz, CDsOD) δ 3.95-3.84 (m, 1H), 3.72-3.64 (m, 2H), 3.57-3.51 (m, 1H), 3.45-3.36 (m, 1H), 2.55 (s, 3H), 2.34-2.24 (m, 2H), 1.47 (s, 9H).

### Step 2: Synthesis of N-methylpyrrolidine-3-sulfonamide (64-3)

The experimental operation was as described in step 2 in Example **59,** using **64-2** as the reactant to obtain the title product.

¹H NMR (400 MHz, CDsOD) δ 4.15-4.06 (m, 1H), 3.73-3.58 (m, 2H), 3.54-3.36 (m, 2H), 2.55 (s, 3H), 2.50-2.41 (m, 2H)

### Step 3: Synthesis of 1-(6-((1-(4-(difluoromethyl)phenyl)-4-methyl-1H-1,2,3-triazol-5-yl)methoxy)pyridazin-3-yl)-N-methylpyrrolidine-3-sulfonamide (64)

The experimental operation was as described in Example **1,** using **A8** and intermediate 64-3 as reactants to obtain the title product.

MS (ESI) m/z [M+H]⁺ = 480.1.

¹H NMR (400 MHz, CDsOD) *δ* 7.77 (s, 4H), 7.09-7.05 (m, 1H), 7.02-6.74 (m, 2H), 5.48 (s, 2H), 4.09-4.01 (m, 1H), 3.86-3.81 (m, 2H), 3.71-3.67 (m, 1H), 3.57-3.50 (m, 1H), 2.76 (s, 3H), 2.47 (s, 3H), 2.46-2.43 (m, 2H).

### Example 65:

### (R)-8-(6-((1-(4-(Difluoromethyl)phenyl)-4-methyl-1H-1,2,3-triazol-5-yl)methoxy)pyridazin-3-yl)hexahydro-1H-pyrazino[1,2-a]pyrazine-1,4(6H)-dione (65)

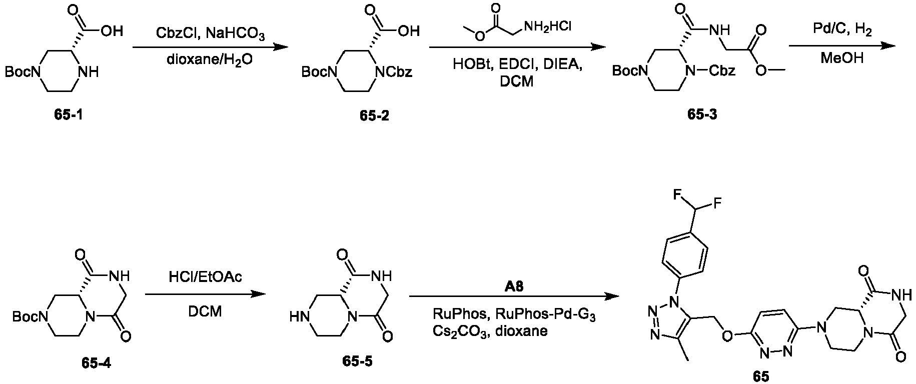

### Step 1: Synthesis of (R)-1-((benzyloxy)carbonyl)-4-(tert-butoxycarbonyl)piperazine-2-carboxylic acid (65-2)

Compound **65-1** (2.00 g, 8.70 mmol) was dissolved with dioxane (20.0 mL) and water (20.0 mL) at room temperature, then solid sodium bicarbonate (1.46 g, 17.4 mmol) and benzyl chloroformate (1.78 g, 10.4 mmol) were added thereto, and the reaction mixture was stirred at 25°C for 16 hours. Product generation was detected by LCMS. The reaction mixture was diluted with water (20.0 mL) and added with hydrochloric acid (1.0 M) to adjust the pH to about 3-4, and then extracted with ethyl acetate (20.0 mL × 3). The organic phases were combined and concentrated to dryness under reduced pressure. The crude product was purified by flash silica gel column chromatography (dichloromethane: methanol = 20:1) to obtain (2.36 g, colorless oil).

MS (ESI) m/z [M-t-Bu+H]⁺ = 306.1.

¹H NMR (400 MHz, CDCl₃) δ 7.35-7.31 (m, 5H), 5.22-5.13 (m, 2H), 4.83-4.58 (m, 2H), 3.99-3.88 (m, 2H), 3.32-3.08 (m, 2H), 2.86-2.83 (m, 1H), 1.43 (s, 9H).

### Step 2: Synthesis of 1-benzyl 4-tert-butyl (R)-2-((2-methoxy-2-oxoethyl)carbamoyl)piperazine-1,4-dicarboxylate (65-3)

Compound **65-2** (1.00 g, 2.70 mmol) was dissolved in anhydrous dichloromethane (25.0 mL) at room temperature, then HOBt (556 mg, 4.1 mmol), EDCI (789 mg, 4.1 mmol), diisopropylethylamine (1.06 g, 8.2 mmol), and methyl glycinate hydrochloride (0.4 g, 3.24 mmol) were added thereto, and the reaction mixture was stirred at 25°C for 3 hours. Product generation was detected by LCMS. The reaction mixture was diluted with water (25.0 mL) and extracted with dichloromethane (25.0 mL × 3). The organic phases were combined and concentrated to dryness under reduced pressure. The crude product was purified by flash silica gel column chromatography (dichloromethane: methanol = 10:1) to obtain (800 mg, colorless oil).

MS (ESI) m/z [M-t-Bu+H]⁺ = 380.1.

¹H NMR (400 MHz, CDCl₃) δ 7.35-7.26 (m, 5H), 6.73-6.58 (m, 1H), 5.19-5.15 (m, 2H), 4.72-4.49 (m, 2H), 4.05-3.92 (m, 2H), 3.91-3.90 (m, 2H), 3.73 (s, 3H), 3.25-3.12 (m, 2H), 3.00-2.96 (m, 1H), 1.44 (s, 9H).

### Step 3: Synthesis of tert-butyl (R)-6,9-dioxohexahydro-1H-pyrazino[1,2-a]pyrazine-2(6H)-carboxylate (65-4)

Compound **65-3** (800 mg, 1.84 mmol) was dissolved in methanol (10.0 mL) at room temperature, then 10% wet palladium/carbon (100 mg) was added thereto. The reaction mixture was replaced with hydrogen three times, and then reacted at 20°C for 16 hours under a hydrogen atmosphere (15 psi). Product generation was detected by LCMS. The reaction mixture was filtered, and the filter cake was washed with methanol (25.0 mL). The organic phases were combined and concentrated to dryness under reduced pressure. The crude product was purified by flash silica gel column chromatography (dichloromethane: methanol = 20:1) to obtain (300 mg, white solid).

MS (ESI) m/z [M-Boc+H]⁺ = 214.1.

¹H NMR (400 MHz, CDCl₃) δ 6.95 (s, 1H), 4.56-4.53 (m, 2H), 4.15-4.07 (m, 3H), 3.96 (dd, *J* = 4.0, 10.8 Hz, 1H), 2.84-2.73 (m, 2H), 2.70-2.67 (m, 1H), 1.47 (s, 9H).

### Step 4: Synthesis of (R)-hexahydro-1H-pyrazino[1,2-a]pyrazine-1,4-(6H)-dione (65-5)

Compound **65-4** (300 mg, 1.11 mmol) was dissolved in dichloromethane (3.0 mL) at room temperature, then hydrochloric acid/ethyl acetate solution (1.5 mL, 4.0 M) was slowly added dropwise thereto, and the reaction mixture was stirred at 20°C for 3 hours. LCMS detected that all raw materials were consumed. The reaction mixture was directly concentrated to obtain the hydrochloride of the title product (212 mg, white solid, crude product).

¹H NMR (400 MHz, CDCl₃) δ 4.87-4.68 (m, 1H), 4.45-4.41 (m, 1H), 4.03-4.02 (m, 2H), 3.79-3.75 (m, 1H), 3.49 (d, *J* = 11.6Hz, 1H), 3.25 (t, *J* = 12.4 Hz, 1H), 3.10-3.01 (m, 2H).

### Step 5: Synthesis of (R)-8-(6-((1-(4-(difluoromethyl)phenyl)-4-methyl-1H-1,2,3-triazol-5-yl)methoxy)pyridazin-3-yl)hexahydro-1H-pyrazino[1,2-a]pyrazine-1,4(6H)-dione (65)

The experimental operation was as described in Example **1,** using **A8** and intermediate **65-5** as reactants to obtain the title product.

MS (ESI) m/z [M+H]⁺ = 485.1.

¹H NMR (400 MHz, CD₃OD) δ 7.77 (s, 4H), 7.43 (d, *J* = 9.4 Hz, 1H), 7.03-6.75 (m, 2H), 5.51 (s, 2H), 4.59-4.54 (m, 2H), 4.23-4.19 (m, 2H), 4.02 (s, 2H), 3.08-3.02 (m, 1H), 3.00-2.95 (m, 2H), 2.48 (s, 3H).

### Example 66:

### 3-(6-((1-(4-(Difluoromethyl)phenyl)-4-methyl-1H-1,2,3-triazol-5-yl)methoxy)pyridazin-3-yl)-5-(hydroxymethyl)oxazolidin-2-one (66)

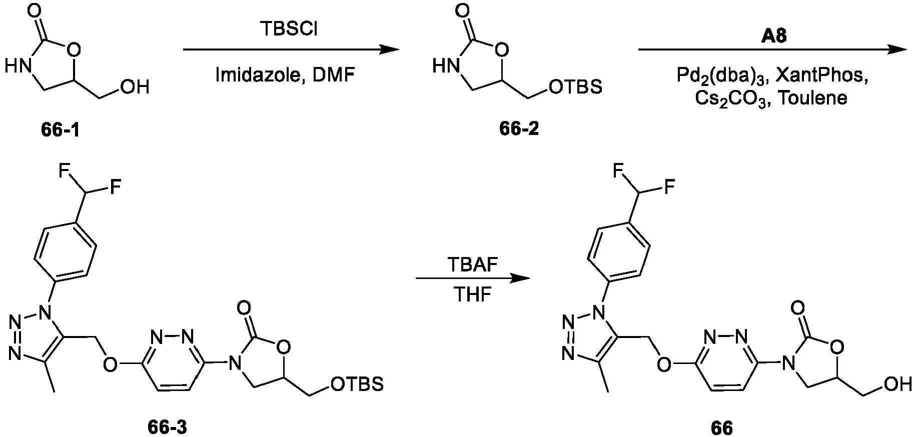

### Step 1: Synthesis of 5-(((tert-butyldimethylsilyl)oxy)methyl)oxazolidin-2-one (66-2)

**66-1** (300 mg, 2.56 mmol) and imidazole (348 mg, 5.12 mmol) were dissolved in *N,N-*dimethylformamide (10 mL) under an argon atmosphere, then *tert*-butylchlorodimethylsilane (463 mg, 3.07 mmol) was added thereto, and the reaction was stirred at 25°C for 2 hours. The completion of the reaction was monitored by TLC. The reaction mixture was quenched by adding water (50 mL) and extracted with ethyl acetate (100 mL × 2). The organic phases were combined, washed with saturated brine (50 mL), dried over anhydrous sodium sulfate, filtered, and the filtrate was concentrated under reduced pressure. The title product (330 mg, yellow oil) was obtained.

MS (ESI) m/z [M+H]⁺ = 232.1.

### Step 2: Synthesis of 5-(((tert-butyldimethylsilyl)oxy)methyl)-3-(6-((1-(4-(difluoromethyl)phenyl)-4-methyl-1H-1,2,3-triazol-5-yl)methoxy)pyridazin-3-yl)oxazolidin-2-one (66-3)

The experimental operation was as described in Example **18,** using **A8** and **66-2** as reactants to react in toluene to obtain the title product.

MS (ESI) m/z [M+H]⁺ = 547.2.

### Step 3: Synthesis of 3-(6-((1-(4-(difluoromethyl)phenyl)-4-methyl-1H-1,2,3-triazol-5-yl)methoxy)pyridazin-3-yl)-5-(hydroxymethyl)oxazolidin-2-one (66)

**66-3** (60 mg, 0.11 mmol) was dissolved in tetrahydrofuran (5 mL) under an argon atmosphere, then a solution of tetrabutyl ammonium fluoride in tetrahydrofuran (3 mL, 1.5 M) was added thereto, and the reaction was stirred at 25°C for 2 hours. The completion of the reaction was monitored by TLC. The reaction mixture was added with water (30 mL) and extracted with ethyl acetate (50 mL × 2). The organic phases were combined, washed with saturated brine (50 mL), dried over anhydrous sodium sulfate, filtered, and the filtrate was concentrated under reduced pressure. The residue was purified sequentially by preparative thin-layer chromatography (petroleum ether: ethyl acetate = 0:1) and reversed-phase C18 flash preparative chromatography (acetonitrile: water = 0 to 40%) to obtain the title product (13 mg, white solid).

MS (ESI) m/z [M+H]⁺ = 433.1.

¹H NMR (400 MHz, DMSO-*d*₆) δ 8.32 (d, *J* = 9.8 Hz, 1H), 7.78 (s, 4H), 7.26 (d, *J* = 9.8 Hz, 1H), 7.13 (t, *J* = 55.6 Hz, 1H), 5.55 (s, 2H), 5.24-5.22 (m, 1H), 4.76-4.74 (m, 1H), 4.19 (t, *J* = 9.6 Hz, 1H), 4.00-3.98 (m, 1H), 3.70-3.68 (m, 1H), 3.61-3.53 (m, 1H).

### Example 67:

### 1-(2-Aminoethyl)-4-(6-((1-(4-(difluoromethyl)phenyl)-4-methyl-1H-1,2,3-triazol-5-yl)methoxy)pyridazin-3-yl)piperazin-2-one (67)

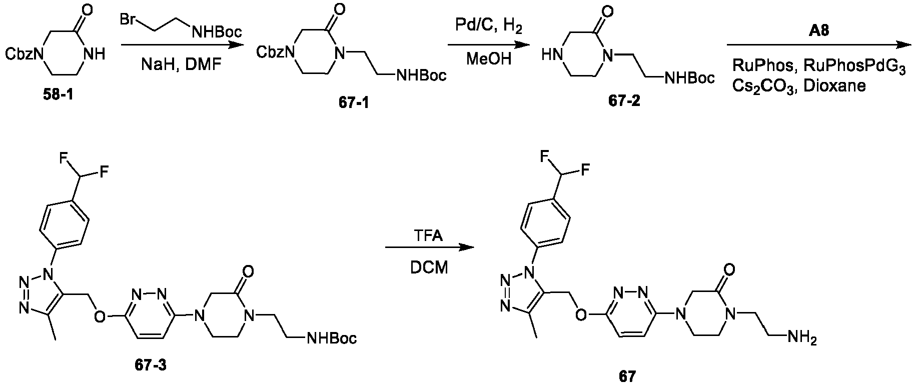

### Step 1: Synthesis of benzyl 4-(2-(((tert-butoxycarbonyl)-1-2-azaalkyl)ethyl)-3-oxopiperazine-1-carboxylate (67-1)

**58-1** (700 mg, 2.99 mmol) was dissolved in *N*,*N-*dimethylformamide (20 mL) under an argon atmosphere, then sodium hydride (160 mg, 6.58 mmol, 60%) was added thereto, and the reaction was stirred at 25°C for 10 minutes. *tert*-Butyl bromoethylcarbamate (740 mg, 3.29 mmol) was added thereto, and the reaction was stirred at 25°C for another 16 hours. The completion of the reaction was monitored by TLC. The reaction mixture was quenched by adding water (50 mL) and extracted with ethyl acetate (100 mL × 2). The organic phases were combined, washed with saturated brine (100 mL), dried over anhydrous sodium sulfate, filtered, and the filtrate was concentrated under reduced pressure. The residue was purified by reversed-phase C18 flash preparative chromatography (acetonitrile: water = 0 to 40%) to obtain the title product (200 mg, yellow oily liquid).

MS (ESI) m/z [M+H]⁺ = 378.2.

### Step 2: Synthesis of tert-butyl (2-(2-oxopiperazin-1-yl)ethyl)carbamate (67-2)

**67-1** (150 mg, 0.4 mmol) was dissolved in methanol (20 mL) under an argon atmosphere, then palladium on carbon (40 mg) was added thereto. The system was replaced with hydrogen three times, and the reaction was stirred at 25°C for another 16 hours under a hydrogen atmosphere. The completion of the reaction was monitored by TLC. The reaction mixture was filtered, then the filter cake was washed with methanol (10 mL × 2), and the filtrate was concentrated under reduced pressure to obtain the title product (80 mg, yellow oily liquid).

MS (ESI) m/z [M+H]⁺ = 244.1.

### Step 3: Synthesis of tert-butyl (2-(4-(6-((1-(4-(difluoromethyl)phenyl)-4-methyl-1H-1,2,3-triazol-5-yl)methoxy)pyridazin-3-yl)-2-oxopiperazin-1-yl)ethyl)carbamate (67-3)

The experimental operation was as described in Example **1,** using intermediate **A8** and intermediate **67-2** as reactants to obtain the title product.

MS (ESI) m/z [M+H]⁺ = 559.2.

### Step 4: Synthesis of 1-(2-aminoethyl)-4-(6-((1-(4-(difluoromethyl)phenyl)-4-methyl-1H-1,2,3-triazol-5-yl)methoxy)pyridazin-3-yl)piperazin-2-one (67)

**67-3** (40 mg, 0.072 mmol) was dissolved in dichloromethane (5 mL) under an argon atmosphere, then trifluoroacetic acid (2 mL) was added thereto, and the reaction was stirred at 25°C for 2 hours. The completion of the reaction was monitored by TLC. The reaction mixture was quenched by adding saturated sodium bicarbonate solution (50 mL) and extracted with ethyl acetate (50 mL × 2). The organic phases were combined, dried over anhydrous sodium sulfate, filtered, and the filtrate was concentrated under reduced pressure. The residue was purified by reversed-phase C18 flash preparative chromatography (acetonitrile: water = 0 to 40%) to obtain the title product (2 mg, white solid).

MS (ESI) m/z [M+H]⁺ = 459.2.

¹H NMR (400MHz, DMSO-*d*₆) δ 7.78 (s, 4H), 7.40 (d, *J* = 9.6 Hz, 1H), 7.13 (t, *J* = 55.6 Hz, 1H), 7.04 (d, *J* = 9.6 Hz, 1H), 5.44 (s, 2H), 4.45 (br. s, 2H), 4.02 (s, 2H), 3.73-3.71 (m, 2H), 3.47-3.45 (m, 2H), 3.30-3.27 (m, 2H), 2.66-2.65 (m, 2H), 2.38 (s, 3H).

### Example 68:

### 2-(6-((1-(4-(Difluoromethyl)phenyl)-4-methyl-1H-1,2,3-triazol-5-yl)methoxy)pyridazin-3-yl)hexahydro-1H-pyrazino[1,2-a]pyrazin-4-(6H)-one (68)

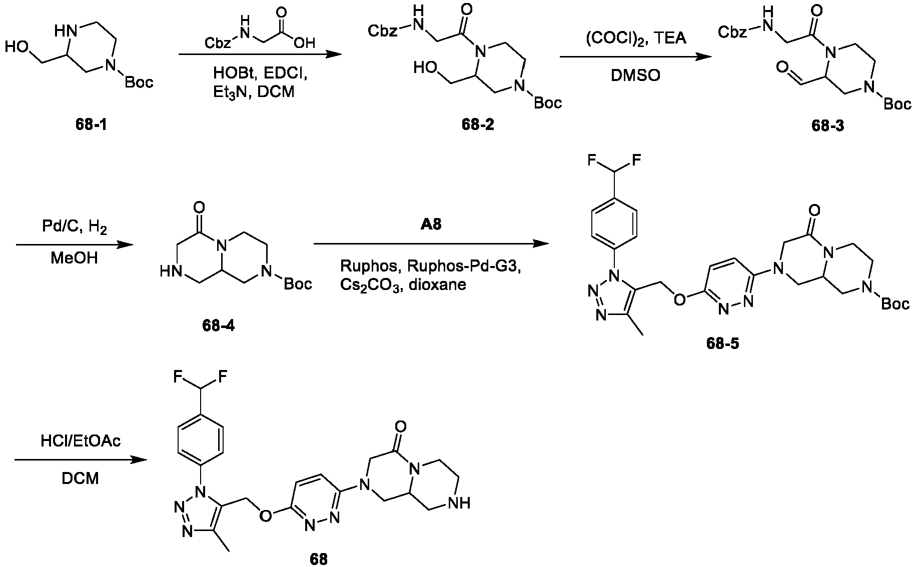

### Step 1: Synthesis of tert-butyl 4-(2-(((benzyloxy)carbonyl)amino)acetyl)-3-(hydroxymethyl)piperazine-1-carboxylate (68-2)

**68-1** (1000 mg, 4.62 mmol), HOBt (625 mg, 4.63 mol), EDCI (886 mg, 4.62 mmol), triethylamine (936 mg, 9.25 mmol), and N-carbobenzyloxyglycine (645 mg, 3.08 mmol) were sequentially dissolved in dichloromethane (20 mL) at room temperature, and the reaction mixture was reacted at 20°C for 16 hours. Product generation was shown by LCMS. The reaction mixture was diluted with water (20 mL) and extracted with dichloromethane (20 mL × 3). The organic phases were combined, concentrated under reduced pressure, and the residue was purified by flash silica gel column chromatography (methanol: dichloromethane = 1:20) to obtain the title product (800 mg, white solid).

(ESI) m/z [M+H]⁺ = 408.2.

¹H NMR (400 MHz, CDCl₃) δ 7.36-7.30 (m, 5H), 5.79 (s, 1H), 5.11 (s, 2H), 4.63-4.36 (m, 1H), 4.14-3.99 (m, 4H), 3.82-3.26 (m, 4H), 2.96-2.86 (m, 2H), 1.46 (s, 9H).

### Step 2: Synthesis of tert-butyl 4-(2-(((benzyloxy)carbonyl)amino)acetyl)-3-formylpiperazine-1-carboxylate (68-3)

Oxalyl chloride (206 mg, 1.62 mmol) was slowly added dropwise to a solution of dimethyl sulfoxide (230 mg, 2.94 mmol) in anhydrous dichloromethane (5.0 mL) at -60°C, and the mixture was stirred for 5 minutes. A solution of compound **68-2** (600 mg, 1.47 mmol) in anhydrous dichloromethane (5.0 mL) was slowly added dropwise to the above mixture, and the resulting mixture was stirred for another 15 minutes. Triethylamine (745 mg, 7.36 mmol) was added dropwise thereto, and the reaction mixture was slowly warmed to room temperature and stirred for 1 hour. TLC showed that the reaction was completed. The reaction was quenched by adding water (10.0 mL) and extracted with dichloromethane (10 mL × 3). The organic phases were combined and concentrated under reduced pressure. The residue was purified by flash silica gel column chromatography (methanol: dichloromethane = 1:20) to obtain the title product (300 mg, colorless oil), which was used directly in the following step without further purification.

### Step 3: Synthesis of tert-butyl 6-oxo-hexahydro-1H-pyrazino[1,2-a]pyrazine-2(6H)-carboxylate (68-4)

**68-3** (150 mg, 0.74 mol) was dissolved in methanol (5 mL) at room temperature, and then 10% wet palladium/carbon (30 mg) was added thereto. The system was replaced with hydrogen three times, and the reaction mixture was stirred at 20°C for 16 hours under a hydrogen atmosphere. TLC showed that the reaction was completed. The reaction mixture was filtered, and the filter cake was washed with methanol (10 mL). The filtrates were combined and concentrated under reduced pressure to obtain the title product (100 mg, colorless oil).

MS (ESI) m/z [M+H]⁺ = 256.2.

### Step 4: Synthesis of tert-butyl 8-(6-((1-(4-(difluoromethyl)phenyl)-4-methyl-1H-1,2,3-triazol-5-yl)methoxy)pyridazin-3-yl)-6-oxohexahydro-1H-pyrazino[1,2-a]pyrazine-2-(6H)-carboxylate (68-5)

The experimental operation was as described in Example **1,** using **A8** and intermediate 68-4 as reactants to obtain the title product.

MS (ESI) m/z [M+H]⁺ = 571.2.

### Step 5: Synthesis of 2-(6-((1-(4-(difluoromethyl)phenyl)-4-methyl-1H-1,2,3-triazol-5-yl)methoxy)pyridazin-3-yl)hexahydro-1H-pyrazino[1,2-a]pyrazin-4-(6H)-one (68)

**68-5** (137.0 mg, 0.24 mmol, 84%) was dissolved in anhydrous dichloromethane (4.0 mL) at room temperature, then hydrochloric acid/ethyl acetate solution (2 mL, 4.0 M) was added dropwise thereto, and the reaction mixture was stirred at 20°C for 2 hours. LCMS showed that the reaction was completed. The reaction mixture was concentrated under reduced pressure to remove the solvent, and the crude product was separated by high performance liquid chromatography (chromatographic column: Phenomenex C18 80 * 40 mm * 3 µm; mobile phase: water (0.05% ammonia hydroxide) - acetonitrile]; B%: 23% to 53%, 8 min; flow rate: 30 mL/min) to obtain the title product (25.8 mg, light yellow solid).

MS (ESI) m/z [M+H]⁺ = 471.2.

¹H NMR (400 MHz, CDsOD) δ 7.76 (s, 4H), 7.39 (d, *J* = 9.4 Hz, 1H), 7.01 (d, *J* = 9.4 Hz, 1H), 6.89 (t, *J* = 55.6 Hz, 1H), 5.50 (s, 2H), 4.51-4.48 (m, 1H), 4.39-4.35 (m, 1H), 4.16-4.14 (m, 1H), 4.01-3.96 (m, 1H), 3.65-3.61 (m, 1H), 3.27-3.24 (m, 1H), 3.04-3.01 (m, 2H), 2.69-2.65 (m, 2H), 2.56-2.51 (m, 1H), 2.47 (s, 3H).

### Example 69:

### 2-(6-((1-(4-(Difluoromethyl)phenyl)-4-methyl-1H-1,2,3-triazol-5-yl)methoxy)pyridazin-3-yl)-8-methylhexahydro-1H-pyrazino[1,2-a]pyrazin-4(6H)-one (69)

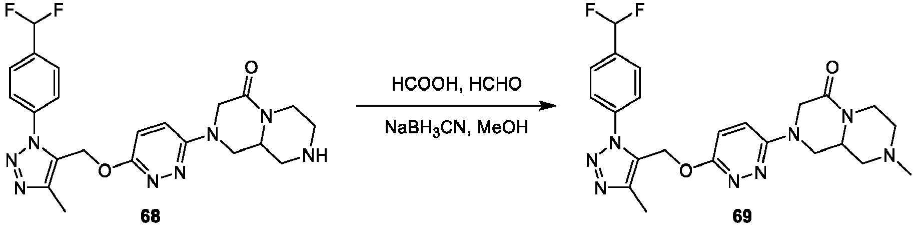

Example **68** (70.0 mg, 0.17 mmol, hydrochloride) was dissolved in anhydrous methanol (2.0 mL) at room temperature, and then triethylamine (34.5 mg, 0.34 mol) was added thereto. The reaction mixture was stirred at 20°C for 30 minutes, and then concentrated under reduced pressure. The residue was dissolved in anhydrous methanol (2.0 mL), then 37% formaldehyde aqueous solution (138 mg, 1.7 mmol) and formic acid (78.3 mg, 1.7 mmol) were sequentially added thereto. The reaction mixture was replaced with nitrogen and stirred at 20°C for 1 hour. Sodium cyanoborohydride (32 mg, 0.51 mmol) was added thereto, and the reaction mixture was stirred for another 16 hours. LCMS showed that the reaction was completed. The reaction mixture was concentrated under reduced pressure, and the residue was diluted with water (10 mL) and extracted with dichloromethane (10 mL × 3). The organic phases were combined, concentrated under reduced pressure, and the residue was separated by high performance liquid chromatography (chromatographic column: Boston Green ODS 150 * 30 mm * 5 µm; mobile phase: water (0.225% trifluoroacetic acid) - acetonitrile]; B%: 10% to 40%, 7 min; flow rate: 35 mL/min) to obtain the title product (16.0 mg, white solid).

MS (ESI) m/z [M+H]⁺ = 485.2.

¹H NMR (400 MHz, CDsOD) δ 7.76 (s, 4H), 7.39 (d, *J* = 9.4 Hz, 1H), 7.01 (d, *J* = 9.4 Hz, 1H), 6.89 (t, *J* = 55.6 Hz, 1H), 5.50 (s, 2H), 4.55-4.52 (m, 1H), 4.39-4.35 (m, 1H), 4.23-4.20 (m, 1H), 3.99-3.95 (m, 1H), 3.75-3.71 (m, 1H), 3.32-3.29 (m, 1H), 3.01-2.91 (m, 2H), 2.83-2.80 (m, 1H), 2.47 (s, 3H), 2.36 (s, 3H), 2.08-2.05 (m, 1H), 2.00-1.95 (m, 1H).

### Example 70:

### 1-(6-((1-(4-(Difluoromethyl)phenyl)-4-methyl-1H-1,2,3-triazol-5-yl)methoxy)pyridin-3-yl)piperazin-2-one (70)

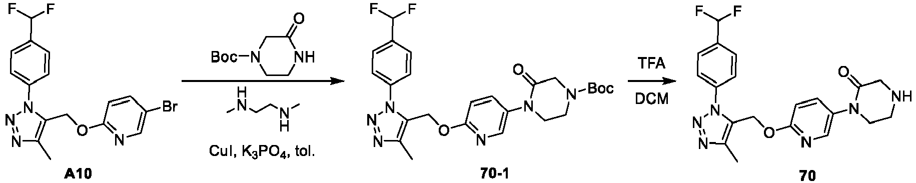

### Step 1: Synthesis of tert-butyl 4-(6-((1-(4-(difluoromethyl)phenyl)-4-methyl-1H-1,2,3-triazol-5-yl)methoxy)pyridin-3-yl)-3-oxopiperazine-1-carboxylate (70-1)

**A10** (0.13 g, 0.33 mmol) and *tert*-butyl 3-oxopiperazine-1-carboxylate (66 mg, 0.33 mmol) were dissolved in toluene (1 mL) under an argon atmosphere, then *N1,N2-*dimethylethane-1,2-diamine (29 mg, 0.033 mmol), potassium phosphate (140 mg, 0.66 mmol), and cuprous iodide (63 mg, 0.033 mmol) were sequentially added thereto, and the reaction mixture was stirred at 110°C for 16 hours. The completion of the reaction was monitored by TLC. The reaction mixture was diluted with dichloromethane (100 mL) and filtered. The filtrate was concentrated under reduced pressure. The residue was purified by preparative thin-layer chromatography (petroleum ether: ethyl acetate = 0:1) to obtain the title product (85 mg, yellow solid).

MS (ESI) m/z [M+H]⁺ = 515.2.

### Step 2: 1-(6-((1-(4-(Difluoromethyl)phenyl)-4-methyl-1H-1,2,3-triazol-5-yl)methoxy)pyridin-3-yl)piperazin-2-one (70)

**70-1** (82 mg, 0.16 mmol) was dissolved in dichloromethane (5 mL) under an argon atmosphere, then trifluoroacetic acid (2 mL) was added dropwise thereto, and the reaction was stirred at room temperature for 2 hours. The completion of the reaction was monitored by LCMS. The reaction mixture was poured into saturated sodium bicarbonate solution (100 mL) at 0°C and extracted with dichloromethane (80 mL × 2, containing 10% methanol). The organic phase was dried over anhydrous sodium sulfate and concentrated under reduced pressure. The residue was purified by reversed-phase C18 flash preparative chromatography (acetonitrile: water = 0 to 40%) to obtain the title product (33 mg, yellow solid).

MS (ESI) m/z [M+H]⁺ = 416.2.

¹H NMR (400 MHz, DMSO-*d*₆) δ 8.06 (d, *J* = 2.6 Hz, 1H), 7.80 (s, 4H), 7.68 (dd, *J* = 2.6, 8.8 Hz, 1H), 7.22 (t, *J* = 55.6 Hz, 1H), 6.82 (d, *J* = 8.8 Hz, 1H), 5.41 (s, 2H), 3.55 (t, *J*= 5.2 Hz, 2H), 3.37 (s, 2H), 2.99 (t, *J* = 5.2 Hz, 2H), 2.76 (br. s, 1H), 2.40 (s, 3H).

### Example 71:

### 1-(6-((1-(4-(Difluoromethyl)phenyl)-4-methyl-1H-1,2,3-triazol-5-yl)methoxy)pyridazin-3-yl)piperazin-one (71)

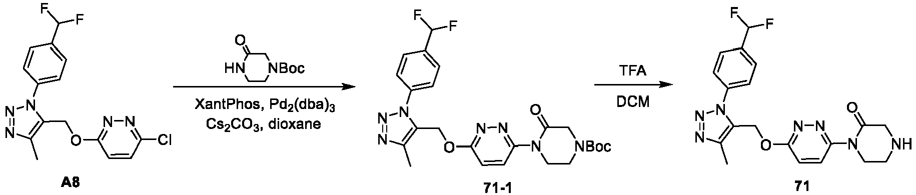

### Step 1: Synthesis of tert-butyl 4-(6-((1-(4-(difluoromethyl)phenyl)-4-methyl-1H-1,2,3-triazol-5-yl)methoxy)pyridazin-3-yl)-3-oxopiperazine-1-carboxylate (71-1)

The experimental operation was as described in Example **18,** using **A8** and *tert*-butyl 3-oxopiperazine-1-carboxylate as reactants to obtain the title product.

MS (ESI) m/z [M+H]⁺ = 516.2.

### Step 2: Synthesis of 1-(6-((1-(4-(difluoromethyl)phenyl)-4-methyl-1H-1,2,3-triazol-5-yl)methoxy)pyridazin-3-yl)piperazin-one (71)

**71-1** (50 mg, 0.097 mmol) was dissolved in dichloromethane (5 mL) under an argon atmosphere, then trifluoroacetic acid (2 mL) was added thereto, and the reaction was stirred at 25°C for 2 hours. The completion of the reaction was monitored by TLC. The reaction mixture was quenched by adding saturated sodium bicarbonate solution (30 mL) and extracted with ethyl acetate (30 mL × 2). The organic phases were combined, washed with saturated brine (50 mL), dried over anhydrous sodium sulfate, filtered, and the filtrate was concentrated under reduced pressure. The residue was purified sequentially by preparative thin-layer chromatography (petroleum ether: ethyl acetate = 0:1) and reversed-phase C18 flash preparative chromatography (acetonitrile: water = 0 to 40%) to obtain the title product (13 mg, white solid).

MS (ESI) m/z [M+H]⁺ = 416.2.

¹H NMR (400 MHz, DMSO-*d*₆) δ 7.91 (d, *J* = 9.4 Hz, 1H), 7.78 (s, 4H), 7.18 (d, *J* = 9.4 Hz, 1H), 7.12 (t, *J* = 55.6 Hz, 1H), 5.56 (s, 2H), 3.83 (t, *J* = 5.4 Hz, 2H), 3.43 (s, 2H), 3.01 (t, *J* = 5.4 Hz, 2H), 2.40 (s, 3H).

### Example 72:

### 4-(6-((1-(5-Chloropyridin-2-yl)-4-methyl-1H-1,2,3-triazol-5-yl)methoxy)pyridazin-3-yl)piperazin-2-one (72)

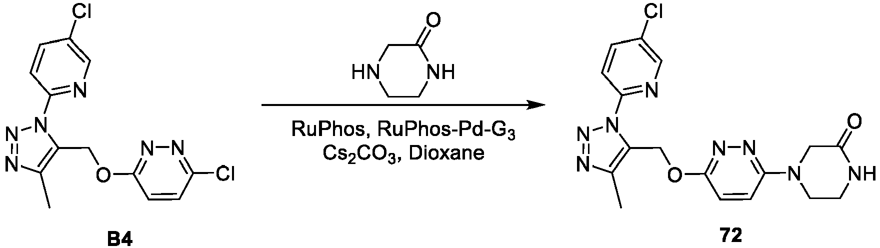

The experimental operation was as described in Example **1,** using intermediate **B4** and 2-piperazinone as reactants to obtain the title product.

MS (ESI) m/z [M+H]⁺ = 401.2.

¹H NMR (400 MHz, DMSO-*d*₆) δ 8.59 (d, *J* = 2.4 Hz, 1H), 8.24 (dd, *J* = 8.8, 2.4 Hz, 1H), 8.07 (s, 1H), 8.00 (d, *J* = 8.8 Hz, 1H), 7.37 (d, *J* = 9.6 Hz, 1H), 6.98 (d, *J* = 9.6 Hz, 1H), 5.79 (s, 2H), 3.96 (s, 2H), 3.65 (t, *J* = 5.2 Hz, 2H), 3.27 (t, *J* =5.2Hz, 2H), 2.38 (s, 3H).

### Example 73:

### 4-(6-((1-(5-Fluoropyridin-2-yl)-4-methyl-1H-1,2,3-triazol-5-yl)methoxy)pyridazin-3-yl)piperazin-2-one (73)

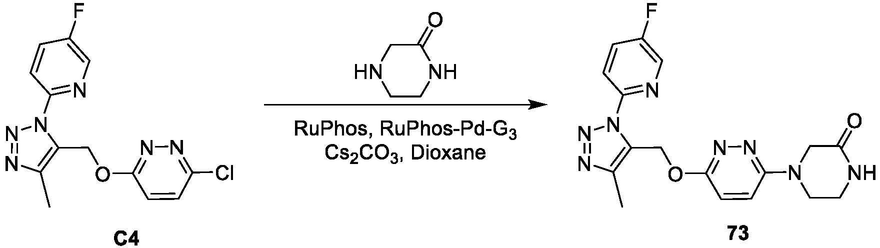

The experimental operation was as described in Example **1,** using intermediate **C4** and 2-piperazinone as reactants to obtain the title product.

MS (ESI) m/z [M+H]⁺ = 385.2.

¹H NMR (400 MHz, DMSO-*d*₆) δ 8.55 (s, 1H), 8.10-7.97 (m, 3H), 7.36 (d, *J* = 9.8 Hz, 1H), 6.96 (d, *J* = 9.8 Hz, 1H), 5.74 (s, 2H), 3.95 (s, 2H), 3.65-3.67 (m, 2H), 3.28-3.24 (m, 2H), 2.37 (s, 3H).

### Example 74:

### 4-(6-((1-(6-(Difluoromethyl)pyridin-3-yl)-4-methyl-1H-1,2,3-triazol-5-yl)methoxy)pyridazin-3-yl)piperazin-2-one (74)

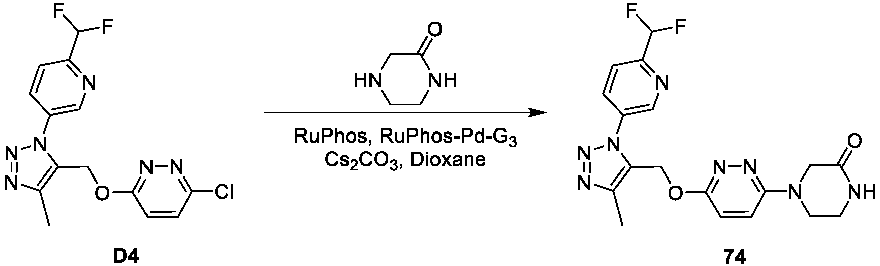

The experimental operation was as described in Example **1,** using intermediate **D4** and 2-piperazinone as reactants to obtain the title product.

MS (ESI) m/z [M+H]⁺ = 417.2.

¹H NMR (400 MHz, DMSO-*d*₆) δ 8.97 (s, 1H), 8.32 (d, *J=* 8.6 Hz, 1H), 8.06 (s, 1H), 7.94 (d, *J* = 8.2 Hz, 1H), 7.38 (d, *J* = 9.6 Hz, 1H), 7.06 (t, *J* = 54.8 Hz, 1H), 7.01 (d, *J* = 9.6 Hz, 1H), 5.49 (s, 2H), 3.96 (s, 2H), 3.65-3.63 (m, 2H), 3.27-3.25 (m, 2H), 2.41 (s, 3H).

### Example 75:

### 4-(6-((1-(4-Cyanophenyl)-4-methyl-1H-1,2,3-triazol-5-yl)methoxy)pyridazin-3-yl)piperazin-2-one (75)

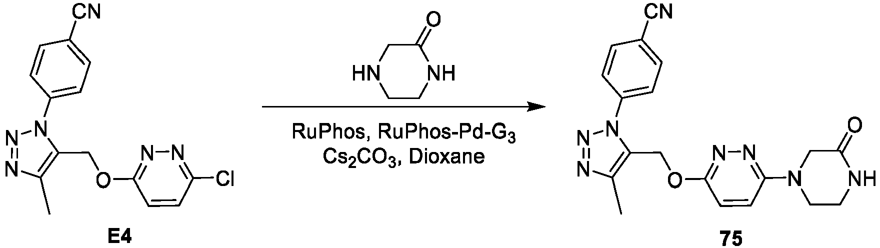

The experimental operation was as described in Example **1**, using intermediate **E4** and 2-piperazinone as reactants to obtain the title product.

MS (ESI) m/z [M+H]⁺ = 391.2.

¹H NMR (400 MHz, DMSO-*d*₆) δ 8.09-8.07 (m, 3H), 7.86 (d, *J* = 8.4 Hz, 2H), 7.40 (d, *J* = 9.6 Hz, 1H), 7.01 (d, *J* = 9.6 Hz, 1H), 5.49 (s, 2H), 3.97 (s, 2H), 3.66 (t, *J* = 5.2 Hz, 2H), 3.33-3.27 (m, 2H), 2.39 (s, 3H).

### Example 76:

### 4-(6-((1-(4-Fluorophenyl)-4-methyl-1H-1,2,3-triazol-5-yl)methoxy)pyridazin-3-yl)piperazin-2-one (76)

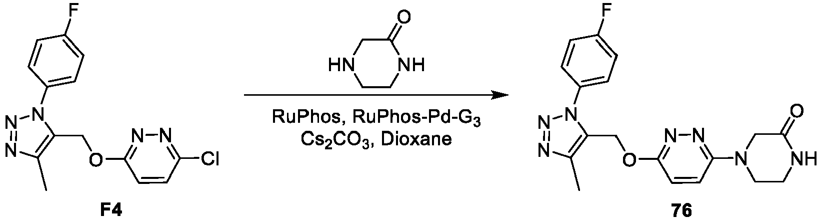

The experimental operation was as described in Example **1**, using intermediate **F4** and 2-piperazinone as reactants to obtain the title product.

MS (ESI) m/z [M+H]⁺ = 384.1.

¹H NMR (400 MHz, CDsOD) δ 7.70-7.61 (m, 2H), 7.38-7.28 (m, 3H), 7.02 (d, *J=* 9.6 Hz, 1H), 5.45 (s, 2H), 4.13 (s, 2H), 3.80-3.71 (m, 2H), 3.51-3.42 (m, 2H), 2.46 (s, 3H).

### Example 77:

### (S)-8-(6-((1-(4-Fluorophenyl)-4-methyl-1H-1,2,3-triazol-5-yl)methoxy)pyridazin-3-yl)octahydro-1H-pyrazino[1,2-a]pyrazin-1-one (77)

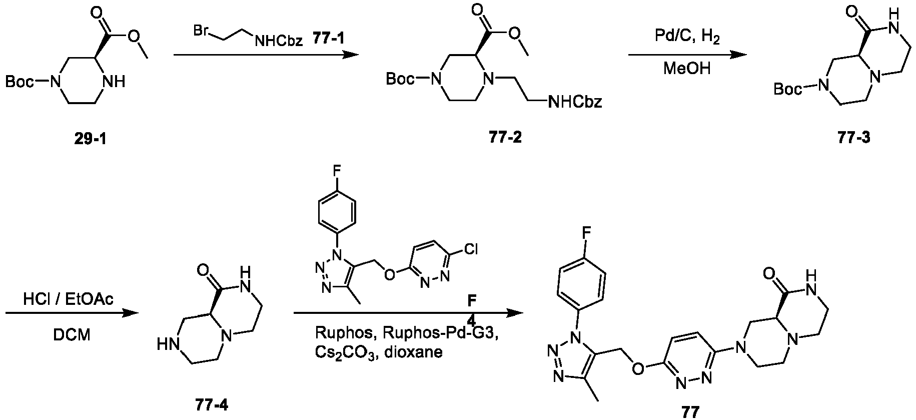

### Step 1: Synthesis of 1-(tert-butyl) 3-methyl (S)-4-(2-(((benzyloxy)carbonyl)amino)ethyl)piperazine-1,3-dicarboxylate (77-2)

**77-1** (1.0 g, 4.09 mmol) and **77-1** (1.27 g, 4.91 mmol) were dissolved in anhydrous DMF (15.0 mL) at room temperature, then potassium carbonate (678 mg, 4.91 mmol) was added thereto, and the reaction mixture was reacted at 100°C for 16 hours. TLC showed that the reaction was completed. The reaction mixture was diluted with water (40 mL), extracted with dichloromethane (30 mL × 3), and the organic phases were combined, washed with saturated brine (40 mL × 2), dried over anhydrous sodium sulfate, and filtered. The filtrate was concentrated under reduced pressure, and the residue was purified by flash silica gel column chromatography (methanol: dichloromethane = 0: 1 to 1:10) to obtain the title product (370 mg, yellow oil).

MS (ESI) m/z [M+H]⁺ = 422.5.

### Step 2: Synthesis of tert-butyl (S)-tert-butyl-9-oxohexahydro-1H-pyrazino[1,2-a]pyrazine-2(6H)-carboxylate (77-3)

**77-2** (370 mg, 0.87 mmol) was dissolved in methanol (6.0 mL) at room temperature, then palladium/carbon (30 mg, 10%) was added thereto, and the reaction was carried out for 16 hours under a hydrogen (15 psi) atmosphere. TLC showed that the reaction was completed. The reaction mixture was filtered, and the filtrate was concentrated under reduced pressure to obtain the title product (210 mg, yellow oil).

### Step 3: Synthesis of (S)-octahydro-1H-pyrazino[1,2-a]pyrazin-1-one (77-4)

**77-2** (60 mg, 0.24 mmol) was dissolved in anhydrous dichloromethane (4.0 mL) at room temperature, then hydrochloric acid/ethyl acetate solution (4.0 mL, 4.0 M) was slowly added dropwise thereto, and the reaction was stirred for 1 hour. TLC showed that the reaction was completed. The reaction mixture was concentrated under reduced pressure to obtain the title product (50 mg, yellow solid, hydrochloride).

### Step 4: Synthesis of (S)-8-(6-((1-(4-fluorophenyl)-4-methyl-1H-1,2,3-tiiazol-5-yl)methoxy)pyridazin-3-yl)octahydro-1H-pyrazino[1,2-a]pyrazin-1-one (77)

The experimental operation was as described in Example **1**, using intermediates **F4** and **77-4** as reactants to obtain the title product.

MS (ESI) m/z [M+H]⁺ = 439.0.

¹H NMR (400 MHz, CDsOD) δ 7.66-7.63 (m, 2H), 7.39-7.33 (m, 3H), 7.01 (d, *J=* 9.4 Hz, 1H), 5.45 (s, 2H), 4.49-4.45 (m, 1H), 4.12-4.08 (m, 1H), 3.52-3.50 (m, 1H), 3.26-3.23 (m, 1H), 3.05-3.00 (m, 3H), 2.99-2.83 (m, 2H), 2.62-2.59 (m, 1H), 2.46-2.44 (m, 4H).

### Example 78:

### 1-(4-(Difluoromethyl)phenyl)-5-(((6-(3-oxopiperazin-1-yl)pyridazin-3-yl)oxy)methyl)-1H-1,2,3-triazole-4-carbonitrile (78)

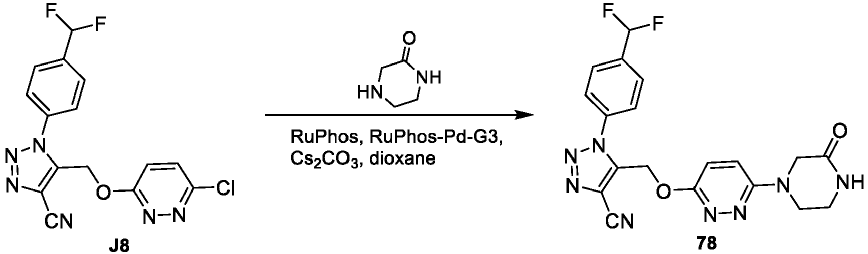

The experimental operation was as described in Example **1**, using intermediate **J8** and 2-piperazinone as reactants to obtain the title product.

MS (ESI) m/z [M+H]⁺ = 427.2.

¹H NMR (400 MHz, CDsOD) δ 7.84 (s, 4H), 7.38 (d, *J* = 10.0 Hz, 1H), 7.09 (d, *J* = 9.6 Hz, 1H), 6.93 (t, *J* = 55.6 Hz, 1H), 5.70 (s, 2H), 4.13 (s, 2H), 3.77-3.74 (m, 2H), 3.46-3.43 (m, 2H)

### Example 79:

### 4-(6-((1-(4-(((tert-Butyldimethylsilyl)oxy)methyl)phenyl)-4-methyl-1H-1,2,3-triazol-5-yl)methoxy)pyridazin-3-yl)piperazin-2-one (79)

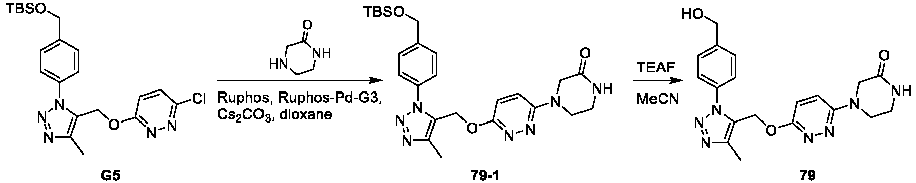

### Step 1: Synthesis of 4-(6-((1-(4-(((tert-butyldimethylsilyl)oxy)methyl)phenyl)-4-methyl-1H-1,2,3-triazol-5-yl)methoxy)pyridazin-3-yl)piperazin-2-one (79-1)

The experimental operation was as described in Example **1**, using intermediate **G5** and 2-piperazinone as reactants to obtain the title product.

¹H NMR (400 MHz, CDsOD) δ 7.49-7.64 (m, 4H), 7.35 (d, *J* = 9.6 Hz, 1H), 7.02 (d, *J* = 9.6 Hz, 1H), 5.48 (s, 2H), 5.45 (s, 2H), 4.12 (s, 2H), 3.75 (t, *J* = 5.6 Hz, 2H), 3.45 (t, *J* = 5.6 Hz,2H), 2.46 (s, 3H), 0.95 (s, 9H), 0.12 (s, 6H)

### Step 2: Synthesis of 4-(6-((1-(4-(hydroxymethyl)phenyl)-4-methyl-1H-1,2,3-triazol-5-yl)methoxy)pyridazin-3-yl)piperazin-2-one (79)

**79-1** (60.0 mg, 0.118 mmol) and TEAF (98.5 mg, 0.60 mmol) were added to anhydrous acetonitrile (1.0 mL) at room temperature. The system was replaced with nitrogen three times, and the reaction was carried out at 40°C for 5 hours. The reaction mixture was concentrated under reduced pressure, and the crude product was purified by flash silica gel column chromatography (dichloromethane: methanol = 99: 1 to 10: 1) to obtain the title product (39 mg, white solid).

MS (ESI) m/z [M+H]⁺ = 396.5.

¹H NMR (400 MHz, DMSO-*d₆*) δ 8.07 (br. s, 1H), 7.61-7.47 (m, 4H), 7.40 (d, *J* = 9.6 Hz, 1H), 7.07 (d, *J* = 9.6 Hz, 1H), 5.42-5.33 (m, 3H), 4.57 (d, *J* = 5.2 Hz, 2H), 3.69-3.64 (m, 2H), 3.46-3.43 (m, 2H), 2.38 (s, 3H)

### Example 80:

### 4-(6-((1-(4-(Difluoromethyl)phenyl)-4-(hydroxymethyl)-1H-1,2,3-triazol-5-yl)methoxy)pyridazin-3-yl)piperazin-2-one (80)

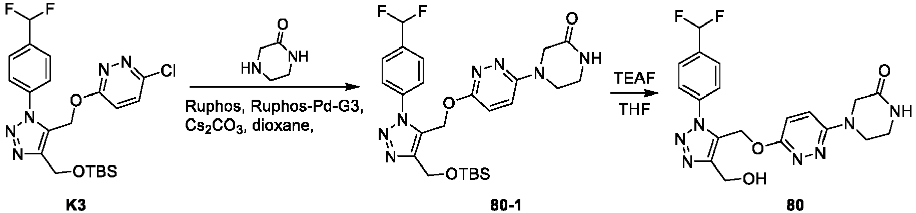

The experimental operation was as described in Example **79**, using intermediate **K3** and 2-piperazinone as reactants, and undergoing the steps of coupling and deprotection reactions to obtain the title product.

MS (ESI) m/z [M+H]⁺ = 432.1.

¹H NMR (400 MHz, CDsOD) δ 7.78 (s, 4H), 7.34 (d, *J* = 9.6 Hz, 1H), 7.04-6.74 (m, 2H), 5.60 (s, 2H), 4.60 (s, 2H), 4.12 (s, 2H), 3.77-3.73 (m, 2H), 3.47-3.43 (m, 2H).

### Example 81 and Example 82:

### 4-(6-((1-(4-(Difluoromethyl)phenyl)-4-methyl-1H-1,2,3-triazol-5-yl)methoxy)-5-methylpyridazin-3-yl)piperazin-2-one (81)

### 4-(6-((1-(4-(Difluoromethyl)phenyl)-4-methyl-1H-1,2,3-triazol-5-yl)methoxy)-4-methylpyridazin-3-yl)piperazin-2-one (82)

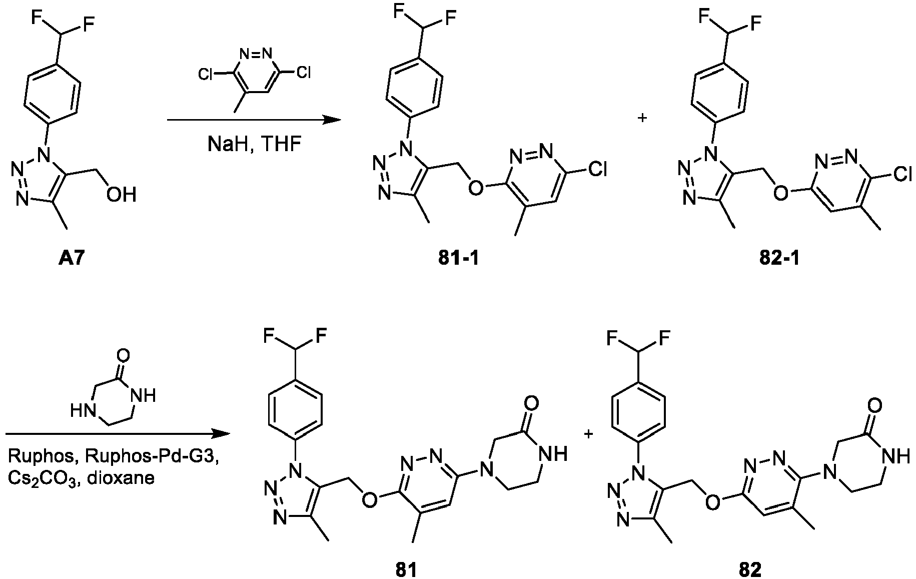

### Step 1: Synthesis of a mixture of 6-chloro-3-((1-(4-(difluoromethyl)phenyl)-4-methyl-1H-1,2,3-triazol-5-yl)methoxy)-4-methylpyridazine (81-1) and 3-chloro-6-((1-(4-(difluoromethyl)phenyl)-4-methyl-1H-1,2,3-triazol-5-yl)methoxy)-4-methylpyridazine (82-1)

Intermediate **A7** (200 mg, 0.84 mmol) was dissolved in anhydrous tetrahydrofuran (4 mL) at 0°C, then sodium hydride (30.2 mg, 1.26 mmol, 60%) was added thereto, and the mixture was stirred at this temperature for 0.5 hours. 3,6-Dichloro-4-methylpyridazine (164 mg, 1.01 mmol) was added thereto, and the reaction mixture was warmed to room temperature and stirred for 3 hours. The reaction endpoint was monitored by TLC. The reaction mixture was added with water (30 mL) to quench the reaction, and extracted with ethyl acetate (30 mL × 3). The organic phases were combined, dried over anhydrous sodium sulfate, filtered, and the filtrate was concentrated under reduced pressure. The residue was purified by flash silica gel column chromatography (ethyl acetate: petroleum ether = 0:1 to 1:1) to obtain a mixture of the title products **81-1** and **82-1** (240 mg, yellow solid).

**81-1:** MS (ESI) m/z [M+H]⁺ = 366.1.

**82-1:** MS (ESI) m/z [M+H]⁺ = 366.1.

### Step 2: Synthesis of 4-(6-((1-(4-(difluoromethyl)phenyl)-4-methyl-1H-1,2,3-triazol-5-yl)methoxy)-5-methylpyridazin-3-yl)piperazin-2-one (81) and 4-(6-((1-(4-(difluoromethyl)phenyl)-4-methyl-1H-1,2,3-triazol-5-yl)methoxy)-4-methylpyridazin-3-yl)piperazin-2-one (82)

The mixture of **81-1** and **82-1** from step 1 (370 mg, 1.01 mmol) and 2-piperazinone (121 mg, 1.21 mmol) were dissolved in dioxane (10.0 mL), then 2-dicyclohexylphosphino-2',6'-di-i-propoxy-1,1'-biphenyl (47 mg, 0.1 mmol), cesium carbonate (658 mg, 2.0 mmol), and methanesulfonato(2-dicyclohexylphosphino-2',6'-di-i-propoxy-1, 1'-biphenyl)(2'-amino-1,1'-biphenyl-2-yl)palladium(II) (84 mg, 0.1 mmol) were sequentially added thereto, and the reaction was stirred at 100°C for 16 hours. The completion of the reaction was monitored by LCMS. The reaction mixture was diluted with dichloromethane (50 mL), filtered, and the filtrate was concentrated under reduced pressure. The residue was purified by preparative thin-layer chromatography (dichloromethane: methanol = 15:1) to obtain the title products **81** (3 mg, white solid) and **82** (3 mg, white solid), respectively.

### Compound 81:

MS (ESI) m/z [M+H]⁺ = 430.2.

¹H NMR (400 MHz, DMSO-*d*₆) δ 8.02 (br. s, 1H), 7.79 (s, 4H), 7.25 (s, 1H), 7.13 (s, *J* = 55.6 Hz, 1H), 7.25 (s, 1H), 5.51 (s, 2H), 3.94 (s, 2H), 3.62-3.58 (m, 2H), 3.27-3.23 (m, 2H), 2.40 (s, 3H), 1.85 (s, 3H).

### Compound 82:

MS (ESI) m/z [M+H]⁺ = 430.2.

¹H NMR (400 MHz, DMSO-*d*₆) δ 7.87 (br. s, 1H), 7.78 (s, 4H), 7.13 (s, *J* = 55.6 Hz, 1H), 6.99 (s, 1H), 5.50 (s, 2H), 3.69 (s, 2H), 3.27-3.20 (m, 4H), 2.40 (s, 3H), 2.23 (s, 3H).

### Example 83:

### 4-(6-((4-Cyclopropyl-1-(4-(difluoromethyl)phenyl)-1H-1,2,3-triazol-5-yl)methoxy)pyridazin-3-yl)piperazin-2-one (83)

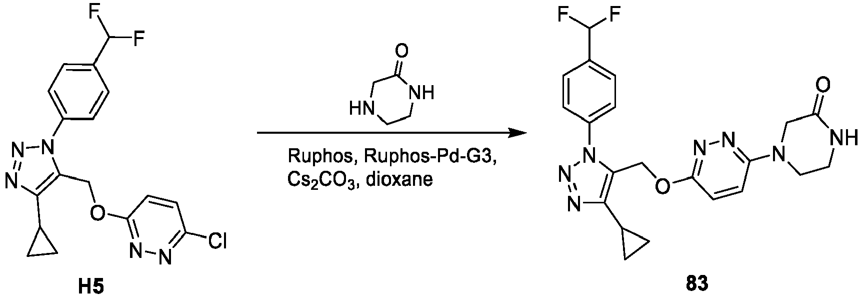

The experimental operation was as described in Example **1**, using intermediate **H5** and 2-piperazinone as reactants to obtain the title product.

MS (ESI) m/z [M+H]⁺ = 442.1.

¹H NMR (400 MHz, CDCl₃) δ 7.70 (d, *J* = 8.4 Hz, 2H), 7.65 (d, *J* = 8.4 Hz, 2H), 7.02 (d, *J* = 9.4 Hz, 1H), 6.94 (d, *J* = 9.4 Hz, 1H), 6.57 (t, *J* = 55.6 Hz, 1H), 6.15 (br. s, 1H), 5.54 (s, 2H), 4.14 (s, 2H), 3.94-3.91 (m, 2H), 3.59-3.55 (m, 2H), 2.04-2.00 (m, 1H), 1.15-1.12 (m, 2H), 1.04-1.01 (m, 2H).

### Example 84:

### 7-(6-((1-(4-(Difluoromethyl)phenyl)-4-methyl-1H-1,2,3-triazol-5-yl)methoxy)pyridazin-3-yl)-5,6,7,8-tetrahydro-[1,2,4]triazolo[4,3-a]pyrazine (84)

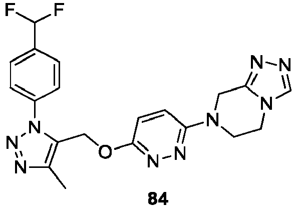

The experimental operation was as described in Example **1**, using **A8** and 5,6,7,8-tetrahydro-[1,2,4]triazolo[4,3-*a*]pyrazine hydrochloride as reactants to obtain the title product.

MS (ESI) m/z [M+H]⁺ = 439.9.

¹H NMR (400 MHz, CDsOD) *δ* 8.48 (s, 1H), 7.76 (s, 4H), 7.51 (d, *J* = 9.6 Hz, 1H), 7.05 (d, *J* = 9.6 Hz, 1H), 6.88 (t, *J* = 55.6 Hz, 1H), 5.51 (s, 2H), 4.93 (s, 2H), 4.28 (t, *J* = 5.6 Hz, 2H), 4.05 (t, *J* = 5.6 Hz, 2H), 2.48 (s, 3H)

### Example 85:

### 6-(6-((1-(4-(Difluoromethyl)phenyl)-4-methyl-1H-1,2,3-triazol-5-yl)methoxy)pyridazin-3-yl)-2-oxa-6-azaspiro[3.5]nonane (85)

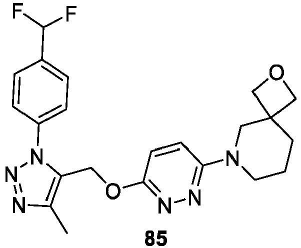

The experimental operation was as described in Example **1**, using **A8** and 2-oxa-6-azaspiro[3.5]nonane as reactants to obtain the title product.

MS (ESI) m/z [M+H]⁺ = 443.2.

¹H NMR (400 MHz, DMSO-*d*₆) δ 7.79 (s, 4H), 7.47 (d, *J* = 9.4 Hz, 1H), 7.14 (t, *J* = 55.6 Hz, 1H), 7.98 (d, *J* = 9.4 Hz, 1H), 5.46 (s, 2H), 4.26 (s, 2H), 3.64 (s, 2H), 3.33-3.30 (m, 2H), 2.40 (s, 3H), 2.03 (s, 2H), 1.83-1.80 (m, 2H), 1.54-1.51 (m, 2H).

### Example 86:

### 2-(6-((1-(4-(Difluoromethyl)phenyl)-4-methyl-1H-1,2,3-triazol-5-yl)methoxy)pyridazin-3-yl)-2,6-diazaspiro[3.4]octan-5-one (86)

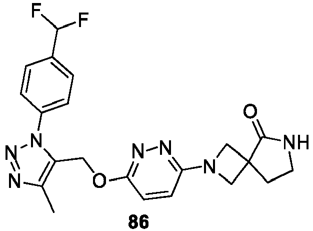

The experimental operation was as described in Example **1**, using **A8** and 2,6-diazaspiro[3.4]octan-5-one hydrochloride as reactants to obtain the title product.

MS (ESI) m/z [M+H]⁺ = 442.2.

¹H NMR (400 MHz, CDsOD) δ 7.77 (s, 4H), 6.97 (d, *J* = 9.4 Hz, 1H), 6.93 (d, *J* = 9.4 Hz, 1H), 6.89 (t, *J* = 55.6 Hz, 1H), 5.48 (s, 2H), 4.22 (d, *J* = 8.0 Hz, 2H), 3,99 (d, *J* = 8.0 Hz, 2H), 3.36 (d, *J* = 6.8 Hz, 2H), 2.53 (d, *J* = 6.8 Hz, 2H), 2.48 (s, 3H).

### Example 87:

### 2-(6-((1-(4-(Difluoromethyl)phenyl)-4-methyl-1H-1,2,3-triazol-5-yl)methoxy)pyridazin-3-yl)-2,6-diazaspiro[3.4]octan-7-one (87)

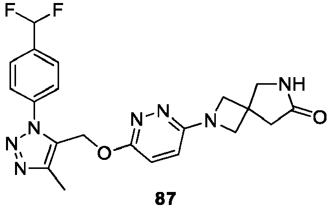

The experimental operation was as described in Example **1**, using **A8** and 2,6-diazaspiro[3.4]octan-7-one *p*-toluenesulfonate as reactants to obtain the title product.

MS (ESI) m/z [M+H]⁺ = 442.2.

¹H NMR (400 MHz, CDsOD) δ 7.76 (s, 4H), 6.96 (d, *J* = 9.4 Hz, 1H), 6.91 (d, *J* = 9.4 Hz, 1H), 6.89 (t, *J* = 55.6 Hz, 1H), 5.49 (s, 2H), 4.05 (s, 4H), 3.66 (s, 2H), 2.67 (s, 2H), 2.47 (s, 3H).

### Example 88:

### N-(1-(6-((1-(4-(Difluoromethyl)phenyl)-4-methyl-1H-1,2,3-triazol-5-yl)methoxy)pyridazin-3-yl)pyrrolidin-3-yl)acetamide (88)

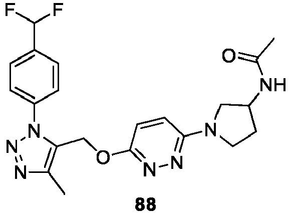

The experimental operation was as described in Example **1**, using **A8** and *N-*(pyrrolidin-3-yl)acetamide as reactants to obtain the title product.

MS (ESI) m/z [M+H]⁺ = 444.2.

¹H NMR (400 MHz, DMSO-*d*₆) δ 8.13 (d, *J* = 6.4 Hz, 1H), 7.79 (s, 4H), 7.14 (t, *J* = 55.6 Hz, 1H), 6.99-6.94 (m, 2H), 5.42 (s, 2H), 4.34-4.30 (m, 1H), 3.58-3.56 (m, 1H), 3.47-3.41 (m, 2H), 3.23-3.21 (m, 1H), 2.39 (s, 3H), 2.16-2.11 (m, 1H), 1.88-1.83 (m, 1H), 1.80 (s, 3H).

### Example 89:

### 4-(6-((1-(4-(Difluoromethyl)phenyl)-4-methyl-1H-1,2,3-triazol-5-yl)methoxy)pyridazin-3-yl)-5-methylpiperazin-2-one (89)

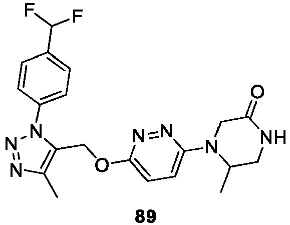

The experimental operation was as described in Example **1**, using **A8** and 5-methylpiperazin-2-one as reactants to obtain the title product.

MS (ESI) m/z [M+H]⁺ = 430.1.

¹H NMR (400 MHz, DMSO-*d*₆) δ 8.06 (br. s, 1H), 7.79 (s, 4H), 7.32 (d, *J* = 9.6 Hz, 1H), 7.13 (t, *J* = 55.6 Hz, 1H), 7.01 (d, *J* = 9.6 Hz, 1H), 5.45 (s, 2H), 4.57 (br. s, 1H), 4.18-4.13 (m, 1H), 3.66-3.62 (m, 1H), 3.51-3.50 (m, 1H), 3.11-3.07 (m, 1H), 2.40 (s, 3H), 1.00 (d, *J* = 3.2 Hz, 3H).

### Example 90:

### 3-((1-(4-(Difluoromethyl)phenyl)-4-methyl-1H-1,2,3-triazol-5-yl)methoxy)-6-(5,6-dihydro-2H-pyran-3-yl)pyridazine (90)

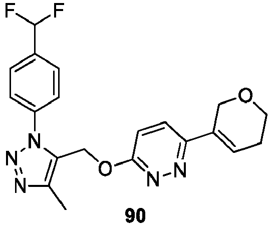

The experimental operation was as described in step 3 in Example **57**, using **A8** and 2-(5,6-dihydro-2*H*-pyran-3-yl)-4,4,5,5-tetramethyl-1,3,2-dioxaborane as reactants to obtain the title product.

MS (ESI) m/z [M+H]⁺ = 400.2.

¹H NMR (400 MHz, DMSO-*d*₆) δ 7.94 (d, *J* = 9.6 Hz, 1H), 7.78 (s, 4H), 7.17 (d, *J* = 9.6 Hz, 1H), 7.13 (t, *J* = 55.6 Hz, 1H), 6.76 (br. s, 1H), 5.59 (s, 2H), 4.56 (s, 2H), 3.74 (t, *J* = 5.4 Hz, 2H), 2.41 (s, 3H), 2.30-2.28 (m, 2H).

### Example 91:

### 2-(6-((1-(4-(Difluoromethyl)phenyl)-4-methyl-1H-1,2,3-triazol-5-yl)methoxy)pyridazin-3-yl)-8-(2,2,2-trifluoroethyl)octahydro-4H-pyrazino[1,2-a]pyrazin-4-one (91)

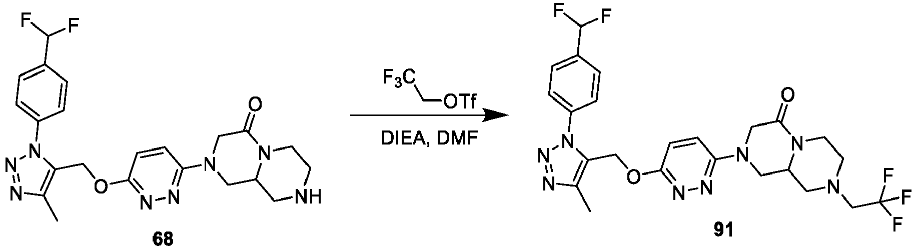

Example **68** (50.0 mg, 0.11 mmol) was dissolved in anhydrous *N,N-*dimethylformamide (2.0 mL) at room temperature, then diisopropylethylamine (41.2 mg, 0.32 mol) and trifluoroethyl triflate (37 mg, 0.16 mmol) were sequentially added thereto, and the reaction mixture was stirred for 16 hours. The reaction endpoint was monitored by LCMS. The reaction was quenched by adding water (10 mL), and the reaction mixture was extracted with dichloromethane (10 mL × 3). The organic phases were combined and concentrated under reduced pressure, and the residue was purified by high performance liquid chromatography (chromatographic column: Phenomenex C18 150 * 40 mm * 5 µm; mobile phase: water (0.225% trifluoroacetic acid) - acetonitrile]; B%: 30% to 60%; flow rate: 60 mL/min) to obtain the title product (16.8 mg, white solid).

MS (ESI) m/z [M+H]⁺ = 553.2.

¹H NMR (400 MHz, CDsOD) δ 7.76 (s, 4H), 7.41 (d, *J* = 9.6 Hz, 1H), 7.02 (d, *J* = 9.6 Hz, 1H), 6.88 (t, *J* = 55.6 Hz, 1H), 5.50 (s, 2H), 4.51-4.48 (m, 1H), 4.38-4.34 (m, 1H), 4.20-4.15 (m, 1H), 4.00-3.96 (m, 1H), 3.76-3.73 (m, 1H), 3.43-3.20 (m, 1H), 3.19-3.15 (m, 2H), 3.11-2.99 (m, 2H), 2.87-2.84 (m, 1H), 2.47 (s, 3H), 2.45-2.41 (m, 1H), 2.37-2.32 (m, 1H).

### Example 92:

### 2-(6-((1-(4-(Difluoromethyl)phenyl)-4-methyl-1H-1,2,3-triazol-5-yl)methoxy)pyridazin-3-yl)-8-(pyrimidin-2-ylmethyl)octahydro-4H-pyrazino[1,2-a]pyrazin-4-one (92)

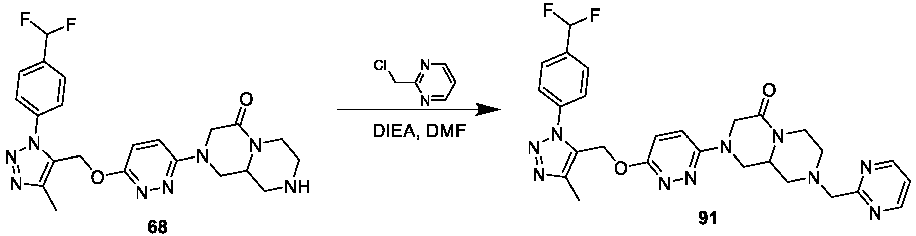

The experimental operation was as described in Example **91**, using **68** and 2-(chloromethyl)pyrimidine as reactants to obtain the title product.

MS (ESI) m/z [M+H]⁺ = 563.2.

¹H NMR (400 MHz, CDsOD) δ 8.80 (d, *J* = 4.8 Hz, 2H), 7.76 (s, 4H), 7.46-7.36 (m, 2H), 6.99 (d, *J* = 9.6 Hz, 1H), 6.88 (t, *J* = 55.6 Hz, 1H), 5.50 (s, 2H), 4.52-4.49 (m, 1H), 4.39-4.35 (m, 1H), 4.19-4.17 (m, 1H), 3.99-3.95 (m, 1H), 3.86 (s, 2H), 3.81-3.77 (m, 1H), 3.28-3.26 (m, 1H), 3.10-2.99 (m, 2H), 2.91-2.89 (m, 1H), 2.47 (s, 3H), 2.27-2.24 (m, 1H), 2.20-2.14 (m, 1H).

### Example 93:

### 4-(6-((1-(4-(Difluoromethyl)phenyl)-4-methyl-1H-1,2,3-triazol-5-yl)methoxy)pyridazin-3-yl)-1-(pyrimidin-2-ylmethyl)piperazin-2-one (93)

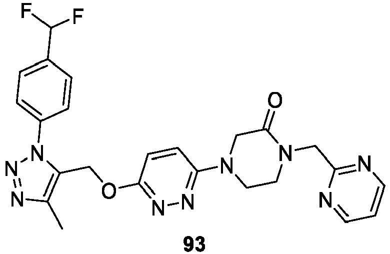

The experimental operation was as described in Example **59**, using **A8**, *tert*-butyl 3-oxopiperazine-1-carboxylate, and 2-(chloromethyl)pyrimidine as raw materials, and undergoing the steps of alkylation, deprotection, and coupling reactions to obtain the title product.

MS (ESI) m/z [M+H]⁺ = 508.2.

¹H NMR (400 MHz, DMSO-*d*₆) δ 8.75 (d, *J* = 4.8 Hz, 2H), 7.79 (s, 4H), 7.46-7.39 (m, 2H), 7.14 (t, *J* = 55.6 Hz, 1H), 7.06 (d, *J* = 9.6 Hz, 1H), 5.47 (s, 2H), 4.77 (s, 2H), 4.13 (s, 2H), 3.83-3.80 (m, 2H), 3.61-3.58 (m, 2H), 2.41 (s, 3H).

### Example 94:

### 4-(6-((1-(4-Fluorophenyl)-4-methyl-1H-1,2,3-triazol-5-yl)methoxy)pyridazin-3-yl)-1-(2,2,2-trifluoroethyl)piperazin-2-one (94)

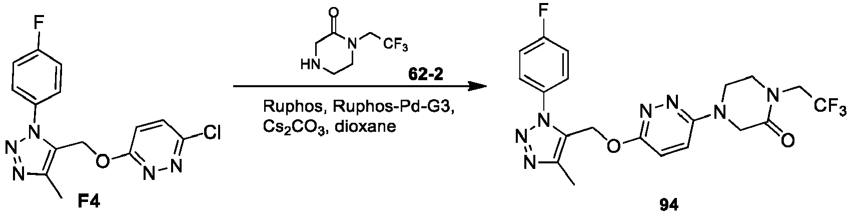

The experimental operation was as described in Example **1**, using intermediates **F4** and **62-2** as reactants to obtain the title product.

MS (ESI) m/z [M+H]⁺ = 466.2.

¹H NMR (400 MHz, CDsOD) δ 7.67-7.63 (m, 2H), 7.38-7.31 (m, 3H), 7.03 (d, *J* = 9.6 Hz, 1H), 5.46 (s, 2H), 4.25-4.19 (m, 4H), 3.85-3.83 (m, 2H), 3.71-3.68 (m, 2H), 2.46 (s, 3H).

### Example 95:

### 4-(6-((1-(4-(Difluoromethyl)phenyl)-4-methyl-1H-1,2,3-triazol-5-yl)methoxy)pyridazin-3-yl)-1-(2-morpholinoethyl)piperazin-2-one (95)

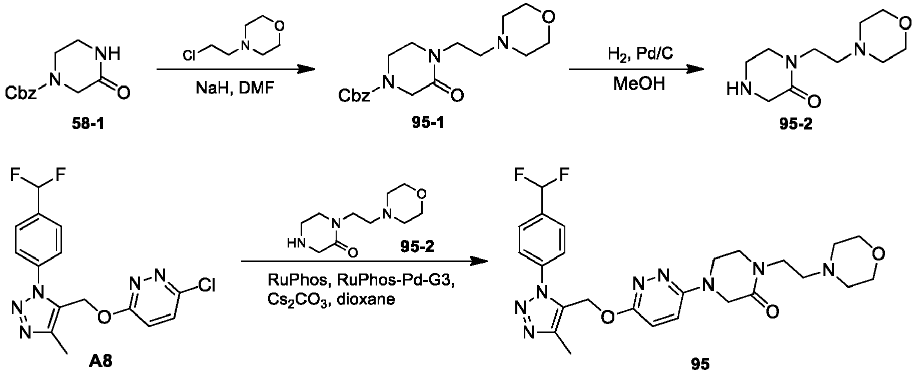

The experimental operation was as described in step 1 of Example **59** and steps 2 to 3 of Example **58**, using **A8**, *tert*-butyl 3-oxopiperazine-1-carboxylate, and 4-(2-chloroethyl)morpholine as raw materials, and undergoing the steps of alkylation, deprotection, and coupling reactions to obtain the title product.

MS (ESI) m/z [M+H]⁺ = 529.2.

¹H NMR (400 MHz, DMSO-*d*₆) δ 7.78 (s, 4H), 7.41 (d, *J* = 9.6 Hz, 1H), 7.13 (t, *J* = 55.6 Hz, 1H), 7.01 (d, *J* = 9.6 Hz, 1H), 5.45 (s, 2H), 4.02 (s, 2H), 3.73-3.72 (m, 2H), 3.49-3.45 (m, 8H), 2.49-2.35 (m, 9H).

### Example 96:

### 4-(6-((1-(4-(Difluoromethyl)phenyl)-4-methyl-1H-1,2,3-triazol-5-yl)methoxy)pyridazin-3-yl)-1-((tetrahydrofuran-2-yl)methyl)piperazin-2-one (96)

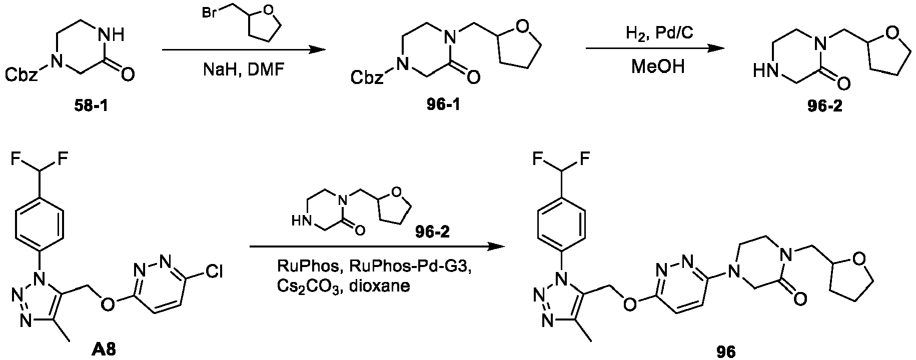

The experimental operation was as described in step 1 of Example **59** and steps 2 to 3 of Example **58**, using **A8**, *tert*-butyl 3-oxopiperazine-1-carboxylate, and 2-(bromomethyl)tetrahydrofuran as raw materials, and undergoing the steps of alkylation, deprotection, and coupling reactions to obtain the title product.

MS (ESI) m/z [M+H]⁺ = 500.2.

¹H NMR (400 MHz, DMSO-*d*₆) δ 7.77 (s, 4H), 7.39 (d, *J* = 9.6 Hz, 1H), 7.12 (t, *J* = 55.6 Hz, 1H), 7.03 (d, *J* = 9.6 Hz, 1H), 5.45 (s, 2H), 4.04 (s, 2H), 3.99-3.96 (m, 2H), 3.73-3.70 (m, 2H), 3.59-3.47 (m, 4H), 3.30-3.26 (m, 1H), 2.38 (s, 3H), 1.90-1.74 (m, 3H), 1.51-1.44 (m, 1H).

### Example 97:

### 1-(Cyclopropylmethyl)-4-(6-((1-(4-(difluoromethyl)phenyl)-4-methyl-1H-1,2,3-triazol-5-yl)methoxy)pyridazin-3-yl)piperazin-2-one (97)

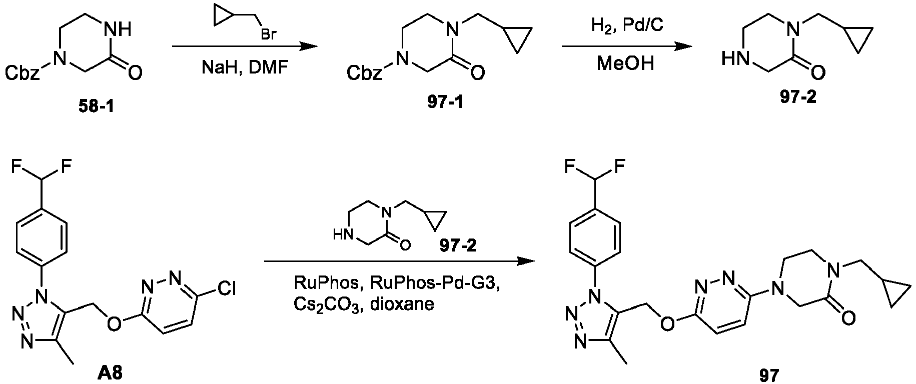

The experimental operation was as described in step 1 of Example **59** and steps 2 to 3 of Example **58**, using **A8**, benzyl 3-oxopiperazine-1-carboxylate, and bromomethyl cyclopropane as raw materials, and undergoing the steps of alkylation, deprotection, and coupling reactions to obtain the title product.

MS (ESI) m/z [M+H]⁺ = 470.2

¹H NMR (400 MHz, DMSO-*d*₆) δ 7.77 (s, 4H), 7.41 (d, *J* = 9.6 Hz, 1H), 7.12 (t, *J* = 55.6 Hz, 1H), 7.04 (d, *J* = 9.6 Hz, 1H), 5.45 (s, 2H), 4.03 (s, 2H), 3.74-3.72 (m, 2H), 3.51-3.49 (m, 2H), 3.21-3.19 (m, 2H), 2.38 (s, 3H), 0.96-0.94 (m, 1H), 0.43-0.41 (m, 2H), 0.21-0.19 (m, 2H).

### Example 98:

### 4-(6-((1-(4-(Difluoromethyl)phenyl)-4-methyl-1H-1,2,3-triazol-5-yl)methoxy)pyridazin-3-yl)-1-((tetrahydrofuran-3-yl)methyl)piperazin-2-one (98)

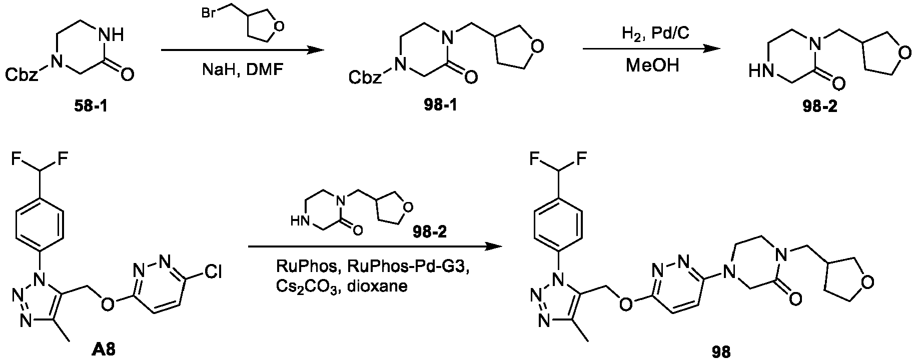

The experimental operation was as described in step 1 of Example **59** and steps 2 to 3 of Example **58**, using **A8**, benzyl 3-oxopiperazine-1-carboxylate, and 3-(bromomethyl)tetrahydrofuran as raw materials, and undergoing the steps of alkylation, deprotection, and coupling reactions to obtain the title product.

MS (ESI) m/z [M+H]⁺ = 500.2

¹H NMR (400 MHz, DMSO-*d*₆) δ 7.77 (s, 4H), 7.40 (d, *J* = 9.6 Hz, 1H), 7.13 (t, *J* = 55.6 Hz, 1H), 7.03 (d, *J* = 9.6 Hz, 1H), 5.45 (s, 2H), 4.04 (s, 2H), 3.73-3.71 (m, 3H), 3.64-3.57 (m, 2H), 3.44-3.42 (m, 2H), 3.38-3.35 (m, 3H), 2.54-2.52 (m, 1H), 2.38 (s, 3H), 1.88-1.85 (m, 1H), 1.53-1.48 (m, 1H).

### Example 99:

### 2-(4-(6-((1-(4-(Difluoromethyl)phenyl)-4-methyl-1H-1,2,3-triazol-5-yl)methoxy)pyridazin-3-yl)-2-oxopiperazin-1-yl)acetonitrile (99)

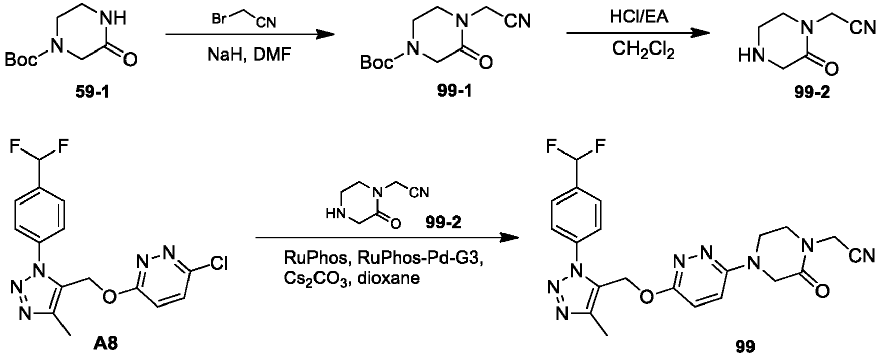

The experimental operation was as described in Example **59**, using **A8**, *tert*-butyl 3-oxopiperazine-1-carboxylate, and bromoacetonitrile as raw materials, and undergoing the steps of alkylation, deprotection, and coupling reactions to obtain the title product.

MS (ESI) m/z [M+H]⁺ = 455.1

¹H NMR (400 MHz, DMSO-*d*₆) δ 7.78 (s, 4H), 7.45 (d, *J* = 9.6 Hz, 1H), 7.13 (t, *J* = 55.6 Hz, 1H), 7.06 (d, *J* = 9.6 Hz, 1H), 5.46 (s, 2H), 4.47 (s, 2H), 4.15 (s, 2H), 3.83-3.81 (m, 2H), 3.53-3.51 (m, 2H), 2.39 (s, 3H).

### Example 100:

### 4-(6-((1-(4-(Difluoromethyl)phenyl)-4-methyl-1H-1,2,3-triazol-5-yl)methoxy)pyridazin-3-yl)-1-(oxetan-3-ylmethyl)piperazin-2-one (100)

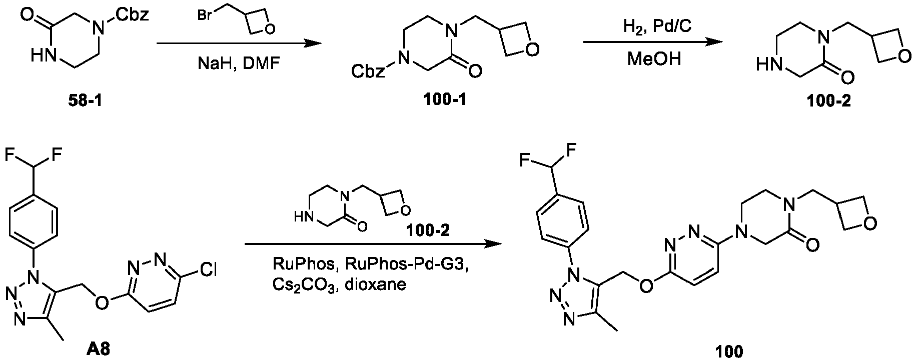

The experimental operation was as described in step 1 of Example **59** and steps 2 to 3 of Example **58**, using **A8**, benzyl 3-oxopiperazine-1-carboxylate, and 3-(bromomethyl)oxetane as raw materials, and undergoing the steps of alkylation, deprotection, and coupling reactions to obtain the title product.

MS (ESI) m/z [M+H]⁺ = 486.2

¹H NMR (400 MHz, DMSO-*d*₆) δ 7.77 (s, 4H), 7.39 (d, *J* = 9.6 Hz, 1H), 7.13 (t, *J* = 55.6 Hz, 1H), 7.03 (d, *J* = 9.6 Hz, 1H), 5.45 (s, 2H), 4.61-4.57 (m, 2H), 4.33-4.31 (m, 2H), 4.02 (s, 2H), 3.70-3.65 (m, 4H), 3.39-3.36 (m, 2H), 3.24-3.19 (m, 1H), 2.38 (s, 3H).

### Example 101:

### 1-(6-((1-(4-(Difluoromethyl)phenyl)-4-methyl-1H-1,2,3-triazol-5-yl)methoxy)pyridazin-3-yl)-N-(1-methyl-1H-pyrazol-3-yl)azetidine-3-carboxamide (101)

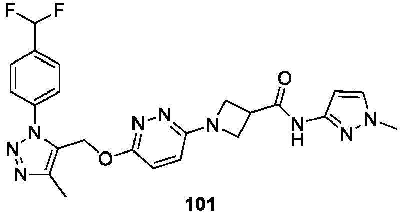

The experimental operation was as described in Example **38**, using Example **37** and 1-methyl-1*H*-pyrazol-3-amine as reactants to obtain the title product.

MS (ESI) m/z [M+H]⁺ = 496.2.

¹H NMR (400 MHz, DMSO-*d*₆) δ 10.52 (s, 1H), 7.78 (s, 4H), 7.53 (s, 1H), 7.13 (t, *J* = 55.6 Hz, 1H), 6.99 (d, *J* = 9.4 Hz, 1H), 6.92 (d, *J* = 9.4 Hz, 1H), 6.45 (s, 1H), 5.43 (s, 2H), 4.13-4.09 (m, 2H), 4.04-4.00 (m, 2H), 3.72 (s, 3H), 3.69-3.67 (m, 1H), 2.39 (s, 3H).

### Example 102:

### 1-(6-((1-(4-(Difluoromethyl)phenyl)-4-methyl-1H-1,2,3-triazol-5-yl)methoxy)pyridazin-3-yl)-N-(1-methyl-1H-pyrazol-5-yl)azetidine-3-carboxamide (102)

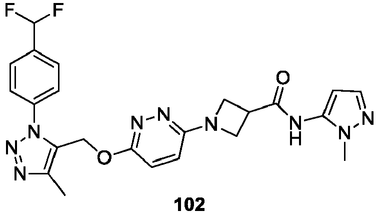

The experimental operation was as described in Example **38**, using Example **37** and 1-methyl-1*H*-pyrazol-5-amine as reactants to obtain the title product.

MS (ESI) m/z [M+H]⁺ = 496.2

¹H NMR (400 MHz, DMSO-*d*₆) δ 10.07 (s, 1H), 7.78 (s, 4H), 7.31 (s, 1H), 7.13 (t, *J* = 55.6 Hz, 1H), 7.00 (d, *J* = 9.4 Hz, 1H), 6.94 (d, *J* = 9.4 Hz, 1H), 6.20 (s, 1H), 5.43 (s, 2H), 4.19-4.15 (m, 2H), 4.09-4.05 (m, 2H), 3.76-3.75 (m, 1H), 3.65 (s, 3H), 2.39 (s, 3H).

### Example 103:

### 1-(6-((1-(4-(Difluoromethyl)phenyl)-4-methyl-1H-1,2,3-triazol-5-yl)methoxy)pyridazin-3-yl)-N-(1-methyl-1H-pyrazol-4-yl)azetidine-3-carboxamide (103)

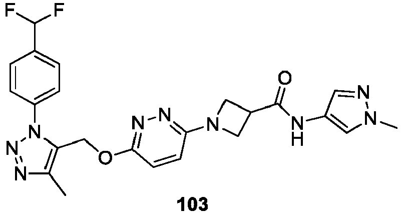

The experimental operation was as described in Example **38**, using Example **37** and 1-methyl-1*H*-pyrazol-4-amine as reactants to obtain the title product.

MS (ESI) m/z [M+H]⁺ = 496.2

¹H NMR (400 MHz, DMSO-*d*₆) δ 10.07 (s, 1H), 7.86 (s, 1H), 7.78 (s, 4H), 7.39 (s, 1H), 7.13 (t, *J* = 55.6 Hz, 1H), 6.99 (d, *J* = 9.6 Hz, 1H), 6.92 (d, *J* = 9.6 Hz, 1H), 5.43 (s, 2H), 4.14-4.10 (m, 2H), 4.04-4.01 (m, 2H), 3.76 (s, 3H), 3.63-3.60 (m, 1H), 2.38 (s, 3H).

### Example 104:

### (1-(6-((1-(4-(Difluoromethyl)phenyl)-4-methyl-1H-1,2,3-triazol-5-yl)methoxy)pyridazin-3-yl)azetidin-3-yl)(morpholino)methanone (104)

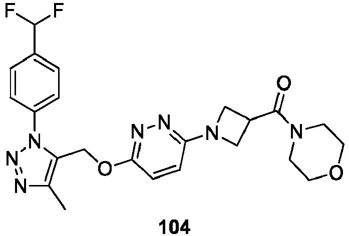

The experimental operation was as described in Example **38**, using Example **37** and morpholine as reactants to obtain the title product.

MS (ESI) m/z [M+H]⁺ = 486.2

¹H NMR (400 MHz, DMSO-*d*₆) δ 7.78 (s, 4H), 7.13 (t, *J* = 55.6 Hz, 1H), 6.98 (d, *J* = 9.6 Hz, 1H), 6.92 (d, *J* = 9.6 Hz, 1H), 5.42 (s, 2H), 4.15-4.11 (m, 2H), 4.06-4.03 (m, 2H), 3.87-3.79 (m, 1H), 3.66-3.65 (m, 4H), 3.46-3.45 (m, 2H), 3.32-3.31 (m, 2H), 2.38 (s, 3H).

### Example 105:

### 9-(6-((1-(4-(Difluoromethyl)phenyl)-4-methyl-1H-1,2,3-triazol-5-yl)methoxy)pyridazin-3-yl)-6-oxa-2-thia-9-azaspiro[4.5]decane 2,2-dioxide (105)

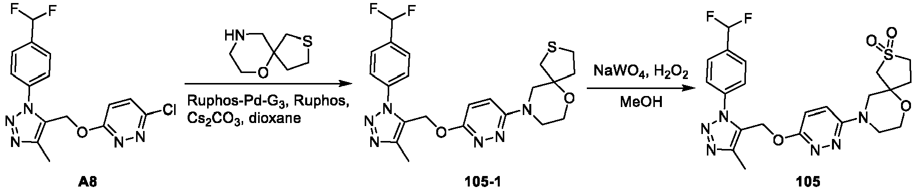

### Step 1: Synthesis of 9-(6-((1-(4-(difluoromethyl)phenyl)-4-methyl-1H-1,2,3-triazol-5-yl)methoxy)pyridazin-3-yl)-6-oxa-2-thia-9-azaspiro[4.5]decane (31-1)

The experimental operation was as described in Example **1**, using intermediate **A8** and 6-oxa-2-thia-9-azaspiro[4.5]decane as reactants to obtain the title product.

MS (ESI) m/z [M+H]⁺ = 475.2.

### Step 2: Synthesis of 9-(6-((1-(4-(difluoromethyl)phenyl)-4-methyl-1H-1,2,3-triazol-5-yl)methoxy)pyridazin-3-yl)-6-oxa-2-thia-9-azaspiro[4.5]decane 2,2-dioxide (31)

**31-1** (150 mg, 0.31 mmol) was dissolved in methanol (10 mL) at room temperature, then sodium tungstate (168 mg, 0.62 mmol) and hydrogen peroxide (0.17 mL, 1.55 mmol, 30%) were sequentially added thereto. The reaction was stirred for 1 hour. The reaction endpoint was monitored by LCMS. The reaction mixture was poured into saturated sodium thiosulfate solution (30 mL), and the mixture was extracted with ethyl acetate (30 mL × 3). The organic phases were combined, dried over anhydrous sodium sulfate, filtered, and the filtrate was concentrated under reduced pressure. The residue was purified by reversed-phase C18 flash preparative chromatography (acetonitrile: water = 0 to 40%) to obtain the title product (85 mg, white solid).

MS (ESI) m/z [M+H]⁺= 507.1.

¹H NMR (400 MHz, DMSO-*d*₆) δ 7.79 (s, 4H), 7.43 (d, *J* = 9.4 Hz, 1H), 7.14 (t, *J* = 55.6 Hz, 1H), 7.06 (d, *J* = 9.4 Hz, 1H), 5.46 (s, 2H), 3.82-3.76 (m, 3H), 3.64-3.61 (m, 1H), 3.49-3.45 (m, 1H), 3.27-3.22 (m, 5H), 2.38-2.29 (m, 4H), 2.25-2.21 (m, 1H).

### Example 106:

### 1-(6-((1-(4-(Difluoromethyl)phenyl)-4-methyl-1H-1,2,3-triazol-5-yl)methoxy)pyridazin-3-yl)-4-(methylsulfonyl)-1,4-diazepane (106)

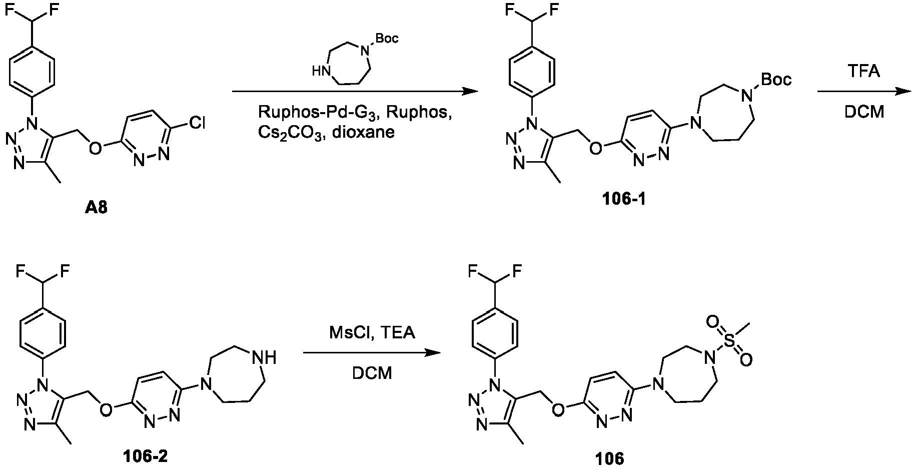

### Step 1: Synthesis of tert-butyl 4-(6-((1-(4-(difluoromethyl)phenyl)-4-methyl-1H-1,2,3-triazol-5-yl)methoxy)pyridazin-3-yl)-1,4-azepane-1-carboxylate (106-1)

The experimental operation was as described in Example **1**, using intermediate **A8** and *tert*-butyl *tert*-butyl-1,4-diazepane-1-carboxylate as reactants to obtain the title product.

MS (ESI) m/z [M+H]⁺ = 516.2.

### Step 2: Synthesis of 1-(6-((1-(4-(difluoromethyl)phenyl)-4-methyl-1H-1,2,3-triazol-5-yl)methoxy)pyridazin-3-yl)-1,4-diazepane (106-2)

The experimental operation was as described in step 2 in Example **54**, using intermediate **106-1** as the reactant to obtain the title product.

MS (ESI) m/z [M+H]⁺ = 416.2.

### Step 3: Synthesis of 1-(6-((1-(4-(difluoromethyl)phenyl)-4-methyl-1H-1,2,3-triazol-5-yl)methoxy)pyridazin-3-yl)-4-(methylsulfonyl)-1,4-diazepane (106)

Compound **106-2** (200 mg, 0.48 mmol) was dissolved in dichloromethane (5 mL) at 0°C, then triethylamine (145 mg, 1.44 mmol) and methanesulfonyl chloride (82 mg, 0.72 mmol) were sequentially added thereto, and the reaction mixture was warmed to room temperature and stirred for 1 hour. The reaction endpoint was monitored by LCMS. The reaction was quenched by adding water (10 mL), and the reaction mixture was extracted with dichloromethane (15 mL × 2). The organic phases were combined, dried over anhydrous sodium sulfate, filtered, and the filtrate was concentrated under reduced pressure. The residue was purified by preparative thin-layer chromatography (ethyl acetate: methanol = 10:1) to obtain the title product (24 mg, white solid).

MS (ESI) m/z [M+H]⁺ = 494.2.

¹H NMR (400 MHz, DMSO-*d*₆) δ 7.78 (s, 4H), 7.25 (d, *J* = 9.6 Hz, 1H), 7.13 (t, *J* = 55.6 Hz, 1H), 6.97 (d, *J* = 9.6 Hz, 1H), 5.43 (s, 2H), 3.78 (br. s, 2H), 3.69 (br. s, 2H), 3.39 (br. s, 2H), 3.19 (br. s, 2H), 2.80 (s, 3H), 2.39 (s, 2H), 1.81 (s, 2H).

### Example 107:

### (S)-8-(6-((1-(4-(Difluoromethyl)phenyl)-4-methyl-1H-1,2,3-triazol-5-yl)methoxy)pyridazin-3-yl)-2-methyloctahydro-1H-pyrazino[1,2-a]pyrazin-1-one (107)

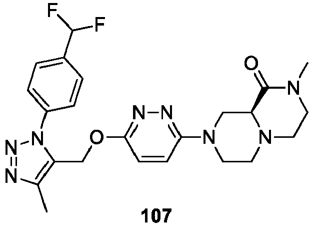

The experimental operation was as described in Example **26**, using compound **68** and methyl iodide as reactants to obtain the title product.

MS (ESI) m/z [M+H]⁺ = 485.1.

¹H NMR (400 MHz, DMSO-*d*₆) δ 7.78 (s, 4H), 7.38 (d, *J* = 9.6 Hz, 1H), 7.13 (t, *J* = 55.6 Hz, 1H), 6.98 (d, *J* = 9.6 Hz, 1H), 5.45 (s, 2H), 4.51-4.46 (m, 1H), 4.01-3.98 (m, 1H), 3.48-3.41 (m, 1H), 3.30-3.23 (m, 1H), 3.18-3.12 (m, 1H), 2.97-2.87 (m, 3H), 2.82 (s, 3H), 2.67-2.56 (m, 2H), 2.39 (s, 3H), 2.27-2.20 (m, 1H).

### Example 108:

### 1-Cyclopropyl-4-(6-((1-(4-(difluoromethyl)phenyl)-4-methyl-1H-1,2,3-triazol-5-yl)methoxy)pyridazin-3-yl)piperazin-2-one (108)

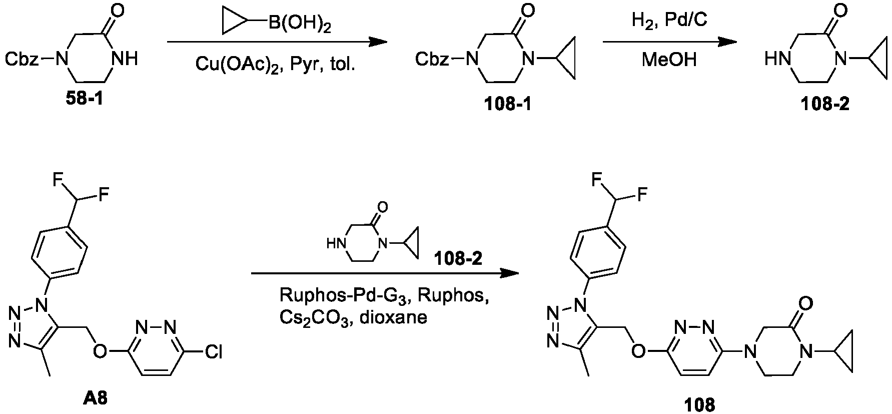

### Step 1: Synthesis of benzyl 4-cyclopropyl-3-oxopiperazine-1-carboxylate (108-1)

Compound **58-1** (1 g, 4.27 mmol), cyclopropylboronic acid (0.73 g, 8.54 mmol), cesium carbonate (0.70 g, 2.13 mmol), pyridine (1.01 g, 12.81 mmol), and copper acetate (0.78 g, 4.27 mmol) were mixed into toluene (30 mL) under an air atmosphere, and the reaction was stirred at 110°C for 16 hours. The reaction endpoint was monitored by LCMS. The reaction mixture was filtered, and the filter cake was washed with ethyl acetate (10 mL × 2). The filtrates were combined and concentrated under reduced pressure, and the residue was purified by flash silica gel column chromatography (ethyl acetate: petroleum ether = 0:1 to 1:0) to obtain the title product (500 mg, colorless oil).

MS (ESI) m/z [M+H]⁺ = 275.1.

### Step 2: Synthesis of 1-cyclopropylpiperazin-2-one (108-2)

The experimental operation was as described in step 2 in Example **58**, using intermediate **108-1** as the reactant to obtain the title product.

MS (ESI) m/z [M+H]⁺ = 141.1.

### Step 3: Synthesis of 1-cyclopropyl-4-(6-((1-(4-(difluoromethyl)phenyl)-4-methyl-1H-1,2,3-triazol-5-yl)methoxy)pyridazin-3-yl)piperazin-2-one (108)

The experimental operation was as described in Example **1**, using intermediates **A8** and **108-2** as reactants to obtain the title product.

MS (ESI) m/z [M+H]⁺ = 456.1.

¹H NMR (400 MHz, DMSO-*d*₆) δ 7.77 (s, 4H), 7.37 (d, *J* = 9.6 Hz, 1H), 7.12 (t, *J* = 55.6 Hz, 1H), 7.00 (d, *J* = 9.6 Hz, 1H), 5.44 (s, 2H), 4.00 (s, 2H), 3.69-3.67 (m, 2H), 3.34-3.32 (m, 2H), 2.75-2.73 (m, 1H), 2.38 (s, 3H), 0.69-0.62 (m, 4H).

### Example 109:

### 4-(6-((1-(4-(Difluoromethyl)phenyl)-4-methyl-1H-1,2,3-triazol-5-yl)methoxy)pyridazin-3-yl)-1-(2-(4,4-difluoropiperidin-1-yl)ethyl)piperazin-2-one (109)

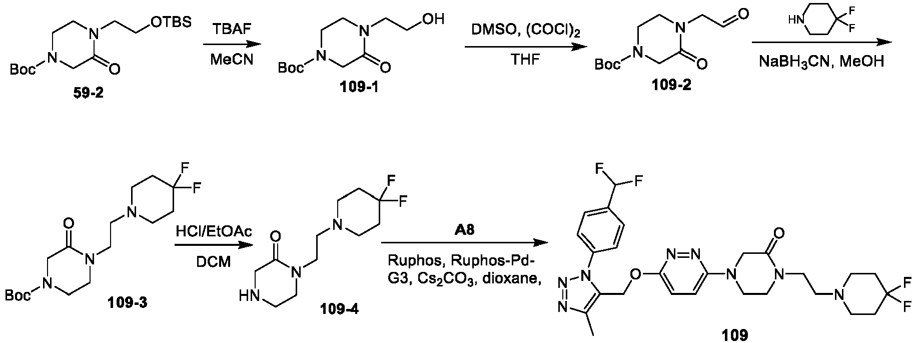

### Step 1: Synthesis of tert-butyl 4-(2-hydroxyethyl)-3-oxopiperazine-1-carboxylate (109-1)

Compound **59-2** (1.50 g, 4.18 mmol) and tetrabutylammonium fluoride (3.50 g, 20.9 mmol) were sequentially added to acetonitrile (20.0 mL) at room temperature, and the reaction was carried out at 40°C for 15 hours. The reaction endpoint was monitored by TLC. The reaction mixture was concentrated under reduced pressure, and the residue was diluted with water (100 mL), extracted with dichloromethane (50 mL × 3). The organic phases were combined, dried over anhydrous sodium sulfate, filtered, and the filtrate was concentrated under reduced pressure. The crude product was purified by flash silica gel column chromatography (dichloromethane: methanol = 99:1 to 20:1) to obtain the title product (998 mg, colorless oil).

¹H NMR (400 MHz, CDCl₃) *δ* 4.12 (s, 2H), 3.83 (t, *J* = 5.2 Hz, 2H), 3.67 (t, *J* = 5.2 Hz, 2H), 3.59 (t, *J* = 5.2 Hz, 2H), 3.47 (t, *J* = 5.2 Hz, 2H), 1.48 (s, 9H).

### Step 2: Synthesis of tert-butyl 3-oxo-4-(2-oxoethyl)piperazine-1-carboxylate (109-2)

Oxalyl chloride (286 mg, 2.25 mmol) was dissolved in dichloromethane (10.0 mL) at -65°C, then dimethyl sulfoxide (384 mg, 4.91 mmol) was added thereto, and the mixture was stirred for 10 minutes. Compound **109-1** (500 mg, 2.05 mmol) was added dropwise to the above mixture, and the reaction mixture was stirred at this temperature for 1 hour. Triethylamine (1.04 g, 2.05 mmol) was added thereto, and the reaction mixture was slowly warmed to room temperature and stirred for another 1 hour. The reaction mixture was diluted with water (10 mL) and most of the solvent was removed under reduced pressure. The residue was diluted with water (100 mL) and extracted with dichloromethane (50 mL × 3). The organic phases were combined, dried over anhydrous sodium sulfate, filtered, and the filtrate was concentrated under reduced pressure. The crude product was purified by flash silica gel column chromatography (dichloromethane: methanol = 99:1 to 30:1) to obtain the title product (290 mg, colorless oil).

¹H NMR (400 MHz, CDCl₃) *δ* 9.62 (s, 1H), 4.28 (s, 2H), 4.16 (s, 2H), 3.73 (t, *J* = 5.2 Hz, 2H), 3.43-3.39 (m, 2H), 1.9 (s, 9H).

### Step 3: Synthesis of tert-butyl 4-(2-(4,4-difluoropiperidin-1-yl)ethyl)-3-oxopiperazine-1-carboxylate (109-3)

4,4-Difluoropiperidine (212 mg, 1.98 mmol) and triethylamine (200 mg, 1.98 mmol) were sequentially added to methanol (4.0 mL) at room temperature, stirred for 0.5 hours, and then compound **109-2** (240 mg, 1.98 mmol) and acetic acid (238 mg, 3.96 mmol) were added thereto, and the reaction was stirred for another 2 hours. Sodium cyanoborohydride (249 mg, 3.96 mmol) was added thereto, and the reaction was stirred for 3 hours. The reaction endpoint was monitored by TLC. The reaction mixture was quenched with water (10 mL) and concentrated under reduced pressure, and the residue was diluted with water (100 mL) and extracted with dichloromethane (50 mL × 3). The organic phases were combined, dried over anhydrous sodium sulfate, filtered, and the filtrate was concentrated under reduced pressure. The crude product was purified by flash silica gel column chromatography (dichloromethane: methanol = 50:1 to 20:1) to obtain the title product (180 mg, colorless oil).

MS (ESI) m/z [M+H]⁺ = 348.2.

### Step 4: Synthesis of 1-(2-(4,4-difluoropiperidin-1-yl)ethyl)piperazin-2-one (109-4)

Compound **109-3** (180 mg, 0.54 mmol) was dissolved in dichloromethane (4 mL) at room temperature, then a solution of hydrogen chloride in ethyl acetate (4 mL, 4.0 M) was added thereto. The reaction mixture was stirred at 24°C for 1 hour. The reaction endpoint was monitored by TLC. The reaction mixture was concentrated under reduced pressure to obtain the title product (150 mg, white solid).

### Step 5: Synthesis of 4-(6-((1-(4-(difluoromethyl)phenyl)-4-methyl-1H-1,2,3-triazol-5-yl)methoxy)pyridazin-3-yl)-1-(2-(4,4-difluoropiperidin-1-yl)ethyl)piperazin-2-one (109)

The experimental operation was as described in Example **1**, using intermediates **A8** and **109-4** as reactants to obtain the title product.

MS (ESI) m/z [M+H]⁺ = 563.1

¹H NMR (400 MHz, CDsOD) δ 7.77 (s, 4H), 7.35 (d, *J* = 9.6 Hz, 1H), 7.01(d, *J* = 9.6 Hz, 1H), 6.88 (t, *J* = 55.6 Hz, 1H), 5.50 (s, 2H), 4.16 (s, 2H), 3.83-3.80 (m, 2H), 3.62-3.57 (m, 4H), 2.64-2.61 (m, 6H), 2.47 (s, 3H), 1.95-1.86 (m, 4H).

### Example 110:

### 4-(6-((1-(4-(Difluoromethyl)phenyl)-4-methyl-1H-1,2,3-triazol-5-yl)methoxy)pyridazin-3-yl)-1-(2-(3,3-difluoropyrrolidin-1-yl)ethyl)piperazin-2-one (110)

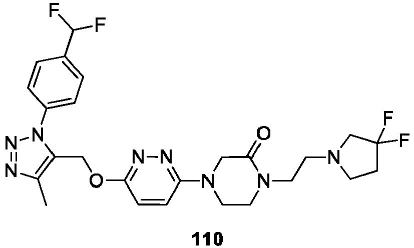

The experimental operation was as described in steps 3 to 5 in the synthesis of Example **109**, using intermediate **109-2**, 3,3-difluoropyrrolidine, and **A8** as reactants to obtain the title product.

MS (ESI) m/z [M+Na]⁺ = 571.3

¹H NMR (400 MHz, CDsOD) δ 7.76 (s, 4H), 7.34 (d, *J* = 9.6 Hz, 1H), 7.01 (d, *J* = 9.6 Hz, 1H), 6.88 (t, *J* = 55.6 Hz, 1H), 5.49 (s, 2H), 4.15 (s, 2H), 3.82-3.79 (m, 2H), 3.62-3.57 (m, 4H), 3.03 (t, *J* = 13.2 Hz, 2H), 2.87-2.85 (m, 2H), 2.77-2.75 (m, 2H), 2.47 (s, 3H), 2.27-2.20 (m, 2H).

### Example 111:

### 4-(6-((1-(4-(Difluoromethyl)phenyl)-4-methyl-1H-1,2,3-triazol-5-yl)methoxy)pyridazin-3-yl)-1-(1-methyl-1H-pyrazol-4-yl)piperazin-2-one (111)

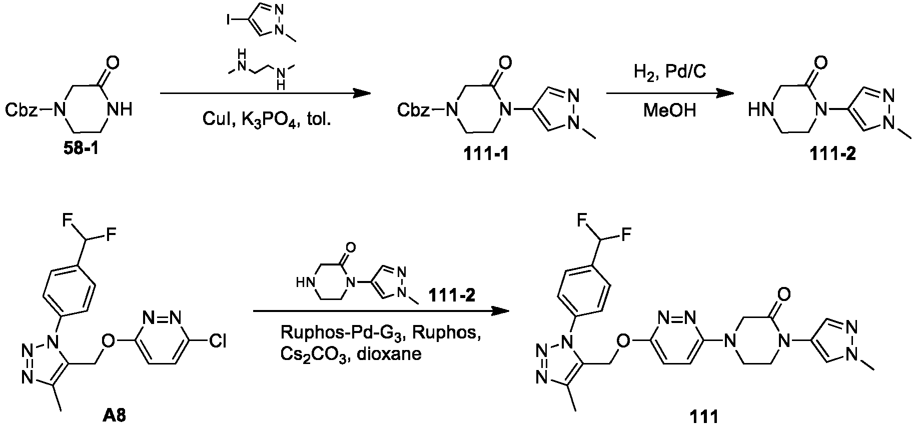

### Step 1: Synthesis of tert-butyl benzyl 4-(1-methyl-1H-pyrazol-4-yl)-3-oxopiperazine-1-carboxylate (111-1)

The experimental operation was as described in step 1 in Example **70**, using benzyl 3-oxopiperazine-1-carboxylate and 4-iodo-1-methyl-1*H*-pyrazole as reactants to obtain the title product.

MS (ESI) m/z [M+H]⁺ = 315.1.

### Step 2: Synthesis of 1-(1-methyl-1H-pyrazol-4-yl)piperazin-2-one (111-2)

The experimental operation was as described in step 2 in Example **58**, using intermediate **111-1** as the reactant to obtain the title product.

MS (ESI) m/z [M+H]⁺ = 181.1.

### Step 3: Synthesis of 4-(6-((1-(4-(difluoromethyl)phenyl)-4-methyl-1H-1,2,3-triazol-5-yl)methoxy)pyridazin-3-yl)-1-(1-methyl-1H-pyrazol-4-yl)piperazin-2-one (111)

The experimental operation was as described in Example **1**, using intermediates **A8** and **111-2** as reactants to obtain the title product.

MS (ESI) m/z [M+H]⁺ = 496.1.

¹H NMR (400 MHz, DMSO-*d*₆) δ 8.03 (s, 1H), 7.78 (s, 4H), 7.63 (s, 1H), 7.48 (d, *J* = 9.6 Hz, 1H), 7.12 (t, *J* = 55.6 Hz, 1H), 7.06 (d, *J* = 9.6 Hz, 1H), 5.45 (s, 2H), 4.21 (s, 2H), 3.88-3.85 (m, 2H), 3.80 (s, 3H), 3.76-3.74 (m, 2H), 2.39 (s, 3H).

### Example 112:

### 4-(6-((1-(4-(Difluoromethyl)phenyl)-4-methyl-1H-1,2,3-triazol-5-yl)methoxy)pyridazin-3-yl)-1-(1-methyl-1H-pyrazol-3-yl)piperazin-2-one (112)

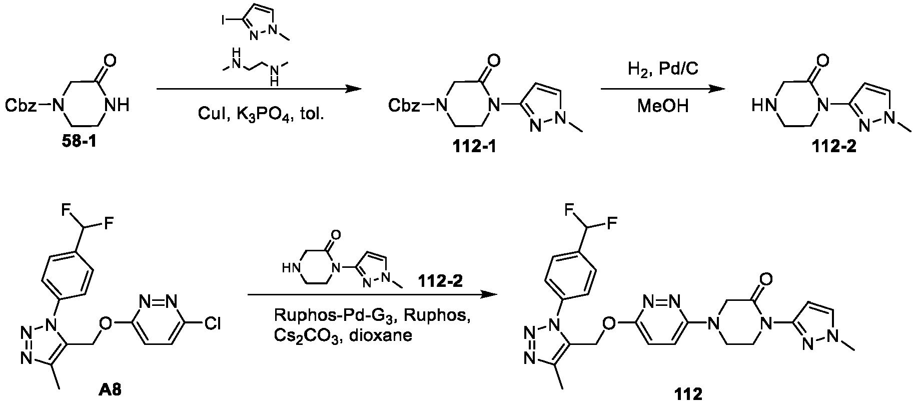

The experimental operation was as described in Example **111**, using benzyl 3-oxopiperazine-1-carboxylate, 3-iodo-1-methylpyrazole, and **A8** as raw materials, and undergoing the steps of coupling, deprotection, and coupling reactions to obtain the title product.

MS (ESI) m/z [M+H]⁺ = 496.1.

¹H NMR (400 MHz, DMSO-*d*₆) δ 7.77 (s, 4H), 7.61 (s, 1H), 7.43 (d, *J* = 9.6 Hz, 1H), 7.12 (t, *J* = 55.6 Hz, 1H), 7.05 (d, *J* = 9.6 Hz, 1H), 6.62 (s, 1H), 5.45 (s, 2H), 4.23 (s, 2H), 3.94-3.92 (m, 2H), 3.85-3.83 (m, 2H), 3.76 (s, 3H), 2.39 (s, 3H).

### Example 113:

### 1-(1-(Difluoromethyl)-1H-pyrazol-4-yl)-4-(6-((1-(4-(difluoromethyl)phenyl)-4-methyl-1H-1,2,3-triazol-5-yl)methoxy)pyridazin-3-yl)piperazin-2-one (113)

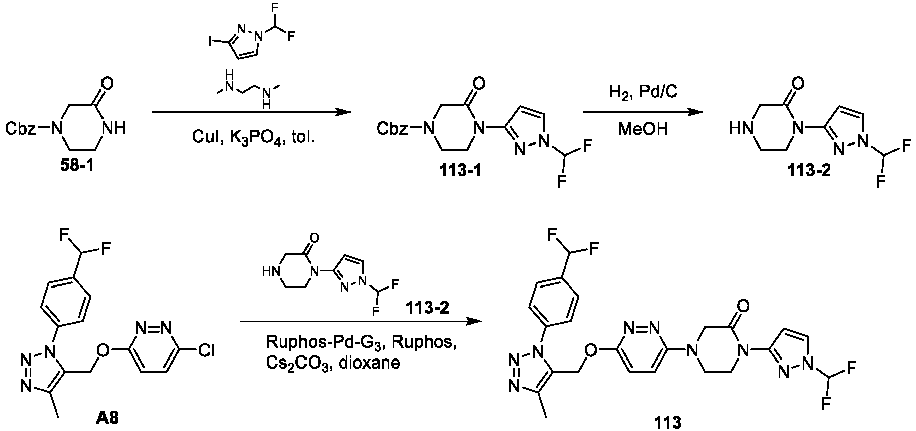

The experimental operation was as described in Example **111**, using benzyl 3-oxopiperazine-1-carboxylate, 3-iodo-1-(difluoromethyl)pyrazole, and **A8** as raw materials, and undergoing the steps of coupling, deprotection, and coupling reactions to obtain the title product.

MS (ESI) m/z [M+H]⁺ = 532.1

¹H NMR (400 MHz, DMSO-*d*₆) δ 8.51 (s, 1H), 8.09 (s, 1H), 7.92-7.63 (m, 5H), 7.49 (d, *J* = 9.6 Hz, 1H), 7.12 (t, *J* = 55.6 Hz, 1H), 7.07 (d, *J* = 9.6 Hz, 1H), 5.45 (s, 2H), 4.27 (s, 2H), 3.91-3.88 (m, 2H), 3.84-3.82 (m, 2H), 2.39 (s, 3H).

### Example 114:

### 4-(6-((1-(4-(Difluoromethyl)phenyl)-4-methyl-1H-1,2,3-triazol-5-yl)methoxy)pyridazin-3-yl)-1-(pyridin-pyridin-4-yl)piperazin-2-one (114)

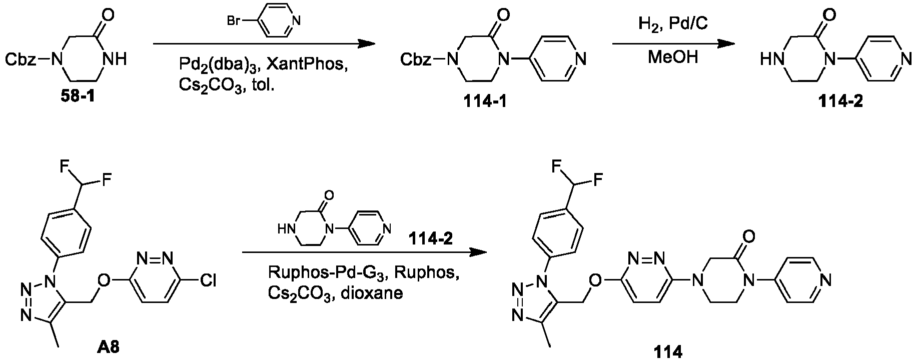

### Step 1: Synthesis of tert-butyl benzyl-3-oxo-4-(pyridin-4-yl)piperazine-1-carboxylate (114-1)

The experimental operation was as described in Example **18**, using benzyl 3-oxopiperazine-1-carboxylate and 4-bromopyridine as reactants and toluene as the solvent to obtain the title product.

MS (ESI) m/z [M+H]⁺ = 312.1.

### Step 2: Synthesis of 1-(pyridin-4-yl)piperazin-2-one (114-2)

The experimental operation was as described in step 2 in Example **58**, using intermediate **114-1** as the reactant to obtain the title product.

MS (ESI) m/z [M+H]⁺ = 178.1.

### Step 3: Synthesis of 4-(6-((1-(4-(difluoromethyl)phenyl)-4-methyl-1H-1,2,3-triazol-5-yl)methoxy)pyridazin-3-yl)-1-(pyridin-pyridin-4-yl)piperazin-2-one (114)

The experimental operation was as described in Example **1**, using intermediates **A8** and **114-2** to obtain the title product.

MS (ESI) m/z [M+H]⁺ = 493.1.

¹H NMR (400 MHz, DMSO-*d*₆) δ 8.55 (d, *J* = 5.6 Hz, 2H), 7.78 (s, 4H), 7.52 (d, *J* = 5.6 Hz, 2H), 7.44 (d, *J* = 9.6 Hz, 1H), 7.13 (t, *J* = 55.6 Hz, 1H), 7.07 (d, *J* = 9.6 Hz, 1H), 5.46 (s, 2H), 4.29 (s, 2H), 3.91-3.86 (m, 4H), 2.39 (s, 3H).

### Example 115:

### 2-(6-((1-(4-(Difluoromethyl)phenyl)-4-methyl-1H-1,2,3-triazol-5-yl)methoxy)pyridazin-3-yl)-N-ethyl-2-azabicyclo[2.1.1]hexane-1-carboxamide (115)

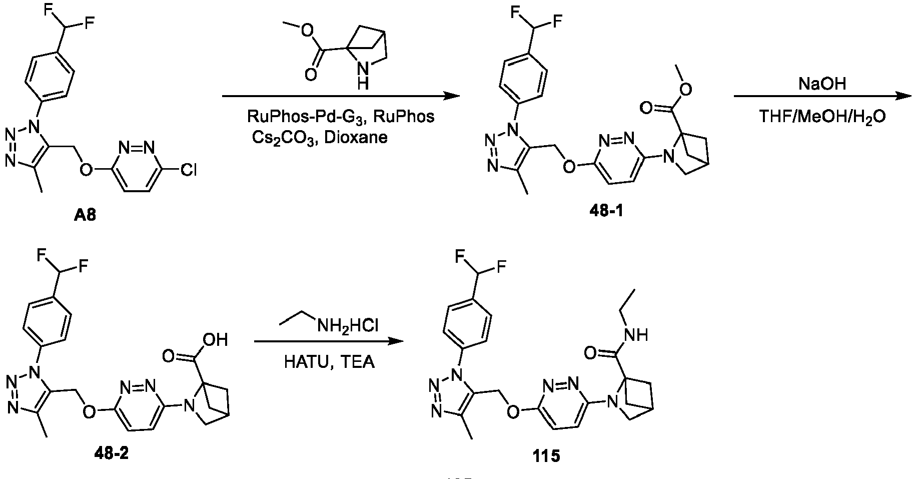

The experimental operation was as described in Example **40**, using intermediate **A8** and methyl 2-azabicyclo[2.1.1]hexane-1-carboxylate as raw materials, and undergoing the steps of coupling, hydrolysis, and condensation reactions to obtain the title product.

MS (ESI) m/z [M+H]⁺= 470.1.

¹H NMR (400 MHz, DMSO-*d*₆) δ7.79 (s, 4H), 7.53 (t, *J* = 5.6 Hz, 1H), 7.15 (t, *J* = 55.6 Hz, 1H), 7.06 (d, *J* = 9.4 Hz, 1H), 7.01 (d, *J* = 9.4 Hz, 1H), 5.44 (s, 2H), 4.44 (s, 2H), 3.08-3.03 (m, 2H), 2.79-2.77 (m, 1H), 2.39 (s, 3H), 2.04-2.02 (m, 2H), 1.58-1.57 (m, 2H), 0.96 (t, *J* = 7.2 Hz, 3H).

### Example 116:

### (1R,4R)-5-(6-((1-(4-(Difluoromethyl)phenyl)-4-methyl-1H-1,2,3-triazol-5-yl)methoxy)pyridazin-3-yl)-2,5-diazabicyclo[2.2.1]heptan-3-one (116)

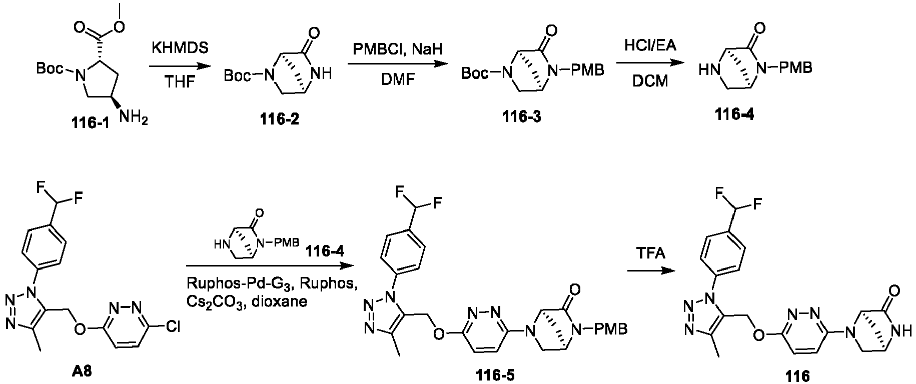

### Step 1: Synthesis of tert-butyl (1R,4R)-6-oxo-2,5-diazabicyclo[2.2.1]heptane-2-carboxylate (116-2)

Compound **116-1** (244 mg, 1.0 mmol) was dissolved in anhydrous tetrahydrofuran (10 mL) at 0°C under a nitrogen atmosphere, then KHMDS (1.2 mL, 1.0 M tetrahydrofuran solution) was added dropwise thereto, and the reaction was stirred at this temperature for 2 hours. The reaction endpoint was monitored by LCMS. The reaction was quenched by dropwise addition of acetic acid (1.0 mL) and diluted with water (50 mL) and ethyl acetate (50 mL) to obtain a white mixture. The mixture was filtered, and the filtrate was separated into phases. The organic phase was separated, dried over anhydrous sodium sulfate, filtered, and the filtrate was concentrated under reduced pressure to obtain the product (150 mg, white solid).

MS (ESI) m/z [M+H-56]⁺ = 157.1.

### Step 2: Synthesis of tert-butyl (1R,4R)-5-(4-methoxybenzyl)-6-oxo-2,5-diazabicyclo[2.2.1]heptane-2-carboxylate (116-3)

Compound **116-2** (150 mg, 0.71 mmol) and PMBCl (133 mg, 0.85 mmol) were dissolved in *N,N*-dimethylformamide (5 mL) under a nitrogen atmosphere at 0°C, then sodium hydride (57 mg, 0.14 mmol, 60%) was added thereto, and the reaction mixture was warmed to room temperature and stirred for 1 hour. The reaction endpoint was monitored by LCMS. The reaction mixture was quenched with water (20 mL) and extracted with ethyl acetate (30 mL × 2), and the organic phases were combined, dried over anhydrous sodium sulfate, filtered. The filtrate was concentrated under reduced pressure, and the residue was purified by flash silica gel column chromatography (ethyl acetate: petroleum ether = 0:1 to 4:1) to obtain the title product (160 mg, yellow oil).

MS (ESI) m/z [M+H]⁺ = 333.2.

### Step 3: Synthesis of (1R,4R)-2-(4-methoxybenzyl)-2,5-diazabicyclo[2.2.1]heptan-3-one (116-4)

Compound **116-3** (160 mg, 0.48 mmol) was dissolved in dichloromethane (5 mL) at room temperature, then a solution of hydrogen chloride in ethyl acetate (1.0 mL, 6.0 M) was added dropwise thereto, and the reaction mixture was stirred for 0.5 hours. The reaction endpoint was monitored by LCMS. The reaction mixture was poured into saturated sodium bicarbonate solution (50 mL) and extracted with dichloromethane (50 mL × 3). The organic phases were combined, dried over anhydrous sodium sulfate, filtered, and the filtrate was concentrated under reduced pressure to obtain the product (100 mg, yellow oil), which was used directly in the next reaction step without further purification.

MS (ESI) m/z [M+H]⁺ = 233.1.

### Step 4: Synthesis of (1R,4R)-5-(6-((1-(4-(difluoromethyl)phenyl)-4-methyl-1H-1,2,3-triazol-5-yl)methoxy)pyridazin-3-yl)-2-(4-methoxybenzyl)-2,5-diazabicyclo[2.2.1]heptan-3-one (116-5)

The experimental operation was as described in Example **1**, using intermediates **A8** and **116-4** as reactants to obtain the title product.

MS (ESI) m/z [M+H]⁺ = 547.2.

### Step 5: Synthesis of (1R,4R)-5-(6-((1-(4-(difluoromethyl)phenyl)-4-methyl-1H-1,2,3-triazol-5-yl)methoxy)pyridazin-3-yl)-2,5-diazabicyclo[2.2.1]heptan-3-one (116)

Compound **116-5** (110 mg, 0.018 mmol) was dissolved in trifluoroacetic acid (20 mL) at room temperature, and the reaction was heated to 100°C and stirred for 48 hours. The reaction endpoint was monitored by LCMS. The reaction mixture was concentrated under reduced pressure, and the residue was dissolved in saturated sodium bicarbonate (30 mL), and extracted with dichloromethane (50 mL × 3, containing 10% isopropanol). The organic phases were combined, dried over anhydrous sodium sulfate, filtered, and the filtrate was concentrated under reduced pressure. The residue was purified by reversed-phase C18 flash preparative chromatography (acetonitrile: water = 0 to 40%) to obtain the title product (5 mg, white solid).

MS (ESI) m/z [M+H]⁺ = 428.2.

¹H NMR (400 MHz, DMSO-*d*₆) δ 7.90-7.88 (m, 2H), 7.83-7.79 (m, 4H), 7.14 (t, *J* = 55.6 Hz, 1H), 6.46 (d, *J* = 9.4 Hz, 1H), 5.46 (s, 2H), 4.37-4.32 (m, 1H), 4.23-4.21 (m, 1H), 3.88-3.84 (m, 1H), 3.14-3.08 (m, 2H), 2.63-2.58 (m, 1H), 2.47 (s, 3H).

### Example 117:

### (S)-8-(6-((1-(4-(Difluoromethyl)phenyl)-4-methyl-1H-1,2,3-triazol-5-yl)methoxy)pyridazin-3-yl)hexahydro-1H-pyrazino[1,2-a]pyrazin-1,3-(2H)-dione (117)

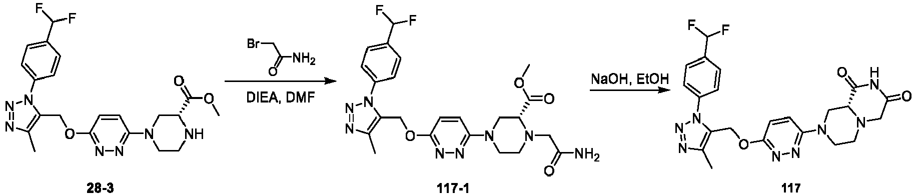

### Step 1: Synthesis of methyl (R)-1-(2-amino-2-oxoethyl)-4-(6-((1-(4-(difluoromethyl)phenyl)-4-methyl-1H-1,2,3-triazol-5-yl)methoxy)pyridazin-3-yl)piperazine-2-carboxylate (117-1)

Compound **28-3** (110 mg, 0.22 mmol), 2-bromoacetamide (37 mg, 0.27 mmol), and diisopropylethylamine (86 mg, 0.66 mol) were sequentially added to anhydrous *N,N-*dimethylformamide (4.0 mL) under a nitrogen atmosphere, and the reaction was heated to 80°C and stirred for 14 hours. The reaction endpoint was monitored by LCMS. The reaction mixture was diluted with ethyl acetate (100 mL), washed with saturated brine (10.0 mL × 3), then the organic phase was dried over anhydrous sodium sulfate, filtered, and the filtrate was concentrated under reduced pressure. The crude product was purified by flash silica gel column chromatography (dichloromethane: methanol = 100:1 to 20:1) to obtain the title product (65 mg, yellow solid).

MS (ESI) m/z [M+H]⁺ = 517.0.

¹H NMR (400 MHz, CDsOD) δ 7.76 (s, 4H), 7.33 (d, *J* = 6.0 Hz, 1H), 7.04-6.73 (m, 2H), 5.49 (s, 2H), 4.20 (dd, *J* = 3.2, 12.8 Hz, 1H), 3.89-3.80 (m, 1H), 3.70 (t, *J* = 4.0 Hz, 1H), 3.66 (s, 3H), 3.57 (dd, *J* = 3.6, 12.8 Hz, 1H), 3.44-3.32 (m, 3H), 3.22-3.14 (m, 1H), 2.71-2.63 (m, 1H), 2.47 (s, 3H).

### Step 2: Synthesis of (S)-8-(6-((1-(4-(difluoromethyl)phenyl)-4-methyl-1H-1,2,3-triazol-5-yl)methoxy)pyridazin-3-yl)hexahydro-1H-pyrazino[1,2-a]pyrazin-1,3-(2H)-dione (117)

Compound **117-1** (65 mg, 0.13 mmol) and sodium hydroxide (5 mg, 0.13 mmol) were sequentially added to anhydrous ethanol (5.0 mL) at room temperature, and the reaction was stirred for 1 hour. The reaction endpoint was monitored by TLC. The reaction mixture was quenched with saturated ammonium chloride aqueous solution (20.0 mL), extracted with ethyl acetate (20.0 mL × 3), and the organic phases were combined, dried over anhydrous sodium sulfate, and filtered. The filtrate was concentrated under reduced pressure, and the crude product was purified by flash silica gel column chromatography (dichloromethane: methanol = 100:1 to 10:1) to obtain the title product (33 mg, white solid).

MS (ESI) m/z [M+H]⁺ = 485.0

¹H NMR (400 MHz, CDsOD) δ 7.77 (s, 4H), 7.42 (d, *J* = 9.6 Hz, 1H), 7.02 (d, *J* = 9.6 Hz, 1H), 6.88 (t, *J* = 55.6 Hz, 1H), 5.50 (s, 2H), 4.38-4.35 (m, 1H), 3.92-3.89 (m, 1H), 3.63-3.59 (m, 1H), 3.36-3.34 (m, 1H), 3.25-3.20 (m, 3H), 3.00-2.98 (m, 1H), 2.52-2.51 (m, 1H), 2.47 (s, 3H).

### Example 118:

### (S)-7-(6-((1-(4-(Difluoromethyl)phenyl)-4-methyl-1H-1,2,3-triazol-5-yl)methoxy)pyridazin-3-yl)tetrahydroimidazo[1,5-a]pyrazine-1,3(2H,5H)-dione (118)

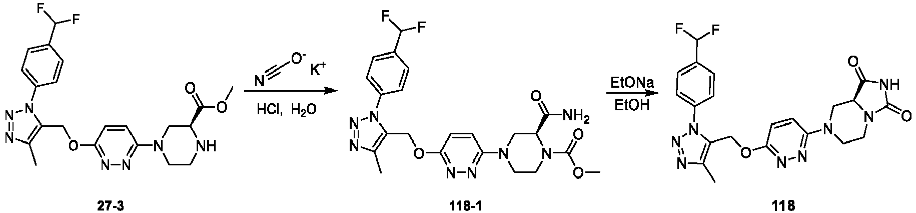

### Step 1: Synthesis of tert-butyl (S)-methyl 2-carbamoyl-4-(6-((1-(4-(difluoromethyl)phenyl)-4-methyl-1H-1,2,3-triazol-5-yl)methoxy)pyridazin-3-yl)piperazine-1-carboxylate (118-1)

Compound **27-3** (130 mg, 0.28 mmol) and concentrated hydrochloric acid (1 mL) were dissolved in dioxane (2 mL) at room temperature, then a solution of potassium isocyanate (46 mg, 0.57 mmol) in water (0.28 mL) was added thereto, and the reaction was stirred for 16 hours. The reaction endpoint was monitored by LCMS. The reaction mixture was concentrated under reduced pressure to remove the solvent. The crude product was added with water (30 mL) and extracted with dichloromethane (30.0 mL × 3). The organic phases were combined, dried over anhydrous sodium sulfate, filtered, and the filtrate was concentrated under reduced pressure to obtain the title product (134 mg, yellow solid).

MS (ESI) m/z [M+H]⁺ = 503.1.

### Step 2: Synthesis of (S)-7-(6-((1-(4-(difluoromethyl)phenyl)-4-methyl-1H-1,2,3-triazol-5-yl)methoxy)pyridazin-3-yl)tetrahydroimidazo[1,5-a]pyrazine-1,3(2H,5H)-dione (118)

Compound **118-1** (84 mg, 0.16 mmol) was dissolved in ethanol (3.0 mL) at room temperature, then sodium ethoxide (17 mg, 0.25 mmol) was added thereto, and the reaction was stirred for 3 hours. The reaction endpoint was monitored by TLC. The reaction mixture was concentrated under reduced pressure, and the crude product was purified by flash silica gel column chromatography (dichloromethane: methanol = 100:1 to 10:1) to obtain the title product (62 mg, off-white solid).

MS (ESI) m/z [M+H]⁺ = 471.0.

¹H NMR (400 MHz, DMSO-*d*₆) δ 10.97 (br. s, 1H), 7.79 (s, 4H), 7.53 (d, *J* = 9.6 Hz, 1H), 7.06 (d, *J* = 9.6 Hz, 1H), 7.14 (t, *J* = 55.6 Hz, 1H), 5.47 (s, 2H), 4.51-4.47 (m, 1H), 4.18-4.15 (m, 2H), 3.85-3.83 (m, 1H), 3.02-2.94 (m, 3H), 2.40 (s, 3H).

### Example 119:

### 6-(6-((1-(4-(Difluoromethyl)phenyl)-4-methyl-1H-1,2,3-triazol-5-yl)methoxy)pyridazin-3-yl)hexahydro-2H-pyrido[4,3-b][1,4]oxazin-3(4H-one (119)

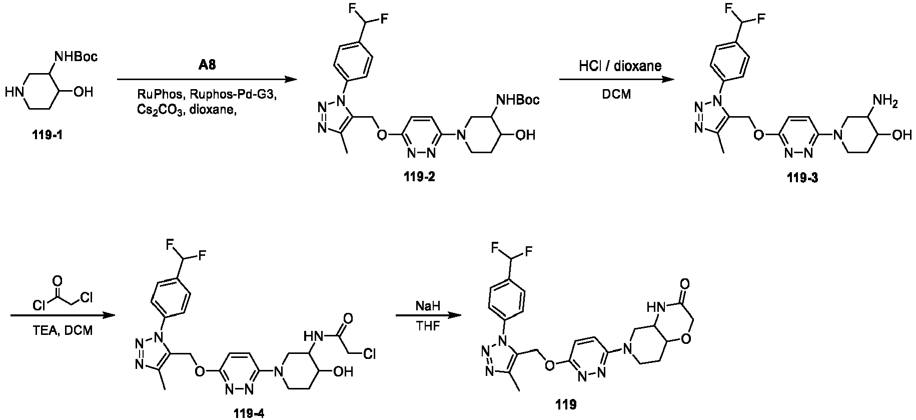

### Step 1: Synthesis of tert-butyl 1-(6-((1-(4-(difluoromethyl)phenyl)-4-methyl-1H-1,2,3-triazol-5-yl)methoxy)pyridazin-3-yl)-4-hydroxypiperidin-3-yl)carbamate (119-2)

The experimental operation was as described in Example **1**, using intermediates **A8** and **119-1** as reactants to obtain the title product.

MS (ESI) m/z [M+H]⁺ = 532.1.

¹H NMR (400 MHz, CDsOD) δ 7.77 (s, 4H), 7.38 (d, *J* = 9.6 Hz, 1H), 7.04-6.74 (m, 2H), 5.48 (s, 2H), 4.10-4.03 (m, 2H), 3.64-3.56 (m, 1H), 3.45-3.35 (m, 1H), 3.08-2.96 (m, 1H), 2.85 (dd, *J* = 9.6, 13.2 Hz, 1H), 2.47 (s, 3H), 2.08-2.02 (m, 1H), 1.66-1.53 (m, 1H), 1.45 (s, 9H).

### Step 2: Synthesis of 3-amino-1-(6-((1-(4-(difluoromethyl)phenyl)-4-methyl-1H-1,2,3-triazol-5-yl)methoxy)pyridazin-pyridin-3-yl)piperidin-4-ol (119-3)

The experimental operation was as described in step 4 in Example **109**, using intermediate **119-2** as the reactant to obtain the title product.

### Step 3: Synthesis of 2-chloro-N-(1-(6-((1-(4-(difluoromethyl)phenyl)-4-methyl-1H-1,2,3-triazol-5-yl)methoxy))pyridazin-3-yl)-4-hydroxypiperidin-3-yl)acetamide (119-4)

Compound **119-3** (35 mg, 0.075 mmol) and triethylamine (23 mg, 0.23 mml) were added to anhydrous dichloromethane (3.0 mL) under a nitrogen atmosphere, then chloroacetyl chloride (13 mg, 0.11 mol) was slowly added dropwise thereto, and the reaction was stirred at room temperature for 2 hours. The reaction endpoint was monitored by LCMS. The reaction mixture was diluted with dichloromethane (50.0 mL), and the organic phase was sequentially washed with saturated sodium bicarbonate aqueous solution (5.0 mL × 3) and saturated brine (5.0 mL × 3), dried over anhydrous sodium sulfate, filtered, and the filtrate was concentrated under reduced pressure. The title product was obtained (100 mg, brown oil).

MS (ESI) m/z [M+H]⁺ = 530.1.

### Step 4: Synthesis of 6-(6-((1-(4-(difluoromethyl)phenyl)-4-methyl-1H-1,2,3-triazol-5-yl)methoxy)pyridazin-3-yl)hexahydro-2H-pyrido[4,3-b][1,4]oxazin-3(4H)-one (119)

Compound **119-4** (38 mg, 0.075 mmol) was dissolved in anhydrous tetrahydrofuran (5.0 mL) at 0°C under a nitrogen atmosphere, then sodium hydride (6 mg, 0.15 mmol, 60%) was added thereto, and the reaction mixture was warmed to room temperature and stirred for 1 hour. The reaction endpoint was monitored by LCMS. The reaction mixture was quenched with saturated ammonium chloride aqueous solution (10.0 mL) and extracted with ethyl acetate (20.0 mL × 3). The organic phases were combined, dried over anhydrous sodium sulfate, filtered, and the filtrate was concentrated under reduced pressure. The crude product was purified by preparative thin-layer chromatography (dichloromethane: methanol = 15:1) to obtain the title product (3 mg, white solid).

MS (ESI) m/z [M+H]⁺ = 472.0.

¹H NMR (400 MHz, CDsOD) δ 7.77 (s, 4H), 7.37 (d, *J* = 9.6 Hz, 1H), 6.98 (d, *J* = 9.6 Hz, 1H), 6.89 (t, *J* = 55.6 Hz, 1H), 5.50 (s, 2H), 4.44-4.40 (m, 1H), 4.25-4.23 (m, 3H), 3.55-3.52 (m, 1H), 3.04-3.02 (m, 1H), 2.78-2.72 (m, 1H), 2.47 (s, 3H), 2.05-2.02 (m, 1H), 1.69-1.65 (m, 1H).

### Example 120:

### (S)-2-(6-((1-(4-(Difluoromethyl)phenyl)-4-methyl-1H-1,2,3-triazol-5-yl)methoxy)pyridazin-3-yl)octahydropyrazino[1,2-a][1,4]diaza-10(2H)-one (120)

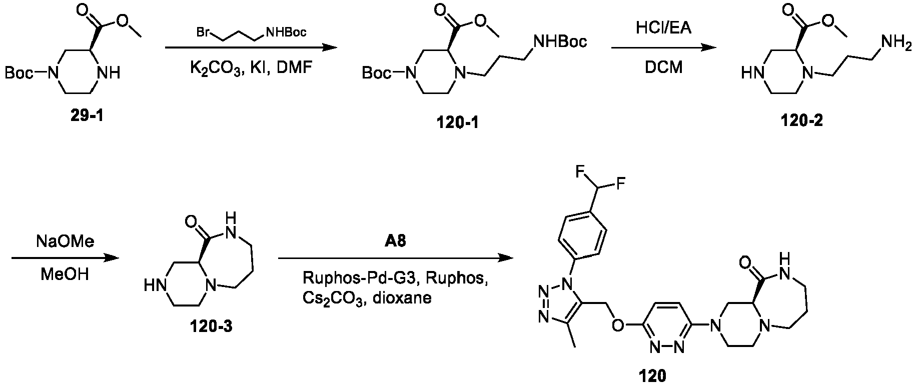

### Step 1: Synthesis of 1-tert-butyl 3-methyl (S)-4-(3-((tert-butoxycarbonyl)amino)propyl)piperazine-1,3-dicarboxylate (120-1)

Intermediate **29-1** (900 mg, 3.68 mmol) and *tert*-butyl (3-bromopropyl)carbamate (1.31 g, 5.52 mmol) were dissolved in DMF (20 mL) at room temperature, then potassium iodide (120 mg, 0.74 mmol) and potassium carbonate (1.02 g, 7.26 mmol) were sequentially added thereto, and the reaction mixture was stirred at 60°C for 16 hours. Product generation was shown by LCMS. The reaction was added with ice water (30 mL) and extracted with ethyl acetate (50 mL × 3). The organic phases were combined, dried over anhydrous sodium sulfate, and filtered, and the filtrate was concentrated under reduced pressure to obtain the crude product of the title product (1.3 g, yellow solid).

MS (ESI) m/z [M+H]⁺ = 402.3.

### Step 2: Synthesis of methyl (S)-1-(3-aminopropyl)piperazine-2-carboxylate (120-2)

The experimental operation was as described in step 2 in Example **59**, using intermediate **120-1** as the reactant to obtain the title product.

MS (ESI) m/z [M+H]⁺ = 202.3.

### Step 3: Synthesis of (S)-octahydropyrazino[1,2-a][1,4]diaza-10(2H)-one (120-3)

Intermediate **120-2** (500 mg, 1.82 mmol) was dissolved in anhydrous methanol (15 mL) at room temperature, then sodium methoxide (492 mg, 9.1 mmol) was added thereto, and the reaction mixture was stirred at room temperature for 16 hours. Product generation was shown by LCMS. The reaction mixture was concentrated under reduced pressure to obtain the crude product of the title product (300 mg, white solid).

MS (ESI) m/z [M+H]⁺ = 170.1.

### Step 4: Synthesis of (S)-2-(6-((1-(4-(difluoromethyl)phenyl)-4-methyl-1H-1,2,3-triazol-5-yl)methoxy)pyridazin-3-yl)octahydropyrazino[1,2-a][1,4]diaza-10(2H)-one (120)

The experimental operation was as described in Example **1**, using intermediates **A8** and **120-3** as reactants to obtain the title product.

MS (ESI) m/z [M+H]⁺ = 485.2.

¹H NMR (400 MHz, CDCl₃) δ 7.75 (s, 4H), 7.37 (d, *J=* 9.6 Hz, 1H), 6.93 (d, *J=* 9.6 Hz, 1H), 6.85 (t, *J* = 56.0 Hz, 1H), 5.46 (s, 2H), 3.83-3.81 (m, 1H), 3.75-3.73 (m, 1H), 3.56-3.45 (m, 3H), 3.23-3.18 (m, 2H), 3.00-2.97 (m, 2H), 2.73-2.71 (m, 1H), 2.47 (s, 3H), 1.82-1.80 (m, 1H), 1.73-1.71 (m, 1H), 0.85-0.83 (m, 1H).

### Example 121:

### 3-(6-((1-(4-(Difluoromethyl)phenyl)-4-methyl-1H-1,2,3-triazol-5-yl)methoxy)pyridazin-3-yl)-N-ethyl-3-azabicyclo[3.1.1]heptane-1-carboxamide (121)

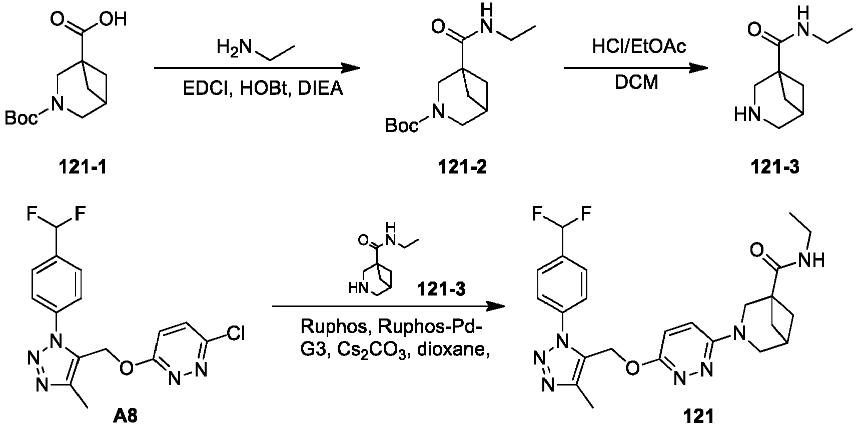

### Step 1: Synthesis of tert-butyl 1-(ethylcarbamoyl)-3-azabicyclo[3.1.1]heptane-3-carboxylate (121-2)

Compound **121-1** (100 mg, 0.41 mmol), ethylamine (50 mg, 0.62 mmol), 1-hydroxybenzotriazole (84 mg, 0.62 mmol), and diisopropylethylamine (84 mg, 0.62 mmol) were dissolved in anhydrous dichloromethane (4.0 mL) at room temperature, then EDCI (120 mg, 0.62 mmol) was added thereto, and the reaction was stirred at room temperature for 16 hours. The reaction endpoint was monitored by TLC. The reaction mixture was concentrated under reduced pressure, and the crude product was purified by flash silica gel column chromatography (dichloromethane: methanol = 1:0 to 10: 1) to obtain the title product (100 mg, yellow solid).

¹H NMR (400 MHz, CDCl₃) δ 5.49-5.25 (m, 1H), 3.56 (d, *J* = 6.8 Hz, 2H), 3.49-3.40 (m, 2H), 3.29-3.14 (m, 2H), 2.42-2.09 (m, 3H), 1.62-1.58 (m, 1H), 1.54-1.48 (m, 1H), 1.44-1.38 (m, 9H), 1.13-1.01 (m, 3H).

### Step 2: Synthesis of N-ethyl-3-azabicyclo[3.1.1]heptane-1-carboxamide (121-3)

The experimental operation was as described in step 4 in Example **109**, using intermediate **121-2** as the reactant to obtain the title product.

### Step 3: Synthesis of 3-(6-((1-(4-(difluoromethyl)phenyl)-4-methyl-1H-1,2,3-triazol-5-yl)methoxy)pyridazin-3-yl)-N-ethyl-3-azabicyclo[3.1.1]heptane-1-carboxamide (121)

The experimental operation was as described in Example **1**, using intermediates **A8** and **121-3** as reactants to obtain the title product.

MS (ESI) m/z [M+H]⁺ = 484.0.

¹H NMR (400 MHz, DMSO-*d*₆) δ 7.80 (s, 4H), 7.75 (t, *J* = 5.6 Hz, 1H), 7.29-6.99 (m, 3H), 5.46 (s, 2H), 3.63-3.55 (m, 4H), 3.14-3.07 (m, 2H), 2.51-2.49 (m, 1H), 2.41 (s, 3H), 2.27-2.24 (m, 2H), 1.58-1.56 (m, 2H), 1.03 (t, *J* = 7.2 Hz, 3H).

### Example 122:

### (S)-3-(6-((1-(4-(Difluoromethyl)phenyl)-4-methyl-1H-1,2,3-triazol-5-yl)methoxy)pyridazin-3-yl)-2,trans-3,4,4a-tetrahydro-1H-pyrazino[1,2-a]quinoxalin-5(6H)-one (122)

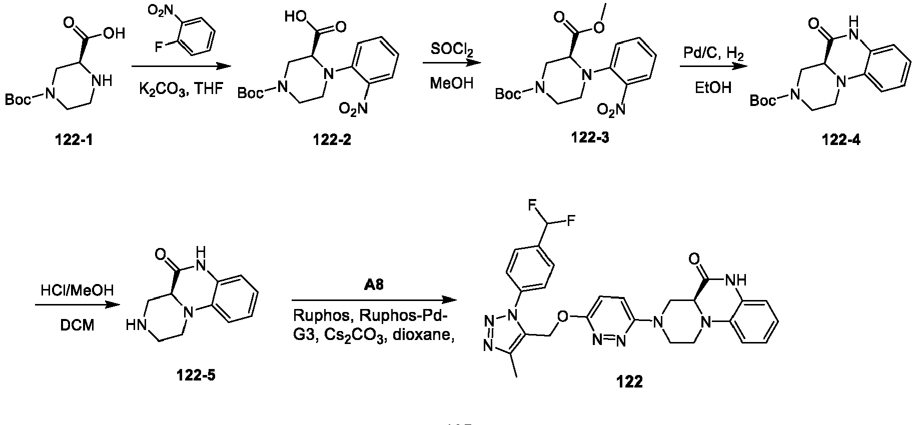

### Step 1: Synthesis of (S)-4-(tert-butoxycarbonyl)-1-(2-nitrophenyl)piperazine-2-carboxylic acid (122-2)

Compound **122-1** (2 g, 8.69 mmol) and potassium carbonate (4.8 g, 34.7 mmol) were dissolved in tetrahydrofuran (30 mL), then 1-fluoro-2-nitrobenzene (1.35 g, 9.55 mmol) was slowly added thereto, and the reaction mixture was heated to 60°C and stirred for 12 hours. The reaction endpoint was monitored by LCMS. The reaction mixture was filtered to obtain the crude product. The crude product was dissolved in ethyl acetate (15 mL) and water (10 mL). The aqueous phase was separated, then the pH of the aqueous phase was adjusted to 5.0 with dilute hydrochloric acid (1.0 M), and the aqueous phase was extracted with ethyl acetate (20 mL × 3). The organic phases were combined, dried over anhydrous sodium sulfate, filtered, and the filtrate was concentrated under reduced pressure to obtain the title product (1.1 g, yellow solid), which was used directly in the next reaction step without further purification.

MS (ESI) m/z [M+Na]⁺ = 374.0.

¹H NMR (400 MHz, CDCl₃) δ 7.80-7.78 (m, 1H), 7.49 (t, *J* = 7.2 Hz, 1H), 7.31 (d, *J* = 8.4 Hz, 1H), 7.11 (t, *J* = 7.6 Hz, 1H), 4.46-4.30 (m, 1H), 4.05-3.90 (m, 2H), 3.80-3.68 (m, 1H), 3.45-3.40 (m, 1H), 3.17 (t, *J* = 11.2 Hz, 1H), 2.94 (d, *J* = 11.6 Hz, 1H), 1.43 (s, 9H).

### Step 2: Synthesis of 1-tert-butyl 3-methyl (S)-4-(2-nitrophenyl)piperazine-1,3-dicarboxylate (122-3)

Compound **122-2** (400 mg, 1.14 mmol) was dissolved in methanol (10 mL) under a nitrogen atmosphere, then thionyl chloride (2.71 g, 22.8 mmol) was slowly added thereto, and the reaction was heated to 70°C and stirred for 2 hours. The reaction endpoint was monitored by LCMS. The reaction mixture was concentrated under reduced pressure, and the residue was dissolved in dichloromethane (20 mL) and water (5 mL). The pH of the aqueous phase was adjusted to 8.0 with saturated sodium bicarbonate aqueous solution. The mixture was extracted with dichloromethane (20 mL × 2). The organic phases were combined, dried over anhydrous sodium sulfate, filtered, and the residue was concentrated under reduced pressure to obtain the title product (330 mg, yellow solid), which was used directly in the next reaction step without further purification.

### Step 3: Synthesis of tert-butyl (S)-5-oxo-1,2,4,4a,5,6-hexahydro-3H-pyrazino[1,2-a]quinoxaline-3-carboxylate (122-4)

Compound **122-3** (210 mg, 0.57 mmol) was dissolved in ethanol (10 mL) under a nitrogen atmosphere, then palladium/carbon (20 mg, 10% purity) was added thereto, and the reaction mixture was stirred for 3 hours at 30°C under a hydrogen atmosphere (50 psi). The reaction endpoint was monitored by LCMS. The reaction mixture was filtered, and the filtrate was concentrated under reduced pressure to obtain the title product (100 mg, yellow oil), which was directly used in the next reaction step without further purification.

MS (ESI) m/z [M-Boc+H]⁺ = 204.1.

### Step 4: Synthesis of (S)-2,3,4,4a-tetrahydro-1H-pyrazino[1,2-a]quinoxalin-5(6H)-one (122-5)

The experimental operation was as described in step 4 in Example **109**, using intermediate **122-4** as the reactant to obtain the title product.

### Step 5: Synthesis of (S)-3-(6-((1-(4-(difluoromethyl)phenyl)-4-methyl-1H-1,2,3-triazol-5-yl)methoxy)pyridazin-3-yl)-2,trans-3,4,4a-tetrahydro-1H-pyrazino[1,2-a]quinoxalin-5(6H)-one (122)

The experimental operation was as described in Example **1**, using intermediates **A8** and **122-5** as reactants to obtain the title product.

MS (ESI) m/z [M+H]⁺ = 519.2.

¹H NMR (400 MHz, CDCl₃) δ 7.73-7.67 (m, 5H), 7.23-7.25 (m, 1H), 7.07 (t, *J* = 8.0 Hz, 1H), 6.94-6.57 (m, 5H), 5.47 (s, 2H), 4.63-4.61 (m, 1H), 4.53-4.50 (m, 1H), 3.76-3.66 (m, 2H), 3.27-3.19 (m, 2H), 3.01-2.97 (m, 1H), 2.50 (s, 3H).

### Example 123 and Example 124:

### cis-7-(6-((1-(4-(Difluoromethyl)phenyl)-4-methyl-1H-1,2,3-triazol-5-yl)methoxy)pyridazin-3-yl)octahydro-2,7-naphthyridin-1(2H)-one (123)

### trans-7-(6-((1-(4-(Difluoromethyl)phenyl)-4-methyl-1H-1,2,3-triazol-5-yl)methoxy)pyridazin-3-yl)octahydro-2,7-naphthyridin-1(2H)-one (124)

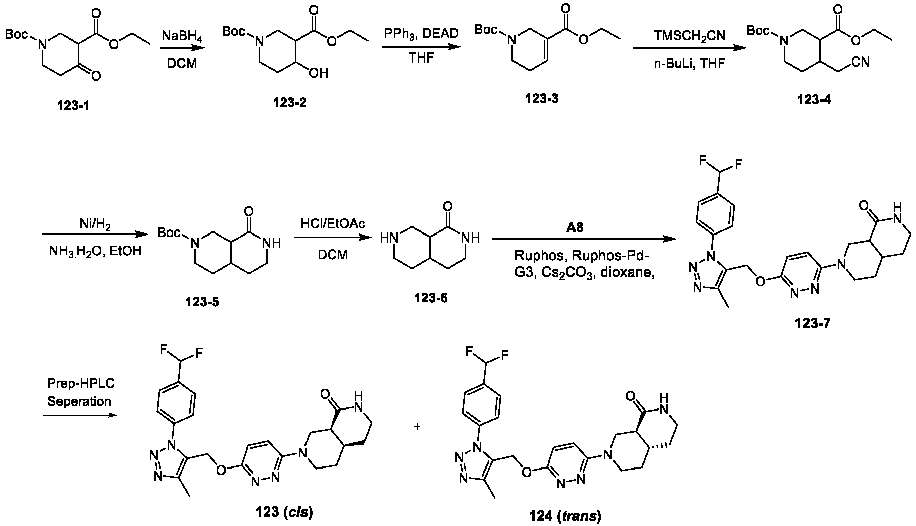

### Step 1: Synthesis of 1-tert-butyl 3-ethyl 4-hydroxypiperidine-1,3-dicarboxylate (123-2)

Compound **123-1** (5.0 g, 18.4 mmol) was dissolved in anhydrous ethanol (50.0 mL) at 0°C, then sodium borohydride (421 mg, 11.0 mmol) was added thereto, and the reaction mixture was stirred for 2 hours. The reaction endpoint was monitored by TLC. The reaction mixture was concentrated under reduced pressure and diluted with dichloromethane (100.0 mL). The organic phase was washed with 1.0 M sodium hydroxide aqueous solution (15 mL × 3), dried over anhydrous sodium sulfate, filtered, and the filtrate was concentrated under reduced pressure. The crude product was purified by flash silica gel column chromatography (petroleum ether: ethyl acetate = 5:1 to 3:1) to obtain the title product (2.30 g, colorless oil).

MS (ESI) m/z [M+H]⁺ = 217.9.

¹H NMR (400 MHz, CDsOD) δ 4.33 (s, 1H), 4.21-4.07 (m, 2H), 3.91 (d, *J* = 4.0 Hz, 1H), 3.76-3.65 (m, 1H), 3.48 (br. s, 1H), 3.26-3.20 (m, 1H), 2.60-2.56 (m, 1H), 1.94-1.76 (m, 1H), 1.72-1.60 (m, 1H), 1.45 (s, 9H), 1.31-1.23 (m, 3H)

### Step 2: Synthesis of 1-tert-butyl 3-ethyl 5,6-dihydropyridine-1,3(2H)-dicarboxylate (123-3)

Compound **123-2** (1.0 g, 3.66 mmol) and triphenylphosphine (2.39 g, 9.10 mmol) were sequentially added to anhydrous tetrahydrofuran (40.0 mL) at 0°C under a nitrogen atmosphere, then DEAD (1.27 g, 7.32 mmol) was added thereto, and the reaction was warmed to room temperature and stirred for 12 hours. The reaction endpoint was monitored by LCMS. Ethyl acetate (100 mL) was added to the reaction mixture. The organic phase was separated, washed with saturated brine (15.0 mL × 3), dried over anhydrous sodium sulfate, filtered, and the filtrate was concentrated under reduced pressure. The crude product was purified by flash silica gel column chromatography (petroleum ether: ethyl acetate = 1:0 to 5:1) to obtain the title product (830 mg, colorless oil).

MS (ESI) m/z [M+H]⁺ = 178.9.

¹H NMR (400 MHz, CDsOD) δ 7.11-7.05 (m, 1H), 4.26-4.18 (m, 2H), 4.09 (d, *J* = 2.0 Hz, 2H), 3.49 (s, 2H), 2.36-2.28 (m, 2H), 1.48 (s, 9H), 1.29 (t, *J* = 7.2 Hz, 3H)

### Step 3: Synthesis of 1-(tert-butyl) 3-ethyl 4-(cyanomethyl)piperidine-1,3-dicarboxylate (123-4)

Trimethylsilyl acetonitrile (107 mg, 0.840 mmol) was dissolved in anhydrous tetrahydrofuran (5.0 mL) at -78°C under a nitrogen atmosphere, then a solution of *n-*butyllithium in tetrahydrofuran (0.47 mL, 1.18 mmol, 2.5 M) was added thereto, and the reaction was stirred for 30 minutes. Compound **123-3** (200 mg, 0.784 mmol) was added thereto, and the reaction was stirred at this temperature for another 2 hours. The reaction endpoint was monitored by TLC. The reaction mixture was naturally warmed to room temperature, and water (15.0 mL) was added to quench the reaction. The reaction mixture was extracted with ethyl acetate (30.0 mL × 3), then the organic phases were combined, dried over anhydrous sodium sulfate, filtered, and the filtrate was concentrated under reduced pressure to obtain the crude product (500 mg). The obtained crude product was dissolved in acetonitrile (6.0 mL), then cesium carbonate (767 mg, 2.35 mmol) was added thereto, and the reaction mixture was stirred at room temperature for 12 hours. Ethyl acetate (100 mL) was added to the reaction mixture. The organic phase was washed with saturated brine (15.0 mL × 3), dried over anhydrous sodium sulfate, filtered, and the filtrate was concentrated under reduced pressure. The crude product was purified by flash silica gel column chromatography (petroleum ether: ethyl acetate = 1:0 to 5:1) to obtain the title product (100 mg, colorless oil).

¹H NMR (400 MHz, CDsOD) δ 4.25-4.12 (m, 2H), 3.26-3.05 (m, 1H), 3.00-2.73 (m, 2H), 2.70-2.28 (m, 3H), 2.23-2.09 (m, 1H), 1.92-1.86 (m, 1H), 1.84-1.43 (m, 11H), 1.33-1.24 (m, 3H)

### Step 4: Synthesis of tert-butyl 8-oxooctahydro-2,7-naphthyridine-2(1H)-carboxylate (123-5)

Compound **123-4** (310 mg, 1.04 mmol) was dissolved in ethanol (10.0 mL) at room temperature, then ammonia hydroxide (1.0 mL) and Raney nickel (200 mg) were sequentially added to the above mixture, and the reaction was stirred for 12 hours under a hydrogen atmosphere. The reaction endpoint was monitored by LCMS. The reaction mixture was filtered, and the filtrate was concentrated under reduced pressure to obtain the title product (120 mg, colorless oil).

MS (ESI) m/z [M+H]⁺ = 198.9.

¹H NMR (400 MHz, DMSO-*d*₆) δ 7.44-7.36 (m, 1H), 4.33 (br. s, 1H), 3.53 (br. s, 1H), 3.21-3.06 (m, 4H), 2.31-2.22 (m, 1H), 2.06 (d, *J* = 3.6 Hz, 1H), 1.79-1.51 (m, 4H), 1.38 (s, 9H).

### Step 5: Synthesis of octahydro-2,7-naphthyridin-1(2H)-one (123-6)

The experimental operation was as described in step 4 in Example **109**, using intermediate **123-5** as the reactant to obtain the title product.

### Step 6: Synthesis of 7-(6-((1-(4-(difluoromethyl)phenyl)-4-methyl-1H-1,2,3-triazol-5-yl)methoxy)pyridazin-3-yl)octahydro-2,7-naphthyridin-1(2H)-one (123-7)

The experimental operation was as described in Example **1**, using intermediates **A8** and **123-6** as reactants to obtain the title product.

### Step 7: Synthesis of cis-7-(6-((1-(4-(difluoromethyl)phenyl)-4-methyl-1H-1,2,3-triazol-5-yl)methoxy)pyridazin-3-yl)octahydro-2,7-naphthyridin-1(2H)-one (123) and trans-7-(6-((1-(4-(difluoromethyl)phenyl)-4-methyl-1H-1,2,3-triazol-5-yl)methoxy)pyridazin-3-yl)octahydro-2,7-naphthyridin-1(2H)-one (124)

The crude product **123-7** obtained in step 6 was purified by high performance liquid chromatography (separation conditions: chromatographic column: Phenomenex C18 150 * 40 mm * 5 µm; mobile phase: water (0.225% trifluoroacetic acid solution) - acetonitrile; gradient: B%: 5% to 35%/10 min; flow rate: 60 mL/min) to obtain the title compounds **123** (15 mg, white solid) and **124** (19 mg, white solid).

### Analytical data of compound 123:

HPLC retention time of 2.529 min; HPLC analysis conditions: chromatographic column: Ultimate C18 3 * 50 mm 3 µm; mobile phase: A: 1.5 mL trifluoroacetic acid/4L water, B: acetonitrile; gradient: B%: 10% to 80%/6 min; flow rate: 1.2 mL/min; column temperature: 50°C.

MS (ESI) m/z [M+H]⁺ = 470.2.

¹H NMR (400 MHz, CDsOD) δ 7.76 (s, 4H), 7.41 (d, *J* = 9.6 Hz, 1H), 7.03-6.75 (m, 2H), 5.47 (s, 2H), 4.36-4.35 (m, 1H), 4.16-4.12 (m, 1H), 3.41-3.37 (m, 1H), 3.27-3.25 (m, 1H), 3.20-3.16 (m, 1H), 3.10-3.03 (m, 1H), 2.59-2.58 (m, 1H), 2.47 (s, 3H), 2.30-2.26 (m, 1H), 1.95-1.92 (m, 2H), 1.73-1.67 (m, 2H).

### Analytical data of compound 124:

HPLC analysis retention time of 2.672 min; HPLC analysis conditions: chromatographic column: Ultimate C18 3 * 50 mm 3 µm; mobile phase: A: 1.5 mL trifluoroacetic acid/4L water, B: acetonitrile; gradient: B%: 10% to 80%/6 min; flow rate: 1.2 mL/min; column temperature: 50°C.

MS (ESI) m/z [M+H]⁺ = 470.1.

¹H NMR (400 MHz, CDsOD) δ 7.77 (s, 4H), 7.37 (d, *J* = 9.6 Hz, 1H), 7.02-6.74 (m, 2H), 5.48 (s, 2H), 4.57-4.55 (m, 1H), 4.31-4.27 (m, 1H), 3.37-3.33 (m, 2H), 2.88-2.87 (m, 1H), 2.76-2.70 (m, 1H), 2.47 (s, 3H), 2.13-2.12 (m, 1H), 1.88-1.77 (m, 3H), 1.65-1.55 (m, 1H), 1.41-1.45 (m, 1H).

### Example 125:

### N-Ethyl-1-(6-((1-(4-fluorophenyl)-4-methyl-1H-1,2,3-triazol-5-yl)methoxy)pyridazin-3-yl)azetidine-3-carboxamide (125)

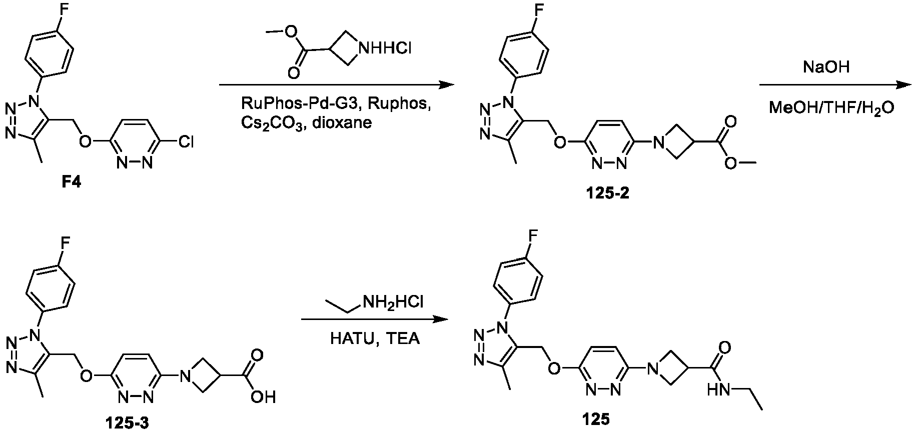

The experimental operation was as described in Examples **37** and **38**, using **F4** and methyl azetidine-3-carboxylate hydrochloride as raw materials, and undergoing the steps of coupling, hydrolysis, and condensation reactions to obtain the title product.

MS (ESI) m/z [M+H]⁺ = 412.2.

¹H NMR (400 MHz, DMSO-*d*₆) δ 7.99 (br. s, 1H), 7.68-7.64 (m, 2H), 7.48-7.44 (m, 2H), 6.97 (d, *J* = 9.6 Hz, 1H), 6.90 (d, *J* = 9.6 Hz, 1H), 5.38 (s, 2H), 4.07-4.03 (m, 2H), 3.96-3.93 (m, 2H), 3.44-3.41 (m, 1H), 3.12-3.05 (m, 2H), 2.37 (s, 3H), 1.00 (t, *J* = 7.2 Hz, 3H).

### Example 126:

### (S)-8-(6-((1-(4-Fluorophenyl)-4-methyl-1H-1,2,3-triazol-5-yl)methoxy)pyridazin-3-yl)octahydropyrazino[2,1-c][1,4]oxazine (126)

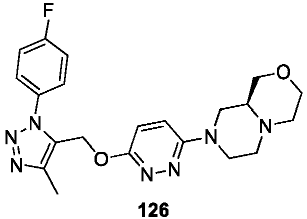

The experimental operation was as described in Example **1**, using **F4** and (*S*)-octahydropyrazino[2,1-c][1,4]oxazine hydrochloride as reactants to obtain the title product.

MS (ESI) m/z [M+H]⁺ = 426.2.

¹H NMR (400 MHz, DMSO-*d*₆) δ 7.68-7.64 (m, 2H), 7.44-7.36 (m, 3H), 6.99 (d, *J* = 9.6 Hz, 1H), 5.40 (s, 2H), 4.06-4.03 (m, 1H), 3.95-3.92 (m, 1H), 3.76-3.69 (m, 2H), 3.55-3.49 (m, 1H), 3.17-3.12 (m,1H), 2.90-2.84 (m, 1H), 2.80-2.77 (m, 1H), 2.67-2.64 (m, 1H), 2.43-2.40 (m, 1H), 2.37 (s, 3H), 2.23-2.14 (m, 3H).

### Example 127:

### 4-(6-((4-(4-(Difluoromethyl)phenyl)-1-methyl-1H-1,2,3-triazol-5-yl)methoxy)pyridazin-3-yl)piperazin-2-one (127)

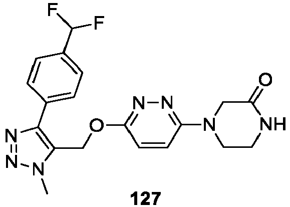

The experimental operation was as described in Example **1**, using intermediate **N7** and 2-piperazinone as reactants to obtain the title product.

MS (ESI) m/z [M+H]⁺ = 416.1.

¹H NMR (400 MHz, CDsOD) δ 7.87 (d, *J* = 8.0 Hz, 2H), 7.65 (d, *J* = 8.0 Hz, 2H), 7.38 (d, *J* = 9.6 Hz, 1H), 7.11 (d, *J* = 9.6 Hz, 1H), 6.82 (t, *J* = 55.6 Hz, 1H), 5.68 (s, 2H), 4.27 (s, 3H), 4.15 (s, 2H), 3.79-3.75 (m, 2H), 3.48-3.46 (m, 2H).

### Example 128:

### 4-(6-((4-(4-Fluorophenyl)-1-methyl-1H-1,2,3-triazol-5-yl)methoxy)pyridazin-3-yl)piperazin-2-one (128)

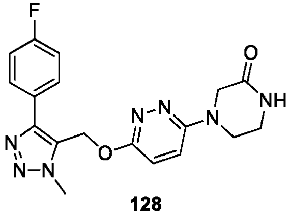

The experimental operation was as described in Example **1**, using intermediate **P6** and 2-piperazinone as reactants to obtain the title product.

MS (ESI) m/z [M+H]⁺ = 384.1.

¹H NMR (400 MHz, DMSO-*d*₆) δ 8.08 (s, 1H), 7.79-7.76 (m, 2H), 7.45 (d, *J* = 9.6 Hz, 1H), 7.32 (dd, *J* = 8.8, 9.2 Hz, 2H), 7.15 (d, *J* = 9.6 Hz, 1H), 5.60 (s, 2H), 4.16 (s, 3H), 4.00 (s, 2H), 3.70-3.67 (m, 2H), 3.30-3.28 (m, 2H).

### Example 129:

### (S)-8-(6-((4-Methyl-1-(p-tolyl)-1H-1,2,3-triazol-5-yl)methoxy)pyridazin-3-yl)octahydro-1H-pyrazino[1,2-a]pyrazin-1-one (129)

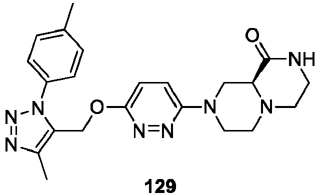

The experimental operation was as described in Example **1**, using intermediates **L4** and **77-4** as reactants to obtain the title product.

MS (ESI) m/z [M+H]⁺ = 435.2.

¹H NMR (400 MHz, DMSO-*d*₆) δ 7.79 (s, 1H), 7.46 (d, *J* = 7.9 Hz, 2H), 7.40-7.35 (m, 3H), 7.00 (d, *J* = 9.6 Hz, 1H), 5.37 (s, 2H), 4.47-4.39 (m, 1H), 4.03-3.96 (m, 1H), 3.32-3.29 (m, 1H), 3.09-3.04 (m, 1H), 2.94-2.84 (m, 3H), 2.65-2.61 (m, 2H), 2.43-2.36 (m, 7H), 2.28-2.22 (m, 1H).

### Example 130:

### (S)-8-(6-((1-(4-(Difluoromethoxy)phenyl)-4-methyl-1H-1,2,3-triazol-5-yl)methoxy)pyridazin-3-yl)octahydro-2H-pyrazino[1,2-a]pyrazin-1(6H)-one (130)

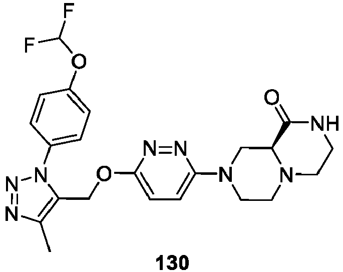

The experimental operation was as described in Example **1**, using intermediates **M4** and **77-4** as reactants to obtain the title product.

MS (ESI) m/z [M+H]⁺ = 487.2.

¹H NMR (400 MHz, DMSO-*d*₆) δ 7.79 (br. s, 1H), 7.69 (d, *J* = 8.4 Hz, 2H), 7.54-7.17 (m, 5H), 7.01 (d, *J* = 9.6 Hz, 1H), 5.42 (s, 2H), 4.49-4.41 (m, 1H), 4.04-4.01 (m, 1H), 3.29-3.25 (m, 1H), 3.09-3.07 (m, 1H), 2.92-2.85 (m, 3H), 2.67-2.59 (m, 2H), 2.44-2.41 (m, 1H), 2.39 (s, 3H), 2.30-2.24 (m, 3H).

### Example 131:

### 4-(6-((4-Chloro-1-(4-(difluoromethyl)phenyl)-1H-1,2,3-triazol-5-yl)methoxy)pyridazin-3-yl)piperazin-2-one (131)

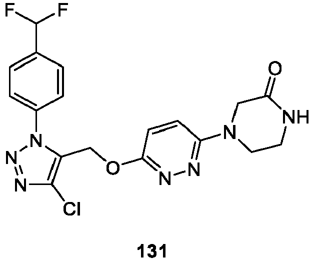

The experimental operation was as described in Example **1**, using intermediate **Q3** and 2-piperazinone as reactants to obtain the title product.

MS (ESI) m/z [M+H]⁺ = 436.1.

¹H NMR (400 MHz, CDsOD) δ 7.81-7.76 (m, 4H), 7.35 (d, *J* = 9.6 Hz, 1H), 7.01 (d, *J* = 9.6 Hz, 1H), 6.89 (t, *J* = 55.6 Hz, 1H), 5.52 (s, 2H), 4.12 (s, 2H), 3.77-3.74 (m, 2H), 3.47-3.44 (m, 2H).

### Example 132:

### 4-(6-((1-(4-(Difluoromethyl)phenyl)-1H-1,2,3-triazol-5-yl)methoxy)pyridazin-3-yl)piperazin-2-one (132)

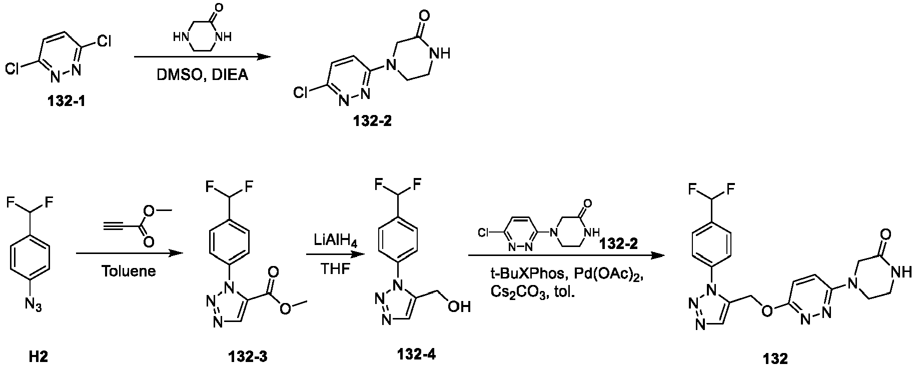

### Step 1: Synthesis of 4-(6-chloropyridazin-3-yl)piperazin-2-one (132-2)

Compound **132-1** (1.96 g, 13.2 mmol) was dissolved in dimethyl sulfoxide (30.0 mL) at room temperature, then *N,N-*diisopropylethylamine (5.13 g, 39.7 mmol) and 2-piperazinone (1.40 g, 14.0 mmol) were sequentially added thereto, and the reaction was heated to 130°C and stirred for 16 hours. The reaction endpoint was monitored by TLC. The reaction mixture was filtered, and the filter cake was washed with methyl *tert*-butyl ether (20.0 mL). The filter cake was collected and dried under vacuum to obtain the title product (1.70 g, brown solid).

MS (ESI) m/z [M+H]⁺ = 213.1.

¹H NMR (400 MHz, CDCl₃) δ 7.40 (d, *J* = 9.6 Hz, 1H), 7.15 (d, *J* = 9.6 Hz, 1H), 4.16 (s, 2H), 3.88 (t, *J* = 5.6 Hz, 2H), 3.48 (t, *J* = 5.6 Hz, 2H).

### Step 2: Synthesis of methyl 1-(4-(difluoromethyl)phenyl)-1H-1,2,3-triazole-5-carboxylate (132-3)

The experimental operation was as described in step 2 in the synthesis of intermediate **H5**, using **H2** and methyl propiolate as reactants to obtain the title product.

MS (ESI) m/z [M+H]⁺ = 254.1.

¹H NMR (400 MHz, CDCl₃) δ 8.28 (s, 1H), 7.71-7.69 (m, 2H), 7.62-7.60 (m, 2H), 6.75 (t, *J* = 56.0 Hz, 1H), 3.87 (s, 3H).

### Step 3: Synthesis of (1-(4-(difluoromethyl)phenyl)-1H-1,2,3-triazol-5-yl)methanol (132-4)

The experimental operation was as described in step 1 in the synthesis of intermediate **K3**, using **132-3** as the reactant to obtain the title product.

MS (ESI) m/z [M+H]⁺ = 226.1.

### Step 4: Synthesis of 4-(6-((1-(4-(difluoromethyl)phenyl)-1H-1,2,3-triazol-5-yl)methoxy)pyridazin-3-yl)piperazin-2-one (132)

Compound **132-4** (100.0 mg, 0.44 mmol) was dissolved in anhydrous toluene (3.0 mL) under a nitrogen atmosphere, and then cesium carbonate (434 mg, 1.33 mol), **132-2** (113.3 mg, 0.53 mmol), 2-di-*tert*-butylphosphino-2',4',6'-triisopropylbiphenyl (18.8 mg, 0.044 mmol), and palladium acetate (9.97 mg, 0.044 mmol) were sequentially added thereto, and the reaction was heated to 110°C and stirred for 16 hours. The reaction endpoint was monitored by TLC. The reaction mixture was cooled to room temperature, diluted with water (10.0 mL), and extracted with ethyl acetate (10.0 mL × 3). The organic phases were combined, concentrated under reduced pressure, and the residue was purified by high performance liquid chromatography (chromatographic column: Phenomenex C18 150 * 40 mm * 5 µm; mobile phase: water (0.225% trifluoroacetic acid solution) - acetonitrile]; B%: 12% to 42%; flow rate: 60 mL/min) to obtain the title product (12.7 mg, white solid).

MS (ESI) m/z [M+H]⁺ = 402.1.

¹H NMR (400 MHz, CD₃OD) δ 8.04 (s, 1H), 7.82-7.77 (m, 4H), 7.36 (d, *J=* 9.6 Hz, 1H), 7.04 (d, *J* = 9.6 Hz, 1H), 6.90 (t, *J* = 55.6 Hz, 1H), 5.57 (s, 2H), 4.13 (s, 2H), 3.77-3.74 (m, 2H), 3.47-3.44 (m, 2H).

### Example 133:

### 3-((1-(4-(Difluoromethyl)phenyl)-4-methyl-1H-1,2,3-triazol-5-yl)methoxy)-6-(3-oxopiperazin-1-yl)pyridazine-4-carbonitrile (133)

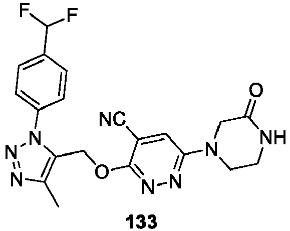

The experimental operation was as described in Example **1,** using intermediate **A12** and 2-piperazinone as reactants to obtain the title product.

MS (ESI) m/z [M+H]⁺ = 441.1.

¹H NMR (400 MHz, CDsOD) δ 7.80 (s, 1H), 7.79 (s, 4H), 6.91 (t, *J* = 55.6 Hz, 1H), 5.65 (s, 2H), 4.16 (s, 2H), 3.82-3.80 (m, 2H), 3.47-3.45 (m, 2H), 2.53 (s, 3H).

### Example 134 and Example 135:

### 4-(6-((1-(4-(Difluoromethyl)phenyl)-4-methyl-1H-1,2,3-triazol-5-yl)methoxy)-5-methoxypyridazin-3-yl)piperazin-2-one (134)

### 4-(6-((1-(4-(Difluoromethyl)phenyl)-4-methyl-1H-1,2,3-triazol-5-yl)methoxy)-4-methoxypyridazin-3-yl)piperazin-2-one (135)

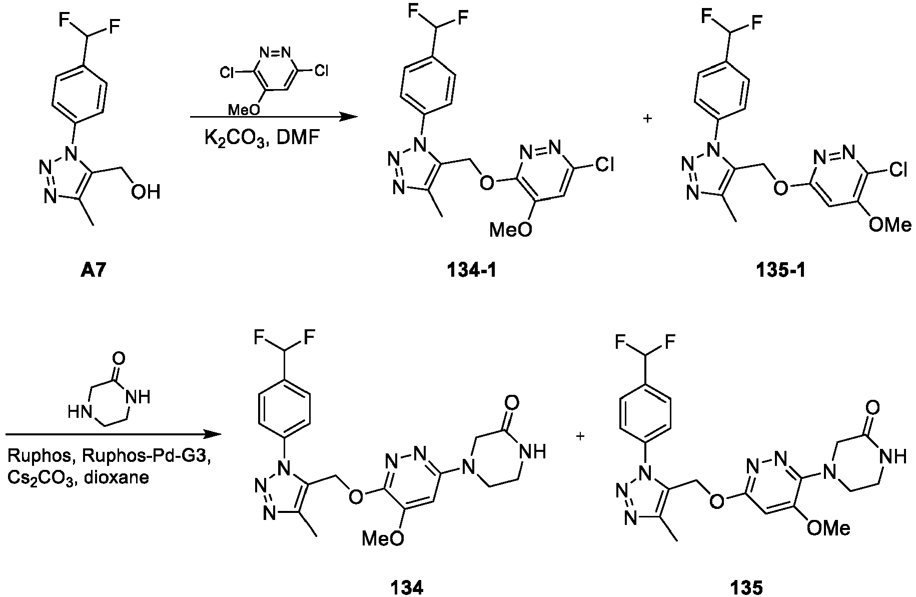

### Step 1: Synthesis of 6-chloro-3-((1-(4-(difluoromethyl)phenyl)-4-methyl-1H-1,2,3-triazol-5-yl)methoxy)-4-methoxypyridazine (134-1) and 3-chloro-6-((1-(4-(difluoromethyl)phenyl)-4-methyl-1H-1,2,3-triazol-5-yl)methoxy)-4-methoxypyridazine (135-1)

Intermediate **A7** (300 mg, 1.25 mmol) and 3,6-dichloro-4-methoxypyridazine (268 mg, 1.5 mmol) were dissolved in *N,N*-dimethylformamide (8 mL) at room temperature, then potassium carbonate (2.1 g, 15.0 mmol) was added thereto, and the reaction mixture was heated to 80°C and stirred for 1 hour. The reaction endpoint was monitored by TLC. The reaction mixture was cooled to room temperature, filtered, and the filtrate was diluted with ethyl acetate (150 mL). The organic phase was washed with saturated brine (150 mL × 3), dried over anhydrous sodium sulfate, filtered, and the filtrate was concentrated under reduced pressure. The residue was purified by flash silica gel column chromatography (ethyl acetate: petroleum ether = 0: 1 to 1:1) to obtain a mixture of the title products **134-1** and **135-1** (195 mg, white solid).

**134-1:** MS (ESI) m/z [M+H]⁺ = 382.1.

**135-1:** MS (ESI) m/z [M+H]⁺ = 382.1.

### Step 2: Synthesis of 4-(6-((1-(4-(difluoromethyl)phenyl)-4-methyl-1H-1,2,3-triazol-5-yl)methoxy)-5-methoxypyridazin-3-yl)piperazin-2-one (134) and 4-(6-((1-(4-(difluoromethyl)phenyl)-4-methyl-1H-1,2,3-triazol-5-yl)methoxy)-4-methoxypyridazin-3-yl)piperazin-2-one (135)

The experimental operation was as described in step 2 in the synthesis of Examples **81** and **82,** and the title products **134** and **135** were obtained respectively.

### Compound 134:

MS (ESI) m/z [M+H]⁺ = 446.2.

¹H NMR (400 MHz, DMSO-*d*₆) δ 8.06 (br. s, 1H), 7.78 (s, 4H), 7.14 (t, *J=* 55.6 Hz, 1H), 6.80 (s, 1H), 5.45 (s, 2H), 3.99 (s, 2H), 3.82 (s, 3H), 3.70-3.68 (m, 2H), 3.32-3.30 (m, 2H), 2.39 (s, 3H).

### Compound 135:

MS (ESI) m/z [M+H]⁺ = 446.2.

¹H NMR (400 MHz, DMSO-*d*₆) δ 7.86 (br. s, 1H), 7.79 (s, 4H), 7.13 (t, *J=* 55.6 Hz, 1H), 6.66 (s, 1H), 5.47 (s, 2H), 3.85 (s, 3H), 3.78 (s, 2H), 3.48-3.46 (m, 2H), 3.22-3.20 (m, 2H), 2.40 (s, 3H).

### Example 136 and Example 137:

### 4-(8-((1-(4-(Difluoromethyl)phenyl)-4-methyl-1H-1,2,3-triazol-5-yl)methoxy)pyrido[2,3-d]pyridazin-5-yl)piperazin-2-one (136)

### 4-(5-((1-(4-(Difluoromethyl)phenyl)-4-methyl-1H-1,2,3-triazol-5-yl)methoxy)pyrido[2,3-d]pyridazin-8-yl)piperazin-2-one (137)

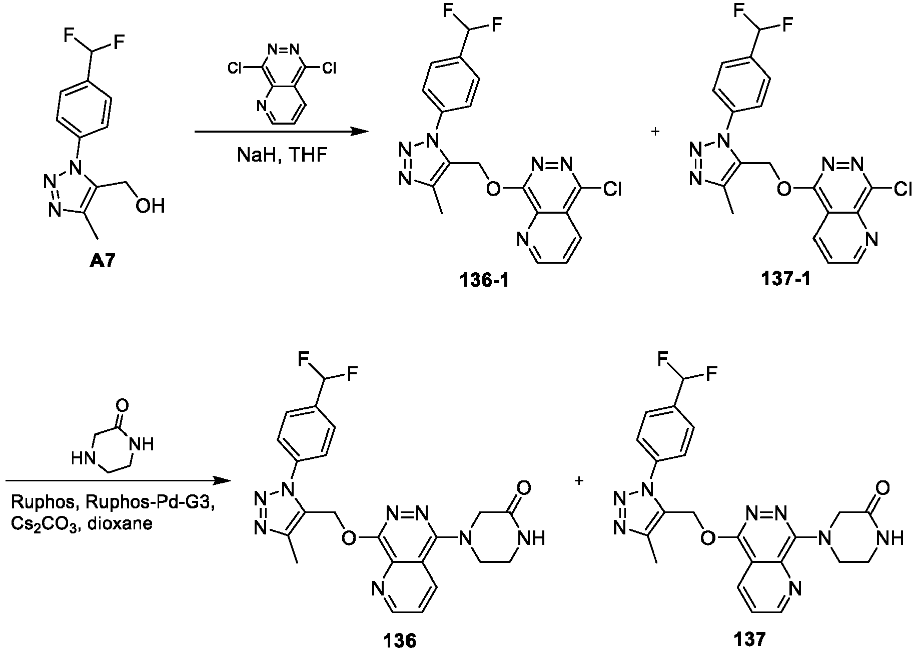

The experimental operation was as described in Examples **81** and **82,** using intermediate **A7** and 5,8-dichloropyrido[2,3-d]pyridazine as starting materials, and undergoing the steps of substitution and coupling reactions to obtain the title products **136** and **137,** respectively.

### Compound 136:

MS (ESI) m/z [M+H]⁺ = 467.3.

¹H NMR (400MHz, DMSO-*d*₆) δ 9.18 (dd, *J* = 2.0, 4.4 Hz, 1H), 8.51 (dd, *J* = 1.6, 8.4 Hz, 1H), 7.98-7.94 (m, 2H), 7.87 (d, *J* = 8.4 Hz, 2H), 7.75 (d, *J* = 8.4 Hz, 2H), 7.10 (t, *J* = 55.6 Hz, 1H), 5.68 (s, 2H), 3.91 (s, 2H), 3.54 (t, *J* =5.2 Hz, 2H), 3.39-3.36 (m, 2H), 2.46 (s, 3H).

### Compound 137:

MS (ESI) m/z [M+H]⁺ = 467.3.

¹H NMR (400MHz, DMSO-*d*₆) δ 9.18 (dd, *J* = 2.0, 4.4 Hz, 1H), 8.15 (dd, *J* = 1.6, 8.4 Hz, 1H), 7.99 (s, 1H), 7.86-7.82 (m, 3H), 7.75 (d, *J=* 8.4 Hz, 2H), 7.10 (t, *J* = 55.6 Hz, 1H), 5.71 (s, 2H), 4.26 (s, 2H), 4.04 (t, *J* =5.2 Hz, 2H), 3.36-3.34 (m, 2H), 2.46 (s, 3H).

### Example 138

### 1-(2-(1H-Pyrazol-1-yl)ethyl)-4-(6-((1-(4-(difluoromethyl)phenyl)-4-methyl-1H-1,2,3-triazol-5-yl)methoxy)pyridazin-3-yl)piperazin-2-one (138)

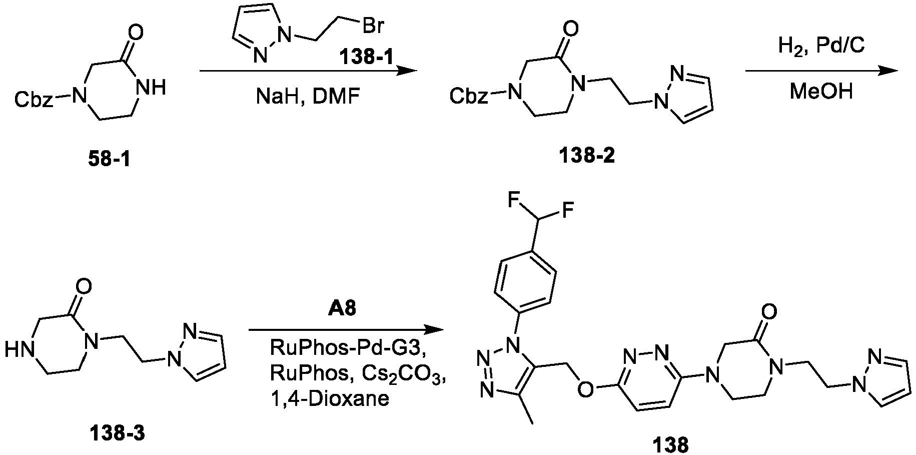

The experimental operation was as described in step 1 of Example **59** and steps 2 to 3 of Example **58,** using compounds **58-1** and **138-1** as starting materials to obtain the title product.

MS (ESI) m/z [M+H]⁺ = 510.3.

¹H NMR (400 MHz, DMSO-*d*₆) δ 7.78 (s, 4H), 7.63 (s, 1H), 7.40 (s, 1H), 7.37 (d, *J* = 9.6 Hz, 1H), 7.12 (t, *J* = 55.6 Hz, 1H), 7.03 (d, *J* = 9.6 Hz, 1H), 6.14 (s, 1H), 5.45 (s, 2H), 4.26 (t, *J* = 5.8 Hz, 2H), 4.02 (s, 2H), 3.66 (t, *J* = 5.8 Hz, 2H), 3.58 (t, *J* = 4.8 Hz, 2H), 2.97 (t, *J* = 4.8 Hz, 2H), 2.39 (s, 3H).

### Example 139

### 4-(6-((1-(4-(Difluoromethyl)phenyl)-4-methyl-1H-1,2,3-triazol-5-yl)methoxy)pyridazin-3-yl)-1-(2-methoxyethyl)piperazin-2-one (139)

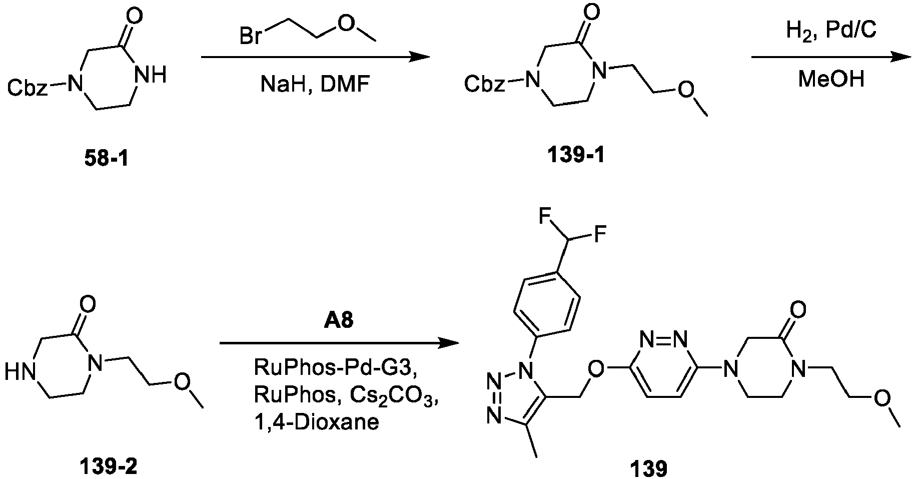

The experimental operation was as described in step 1 of Example **59** and steps 2 to 3 of Example **58,** using compound **58-1** and 2-bromoethyl methyl ether as starting materials to obtain the title product.

MS (ESI) m/z [M+H]⁺ = 474.2.

¹H NMR (400 MHz, DMSO-*d*₆) δ 7.77 (s, 4H), 7.39 (d, *J* = 9.6 Hz, 1H), 7.12 (t, *J* = 56.0 Hz, 1H), 7.03 (d, *J* = 9.6 Hz, 1H), 5.44 (s, 2H), 4.03 (s, 2H), 3.77 - 3.63 (m, 2H), 3.54 - 3.41 (m, 6H), 3.21 (s, 3H), 2.38 (s, 3H).

### Example 140

### 4-(6-((1-(4-(Difluoromethyl)phenyl)-4-methyl-1H-1,2,3-triazol-5-yl)methoxy)pyridazin-3-yl)-1-(3-methoxypropyl)piperazin-2-one (140)

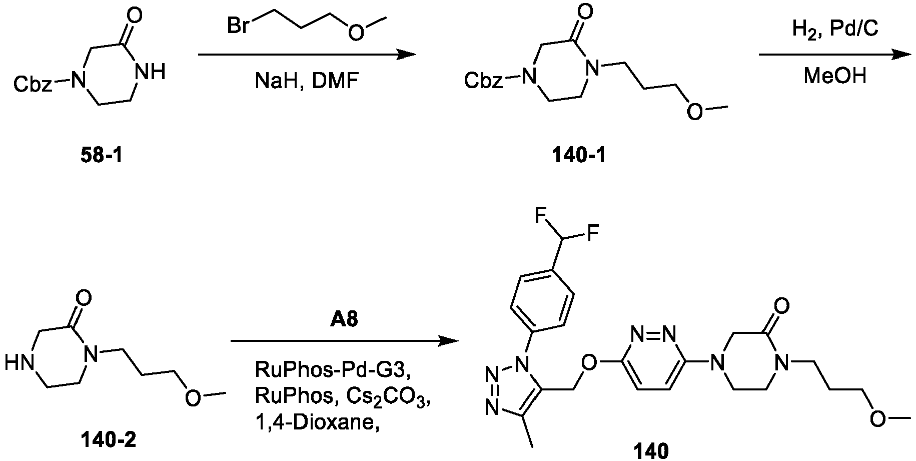

The experimental operation was as described in step 1 of Example **59** and steps 2 to 3 of Example **58,** using compound **58-1** and 3-bromopropyl methyl ether as starting materials to obtain the title product.

MS (ESI) m/z [M+H]⁺ = 488.2.

¹H NMR (400 MHz, DMSO-*d*₆) δ 7.77 (s, 4H), 7.39 (d, *J* = 9.6 Hz, 1H), 7.12 (d, *J* = 55.6 Hz, 1H), 7.02 (t, *J* = 9.6 Hz, 1H), 5.44 (s, 2H), 4.01 (s, 2H), 3.77 - 3.68 (m, 2H), 3.22-3.40 (m, 6H), 3.21 (s, 3H), 2.38 (s, 3H), 1.73 - 1.68 (m, 2H).

### Example 141

### 4-(6-((1-(4-(Difluoromethyl)phenyl)-4-methyl-1H-1,2,3-triazol-5-yl)methoxy)pyridazin-3-yl)-1-(2-methoxypropyl)piperazin-2-one (141)

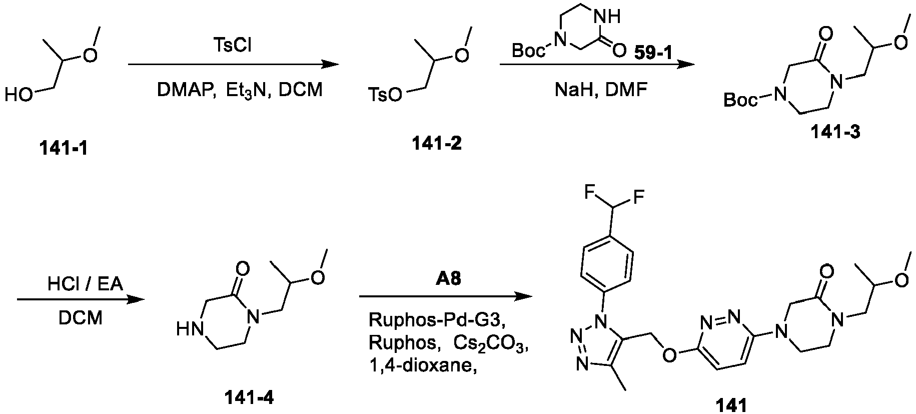

### Step 1: Synthesis of 2-methoxypropyl 4-methylbenzenesulfonate (141-2)

Compound **141-1** (1.0 g, 11.1 mmol) was dissolved in pyridine (12 mL) and dichloromethane (12 mL), then *p*-toluenesulfonyl chloride (2.5 g, 13.3 mmol) was added thereto, and the reaction mixture was stirred at 16°C for 16 hours. TLC showed that new points were generated. The reaction mixture was concentrated under reduced pressure, and the residue was diluted with water (20 mL) and extracted with EA (20 mL × 2). The organic phase was washed with saturated brine (30 mL × 2), dried over anhydrous sodium sulfate, and filtered. The filtrate was concentrated under reduced pressure to obtain the title product (2.1 g, colorless oil).

¹H NMR (400 MHz, CDCl₃) δ 7.79 (d, *J* = 8.4 Hz, 2H), 7.34 (d, *J* **=** 8.4 Hz, 2H), 3.95 (d, *J* = 5.6 Hz, 2H), 3.60 - 3.52 (m, 1H), 3.28 (s, 3H), 2.44 (s, 3H), 1.10 (d, *J* = 6.4 Hz, 3H).

### Steps 2 to 4: Synthesis of 4-(6-((1-(4-(difluoromethyl)phenyl)-4-methyl-1H-1,2,3-triazol-5-yl)methoxy)pyridazin-3-yl)-1-(2-methoxypropyl)piperazin-2-one (141)

The experimental operation was as described in Example **59,** using **141-2** and **59-1** as raw materials to obtain the title product.

MS (ESI) m/z [M+H]⁺ = 488.0.

¹H NMR (400 MHz, CDsOD) δ 7.76 (s, 4H), 7.34 (d, *J* = 9.6 Hz, 1H), 7.04 - 6.72 (m, 2H), 5.50 (s, 2H), 4.92 -4.90 (m, 2H), 4.16 (s, 2H), 3.84 - 3.76 (m, 2H), 3.66 - 3.56 (m, 4H), 3.38 - 3.33 (m, 1H), 3.31 (s, 1 H), 2.47 (s, 3H), 1.13 (d, *J* = 6.2 Hz, 3H)_{∘}

### Example 142

### 4-(6-((1-(4-(Difluoromethyl)phenyl)-4-methyl-1H-1,2,3-triazol-5-yl)methoxy)pyridazin-3-yl)-1-(1-isopropylazetidin-3-yl)piperazin-2-one (142)

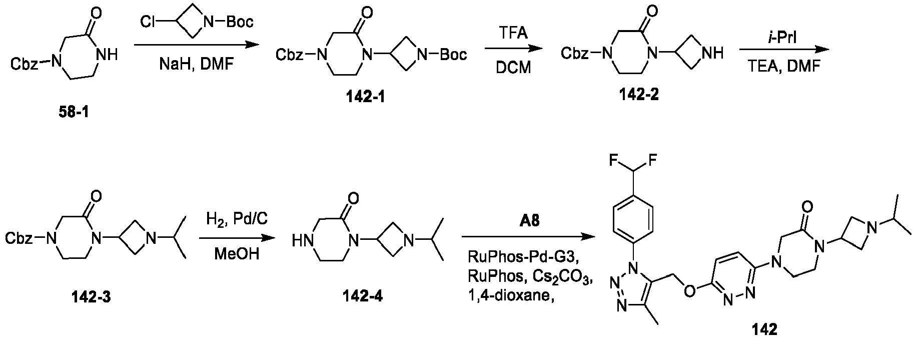

### Step 1: Synthesis of benzyl 4-(1-(tert-butoxycarbonyl)azetidin-3-yl)-3-oxopiperazine-1-carboxylate (142-1)

The experimental operation was as described in step 1 in Example **59,** using **58-1** and *tert*-butyl 3-chloroazetidine-1-carboxylate as raw materials to obtain the title product.

MS (ESI) m/z [M+H]⁺ = 390.2.

### Step 2: Synthesis of benzyl 4-(azetidin-3-yl)-3-oxopiperazine-1-carboxylate (142-2)

The experimental operation was as described in step 2 in Example **27,** using **142-1** as the raw material to obtain the title product.

MS (ESI) m/z [M+H]⁺ = 290.1.

### Step 3: Synthesis of benzyl 4-(1-isopropylazetidin-3-yl-3-oxopiperazine-1-carboxylate (142-3)

**142-2** (300 mg, 1.04 mmol) was dissolved in DMF (7 mL), then 2-iodopropane (212 mg, 1.25 mmol) and triethylamine (210 mg, 2.08 mmol) were added thereto under a nitrogen atmosphere, and the reaction was stirred at room temperature overnight. LCMS showed that the reaction was complete. The reaction mixture was added with ethyl acetate and washed with saturated sodium chloride solution (3 × 20 mL). The organic phases were combined, dried over anhydrous ammonium sulfate, filtered, and subjected to rotary evaporation. The crude product was purified by flash silica gel column chromatography (petroleum ether: ethyl acetate = 1:1) to obtain the title product (260 mg, crude product).

MS (ESI) m/z [M+H]⁺ = 332.2.

### Step 4: Synthesis of 1-(1-isopropylazetidin-3-yl)piperazin-2-one (142-4)

The experimental operation was as described in step 3 in Example **58,** using **142-3** as the raw material to obtain the title product.

MS (ESI) m/z [M+H]⁺ = 198.2

### Step 5: Synthesis of 4-(6-((1-(4-(difluoromethyl)phenyl)-4-methyl-1H-1,2,3-triazol-5-yl)methoxy)pyridazin-3-yl)-1-(1-isopropylazetidin-3-yl)piperazin-2-one (142)

The experimental operation was as described in Example **1,** using **A8** and **142-4** as raw materials to obtain the title product.

MS (ESI) m/z [M+H]⁺ = 513.2.

¹H NMR (400 MHz, DMSO-*d*₆) δ 7.78 (s, 4H), 7.41 (d, *J* = 9.6 Hz, 1H), 7.14 (t, *J*= 55.6 Hz, 1H), 7.04 (d, *J* = 9.6 Hz, 1H), 5.45 (s, 2H), 4.72 (s, 1H), 4.05 (s, 2H), 3.77 - 3.72 (m, 2H), 3.57 - 3.50 (m, 4H), 3.42 - 3.39 (m, 2H), 2.42 - 2.34 (m, 4H), 0.91 (s, 6H).

### Example 143

### 4-(4-(6-((1-(4-(Difluoromethyl)phenyl)-4-methyl-1H-1,2,3-triazol-5-yl)methoxy)pyridazin-3-yl)-2-oxopiperazin-1-yl)butanenitrile (143)

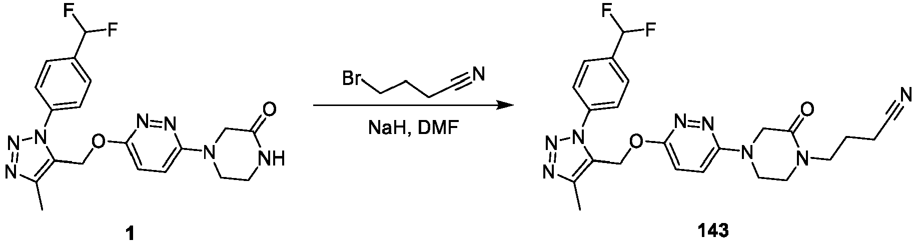

The experimental operation was as described in Example **26,** using compound **1** and 4-bromobutanenitrile raw materials to obtain the title product.

MS (ESI) m/z [M+H]⁺ = 483.2.

¹H NMR (400 MHz, DMSO-*d*₆) δ 7.77 (s, 4H), 7.40 (d, *J* = 9.6 Hz, 1H), 7.13 (t, *J* = 55.6 Hz, 1H), 7.03 (d, *J* = 9.6 Hz, 1H), 5.45 (s, 2H), 4.03 (s, 2H), 3.81 - 3.65 (m, 2H), 3.46 - 3.36 (m, 4H), 2.47 - 2.42 (m, 2H), 2.38 (s, 3H), 1.87 - 1.71 (m, 2H).

### Example 144

### 1-(3-(4-Chlorophenyl)propyl)-4-(6-((1-(4-(difluoromethyl)phenyl)-4-methyl-1H-1,2,3-triazol-5-yl)methoxy)pyridazin-3-yl)piperazin-2-one (144)

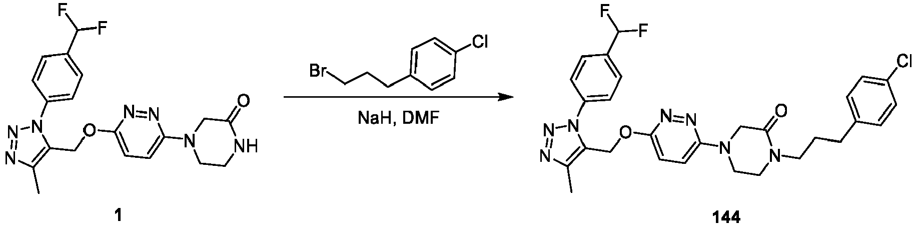

The experimental operation was as described in Example **26,** using compound **1** and 1-(3-bromopropyl)-4-chlorobenzene as raw materials to obtain the title product.

MS (ESI) m/z [M+H]⁺ = 568.2.

¹H NMR (400 MHz, DMSO-*d*₆) δ 7.77 (s, 4H), 7.40 (d, *J* = 9.6 Hz, 1H), 7.30 (d, *J* = 8.4 Hz, 2H), 7.22 (d, *J* = 8.4 Hz, 2H), 7.12 (t, *J* = 55.6 Hz, 1H), 7.03 (d, *J* = 9.6 Hz, 1H), 5.44 (s, 2H), 4.01 (s, 2H), 3.70 (t, *J* = 5.4 Hz, 2H), 3.44 - 3.37 (m, 4H), 2.56 - 2.49 (m, 2H), 2.38 (s, 3H), 1.85 - 1.67 (m, 2H).

### Example 145

### 4-(3-(4-(6-((1-(4-(Difluoromethyl)phenyl)-4-methyl-1H-1,2,3-triazol-5-yl)methoxy)pyridazin-3-yl)-2-oxopiperazin-1-yl)propyl)benzonitrile (145)

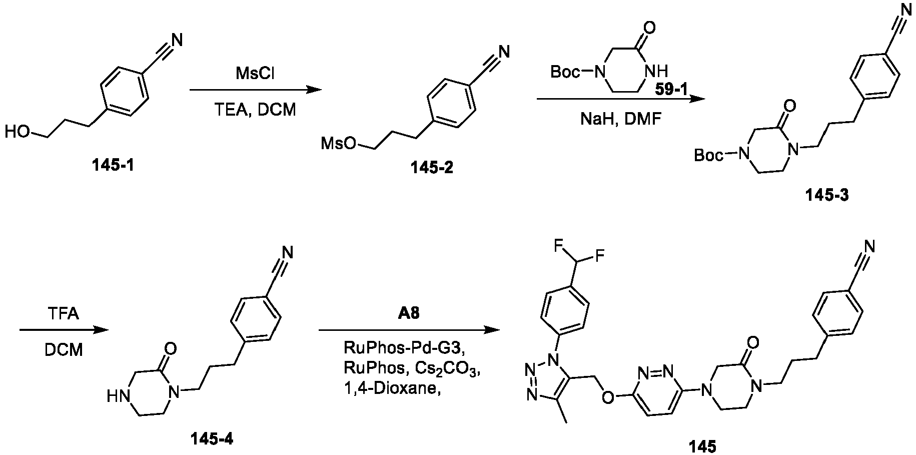

### Step 1: Synthesis of 3-(4-cyanophenyl)propyl methanesulfonate (145-2)

The experimental operation was as described in step 3 in Example **106,** using **145-1** and methanesulfonyl chloride as raw materials to obtain the title product.

MS (ESI) m/z [M+H]⁺ = 240.1.

### Step 2: Synthesis of tert-butyl 4-(3-(4-cyanophenyl)propyl)-3-oxopiperazine-1-carboxylate (145-3)

The experimental operation was as described in step 1 in Example **59,** using compound **145-2** and compound **59-1** as raw materials to obtain the title product.

MS (ESI) m/z [M+H]⁺ = 344.2.

### Step 3: Synthesis of 4-(3-(2-oxopiperazin-1-yl)propyl)benzonitrile (145-4)

The experimental operation was as described in step 2 in Example **27,** using compound **145-3** as the raw material to obtain the title product.

MS (ESI) m/z [M+H]⁺ = 244.1.

### Step 4: Synthesis of 4-(3-(4-(6-((1-(4-(difluoromethyl)phenyl)-4-methyl-1H-1,2,3-triazol-5-yl)methoxy)pyridazin-3-yl)-2-oxopiperazin-1-yl)propyl)benzonitrile (145)

The experimental operation was as described in Example **1,** using compound **A8** and compound **145-4** as raw materials to obtain the title product.

MS (ESI) m/z [M+H]⁺ = 559.2.

¹H NMR (400 MHz, DMSO-*d*₆) δ 7.78 (s, 4H), 7.73 (d, *J* = 8.2 Hz, 2H), 7.48 - 7.35 (m, 3H), 7.14 (t, *J* = 55.6 Hz, 1H), 7.05 (d, *J* = 9.6 Hz, 1H), 5.45 (s, 2H), 4.02 (s, 2H), 3.72 (m, 2H), 3.44 - 3.36 (m, 4H), 2.70 - 2.58 (m, 2H), 2.39 (s, 3H), 1.89 - 1.72 (m, 2H).

### Example 146

### 4-(3-(4-(6-((4-Chloro-1-(4-(difluoromethyl)phenyl)-1H-1,2,3-triazol-5-yl)methoxy)pyridazin-3-yl)-2-oxopiperazin-1-yl)propyl)benzoic acid (146)

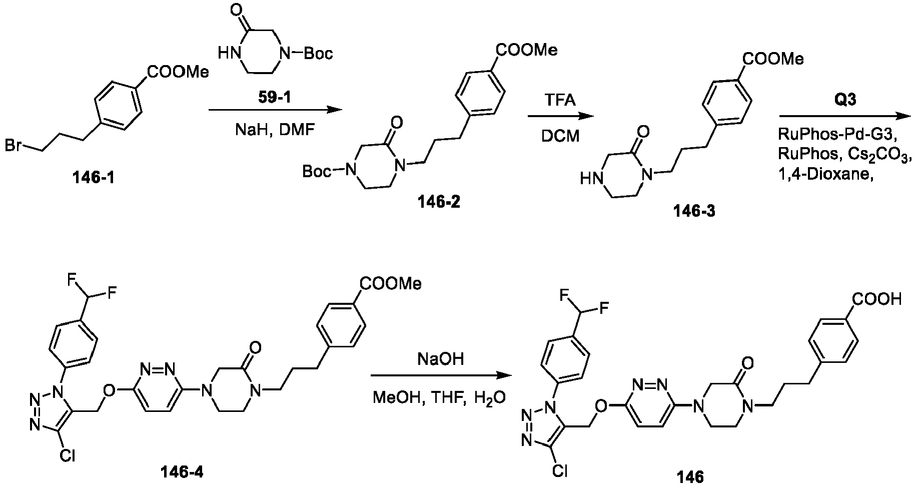

### Step 1: Synthesis of tert-butyl 4-(3-(4-(methoxycarbonyl)phenyl)propyl)-3-oxopiperazine-1-carboxylate (146-2)

The experimental operation was as described in step 1 in Example **59,** using compounds **146-1** and **59-1** as raw materials to obtain the title product.

MS (ESI) m/z [M+H]⁺ = 377.2.

### Step 2: Synthesis of methyl 4-(3-(2-oxopiperazin-1-yl)propyl)benzoate (146-3)

The experimental operation was as described in step 2 in Example **27,** using compound **146-2** as the raw material to obtain the title product.

MS (ESI) m/z [M+H]⁺ = 277.1.

### Step 3: Synthesis of methyl 4-(3-(4-(6-((4-chloro-1-(4-(difluoromethyl)phenyl)-1H 1,2,3-triazol-5-yl)methoxy)pyridazin-3-yl)-2-oxopiperazin-1-yl)propyl)benzoate (146-4)

The experimental operation was as described in Example **1,** using compounds **145-3** and **A8** as raw materials to obtain the title product.

MS (ESI) m/z [M+H]⁺ = 612.2.

### Step 4: Synthesis of 4-(3-(4-(6-((4-chloro-1-(4-(difluoromethyl)phenyl)-1H-1,2,3-triazol-5-yl)methoxy)pyridazin-3-yl)-2-oxopiperazin-1-yl)propyl)benzoic acid (146)

The experimental operation was as described in step 3 in Example **27,** using compound **145-4** as the raw material to obtain the title product.

MS (ESI) m/z [M+H]⁺ = 598.2.

¹H NMR (400 MHz, DMSO-*d*₆) δ 8.05 - 7.65 (m, 6H), 7.41 (d, *J* = 9.6 Hz, 1H), 7.17 (d, *J* = 7.4 Hz, 2H), 7.14 (t, *J* = 55.6 Hz, 1H), 7.03 (d, *J* = 9.6 Hz, 1H), 5.46 (s, 2H), 4.02 (s, 2H), 3.76 - 3.63 (m, 2H), 3.44 - 3.37 (m, 4H), 2.61 - 2.51 (m, 2H), 1.90 - 1.69 (m, 2H).

### Example 147

### (S)-8-(6-((1-(4-(Difluoromethyl)phenyl)-4-methyl-1H-1,2,3-triazol-5-yl)methoxy)pyridazin-3-yl)-2-(oxetan-3-yl)hexahydro-2H-pyrazino[1,2-a]pyrazin-1(6H)-one (147)

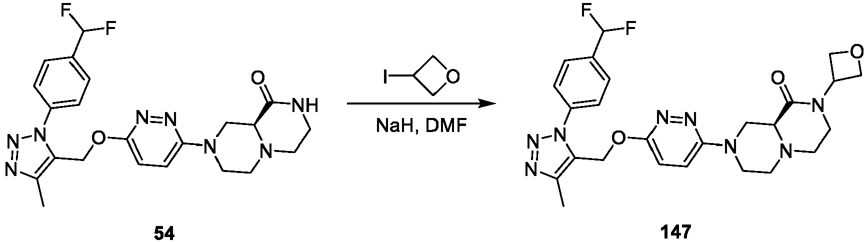

The experimental operation was as described in Example **26,** using compound **54** and 3-iodooxetane as raw materials to obtain the title product.

MS (ESI) m/z [M+H]⁺ = 527.2.

¹H NMR (400 MHz, CD₃OD) δ 7.77 (s, 4H), 7.37 (d, *J* = 9.6 Hz, 1H), 6.98 (d, *J* = 9.6 Hz, 1H), 6.89 (t, *J* = 56.0 Hz, 1H), 5.50 (s, 2H), 5.34 - 5.21 (m, 1H), 4.84 - 4.75 (m, 5H), 4.51-4.45 (m, 1H), 4.15 - 4.04 (m, 1H), 3.76 - 3.61 (m, 1H), 3.54-3.50 (m, 1H), 3.17-3.12 (m, 1H), 3.09 - 2.97 (m, 2H), 2.92 - 2.75 (m, 2H), 2.73 - 2.61 (m, 1H), 2.43 (s, 3H).

### Example 148

### (S)-2-Cyclopropyl-8-(6-((1-(4-(difluoromethyl)phenyl)-4-methyl-1H-1,2,3-triazo1-5-yl)methoxy)pyridazin-3-yl)hexahydro-2H-pyrazino[1,2-a]pyrazin-1(6H)-one (148)

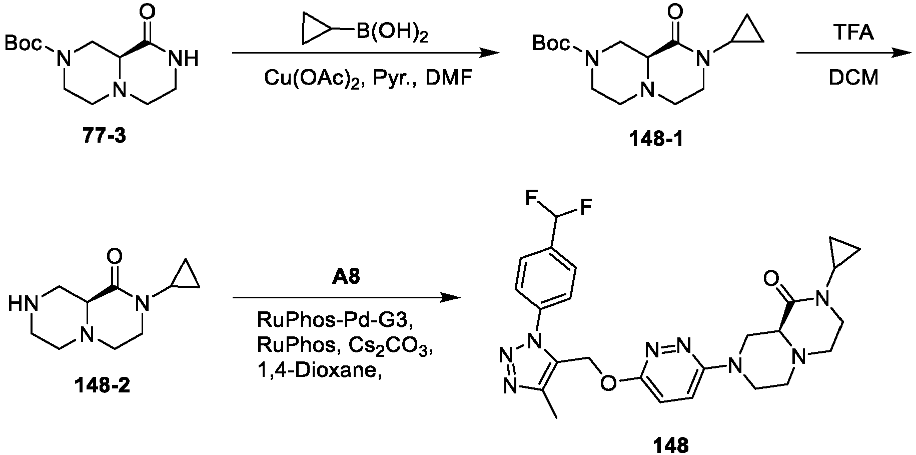

### Step 1: Synthesis of tert-butyl (S)-8-cyclopropyl-9-oxooctahydro-2H-pyrazino[1,2-a]pyrazine-2-carboxylate (148-1)

The experimental operation was as described in step 1 in Example **108,** using compound **77-3** and cyclopropylboronic acid as raw materials to obtain the title product.

MS (ESI) m/z [M+H]⁺ = 295.2.

### Step 2: Synthesis of (S)-2-cyclopropylhexahydro-2H-pyrazino[1,2-a]pyrazin-1(6H)-one (148-2)

The experimental operation was as described in step 2 in Example **27,** using compound 148-1 as the raw material to obtain the title product.

MS (ESI) m/z [M+H]⁺ = 195.1.

### Step 3: Synthesis of (S)-2-cyclopropyl-8-(6-((1-(4-(difluoromethyl)phenyl)-4-methyl-1H-1,2,3-triazol-5-yl)methoxy)pyridazin-3-yl)hexahydro-2H-pyrazino[1,2-a]pyrazin-1(6H)-one (148)

The experimental operation was as described in Example **1,** using compounds **148-2** and **A8** as raw materials to obtain the title product.

MS (ESI) m/z [M+H]⁺ = 511.2.

¹H NMR (400 MHz, DMSO-*d*₆) δ 7.78 (s, 4H), 7.38 (d, *J* = 9.8 Hz, 1H), 7.13 (t, *J* = 56.0 Hz, 1H), 6.99 (d, *J* = 9.8 Hz, 1H), 5.45 (s, 2H), 4.49 (t, *J* = 9.4 Hz, 1H), 4.04 - 3.96 (m, 1H), 3.37 - 3.34 (m, 1H), 3.13 - 3.06 (m, 1H), 2.95 - 2.81 (m, 3H), 2.73 - 2.71 (m, 1H), 2.64 - 2.57 (m, 2H), 2.46 - 2.36 (m, 4H), 2.26 - 2.15 (m, 1H), 0.74 - 0.50 (m, 4H).

### Example 149

### 1-(6-((1-(4-(Difluoromethyl)phenyl)-4-methyl-1H-1,2,3-triazol-5-yl)methoxy)pyridazin-3-yl)-N-(3-methoxypyridin-4-yl)azetidine-3-carboxamide (149)

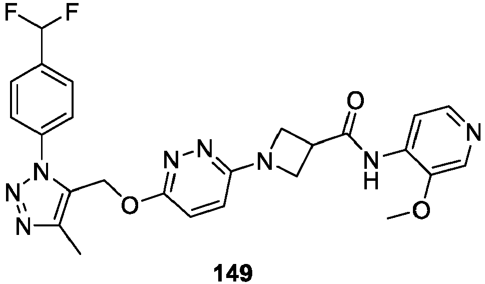

The experimental operation was as described in Example **38,** using compound **37** and 4-amino-3-methoxypyridine as raw materials to obtain the title product.

MS (ESI) m/z [M+H]⁺ = 523.2.

¹H NMR (400 MHz, DMSO-*d*₆) δ 9.71 (s, 1H), 8.31 (s, 1H), 8.14 (d, *J* = 5.2 Hz, 1H), 8.11 (d, *J* = 5.4 Hz, 1H), 7.78 (s, 4H), 7.14 (t, *J* = 53.2 Hz, 1H), 6.99 (d, *J* = 4.7 Hz, 1H), 6.94 (d, *J* = 9.5 Hz, 1H), 5.43 (s, 2H), 4.19 - 4.09 (m, 2H), 4.08 - 4.00 (m, 2H), 4.00 - 3.95 (m, 1H), 3.93 (s, 3H), 2.39 (s, 3H).

### Example 150

### 1-(6-((1-(4-(Difluoromethyl)phenyl)-4-methyl-1H-1,2,3-triazol-5-yl)methoxy)pyridazin-3-yl)-N-(1-isopropyl-1H-pyrazol-4-yl)azetidine-3-carboxamide (150)

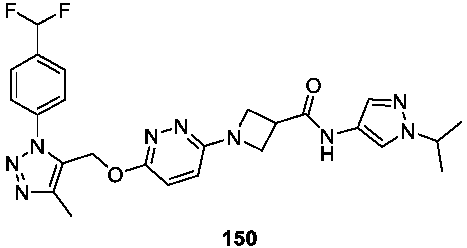

The experimental operation was as described in Example **38,** using compound **37** and 1-isopropyl-4-aminopyrazole as raw materials to obtain the title product.

MS (ESI) m/z [M+H]⁺ = 524.2.

¹H NMR (400 MHz, DMSO-*d*₆) δ 10.08 (s, 1H), 7.89 (s, 1H), 7.78 (s, 4H), 7.40 (s, 1H), 7.14 (t, *J* = 54.0 Hz, 1H), 6.99 (d, *J* = 6.4 Hz, 1H), 6.92 (d, *J* = 9.4 Hz, 1H), 5.43 (s, 2H), 4.54 - 4.33 (m, 1H), 4.21 - 4.07 (m, 2H), 4.08 - 3.93 (m, 2H), 3.72 - 3.49 (m, 1H), 2.39 (s, 3H), 1.35 (d, *J* = 6.6 Hz, 6H).

### Example 151

### 1-(6-((1-(4-(Difluoromethyl)phenyl)-4-methyl-1H-1,2,3-triazol-5-yl)methoxy)pyridazin-3-yl)-N (2-fluoropyridin-3-yl)azetidine-3-carboxamide (151)

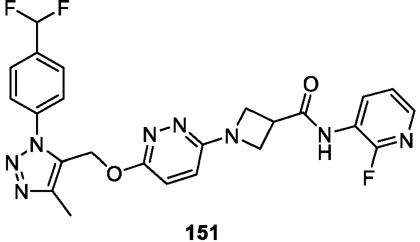

The experimental operation was as described in Example **38,** using compound **37** and 3-amino-2-fluoropyridine as raw materials to obtain the title product.

MS (ESI) m/z [M+H]⁺ = 511.2.

¹H NMR (400 MHz, DMSO-*d*₆) δ 10.10 (s, 1H), 8.45 (t, *J* = 9.0 Hz, 1H), 7.93 (d, *J* = 4.6 Hz, 1H), 7.78 (s, 4H), 7.42 - 7.29 (m, 1H), 7.14 (t, *J* = 55.6 Hz, 1H), 6.99 (d, *J* = 9.6 Hz, 1H), 6.94 (d, *J* = 9.6 Hz, 1H), 5.43 (s, 2H), 4.21 - 4.11 (m, 2H), 4.11 - 4.01 (m, 2H), 3.91 - 3.76 (m, 1H), 2.39 (s, 3H).

### Example 152

### 1-(6-((1-(4-(Difluoromethyl)phenyl)-4-methyl-1H-1,2,3-triazol-5-yl)methoxy)pyridazin-3-yl)-N-(3-methoxypyridin-2-yl)azetidine-3-carboxamide (152)

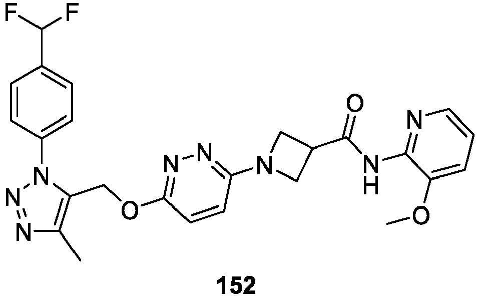

The experimental operation was as described in Example **38,** using compound **37** and 2-amino-3-methoxypyridine as raw materials to obtain the title product.

MS (ESI) m/z [M+H]⁺ = 523.2.

¹H NMR (400 MHz, DMSO-*d*₆) δ 9.76 (s, 1H), 7.94 (d, *J* = 4.8 Hz, 1H), 7.78 (s, 4H), 7.46 (d, *J* = 8.2 Hz, 1H), 7.22 (dd, *J* = 8.0, 4.6 Hz, 1H), 7.13 (t, *J* = 55.6 Hz, 1H), 6.98 (d, *J* = 9.6 Hz, 1H), 6.93 (d, *J* = 9.6 Hz, 1H), 5.43 (s, 2H), 4.18 - 4.08 (m, 2H), 4.08 - 3.98 (m, 2H), 3.83 - 3.75 (m, 4H), 2.38 (s, 3H).

### Example 153

### N-(1-(Difluoromethyl)-1H-pyrazol-4-yl)-1-(6-((1-(4-(difluoromethyl)phenyl)-4-methyl-1H-1,2,3-triazol-5-yl)methoxy)pyridazin-3-yl)azetidine-3-carboxamide (153)

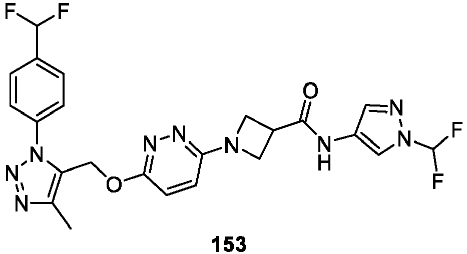

The experimental operation was as described in Example **38,** using compound **37** and 1-difluoromethyl-4-aminopyrazole as raw materials to obtain the title product.

MS (ESI) m/z [M+H]⁺ = 532.2.

¹H NMR (400 MHz, DMSO-*d*₆) δ 10.40 (s, 1H), 8.33 (s, 1H), 7.90 - 7.58 (m, 6H), 7.13 (t, *J* = 55.6 Hz, 1H), 6.99 (d, *J* = 9.4 Hz, 1H), 6.93 (d, *J* = 9.4 Hz, 1H), 5.43 (s, 2H), 4.21 - 4.10 (m, 2H), 4.09 - 4.00 (m, 2H), 3.74 - 3.55 (m, 1H), 2.39 (s, 3H).

### Example 154

### (1-(6-((1-(4-(Difluoromethyl)phenyl)-4-methyl-1H-1,2,3-triazol-5-yl)methoxy)pyridazin-3-yl)azetidin-3-yl)(3-hydroxypiperidin-1-yl)methanone (154)

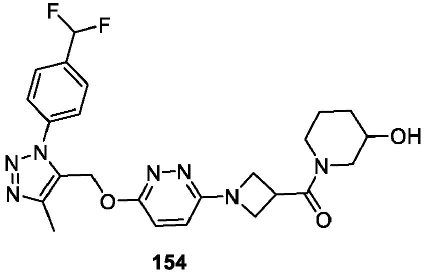

The experimental operation was as described in Example **38,** using compound **37** and 3-hydroxypiperidine as raw materials to obtain the title product.

MS (ESI) m/z [M+H]⁺ = 500.2.

¹H NMR (400 MHz, DMSO-*d*₆) δ 7.78 (s, 4H), 7.13 (t, *J* = 56.0 Hz, 1H), 6.98 (d, *J* = 9.6 Hz, 1H), 6.92 (d, *J* = 9.6 Hz, 1H), 5.42 (s, 2H), 4.87 (s, 1H), 4.15 - 4.06 (m, 5H), 3.81 - 3.79 (m, 1H), 3.53 - 3.46 (m, 2H), 2.98 - 2.94 (m, 1H), 2.68 - 2.62 (m, 1H), 2.38 (s, 3H), 1.83 - 1.65 (m, 2H), 1.43 - 1.30 (m, 2H).

### Example 155

### 1-(6-((1-(4-(Difluoromethyl)phenyl)-4-methyl-1H-1,2,3-triazol-5-yl)methoxy)pyridazin-3-yl)-N-isopropylazetidine-3-carboxamide (155)

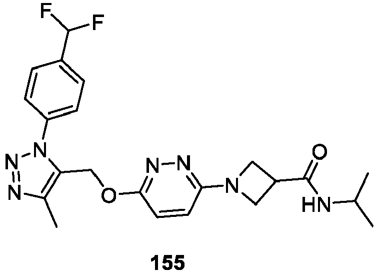

The experimental operation was as described in Example **38,** using compound **37** and isopropylamine as raw materials to obtain the title product.

MS (ESI) m/z [M+H]⁺ = 458.2.

¹H NMR (400 MHz, DMSO-*d*₆) δ 7.86 (d, *J* = 8.0 Hz, 1H), 7.78 (s, 4H), 7.13 (t, *J* = 56.0 Hz, 1H), 6.97 (d, *J* = 9.6 Hz, 1H), 6.90 (d, *J* = 9.6 Hz, 1H), 5.42 (s, 2H), 4.06 - 4.02 (m, 2H), 3.95 - 3.91 (m, 2H), 3.86 - 3.80 (m, 1H), 3.43 - 3.41 (m, 1H), 2.38 (s, 3H), 1.04 (d, *J* = 4.0 Hz, 6H).

### Example 156

### (1-(6-((1-(4-(Difluoromethyl)phenyl)-4-methyl-1H-1,2,3-triazol-5-yl)methoxy)pyridazin-3-yl)azetidin-3-yl)(2,6-dimethylmorpholino)methanone (156)

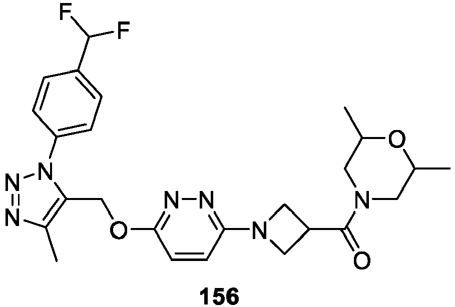

The experimental operation was as described in Example **38,** using compound **37** and 2,6-dimethylmorpholine as raw materials to obtain the title product.

MS (ESI) m/z [M+H]⁺ = 514.2.

¹H NMR (400 MHz, DMSO-*d*₆) δ 7.78 (s, 4H), 7.13 (t, *J* = 56.0 Hz, 1H), 6.98 (d, *J* = 9.6 Hz, 1H), 6.92 (d, *J* = 9.6 Hz, 1H), 5.42 (s, 2H), 4.23 - 4.20 (m, 1H), 4.14 - 4.10 (m, 2H), 4.06 - 3.99 (m, 2H), 3.86 - 3.84 (m, 1H), 3.49 - 3.45 (m, 3H), 2.70 - 2.64 (m, 1H), 2.38 (s, 3H), 2.30 - 2.24 (m, 1H), 1.09 - 1.06 (m, 6H).

### Example 157

### (2-Oxa-5-azabicyclo[2.2.1]heptan-5-yl)(1-(6-((1-(4-(difluoromethyl)phenyl)-4-methyl-1H-1,2,3-triazol-5-yl)methoxy)pyridazin-3-yl)azetidin-3-yl)methanone (157)

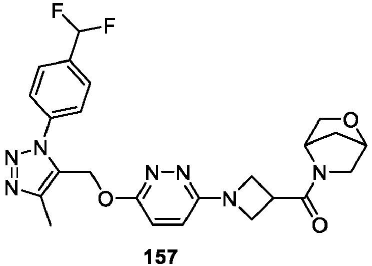

The experimental operation was as described in Example **38,** using compound **37** and 2-oxa-5-azabicyclo[2.2.1]heptane as raw materials to obtain the title product.

MS (ESI) m/z [M+H]⁺ = 498.2.

¹H NMR (400 MHz, DMSO-*d*₆) δ 7.77 (s, 4H), 7.12 (t, *J* = 56.0 Hz, 1H), 6.98 (d, *J* = 9.6 Hz, 1H), 6.90 (d, *J* = 9.6 Hz, 1H), 5.42 (s, 2H), 4.71 - 4.50 (m, 2H), 4.12 - 4.01 (m, 4H), 3.73 - 3.71 (m, 1H), 3.68 - 3.66 (m, 2H), 3.28 - 3.26 (m, 1H), 3.17 - 3.15 (m, 1H), 2.38 (s, 3H), 1.80 - 1.75 (m, 2H).

### Example 158

### (1-(6-((1-(4-(Difluoromethyl)phenyl)-4-methyl-1H-1,2,3-triazol-5-yl)methoxy)pyridazin-3-yl)azetidin-3-yl)(2,2-dimethylmorpholino)methanone (158)

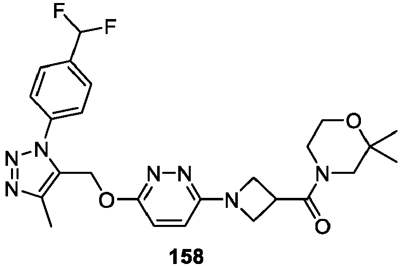

The experimental operation was as described in Example **38,** using compound **37** and 2,2-dimethylmorpholine as raw materials to obtain the title product.

MS (ESI) m/z [M+H]⁺ = 514.2.

¹H NMR (400 MHz, DMSO-*d*₆) δ 7.77 (s, 4H), 7.12 (t, *J* = 56.0 Hz, 1H), 6.98 (d, *J* = 9.6 Hz, 1H), 6.92 (d, *J* = 9.6 Hz, 1H), 5.42 (s, 2H), 4.17 - 4.11 (m, 2H), 4.05 - 4.00 (m, 2H), 3.92 - 3.86 (m, 1H), 3.81 - 3.77 (m, 1H), 3.58 - 3.55 (m, 2H), 3.39 - 3.37 (m, 2H), 3.29 - 3.26 (m, 1H), 2.38 (s, 3H), 1.12 - 1.08 (m, 6H).

### Example 159

### 1-(6-((1-(4-(Difluoromethyl)phenyl)-4-methyl-1H-1,2,3-triazol-5-yl)methoxy)pyridin-3-yl)-N-ethylazetidine-3-carboxamide (159)

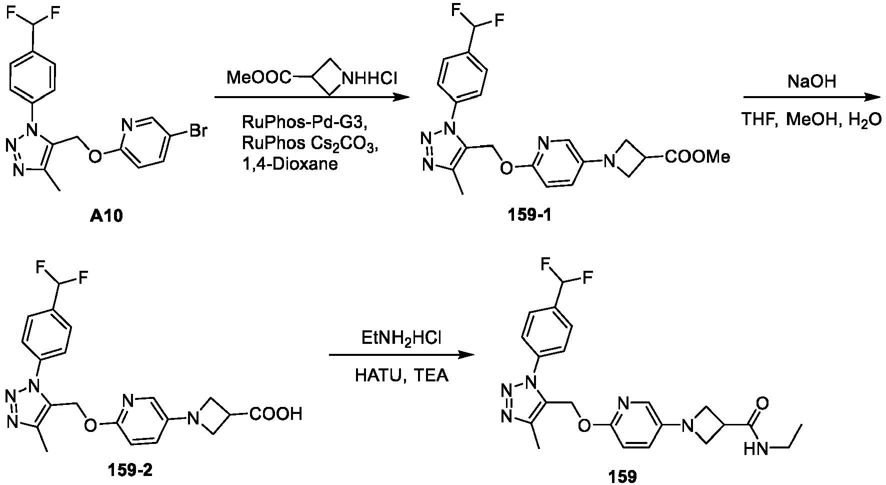

The experimental operation was as described in steps 1 to 2 in Example **35** and Example **38,** using compound **A10** and methyl azetidine-3-carboxylate hydrochloride as raw materials to obtain the title product.

MS (ESI) m/z [M+H]⁺ = 443.2.

¹H NMR (400 MHz, DMSO-*d*₆) δ 7.96 (t, *J* = 5.4 Hz, 1H), 7.85 - 7.70 (m, 4H), 7.27 (s, 1H), 7.13 (t, *J* = 55.6 Hz, 1H), 6.91 (dd, *J* = 8.8, 2.1 Hz, 1H), 6.62 (d, *J* = 8.8 Hz, 1H), 5.28 (s, 2H), 3.95 - 3.84 (m, 2H), 3.79 - 3.74 (m, 1H), 3.74 - 3.67 (m, 2H), 3.10-3.03 (m, 2H), 2.36 (s, 3H), 0.99 (t, *J* = 7.2 Hz, 3H).

### Example 160

### (1-(6-((1-(4-(Difluoromethyl)phenyl)-4-methyl-1H-1,2,3-triazol-5-yl)methoxy)pyridin-3-yl)azetidin-3-yl)(morpholino)methanone (160)

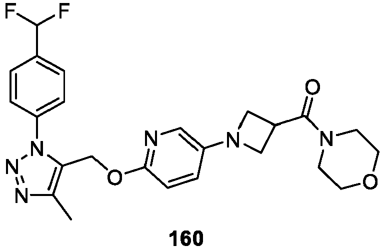

The experimental operation was as described in steps 1 to 2 in Example **35** and Example **38,** using compound **A10** and morpholine as raw materials to obtain the title product.

MS (ESI) m/z [M+H]⁺ = 485.2.

¹H NMR (400 MHz, DMSO-*d*₆) δ 7.77 (s, 4H), 7.29 (d, *J* = 2.4 Hz, 1H), 7.13 (t, *J* = 55.6 Hz, 1H), 6.93 (d, *J* = 8.8 Hz, 1H), 6.62 (d, *J* = 8.8 Hz, 1H), 5.28 (s, 2H), 4.01 - 3.90 (m, 2H), 3.86 - 3.74 (m, 3H), 3.59 - 3.50 (m, 4H), 3.48 - 3.45 (m, 2H), 3.31 - 3.25 (m, 2H), 2.36 (s, 3H).

### Example 161

### (S)-8-(6-((1-(4-(Methoxymethyl)phenyl)-4-methyl-1H-1,2,3-triazol-5-yl)methoxy)pyridazin-3-yl)hexahydro-2H-pyrazino[1,2-a]pyrazin-1(6H)-one (161)

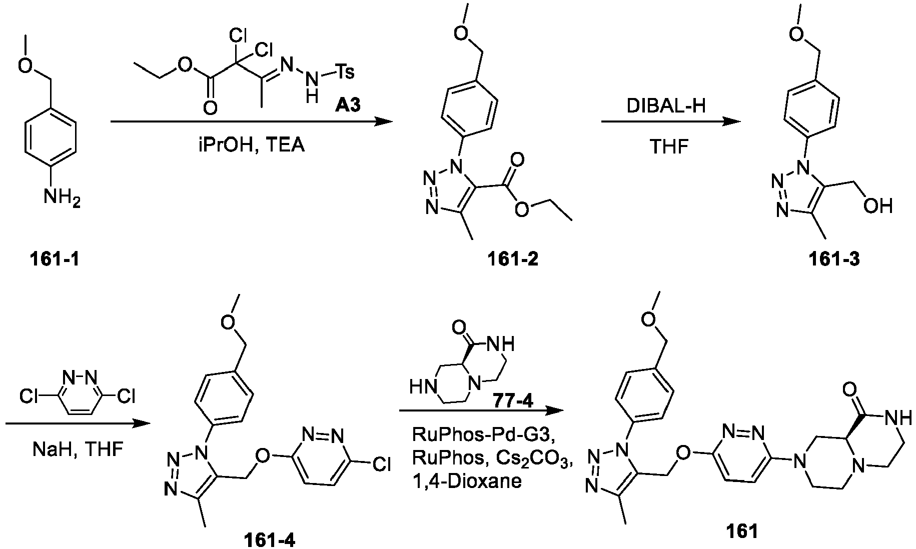

The experimental operation was as described in steps 4 to 6 in the synthesis of intermediate **A8** and Example **1,** using compounds **161-1** and **A3** as raw materials to obtain the title product.

MS (ESI) m/z [M+H]⁺ = 465.2.

¹H NMR (400 MHz, DMSO-*d*₆) δ 7.78 (s, 1H), 7.57 (d, *J* = 8.0 Hz, 2H), 7.49 (d, *J* = 8.0 Hz, 2H), 7.39 (d, *J* = 9.6 Hz, 1H), 7.00 (d, *J* = 9.6 Hz, 1H), 5.40 (s, 2H), 4.48 (s, 2H), 4.44 - 4.42 (m, 1H), 4.01 - 3.99 (m, 1H), 3.33 (s, 3H), 3.32 - 3.31 (m, 1H), 3.08 - 3.06 (m, 1H), 2.94 - 2.87 (m, 3H), 2.70 - 2.58 (m, 2H), 2.45 - 2.36 (m, 4H), 2.27 - 2.24 (m, 1H).

### Example 162

### (S)-8-(6-((1-(4-(Fluoromethyl)phenyl)-4-methyl-1H-1,2,3-triazol-5-yl)methoxy)pyridazin-3-yl)hexahydro-2H-pyrazino[1,2-a]pyrazin-1(6H)-one (162)

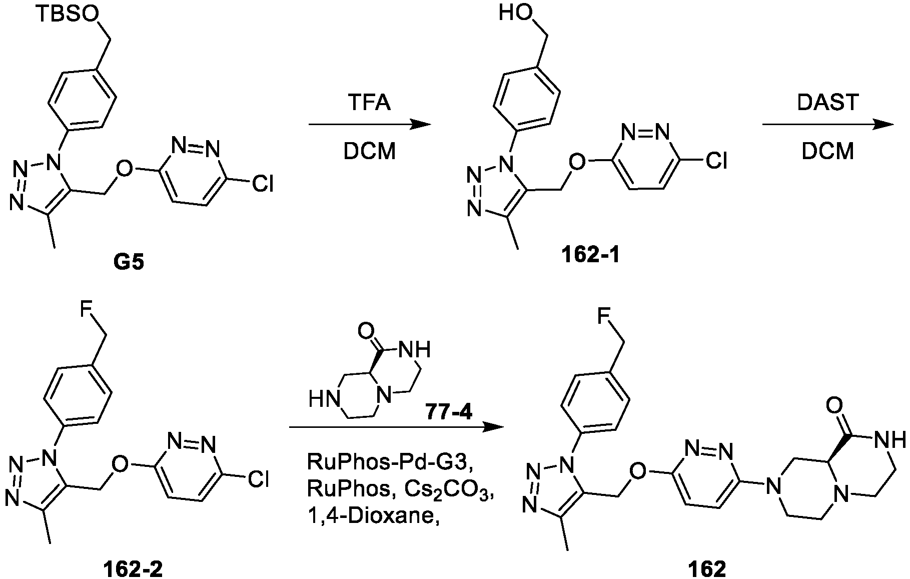

### Step 1: Synthesis of (4-(5-(((6-chloropyridazin-3-yl)oxy)methyl)-4-methyl-1H-1,2,3-triazol-1-yl)phenyl)methanol (162-1)

The experimental operation was as described in step 2 in Example **27,** using **G5** as the raw material to obtain the title product.

MS (ESI) m/z [M+H]⁺ = 332.1.

### Step 2: Synthesis of 3-chloro-6-((1-(4-(fluoromethyl)phenyl)-4-methyl-1H-1,2,3-triazol-5-yl)methoxy)pyridazine (162-2)

**162-1** (1 g, 3.01 mmol) was added to dichloromethane (20 mL), cooled to 0°C, then diethylaminosulfur trifluoride (799 mg, 3.61 mmol) was added thereto, and the reaction mixture was stirred at 0°C for 2 hours. The reaction mixture was added with sodium bicarbonate solution to adjust the pH to 8, and extracted with dichloromethane (20 mL × 3). The organic phase was dried over anhydrous sodium sulfate and filtered, and the filtrate was concentrated under reduced pressure. The residue was purified by flash silica gel column chromatography (petroleum ether/ethyl acetate = 3:1) to obtain the title product (200 mg, yellow solid).

MS (ESI) m/z [M+H]⁺ = 334.1.

### Step 3: Synthesis of (S)-8-(6-((1-(4-(fluoromethyl)phenyl)-4-methyl-1H-1,2,3-triazol-5-yl)methoxy)pyridazin-3-yl)hexahydro-2H-pyrazino[1,2-a]pyrazin-1(6H)-one (162)

The experimental operation was as described in Example **1,** using compounds **162-2** and **77-4** as raw materials to obtain the title product.

MS (ESI) m/z [M+H]⁺ = 453.2.

¹H NMR (400 MHz, DMSO-*d*₆) δ 7.78 (s, 1H), 7.63 (m, 4H), 7.39 (d, *J* = 9.6 Hz, 1H), 6.99 (d, *J* = 9.6 Hz, 1H), 5.50 (d, *J* = 47.4 Hz, 2H), 5.42 (s, 2H), 4.50 - 4.36 (m, 1H), 4.08 - 3.88 (m, 1H), 3.27 - 3.18 (m, 1H), 3.14 - 2.99 (m, 1H), 2.98 - 2.77 (m, 3H), 2.69 - 2.55 (m, 2H), 2.45 - 2.32 (m, 4H), 2.31 - 2.16 (m, 1H).

### Example 163

### (S)-8-(6-((1-(4-Chlorophenyl)-4-methyl-1H-1,2,3-triazol-5-yl)methoxy)pyridazin-3-yl)hexahydro-2H-pyrazino[1,2-a]pyrazin-1(6H)-one (163)

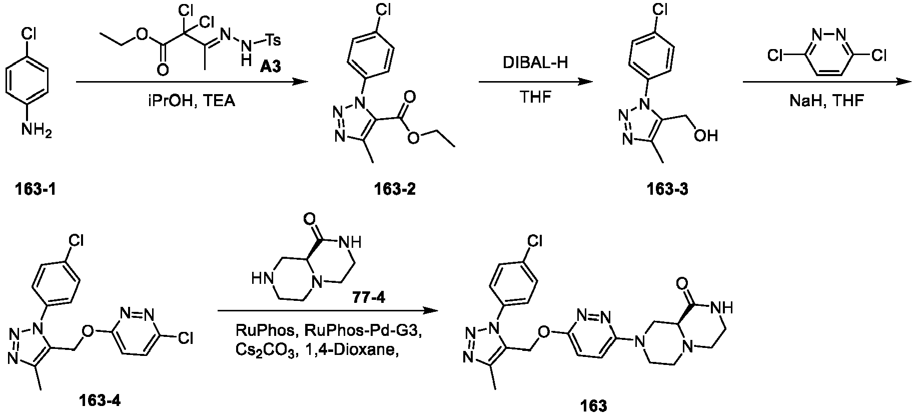

The experimental operation was as described in steps 4 to 6 in the synthesis of intermediate **A8** and Example **1,** using compounds **163-1** and **A3** as raw materials to obtain the title product.

MS (ESI) m/z [M+H]⁺ = 455.2.

¹H NMR (400 MHz, CDsOD) δ 7.64-7.58 (m, 4H), 7.38 (d, *J* = 9.8 Hz, 1H), 7.69 (d, *J* = 9.8 Hz, 1H), 5.50 (s, 2H), 4.47 (d, *J* = 5.6 Hz, 1H), 4.12 - 4.09 (m, 1H), 3.50 - 3.46 (m, 1H), 3.24 - 3.20 (m, 1H), 3.10 - 2.94 (m, 3H), 2.90 - 2.80 (m, 2H), 2.65 - 2.55 (m, 1H), 2.50 - 2.40 (m, 4H).

### Example 164

### 4-(6-((1-(4-Chlorophenyl)-4-methyl-1H-1,2,3-triazol-5-yl)methoxy)pyridazin-3-yl)piperazin-2-one (164)

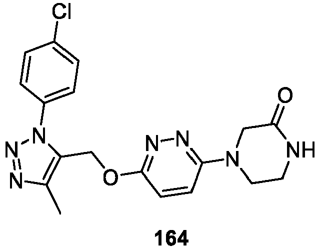

The experimental operation was as described in Example **1,** using compound **163-4** and 2-piperazinone as raw materials to obtain the title product.

MS (ESI) m/z [M+H]⁺ = 400.1.

¹H NMR (400 MHz, CDsOD) δ 7.63-7.57 (m, 4H), 7.34 (d, *J* = 9.8 Hz, 1H), 6.99 (d, *J* = 9.8 Hz, 1H), 5.47 (s, 2H), 4.12 (s, 2H), 3.76 - 3.73 (m, 2H), 3.66 - 3.44 (m, 2H), 2.46 (s, 3H).

### Example 165

### (S)-8-(6-((4-Methyl-1-(4-(trifluoromethyl)phenyl)-1H-1,2,3-triazol-5-yl)methoxy)pyridazin-3-yl)hexahydro-2H-pyrazino[1,2-a]pyrazin-1(6H)-one (165)

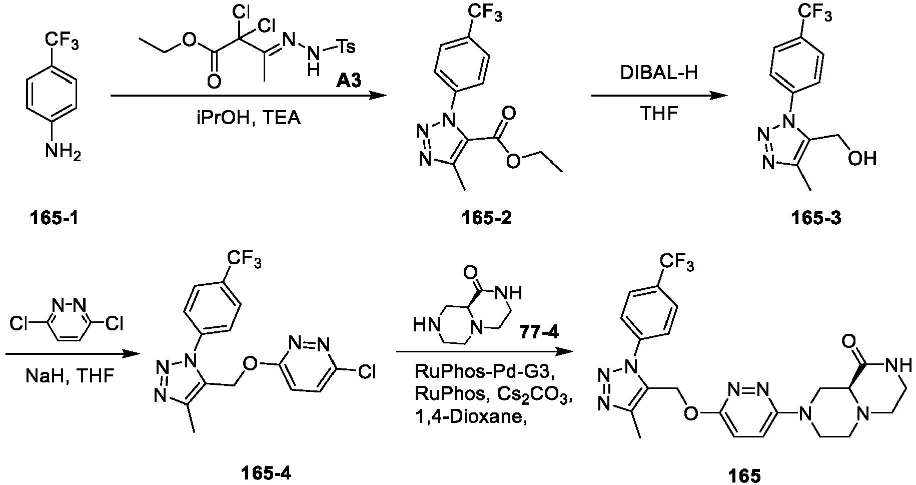

The experimental operation was as described in steps 4 to 6 in the synthesis of intermediate **A8** and Example **1,** using compounds **165-1** and **A3** as raw materials to obtain the title product.

MS (ESI) m/z [M+H]⁺ = 489.1.

¹H NMR: (400 MHz, CD₃OD) *δ* 7.98 - 7.84 (m, 4H), 7.38 (d, *J* = 9.6 Hz, 1H), 6.99 (d, *J* = 9.6 Hz, 1H), 5.53 (s, 2H), 4.52 - 4.40 (m, 1H), 4.16 - 4.02 (m, 1H), 3.58 - 3.43 (m, 1H), 3.26 - 3.20 (m, 1H), 3.09 - 2.99 (m, 3H), 2.90 - 2.79 (m, 2H), 2.64 - 2.56 (m, 1H), 2.48 (s, 3H), 2.46 - 2.39 (m, 1H).

### Example 166

### 2-(4-(4-Methyl-5-(((6-(3-oxopiperazin-1-yl)pyridazin-3-yl)oxy)methyl)-1H-1,2,3-triazol-1-yl)phenyl)malononitrile (166)

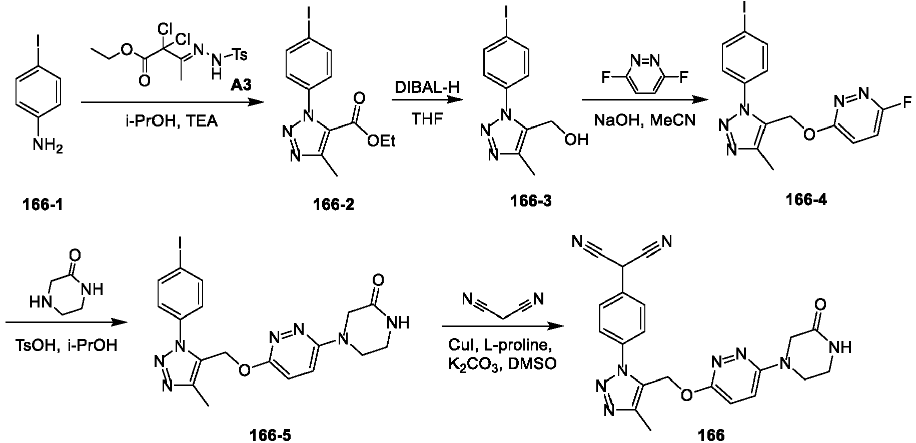

### Step 1: Synthesis of ethyl 1-(4-iodophenyl)-4-methyl-1H-1,2,3-triazole-5-carboxylate (166-2)

The experimental operation was as described in step 4 in the synthesis of intermediate **A8,** using compounds **166-1** and **A3** as raw materials to obtain the title product.

MS (ESI) m/z [M+H]⁺ = 357.9.

### Step 2: Synthesis of (1-(4-iodophenyl)-4-methyl-1H-1,2,3-triazol-5-yl)methanol (166-3)

The experimental operation was as described in step 5 in the synthesis of intermediate **A8,** using compound **166-2** as the raw material to obtain the title product.

¹H NMR: (400 MHz, CDCl₃) *δ* 7.91 - 7.84 (m, 2H), 7.50 - 7.42 (m, 2H), 4.67 (s, 2H), 2.46 (s, 3H).

### Step 3: Synthesis of 3-fluoro-6-((1-(4-iodophenyl)-4-methyl-1H-1,2,3-triazol-5-yl)methoxy)pyridazine (166-4)

Compound **166-3** (2.17 g, 6.89 mmol), 2,6-difluoropyridazine (800 mg, 6.89 mmol), and sodium hydroxide (551 mg, 13.8 mmol) were sequentially added to acetonitrile (20 mL) at room temperature, and the reaction was carried out at 80°C for 15 hours. TLC showed that the reaction was completed. The reaction mixture was concentrated under reduced pressure, and the residue was diluted with water (100 mL) and extracted with dichloromethane (50 mL × 3). The organic phase was dried over anhydrous sodium sulfate and filtered, and the filtrate was concentrated under reduced pressure. The residue was purified by flash silica gel column chromatography (ethyl acetate: petroleum ether = 1:5 to 1:1) to obtain the title product (1.1 g, yellow solid).

MS (ESI) m/z [M+H]⁺ = 411.9.

¹H NMR: (400 MHz, CDCl₃) *δ* 7.92 - 7.80 (m, 2H), 7.36 - 7.30 (m, 2H), 7.23 -7.08 (m, 2H), 5.52 (s, 2H), 2.50 (s, 3H).

### Step 4: Synthesis of 4-(6-((1-(4-iodophenyl)-4-methyl-1H-1,2,3-triazol-5-yl)methoxy)pyridazin-3-yl)piperazin-2-one (166-5)

Compound **166-4** (500 mg, 1.22 mmol), *p*-toluenesulfonic acid (229 mg, 1.22 mmol), and 2-piperazinone (609 mg, 6.08 mmol) were sequentially added to isopropanol (5 mL) at room temperature, and the reaction was carried out at 80°C for 15 hours. TLC showed that new points were generated. The reaction mixture was concentrated under reduced pressure, and the residue was diluted with water (50 mL) and extracted with dichloromethane (50 mL × 3). The organic phase was dried over anhydrous sodium sulfate and filtered, and the filtrate was concentrated under reduced pressure. The residue was purified by flash silica gel column chromatography (dichloromethane: methanol = 50:1 to 10:1) to obtain the title product (260 mg, yellow solid).

MS (ESI) m/z [M+H]⁺ = 492.0.

¹H NMR: (400 MHz, CDCl₃) *δ* 7.89 - 7.80 (m, 2H), 7.40 - 7.30 (m, 2H), 7.08 - 6.87 (m, 2H), 6.32 (br. s, 1H), 5.45 (s, 2H), 4.16 (s, 2H), 3.98-3.89 (m, 2H), 3.62-3.54 (m, 2H), 2.50 (s, 3H)

### Step 5: Synthesis of 2-(4-(4-methyl-5-(((6-(3-oxopiperazin-1-yl)pyridazin-3-yl)oxy)methyl)-1H-1,2,3-triazol-1-yl)phenyl)malononitrile (166)

Compound **166-5** (60 mg, 0.122 mmol), malononitrile (32.3 mg, 0.489 mmol), potassium carbonate (67.5 mg, 0.489 mmol), CuI (4.65 mg, 24.4 µmol), and *L*-proline (2.79 mg, 24.4 µmol) were sequentially added to dimethyl sulfoxide (2 mL) at room temperature. The system was replaced with nitrogen three times, and the reaction was carried out at 80°C for 15 hours. TLC showed that new points were generated. The reaction mixture was cooled to room temperature, filtered to remove insoluble matter, and the filtrate was purified by high performance liquid chromatography (chromatographic column: Ultimate C18 3 * 50 mm 3 µm; mobile phase: 1.5 mL trifluoroacetic acid/4 L water, acetonitrile; gradient: 10% to 80%/6 min; flow rate: 1.2 mL/min; column temperature: 50°C) to obtain the title product (16 mg, yellow solid).

MS (ESI) m/z [M+H]⁺ 430.0.

¹H NMR: (400 MHz, CDsOD) *δ* 8.32 (s, 1H), 7.81 (d, *J* = 9.6 Hz, 1H), 7.49 (d, *J* = 9.6 Hz, 1H), 7.40 - 7.14 (m, 2H), 7.09 - 6.61 (m, 2H), 5.34 (s, 2H), 4.10 (s, 2H), 3.82 - 3.71 (m, 2H), 3.37 - 3.30 (m, 2H), 2.38 (s, 3H).

### Example 167

### 4-(6-((1-(4-(Difluoromethyl)-2-fluorophenyl)-4-methyl-1H-1,2,3-triazol-5-yl)methoxy)pyridazin-3-yl)piperazin-2-one (167)

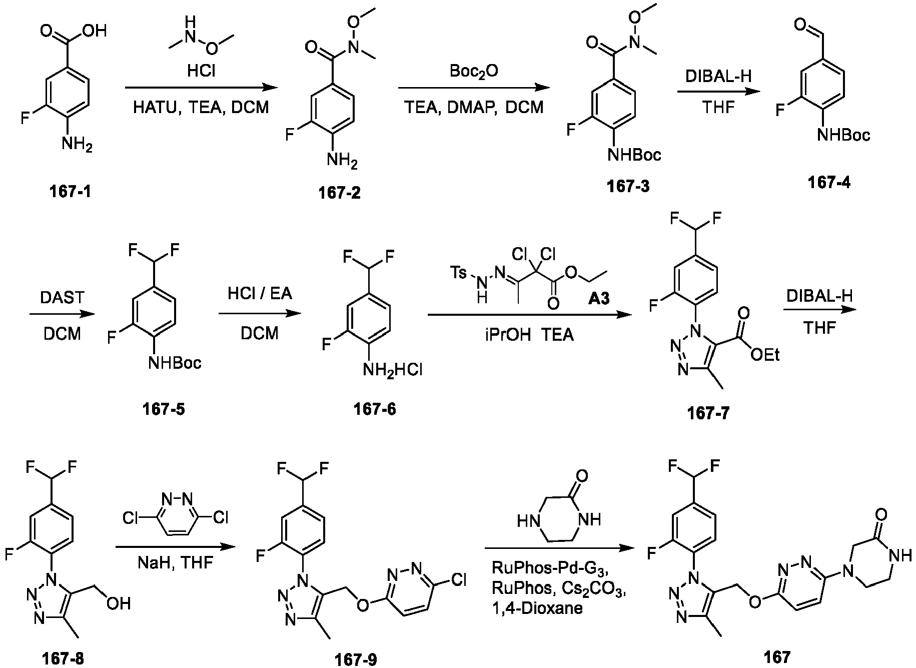

### Step 1: Synthesis of 4-amino-3-fluoro-N-methoxy-N-methylbenzamide (167-2)

The experimental operation was as described in Example **38,** using compound **167-1** as the raw material to obtain the title product.

MS (ESI) m/z [M+H]⁺ = 199.1.

### Step 2: Synthesis of tert-butyl (4-(methoxy(methyl)carbamoyl)phenyl)carbamate (167-3)

Compound **167-2** (10 g, 50.5 mmol) was dissolved in dichloromethane (100 mL), then di-tert-butyl dicarbonate (13.21 g, 60.6 mmol), triethylamine (15.3 g, 151 mmol), and 4-dimethylaminopyridine (1.23 g, 10.1 mmol) were sequentially added thereto, and the reaction was stirred at room temperature for 3 hours. Product generation was monitored by LCMS. The reaction mixture was quenched by adding saturated ammonium chloride (100 mL), and the phases were separated. The aqueous phase was extracted with dichloromethane (200 mL × 3). The organic phases were combined, washed with saturated brine (200 mL), dried over anhydrous sodium sulfate, filtered, and the filtrate was concentrated under reduced pressure. The residue was purified by silica gel column chromatography (petroleum ether: ethyl acetate = 2:1) to obtain the title product (11.2 g, yellow oil).

MS (ESI) m/z [M+H]⁺ = 399.2.

### Step 3: Synthesis of tert-butyl (2-fluoro-4-formylphenyl)carbamate (167-4)

The experimental operation was as described in step 5 in the synthesis of intermediate **A8,** using **167-3** as the raw material to obtain the title product.

MS (ESI) m/z [M+Na-COO*^{t}*Bu]⁺ = 362.2.

### Step 4: Synthesis of tert-butyl (4-(difluoromethyl)-2-fluorophenyl)carbamate (167-5)

Compound **167-4** (5.4 g, 22.6 mmol) was dissolved in dichloromethane (20 mL), then diethylaminosulfur trifluoride (10.9 g, 67.7 mmol) was added dropwise thereto, and the reaction was stirred at room temperature for 16 hours. The completion of the reaction was monitored by LCMS. The reaction mixture was quenched by adding saturated sodium bicarbonate solution (30 mL), and the phases were separated. The aqueous phase was extracted with dichloromethane (30 mL × 3). The organic phases were combined, washed with saturated brine (100 mL), dried over anhydrous sodium sulfate, filtered, and the filtrate was concentrated under reduced pressure to remove the solvent. The crude product of the title product was obtained (4.1 g, yellow oil).

MS (ESI) m/z [M+Na]⁺ = 384.2

### Step 5: Synthesis of 4-(difluoromethyl)-2-fluoroaniline hydrochloride (167-6)

The experimental operation was as described in step 2 in Example **59,** using compound **167-5** as the raw material to obtain the title product.

MS (ESI) m/z [M+H]⁺ = 162.1.

### Steps 6 to 9: Synthesis of 4-(6-((1-(4-(difluoromethyl)-2-fluorophenyl)-4-methyl-1H-1,2,3-triazol-5-yl)methoxy)pyridazin-3-yl)piperazin-2-one (167)

The experimental operation was as described in steps 4 to 6 in the synthesis of intermediate **A8** and Example **1,** using compounds **167-6** and **A3** as raw materials to obtain the title product.

MS (ESI) m/z [M+H]⁺ = 434.1.

¹H NMR (400 MHz, CDCl₃) δ 7.64 (t, *J* = 7.8 Hz, 1H), 7.44 (m, 2H), 6.94 (d, *J* = 9.6 Hz, 1H), 6.76 (d, *J* = 9.6 Hz, 1H), 6.70 (t, *J* = 56.0 Hz, 1H), 6.14 (s, 1H), 5.44 (s, 2H), 4.09 (s, 2H), 3.97 - 3.73 (m, 2H), 3.58 - 3.50 (m, 2H), 2.50 (s, 3H).

### Example 168

### (S)-8-(6-((1-(4-(Difluoromethyl)-2-fluorophenyl)-4-methyl-1H-1,2,3-triazol-5-yl)methoxy)pyridazin-3-yl)hexahydro-2H-pyrazino[1,2-a]pyrazin-1(6H)-one (168)

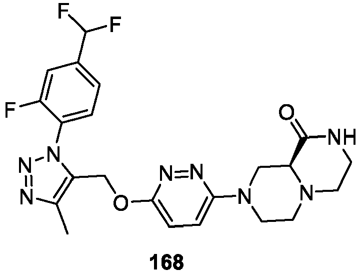

The experimental operation was as described in Example **1,** using compounds **77-4** and **167-9** as raw materials to obtain the title product.

MS (ESI) m/z [M+H]⁺ = 489.2.

¹H NMR (400 MHz, DMSO-*d*₆) δ 8.25 (br. s, 1H), 7.86 (d, *J* = 8.0 Hz, 1H), 7.81 (d, *J* = 12.4 Hz, 1H), 7.64 (d, *J* = 8.0 Hz, 1H), 7.48 (d, *J* = 9.6 Hz, 1H), 7.16 (t, *J* = 55.6 Hz, 1H), 6.98 (d, *J* = 9.6 Hz, 1H), 5.41 (s, 2H), 4.68 - 4.38 (m, 2H), 4.26 - 3.97 (m, 2H), 3.56 - 3.22 (m, 4H), 3.22 - 2.91 (m, 3H), 2.43 (s, 3H).

### Example 169

### 4-(5-((1-(4-(Difluoromethyl)phenyl)-4-methyl-1H-1,2,3-triazol-5-yl)methoxy)pyrazin-2-yl)piperazin-2-one (169)

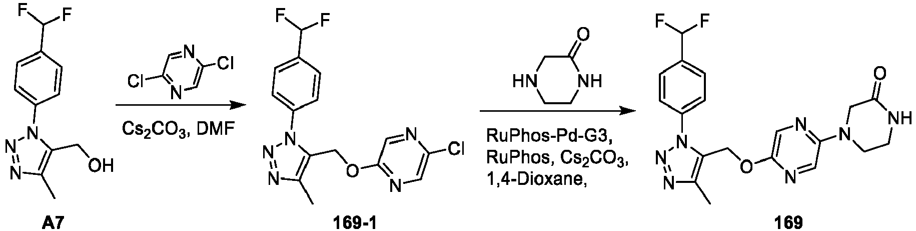

The experimental operation was as described in step 4 in the synthesis of intermediate **J8** and Example **1,** using compound **A7** and 2,5-dichloropyrazine as raw materials to obtain the title product.

MS (ESI) m/z [M+H]⁺ = 416.0.

¹H NMR (400 MHz, CDsOD) δ 7.63-7.91 (m, 6H), 6.90 (t, *J* = 55.6 Hz, 1H), 5.41 (s, 2H), 4.03 (s, 2H), 3.78 - 3.67 (m, 2H), 3.50 - 3.40 (m, 2H), 2.47 (s, 3H).

### Example 170

### 4-(6-((1-(4-(Difluoromethyl)phenyl)-4-methyl-1H-1,2,3-triazol-5-yl)methoxy)pyridin-3-yl)piperidin-2-one (170)

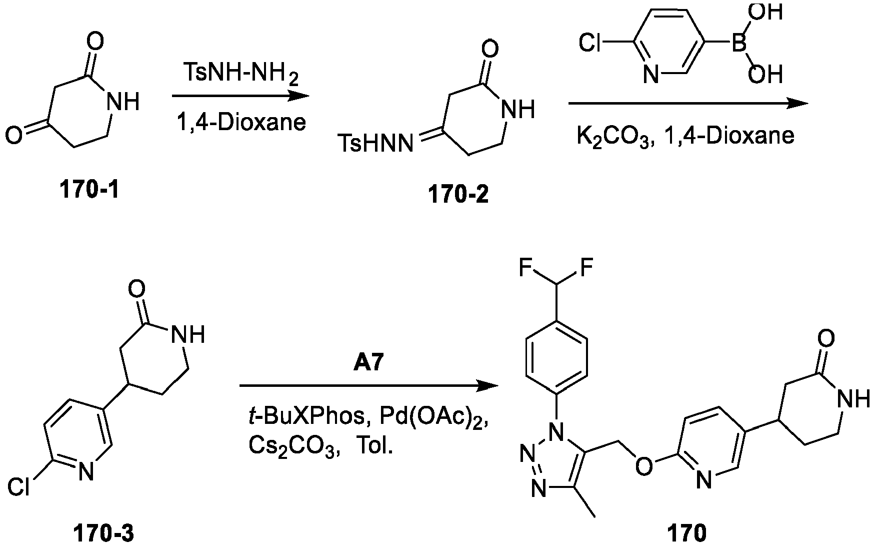

### Step 1: Synthesis of (Z/E)-4-methyl-N'-(2-oxopiperidin-4-ylidene)benzenesulfonohydrazide (170-2)

Compound **170-1** (2 g, 17.7 mmol), p-methylsulfonylhydrazine (3.29 g, 17.7 mmol), and sodium ethoxide (2.41 g, 35.4 mmol) were dissolved in methanol (30 mL), and the reaction mixture was stirred at 23°C for 12 hours. Product generation was shown by LCMS. The reaction mixture was filtered to obtain the title product (2.5 g, yellow solid).

¹H NMR (400 MHz, DMSO-*d*₆) δ 7.65 (br. s, 1H), 7.48 (d, *J* = 8.0 Hz, 2H), 7.10 (d, *J* = 8.0 Hz, 2H), 3.10 - 3.07 (m, 2H), 3.07 (s, 2H), 2.28 (s, 3H), 2.26 - 2.23 (m, 2H).

### Step 2: Synthesis of 4-(6-chloropyridin-3-yl)piperidin-2-one (170-3)

Compound **170-2** (500 mg, 1.78 mmol), 2-chloro-5-pyridineboronic acid (420 mg, 2.67 mmol), and potassium carbonate (368 mg, 2.67 mmol) were dissolved in 1,4-dioxane (10 mL), and the reaction mixture was stirred at 110°C for 12 hours. Product generation was detected by LCMS. The reaction mixture was filtered, and the filtrate was concentrated to obtain the crude product. The crude product was purified by high performance liquid chromatography (chromatographic column: Phenomenx C18 150 * 40 mm * 5 µm; mobile phase: water (FA) - ACN; gradient: 8% to 38%/10min; flow rate: 60 mL/min) to obtain the title product (150 mg, yellow solid).

MS (ESI) m/z [M+H]⁺ = 211.0.

### Step 3: Synthesis of 4-(6-((1-(4-(difluoromethyl)phenyl)-4-methyl-1H-1,2,3-triazol-5-yl)methoxy)pyridin-3-yl)piperidin-2-one (170)

The experimental operation was as described in step 4 in Example **132,** using compounds **170-3** and **A8** as raw materials to obtain the title product.

MS (ESI) m/z [M+H]⁺ = 414.4.

¹H NMR (400 MHz, CDCl₃) δ 7.98 (d, *J* = 2.4 Hz, 1H), 7.75 (d, *J* = 8.4 Hz, 2H), 7.67 (d, *J* = 8.4 Hz, 2H), 7.49 (dd, *J* = 8.4 Hz, 2.4Hz, 1H), 6.75 (d, *J* = 8.4 Hz, 1H), 6.73 (t, *J* = 55.6 Hz, 1H), 5.93 - 5.91 (m, 1H), 5.36 (s, 2H), 3.43 - 3.45 (m, 2H), 3.15 - 3.06 (m, 1H), 2.72 - 2.65 (m, 1H), 2.51 (s, 3H), 2.48 - 2.36 (m, 1H), 2.15 - 2.05 (m, 1H), 1.94 - 1.82 (m, 1H).

### Example 171

### 1-(4-(6-((1-(4-(Difluoromethyl)phenyl)-4-methyl-1H-1,2,3-triazol-5-yl)methoxy)pyridazin-3-yl)piperazin-1-yl)ethan-1-one (171)

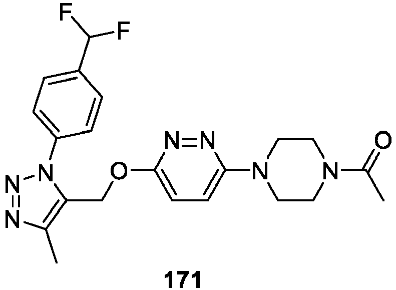

The experimental operation was as described in Example **1,** using compound **A8** and 1-acetylpiperazine as raw materials to obtain the title product.

MS (ESI) m/z [M+H]⁺ = 444.2.

¹H NMR (400 MHz, DMSO-*d*₆) δ 7.79 (s, 4H), 7.41 (d, *J* = 9.6 Hz, 1H), 7.14 (t, *J* = 55.6 Hz, 1H), 7.03 (d, *J* = 9.6 Hz, 1H), 5.46 (s, 2H), 3.57 - 3.51 (m, 4H), 3.51 - 3.45 (m, 2H), 3.45 - 3.39 (m, 2H), 2.40 (s, 3H), 2.04 (s, 3H).

### Example 172

### 1-(6-((1-(4-(Difluoromethyl)phenyl)-4-methyl-1H-1,2,3-triazol-5-yl)methoxy)pyridazin-3-yl)-N-ethyl-1,2,5,6-tetrahydropyridine-3-carboxamide (172)

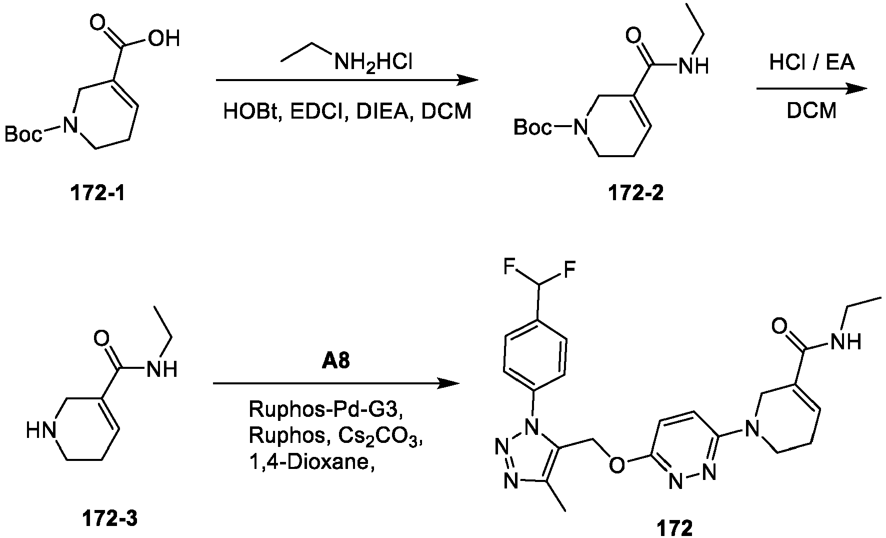

### Step 1: tert-Butyl 5-(ethylcarbamoyl)-3,6-dihydropyridine-1(2H)-carboxylate (172-2)

Compound **172-1** (500 mg, 2.2 mmol), ethylamine hydrochloride (149 mg, 3.3 mmol), HOBt (446 mg, 3.3 mmol), EDCI (633 mg, 3.3 mmol), and DIEA (569 mg, 4.4 mmol) were added to dichloromethane (30 mL) at room temperature, and the reaction was carried out at 20°C for 12 hours. TLC showed that new points were generated. The reaction mixture was added with water (100 mL) and extracted with dichloromethane (50 mL × 3). The organic phase was dried over anhydrous sodium sulfate and filtered, and the filtrate was concentrated under reduced pressure. The crude product was purified by flash silica gel column chromatography (ethyl acetate: petroleum ether = 1:50 to 1:1) to obtain the title product (460 mg, colorless liquid).

¹H NMR (400 MHz, CDCl₃) *δ* 5.72 (br. s, 1H), 4.14 - 4.09 (m, 2H), 3.47 (t, *J* = 5.6 Hz, 2H), 3.39 - 3.34 (m, 2H), 2.27 - 2.23 (m, 2H), 1.47 (s, 9H), 1.17 (t, *J* = 7.2 Hz, 3H)

### Step 2: Synthesis of N-ethyl-1,2,5,6-tetrahydropyridine-3-carboxamide (172-3)

The experimental operation was as described in step 2 in Example **59,** using compound **172-2** as the raw material to obtain the title product.

### Step 3: Synthesis of 1-(6-((1-(4-(difluoromethyl)phenyl)-4-methyl-1H-1,2,3-triazol-5-yl)methoxy)pyridazin-3-yl)-N-ethyl-1,2,5,6-tetrahydropyridine-3-carboxamide (172)

The experimental operation was as described in Example **1,** using compounds **172-3** and **A8,** and 1-acetylpiperazine as raw materials to obtain the title product.

MS (ESI) m/z [M+H]⁺ = 470.1.

¹H NMR: (400 MHz, CDsOD) *δ* 7.77 (s, 4H), 7.37 (d, *J* = 9.6 Hz, 1H), 6.98 (d, *J* = 9.6 Hz, 1H), 6.89 (t, *J* = 55.6 Hz, 1H), 6.72 - 6.66 (m, 1H), 5.49 (s, 2H), 4.16-4.20 (m, 2H), 3.66 (t, *J* = 5.6 Hz, 2H), 3.25-3.30 (m, 2H), 2.48 (s, 3H), 2.36-2.44 (m, 2H), 1.16 (t, *J* = 7.2 Hz, 3H).

### Example 173 and Example 174

### (R)-8-(6-((1-(4-(Difluoromethyl)phenyl)-4-methyl-1H-1,2,3-triazol-5-yl)methoxy)pyridazin-3-yl)hexahydropyrazino[2,1-c][1,4]oxazin-6(1H)-one (173)

### (S)-8-(6-((1-(4-(Difluoromethyl)phenyl)-4-methyl-1H-1,2,3-triazol-5-yl)methoxy)pyridazin-3-yl)hexahydropyrazino[2,1-c][1,4]oxazin-6(1H)-one (173)

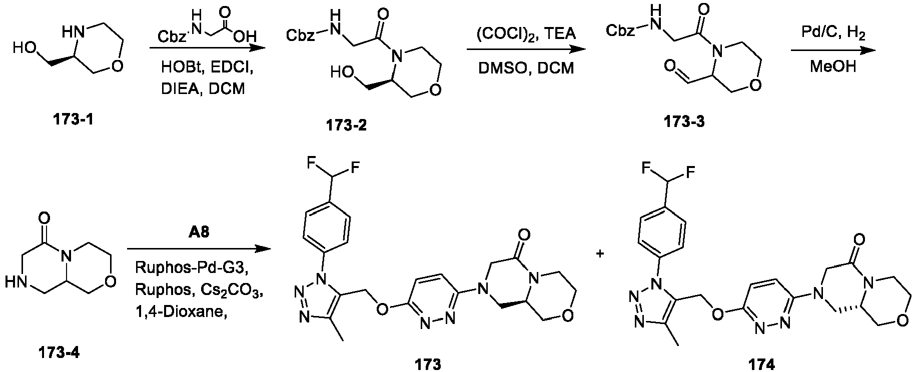

The experimental operation was as described in steps 1 to 4 in Example **68,** using compound **173-1** and *N*-benzyloxycarbonylglycine as raw materials, and the obtained enantiomers were separated by SFC (chromatographic column: DAICEL CHIRALPAK AS (250 mm * 30 mm, 10 µm); mobile phase: 4 mL ammonia hydroxide / 4 L ethanol, carbon dioxide; gradient: 40% to 40%; flow rate: 80 mL/min) to obtain the title product.

### Compound 173:

MS (ESI) m/z [M+H]⁺ = 472.1.

¹H NMR (400 MHz, CD₃OD) δ 7.77 (s, 4H), 7.40 (d, *J* = 9.6 Hz, 1H), 7.02 (d, *J* = 9.6 Hz, 1H), 6.83 (d, *J* = 55.6 Hz, 1H), 5.50 (s, 2H), 4.47 - 4.35 (m, 2H), 4.25 - 4.14 (m, 1H), 4.01 - 3.91 (m, 3H), 3.79 (s, 1H), 3.54 - 3.45 (m, 1H), 3.23 - 3.11 (m, 2H), 2.95 - 2.82 (m, 1H), 2.48 (s, 3H).

### Compound 174:

MS (ESI) m/z [M+H]⁺ = 472.1.

¹H NMR (400 MHz, CDsOD) δ 7.77 (s, 4H), 7.40 (d, *J* = 9.6 Hz, 1H), 7.03 (d, *J* = 9.6 Hz, 1H), 6.83 (d, *J* = 55.6 Hz, 1H), 5.50 (s, 2H), 4.47 - 4.36 (m, 2H), 4.24 - 4.15 (m, 1H), 4.00 - 3.91 (m, 3H), 3.79 (t, *J* = 10.0 Hz, 1H), 3.51 - 3.45 (m, 1H), 3.27 (s, 1H), 3.20-3.14 (m, 1H), 2.95 - 2.83 (m, 1H), 2.48 (s, 3H).

### Example 175 and Example 176

### (R)-8-(6-((1-(4-(Difluoromethyl)phenyl)-4-methyl-1H-1,2,3-triazol-5-yl)methoxy)pyridazin-3-yl)hexahydropyrazino[2,1-c][1,4]thiazin-6(1H)-one 2,2-dioxide (175)

### (S)-8-(6-((1-(4-(Difluoromethyl)phenyl)-4-methyl-1H-1,2,3-triazol-5-yl)methoxy)pyridazin-3-yl)hexahydropyrazino[2,1-c][1,4]thiazin-6(1H)-one 2,2-dioxide (176)

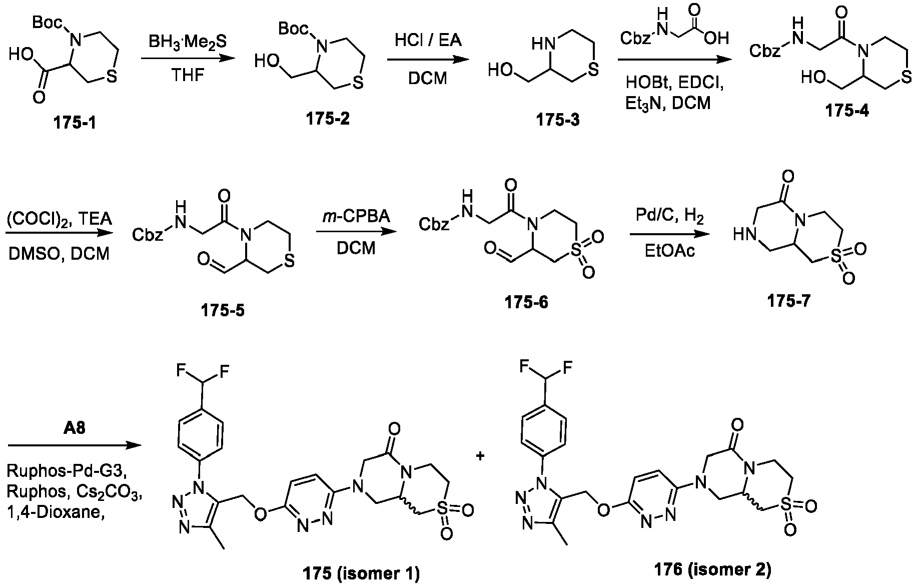

### Step 1: Synthesis of tert-butyl 3-(hydroxymethyl)thiomorpholine-4-carboxylate (175-2)

Compound **175-1** (2.0 g, 8.09 mmol) was added to tetrahydrofuran (20 mL) at room temperature, then BH₃·Me₂S (2.43 mL, 10 M, 24.3 mmol) was added dropwise thereto. After the dropwise addition, the reaction mixture was heated to 70°C and reacted for 5 hours. TLC showed that new points were generated. The reaction mixture was quenched by slow dropwise addition of methanol (2 mL), and stirred at 70°C for another 1 hour. The reaction mixture was concentrated under reduced pressure, and the crude product was purified by column chromatography (dichloromethane: methanol = 99:1 to 20:1) to obtain the title product (1.4 g, white solid).

¹H NMR (400 MHz, CDCl₃) *δ* 4.67 - 4.37 (m, 1H), 4.33 - 4.12 (m, 1H), 4.04 - 3.86 (m, 2H), 3.28 - 3.01 (m, 1H), 2.98 - 2.87 (m, 1H), 2.77 - 2.55 (m, 2H), 2.49 - 2.37 (m, 1H), 1.47 (s, 9H)

### Step 2: Synthesis of thiomorpholin-3-ylmethanol (175-3)

The experimental operation was as described in step 2 in Example **59,** using **175-2** as the raw material to obtain the title product.

### Step 3: Synthesis of benzyl (2-(3-(hydroxymethyl)thiomorpholino)-2-oxoethyl)carbamate (175-4)

The experimental operation was as described in step 1 in Example **68,** using **175-3** and N-benzyloxycarbonylglycine as raw materials to obtain the title product.

¹H NMR (400 MHz, CDCl₃) *δ* 7.41 - 7.29 (m, 5H), 5.90 - 5.73 (m, 1H), 5.13 (s, 2H), 5.02 - 4.70 (m, 1H), 4.30 - 4.13 (m, 1H), 4.11 - 3.76 (m, 4H), 3.09 - 2.39 (m, 5H), 2.23 - 1.94 (m, 1H).

### Step 4: Synthesis of benzyl (2-(3-formylthiomorpholino)-2-oxoethyl)carbamate (175-5)

The experimental operation was as described in step 2 in Example **68,** using **175-4** as the raw material to obtain the title product.

¹H NMR (400 MHz, CDCl₃) *δ* 7.43 - 7.32 (m, 5H), 5.83 - 5.74 (m, 1H), 5.21 - 5.12 (m, 2H), 4.95 - 4.86 (m, 1H), 4.45 - 4.33 (m, 1H), 4.07 - 3.97 (m, 1H), 3.82 - 3.71 (m, 1H), 3.04 - 2.92 (m, 2H), 2.80 - 2.71 (m, 1H), 2.64 - 2.51 (m, 2H).

### Step 5: Synthesis of benzyl (2-(3-formyl-1,1-dioxidothiomorpholino)-2-oxoethyl)carbamate (175-6)

Compound **175-5** (300 mg, 0.931 mmol) and *m*-CPBA (642 mg, 3.72 mmol) were sequentially added to dichloromethane (20 mL) and reacted at 22°C for 3 hours. TLC showed that new points were generated. The reaction mixture was added with saturated sodium thiosulfate (30 mL) and extracted with dichloromethane (10 mL × 3). The organic phase was dried over anhydrous sodium sulfate and filtered, and the filtrate was concentrated under reduced pressure. The crude product was purified by column chromatography (dichloromethane: methanol = 100:1 to 50:1) to obtain the title product (320 mg, white solid).

¹H NMR (400 MHz, CDCl₃) *δ* 7.43 - 7.32 (m, 5H), 5.88 - 5.63 (m, 1H), 5.24 - 5.03 (m, 3H), 4.54 - 4.35 (m, 1H), 4.24 - 4.07 (m, 1H), 4.07 - 3.94 (m, 1H), 3.85 - 3.56 (m, 1H), 3.42 - 2.92 (m, 5H)

### Step 6: Synthesis of hexahydropyrazino[2,1-c][1,4]thiazin-6(1H)-one 2,2-dioxide (175-7)

The experimental operation was as described in step 3 in Example **68,** using **175-6** as the raw material to obtain the title product.

### Step 7: Synthesis of (R or S)-8-(6-((1-(4-(difluoromethyl)phenyl)-4-methyl-1H-1,2,3-triazol-5-yl)methoxy)pyiidazin-3-yl)hexahydropyrazino[2,1-c][1,4]thiazin-6(1H)-one 2,2-dioxide (175) and (S or R)-8-(6-((1-(4-(difluoromethyl)phenyl)-4-methyl-1H-1,2,3-triazol-5-yl)methoxy)pyridazin-3-yl)hexahydropyrazino[2,1-c][1,4]thiazin-6(1H)-one 2,2-dioxide (176)

The experimental operation was as described in Example **1,** using compounds **175-7** and **A8** as raw materials, and the obtained enantiomers were separated by SFC (chromatographic column: DAICEL CHIRALPAK AS (250 mm * 30 mm, 10 µm); mobile phase: 4 mL ammonia hydroxide / 4 L ethanol, carbon dioxide; gradient: 50% to 50%; flow rate: 80 mL/min) to obtain the title product.

### Compound 175:

Chiral analysis retention time: Rt = 5.230 min. (Chiral analysis conditions: chromatographic column: Chiralpak AS-3 100 * 4.6 mm I.D., 3 µm; mobile phase: A: carbon dioxide, B: ethanol (0.05% DEA); gradient: 5% to 40% B for 4 min, maintained 40% B for 2.5 min, then 5% B for 1.5 min; flow rate: 2.8 mL/min; column temperature: 35°C, column pressure: 1500 psi).

MS (ESI) m/z [M+H]⁺ = 520.1. ¹H NMR (400 MHz, CDsOD) *δ* 7.76 (s, 4H), 7.38 (d, *J* = 9.6 Hz, 1H), 7.03 (d, *J* = 9.6 Hz, 1H), 6.89 (t, *J* = 55.6 Hz, 1H), 5.51 (s, 2H), 5.08 - 5.01 (m, 1H), 4.44 - 4.40 (m, 1H), 4.20 - 4.06 (m, 2H), 3.97 (d, *J* = 17.6 Hz, 1H), 3.69 - 3.80 (m, 1H), 3.42 - 3.53 (m, 1H), 3.28 - 3.08 (m, 4H), 2.47 (s, 3H).

### Compound 176:

Chiral analysis retention time: Rt = 5.729 min. (Chiral analysis conditions: chromatographic column: Chiralpak AS-3 100 * 4.6 mm I.D., 3 µm; mobile phase: A: carbon dioxide, B: ethanol (0.05% DEA); gradient: 5% to 40% B for 4 min, maintained 40% B for 2.5 min, then 5% B for 1.5 min; flow rate: 2.8 mL/min; column temperature: 35°C, column pressure: 1500 psi).

MS (ESI) m/z [M+H]⁺ = 520.1.

¹H NMR: (400 MHz, CD₃OD) *δ* 7.76 (s, 4H), 7.38 (d, *J* = 9.6 Hz, 1H), 7.03 (d, *J* = 9.6 Hz, 1H), 6.89 (t, *J* = 55.6 Hz, 1H), 5.51 (s, 2H), 5.08-5.01 (m, 1H), 4.44 - 4.40 (m, 1H), 4.20 - 4.06 (m, 2H), 3.97 (d, *J* = 17.6 Hz, 1H), 3.69 - 3.80 (m, 1H), 3.42 - 3.53 (m, 1H), 3.26 - 3.10 (m, 4H), 2.48 (s, 3H).

### Example 177

### (3S,9aR)-8-(6-((1-(4-(Difluoromethyl)phenyl)-4-methyl-1H-1,2,3-triazol-5-yl)methoxy)pyridazin-3-yl)tetrahydro-1H-3,9a-methanopyrazino[2,1-c][1,4]oxazin-6(7H)-one (177)

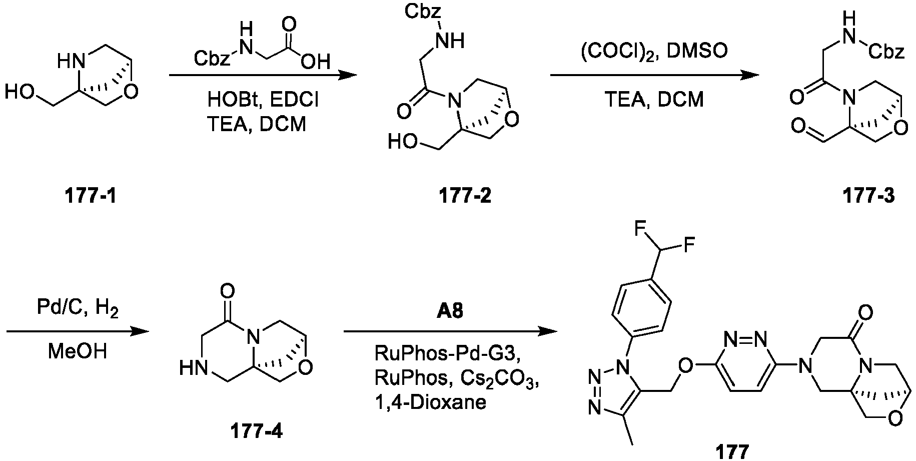

The experimental operation was as described in steps 1 to 4 in Example **68,** using compound **177-1** and *N*-benzyloxycarbonylglycine as raw materials to obtain the title product.

MS (ESI) m/z [M+H]⁺ = 484.2.

¹H NMR (400 MHz, DMSO-*d*₆) δ 7.78 (s, 4H), 7.46 (d, *J* = 9.6 Hz, 1H), 7.13 (t, *J* = 56.0 Hz, 1H), 7.05 (d, *J* = 9.6 Hz, 1H), 5.46 (s, 2H), 4.68 - 4.62 (m, 2H), 4.43 - 4.37 (m, 1H), 3.74 - 3.70 (m, 2H), 3.67 - 3.56 (m, 2H), 3.45 - 3.41 (m, 1H), 3.30 - 3.25 (m, 1H), 2.39 (s, 3H), 2.06 - 2.03 (m, 1H), 1.78 - 1.75 (m, 1H).

### Example 178

### (S)-8-(5-((1-(4-(Difluoromethyl)phenyl)-4-methyl-1H-1,2,3-triazol-5-yl)methoxy)pyrazin-2-yl)hexahydro-2H-pyrazino[1,2-a]pyrazin-1(6H)-one (178)

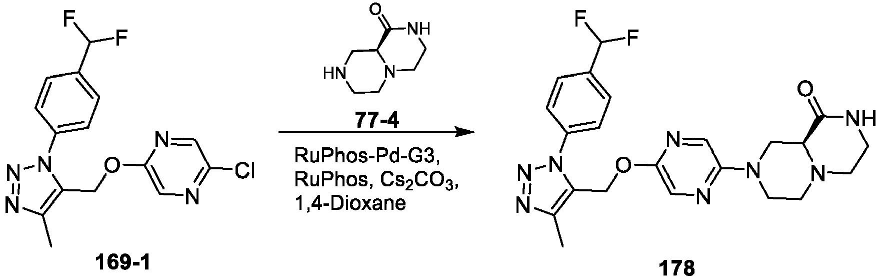

The experimental operation was as described in Example **1,** using compounds **169-1** and **77-4** as raw materials to obtain the title product.

MS (ESI) m/z [M+H]⁺ = 471.1.

¹H NMR (400 MHz, CDsOD) δ 7.65-7.86 (m, 6H), 6.90 (t, *J* = 56.0 Hz, 1H), 5.40 (s, 2H), 4.52 - 4.46 (m, 1H), 4.06 - 4.02 (m, 1H), 3.54 - 3.46 (m, 1H), 3.26 - 3.20 (m, 1H), 3.07 - 2.89 (m, 3H), 2.87 - 2.83 (m, 1H), 2.75 - 2.67 (m, 1H), 2.61 - 2.55 (m, 1H), 2.47 (s, 3H), 2.38 - 2.45 (m, 1H).

### Example 179

### 2-(6-((1-(4-(Difluoromethyl)phenyl)-4-methyl-1H-1,2,3-triazol-5-yl)methoxy)pyridazin-3-yl)tetrahydro-6H-7,8a-methanopyrrolo[1,2-a]pyrazin-4(1H)-one

(**179**)

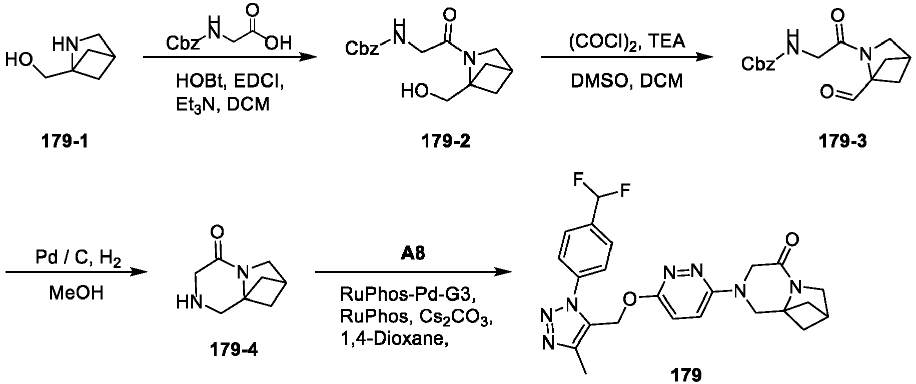

The experimental operation was as described in steps 1 to 4 in Example **68,** using compound **179-1** and *N*-benzyloxycarbonylglycine as raw materials to obtain the title product.

MS (ESI) m/z [M+H]⁺ = 468.2.

¹H NMR (400 MHz, DMSO-*d*₆) δ 7.76 (s, 4H), 7.48 (d, *J* = 9.6 Hz, 1H), 7.11 (t, *J* = 56.0 Hz, 1H), 7.01 (d, *J* = 9.6 Hz, 1H), 5.44 (s, 2H), 4.05 (s, 2H), 3.93 (s, 2H), 3.28 (s, 2H), 2.78 (s, 1H), 2.37 (s, 3H), 1.93 - 1.90 (m, 2H), 1.27 - 1.23 (m, 2H).

### Example 180

### 2-(6-((1-(4-(Difluoromethyl)phenyl)-4-methyl-1H-1,2,3-triazol-5-yl)methoxy)pyridazin-3-yl)hexahydro-3H-imidazo[5,1-c][1,4]oxazin-3-one (180)

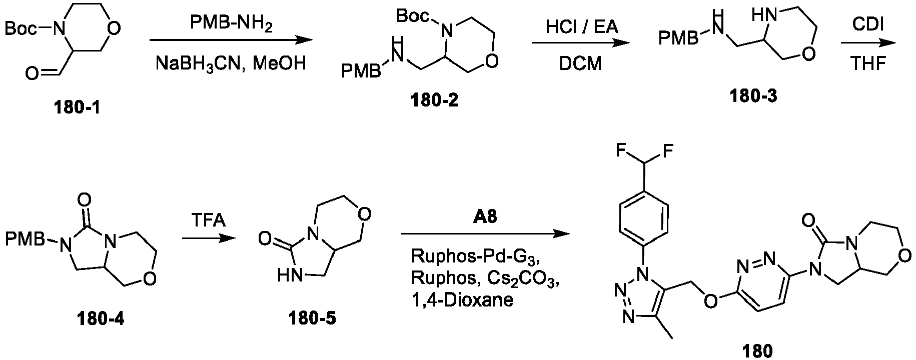

### Step 1: Synthesis of tert-butyl 3-(((4-methoxybenzyl)amino)methyl)morpholine-4-carboxylate (180-2)

Compound **180-1** (3.0 g, 13.9 mmol) and 4-methoxybenzylamine (2.29 g, 16.7 mmol) were added to methanol (50.0 mL) at room temperature, and then acetic acid was added thereto to adjust the pH of the reaction mixture to 6. The reaction was carried out at 25°C for 2 hours, then sodium cyanoborohydride (2.63 g, 41.8 mmol) was added thereto, and the reaction was carried out at 25°C for another 12 hours. TLC showed that new points were generated. The reaction mixture was diluted with dichloromethane (150 mL), washed with saturated sodium bicarbonate aqueous solution (50 mL × 3) and saturated brine (50 mL × 3). The organic phase was dried over anhydrous sodium sulfate, filtered, and concentrated under reduced pressure. The crude product was purified by silica gel column chromatography (dichloromethane: methanol = 1:0 to 10:1) to obtain the title product (1.9 g, yellow oil).

### Step 2: Synthesis of N-(4-methoxybenzyl)-1-(morpholin-3-yl)methylamine (180-3)

The experimental operation was as described in step 2 in Example **59,** using compound **180-2** as the raw material to obtain the title product.

¹H NMR (400 MHz, CDsOD) *δ* 7.52 (d, *J* = 8.8, 2H), 7.02 (d, *J* = 8.8, 2H), 4.26 (s, 2H), 4.13 - 4.08 (m, 1H), 4.06 - 3.89 (m, 1H), 3.88 - 3.83 (m, 1H), 3.82 (s, 3H), 3.80-3.77 (m, 1H), 3.77 - 3.72 (m, 1H), 3.48-3.40 (m, 3H), 3.30 - 3.25 (m, 1H).

### Step 3: Synthesis of 2-(4-methoxybenzyl)hexahydro-3H-imidazo[5,1-c][1,4]oxazin-3-one (180-4)

Compound **180-3** (700 mg, 2.96 mmol), CDI (1.44 g, 8.89 mmol), and DBU (1.80 g, 11.85 mmol) were sequentially added to anhydrous tetrahydrofuran (10 mL) at room temperature. The system was replaced with nitrogen three times, and the reaction was carried out at 75°C for 2 hours. Product generation was shown by LCMS. The reaction mixture was diluted with water (40.0 mL) and extracted with ethyl acetate (40.0 mL × 3). The organic phase was dried over anhydrous sodium sulfate and filtered, and the filtrate was concentrated under reduced pressure. The crude product was purified by silica gel column chromatography (dichloromethane: methanol = 1:0 to 10:1) to obtain the title product (550 mg, yield: 70.7%).

MS (ESI) m/z [M+H]⁺ = 262.9.

¹H NMR (400 MHz, CDCl₃) *δ* 7.21 - 7.16 (m, 2H), 6.90 - 6.84 (m, 2H), 4.33 (d, *J* = 2.4 Hz, 2H), 3.84 - 3.67 (m, 7H), 3.50 - 3.40 (m, 1H), 3.27 - 3.19 (m, 2H), 3.18 - 3.05 (m, 1H), 2.75 - 2.69 (m, 1H).

### Step 4: Synthesis of hexahydro-3H-imidazo[5,1-c][1,4]oxazin-3-one (180-5)

Compound **186-4** (550 mg, 2.10 mmol) was added to anhydrous dichloromethane (3 mL) at room temperature, then TFA (3.00 mL) was slowly added thereto. The reaction was carried out at 50°C for 15 hours. TLC showed that new points were generated. The reaction mixture was added with saturated sodium bicarbonate aqueous solution to adjust the pH to 8, and extracted with dichloromethane (40 mL × 3). The organic phase was dried over anhydrous sodium sulfate, filtered, and concentrated under reduced pressure to obtain the title product (400 mg, brown oil), and the crude product was directly used in the next reaction step without further purification.

### Step 4: Synthesis of 2-(6-((1-(4-(difluoromethyl)phenyl)-4-methyl-1H-1,2,3-triazol-5-yl)methoxy)pyridazin-3-yl)hexahydro-3H-imidazo[5,1-c][1,4]oxazin-3-one (180)

The experimental operation was as described in Example **1,** using compounds **180-5** and **A8** as raw materials to obtain the title product.

MS (ESI) m/z [M+H]⁺ = 507.1.

¹H NMR (400 MHz, CDsOD) *δ* 7.74 (d, *J* = 8.4 Hz, 2H), 7.63 (d, *J* = 8.4 Hz, 2H), 7.39 (d, *J* = 9.6 Hz, 1H), 7.00 (d, *J* = 9.6 Hz, 1H), 6.89 (t, *J* = 55.6 Hz, 1H), 5.56 (s, 2H), 4.17 - 4.12 (m, 1H), 3.99 - 3.86 (m, 3H), 3.82 - 3.75 (m, 1H), 3.67 - 3.63 (m, 1H), 3.47 - 3.43 (m, 1H), 3.35 - 3.33 (m, 1H), 3.19 - 3.13 (m, 1H), 2.49 (s, 3H).

### Example 181

### (S)-8-(6-((4-Chloro-1-(4-(difluoromethyl)phenyl)-1H-1,2,3-triazol-5-yl)methoxy)pyridazin-3-yl)hexahydro-2H-pyrazino[1,2-a]pyrazin-1(6H)-one (181)

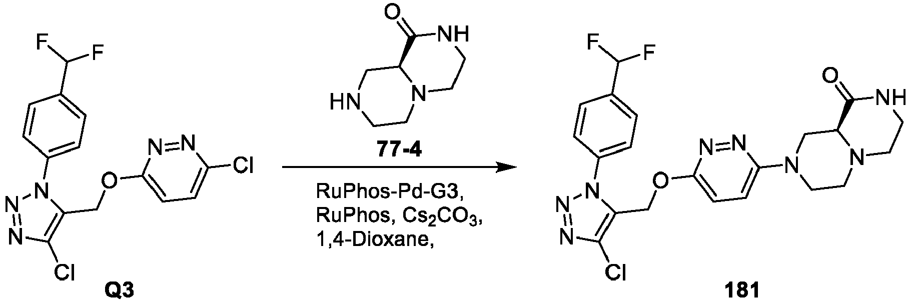

The experimental operation was as described in Example **1,** using compounds **Q3** and **77-4** as raw materials to obtain the title product.

MS (ESI) m/z [M+H]⁺ = 491.1.

¹H NMR (400 MHz, CDsOD) *δ* 7.81 - 7.76 (m, 4H), 7.38 (d, *J* = 9.6 Hz, 1H), 6.98 (d, *J* = 9.6 Hz, 1H), 6.89 (t, *J* = 55.6 Hz, 1H), 5.51 (s, 2H), 4.51 - 4.43 (m, 1H), 4.12 - 4.08 (m, 1H), 3.55 - 3.45 (m, 1H), 3.26 - 3.22 (m, 1H), 3.08 - 2.99 (m, 3H), 2.89 - 2.81 (m, 2H), 2.63 - 2.56 (m, 1H), 2.48 - 2.42 (m, 1H).

### Example 182 and Example 183

### (R)-8-(6-((4-Chloro-1-(4-(difluoromethyl)phenyl)-1H-1,2,3-triazol-5-yl)methoxy)pyridazin-3-yl)hexahydropyrazino[2,1-c][1,4]oxazin-6(1H)-one (182)

### (S)-8-(6-((4-Chloro-1-(4-(difluoromethyl)phenyl)-1H-1,2,3-triazol-5-yl)methoxy)pyridazin-3-yl)hexahydropyrazino[2,1-c][1,4]oxazin-6(1H)-one (183)

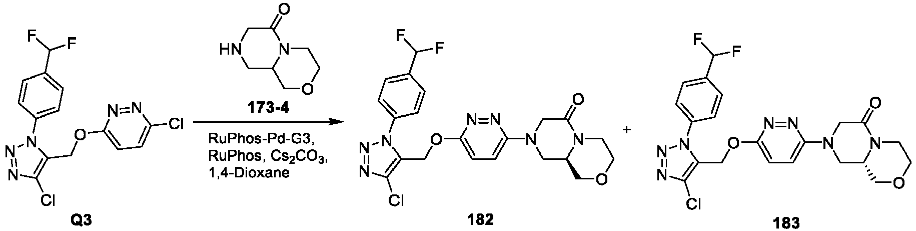

The experimental operation was as described in Example **1,** using compounds **Q3** and **173-4** as raw materials, through SFC (chromatographic column: DAICEL CHIRALCEL OJ (250 mm * 30 mm, 10 µm); mobile phase: ethanol (0.1% ammonia hydroxide)]; flow rate: 80 mL/min) to obtain the title product.

### Compound 182:

Chiral analysis retention time: Rt = 4.056 min. (Chiral analysis conditions: chromatographic column: Chiralcel OJ-3 100 * 4.6 mm I.D., 3 µm; mobile phase: A: carbon dioxide, B: ethanol (0.05% DEA); gradient: 5% to 40% B for 4 min, maintained 40% B for 2.5 min, then 5% B for 1.5 min; flow rate: 2.8 mL/min; column temperature: 35°C, column pressure: 1500 psi).

MS (ESI) m/z [M+H]⁺ = 492.1.

¹H NMR (400 MHz, CDsOD) *δ* 7.81 - 7.76 (m, 4H), 7.39 (d, *J* = 9.6 Hz, 1H), 7.01 (d, *J* = 9.6 Hz, 1H), 6.89 (t, *J* = 55.6 Hz, 1H), 5.52 (s, 2H), 4.44 - 4.38 (m, 2H), 4.22 - 4.18 (m, 1H), 3.98 - 3.93 (m, 3H), 3.81 - 3.76 (m, 1H), 3.52 - 3.45 (m, 1H), 3.30 - 3.26 (m, 1H), 3.20 - 3.15 (m, 1H), 2.93 - 2.85 (m, 1H).

### Compound 183:

Chiral analysis retention time: Rt = 4.402 min. (Chiral analysis conditions: chromatographic column: Chiralcel OJ-3 100 * 4.6 mm I.D., 3 µm; mobile phase: A: carbon dioxide, B: ethanol (0.05% DEA); gradient: 5% to 40% B for 4 min, maintained 40% B for 2.5 min, then 5% B for 1.5 min; flow rate: 2.8 mL/min; column temperature: 35°C, column pressure: 1500 psi).

MS (ESI) m/z [M+H]⁺ = 492.1.

¹H NMR (400 MHz, CD₃OD) *δ* 7.81 - 7.76 (m, 4H), 7.39 (d, *J* = 9.6 Hz, 1H), 7.01 (d, *J* = 9.6 Hz, 1H), 6.89 (t, *J* = 55.6 Hz, 1H), 5.52 (s, 2H), 4.44 - 4.38 (m, 2H), 4.22 - 4.17 (m, 1H), 3.98 - 3.93 (m, 3H), 3.82 - 3.76 (m, 1H), 3.51 - 3.45 (m, 1H), 3.30 - 3.26 (m, 1H), 3.20 - 3.15 (m, 1H), 2.93 - 2.86 (m, 1H).

### Example 184

### (S)-8-(6-((4-(Difluoromethyl)-1-(4-(difluoromethyl)phenyl)-1H-1,2,3-triazol-5-yl)methoxy)pyridazin-3-yl)hexahydro-2H-pyrazino[1,2-a]pyrazin-1(6H)-one (184)

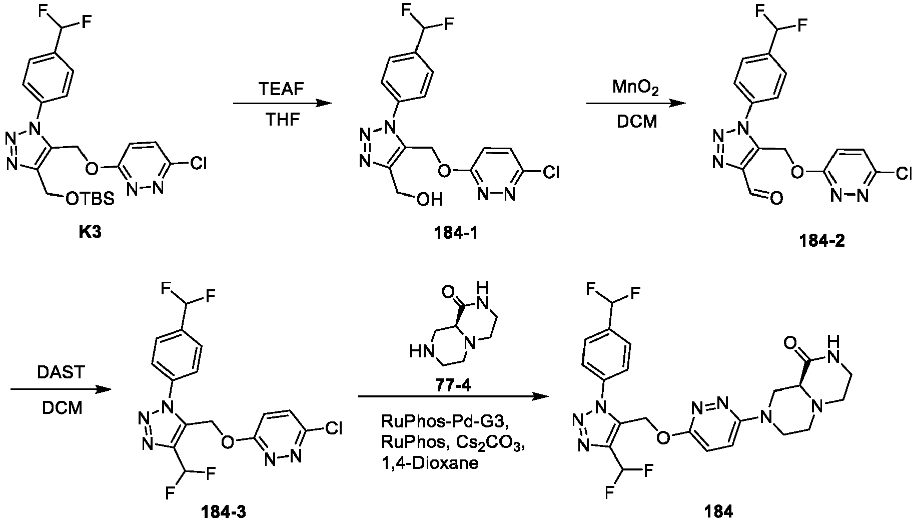

### Step 1: Synthesis of (5-(((6-chloropyridazin-3-yl)oxy)methyl)-1-(4-(difluoromethyl)phenyl)-1H-1,2,3-triazol-4-yl)methanol (184-1)

The experimental operation was as described in step 3 in Example **66,** using compound K3 as the raw material to obtain the title product.

MS (ESI) m/z [M+H]⁺ = 368.0.

### Step 2: Synthesis of 5-(((6-chloropyridazin-3-yl)oxy)methyl)-1-(4-(difluoromethyl)phenyl)-1H-1,2,3-triazole-4-carbaldehyde (184-2)

Compound **184-1** (180 mg, 0.489 mmol) and active manganese dioxide (426 mg, 4.89 mmol) were sequentially added to anhydrous dichloromethane (10 mL) at room temperature. The system was replaced with nitrogen three times, and the reaction was carried out at 24°C for 20 hours. LCMS showed that the reaction was completed. The reaction mixture was filtered and the filtrate was concentrated under reduced pressure to obtain the title product (150 mg, yellow oil).

MS (ESI) m/z [M+H]⁺ = 365.9.

### Step 3: Synthesis of 3-chloro-6-((4-(difluoromethyl)-1-(4-(difluoromethyl)phenyl)-1H-1,2,3-triazol-5-yl)methoxy)pyridazine (184-3)

The experimental operation was as described in step 4 in Example **167,** using compound **184-3** as the raw material to obtain the title product.

MS (ESI) m/z [M+H]⁺ = 387.9.

### Step 4: Synthesis of (S)-8-(6-((4-(Difluoromethyl)-1-(4-(difluoromethyl)phenyl)-1H-1,2,3-triazol-5-yl)methoxy)pyridazin-3-yl)hexahydro-2H-pyrazino[1,2-a]pyrazin-1(6H)-one (184)

The experimental operation was as described in Example **1,** using compounds **184-3** and **77-4** as raw materials to obtain the title product.

MS (ESI) m/z [M+H]⁺ = 507.1.

¹H NMR (400 MHz, CDsOD) *δ* 7.84 - 7.76 (m, 4H), 7.39 (d, *J* = 9.6 Hz, 1H), 7.25 (t, *J* = 54.0 Hz, 1H), 7.00 (d, *J* = 9.6 Hz, 1H), 6.91 (t, *J* = 56.0 Hz, 1H), 5.60 (s, 2H), 4.49 - 4.44 (m, 1H), 4.12 - 4.09 (m, 1H), 3.58 - 3.45 (m, 1H), 3.27 - 3.23 (m, 1H), 3.10 - 2.98 (m, 3H), 2.91 - 2.81 (m, 2H), 2.64 - 2.55 (m, 1H), 2.50 - 2.41 (m, 1H).

### Example 185

### 4-(6-((4-(Difluoromethyl)-1-(4-(difluoromethyl)phenyl)-1H-1,2,3-triazol-5-yl)methoxy)pyridazin-3-yl)piperazin-2-one (185)

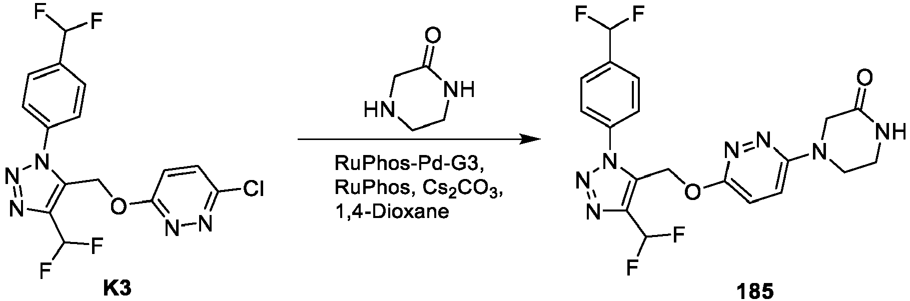

The experimental operation was as described in Example **1,** using compound **K3** and 2-piperazinone as raw materials to obtain the title product.

MS (ESI) m/z [M+H]⁺ = 452.0.

¹H NMR (400 MHz, CDsOD) *δ* 7.81 (s, 4H), 7.35 (d, *J* = 9.6 Hz, 1H), 7.33 (t, *J* = 54.0 Hz, 1H), 7.03 (d, *J* = 9.6 Hz, 1H), 6.91 (t, *J* = 56.0 Hz, 1H), 5.61 (s, 2H), 4.12 (s, 2H), 3.78 - 3.73 (m, 2H), 3.47 - 3.43 (m, 2H).

### Example 186

### 4-(6-((4-Cyclopropylmethyl)-1-(4-(difluoromethyl)phenyl)-1H-1,2,3-triazol-5-yl)methoxy)pyridazin-3-yl)piperazin-2-one (186)

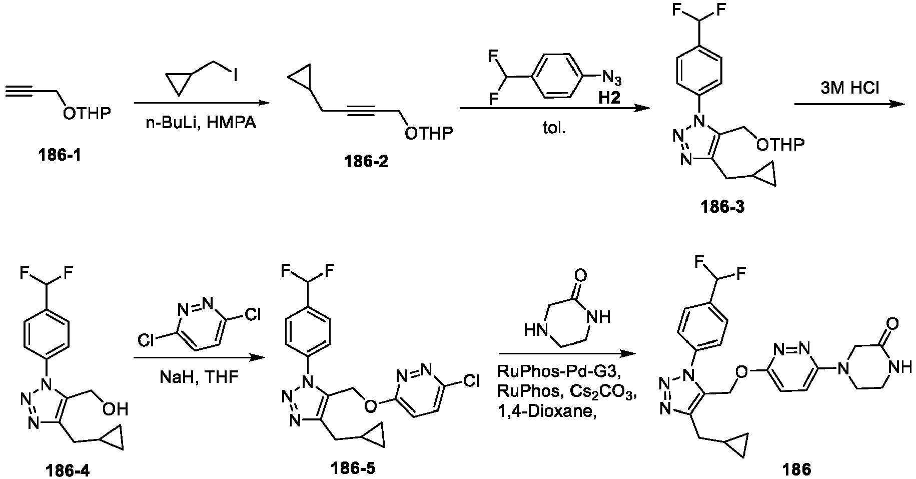

### Step 1: Synthesis of 2-((4-cyclopropylbut-2-yn-1-yl)oxy)tetrahydro-2H-pyran (186-2)

Compound **186-1** (1.4 g, 9.99 mmol) was dissolved in THF (50 mL) at -40°C under a nitrogen atmosphere, and then *n*-butyllithium (4.0 mL, 9.99 mmol, 2.5 M *n*-hexane solution) was added thereto. After the reaction was stirred for 30 minutes, hexamethylphosphoramide (1.43 g, 8 mmol) and (iodomethyl)cyclopropane (0.91 g, 5.00 mmol) were added thereto at - 40°C, and the reaction was stirred at -40°C for another 2 hours. The completion of the reaction was monitored by TLC. The reaction mixture was quenched by adding water (40 mL), and extracted with dichloromethane (50 mL × 3). The organic phases were combined, washed with saturated brine (30 mL), dried over anhydrous sodium sulfate, filtered, and subjected to rotary evaporation. The residue was purified by flash silica gel column chromatography (petroleum ether: ethyl acetate = 10:1) to obtain the title product (1.06 g, white solid).

¹H NMR (400 MHz, DMSO-*d*₆) δ 4.74 - 4.68 (m, 1H), 4.22 - 4.17 (m, 1H), 4.15 - 4.11 (m, 1H), 3.73 - 3.66 (m, 1H), 3.47 - 3.40 (m, 1H), 2.28 - 2.22 (m, 2H), 1.74 - 1.57 (m, 2H), 1.54 - 1.39 (m, 4H), 0.95 - 0.84 (m, 1H), 0.47 - 0.38 (m, 2H), 0.20 - 0.12 (m, 2H).

### Step 2: Synthesis of 4-(cyclopropylmethyl)-1-(4-(difluoromethyl)phenyl)-5-(((tetrahydro-2H-pyran-2-yl)oxy)methyl)-1H-1,2,3-triazole (186-3)

The experimental operation was as described in step 2 in the synthesis of intermediate **H5,** using compounds **186-2** and **H2** as raw materials to obtain the title product.

MS (ESI) m/z [M+H]⁺ = 364.2.

### Step 3: Synthesis of (4-(cyclopropylmethyl)-1-(4-(difluoromethyl)phenyl)-1H-1,2,3-triazol-5-yl)methanol (186-4)

Compound **186-3** (1.12 g, 3.08 mmol) was dissolved in hydrochloric acid solution (10 mL, 3 M aqueous solution), and the reaction mixture was stirred at room temperature for 2 hours. The completion of the reaction was monitored by LCMS. The reaction mixture was added with sodium bicarbonate solution to adjust the pH to 7, diluted with water (30 mL), and then extracted with dichloromethane (containing 10% methanol) (30 mL × 3). The organic phases were combined, washed with saturated brine (30 mL), dried over anhydrous sodium sulfate, filtered, and subjected to rotary evaporation. The residue was purified by flash silica gel column chromatography (dichloromethane: methanol = 10:1) to obtain the title product (620 mg, yellow solid).

MS (ESI) m/z [M+H]⁺ = 280.1.

### Step 4: Synthesis of 3-chloro-6-((4-(cyclopropylmethyl)-1-(4-(difluoromethyl)phenyl)-1H-1,2,3-triazol-5-yl)methoxy)pyridazine (186-5)

The experimental operation was as described in step 5 in the synthesis of intermediate **A8,** using compound **186-4** and 3,6-dichloropyridazine as raw materials to obtain the title product.

MS (ESI) m/z [M+H]⁺ = 392.1

### Step 5: Synthesis of 4-(6-((4-cyclopropylmethyl)-1-(4-(difluoromethyl)phenyl)-1H-1,2,3-triazol-5-yl)methoxy)pyridazin-3-yl)piperazin-2-one (186)

The experimental operation was as described in Example **1,** using compounds **186-5** and **A8** as raw materials to obtain the title product.

MS (ESI) m/z [M+H]⁺ = 456.2.

¹H NMR (400 MHz, DMSO-*d*₆) δ 8.08 (s, 1H), 7.89 - 7.73 (m, 4H), 7.40 (d, *J* = 9.6 Hz, 1H), 7.14 (t, *J* = 55.6 Hz, 1H), 7.04 (d, *J* = 9.6 Hz, 1H), 5.45 (s, 2H), 3.97 (s, 2H), 3.74 - 3.55 (m, 2H), 3.30 - 3.22 (m, 2H), 2.73 (d, *J* = 6.8 Hz, 2H), 1.15 - 0.95 (m, 1H), 0.51 - 0.33 (m, 2H), 0.28 - 0.17 (m, 2H).

### Example 187

### (S)-8-(6-((4-(Cyclopropylmethyl)-1-(4-(difluoromethyl)phenyl)-1H-1,2,3-triazol-5-yl)methoxy)pyridazin-3-yl)hexahydro-2H-pyrazino[1,2-a]pyrazin-1(6H)-one (187)

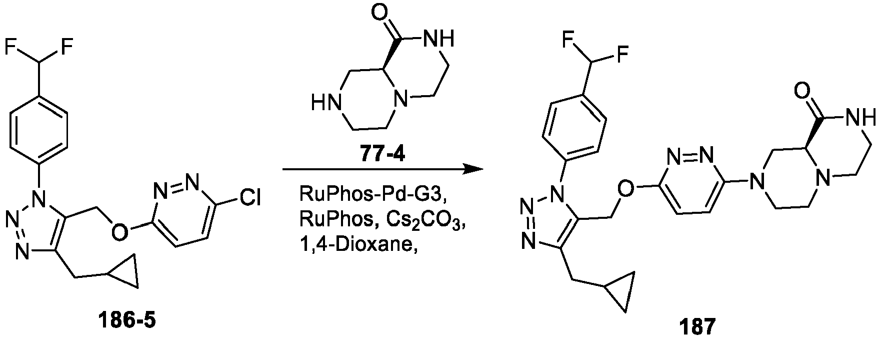

The experimental operation was as described in Example **1,** using compounds **186-5** and **77-4** as raw materials to obtain the title product.

MS (ESI) m/z [M+H]⁺ = 511.2.

¹H NMR (400 MHz, DMSO-*d*₆) δ 7.62 - 7.47 (m, 5H), 7.18 (d, *J* = 9.6 Hz, 1H), 6.91 (t, *J* = 55.6 Hz, 1H), 6.77 (d, *J* = 9.6 Hz, 1H), 5.23 (s, 2H), 4.27 - 4.15 (m, 1H), 3.79 (d, *J* = 12.0 Hz, 1H), 3.08 - 3.03 (m, 1H), 2.91 - 2.79 (m, 1H), 2.76 - 2.59 (m, 3H), 2.50 (d, *J* = 6.8 Hz, 2H), 2.46 - 2.35 (m, 2H), 2.23 - 2.13 (m, 1H), 2.11 - 1.96 (m, 1H), 0.96 - 0.75 (m, 1H), 0.29 - 0.15 (m, 2H), 0.04 - 0.07 (m, 2H).

### Example 188

### 4-(6-((1-(4-(Difluoromethyl)phenyl)-4-(5-methylisoxazol-3-yl)-1H-1,2,3-triazol-5-yl)methoxy)pyridazin-3-yl)piperazin-2-one (188)

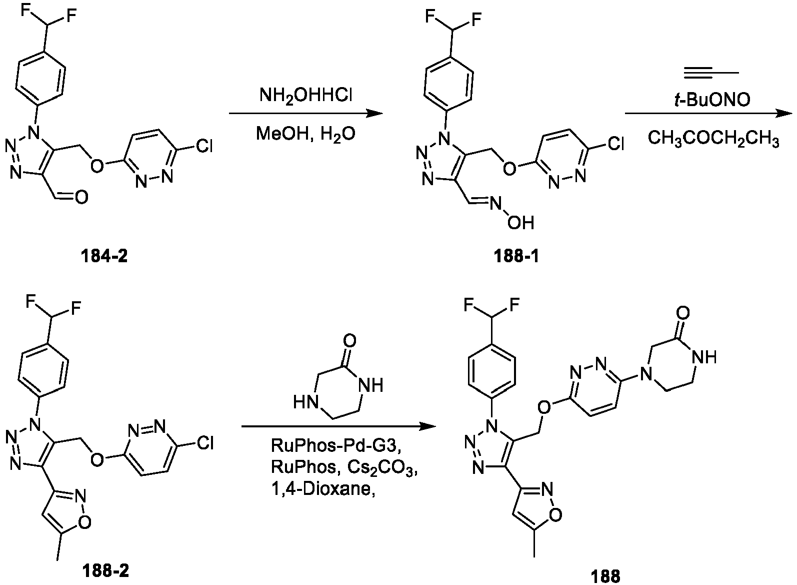

### Step 1: Synthesis of (E)-5-(((6-chloropyridazin-3-yl)oxy)methyl)-1-(4-(difluoromethyl)phenyl)-1H-1,2,3-triazole-4-carbaldehyde oxime (188-1)

Hydroxylamine hydrochloride (46 mg, 0.656 mmol) and sodium carbonate (70 mg, 0.656 mmol) were added to anhydrous methanol (2 mL) and water (2 mL) at room temperature, and reacted at 24°C for 10 minutes. Then compound **184-2** (200 mg, 0.547 mmol) was added thereto, and the reaction mixture was reacted at 24°C for 1 hour. LCMS showed that the reaction was completed. The reaction mixture was diluted with water (20 mL), extracted with ethyl acetate (40 mL × 3), and the organic phase was dried over anhydrous sodium sulfate, filtered, and concentrated under reduced pressure to obtain the title product (280 mg, yellow oil).

MS (ESI) m/z [M+H]⁺ = 380.9.

### Step 2: Synthesis of 3-(5-(((6-chloropyridazin-3-yl)oxy)methyl)-1-(4-(difluoromethyl)phenyl)-1H-1,2,3-triazol-4-yl)-5-methylisoxazole (188-2)

Compound **188-1** (300 mg, 0.788 mmol), propyne (0.9 mL, 0.900 mmol, 1.0 M tetrahydrofuran solution), and tert-butyl nitrite (102 mg, 0.867 mmol) were sequentially added to 2-butanone (5.00 mL) at room temperature. The system was replaced with nitrogen three times, and the reaction was carried out at 70°C for 15 hours. Product generation was shown by LCMS. The reaction mixture was concentrated under reduced pressure, and the crude product was purified by silica gel column chromatography (petroleum ether: ethyl acetate = 1:0 to 2:1) to obtain the title product (300 mg, yellow oil).

MS (ESI) m/z [M+H]⁺ = 419.0.

### Step 3: Synthesis of 4-(6-((1-(4-(difluoromethyl)phenyl)-4-(5-methylisoxazol-3-yl)-1H-1,2,3-triazol-5-yl)methoxy)pyridazin-3-yl)piperazin-2-one (188)

The experimental operation was as described in Example **1,** using compound **188-2** and 2-piperazinone as raw materials to obtain the title product.

MS (ESI) m/z [M+H]⁺ = 483.0.

¹H NMR: (400 MHz, CD₃OD) *δ* 7.81 (d, *J* = 8.8 Hz, 2H), 7.76 (d, *J* = 8.8 Hz, 2H), 7.31 (d, *J* = 9.6 Hz, 1H), 6.92 (d, *J* = 9.6 Hz, 1H), 6.89(t, *J* = 55.6 Hz, 1H), 6.70 (s, 1H), 5.78 (s, 2H), 4.11 (s, 2H), 3.77 - 3.72 (m, 2H), 3.48 - 3.43 (m, 2H), 2.52 (s, 3H).

### Example 189

### 4-(6-((4-Chloro-1-(4-fluorophenyl)-1H-1,2,3-triazol-5-yl)methoxy)pyridazin-3-yl)piperazin-2-one (189)

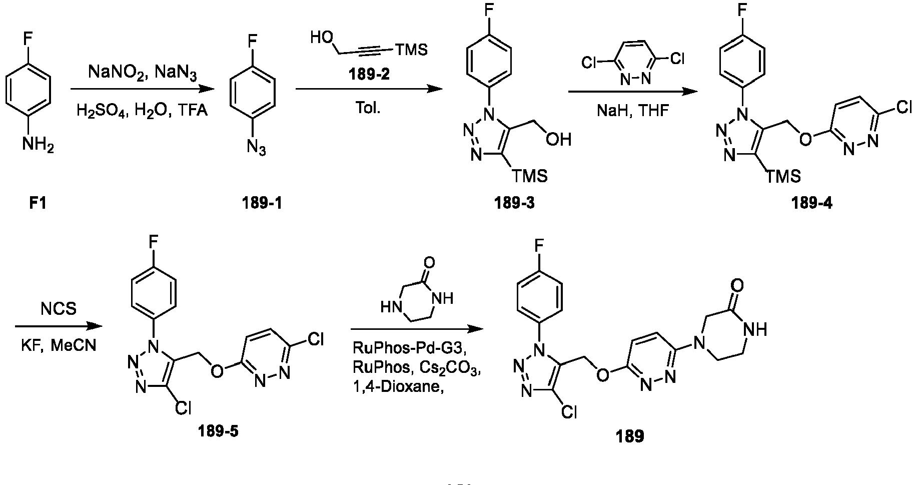

### Step 1: Synthesis of 1-azido-4-fluorobenzene (189-1)

Compound **F1** (10.0 g, 90.0 mmol) was slowly added to a mixed solvent of sulfuric acid (10 mL, 98%) and trifluoroacetic acid (50 mL) at 0°C, then sodium nitrite (8.07 g, 117 mmol) aqueous solution (50 mL) was slowly added dropwise to the above mixture, and the reaction was carried out at 0°C for 0.5 hours. Sodium azide (7.02 g, 108 mmol) aqueous solution (20 mL) was slowly added dropwise to the above mixture at 0°C, and the reaction mixture was stirred at room temperature for another 2 hours. TLC showed that the reaction was completed. 15% sodium hydroxide aqueous solution was slowly added dropwise to the reaction mixture at 0°C until the pH of the reaction mixture was greater than 9, and the mixture was extracted with ethyl acetate (200 mL × 3). The organic phases were combined and concentrated under reduced pressure to obtain the title product (12.5 g, brown liquid, crude product).

¹H NMR (400 MHz, CDsOD) *δ* 7.12 - 7.03 (m, 4H).

### Step 2: Synthesis of (1-(4-fluorophenyl)-4-(trimethylsilyl)-1H-1,2,3-triazol-5-yl)methanol (189-3)

The experimental operation was as described in step 2 in the synthesis of intermediate **H5,** using compounds **189-1** and **189-2** as raw materials to obtain the title product.

MS (ESI) m/z [M+H]⁺ = 266.1.

### Step 3: Synthesis of 3-chloro-6-((1-(4-fluorophenyl)-4-(trimethylsilyl)-1H-1,2,3-triazol-5-yl)methoxy)pyridazine (189-4)

The experimental operation was as described in step 6 in the synthesis of intermediate **A8,** using compound **189-3** and 3,6-dichloropyridazine as raw materials to obtain the title product.

MS (ESI) m/z [M+H]⁺ = 378.0.

¹H NMR: (400 MHz, CDCl₃) *δ* 7.52 - 7.49 (m, 2H), 7.43 (d, *J* = 9.6 Hz, 1H), 7.22 - 7.18 (m, 2H), 6.96 (d, *J* = 9.6 Hz, 1H), 5.51 (s, 2H), 0.39 (s, 9H).

### Step 4: Synthesis of 3-chloro-6-((4-chloro-1-(4-fluorophenyl)-1H-1,2,3-triazol-5-yl)methoxy)pyridazine (189-5)

Compound **189-4** (100 mg, 0.265 mmol) was dissolved in acetonitrile (2.0 mL) at room temperature, then potassium fluoride (94.5 mg, 1.59 mmol) and NCS (353 mg, 2.64 mmol) were sequentially added thereto, and the reaction mixture was stirred at 80°C for 16 hours. TLC showed that the reaction was completed. The reaction mixture was filtered directly, the filter cake was washed with acetonitrile (2.0 mL), and the filtrates were combined and subjected to rotary evaporation until dryness. The crude product was purified by flash silica gel column chromatography (ethyl acetate: petroleum ether = 1:4) to obtain the title product (130 mg, white solid).

MS (ESI) m/z [M+H]⁺ = 340.0.

¹H NMR (400 MHz, CDCl₃) *δ* 7.58 - 7.54 (m, 2H), 7.44 (d, *J* = 9.6 Hz, 1H), 7.25 - 7.23 (m, 2H), 6.99 (d, *J* = 9.6 Hz, 1H), 5.54 (s, 2H).

### Step 5: Synthesis of 4-(6-((4-chloro-1-(4-fluorophenyl)-1H-1,2,3-triazol-5-yl)methoxy)pyridazin-3-yl)piperazin-2-one (189)

The experimental operation was as described in Example **1,** using compound **189-5** and 2-piperazinone as raw materials to obtain the title product.

MS (ESI) m/z [M+H]⁺ = 404.1.

¹H NMR (400 MHz, CDsOD) *δ* 7.70 - 7.67 (m, 2H), 7.37 - 7.32 (m, 3H), 7.02 (d, *J* = 9.6 Hz, 2H), 5.47 (s, 2H), 4.13 (s, 2H), 3.76 (t, *J* = 5.2 Hz, 2H), 3.45 (t, *J* = 5.2 Hz, 2H).

### Example 190

### 4-(6-((4-Chloro-1-(4-chlorophenyl)-1H-1,2,3-triazol-5-yl)methoxy)pyridazin-3-yl)piperazin-2-one (190)

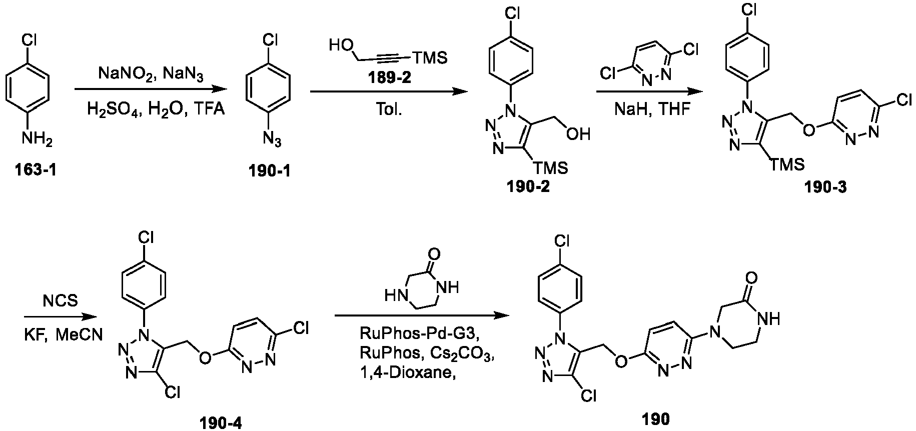

The experimental operation was as described in Example **189,** using compound **163-1** as the raw material to obtain the title product.

MS (ESI) m/z [M+H]⁺ = 420.0.

¹H NMR (400 MHz, CD₃OD) δ 7.68 - 7.57 (m, 4H), 7.35 (d, *J* = 9.6 Hz, 1H), 7.01 (d, *J* = 9.6 Hz, 1H), 5.49 (s, 2H), 4.13 (s, 2H), 3.79 - 3.72 (m, 2H), 3.48 - 3.43 (m, 2H).

### Example 191

### 4-(6-((1-(4-(Difluoromethyl)phenyl)-4-(trifluoromethyl)-1H-1,2,3-triazol-5-yl)methoxy)pyridazin-3-yl)piperazin-2-one (191)

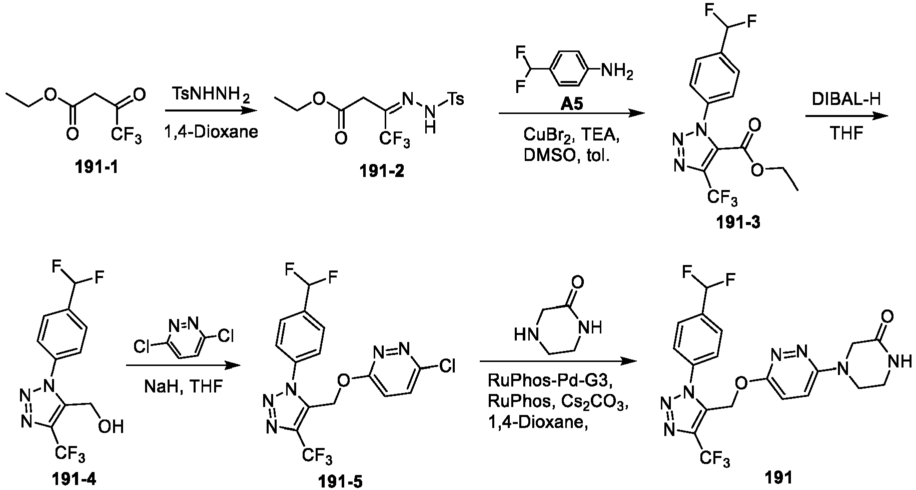

### Step 1: Synthesis of ethyl (Z/E)-4,4,4-trifluoro-3-(2-tosylhydrazono)butanoate (191-2)

**191-1** (10 g, 54.3 mmol) was added to ethanol (100 mL), then *p-*toluenesulfonylhydrazide (10.1 g, 54.3 mmol) was added thereto, and the reaction mixture was reacted at 80°C for 16 hours. The reaction mixture was filtered, and the filter cake was dried under reduced pressure to obtain the title product (10 g, yellow solid).

### Step 2: Synthesis of ethyl 1-(4-(difluoromethyl)phenyl)-4-(trifluoromethyl)-1H-1,2,3-triazole-5-carboxylate (191-3)

Compound **A5** (500 mg, 2.77 mmol, hydrochloride) and compound **191-2** (975 mg, 2.77 mmol) were added to toluene (20 mL), then TEA (839 mg, 8.31 mmol), copper (II) bromide (183 mg, 0.83 mmol), and DMSO (65 mg, 0.83 mmol) were sequentially added thereto, and the reaction was carried out at 100°C for 2 hours. The reaction mixture was concentrated under reduced pressure, and the residue was purified by flash silica gel column chromatography (petroleum ether: ethyl acetate = 1:1) to obtain the title product (30 mg, yellow solid).

MS (ESI) m/z [M+H]⁺ = 336.1.

### Steps 3 to 5: Synthesis of 4-(6-((1-(4-(difluoromethyl)phenyl)-4-(trifluoromethyl)-1H-1,2,3-triazol-5-yl)methoxy)pyridazin-3-yl)piperazin-2-one (191)

The experimental operation was as described in steps 4 to 5 in the synthesis of intermediate **A8** and Example **1,** using compound **191-3** as the raw material to obtain the title product.

MS (ESI) m/z [M+H]⁺ = 470.1.

¹H NMR (400 MHz, CDsOD) δ 7.81 (s, 4H), 7.38 (d, *J* = 9.6 Hz, 1H), 7.02 (d, *J* = 9.6 Hz, 1H), 7.01 (t, *J* = 55.6 Hz, 1H), 5.59 (s, 2H), 4.13 (s, 2H), 3.80 - 3.71 (m, 2H), 3.50 - 3.42 (m, 2H).

### Example 192

### (S)-8-(6-((1-(4-(Difluoromethyl)phenyl)-4-methyl-1H-1,2,3-triazol-5-yl)methoxy)pyridazin-3-yl)-7,8,9,10-tetrahydro-5H-pyrazino[1,2-a]pyrido[3,4-e]pyrazin-6(6aH)-one (192)

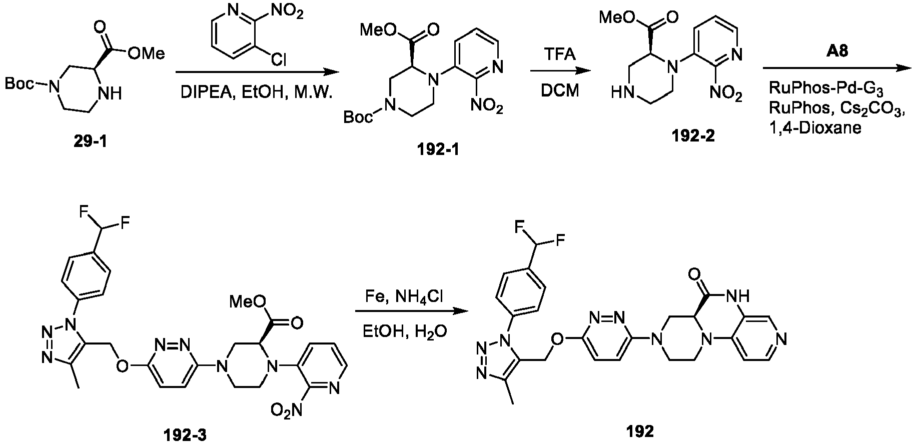

### Step 1: Synthesis of (S)-4-(tert-butoxycarbonyl)-1-(2-nitropyridin-3-yl)piperazine-2-carboxylic acid (192-1)

Compound **29-1** (2 g, 8.19 mmol) was dissolved in ethanol (16 mL), then 4-chloro-3-nitropyridine (1.56 g, 9.83 mmol) and DIEA (3.18 g, 24.6 mmol) were added thereto under a nitrogen atmosphere, and the reaction mixture was stirred at 90°C for 3 hours under microwave irradiation. Product generation was shown by LCMS. The reaction mixture was concentrated under reduced pressure, and the residue was purified by flash silica gel column chromatography (dichloromethane: methanol = 15:1) to obtain the title product (650 mg, yellow oil).

MS (ESI) m/z [M+H]⁺ = 367.2.

### Step 2: Synthesis of methyl (S)-1-(2-nitropyridin-3-yl)piperazine-2-carboxylate (192-2)

The experimental operation was as described in step 2 in Example **27,** using compound 192-1 as the raw material to obtain the title product.

MS (ESI) m/z [M+H]⁺ = 267.1.

### Step 3: Synthesis of methyl (S)-4-(6-((1-(4-(difluoromethyl)phenyl)-4-methyl-1H-1,2,3-triazol-5-yl)methoxy)pyridazin-3-yl)-1-(2-nitropyridin-3-yl)piperazine-2-carboxylate (192-3)

The experimental operation was as described in Example **1,** using compounds **192-2** and **A8** as raw materials to obtain the title product.

MS (ESI) m/z [M+H]⁺ = 582.2.

### Step 4: Synthesis of (S)-8-(6-((1-(4-(difluoromethyl)phenyl)-4-methyl-1H-1,2,3-triazol-5-yl)methoxy)pyridazin-3-yl)-7,8,9,10-tetrahydro-5H-pyrazino[1,2-a]pyrido[3,4-e]pyrazin-6(6aH)-one (192)

**192-2** (40 mg, 0.069 mmol) was dissolved in ethanol (0.6 mL) and water (0.2 mL), then iron powder (11.6 mg, 0.21 mmol) and ammonium chloride (3.69 mg, 0.069 mmol) were added thereto, and the reaction mixture was stirred at 70°C for 2 hours. LCMS showed that the reaction was completed. The reaction mixture was diluted with ethyl acetate (30 mL) and washed with saturated sodium chloride solution (3 × 20 mL). The organic phases were combined, dried over anhydrous ammonium sulfate, filtered, and the solvent was removed under reduced pressure. The residue was purified by flash silica gel column chromatography (dichloromethane: methanol = 15:1), and the obtained crude product was further purified by preparative thin-layer chromatography (petroleum ether: ethyl acetate = 0:1) to obtain the title product (2 mg, white solid).

MS (ESI) m/z [M+H]⁺ = 520.2.

¹H NMR (400 MHz, CDCl₃) δ 8.11 (br. s, 1H), 7.84 (s, 1H), 7.72 - 7.66 (m, 4H), 7.21 (m, *J* = 9.6 Hz, 1H), 7.00 - 6.91 (m, 2H), 6.89 (d, *J* = 9.6 Hz, 1H), 6.67 (t, *J* = 55.6 Hz, 1H), 5.47 (s, 2H), 4.55 - 4.50 (m, 1H), 4.46 - 4.40 (m, 2H), 4.05 - 4.16 (m, 1H), 3.11 - 3.02 (m, 2H), 3.01 - 2.94 (m, 1H), 2.50 (s, 3H).

### Example 193

### (S)-3-(6-((1-(4-(Difluoromethyl)phenyl)-4-methyl-1H-1,2,3-triazol-5-yl)methoxy)pyridazin-3-yl))-6-(4-methoxybenzyl)-2,3,4,4a-tetrahydro-1H-pyrazino[1,2-a]pyrido[2,3-e]pyrazin-5(6H)-one (193)

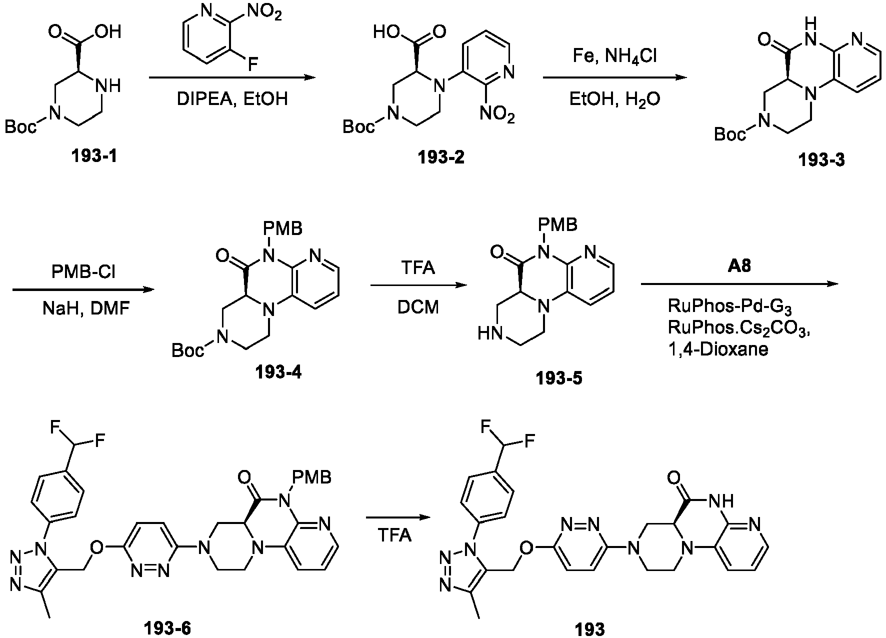

### Step 1: Synthesis of (S)-4-(tert-butoxycarbonyl)-1-(2-nitropyridin-3-yl)piperazine-2-carboxylic acid (193-2)

The experimental operation was as described in step 1 in Example **192,** using compound **193-1** and 3-fluoro-2-nitropyridine as raw materials to obtain the title product.

MS (ESI) m/z [M+H]⁺ = 353.1.

### Step 2: Synthesis of tert-butyl (S)-5-oxo-1,2,4,4a,5,6-hexahydro-3H-pyrazino[1,2-a]pyrido[2,3-e]pyrazine-3-carboxylate (193-3)

The experimental operation was as described in step 4 in Example **192,** using compound **193-2** as the raw material to obtain the title product.

MS (ESI) m/z [M+H]⁺ = 305.1.

### Step 3: Synthesis of tert-butyl (S)-6-(4-methoxybenzyl)-5-oxo-1,2,4,4a,5,6-hexahydro-3H-pyrazino[1,2-a]pyrido[2,3-e]pyrazine-3-carboxylate (193-4)

The experimental operation was as described in step 2 in Example **116,** using compound **193-3** and 4-methoxybenzyl chloride as raw materials to obtain the title product.

MS (ESI) m/z [M+H]⁺ = 425.2.

### Step 4: Synthesis of (S)-6-(4-methoxybenzyl)-2,3,4,4a-tetrahydro-1H-pyrazino[1,2-a]pyrido[2,3-e]pyrazin-5(6H)-one (193-5)

The experimental operation was as described in step 2 in Example **27,** using compound **193-4** as the raw material to obtain the title product.

MS (ESI) m/z [M+H]⁺ = 325.2.

### Step 5: Synthesis of (S)-3-(6-((1-(4-(difluoromethyl)phenyl)-4-methyl-1H-1,2,3-triazol-5-yl)methoxy)pyridazin-3-yl)-6-(4-methoxybenzyl)-2,3,4,4a-tetrahydro-1H-pyrazino[1,2-a]pyrido[2,3-e]pyrazin-5(6H)-one (193-6)

The experimental operation was as described in Example 1, using compounds **193-5** and **A8** as raw materials to obtain the title product.

MS (ESI) m/z [M+H]⁺ = 640.3.

### Step 6: Synthesis of (S)-3-(6-((1-(4-(difluoromethyl)phenyl)-4-methyl-1H-1,2,3-triazol-5-yl)methoxy)pyridazin-3-yl)-2,3,4,4a-tetrahydro-1H-pyrazino[1,2-a]pyrido[2,3-e]pyrazin-5(6H)-one (193)

**193-6** (660 mg, 0.88 mmol) was dissolved in TFA (7 mL), and the reaction mixture was stirred at 100°C for 5 hours under a nitrogen atmosphere. LCMS showed that the reaction was completed. The reaction mixture was slowly added with saturated sodium bicarbonate solution to adjust the pH to 8-9, and extracted with dichloromethane (30 mL × 3). The organic phases were combined, washed with saturated brine (50 mL), dried over anhydrous sodium sulfate, filtered, and the solvent was removed under reduced pressure. The residue was purified by flash silica gel column chromatography (dichloromethane: methanol = 13:1), and the obtained crude product was further purified by reversed-phase C18 flash preparative chromatography (acetonitrile: water = 0 to 35%), and the eluent was collected to obtain (110 mg, white solid).

MS (ESI) m/z [M+H]⁺ = 520.2.

¹H NMR (400 MHz, DMSO-*d₆*) δ 7.78 (s, 4H), 7.72 (d, *J=* 4.8 Hz, 1H), 7.53 (d, *J* = 9.6 Hz, 1H), 7.21 (d, *J* = 8.0 Hz, 1H), 7.13 (t, *J* = 55.6 Hz, 1H), 7.04 (d, *J* = 9.6 Hz, 1H), 6.96 (dd, *J=* 8.0, 4.8 Hz, 1H), 5.46 (s, 2H), 4.81 - 4.44 (m, 1H), 4.35 - 4.15 (m, 1H), 3.82 - 3.57 (m, 2H), 3.05 - 2.87 (m, 2H), 2.85 - 2.67 (m, 1H), 2.40 (s, 3H).

### Example 194

### (S)-8-(6-((1-(4-(Difluoromethyl)phenyl)-4-methyl-1H-1,2,3-triazol-5-yl)methoxy)pyridazin-3-yl)-7,8,9,10-tetrahydro-5H-pyrazino[1,2-a]pyrido[3,2-e]pyrazin-6(6aH)-one (194)

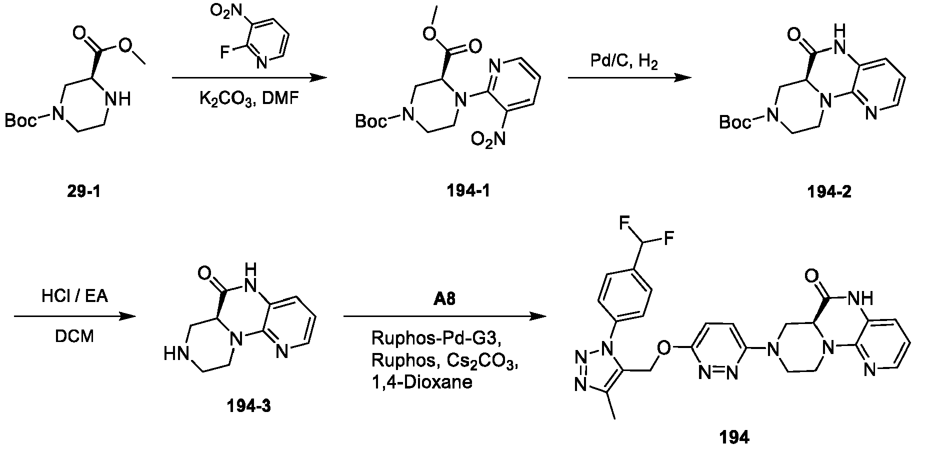

### Step: Synthesis of 1-(tert-butyl) 3-methyl (S)-4-(3-nitropyridin-2-yl)piperazine-1,3-dicarboxylate (194-1)

Compound **29-1** (3 g, 12.3 mmol), 2-fluoro-3-nitropyridine (1.74 g, 12.3 mmol), and potassium carbonate (5.09 g, 36.8 mmol) were mixed into DMF (20 mL), and the reaction mixture was stirred at 80°C for 24 hours. Product generation was shown by LCMS. The reaction mixture was diluted with water (30 mL) and extracted with ethyl acetate (50 mL × 3). The organic phase was dried over anhydrous sodium sulfate and filtered, and the filtrate was concentrated under reduced pressure. The residue was purified by flash silica gel column chromatography (petroleum ether: ethyl acetate = 20:1 to 5:1) to obtain the title product (1.2 g, yellow solid).

MS (ESI) m/z [M+H]⁺ = 311.0.

### Step 2: Synthesis of tert-butyl (S)-6-oxo-5,6,6a,7,9,10-hexahydro-8H-pyrazino[1,2-a]pyrido[3,2-e]pyrazine-8-carboxylate (194-2)

Compound **194-1** (500 mg, 1.37 mmol) was dissolved in ethanol (200 mL), then wet palladium on carbon (100 mg, purity: 10%) was added thereto under a nitrogen atmosphere, and the system was replaced with hydrogen three times. The reaction mixture was stirred at 50°C under a hydrogen atmosphere (15 psi) for 3 hours. Product generation was shown by LCMS. The reaction mixture was filtered, and the filtrate was concentrated to obtain the title product (400 mg, yellow oil).

MS (ESI) m/z [M+H]⁺ = 249.1.

### Step 3: Synthesis of (S)-7,8,9,10-tetrahydro-5H-pyrazino[1,2-a]pyrido[3,2-e]pyrazin-6(6aH)-one hydrochloride (194-3)

The experimental operation was as described in step 2 in Example **59,** using compound **194-2** as the raw material to obtain the title product.

### Step 4: Synthesis of (S)-8-(6-((1-(4-(difluoromethyl)phenyl)-4-methyl-1H-1,2,3-triazol-5-yl)methoxy)pyridazin-3-yl)-7,8,9,10-tetrahydro-5H-pyrazino[1,2-a]pyrido[3,2-e]pyrazin-6(6aH)-one (194)

The experimental operation was as described in Example **1,** using compounds **194-3** and **A8** as raw materials to obtain the title product.

MS (ESI) m/z [M+H]⁺ = 520.1.

¹H NMR (400 MHz, CDCl₃) δ 8.02 (br. s, 1H), 7.95 (s, 1H), 7.73 - 7.67 (m, 4H), 7.22 (m, *J* = 9.6 Hz, 1H), 6.90-7.00 (m, 2H), 6.91 (d, *J=* 9.6 Hz, 1H), 6.37 (t, *J=* 55.6 Hz, 1H), 5.48 (s, 2H), 4.58 - 4.20 (m, 2H), 4.50 - 4.40 (m, 1H), 4.05 - 4.16 (m, 1H), 3.15 - 3.05 (m, 2H), 3.04 - 2.96 (m, 1H), 2.50 (s, 3H).

### Example 195

### (S)-8-(6-((1-(4-(Difluoromethyl)phenyl)-4-methyl-1H-1,2,3-triazol-5-yl)methoxy)pyridazin-3-yl)-2-fluoro-7,8,9,10-tetrahydro-5H-pyrazino[1,2-a]pyrido[3,2-e]pyrazin-6(6aH)-one (195)

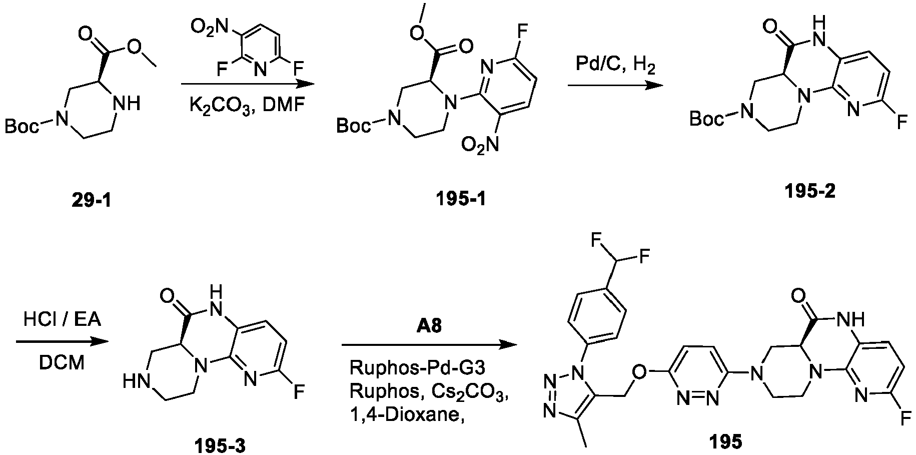

### Step 1: Synthesis of 1-(tert-butyl) 3-methyl (S)-4-(6-fluoro-3-nitropyridin-2-yl)piperazine-1,3-dicarboxylate (195-1)

The experimental operation was as described in step 1 in Example **194,** using compound **29-1** and 2,6-difluoro-3-nitropyridine as raw materials to obtain the title product.

MS (ESI) m/z [M+H-COO*^{t}*Bu]⁺ = 284.9.

¹H NMR (400 MHz, CD₃OD) δ 8.44 (dd, *J* = 8.0, 6.8 Hz, 1H), 6.55 (dd, *J* = 3.2, 11.2 Hz, 1H), 4.83 - 4.76 (m, 1H), 4.54 - 4.50 (m, 1H), 3.96 (s, 1H), 3.76 (s, 3H), 3.56 - 3.46 (m, 1H), 3.43 - 3.38 (m, 2H), 3.17 (s, 1H), 1.46 (s, 9H).

### Step 2: Synthesis of tert-butyl (S)-2-fluoro-6-oxo-5,6,6a,7,9,10-hexahydro-8H-pyrazino[1,2-a]pyrido[3,2-e]pyrazine-8-carboxylate (195-2)

The experimental operation was as described in step 2 in Example **58,** using compound 195-1 as the raw material to obtain the title product.

MS (ESI) m/z [M+H-*t*-Bu]⁺ = 266.9.

¹H NMR (400 MHz, CD₃OD) δ 7.18 - 7.07 (m, 1H), 6.28 (dd, *J* = 2.8, 8.0 Hz, 1H), 4.56 - 4.52 (m, 1H), 4.39 - 4.35 (m, 1H), 4.16 - 4.12 (m, 1H), 3.93 - 3.89 (m, 1H), 2.93 (s, 2H), 2.76 - 2.68 (m, 1H), 1.49 (s, 9H).

### Step 3: Synthesis of (S)-2-fluoro-7,8,9,10-tetrahydro-5H-pyrazino[1,2-a]pyrido[3,2-e]pyrazin-6(6aH)-one hydrochloride (195-3)

The experimental operation was as described in step 2 in Example **59,** using compound **195-2** as the raw material to obtain the title product.

### Step 4: Synthesis of (S)-8-(6-((1-(4-(difluoromethyl)phenyl)-4-methyl-1H-1,2,3-triazol-5-yl)methoxy)pyridazin-3-yl)-2-fluoro-7,8,9,10-tetrahydro-5H-pyrazino[1,2-a]pyrido[3,2-e]pyrazin-6(6aH)-one (195)

The experimental operation was as described in Example **1,** using compounds **195-3** and **A8** as raw materials to obtain the title product.

MS (ESI) m/z [M+H]⁺ = 538.1.

¹H NMR (400 MHz, CDsOD) δ 7.77 (s, 4H), 7.44 (d, *J* = 9.6 Hz, 1H), 7.15 (dd, *J* = 7.2, 8.0 Hz, 1H), 7.01 (d, *J* = 9.6 Hz, 1H), 6.89 (t, *J* = 55.6 Hz, 1H), 6.29 (dd, *J* = 2.4, 8.0 Hz, 1H), 5.51 (s, 2H), 4.68 - 4.62 (m, 1H), 4.47 - 4.42 (m, 1H), 4.26 - 4.20 (m, 1H), 4.12 - 4.08 (m, 1H), 3.08 - 2.86 (m, 3H), 2.48 (s, 3H)

### Example 196

### (S)-8-(6-((1-(4-(Difluoromethyl)phenyl)-4-methyl-1H-1,2,3-triazol-5-yl)methoxy)pyridazin-3-yl)-2-fluoro-7,8,9,10-tetrahydro-5H-pyrazino[1,2-a]pyrido[3,4-e]pyrazin-6(6aH)-one (196)

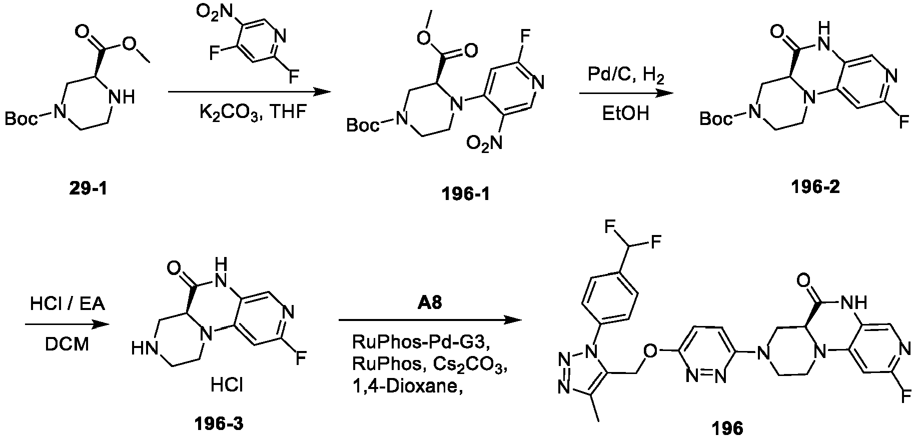

### Step 1: Synthesis of 1-(tert-butyl) 3-methyl (S)-4-(2-fluoro-5-nitropyridin-4-yl)piperazine-1,3-dicarboxylate (196-1)

Compound **29-1** (900 mg, 3.68 mmol) and potassium carbonate (764 mg, 5.53 mmol) were dissolved in THF (10 mL) at 0°C, and the reaction mixture was stirred for 0.5 hours. Then 2,4-difluoro-5-nitropyridine (1.35 g, 9.55 mmol) was added to the reaction, and stirred at 23°C for 12 hours. Product generation was shown by LCMS. The reaction mixture was added with water (10 mL) and extracted with ethyl acetate (20 mL × 3). The organic phase was dried over anhydrous sodium sulfate and filtered, and the filtrate was concentrated under reduced pressure. The residue was purified by flash silica gel column chromatography (ethyl acetate: petroleum ether = 1:2) to obtain the title product (820 mg, yellow oil).

MS (ESI) m/z [M+H]⁺ = 385.1.

### Step 2: Synthesis of tert-butyl (S)-2-fluoro-6-oxo-5,6,6a,7,9,10-hexahydro-8H-pyrazino[1,2-a]pyrido[3,4-e]pyrazine-8-carboxylate (196-2)

The experimental operation was as described in step 2 in Example **194,** using compound **196-1** as the raw material to obtain the title product.

MS (ESI) m/z [M+H]⁺ = 323.0.

### Step 3: Synthesis of (S)-2-fluoro-7,8,9,10-tetrahydro-5H-pyrazino[1,2-a]pyrido[3,4-e]pyrazin-6(6aH)-one hydrochloride (196-3)

The experimental operation was as described in step 2 in Example **59,** using compound **196-2** as the raw material to obtain the title product.

### Step 4: Synthesis of (S)-8-(6-((1-(4-(difluoromethyl)phenyl)-4-methyl-1H-1,2,3-triazol-5-yl)methoxy)pyridazin-3-yl)-2-fluoro-7,8,9,10-tetrahydro-5H-pyrazino[1,2-a]pyrido[3,4-e]pyrazin-6(6aH)-one (196)

The experimental operation was as described in Example **1,** using compounds **196-3** and **A8** as raw materials to obtain the title product.

MS (ESI) m/z [M+H]⁺ = 538.1.

¹H NMR (400 MHz, CDCl₃) δ 7.86 (br. s, 1H), 7.66 - 7.71 (m, 4H), 7.54 (s, 1H), 7.22 (d, *J* = 9.6 Hz, 1H), 6.94 (d, *J* = 9.6 Hz, 1H), 6.72 (t, *J* = 56.0 Hz, 1H), 6.30 (s, 1H), 5.48 (s, 2H), 4.60 - 4.49 (m, 2H), 4.00 - 3.90 (m, 1H), 3.80 - 3.70 (m, 1H), 3.20 - 3.10 (m, 3H), 2.51 (s, 3H).

### Example 197

### (4aS,6aS,9aR)-3-(6-((1-(4-(Difluoromethyl)phenyl)-4-methyl-1H-1,2,3-triazol-5-yl)methoxy)pyridazin-3-yl)decahydrocyclopenta[e]pyrazino[1,2-a]pyrazin-5(1H)-one (197)

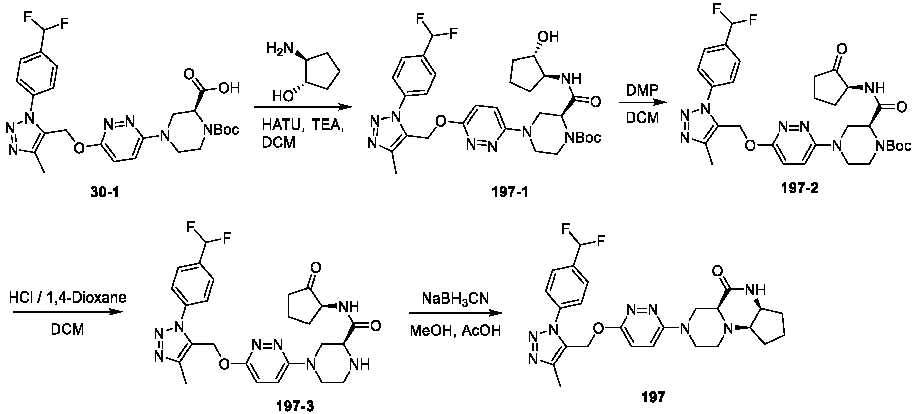

### Step 1: Synthesis of tert-butyl (S)-4-(6-((1-(4-(difluoromethyl)phenyl)-4-methyl-1H-1,2,3-triazol-5-yl)methoxy)pyridazin-3-yl)-2-(((1S,2S)-2-hydroxycyclopentyl)carbamoyl)piperazine-1-carboxylate (197-1)

The experimental operation was as described in Example **38,** using **30-1** and (1*S*,2*S*)-2-aminocyclopentan-1-ol as raw materials to obtain the title product.

MS (ESI) m/z [M+H]⁺ = 629.3.

### Step 2: Synthesis of tert-butyl (S)-4-(6-((1-(4-(difluoromethyl)phenyl)-4-methyl-1H-1,2,3-triazol-5-yl)methoxy)pyridazin-3-yl)-2-(((S)-2-oxocyclopentyl)carbamoyl)piperazine-1-carboxylate (197-2)

**197-1** (400 mg, 0.64 mmol) was dissolved in DCM (10 mL), then Dess-Martin periodinane (542.90 mg, 1.28 mmol) was added thereto under a nitrogen atmosphere, and the reaction mixture was stirred at room temperature for 2 hours. LCMS showed that the reaction was completed. The reaction mixture was filtered, and the filtrate was concentrated under reduced pressure. The residue was purified by flash silica gel column chromatography (dichloromethane: methanol = 19:1) to obtain the title product (350 mg, yellow solid).

MS (ESI) m/z [M+H]⁺ = 627.3.

### Step 3: Synthesis of (S)-4-(6-((1-(4-(difluoromethyl)phenyl)-4-methyl-1H-1,2,3-triazol-5-yl)methoxy)pyridazin-3-yl)-N-((S)-2-oxocyclopentyl)piperazine-2-carboxamide (197-3)

**197-2** (370 mg, 0.58 mmol) was dissolved in DCM (4 mL), then hydrogen chloride (42.3 mg, 1.16 mmol, 4.0 M dioxane solution) was added thereto under a nitrogen atmosphere, and the reaction mixture was stirred at room temperature for 2 hours. LCMS showed that the reaction was completed. The reaction mixture was concentrated under reduced pressure to obtain the title product (120 mg, crude hydrochloride).

MS (ESI) m/z [M+H]⁺ = 527.2.

### Step 4: Synthesis of (4aS,6aS,9aR)-3-(6-((1-(4-(difluoromethyl)phenyl)-4-methyl-1H-1,2,3-triazol-5-yl)methoxy)pyridazin-3-yl)decahydrocyclopenta[e]pyrazino[1,2-a]pyrazin-5(1H)-one (197)

**197-3** (100 mg, 0.19 mmol) was dissolved in methanol (1 mL), then sodium cyanoborohydride (23.9 mg, 0.38 mmol) and acetic acid (1.14 mg, 0.019 mmol) were added thereto under a nitrogen atmosphere, and the reaction mixture was stirred at 65°C for 2 hours. LCMS showed that the reaction was completed. The reaction mixture was diluted with ethyl acetate (50 mL) and washed with saturated brine (3 × 20 mL). The organic phase was dried over anhydrous sodium sulfate, filtered, and the solvent was removed under reduced pressure. The residue was purified by flash silica gel column chromatography (dichloromethane: methanol = 10:1) to obtain the title product (20 mg, white solid).

MS (ESI) m/z [M+H]⁺ = 511.2.

¹H NMR (400 MHz, DMSO-*d*₆) δ 8.03 (s, 1H), 7.78 (s, 4H), 7.38 (d, *J=* 9.6 Hz, 1H), 7.13 (t, *J* = 55.6 Hz, 1H), 6.98 (d, *J* = 9.6 Hz, 1H), 5.44 (s, 2H), 4.55 - 4.39 (m, 1H), 4.13 - 3.92 (m, 1H), 3.17 - 3.01 (m, 2H), 2.85 - 2.72 (m, 1H), 2.71 - 2.51 (m, 3H), 2.37-2.35 (m, 4H), 1.93 - 1.79 (m, 2H), 1.80 - 1.60 (m, 4H).

### Example 198

### Synthesis of (4aS, 6aR, 9aS)-3-(6-((1-(4-(difluoromethyl)phenyl)-4-methyl-1H-1,2,3-triazol-5-yl)methoxy)pyridazin-3-yl)decahydrocyclopenta[e]pyrazino[1,2-a]pyrazin-5(1H)-one (198)

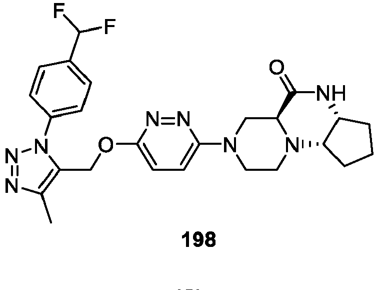

The experimental operation was as described in Example **197,** using **30-1** and (1*R*,2*R*)-2-aminocyclopentan-1-ol as raw materials to obtain the title product.

MS (ESI) m/z [M+H]⁺ = 511.2.

¹H NMR (400 MHz, DMSO-*d*₆) δ 8.03 (s, 1H), 7.78 (s, 4H), 7.38 (d, *J* = 9.6 Hz, 1H), 7.13 (t, *J=* 55.6 Hz, 1H), 6.99 (d, *J=* 9.6 Hz, 1H), 5.45 (s, 2H), 4.58 - 4.34 (m, 1H), 4.15 - 3.91 (m, 1H), 3.10 (m, 2H), 2.88 - 2.70 (m, 2H), 2.70 - 2.53 (m, 2H), 2.42 - 2.32 (m, 4H), 1.92 - 1.77 (m, 2H), 1.79 - 1.66 (m, 4H).

### Example 199

### (4aS)-3-(6-((1-(4-(Difluoromethyl)phenyl)-4-methyl-1H-1,2,3-triazol-5-yl)methoxy)pyridazin-3-yl)decahydro-1H-pyrazino[1,2-a]quinoxalin-5(6H)-one (199)

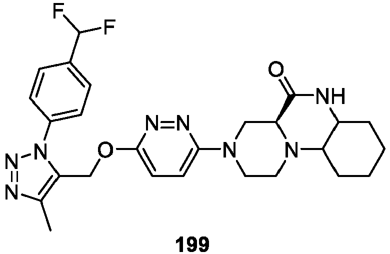

The experimental operation was as described in Example 197, using **30-1** and 2-aminocyclohexan-1-ol as raw materials to obtain the title product.

MS (ESI) m/z [M+H]⁺ = 525.2.

¹H NMR (400 MHz, DMSO-*d*₆) δ 7.91 (s, 1H), 7.79 (s, 4H), 7.39 (d, *J* = 9.6 Hz, 1H), 7.14 (t, *J* = 55.6 Hz, 1H), 7.00 (d, *J* = 9.6 Hz, 1H), 5.46 (s, 2H), 4.52 - 4.33 (m, 1H), 4.11 - 3.89 (m, 1H), 3.31 - 3.17 (m, 4H), 3.16 -3.04 (m, 2H), 2.89 - 2.70 (m, 2H), 2.70 - 2.55 (m, 2H), 2.32 - 2.40 (m, 4H), 2.10 - 1.94 (m, 1H), 1.86 - 1.66 (m, 1H), 1.51 - 1.28 (m, 1H), 1.21 - 0.98 (m, 1H).

### Example 200

### (9aS)-8-(6-((1-(4-(Difluoromethyl)phenyl)-4-methyl-1H-1,2,3-triazol-5-yl)methoxy)pyridazin-3-yl)-3-isopropylhexahydro-2H-pyrazino[1,2-a]pyrazin-1(6H)-one (200)

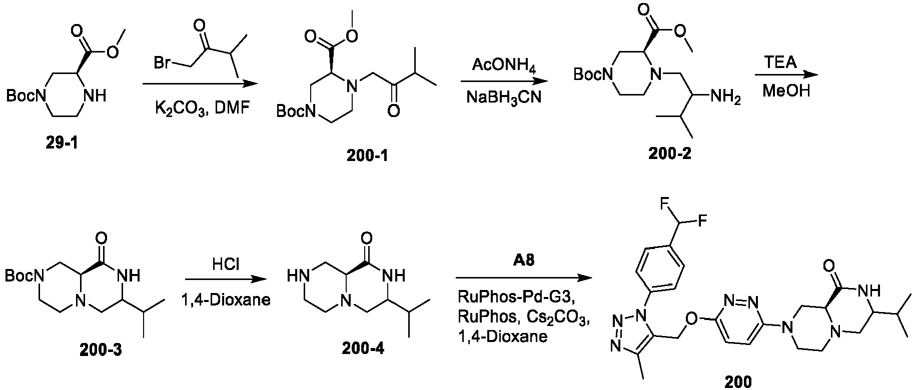

### Step 1: Synthesis of 1-(tert-butyl) 3-methyl (S)-4-(3-methyl-2-oxobutyl)piperazine-1,3-dicarboxylate (200-1)

The experimental operation was as described in step 1 in Example **195,** using compound **29-1** and 1-bromo-3-methylbutan-2-one as raw materials to obtain the title product.

MS (ESI) m/z [M+H]⁺ = 329.2.

### Step 2: Synthesis of 1-(tert-butyl) 3-methyl (3S)-4-(2-amino-3-methylbutyl)piperazine-1,3-dicarboxylate (200-2)

Ammonium acetate (61 mg, 3.05 mmol) and compound **200-1** (1 g, 3.05 mmol) were dissolved in methanol (30 mL) under a nitrogen atmosphere, then sodium cyanoborohydride (1.9 g, 31 mmol) was added thereto, and the reaction mixture was stirred at 25°C for 3 hours. The completion of the reaction was monitored by LCMS. The reaction mixture was quenched by adding water (80 mL), and extracted with EA (100 mL × 3). The organic phases were combined, washed with saturated brine (100 mL), dried over anhydrous sodium sulfate, and filtered, and the filtrate was concentrated under reduced pressure to obtain the title product (5 g, crude product).

MS (ESI) m/z [M+H]⁺ = 330.2.

### Step 3: Synthesis of tert-butyl (9aS)-7-isopropyl-9-oxooctahydro-2H-pyrazino[1,2-a]pyrazine-2-carboxylate (200-3)

Compound **200-2** (1.1 g, 3.34 mmol) and TEA (3.38 g, 33.4 mmol) were dissolved in methanol (30 mL) under a nitrogen atmosphere, and the reaction mixture was stirred at 60°C for 2 hours. The completion of the reaction was monitored by LCMS. The reaction mixture was concentrated under reduced pressure, and the residue was purified by flash silica gel column chromatography (petroleum ether: ethyl acetate = 1:1) to obtain the title product (150 mg, white solid).

MS (ESI) m/z [M+H]⁺ = 298.2.

### Step 4: Synthesis of (9aS)-3-isopropylhexahydro-2H-pyrazino[1,2-a]pyrazin-1(6H)-one (200-4)

The experimental operation was as described in step 3 in Example **197,** using compound **200-3** as the raw material to obtain the title product.

MS (ESI) m/z [M+H]⁺ = 198.2.

### Step 5: Synthesis of (9aS)-8-(6-((1-(4-(difluoromethyl)phenyl)-4-methyl-1H-1,2,3-triazol-5-yl)methoxy)pyridazin-3-yl)-3-isopropylhexahydro-2H-pyrazino[1,2-a]pyrazin-1(6H)-one (200)

The experimental operation was as described in Example **1,** using compounds **200-4** and **A8** as raw materials to obtain the title product.

MS (ESI) m/z [M+H]⁺ = 513.2.

¹H NMR (400 MHz, DMSO-*d*₆) δ 8.00 (s, 1H), 7.78 (s, 4H), 7.38 (d, *J* = 9.6 Hz, 1H), 7.20 (d, *J* = 55.6 Hz, 1H), 6.98 (d, *J* = 9.6 Hz, 1H), 5.44 (s, 2H), 4.55 - 4.31 (m, 1H), 4.13 - 3.90 (m, 1H), 3.41 - 3.36 (m, 1H), 2.96 - 2.73 (m, 4H), 2.60 - 2.51 (m, 1H), 2.46 - 2.33 (m, 4H), 2.27 - 2.10 (m, 1H), 1.98 - 1.73 (m, 1H), 0.89 - 0.87 (m, 6H).

### Example 201 and Example 202

### (9aS)-3-(tert-Butyl)-8-(6-((1-(4-(difluoromethyl)phenyl)-4-methyl-1H-1,2,3-triazol-5-yl)methoxy)pyridazin-3-yl)hexahydro-2H-pyrazino[1,2-a]pyrazin-1(6H)-one (201)

### (9aS)-3-(tert-Butyl)-8-(6-((1-(4-(difluoromethyl)phenyl)-4-methyl-1H-1,2,3-triazol-5-yl)methoxy)pyridazin-3-yl)hexahydro-2H-pyrazino[1,2-a]pyrazin-1(6H)-one (201)

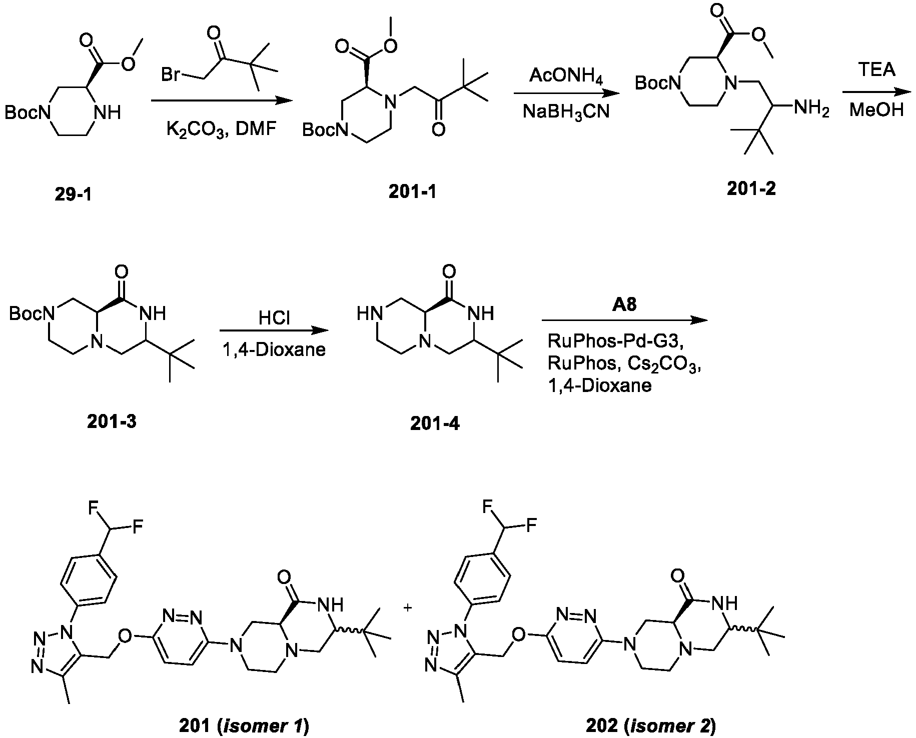

The experimental operation was as described in Example **200,** using **29-1** and 1-bromo-3,3-dimethylbutan-2-one as raw materials to obtain the title product.

### Compound 201:

MS (ESI) m/z [M+H]⁺ = 527.3,

HPLC analysis retention time Rt = 2.629 min. (HPLC analysis conditions: chromatographic column: Gemini C18 3 * 50 mm 3 µm; mobile phase: A: water (containing 0.04% TFA), B: acetonitrile (containing 0.02% TFA); gradient: 10% to 50% to 90% / 5 min; flow rate: 1.0 mL/min; column temperature: 35°C).

¹H NMR (400 MHz, DMSO-*d*₆) δ 7.89 (d, *J* = 3.4 Hz, 1H), 7.78 (s, 4H), 7.39 (d, *J* = 9.6 Hz, 1H), 7.14 (t, *J* = 55.6 Hz, 1H), 6.99 (d, *J* = 9.6 Hz, 1H), 5.45 (s, 2H), 4.52 - 4.37 (m, 1H), 4.02 (d, *J* = 12.0 Hz, 1H), 3.01 (d, *J* = 12.6 Hz, 1H), 2.95 - 2.85 (m, 2H), 2.85 - 2.75 (m, 1H), 2.61 - 2.53 (m, 2H), 2.42 - 2.36 (m, 4H), 2.21 - 2.08 (m, 1H), 0.92 (s, 9H).

### Compound 202:

MS (ESI) m/z [M+H]⁺ = 527.3,

HPLC analysis retention time Rt = 2.75 min. (HPLC analysis conditions: chromatographic column: Gemini C18 3 * 50 mm 3 µm; mobile phase: A: water (containing 0.04% TFA), B: acetonitrile (containing 0.02% TFA); gradient: 10% to 50% to 90% / 5 min; flow rate: 1.0 mL/min; column temperature: 35°C).

¹H NMR (400 MHz, DMSO-*d*₆) δ 7.79 (s, 4H), 7.52 (s, 1H), 7.41 (d, *J* = 9.6 Hz, 1H), 7.14 (t, *J* = 55.6 Hz, 1H), 7.00 (d, *J* = 9.6 Hz, 1H), 5.45 (s, 2H), 4.44 (d, *J=* 11.6 Hz, 1H), 4.02 (d, *J=* 11.6 Hz, 1H), 3.29 - 3.18 (m, 1H), 2.98 - 2.78 (m, 3H), 2.68 - 2.53 (m, 3H), 2.40 (s, 3H), 2.29 - 2.08 (m, 1H), 0.87 (s, 9H).

### Example 203 and Example 204

### (9aS)-3-Cyclopropyl-8-(6-((1-(4-(difluoromethyl)phenyl)-4-methyl-1H-1,2,3-triazol-5-yl)methoxy)pyridazin-3-yl)hexahydro-2H-pyrazino[1,2-a]pyrazin-1(6H)-one (203)

### (9aS)-3-Cyclopropyl-8-(6-((1-(4-(difluoromethyl)phenyl)-4-methyl-1H-1,2,3-triazol-5-yl)methoxy)pyridazin-3-yl)hexahydro-2H-pyrazino[1,2-a]pyrazin-1(6H)-one (204)

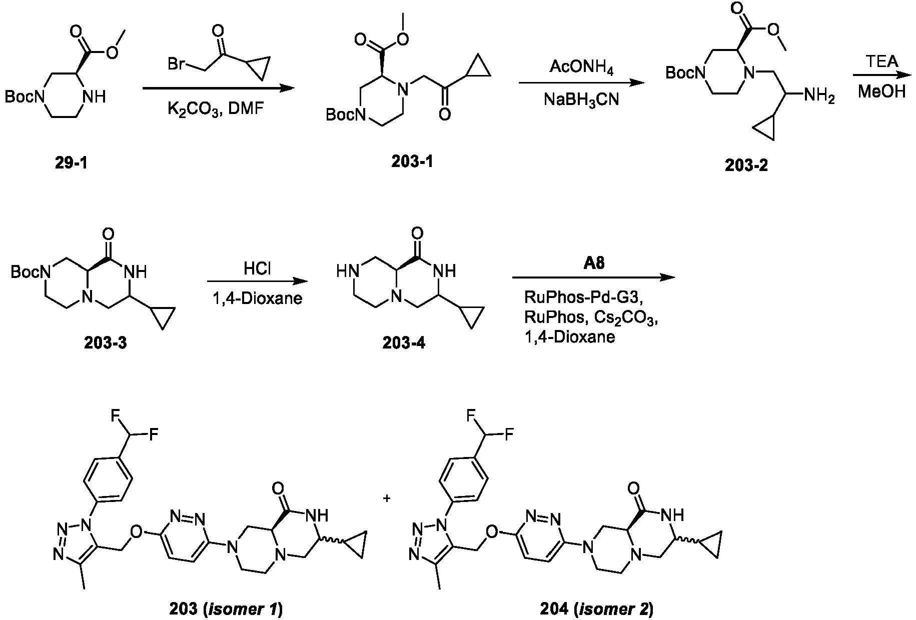

The experimental operation was as described in Example 200, using **29-1** and 2-bromo-1-cyclopropylethan-1-one as raw materials to obtain the title product.

### Compound 203:

MS (ESI) m/z [M+H]⁺ = 511.2,

HPLC analysis retention time Rt = 2.53 min. (HPLC analysis conditions: chromatographic column: Gemini C18 3 * 50 mm 3 µm; mobile phase: A: water (containing 0.04% TFA), B: acetonitrile (containing 0.02% TFA); gradient: 10% to 50% to 90% / 5 min; flow rate: 1.0 mL/min; column temperature: 35°C).

¹H NMR (400 MHz, DMSO-*d*₆) δ 8.04 (d, *J* = 3.4 Hz, 1H), 7.79 (s, 4H), 7.40 (d, *J* = 9.6 Hz, 1H), 7.13 (t, *J* = 55.6 Hz, 1H), 6.99 (d, *J* = 9.6 Hz, 1H), 5.45 (s, 2H), 4.48 (d, *J* = 11.6 Hz, 1H), 4.04 (d, *J* = 11.6 Hz, 1H), 3.29 - 3.19 (m, 1H), 3.00 - 2.77 (m, 3H), 2.64 - 2.51 (m, 3H), 2.39 (s, 3H), 2.32 - 2.15 (m, 1H), 1.34 - 1.17 (m, 1H), 0.50 - 0.40 (m, 1H), 0.39 - 0.28 (m, 2H), 0.24 - 0.10 (m, 1H).

### Compound 203:

MS (ESI) m/z [M+H]⁺ = 511.2,

HPLC analysis retention time Rt = 2.48 min. (HPLC analysis conditions: chromatographic column: Gemini C18 3 * 50 mm 3 µm; mobile phase: A: water (containing 0.04% TFA), B: acetonitrile (containing 0.02% TFA); gradient: 10% to 50% to 90% / 5 min; flow rate: 1.0 mL/min; column temperature: 35°C).

¹H NMR (400 MHz, DMSO-*d*₆) δ 7.88 (m, 1H), 7.79 (s, 4H), 7.40 (d, *J* = 9.6 Hz, 1H), 7.14 (t, *J* = 55.6 Hz, 2H), 7.00 (d, *J* = 9.6 Hz, 1H), 5.45 (s, 2H), 4.44 (d, *J* = 11.4 Hz, 1H), 4.00 (d, *J* = 11.4 Hz, 1H), 3.06 - 2.97 (m, 1H), 2.92 (m, 1H), 2.89 - 2.79 (m, 1H), 2.75 - 2.64 (m, 1H), 2.64 - 2.54 (m, 2H), 2.39 (s, 3H), 2.30 - 2.18 (m, 2H), 0.78 - 0.59 (m, 1H), 0.48 - 0.31 (m, 2H), 0.31 - 0.14 (m, 1H).

### Example 205

### (9aS)-3-(3-Chlorophenyl) -8-(6-((1-(4-(difluoromethyl)phenyl)-4-methyl-1H-1,2,3-triazol-5-yl)methoxy)pyridazin-3-yl)hexahydro-2H-pyrazino[1,2-a]pyrazin-1(6H)-one (205)

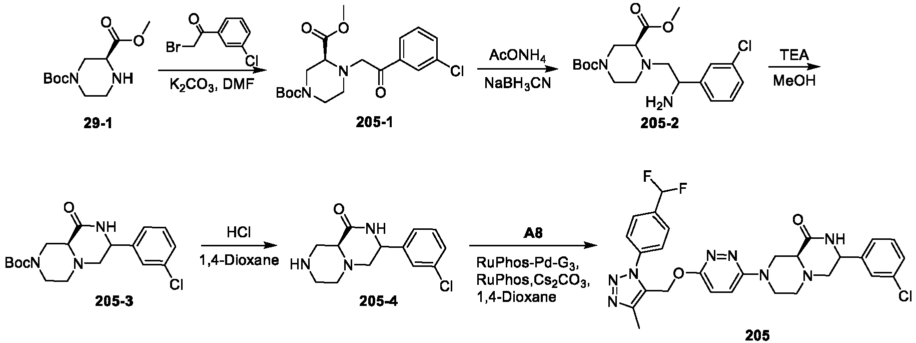

The experimental operation was as described in Example **200,** using **29-1** and 2-bromo-1-(3-chlorophenyl)ethan-1-one as raw materials to obtain the title product.

MS (ESI) m/z [M+H]⁺ = 581.2.

¹H NMR (400 MHz, CD₃OD) δ 7.78 (s, 4H), 7.47 - 7.32 (m, 5H), 7.01 (d, *J* = 9.6 Hz, 1H), 6.90 (t, *J=* 56.0 Hz, 1H), 5.52 (s, 2H), 4.58 - 4.52 (m, 2H), 4.14 - 4.03 (m, 1H), 3.22 - 2.89 (m, 4H), 2.83 - 2.77 (m, 1H), 2.56 - 2.36 (m, 5H).

### Example 206

### (S)-8-(5-((1-(4-(Difluoromethyl)phenyl)-4-methyl-1H-1,2,3-triazol-5-yl)methoxy)pyrido[2,3-d]pyridazin-8-yl)hexahydro-2H-pyrazino[1,2-a]pyrazin-1(6H)-one (206)

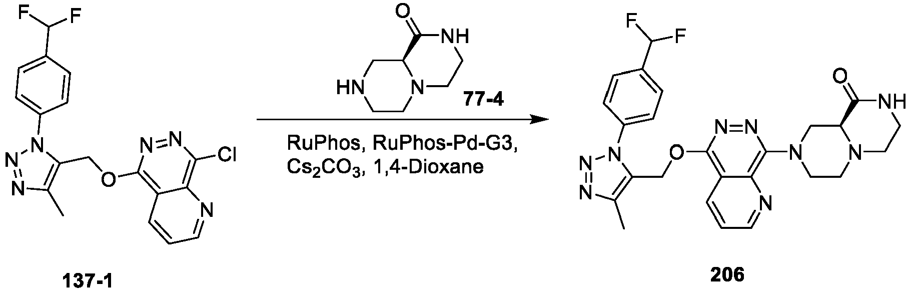

The experimental operation was as described in Example **1,** using compounds **137-1** and **77-4** as raw materials to obtain the title product.

MS (ESI) m/z [M+H]⁺ = 522.2.

¹H NMR (400 MHz, CDCl₃) δ 9.17 (d, *J* = 2.4 Hz, 1H), 8.20 (d, *J* = 6.8 Hz, 1H), 7.70 - 6.63 (m, 5H), 6.69 (t, *J=* 56.0 Hz, 1H), 5.96 (s, 1H), 5.66 (s, 2H), 5.25 - 5.21 (m, 1H), 4.41 - 3.38 (m, 1H), 3.68 - 3.65 (m, 1H), 3.44 - 3.41 (m, 1H), 3.33 - 3.00 (m, 5H), 2.79 - 2.70 (m, 2H), 2.53 (s, 3H).

### Example 207

### (9aS)-8-(6-(1-(4-(Difluoromethyl)phenyl)-4-methyl-1H-1,2,3-triazol-5-yl)methoxy)pyridazin-3-yl)-4-isopropylhexahydro-2H-pyrazino[1,2-a]pyrazin-1(6H)-one (207)

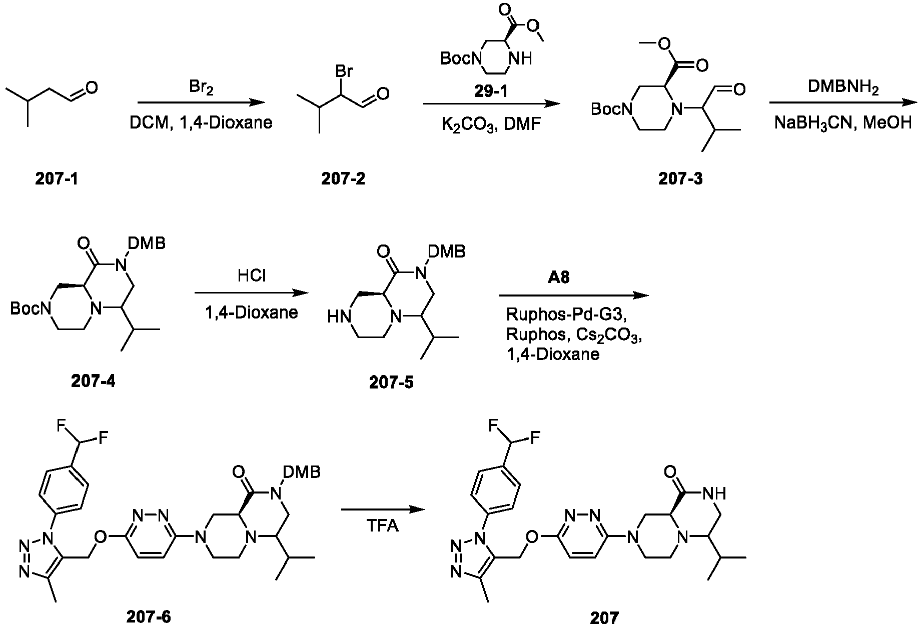

### Step 1: Synthesis of 2-bromo-3-methylbutanal (207-2)

Compound **207-1** (5 g, 58.05 mmol) was dissolved in dichloromethane (4 mL) and 1,4-dioxane (20 mL) under a nitrogen atmosphere, then liquid bromine (9.37 g, 58.6 mmol) was added thereto at 0°C, and the reaction was stirred at 0°C for 3 hours. The reaction mixture was used directly in the next step.

### Steps 2 to 4: Synthesis of (9aS)-2-(3,4-dimethylbenzyl)-4-isopropylhexahydro-2H-pyrazino[1,2-a]pyrazin-1(6H)-one (207-5)

The experimental operation was as described in steps 1 to 3 in Example 200, using compound **207-2** as the raw material to obtain the title product.

MS (ESI) m/z [M+H]⁺ = 316.2.

### Step 5: Synthesis of (9aS)-8-(6-(1-(4-(difluoromethyl)phenyl)-4-methyl-1H-1,2,3-triazol-5-yl)methoxy)pyridazin-3-yl)-2-(3,4-dimethylbenzyl)-4-isopropylhexahydro-2H-pyrazino[1,2-a]pyrazin-1(6H)-one (207-6)

The experimental operation was as described in Example **1,** using compounds **207-5** and **A8** as raw materials to obtain the title product.

MS (ESI) m/z [M+H]⁺ = 631.3.

### Step 6: Synthesis of (9aS)-8-(6-(1-(4-(difluoromethyl)phenyl)-4-methyl-1H-1,2,3-triazol-5-yl)methoxy)pyridazin-3-yl)-4-isopropylhexahydro-2H-pyrazino[1,2-a]pyrazin-1(6H)-one (207)

The experimental operation was as described in step 6 in Example **193,** using compound **207-6** as the raw material to obtain the title product.

MS (ESI) m/z [M+H]⁺ = 513.2.

¹H NMR (400 MHz, DMSO-*d*₆) δ 7.79 (s, 5H), 7.37 (d, *J* = 9.6 Hz, 1H), 7.14 (t, *J* = 55.6 Hz, 1H), 6.99 (d, *J=* 9.6 Hz, 1H), 5.45 (s, 2H), 4.05 - 3.92 (m, 1H), 3.80 - 3.63 (m, 1H), 3.25 - 3.12 (m, 2H), 3.11 - 3.04 (m, 2H), 3.00 - 2.88 (m, 2H), 2.43 - 2.37 (m, 4H), 2.28 - 2.20 (m, 1H), 2.07 - 1.96 (m, 1H), 0.89 - 0.87 (m, 6H).

### Example 208 and Example 209

### (9aS)-8-(6-(1-(4-(Difluoromethyl)phenyl)-4-methyl-1H-1,2,3-triazol-5-yl)methoxy)pyridazin-3-yl)-3-isobutylhexahydro-2H-pyrazino[1,2-a]pyrazin-1(6H)-one (208)

### (9aS)-8-(6-(1-(4-(Difluoromethyl)phenyl)-4-methyl-1H-1,2,3-triazol-5-yl)methoxy)pyridazin-3-yl)-3-isobutylhexahydro-2H-pyrazino[1,2-a]pyrazin-1(6H)-one (209)

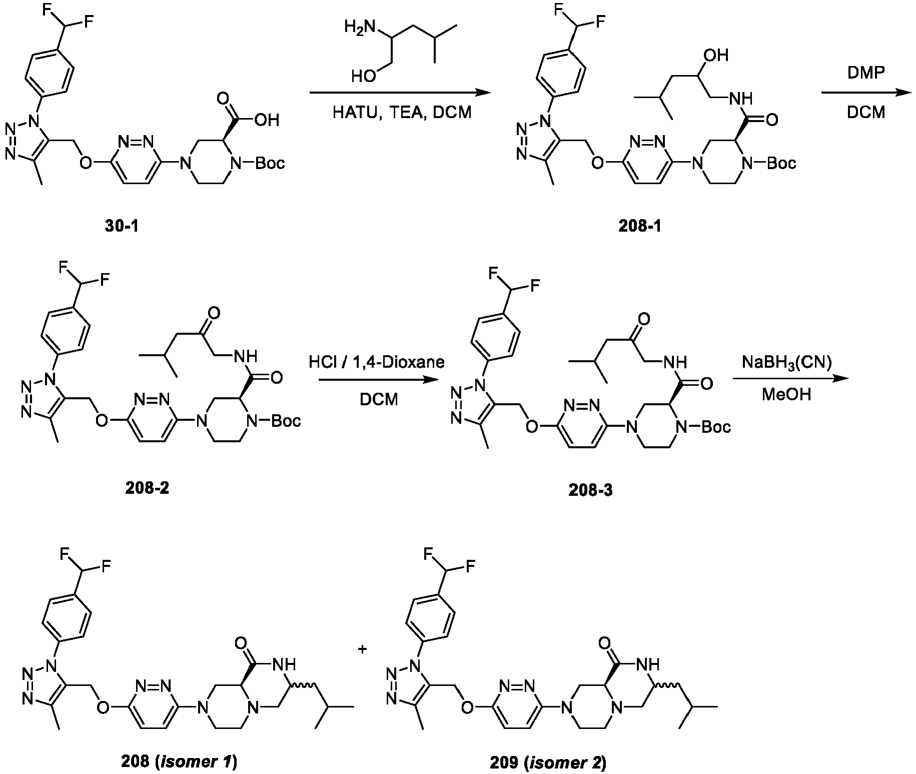

The experimental operation was as described in Example **197,** using **30-1** and 2-amino-4-methylpentan-1-ol as raw materials to obtain the title product.

### Compound 208:

MS (ESI) m/z [M+H]⁺ = 527.3,

HPLC analysis retention time Rt = 2.70 min. (HPLC analysis conditions: chromatographic column: Gemini C18 3 * 50 mm 3 µm; mobile phase: A: water (containing 0.04% TFA), B: acetonitrile (containing 0.02% TFA); gradient: 10% to 50% to 90% / 5 min; flow rate: 1.0 mL/min; column temperature: 35°C).

¹H NMR (400 MHz, CDCl₃) δ 7.70 (d, *J* = 8.4 Hz, 2H), 7.65 (d, *J* = 8.4 Hz, 2H), 7.18 (d, *J* = 9.8 Hz, 1H), 6.86 (d, *J* = 9.8 Hz, 1H), 6.63 (t, *J=* 56.0 Hz, 1H), 5.94 (s, 1H), 5.44 (s, 2H), 4.41 (d, *J* = 12.2 Hz, 1H), 4.31 (d, *J* = 12.2 Hz, 1H), 3.47 - 3.37 (m, 1H), 3.11 - 3.01 (m, 1H), 2.95 - 2.86 (m, 2H), 2.84 - 2.72 (m, 2H), 2.72 - 2.65 (m, 1H), 2.48 (s, 3H), 2.46 - 2.38 (m, 1H), 1.54 - 1.46 (m, 1H), 0.93 (dd, *J=* 6.2, 4.0 Hz, 6H), 0.88 - 0.83 (m, 2H).

### Compound 209:

MS (ESI) m/z [M+H]⁺ = 527.3,

HPLC analysis retention time Rt = 2.78 min. (HPLC analysis conditions: chromatographic column: Gemini C18 3 * 50 mm 3 µm; mobile phase: A: water (containing 0.04% TFA), B: acetonitrile (containing 0.02% TFA); gradient: 10% to 50% to 90% / 5 min; flow rate: 1.0 mL/min; column temperature: 35°C).

¹H NMR (400 MHz, CDCl₃) δ 7.70 (d, *J* = 8.4 Hz, 2H), 7.65 (d, *J* = 8.4 Hz, 2H), 7.19 (d, *J* = 9.6 Hz, 1H), 6.86 (d, *J* = 9.6 Hz, 1H), 6.70 (t, *J* = 56.0 Hz, 1H), 5.75 (s, 1H), 5.44 (s, 2H), 4.39 (d, *J=* 12.2 Hz, 1H), 4.32 (d, *J=* 12.2 Hz, 1H), 3.81 - 3.71 (m, 1H), 3.13 - 3.04 (m, 1H), 3.03 - 2.91 (m, 3H), 2.86 - 2.79 (m, 1H), 2.48 (s, 3H), 2.46 - 2.39 (m, 1H), 2.27 - 2.18 (m, 1H), 1.35 - 1.29 (m, 2H), 0.95 (t, *J* = 7.2 Hz, 6H), 0.89 - 0.83 (m, 1H).

### Example 210 and Example 211

### (9aS)-8-(6-(1-(4-(Difluoromethyl)phenyl)-4-methyl-1H-1,2,3-triazol-5-yl)methoxy)pyridazin-3-yl)-3-(1-methyl-1H-pyrazol-4-yl)hexahydro-2H-pyrazino[1,2-a]pyrazin-1(6H)-one (210)

### (9aS)-8-(6-(1-(4-(Difluoromethyl)phenyl)-4-methyl-1H-1,2,3-triazol-5-yl)methoxy)pyridazin-3-yl)-3-(1-methyl-1H-pyrazol-4-yl)hexahydro-2H-pyrazino[1,2-a]pyrazin-1(6H)-one (211)

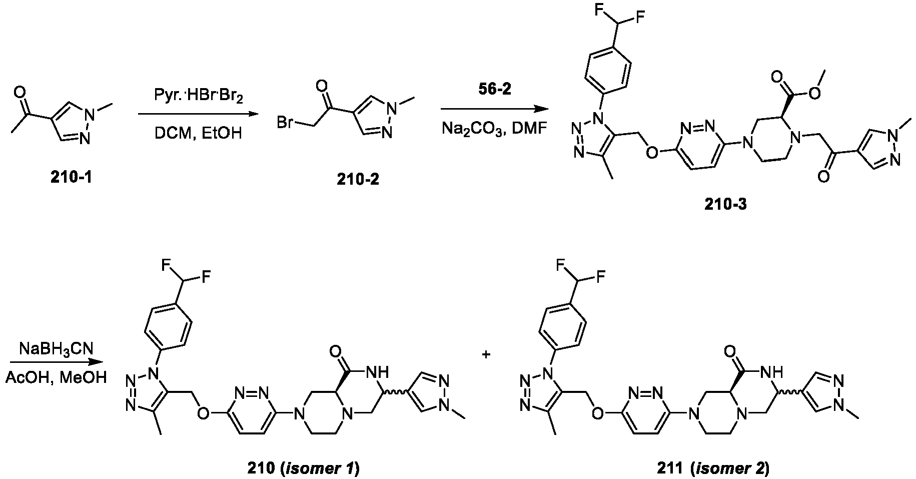

### Step 1: Synthesis of 2-bromo-1-(1-methyl-1H-pyrazol-4-yl)ethan-1-one (210-2)

Compound **210-1** (2 g, 16.1 mmol) was dissolved in a mixed solvent of dichloromethane (16 mL) and ethanol (4 mL) at room temperature, then pyridine tribromide (5.15 g, 16.1 mmol) was added thereto, and the reaction mixture was stirred at room temperature for 1 hour. The reaction was quenched by adding sodium thiosulfate solution (10 mL), stirred at room temperature for 10 minutes, and filtered. The filtrate was collected and concentrated under reduced pressure to obtain the title product (2.73 g, yellow solid).

### Step 2: Synthesis of methyl (S)-4-(6-(1-(4-(difluoromethyl)phenyl)-4-methyl-1H-1,2,3-triazol-5-yl)methoxy)pyridazin-3-yl)-1-(2-(1-methyl-1H-pyrazol-4-yl)-2-oxoethyl)piperazine-2-carboxylate (210-3)

Compound **56-2** (950 mg, 2.07 mmol), **210-2** (0.50 g, 2.48 mmol), and sodium carbonate (0.44 g, 4.14 mmol) were added to DMF (20 mL) at room temperature, and the reaction mixture was stirred at 50°C for 12 hours. The reaction mixture was diluted with water (20 mL) and extracted with ethyl acetate (50 mL × 3). The organic phase was washed with saturated brine (150 mL × 2), dried over anhydrous sodium sulfate, and filtered, and the filtrate was concentrated under reduced pressure to obtain the title product (100 mg, crude product).

MS (ESI) m/z [M+H]⁺ = 583.2.

### Step 3: Synthesis of (9aS)-8-(6-(1-(4-(difluoromethyl)phenyl)-4-methyl-1H-1,2,3-triazol-5-yl)methoxy)pyridazin-3-yl)-3-(1-methyl-1H-pyrazol-4-yl)hexahydro-2H-pyrazino[1,2-a]pyrazin-1(6H)-one (210) and (9aS)-8-(6-(1-(4-(difluoromethyl)phenyl)-4-methyl-1H-1,2,3-triazol-5-yl)methoxy)pyridazin-3-yl)-3-(1-methyl-1H-pyrazol-4-yl)hexahydro-2H-pyrazino[1,2-a]pyrazin-1(6H)-one (211)

The experimental operation was as described in step 4 in Example **197,** using compound **210-3** as the reactant to obtain the title product.

### Compound 210:

MS (ESI) m/z [M+H]⁺ = 551.2;

HPLC analysis retention time Rt = 2.35 min. (HPLC analysis conditions: chromatographic column: NanoChrom C18 4.6 * 50 mm * 3 µm; mobile phase: A: water (containing 0.04% TFA), B: acetonitrile (containing 0.02% TFA); gradient: 5% to 95% / 5 min; flow rate: 1.0 mL/min; column temperature: 40°C).

¹H NMR (400 MHz, CDCl₃) δ 7.70 (d, *J* = 8.4 Hz, 2H), 7.65 (d, *J=* 8.4 Hz, 2H), 7.46 (s, 1H), 7.36 (s, 1H), 7.20 (d, *J* = 9.1 Hz, 1H), 6.87 (d, *J* = 9.6 Hz, 1H), 6.70 (t, *J* = 56.0 Hz, 1H), 5.83 (s, 1H), 5.45 (s, 2H), 4.85 - 4.76 (m, 1H), 4.43 - 4.32 (m, 2H), 3.89 (s, 3H), 3.14 - 3.03 (m, 2H), 3.03 - 2.88 (m, 3H), 2.56 - 2.42 (m, 5H).

### Compound 211:

MS (ESI) m/z [M+H]⁺ = 551.2;

HPLC analysis retention time Rt = 2.35 min. (HPLC analysis conditions: chromatographic column: NanoChrom C18 4.6 * 50 mm * 3 µm; mobile phase: A: water (containing 0.04% TFA), B: acetonitrile (containing 0.02% TFA); gradient: 5% to 95% / 5 min; flow rate: 1.0 mL/min; column temperature: 40°C).

¹H NMR (400 MHz, CDCl₃) δ 7.71 (d, *J* = 8.4 Hz, 2H), 7.65 (d, *J* = 8.4 Hz, 2H), 7.48 (s, 1H), 7.42 (s, 1H), 7.20 (d, *J* = 9.6 Hz, 1H), 6.87 (d, *J* = 9.6 Hz, 1H), 6.70 (t, *J* = 56.0 Hz, 1H), 6.01 (s, 1H), 5.45 (s, 2H), 4.55 - 4.50 (m, 1H), 4.43 - 4.34 (m, 2H), 3.89 (s, 3H), 3.11 - 3.02 (m, 1H), 3.01 - 2.85 (m, 5H), 2.49 (s, 3H), 2.48 - 2.42 (m, 1H).

### Example 212

### 3-(2-((1-(4-(Difluoromethyl)phenyl)-4-methyl-1H-1,2,3-triazol-5-yl)methoxy)pyrimidin-4-yl)oxazolidin-2-one (212)

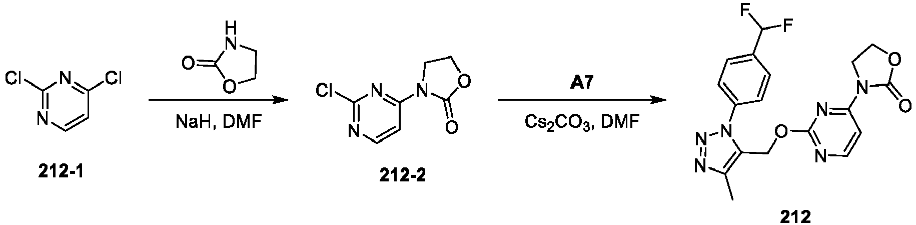

### Step 1: Synthesis of 3-(2-chloropyrimidin-4-yl)oxazolidin-2-one (212-2)

The experimental operation was as described in step 6 in the synthesis of intermediate **A8,** using compound **212-1** and oxazolidin-2-one as reactants and DMF as the solvent to obtain the title product.

### Step 2: Synthesis of 3-(2-((1-(4-(difluoromethyl)phenyl)-4-methyl-1H-1,2,3-triazol-5-yl)methoxy)pyrimidin-4-yl)oxazolidin-2-one (212)

The experimental operation was as described in step 4 in the synthesis of intermediate **J8.** Compounds **212-2** and **A7** were used as reactants and reacted at 100°C to obtain the title product.

MS (ESI) m/z [M+H]⁺ = 403.1.

¹H NMR (400 MHz, CD₃OD) δ 8.35 (d, *J* = 6.0 Hz, 1H), 7.84 (d, *J* = 6.0 Hz, 1H), 7.77 (s, 4H), 6.89 (t, *J=* 56.0 Hz, 1H), 5.53 (s, 2H), 4.50 (d, *J* = 8.0 Hz, 2H), 4.13 (d, *J* = 8.0 Hz, 2H), 2.48 (s, 3H).

### Example 213

### 4-(4-((1-(4-(Difluoromethyl)phenyl)-4-methyl-1H-1,2,3-triazol-5-yl)methoxy)pyrimidin-2-yl)piperazin-2-one (213)

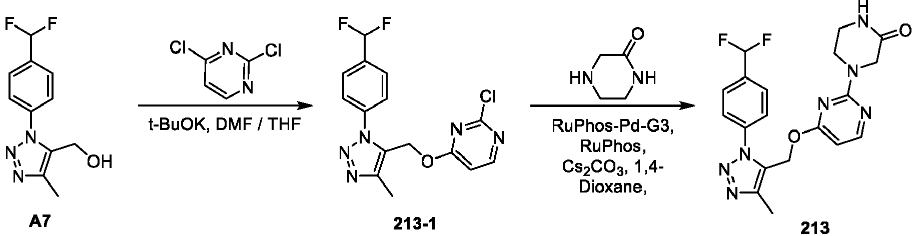

### Step 1: 2-Chloro-4-(1-(4-(difluoromethyl)phenyl)-4-methyl-1H-1,2,3-triazol-5-yl)methoxy)pyrimidine (213-1)

Compound **A7** (200 mg, 0.84 mmol) was dissolved in THF (4 mL), cooled to -78°C, then potassium *tert*-butoxide (112 mg, 0.84 mmol) was added thereto, and the reaction mixture was stirred for 0.5 hours. Then 2,4-dichloropyrimidine (125 mg, 0.84 mmol) (1 mL DMF solution) was added thereto, and the reaction mixture was stirred at -78°C for 1 hour. The reaction mixture was warmed to 0°C, and the reaction was quenched by adding water (20 mL) and extracted with dichloromethane (50 mL × 3). The organic phase was washed with saturated brine (50 mL), dried over anhydrous sodium sulfate, and filtered. The filtrate was concentrated under reduced pressure, and the residue was purified by flash silica gel column chromatography (dichloromethane: methanol = 99:1 to 20:1) to obtain the title product (250 mg, white solid).

### Step 2: Synthesis of 4-(4-((1-(4-(difluoromethyl)phenyl)-4-methyl-1H-1,2,3-triazol-5-yl)methoxy)pyrimidin-2-yl)piperazin-2-one (213)

The experimental operation was as described in Example **1,** using compound **213-1** and 2-piperazinone as raw materials to obtain the title product.

MS (ESI) m/z [M+H]⁺ = 415.9.

¹H NMR (400 MHz, CDsOD) δ 8.09 (d, *J* = 5.6 Hz, 1H), 7.73 (dd, *J* = 8.8 Hz, 4H), 6.88 (t, *J=* 56.0 Hz, 1H), 6.07 (d, *J* = 6.0 Hz, 1H), 5.54 (s, 2H), 4.25 (s, 2H), 3.92 (d, *J* = 5.2 Hz, 2H), 3.36 (d, *J* = 5.2 Hz, 2H), 2.45 (s, 3H).

### Example 214

### 4-(6-((1-(4-Chlorophenyl)-1H-1,2,3-triazol-5-yl)methoxy)pyridazin-3-yl)piperazin-2-one (214)

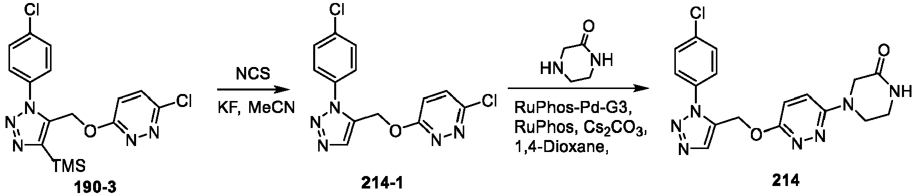

### Step 1: Synthesis of 3-chloro-6-(1-(4-chlorophenyl)-1H-1,2,3-triazol-5-yl)methoxy)pyridazine (214-1)

The experimental operation was as described in step 4 in the synthesis of Example **189,** using compound **190-3** as the reactant to obtain the title product.

MS (ESI) m/z [M+H]⁺ = 321.8.

### Step 2: Synthesis of 4-(6-((1-(4-chlorophenyl)-1H-1,2,3-triazol-5-yl)methoxy)pyridazin-3-yl)piperazin-2-one (214)

The experimental operation was as described in Example **1,** using compound **214-1** and 2-piperazinone as raw materials to obtain the title product.

MS (ESI) m/z [M+H]⁺ = 385.9.

¹H NMR (400 MHz, CDsOD) δ 8.02 (s, 1H), 7.69 - 7.59 (m, 4H), 7.36 (d, *J* = 9.6 Hz, 1H), 7.04 (d, *J* = 9.6 Hz, 1H), 5.54 (s, 2H), 4.13 (s, 2H), 3.78 - 3.73 (m, 2H), 3.49 - 3.43 (m, 2H).

### Example 215

### (9aS)-2-(6-(1-(4-(Difluoromethyl)phenyl)-4-methyl-1H-1,2,3-triazol-5-yl)methoxy)pyridazin-3-yl)-4-isopropylhexahydro-2H-pyrazino[1,2-a]pyrazin-1(6H)-one (215)

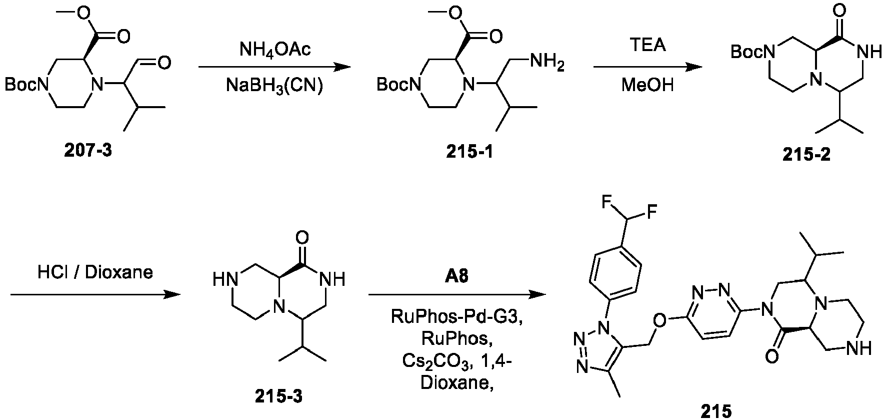

### Steps 1 to 3: Synthesis of (9aS)-4-isopropylhexahydro-2H-pyrazino[1,2-a]pyrazin-1(6H)-one (215-3)

The experimental operation was as described in steps 2 to 4 in the synthesis of Example **200,** using compound **207-3** as the starting material to obtain the title product.

MS (ESI) m/z [M+H]⁺ = 198.2.

### Step 2: (9aS)-2-(6-(1-(4-(Difluoromethyl)phenyl)-4-methyl-1H-1,2,3-triazol-5-yl)methoxy)pyridazin-3-yl)-4-isopropylhexahydro-2H-pyrazino[1,2-a]pyrazin-1(6H)-one (215)

The experimental operation was as described in Example **1,** using compounds **215-3** and **A8** as raw materials to obtain the title product.

MS (ESI) m/z [M+H]⁺ = 513.2.

¹H NMR (400 MHz, DMSO-*d*₆) δ 8.06 (d, *J* = 9.6 Hz, 1H), 7.81 (s, 4H), 7.32 (d, *J* = 9.6 Hz, 1H), 7.16 (t, *J* = 55.6 Hz, 1H), 5.60 (s, 2H), 4.55 - 4.48 (m, 1H), 4.07 - 4.03 (m, 1H), 3.70 - 3.57 (m, 2H), 3.18 - 2.98 (m, 3H), 2.85 - 2.67 (m, 3H), 2.64 - 2.55 (m, 1H), 2.43 (s, 3H), 1.82 - 1.71 (m, 1H), 1.01 - 0.88 (m, 6H).

### Biological experiment method:

Recent research results have revealed that the GABA_{A} receptors mediate at least two modes of inhibition, the phasic inhibition and the tonic inhibition. GABA_{A} receptors in the synapse, due to action potentials, cause the synchronous release of GABA-containing vesicles in the synapse, resulting in a sharp increase in the concentration of GABA in the synaptic gap on a millimolar scale, thus causing the synchronous activation and rapid desensitization of postsynaptic GABA_{A} receptors, forming phasic inhibition. However, GABA_{A} receptors located outside the synapse are typically in a persistent environment of low GABA_{A} concentrations, ranging from tens of nanomoles to a few millimoles. GABA_{A} receptors with high affinity for GABA are continuously asynchronously activated, forming tonic inhibition. Phasic inhibition and tonic inhibition jointly regulate neural excitability and signaling. (Farrant, M. et al., Nat Rev Neurosci, 2005, 6, 215-229). Yeung JY *et al.* disclosed that low concentrations of GABA are more likely to activate the α5-GABA_{A} receptor (Yeung JY et al. Mol Pharmacol, 2003, 63, 2-8). K. Y. Lee reported that sustained high-affinity GABA_{A} currents activated by low concentrations of GABAwere detected in isolated DRG cells cultured for 24 hours. (Lee, K. Y. et al., Neuroscience 2012, 208, 133-142). In 2013, I. Lecker *et al.* disclosed that L-655,708, an α5-GABA_{A} receptor inverse agonist, dose-dependently inhibited the current induced by low concentrations of GABA (5, 50, and 500 nM). When the GABA concentration was increased to 1 µM, the highest concentration of L-655,708 could only suppress 15% of the current. When the GABA concentration continued to increase, L-655,708 had no inhibitory effect on the current induced by GABA. (Lecker, I. et al., British Journal of Anaesthesia, 2013, 110 (S1), i73-i81).

### Effect example

### I. Affinity activities of compounds of the present disclosure on different subtypes of GABA_{A} receptors

The affinity of the compound to each subtype of GABA_{A} receptor was determined by competing for the binding of ³H-flunitrazepam to HEK293 cells stably expressing human α1β3γ2, α2β3γ2, α3β3γ2, and α5β3γ2 receptors.

The cells were suspended in 50 mM Tris-HCl buffer (pH = 7.4), homogenized 10 times on ice with a homogenizer for 20 seconds, and centrifuged at 1000 g for 10 minutes at 4°C. The supernatant was collected, and the above steps were repeated. The supernatant was centrifuged at 4°C (33800 g; Thermo, rotor: A27-8x50) for 60 minutes, and the precipitate was resuspended in Tris buffer (50 mM Tris-HCl, 10 mM MgClz, 0.5 mM EDTA, 10% glycerol). Protein was measured (BCA method, Pierce), and 1 mL aliquots were prepared and stored at - 80°C.

The radioligand competition binding assay was performed in a 200 µL system (96-well plate) containing 100 µL of cell membrane. The concentration of ³H-flunitrazepam was 1 nM and the concentration of the test compound was in the range of 1 × 10⁻⁵ - 10⁻⁶ M. Flumazenil was used as a control. 1 µL of 2 mM flumazenil (final concentration 10 µM) was added to the low signal control well (Low control, LC), and 1 µL of DMSO was added to the high signal control well (High control, HC). The final concentration of target membrane protein was 5 µg/well. All test compound sample storage solutions were 10 mM. The working concentration of the samples was to dilute all the samples to 0.2 mM with DMSO, followed by 4-fold serial gradient dilutions for a total of 8 concentration gradients. The 96-well plate was sealed with a sealing film, and then incubated on a shaker at room temperature for 1 hour. At the same time, the GF/C filter plate was soaked in soaking buffer (0.3% PEI, stored at 4°C) for at least 0.5 hours. After the binding incubation was completed, the cells were collected onto a GF/C filter plate using a cell harvester. The plate was washed four times with washing buffer (50 mM Tris-HCl, pH 7.4, stored at 4°C). After drying in a 50°C oven for 1 hour, the bottom of the dried GF/C filter plate was sealed. The residual radioactivity on the filter membrane was detected using liquid scintillation counting. 50 µL of scintillation fluid was added to each well, and the plate was sealed. The readings were taken using a Microbeta2. The inhibitory activity of the test sample on the binding of ³H-flunitrazepam to GABA_{A} receptor membrane proteins was calculated, and the IC₅₀ of each test sample was calculated by dose-effect curve fitting (GraphPad Prism 5 software), and the Ki of the sample was calculated from the IC₅₀, thus evaluating the binding ability of the sample to the various subtypes of GABA_{A} receptors.

Representative assay results obtained by the above method of determining the binding affinity of HEK293 cells expressing human GABA_{A} receptors are shown in the table below.

**Table 1 Affinities of compounds on α5-GABA_{A} receptors**

| **Example** | **α5-GABA_{A}R Ki (nM)** | **Example** | **α5-GABA_{A}R Ki (nM)** | **Example** | **α5-GABA_{A}R Ki (nM)** |
|---|---|---|---|---|---|
| 1 | 1.98 | 74 | 23.62 | 148 | 2.50 |
| 2 | 2.15 | 75 | 40.18 | 149 | 2.04 |
| 3 | 1.28 | 76 | 2.87 | 150 | 2.35 |
| 4 | 7.25 | 78 | 2.03 | 151 | 1.36 |
| 5 | 0.96 | 79 | 32.50 | 152 | 1.65 |
| 6 | 0.91 | 80 | 0.82 | 153 | 1.89 |
| 7 | 1.00 | 81 | 0.56 | 154 | 5.26 |
| 8 | 0.89 | 82 | 0.63 | 155 | 5.39 |
| 9 | 1.46 | 83 | 2.60 | 156 | 4.13 |
| 10 | 14.39 | 84 | 0.60 | 157 | 3.53 |
| 11 | 0.58 | 85 | 2.24 | 158 | 4.31 |
| 12 | 0.89 | 86 | 8.31 | 159 | 20.00 |
| 13 | 2.20 | 87 | 3.46 | 160 | 6.21 |
| 14 | 1.24 | 88 | 1.78 | 161 | 13.78 |
| 17 | 4.32 | 90 | 0.75 | 162 | 2.58 |
| 18 | 2.13 | 91 | 1.13 | 163 | 26.36 |
| 19 | 0.52 | 92 | 1.11 | 164 | 2.40 |
| 20 | 1.97 | 93 | 3.14 | 167 | 1.08 |
| 21 | 1.24 | 94 | 44.26 | 168 | 2.84 |
| 22 | 2.69 | 95 | 0.67 | 169 | 1.11 |
| 23 | 3.73 | 96 | 1.34 | 170 | 2.30 |
| 24 | 7.72 | 97 | 1.11 | 171 | 1.07 |
| 25 | 24.42 | 98 | 1.53 | 172 | 0.67 |
| 26 | 1.32 | 99 | 1.95 | 173 | 0.55 |
| 27 | 3.65 | 100 | 1.54 | 174 | 1.39 |
| 28 | 4.31 | 101 | 1.15 | 175 | 2.63 |
| 30 | 2.72 | 102 | 2.14 | 176 | 1.34 |
| 31 | 7.45 | 103 | 1.27 | 177 | 1.26 |
| 32 | 2.94 | 104 | 1.77 | 178 | 17.21 |
| 33 | 3.31 | 105 | 1.24 | 179 | 6.33 |
| 34 | 7.08 | 106 | 7.39 | 180 | 1.19 |
| 35 | 9.03 | 107 | 15.64 | 181 | 1.21 |
| 36 | 13.6 | 108 | 1.66 | 182 | 0.71 |
| 37 | 12.3 | 109 | 1.53 | 183 | 1.37 |
| 38 | 2.48 | 110 | 1.60 | 184 | 2.53 |
| 39 | 1.59 | 111 | 0.93 | 185 | 0.96 |
| 40 | 33.59 | 112 | 0.81 | 186 | 4.62 |
| 41 | 42.58 | 113 | 1.17 | 187 | 11.12 |
| 42 | 115 | 114 | 2.82 | 188 | 40.64 |
| 43 | 4.49 | 115 | 1.25 | 189 | 9.44 |
| 44 | 2.31 | 116 | 1.66 | 190 | 1.96 |
| 45 | 3.26 | 117 | 4.87 | 191 | 3.27 |
| 46 | 1.91 | 118 | 1.66 | 192 | 17.41 |
| 47 | 1.10 | 120 | 27.17 | 193 | 1.45 |
| 48 | 2.38 | 121 | 1.73 | 194 | 0.80 |
| 49 | 6.50 | 122 | 2.46 | 195 | 0.54 |
| 50 | 6.43 | 123 | 7.20 | 197 | 4.95 |
| 51 | 6.17 | 124 | 2.40 | 198 | 3.13 |
| 52 | 1.10 | 126 | 40.10 | 199 | 3.17 |
| 53 | 2.97 | 127 | 2.08 | 200 | 1.90 |
| 54 | 2.55 | 128 | 22.51 | 201 | 1.74 |
| 55 | 0.39 | 129 | 9.75 | 202 | 2.32 |
| 56 | 7.99 | 130 | 17.09 | 203 | 1.99 |
| 57 | 0.27 | 131 | 1.34 | 204 | 2.31 |
| 58 | 1.85 | 132 | 12.07 | 205 | 2.10 |
| 59 | 3.97 | 134 | 1.50 | 206 | 0.64 |
| 60 | 1.33 | 135 | 1.05 | 207 | 4.92 |
| 61 | 2.12 | 137 | 0.49 | 208 | 1.96 |
| 62 | 1.18 | 138 | 2.11 | 209 | 2.04 |
| 63 | 1.49 | 139 | 1.37 | 210 | 2.79 |
| 64 | 1.45 | 140 | 1.41 | 211 | 2.02 |
| 65 | 2.37 | 141 | 4.27 | 213 | 54.14 |
| 66 | 4.07 | 142 | 1.80 | 214 | 49.21 |
| 68 | 0.84 | 143 | 1.89 | 215 | 36.08 |
| 69 | 0.90 | 144 | 1.63 | | |
| 71 | 8.86 | 145 | 1.27 | | |
| 72 | 0.46 | 146 | 4.44 | | |
| 73 | 4.93 | 147 | 6.55 | | |

**Table 2 Affinities of compounds on different subtypes of GABA_{A} receptors**

| **Example** | Ki (nM) α1/α2/α3 | **Example** | Ki (nM) α1/α2/α3 |
|---|---|---|---|
| **2** | 145.15/19.80/28.61 | **76** | 832.47/301.30/173.32 |
| **5** | 58.11/12.88/16.78 | **78** | 246.25/45.88/28.43 |
| **6** | 59.81/15.87/14.14 | **80** | 161.01/22.68/15.99 |
| **7** | 122.03/26.20/17.02 | **95** | 6.23/-/- |
| **8** | 80.49/22.22/16.99 | **103** | 52.79/40.11/10.26 |
| **19** | 15.84/10.39/7.34 | **116** | 65.22/50.13/22.73 |
| **21** | 36.73/41.75/21.83 | **122** | 265.91/149.47/67.35 |
| **22** | 87.01/38.62/- | **127** | 399.84/23.68/36.71 |
| **23** | 21.95/13.55/- | **129** | 911.41/167.75/195.30 |
| **24** | 19.26/16.98/- | **148** | 444.65/116.90/56.16 |
| **26** | 47.78/45.39/23.36 | **155** | 389.04/230.77/68.70 |
| **27** | 332.75/106.94/134.61 | **169** | 73.50/27.20/26.27 |
| **43** | 149.37/72.66/18.51 | **173** | 80.61/16.78/14.09 |
| **54** | 293.76/71.57/49.76 | **181** | 139.92/64.21/44.65 |
| **55** | 177.56/56.37/27.52 | **204** | 158.32/70.06/50.26 |
| **56** | -/329.71/- | | |
| **61** | 126.25/26.02/13.14 | | |
| **68** | 56.91/17.11/14.01 | | |

| | | | |
|---|---|---|---|
| Note: "-" means that the experimental test was not carried out. | | | |

It can be seen from Table 1 and Table 2 that the Ki values of the compounds of the present disclosure that replaced ³H-flunitrazepam from the human α5-GABA_{A} receptor were 100 nM or less, and the Ki of most preferred compounds was < 10 nM, indicating that the compounds of the present disclosure have good affinities for the α5-GABA_{A} receptor. In a preferred example, the compound of the present disclosure is selective in its affinity for different subtypes of GABA_{A} receptors, and can selectively bind to the α5 subunit better than α1, α2, and α3-GABA_{A} subunit receptors, especially, with high selectivity for α5-GABA_{A} subunit.

### II. Inverse agonistic activity of compounds of the present disclosure on α5-GABA_{A} receptors

The inventors used electrophysiological methods to determine the inverse agonist efficacy of the drugs to be tested on the α5-GABA_{A} receptor. The detailed methods are as follows:

Different subunits of the GABA_{A} receptor were co-expressed in the HEK293 cell line to construct a fully functional GABA_{A} receptor. The α, β, and γ subunits are essential to form a complete functional GABA_{A} receptor. In this example, the present disclosure had established the following cell model: α5 subunit (see GenBank Accession No.: NM_000810.3 for protein sequences), β3 subunit (see GenBank Accession No.: NM_000814.5 for protein sequences), and γ2 subunit (see GenBank Accession No.: NM_000816.3 for protein sequences) were co-expressed in the HEK293 cell line, followed by screening the monoclonal stably transfected cell line. This cell line expressed a fully functional α5-GABA_{A} receptor.

The monoclonal stably transfected HEK293 cell line expressing α5-GABA_{A} receptor were cultured in 10 cm culture dishes and passaged when the cells grew to 80% to 90% confluent. During passaging, the culture medium was aspirated first, then 3 mL of DPBS phosphate buffer salt (Gibco^{™}) was added to the culture dishes, and the culture dishes were shaken slightly, and DPBS was aspirated. 1 mL of trypsin (TrypLE Express, Gibco^{™}) was added thereto, and the cells were digested at 37°C for 1-2 minutes. Then 3 mL of complete medium (DMEM + 10% FBS (Gibco^{™})) was added and the cells at the bottom of the culture dishes were dispersed. The cell suspension was transferred to a 15 mL centrifugal tube (Corning) and then centrifuged at 200 g for 3 minutes. The supernatant was discarded, 4 mL of complete medium was added, and the cells were gently blown and resuspended for use. If cell passaging was performed, the cell suspension was diluted at a ratio of 1:5 or 1: 10. If cells for electrophysiological detection were prepared, the cell suspension was diluted in a ratio of 1: 12, then added into a 24-well dish (Corning^{™}) in which glass slides were placed and pretreated with Poly-D-Lysine, and the experiment was carried out after the cells adhered to the wall. The cells used for electrophysiology was cultured no more than 48 hours.

Drug concentration setting: The final drug concentration used in drug screening was 100 nM, and the GABA concentration was 0.05 µM. The whole cell patch clamp technique was used in electrophysiological assays, which could refer to the literature (I. Lecker, Y. Yin, D. S. Wang and B. A. Orser, British Journal of Anaesthesia, 2013, 110 (S1), i73-i81). The compositions of the extracellular solution for electrophysiology assay were as follows: 150 mM NaCl, 5 mM KCl, 2.5 mM CaCh, 1 mM MgClz, 10 mM HEPES, and 10 mM glucose (pH 7.4); the formulation of the electrode internal fluid for electrophysiology use was as follows: 140 mM CsCl, 11 mM EGTA, 10 mM HEPES, 2 mM CaClz, 1 mM MgClz, 4 mM MgATP, and 2 mM TEA (pH 7.3). The signal acquisition used an EPC-10 amplifier and the PatchMaster software (HEKA) or Axon700B amplifier and Clampex software (AXON). The recording electrode was pulled from borosilicate glass, and the electrode resistance was 4 to 6 MΩ. The ALA-VC-8PG^{™} system was used for drug application. Single cell that grew independently was selected for recording. During recording, the cell membrane potential was clamped at - 60 mV. During experiment, an extracellular solution was applied outside the cells for about 20 seconds. When the baseline reached to a stable state, the extracellular solution was switched to the GABA solution to induce an inward current. After about 20 to 40 seconds until the current was stable, the solution was switched to a corresponding drug solution to detect the effect of the drug. At last, the solution was switched to the extracellular solution, and the experiment was terminated when the baseline returned to the level before drug application. Only data with a baseline of less than -120 pA that could be recovered after administration would be analyzed subsequently. GABA was diluted at a final concentration of 0.05 µM in the extracellular solution. Then, drugs were diluted at the desired concentration in GABA-containing extracellular solution.

The experimental results were analyzed with the PatchMaster software. During analysis, the leakage current (Iₗₑₐₖ), GABA current before drug application (Iₚᵣₑ), and GABA current after drug application (Iₚₒₛₜ) were measured respectively. The effects of drugs were calculated by the following equation: inverse agonist efficacy (%) = 100 - 100 * (Iₚₒₛₜ - Iₗₑₐₖ) / (Iₚᵣₑ - Iₗₑₐₖ).

### Screening results of compounds:

**Table 3: Inverse agonistic activities of compounds on α5-GABA_{A} receptors**

| **Example** | **Compound inverse agonist efficacy%** | **Example** | **Compound inverse agonist efficacy%** | **Example** | **Compound inverse agonist efficacy%** |
|---|---|---|---|---|---|
| 2 | -58.80 | 62 | -44.63 | 139 | -46.39 |
| 3 | -40.84 | 64 | -41.09 | 141 | -47.21 |
| 5 | -54.51 | 65 | -56.07 | 143 | -52.49 |
| 6 | -42.47 | 66 | -52.73 | 145 | -69.01 |
| 8 | -40.43 | 67 | -50.08 | 146 | -56.48 |
| 11 | -49.06 | 68 | -50.49 | 147 | -54.53 |
| 13 | -43.30 | 69 | -55.08 | 148 | -42.64 |
| 14 | -45.70 | 73 | -41.33 | 154 | -46.60 |
| 18 | -51.45 | 76 | -45.26 | 155 | -42.26 |
| 19 | -53.70 | 78 | -43.69 | 156 | -44.27 |
| 20 | -51.17 | 80 | -54.75 | 157 | -44.23 |
| 21 | -49.31 | 90 | -49.23 | 159 | -49.85 |
| 22 | -48.41 | 92 | -49.69 | 160 | -42.69 |
| 23 | -44.13 | 95 | -51.93 | 162 | -47.67 |
| 26 | -50.32 | 96 | -46.45 | 163 | -43.60 |
| 27 | -59.15 | 97 | -49.24 | 168 | -42.41 |
| 28 | -47.71 | 98 | -53.71 | 169 | -40.48 |
| 30 | -41.91 | 99 | -48.03 | 171 | -41.95 |
| 34 | -42.19 | 100 | -54.63 | 173 | -50.80 |
| 35 | -52.82 | 101 | -43.87 | 178 | -49.74 |
| 36 | -49.04 | 103 | -49.60 | 180 | -40.28 |
| 37 | -50.57 | 104 | -49.94 | 181 | -51.22 |
| 43 | -52.17 | 107 | -49.16 | 189 | -42.14 |
| 44 | -49.65 | 108 | -52.24 | 197 | -52.39 |
| 45 | -50.82 | 110 | -47.51 | 198 | -48.25 |
| 46 | -41.83 | 114 | -51.28 | 199 | -60.16 |
| 50 | -41.47 | 116 | -48.44 | 200 | -60.15 |
| 52 | -45.89 | 122 | -48.70 | 201 | -53.89 |
| 54 | -50.85 | 124 | -48.41 | 203 | -50.78 |
| 55 | -51.63 | 127 | -54.99 | 204 | -68.71 |
| 56 | -53.38 | 128 | -53.87 | 207 | -51.93 |
| 57 | -52.02 | 129 | -56.34 | 208 | -55.85 |
| 58 | -45.95 | 130 | -56.99 | 209 | -52.08 |
| 59 | -44.87 | 131 | -47.57 | 210 | -47.63 |
| 60 | -47.16 | 132 | -53.24 | | |
| 61 | -49.03 | 134 | -45.74 | | |

It can be seen from Table 3 that the compounds of the present disclosure have strong inverse agonistic activity on α5-GABA_{A} receptors. In particular, when the appropriate position in the compound structure contains an amide group, the compound exhibits better inverse agonistic activity of α5-GABA_{A} receptor, such as Example **2.**

### III. Permeability study of compounds of the present disclosure

Brain permeability of compounds was assessed by measuring compound efflux in Madin-Darby canine kidney-II (MDCKII) cells transfected with the human MDR1 gene (Drug metabolism and disposition, 2008, 36(2), 268-275).

The apparent permeability coefficient, Papp, of the compound was calculated by measuring (pH 7.4, 37°C) its permeability from apical to basal (A-B) and basal to apical (B-A) across a monolayer of MDCKII-MDR1 cells. A-B permeability represented drug absorption from the blood to the brain, and B-A permeability represented drug efflux from the brain back into the blood via passive penetration as well as active transport mechanisms. The active transport mechanism was mediated by uptake and efflux transporters expressed on MDCKII-MDR1 cells, primarily by overexpressed human MDR1 P-gp. The same or similar permeability of the compound in the two transport directions indicated passive permeation; the permeability of B-A was higher than the permeability of A-B, indicating that P-gp-mediated efflux was involved in the efflux of the compound, efflux ratio (ER) = Papp (B-A) / Papp (A-B). The larger the efflux ratio, the more obvious the efflux effect, indicating that the compound was less likely to penetrate the blood-brain barrier (BBB).

MDCKII-MDR1 cells were seeded on the polycarbonate microporous membrane of TranswellTM at a density of (0.85 to 5.0) × 10⁶/cm², and used for experiments when the cells reached complete confluence (3 to 5 d). Compounds were dissolved in appropriate solvents (such as DMSO, 1 to 20 mM stock solution), and the stock solution was diluted with HBSS buffer (137 mM NaCl, 5.4 mM KCl, 0.6 mM MgSO₄·7H₂O, 0.5 mM MgCl₂·6H₂O, 0.3 mM Na₂HPO₄·2H₂O, 0.4 mM KH₂PO₄, 5.6 mM glucose, 1.3 mM CaClz, 4.2 mM NaHCO₃) to prepare a transport solution (compound concentration: 0.1 to 300 µM, DMSO concentration < 0.5%), which was placed on the apical or basal side of the monolayer membrane with HBSS buffer on the other side. At the beginning and end of the experiment, samples were collected from the transport solution side. After incubation for a certain period of time (1.5 hours), samples were also collected from the buffer side, and the concentration was determined by HPLC-MS/MS or scintillation counting.

**Table 4: MDCKII-MDR1 permeability of compounds**

| **Example** | **Papp (A-B) (× 10⁻⁶ cm/s)** | **Efflux ratio (ER)** |
|---|---|---|
| 2 | 5.56 | 5.75 |
| 6 | 4.16 | 7.52 |
| 43 | 5.75 | 6.32 |
| 54 | 3.03 | 10.65 |
| 55 | 3.76 | 9.85 |
| 58 | 7.37 | 7.38 |
| 60 | 9.04 | 6.53 |
| 64 | 3.65 | 13.69 |
| 69 | 7.50 | 7.66 |
| 76 | 3.74 | 5.26 |
| 78 | 5.29 | 8.42 |
| 95 | 2.03 | 20.12 |
| 96 | 21.50 | 3.07 |
| 98 | 11.42 | 5.95 |
| 99 | 7.40 | 5.23 |
| 100 | 3.14 | 11.94 |
| 101 | 3.23 | 10.55 |
| 110 | 10.28 | 6.34 |
| 124 | 10.70 | 5.95 |
| 130 | 5.35 | 8.29 |
| 143 | 3.15 | 15.63 |
| 145 | 9.90 | 6.05 |
| 154 | 2.60 | 10.27 |
| 163 | 3.72 | 12.17 |
| 173 | 9.18 | 5.40 |
| 197 | 7.50 | 7.37 |
| 198 | 11.45 | 5.04 |
| 199 | 15.07 | 6.18 |
| 207 | 7.59 | 8.33 |
| 208 | 13.69 | 5.47 |

As can be seen from Table 4, the compounds of the present disclosure have good Papp (A-B) permeability; at the same time, the compounds have a high efflux ratio (ER > 5), indicating that the P-gp-mediated efflux of the compounds is obvious, and the compounds do not readily cross the blood-brain barrier and enter brain tissue. It is suggested that the compounds of the present disclosure can selectively bind to α5-GABA_{A} receptors in the peripheral nervous system and exert analgesic effects on various types of pain.

### IV. Rat pharmacokinetic experiment

The maximum plasma concentration (Cmax) in the rat pharmacokinetic experiment was used to evaluate the absorption of the compounds in rat *in vivo.* The experimental compound was dissolved and then orally administered by gavage (p.o.) to male SD rats. The rats were fasted overnight before drug administration, and blood was collected via venipuncture at 0.25, 0.5, 1, 2, 4, and 7 hours post dose. Approximately 100 µL blood was collected for each sample; at the same time, the whole brain was taken and homogenized with 3 times the amount of PBS for analysis. The LM-MS/MS method was used to determine drug concentrations in plasma and brain tissue, and the proportion of compounds entering the brain (B/P) was calculated based on the AUC of the compound. Pharmacokinetic parameters were calculated using Phoenix WinNonlin 7.0. The administration vehicle was 5% CMC-Na aqueous solution.

**Table 5: Maximum plasma concentration (Cmax) of compounds in rat pharmacokinetics**

| **Example** | **Dosage of administration (mg/kg)** | **Cmax (ng/mL)** |
|---|---|---|
| 2 | 3 | 560 |
| 6 | 3 | 1510 |
| 14 | 3 | 2700 |
| 21 | 1 | 576 |
| 26 | 1 | 637 |
| 43 | 3 | 1080 |
| 54 | 3 | 1750 |
| 55 | 3 | 1620 |
| 56 | 3 | 2050 |
| 60 | 3 | 455 |
| 76 | 3 | 443 |
| 101 | 2 | 426 |
| 104 | 3 | 3890 |
| 108 | 3 | 1780 |
| 124 | 3 | 1730 |
| 155 | 3 | 3017 |
| 169 | 3 | 3176 |
| 173 | 3 | 1240 |
| 181 | 3 | 2670 |
| 197 | 3 | 965 |
| 198 | 3 | 1100 |
| Example 2 in WO2020016443 | 3 | 169 |
| Example 3 in WO2020016443 | 3 | 469 |

**Table 6: Proportion of compounds entering the brain (B/P) in rat pharmacokinetics**

| **Example** | **Dosage of administration (mg/kg)** | **B/P** |
|---|---|---|
| 2 | 3 | 0% |
| 6 | 3 | 0.15% |
| 14 | 3 | 1.35% |
| 21 | 1 | 4.50% |
| 26 | 1 | 3.36% |
| 43 | 3 | 0.17% |
| 54 | 3 | 0.16% |
| 55 | 3 | 0.25% |
| 56 | 3 | 2.61% |
| 60 | 3 | 0% |
| 76 | 3 | 0.50% |
| 101 | 2 | 0% |
| 104 | 3 | 0.95% |
| 108 | 3 | 2.08% |
| 124 | 3 | 0.82% |
| 155 | 3 | 1.66% |
| 169 | 3 | 3.81% |
| 173 | 3 | 1.31% |
| 181 | 3 | 0.99% |
| 197 | 3 | 0.01% |
| 198 | 3 | 1.32% |
| Example 3 in WO2020016443 | 3 | 43.3% |

As can be seen from Table 5, in the rat pharmacokinetic experiment, compared with Examples 2 and 3 in WO2020016443, the compounds of the present disclosure have a higher maximum plasma concentration (Cmax) at the administration dosage, indicating that the compounds have good absorption. At the same time, it can be seen from Table 6 that the proportion of the compounds entering the brain is low (B/P < 5%), suggesting that the compounds can bind to and function on the α5-GABA_{A} receptor in the peripheral nervous system to exert analgesic effects on various types of pain but have little side effects on the central nervous system due to low blood-brain barrier (BBB) permeability.

## Claims

1. A compound of formula I, a *cis-trans* isomer thereof, an enantiomer thereof, a diastereomer thereof, a racemate thereof, a solvate thereof, a hydrate thereof, a pharmaceutically acceptable salt thereof, or a prodrug thereof, 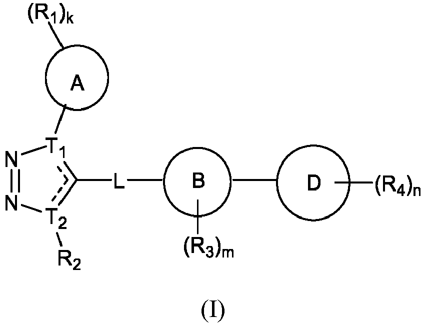 wherein
ring A is selected from a benzene ring or a 5- to 6-membered heteroaryl ring;
each R₁ is independently selected from hydrogen, halogen, cyano, C₁₋₆ alkyl, C₁₋₆ alkoxy, C₁₋₆ alkylamino, C₃₋₆ cycloalkyl, or 3- to 6-membered heterocycloalkyl, and the C₁₋₆ alkyl, C₁₋₆ alkoxy, C₁₋₆ alkylamino, C₃₋₆ cycloalkyl, and/or 3- to 6-membered heterocycloalkyl are optionally substituted by 1 to 3 R';
each R' is independently selected from hydrogen, halogen, hydroxyl, amino, cyano, methyl, cyclopropyl, or methoxy;
k is 0, 1, 2, or 3;
T₁ and T₂ are each independently selected from a carbon atom or a nitrogen atom;
R₂ is selected from hydrogen, halogen, cyano, C₁₋₃ alkyl, C₁₋₃ alkoxy, C₁₋₆ alkylamino, C₃₋₆ cycloalkyl, 3- to 6-membered heterocycloalkyl, or 5- to 6-membered heteroaryl, each of which is optionally substituted by 1 to 3 R';
L is selected from -CHz-O-, -CH=CH-, or -CH₂-NH-;
ring B is selected from a benzene ring or a 5- to 10-membered heteroaryl ring;
each R₃ is independently selected from hydrogen, halogen, cyano, C₁₋₆ alkyl, C₁₋₆ alkoxy, C₁₋₆ alkylamino, C₃₋₆ cycloalkyl, or 3- to 6-membered heterocycloalkyl, each of which is optionally substituted by 1 to 3 R';
m is 0, 1, 2, or 3;
ring D is selected from a 4- to 14-membered heterocycloalkyl ring;
each R₄ is independently selected from hydrogen, halogen, cyano, =O, -R₅, -OR₆, -COOR₆, -C(O)Rs, -NR₆R₇, -NR₆COR₅, -NR₆SO₂R₅, -CH₂-C(O)NR₆R₇, -C(O)NR₆R₇, -SO₂R₆, or - SO₂NR₆R₇;
R₅ is selected from C₁₋₆ alkyl, C₃₋₆ cycloalkyl, 3- to 6-membered heterocycloalkyl, 6- to 10-membered aryl, 6- to 10-membered aryl(C₁₋₃)alkyl, 5- to 10-membered heteroaryl, or 5- to 10-membered heteroaryl(C₁₋₃)alkyl, each of which is optionally substituted by 1 to 3 R;
R₆ and R₇ are each independently selected from hydrogen, hydroxyl, amino, cyano, C₁₋₆ alkyl, C₁₋₆ alkoxy, C₁₋₆ alkylamino, C₃₋₆ cycloalkyl, 3- to 6-membered heterocycloalkyl, 6- to 10-membered aryl, 6- to 10-membered aryl(C₁₋₆)alkyl, 5- to 10-membered heteroaryl, or 5- to 10-membered heteroaryl(C₁₋₆)alkyl, each of which is optionally substituted by 1 to 3 R;
when R₄ is selected from -NR₆R₇, -C(O)NR₆R₇, or -SO₂NR₆R₇, R₆ and R₇ together with the N atom to which they are attached can form a 4- to 7-membered heterocycle, and the 4- to 7-membered heterocycle is optionally substituted by 1 to 3 R;
each R is independently selected from hydrogen, halogen, cyano, hydroxyl, amino, - COOH, C₁₋₆ alkyl, C₁₋₆ alkoxy, C₁₋₆ alkylamino, C₃₋₆ cycloalkyl, or 3- to 6-membered heterocycloalkyl, and the C₁₋₆ alkyl, C₁₋₆ alkoxy, C₁₋₆ alkylamino, C₃₋₆ cycloalkyl, or 3- to 6-membered heterocycloalkyl is optionally substituted by 1 to 3 R';
n is 0, 1, 2, 3, 4, 5, or 6.

2. The compound of formula I, the *cis-trans* isomer thereof, the enantiomer thereof, the diastereomer thereof, the racemate thereof, the solvate thereof, the hydrate thereof, the pharmaceutically acceptable salt thereof, or the prodrug thereof according to claim 1, wherein ring A is selected from a benzene ring or a 5- to 6-membered heteroaryl ring;
each R₁ is independently selected from hydrogen, halogen, cyano, C₁₋₆ alkyl, C₁₋₆ alkoxy, C₁₋₆ alkylamino, C₃₋₆ cycloalkyl, or 3- to 6-membered heterocycloalkyl, and the C₁₋₆ alkyl, C₁₋₆ alkoxy, C₁₋₆ alkylamino, C₃₋₆ cycloalkyl, and/or 3- to 6-membered heterocycloalkyl are optionally substituted by 1 to 3 R';
each R' is independently selected from hydrogen, halogen, hydroxyl, amino, cyano, methyl, cyclopropyl, or methoxy;
k is 0, 1, 2, or 3;
T₁ and/or T₂ are each independently selected from a carbon atom or a nitrogen atom;
R₂ is selected from hydrogen, halogen, cyano, C₁₋₃ alkyl, C₁₋₃ alkoxy, C₁₋₆ alkylamino, C₃₋₆ cycloalkyl, or 3- to 6-membered heterocycloalkyl, each of which is optionally substituted by 1 to 3 R';
L is selected from -CHz-O-, -CH=CH-, or -CH₂-NH-;
ring B is selected from a benzene ring or a 5- to 10-membered heteroaryl ring;
each R₃ is independently selected from hydrogen, halogen, cyano, C₁₋₆ alkyl, C₁₋₆ alkoxy, C₁₋₆ alkylamino, C₃₋₆ cycloalkyl, or 3- to 6-membered heterocycloalkyl, each of which is optionally substituted by 1 to 3 R';
m is 0, 1, 2, or 3;
ring D is selected from a 4- to 14-membered heterocycloalkyl ring;
ring D can be substituted by one or more than one independent R₄, and each R₄ is independently selected from hydrogen, halogen, cyano, =O, -R₅, -OR₆, -COOR₆, -C(O)Rs, - NR₆R₇, -NR₆COR₅, -NR₆SO₂R₅, -C(O)NR₆R₇, -SO₂R₆, or -SO₂NR₆R₇;
R₅ is selected from C₁₋₆ alkyl, C₃₋₆ cycloalkyl, 3- to 6-membered heterocycloalkyl, 6- to 10-membered aryl, 6- to 10-membered aryl(C₁₋₃)alkyl, 5- to 10-membered heteroaryl, or 5- to 10-membered heteroaryl(C₁₋₃)alkyl, each of which is optionally substituted by 1 to 3 R;
R₆ and/or R₇ are each independently selected from hydrogen, hydroxyl, amino, cyano, C₁₋₆ alkyl, C₁₋₆ alkoxy, C₁₋₆ alkylamino, C₃₋₆ cycloalkyl, 3- to 6-membered heterocycloalkyl, 6- to 10-membered aryl, 6- to 10-membered aryl(C₁₋₆)alkyl, 5- to 10-membered heteroaryl, or 5- to 10-membered heteroaryl(C₁₋₆)alkyl, each of which is optionally substituted by 1 to 3 R;
when R₄ is selected from -NR₆R₇, -C(O)NR₆R₇, or -SO₂NR₆R₇, R₆ and R₇ together with the N atom to which they are attached can form a 4- to 7-membered heterocycle, and the 4- to 7-membered heterocycle is optionally substituted by 1 to 3 R;
each R is independently selected from hydrogen, halogen, cyano, hydroxyl, amino, C₁₋₆ alkyl, C₁₋₆ alkoxy, C₁₋₆ alkylamino, C₃₋₆ cycloalkyl, or 3- to 6-membered heterocycloalkyl, and the C₁₋₆ alkyl, C₁₋₆ alkoxy, C₁₋₆ alkylamino, C₃₋₆ cycloalkyl, or 3- to 6-membered heterocycloalkyl is optionally substituted by 1 to 3 R';
n is 0, 1, 2, 3, 4, 5, or 6.

3. The compound of formula I, the *cis-trans* isomer thereof, the enantiomer thereof, the diastereomer thereof, the racemate thereof, the solvate thereof, the hydrate thereof, the pharmaceutically acceptable salt thereof, or the prodrug thereof according to claim 1, wherein it satisfies one or more than one of the following conditions:
(1) R₂ is 5- to 6-membered heteroaryl;
(2) each R₄ is independently -CH₂-C(O)NR₆R₇; and
(3) each R is independently COOH.

4. The compound of formula I, the *cis-trans* isomer thereof, the enantiomer thereof, the diastereomer thereof, the racemate thereof, the solvate thereof, the hydrate thereof, the pharmaceutically acceptable salt thereof, or the prodrug thereof according to at least one of claims 1 to 3, wherein it satisfies one or more than one of the following conditions:
(1) in ring A and R₂, the heteroatom in the 5- to 6-membered heteroaryl is selected from 1, 2, or 3 types of N, O, and S, and the number of the heteroatom is 1, 2, or 3;
(2) in ring B, R₅, R₆, and R₇, the heteroatom in the 5- to 10-membered heteroaryl is selected from 1, 2, or 3 types of N, O, and S, and the number of the heteroatom is 1, 2, or 3;
(3) in R₁, R', R₂, R₃, R₄, and R, the halogen is independently fluorine, chlorine, bromine, or iodine;
(4) in R₁, R₃, R₅, R₆, R₇, and R, the C₁₋₆ alkyl is independently methyl, ethyl, *n*-propyl, isopropyl, *n*-butyl, isobutyl, *sec*-butyl, or *tert*-butyl;
(5) in R₂, the C₁₋₃ alkyl is independently methyl, ethyl, *n*-propyl, or isopropyl;
(6) in R₁, R₃, R₆, R₇, and R, the C₁₋₆ alkoxy is independently methoxy, ethoxy, *n*-propoxy, isopropoxy, *n*-butoxy, isobutoxy, *sec*-butoxy, or *tert*-butoxy; preferably methoxy;
(7) in R₂, the C₁₋₃ alkoxy is independently methoxy, ethoxy, *n*-propoxy, or isopropoxy;
(8) in R₁, R₂, R₃, R₆, R₇, and R, the C₁₋₆ alkylamino is independently -NHCH₃, -N(CH₃)₂, -NHCH₂CH₃, -N(CH₃)CH₂CH₃, -N(CH₂CH₃)₂, -NHCH₂CH₂CH₃, -NHCH(CH₃)₂, or - NHCH₂CH₂CH₂CH₃;
(9) in R₁, R₂, R₃, R₆, R₇, and R, the C₃₋₆ cycloalkyl is independently cyclopropyl, cyclobutyl, cyclopentyl, or cyclohexyl;
(10) in R₁, R₂, R₃, R₆, R₇, and R, the heteroatom in the 3- to 6-membered heterocycloalkyl is selected from 1, 2, or 3 types of N, O, and S, and the number of the heteroatom is 1, 2, or 3;
(11) in ring D, the 4- to 14-membered heterocycloalkyl is a saturated or semi-saturated monocyclic, bicyclic, or tricyclic group having 1, 2, 3, or 4 heteroatoms selected from 1, 2, or 3 types of N, O, and S, wherein the ring directly attached to ring B is not aromatic; preferably, in ring D, the 4- to 14-membered heterocycloalkyl is a saturated monocyclic, bicyclic, or tricyclic group having 1, 2, 3, or 4 heteroatoms selected from 1, 2, or 3 types of N, O, and S;
(12) when R₆ and R₇ together with the N atom to which they are attached can form a 4- to 7-membered heterocycle, the 4- to 7-membered heterocycle is a saturated monocyclic or bicyclic group having 1, 2, or 3 heteroatoms selected from 1, 2, or 3 types of N, O, and S; and
(13) in R₅, R₆, and R₇, the 6- to 10-membered aryl is independently phenyl or naphthyl.

5. The compound of formula I, the *cis-trans* isomer thereof, the enantiomer thereof, the diastereomer thereof, the racemate thereof, the solvate thereof, the hydrate thereof, the pharmaceutically acceptable salt thereof, or the prodrug thereof according to at least one of claims 1 to 3, wherein it satisfies one or more than one of the following conditions:
(1) in ring A and R₂, the 5- to 6-membered heteroaryl is pyridyl or isoxazolyl;
(2) in ring B, R₅, R₆, and R₇, the 5- to 10-membered heteroaryl is pyridyl, pyridazinyl, pyrazinyl, or pyridopyridazinyl;
(3) in R₁, R', R₂, R₃, R₄, and R, the halogen is independently fluorine or chlorine;
(4) in R₂, the C₁₋₃ alkyl is independently methyl;
(5) in R₁, R₂, R₃, R₆, R₇, and R, the C₁₋₆ alkylamino is independently -NMe₂;
(6) in R₁, R₂, R₃, R₆, R₇, and R, the C₃₋₆ cycloalkyl is independently cyclopropyl;
(7) in R₁, R₂, R₃, R₆, R₇, and R, the 3- to 6-membered heterocycloalkyl is oxetanyl, pyrrolidinyl, tetrahydrofuranyl, morpholinyl, tetrahydropyranyl, or piperidinyl;
(8) in ring D, the 4- to 14-membered heterocycloalkyl is 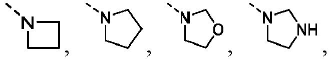 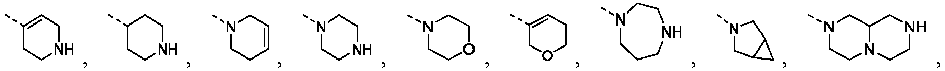 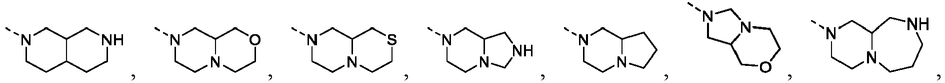 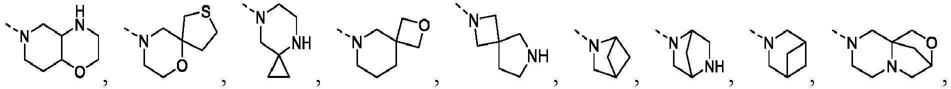 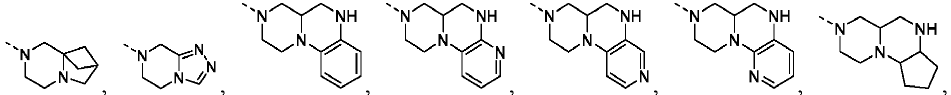 or 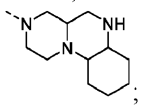
(9) when R₆ and R₇ together with the N atom to which they are attached can form a 4- to 7-membered heterocycle, the 4- to 7-membered heterocycle is 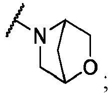 and
(10) in R₅, R₆, and R₇, the 6- to 10-membered aryl is independently phenyl.

6. The compound of formula I, the *cis-trans* isomer thereof, the enantiomer thereof, the diastereomer thereof, the racemate thereof, the solvate thereof, the hydrate thereof, the pharmaceutically acceptable salt thereof, or the prodrug thereof according to at least one of claims 1 to 3, wherein it satisfies one or more than one of the following conditions:
(1) each R₁ is independently hydrogen, halogen, cyano, C₁₋₃ alkyl, C₁₋₃ alkoxy, or C₃₋₆ cycloalkyl, and the C₁₋₃ alkyl, C₁₋₃ alkoxy, or C₃₋₆ cycloalkyl is optionally substituted by 1 to 3 R';
(2) k is 0, 1, or 2;
(3) R₂ is selected from hydrogen, halogen, cyano, C₁₋₃ alkyl, C₃₋₆ cycloalkyl, or 5- to 6-membered heteroaryl, each of which is optionally substituted by 1 to 3 R';
(4) when T₁ is a nitrogen atom, T₂ is a carbon atom, 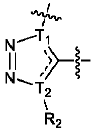 is 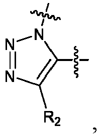 R₂ is selected from hydrogen, halogen, cyano, C₁₋₃ alkyl, C₁₋₃ alkoxy, C₁₋₆ alkylamino, C₃₋₆ cycloalkyl, or 5-to 6-membered heteroaryl, each of which is optionally substituted by 1 to 3 R';
(5) when T₁ is a carbon atom, T₂ is a nitrogen atom, 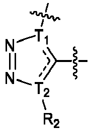 is 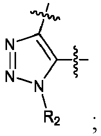 R₂ is selected from C₁₋₃ alkyl, C₃₋₆ cycloalkyl, or 5- to 6-membered heteroaryl, each of which is optionally substituted by 1 to 3 R';
(6) each R₃ is independently hydrogen, halogen, cyano, C₁₋₃ alkyl, or C₁₋₃ alkoxy, each of which is optionally substituted by 1 to 3 R';
(7) m is 0 or 1;
(8) each R₄ is independently selected from hydrogen, halogen, cyano, =O, C₁₋₃ alkyl, C₁₋₃ alkoxy, C₁₋₆ alkylamino, C₃₋₆ cycloalkyl, 3- to 6-membered heterocycloalkyl, phenyl, phenyl(C₁₋₃)alkyl, 5- to 10-membered heteroaryl, 5- to 10-membered heteroaryl(C₁₋₃)alkyl, - COOH, -CH₂-C(O)NR₆R₇, -C(O)NR₆R₇, -SO₂R₆, or -SO₂NR₆R₇; the C₁₋₃ alkyl, C₁₋₃ alkoxy, C₁₋₆ alkylamino, C₃₋₆ cycloalkyl, 3- to 6-membered heterocycloalkyl, phenyl, phenyl(C₁₋₃)alkyl, 5- to 10-membered heteroaryl, or 5- to 10-membered heteroaryl(C₁₋₃)alkyl is optionally substituted by 1 to 3 R; when R₄ is selected from -C(O)NR₆R₇ or -SO₂NR₆R₇, R₆ and R₇ together with the N atom to which they are attached can form a 4- to 7-membered heterocycle, and the 4- to 7-membered heterocycle is optionally substituted by 1 to 3 R;
(9) R₆ and R₇ are each independently hydrogen, C₁₋₆ alkyl, C₃₋₆ cycloalkyl, 3- to 6-membered heterocycloalkyl, phenyl, phenyl(C₁₋₃)alkyl, 5- to 10-membered heteroaryl, or 5- to 10-membered heteroaryl(C₁₋₃)alkyl, each of which is optionally substituted by 1 to 3 R;
(10) each R is independently selected from hydrogen, halogen, cyano, hydroxyl, amino, - COOH, C₁₋₃ alkyl, C₁₋₃ alkoxy, C₁₋₆ alkylamino, C₃₋₆ cycloalkyl, or 3- to 6-membered heterocycloalkyl, and the C₁₋₃ alkyl, C₁₋₃ alkoxy, C₁₋₆ alkylamino, C₃₋₆ cycloalkyl, or 3- to 6-membered heterocycloalkyl is optionally substituted by 1 to 3 R';
(11) n is 0, 1, 2, 3, or 4;
(12) ring A is a benzene ring, a pyridine ring, or a pyrimidine ring;
(13) L is -CHz-O-;
(14) ring B is a benzene ring, a pyridine ring, a pyridazine ring, a pyrazine ring, or a pyridopyridazine ring; and
(15) each R₃ is independently H, F, Cl, CN, Me, or -OCH₃.

7. The compound of formula I, the *cis-trans* isomer thereof, the enantiomer thereof, the diastereomer thereof, the racemate thereof, the solvate thereof, the hydrate thereof, the pharmaceutically acceptable salt thereof, or the prodrug thereof according to at least one of claims 1 to 3, wherein it satisfies one or more than one of the following conditions:
(1) each R₁ is independently halogen, cyano, C₁₋₃ alkyl, or C₁₋₃ alkoxy, and the C₁₋₃ alkyl and/or C₁₋₃ alkoxy are optionally substituted by 1 to 3 R'; each R' is independently hydrogen, halogen, hydroxyl, cyano, or methoxy; for example, each R' is independently hydrogen, halogen, or hydroxyl;
(2) k is 1;
(3) R₂ is selected from hydrogen, halogen, cyano, C₁₋₃ alkyl, or C₃₋₆ cycloalkyl, each of which is optionally substituted by 1 to 3 R';
(4) when T₁ is a nitrogen atom, T₂ is a carbon atom, 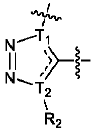 is 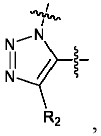 R₂ is selected from hydrogen, halogen, cyano, C₁₋₃ alkyl, C₁₋₃ alkyl substituted by cyclopropyl or hydroxyl, or 5- to 6-membered heteroaryl substituted by C₁₋₃ alkyl;
(5) when T₁ is a carbon atom, T₂ is a nitrogen atom, 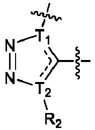 is 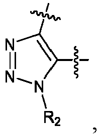 R₂ is selected from Me or CHF₂; preferably, R₂ is Me;
(6) m is 0;
(7) each R₄ is independently selected from the following substituents: hydrogen, cyano, =O, C₁₋₃ alkyl, C₁₋₃ alkoxy, C₁₋₆ alkylamino, C₃₋₆ cycloalkyl, 3- to 6-membered heterocycloalkyl, phenyl, phenyl(C₁₋₃)alkyl, 5- to 10-membered heteroaryl, 5- to 10-membered heteroaryl(C₁₋₃)alkyl, -COOH, -C(O)NR₆R₇, -SO₂R₆, or -SO₂NR₆R₇; the C₁₋₃ alkyl, C₁₋₃ alkoxy, C₁₋₆ alkylamino, C₃₋₆ cycloalkyl, 3- to 6-membered heterocycloalkyl, phenyl, phenyl(C₁₋₃)alkyl, 5- to 10-membered heteroaryl, or 5- to 10-membered heteroaryl(C₁₋₃)alkyl is optionally substituted by 1 to 3 R; when R₄ is selected from -C(O)NR₆R₇ or -SO₂NR₆R₇, R₆ and R₇ together with the N atom to which they are attached can form a 4- to 7-membered heterocycle, and the 4- to 7-membered heterocycle is optionally substituted by 1 to 3 R;
(8) each R is independently selected from hydrogen, halogen, cyano, hydroxyl, amino, C₁₋₃ alkyl, C₁₋₃ alkoxy, C₁₋₆ alkylamino, C₃₋₆ cycloalkyl, or 3- to 6-membered heterocycloalkyl, and the C₁₋₃ alkyl, C₁₋₃ alkoxy, C₁₋₆ alkylamino, C₃₋₆ cycloalkyl, or 3- to 6-membered heterocycloalkyl is optionally substituted by 1 to 3 R';
(9) n is 1 or 2;
(10) ring A is a benzene ring or a pyridine ring;
(11) ring B is a pyridine ring, a pyridazine ring, a pyrazine ring, or a pyridopyridazine ring; for example, ring B is a pyridine ring, a pyridazine ring, or a pyridopyridazine ring; and
(12) each R₃ is independently H, CN, Me, or -OCH₃.

8. The compound of formula I, the *cis-trans* isomer thereof, the enantiomer thereof, the diastereomer thereof, the racemate thereof, the solvate thereof, the hydrate thereof, the pharmaceutically acceptable salt thereof, or the prodrug thereof according to at least one of claims 1 to 3, wherein it satisfies one or more than one of the following conditions:
(1) when T₁ is a nitrogen atom, T₂ is a carbon atom, and R₂ is selected from H, F, Cl, CN, Me, CHF₂, CF₃, -CHzOH, -CH₂OCH₃, 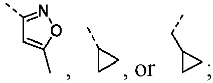 preferably, R₂ is selected from H, Cl, Me, CHzOH, CN, CHF₂, CF₃, 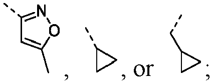 and
(2) each R₁ is independently H, F, Cl, Me, CN, -CH₂F, -CF₂H, CF₃, -OCF₂H, -CH(CN)₂, -CH₂OH, -CH₂OCH₃, or 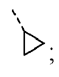 ; preferably, each R₁ is independently F, Cl, Me, -CH₂F, -CF₂H, CF₃, CN, -CH(CN)₂, -CH₂OH, -CH₂OCH₃, or -OCF₂H.

9. The compound of formula I, the *cis-trans* isomer thereof, the enantiomer thereof, the diastereomer thereof, the racemate thereof, the solvate thereof, the hydrate thereof, the pharmaceutically acceptable salt thereof, or the prodrug thereof according to at least one of claims 1 to 3, wherein it satisfies one or more than one of the following conditions:
(1) the structural moiety 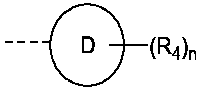 is structural moiety (II) 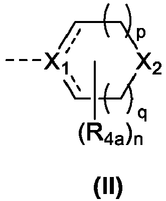 wherein
X₁ is selected from N or C; 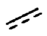
is 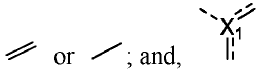 is selected from 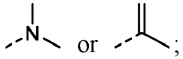
X₂ is a carbon atom, -NR_{4c}-, -O-, or -S(O)_{w}-, the carbon atom is substituted by one or two independent R_{4b}, and each R_{4b} is independently selected from hydrogen, -OR₆, -NR₆R₇, - NR₆COR₅, -C(O)NR₆R₇, or -SO₂NR₆R₇;
R₅ is C₁₋₃ alkyl, C₃₋₆ cycloalkyl, 3- to 6-membered heterocycloalkyl, or 5- to 10-membered heteroaryl, each of which is optionally substituted by 1 to 3 R';
R₆ and R₇ are each independently hydrogen, C₁₋₃ alkyl, C₃₋₆ cycloalkyl, 3- to 6-membered heterocycloalkyl, phenyl, phenyl(C₁₋₃)alkyl, 5- to 10-membered heteroaryl, or 5- to 10-membered heteroaryl(C₁₋₃)alkyl, each of which is optionally substituted by 1 to 3 R;
when R_{4b} is -NR₆R₇, -C(O)NR₆R₇, or -SO₂NR₆R₇, R₆ and R₇ together with the N atom to which they are attached can form a 4- to 7-membered heterocycle, the heterocycle can contain 1 to 3 heteroatoms as ring atoms, the heteroatoms can be independently selected from N, O, and S atoms, and the 4- to 7-membered heterocycle is optionally substituted by 1 to 3 R;
each R_{4c} is independently hydrogen, C₁₋₃ alkyl, C₃₋₆ cycloalkyl, 3- to 6-membered heterocycloalkyl, phenyl, phenyl(C₁₋₃)alkyl, 5- to 10-membered heteroaryl, or 5- to 10-membered heteroaryl(C₁₋₃)alkyl, each of which is optionally substituted by 1 to 3 R;
each R is independently hydrogen, halogen, cyano, hydroxyl, amino, C₁₋₃ alkyl, C₁₋₃ alkoxy, C₁₋₆ alkylamino, C₃₋₆ cycloalkyl, or 3- to 6-membered heterocycloalkyl, and the C₁₋₃ alkyl, C₁₋₃ alkoxy, C₁₋₆ alkylamino, C₃₋₆ cycloalkyl, or 3- to 6-membered heterocycloalkyl is optionally substituted by 1 to 3 R';
each R₄ₐ is independently hydrogen, oxo, cyano, C₁₋₃ alkyl, -COOR₆, -CH₂-C(O)NR₆R₇, -C(O)NR₆R₇, C₃₋₆ cycloalkyl, 3- to 6-membered heterocycloalkyl, or 5- to 6-membered heteroaryl; the C₁₋₃ alkyl, C₃₋₆ cycloalkyl, 3- to 6-membered heterocycloalkyl, or 5- to 6-membered heteroaryl is optionally substituted by 1 to 3 R', and the R' is as defined in at least one of claims 1 to 2, 4 to 5, and 7;
or two R₄ₐ attached to the same carbon atom together form a 3- to 6-membered saturated heterocycle, the heterocycle can contain 1 to 3 heteroatoms as ring atoms, the heteroatoms can be independently selected from N, O, and S atoms, and the 3- to 6-membered heterocycle is optionally substituted by 1 to 3 R;
n is 0, 1, 2, 3, or 4;
p and q are each independently 0, 1, or 2, and p and q are not both 2;
w is 0, 1, or 2;
(2) the structural moiety 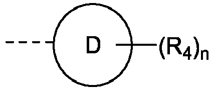 is structural moiety (III) 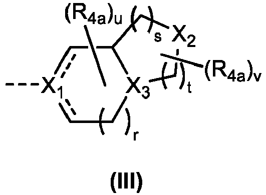 wherein
X₁ is N or C; 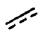
is 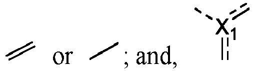 is 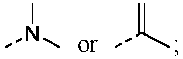
X₂ is a carbon atom substituted by one or two independent R_{4b}, -NR_{4c}-, -O-, or -S(O)_{w}-, and the R_{4b} is hydrogen, -OR₆, -NR₆R₇, -NR₆COR₅, -NR₆SO₂R₅, -C(O)NR₆R₇, -SO₂R₅, or - SO₂NR₆R₇;
R₅ is C₁₋₃ alkyl, C₃₋₆ cycloalkyl, 3- to 6-membered heterocycloalkyl, or 5- to 10-membered heteroaryl, each of which is optionally substituted by 1 to 3 R';
R₆ and R₇ are each independently hydrogen, C₁₋₃ alkyl, C₃₋₆ cycloalkyl, 3- to 6-membered heterocycloalkyl, phenyl, phenyl(C₁₋₃)alkyl, 5- to 10-membered heteroaryl, or 5- to 10-membered heteroaryl(C₁₋₃)alkyl, each of which is optionally substituted by 1 to 3 R;
when R_{4b} is -NR₆R₇, -C(O)NR₆R₇, or -SO₂NR₆R₇, R₆ and R₇ together with the N atom to which they are attached can form a 4- to 7-membered heterocycle, the heterocycle can contain 1 to 3 heteroatoms as ring atoms, the heteroatoms can be independently selected from N, O, and S atoms, and the 4- to 7-membered heterocycle is optionally substituted by 1 to 3 R;
the R_{4c} is hydrogen, C₁₋₃ alkyl, C₃₋₆ cycloalkyl, 3- to 6-membered heterocycloalkyl, phenyl, phenyl(C₁₋₃)alkyl, 5- to 10-membered heteroaryl, or 5- to 10-membered heteroaryl(C₁₋₃)alkyl, each of which is optionally substituted by 1 to 3 R;
each R is independently hydrogen, halogen, cyano, hydroxyl, amino, C₁₋₃ alkyl, C₁₋₃ alkoxy, C₁₋₆ alkylamino, C₃₋₆ cycloalkyl, or 3- to 6-membered heterocycloalkyl, and the C₁₋₃ alkyl, C₁₋₃ alkoxy, C₁₋₆ alkylamino, C₃₋₆ cycloalkyl, or 3- to 6-membered heterocycloalkyl is optionally substituted by 1 to 3 R';
X₃ is CR₄ₐ or N;
each R₄ₐ is independently hydrogen, oxo, cyano, C₁₋₃ alkyl, C₃₋₆ cycloalkyl, 3- to 6-membered heterocycloalkyl, phenyl, or 5- to 6-membered heteroaryl; the C₁₋₃ alkyl, C₃₋₆ cycloalkyl, 3- to 6-membered heterocycloalkyl, phenyl, or 5- to 6-membered heteroaryl is optionally substituted by 1 to 3 R', and the R' is as defined in at least one of claims 1 to 2, 4 to 5, and 7;
u and v are each independently 0, 1, 2, 3, or 4;
r, s, and t are each independently 0, 1, or 2;
w is 0, 1, or 2;
(3) the structural moiety 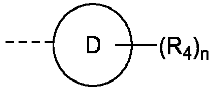 is structural moiety (IV) 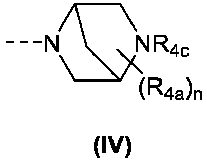 wherein
R_{4c} is hydrogen, C₁₋₃ alkyl, C₃₋₆ cycloalkyl, 3- to 6-membered heterocycloalkyl, phenyl, phenyl(C₁₋₃)alkyl, 5- to 10-membered heteroaryl, or 5- to 10-membered heteroaryl(C₁₋₃)alkyl, each of which is optionally substituted by 1 to 3 R;
each R is independently hydrogen, halogen, cyano, hydroxyl, amino, C₁₋₃ alkyl, C₁₋₃ alkoxy, C₁₋₆ alkylamino, C₃₋₆ cycloalkyl, or 3- to 6-membered heterocycloalkyl, and the C₁₋₃ alkyl, C₁₋₃ alkoxy, C₁₋₆ alkylamino, C₃₋₆ cycloalkyl, or 3- to 6-membered heterocycloalkyl is optionally substituted by 1 to 3 R';
each R₄ₐ is independently hydrogen, cyano, oxo, C₁₋₃ alkyl, C₃₋₆ cycloalkyl, 3- to 6-membered heterocycloalkyl, or 5- to 6-membered heteroaryl; the C₁₋₃ alkyl, C₃₋₆ cycloalkyl, 3-to 6-membered heterocycloalkyl, or 5- to 6-membered heteroaryl is optionally substituted by 1 to 3 R', and the R' is as defined in at least one of claims 1 to 2, 4 to 5, and 7;
n is selected from 0, 1, 2, 3, or 4;
(4) the structural moiety 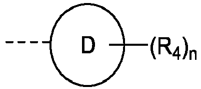 is structural moiety (V) 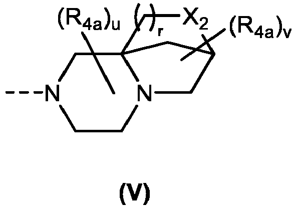 wherein
X₂ is a carbon atom substituted by one or two independent R₄ₐ or -O-,
each R₄ₐ is independently hydrogen, cyano, C₁₋₃ alkyl, C₃₋₆ cycloalkyl, 3- to 6-membered heterocycloalkyl, or 5- to 6-membered heteroaryl, or two R₄ₐ attached to the same carbon atom together form an oxo(C=O) group; the C₁₋₃ alkyl, C₃₋₆ cycloalkyl, 3- to 6-membered heterocycloalkyl, or 5- to 6-membered heteroaryl is optionally substituted by 1 to 3 R', and the R' is as defined in at least one of claims 1 to 2, 4 to 5, and 7;
u and v are each independently selected from 0, 1, 2, 3, or 4;
r is selected from 0 or 1; and
(5) the structural moiety 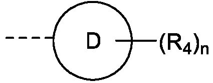 is structural moiety (VI) 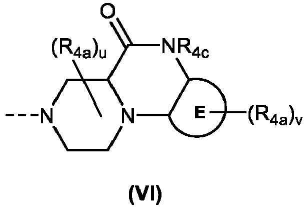 wherein
ring E is a 5- to 6-membered saturated cycloalkyl ring, a benzene ring, or a 5- to 6-membered heteroaryl ring;
each R₄ₐ is independently hydrogen, oxo, halogen, cyano, C₁₋₃ alkyl, C₃₋₆ cycloalkyl, 3- to 6-membered heterocycloalkyl, or 5- to 6-membered heteroaryl; the C₁₋₃ alkyl, C₃₋₆ cycloalkyl, 3- to 6-membered heterocycloalkyl, or 5- to 6-membered heteroaryl is optionally substituted by 1 to 3 R', and the R' is as defined in at least one of claims 1 to 2, 4 to 5, and 7;
R_{4c} is hydrogen, C₁₋₃ alkyl, C₃₋₆ cycloalkyl, or 3- to 6-membered heterocycloalkyl; preferably, R_{4c} is hydrogen;
u and v are each independently 0, 1, 2, 3, or 4.

10. The compound of formula I, the *cis-trans* isomer thereof, the enantiomer thereof, the diastereomer thereof, the racemate thereof, the solvate thereof, the hydrate thereof, the pharmaceutically acceptable salt thereof, or the prodrug thereof according to claim 9, wherein it satisfies one or more than one of the following conditions:
(1) the structural moiety (II) 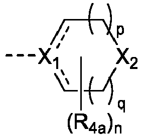 is 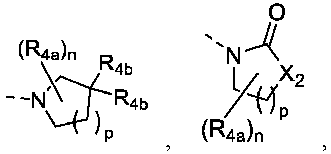 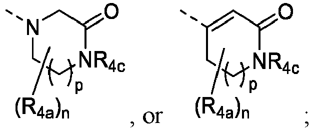
(2) the structural moiety (III) 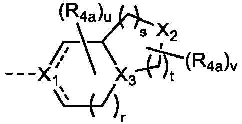 is 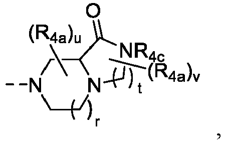 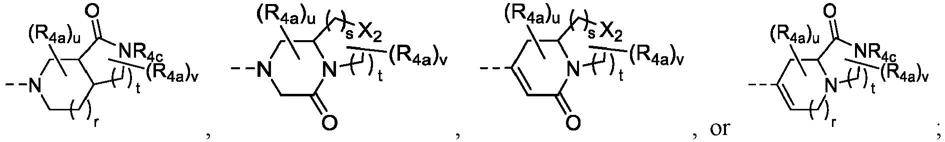 preferably 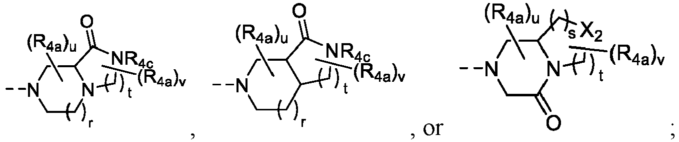
(3) the structural moiety (V) 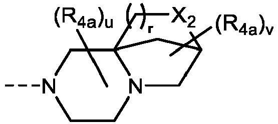 is 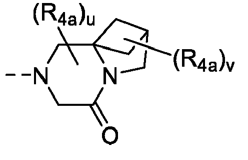 or 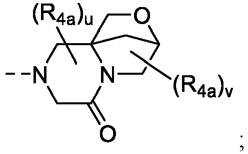
(4) the structural moiety (VI) 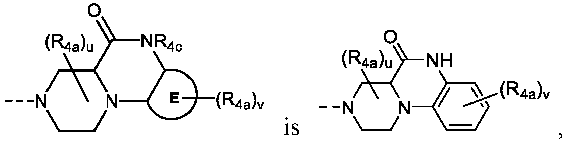 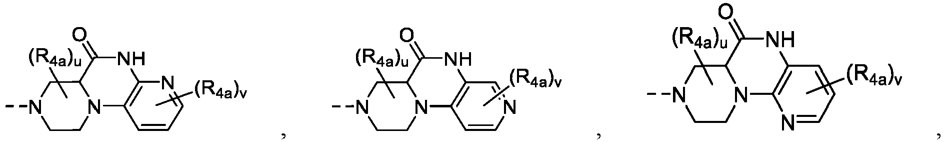 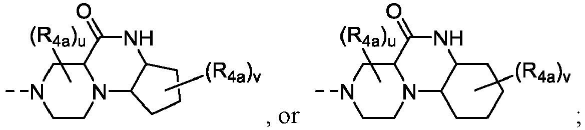
(5) each R₄ₐ is independently H, =O, -F, -Cl, -Me, -i-Pr, -t-Bu, -CH₂CH(CH₃)₂, -COOH, - CN, -CH₂CN, -CH₂OH, -(CH₂)₂OH, -CH₂OCH₃, -CONH₂, -CONHCH₃, -CON(CH₃)₂, - CONHCH₂CH₃, -CH₂CONHCH₂CH₃, 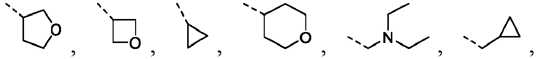 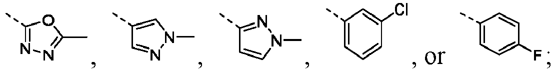
(6) each R_{4b} is independently H, =O, -OCH₃, -NMe₂, -NHCOCH₃, -NHSO₂CH₃, -SO₂Me, -COOH, -CONH₂, -CONHCH₃, -CON(CH₃)₂, -CONHCH₂CH₃, -SO₂NHCH₃, 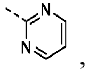 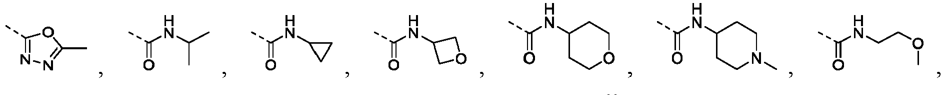 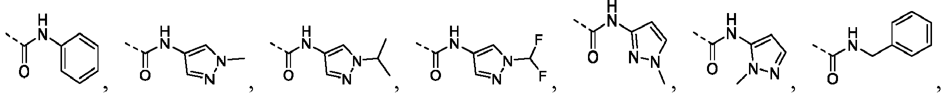 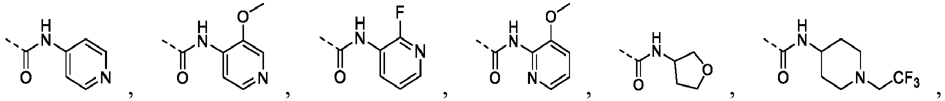 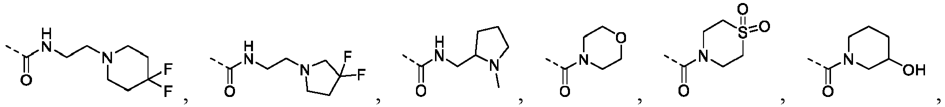 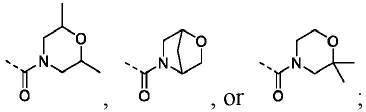 and
(7) each R_{4c} is independently H, -Me, -Et, -*i*-Pr, -CF₃, -CHF₂, -CH₂CF₃, -CH₂CF₂H, - CH₂CN, -(CH₂)₂CN, -(CH₂)₃CN, -(CH₂)₂NH₂, -(CH₂)₂OH, -(CH₂)₂OCH₃, -(CH₂)₃OCH₃, - COCH₃, -COCH(CH₃)₂, -SO₂NHCH₃, -SO₂CH₃,

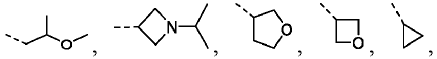

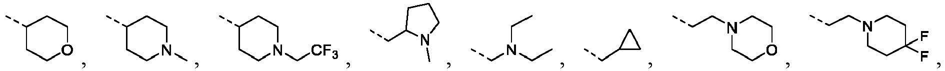

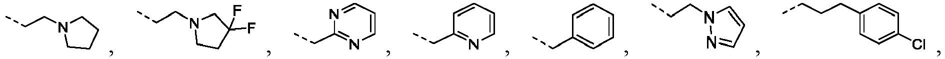

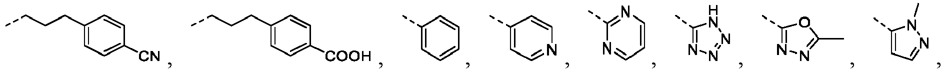

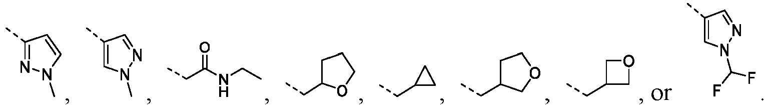

11. The compound of formula I, the *cis-trans* isomer thereof, the enantiomer thereof, the diastereomer thereof, the racemate thereof, the solvate thereof, the hydrate thereof, the pharmaceutically acceptable salt thereof, or the prodrug thereof according to claim 9, wherein it satisfies one or more than one of the following conditions:
(1) the structural moiety (II) 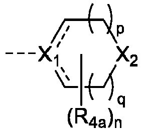 is 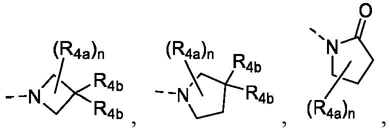 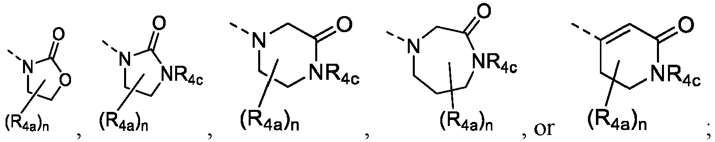
(2) the structural moiety (III) 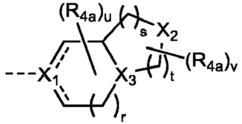 is 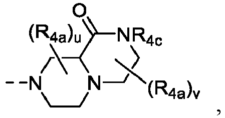 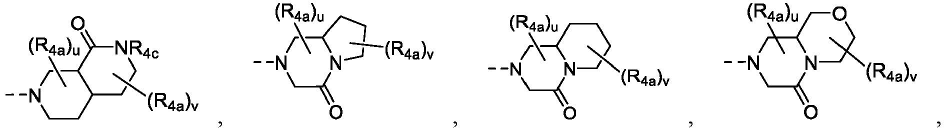 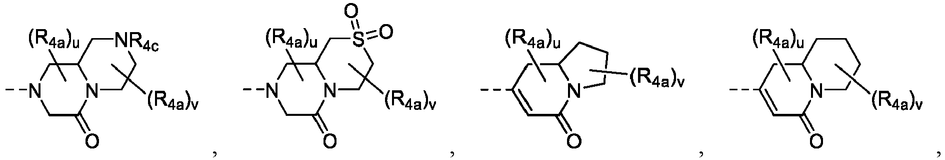 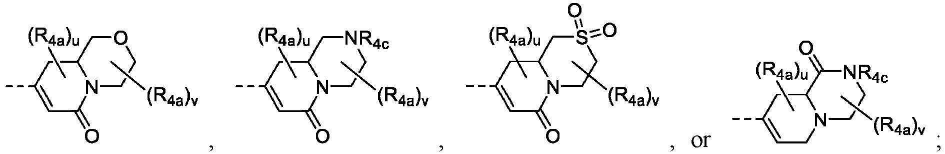 preferably, the structural moiety (III) 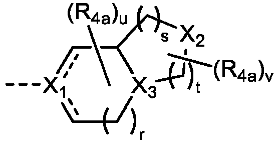 is 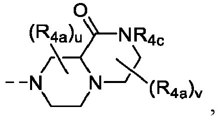 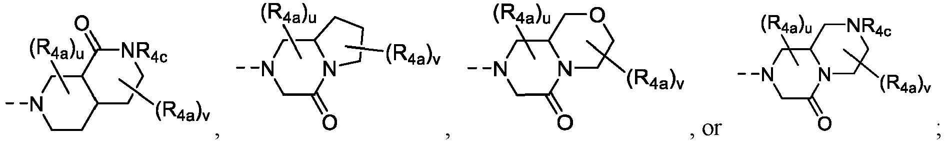
(3) each R₄ₐ is independently H, =O, -F, -Me, -i-Pr, -t-Bu, -CH₂CH(CH₃)₂, -COOH, -CN,
- CH₂OH, -CONH₂, -CONHCH₂CH₃, -CON(CH₃)₂, -CH₂CONHCH₂CH₃, 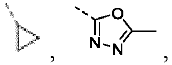 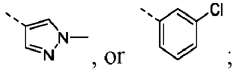
(4) each R_{4b} is independently H, -OCH₃, -NMe₂, -NHCOCH₃, -COOH, -CONHCH₂CH₃, -SO₂NHCH₃, 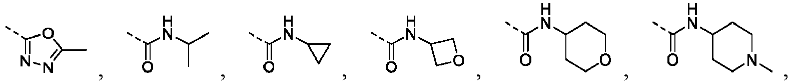 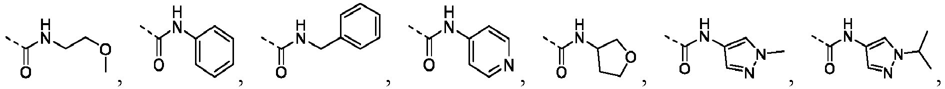 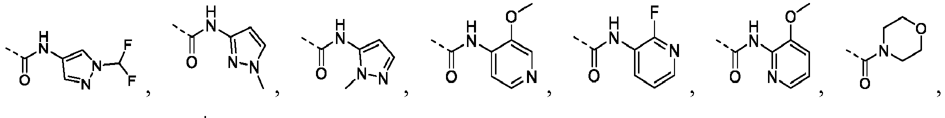 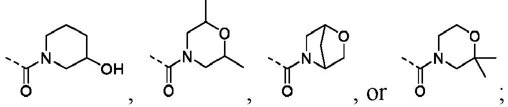 and
(5) each R_{4c} is independently H, -Me, -CH₂CF₃, -CH₂CN, -(CH₂)₃CN, -(CH₂)₂NH₂, - (CH₂)₂OH, -(CH₂)₂OCH₃, -(CH₂)₃OCH₃, -SO₂CH₃, -COCH₃,

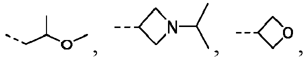

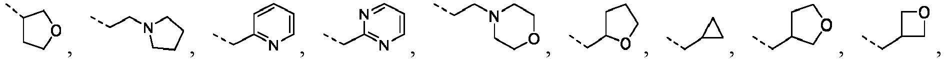

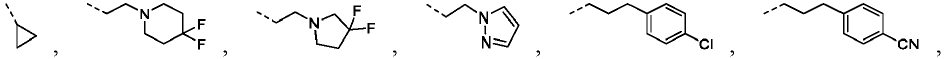

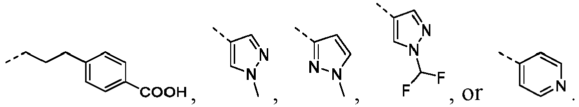

12. The compound of formula I, the *cis-trans* isomer thereof, the enantiomer thereof, the diastereomer thereof, the racemate thereof, the solvate thereof, the hydrate thereof, the pharmaceutically acceptable salt thereof, or the prodrug thereof according to at least one of claims 1 to 3, wherein it satisfies one or more than one of the following conditions:
(1) the structural unit 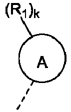 is selected from 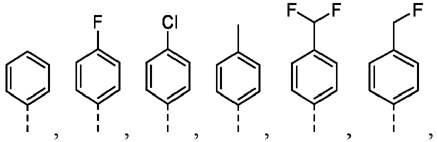 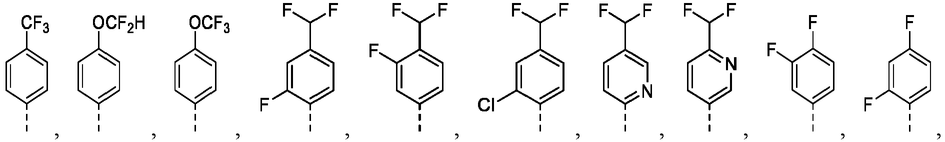 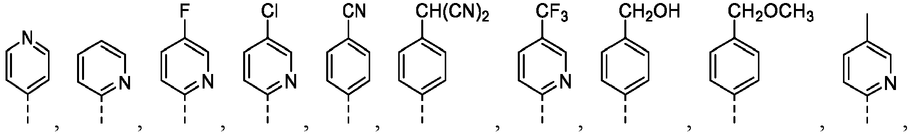 or 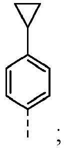 preferably, the structural unit 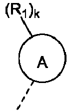 is selected from 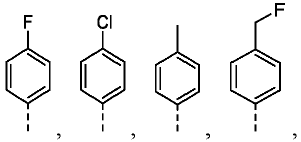 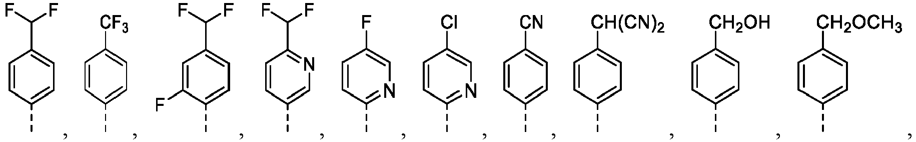 or 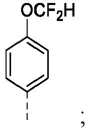
(2) ring D is 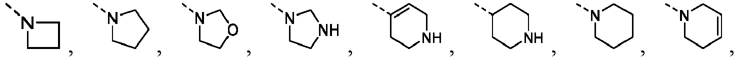 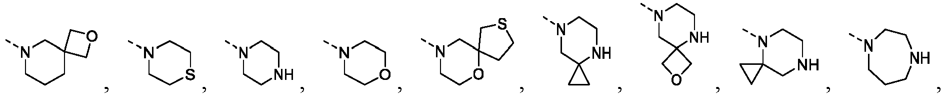 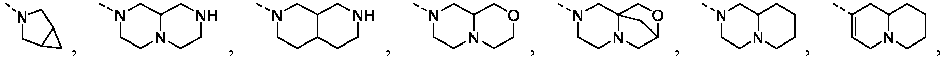 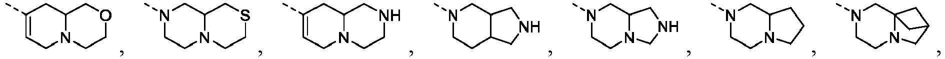 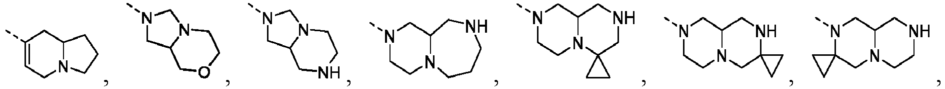 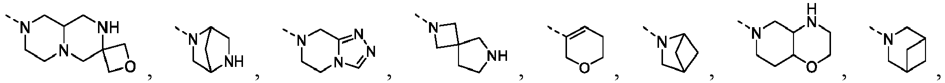 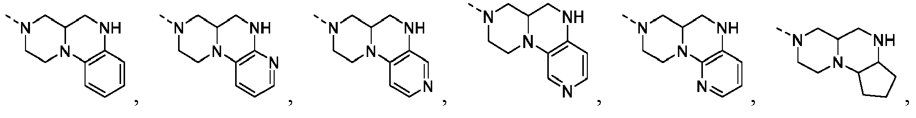 or 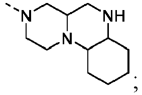 preferably, ring D is 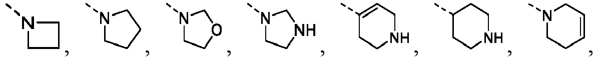 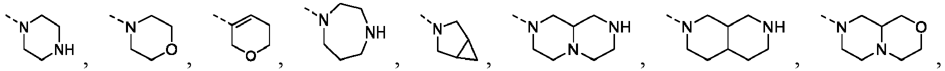 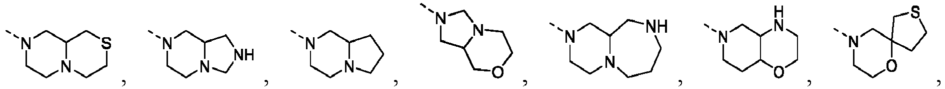 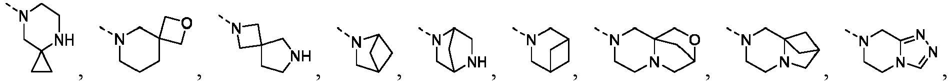 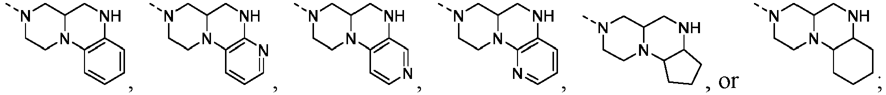 and
(3) each R₄ is independently H, F, Cl, =O, -Me, -COOH, -Et, -i-Pr, -t-Bu, -CH₂CH(CH₃)₂, -CF₃, -CHF₂, -CH₂CF₃, -CH₂CF₂, -CN, -CH₂CN, -(CH₂)₂CN, -(CH₂)₃CN, -CH₂OH, - (CH₂)₂OH, -CH₂OCH₃, -OCH₃, -NMe₂, -NHCOCH₃, -NHSO₂CH₃, -SO₂Me, -CONH₂, - CONHCH₃, -CON(CH₃)₂, -CONHCH₂CH₃, -SO₂NHCH₃, -COCH(CH₃)₂, -CONHCH₂CH₃, - CH₂CONHCH₂CH₃, -(CH₂)₂NH₂, -(CH₂)₂OCH₃, -(CH₂)₃OCH₃, -COCH₃, 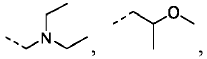 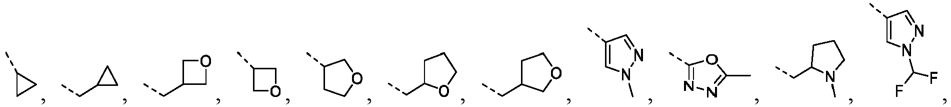 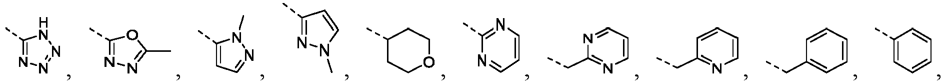 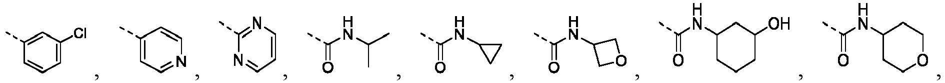 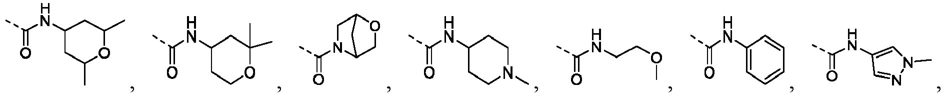 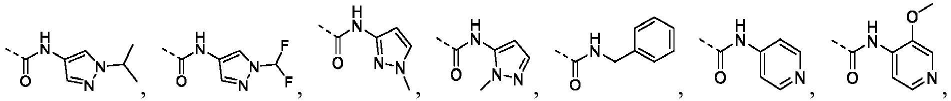 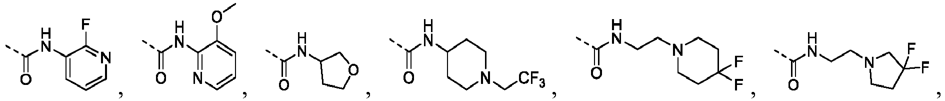 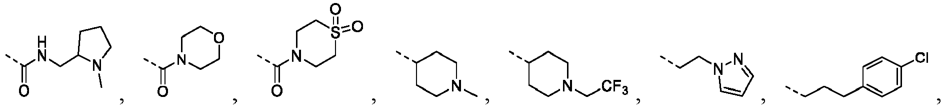 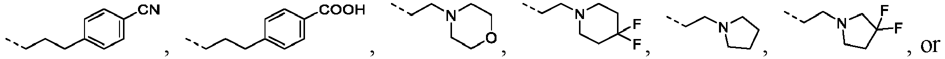 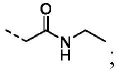 preferably, each R₄ is independently H, F, =O, -Me, -i-Pr, -t-Bu, -CH₂CH(CH₃)₂, - OCH₃, -(CH₂)₂OCH₃, -(CH₂)₃OCH₃, -NMe₂, -COOH, -CN, -CH₂CN, -CH₂OH, -CONH₂, - CONHCH₃, -CONHCH₂CH₃, -CON(CH₃)₂, -CH₂CONHCH₂CH₃, -NHCOCH₃, -CH₂CF₃, - (CH₂)₂NH₂, -(CH₂)₂OH, -SO₂CH₃, -SO₂NHCH₃, -(CH₂)₃CN, -COCH₃,

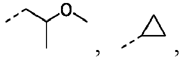

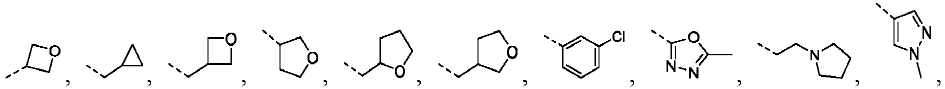

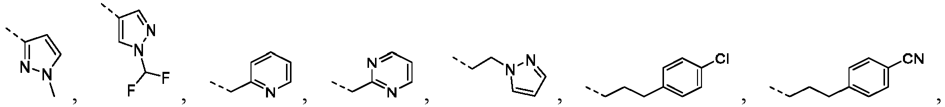

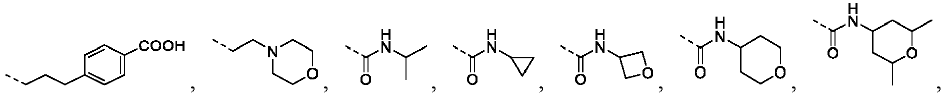

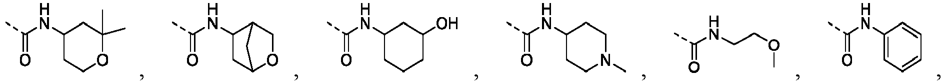

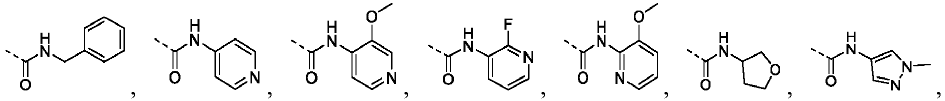

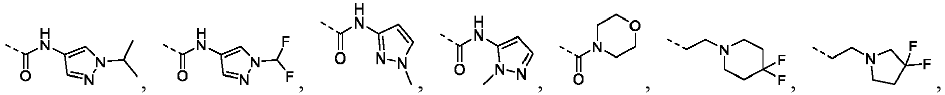

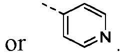

13. The compound of formula I, the *cis-trans* isomer thereof, the enantiomer thereof, the diastereomer thereof, the racemate thereof, the solvate thereof, the hydrate thereof, the pharmaceutically acceptable salt thereof, or the prodrug thereof according to at least one of claims 1 to 3, wherein it satisfies one or more than one of the following conditions:
(1) the structural unit 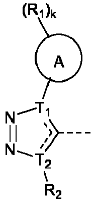 is selected from 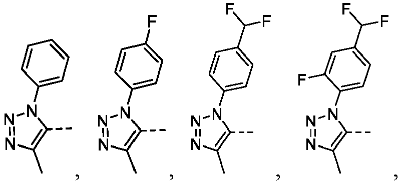 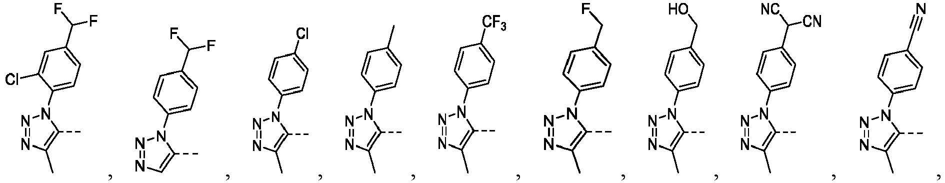 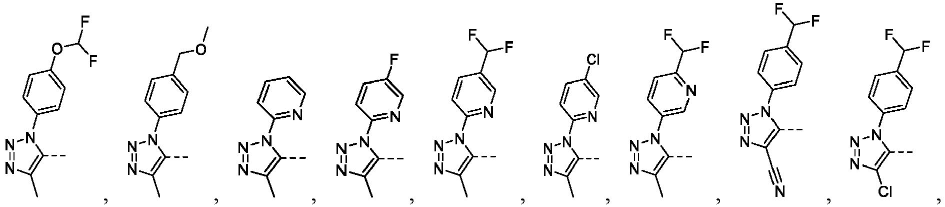 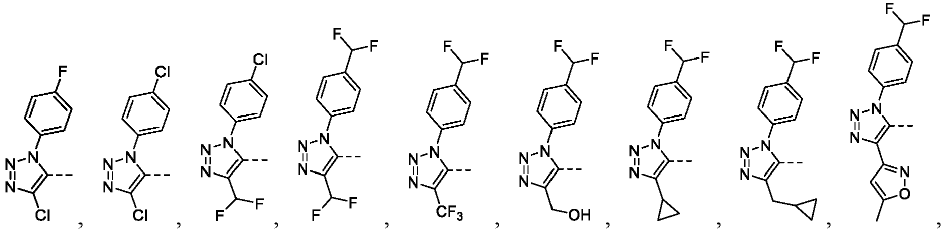 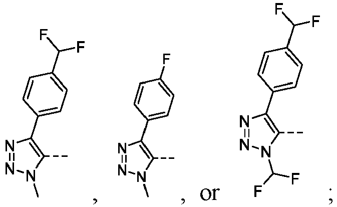 preferably, the structural unit 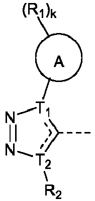 is selected from 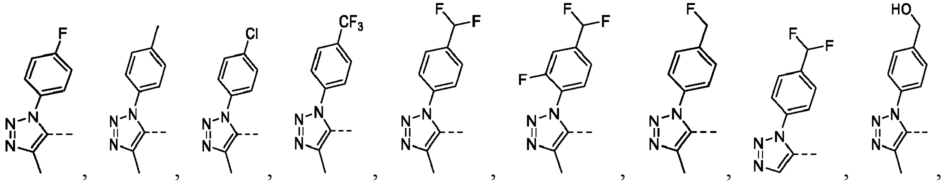 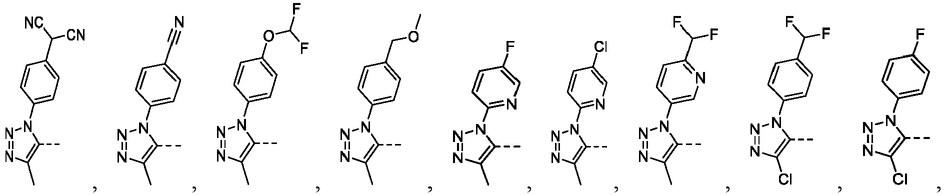 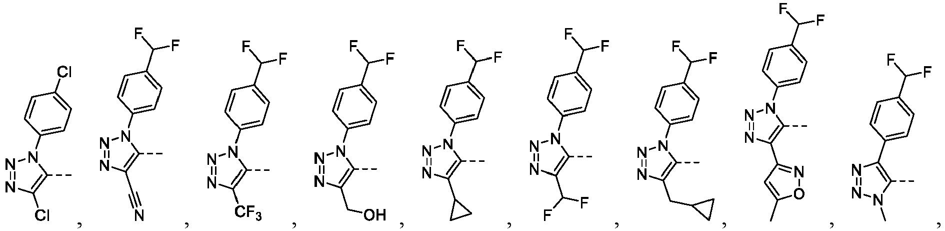 or 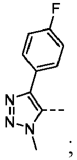
(2) the structural moiety 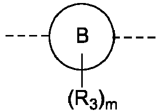 is 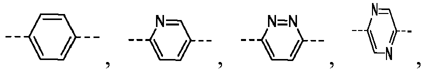 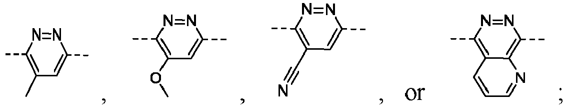 preferably, the structural moiety 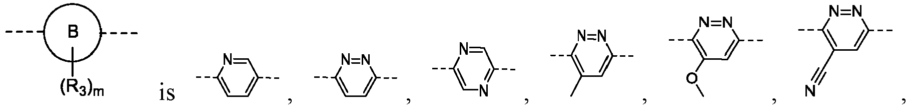 or 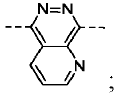
(3) the structural moiety 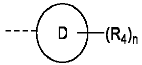 is the following substituents: 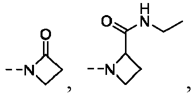 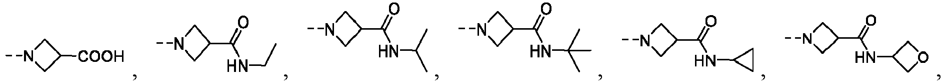 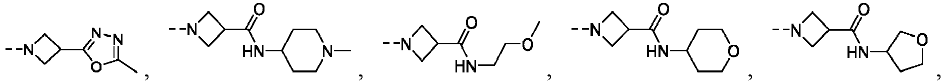 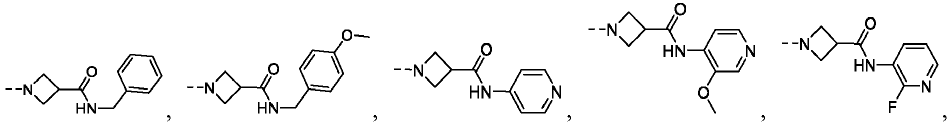 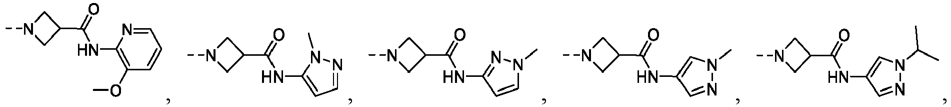 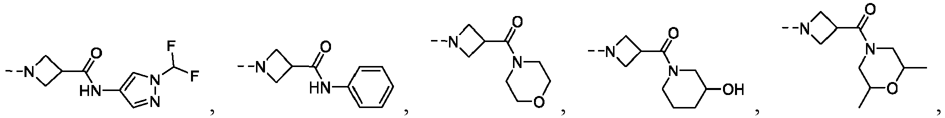 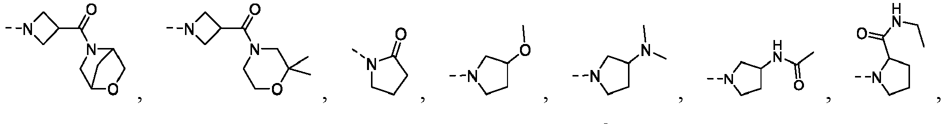 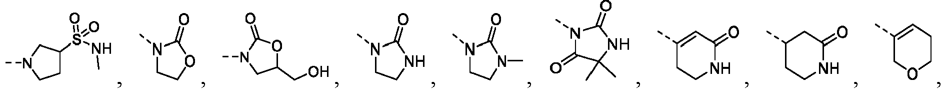 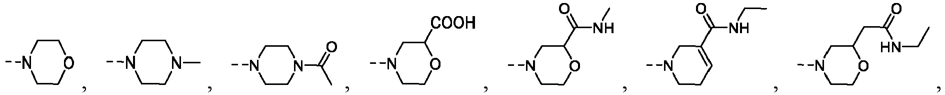 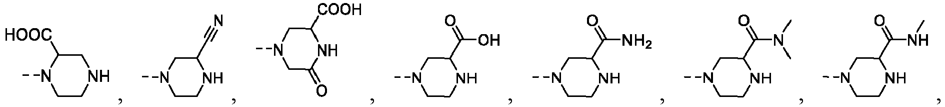 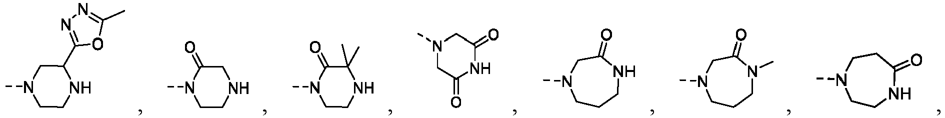 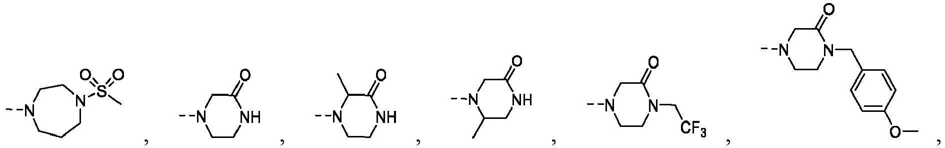 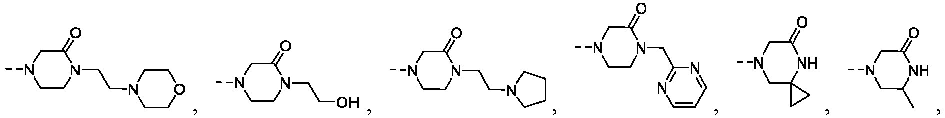 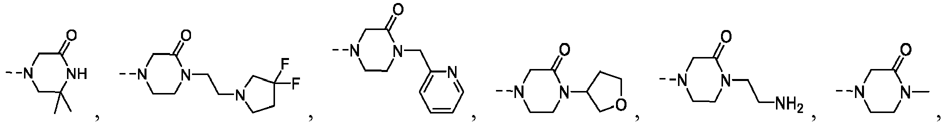 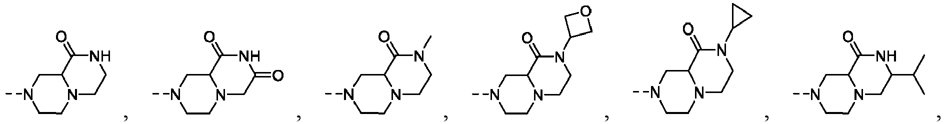 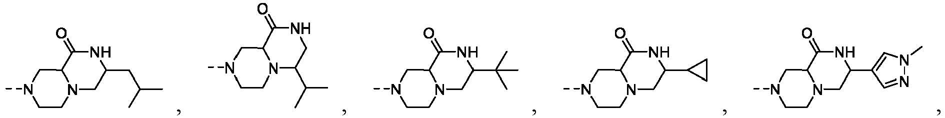 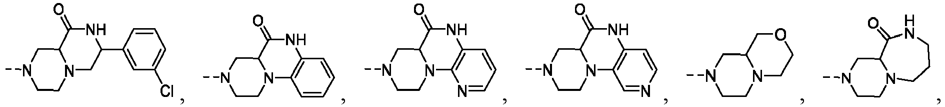 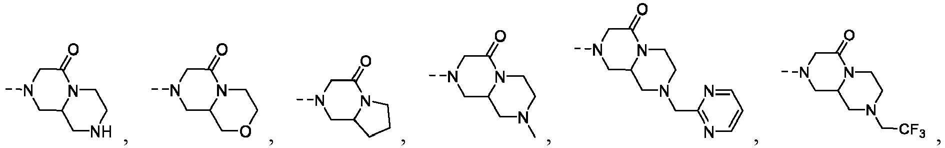 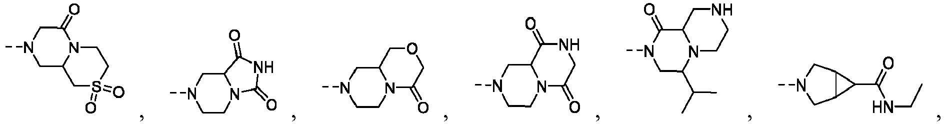 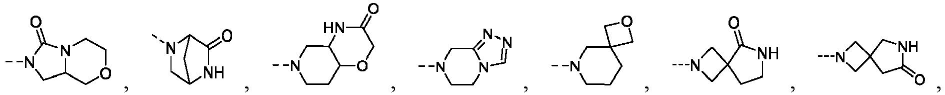 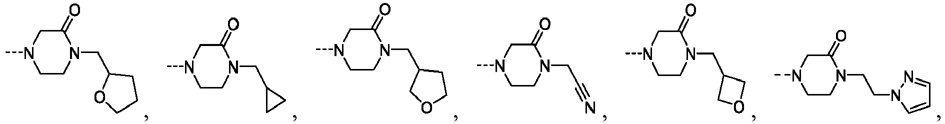 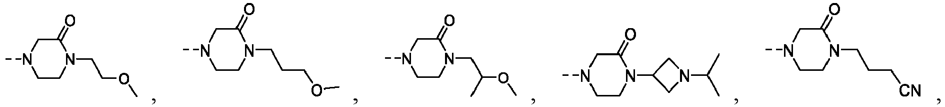 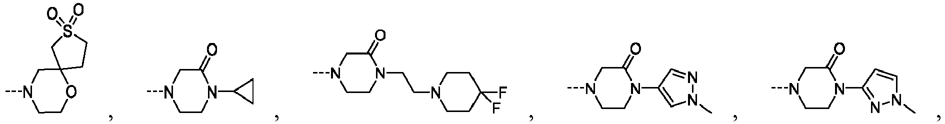 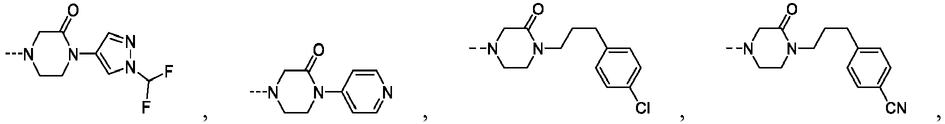 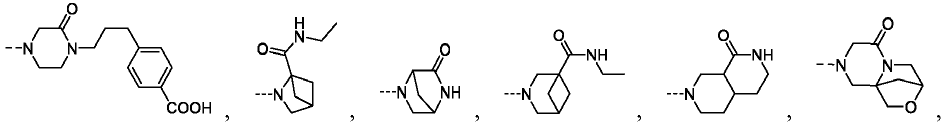 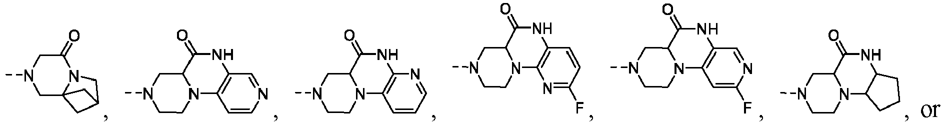 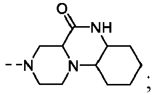 and
(4) preferably, the structural moiety 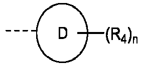 is selected from the following substituents: 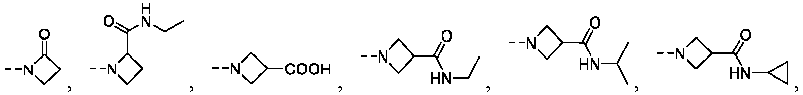 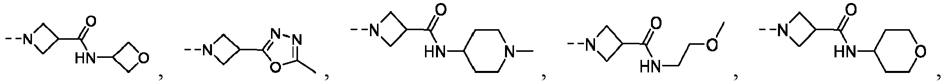 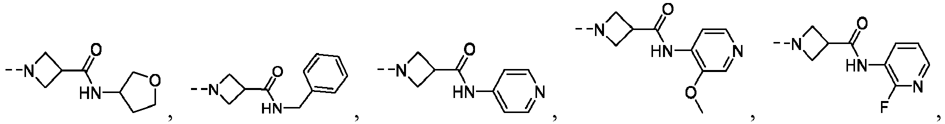 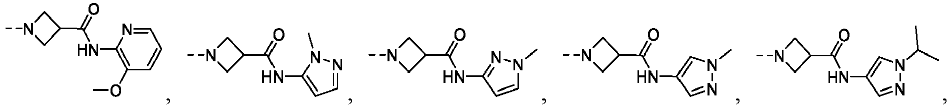 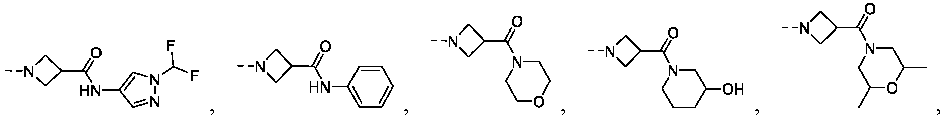 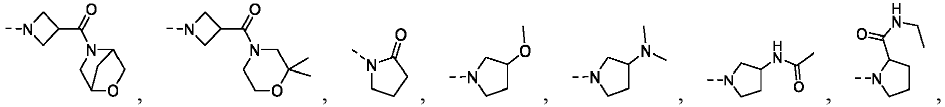 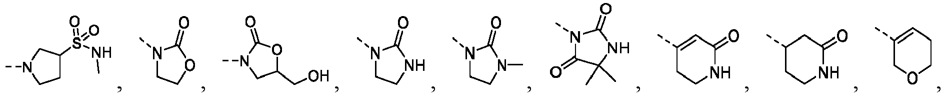 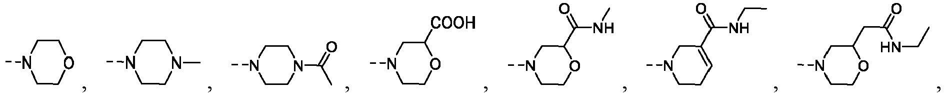 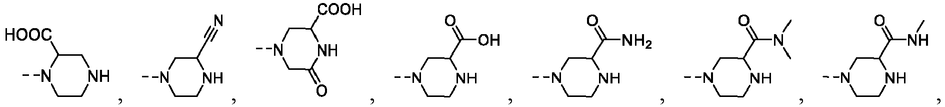 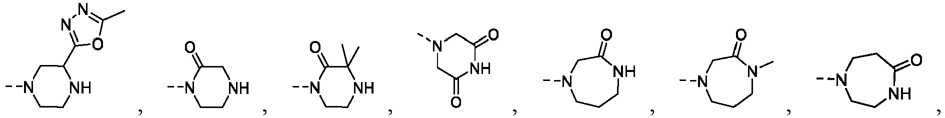 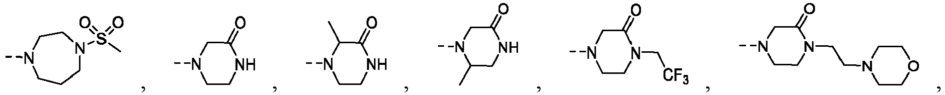 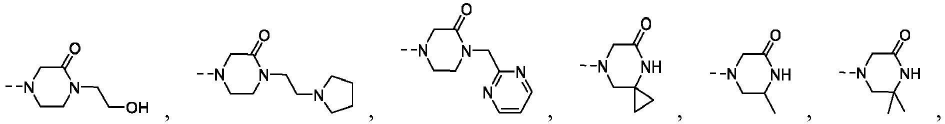 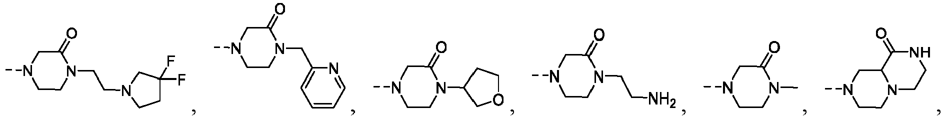 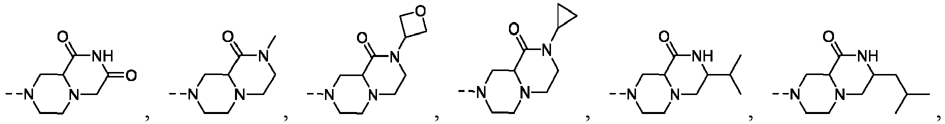 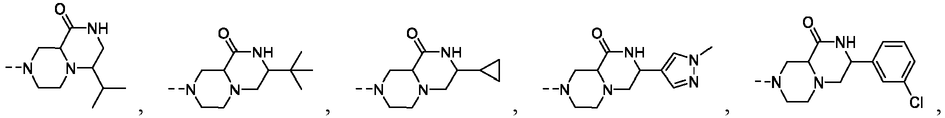 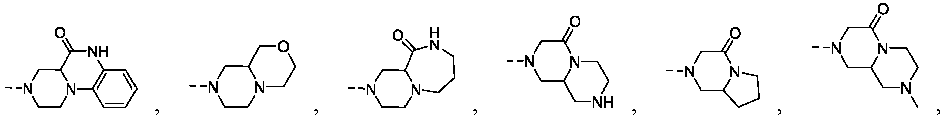 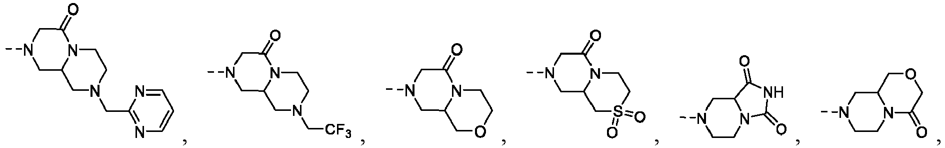 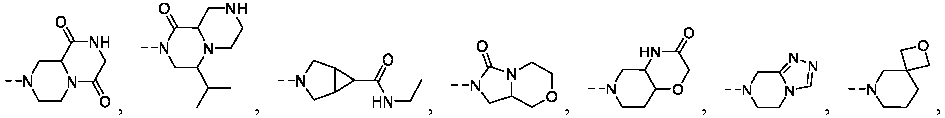 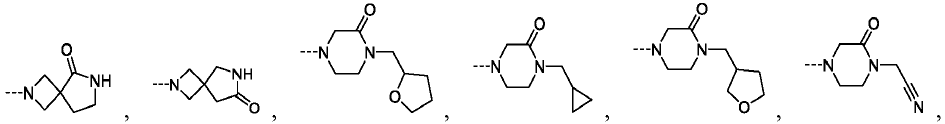 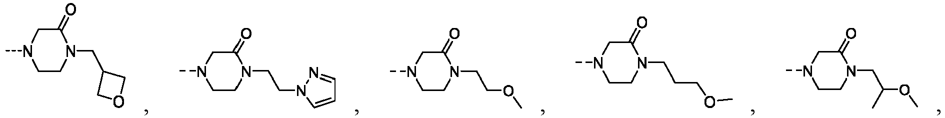 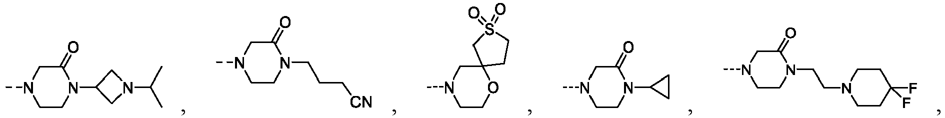 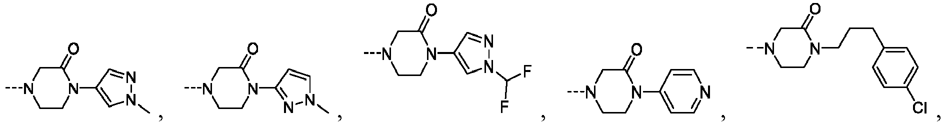 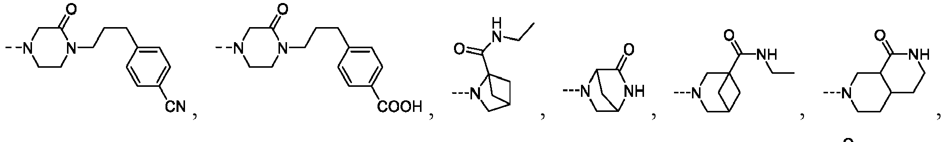 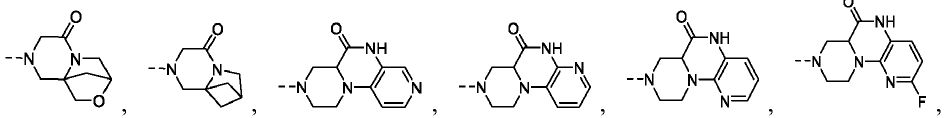 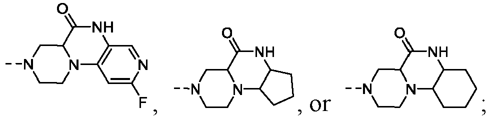 preferably, the structural moiety 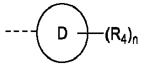 is selected from the following substituents:

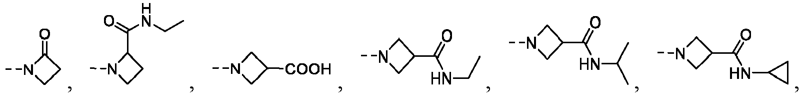

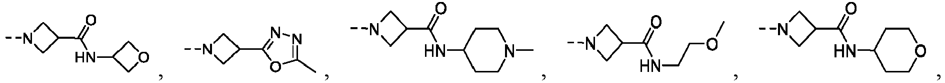

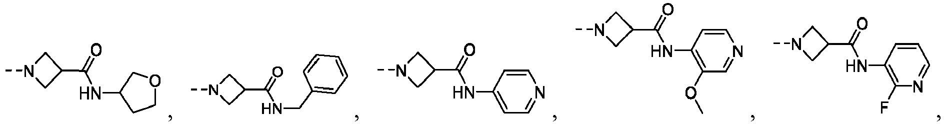

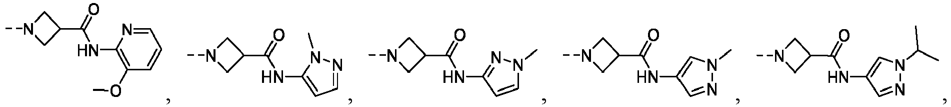

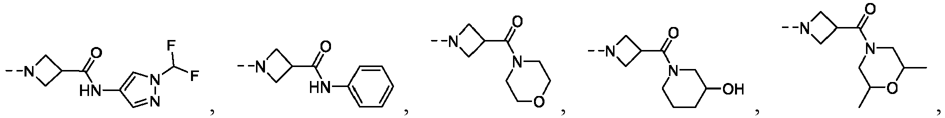

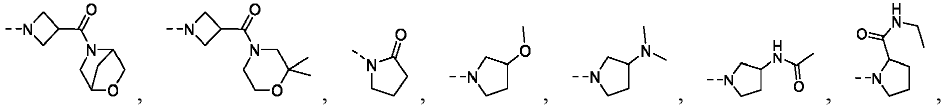

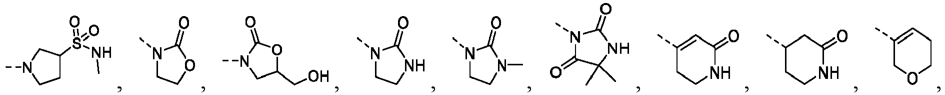

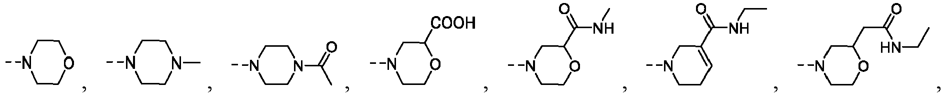

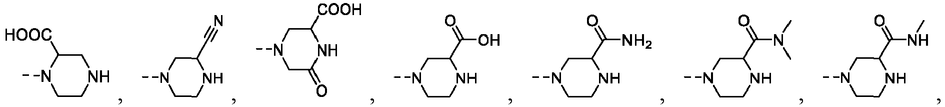

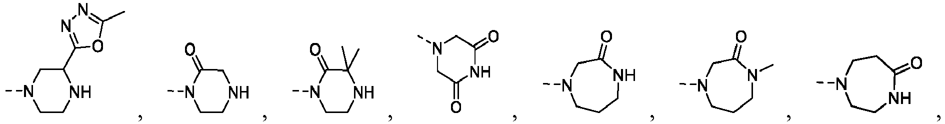

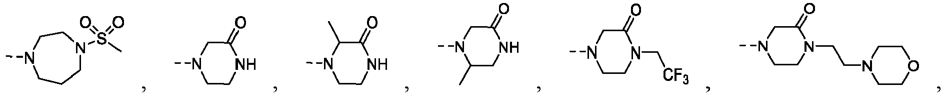

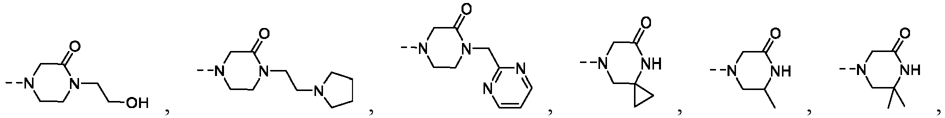

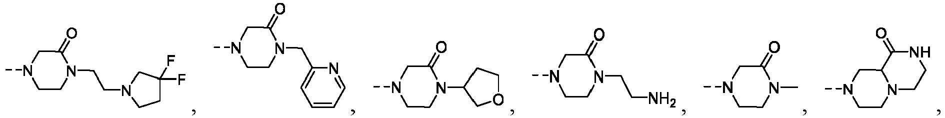

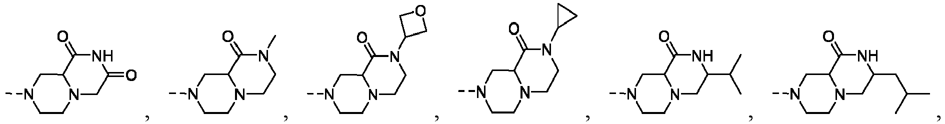

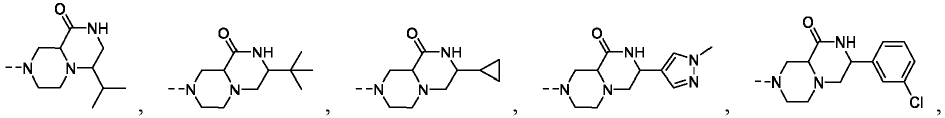

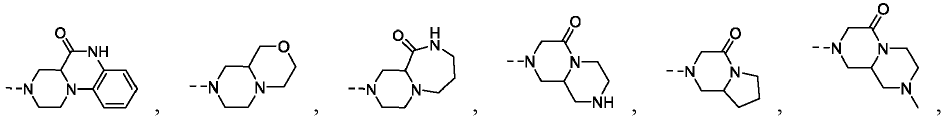

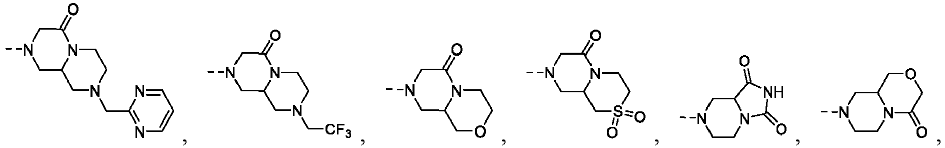

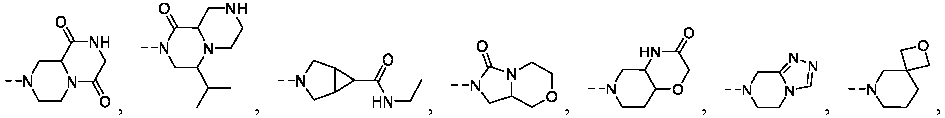

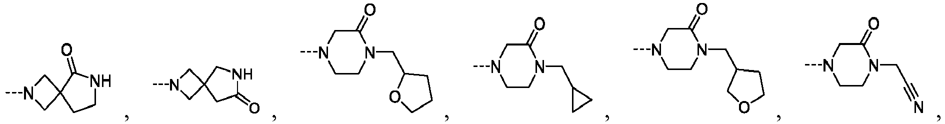

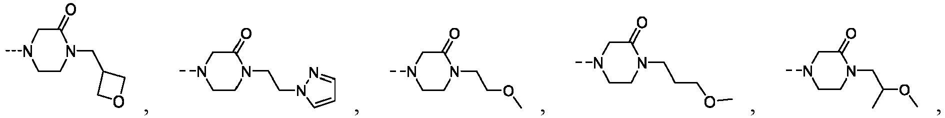

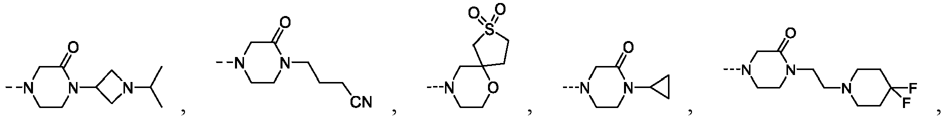

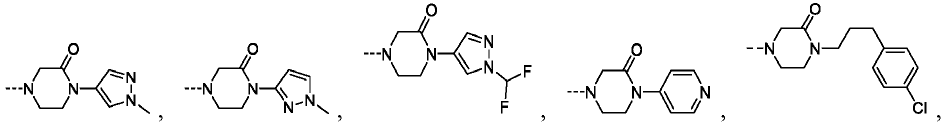

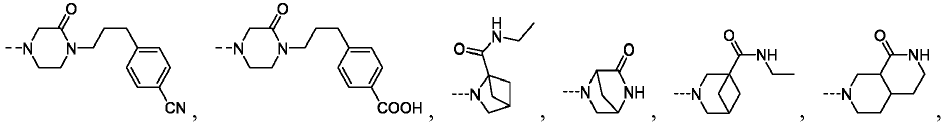

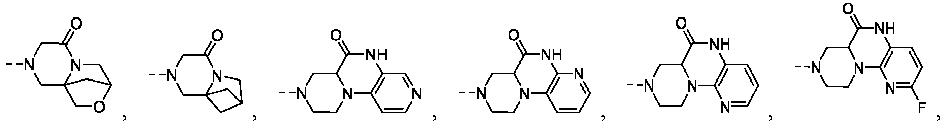

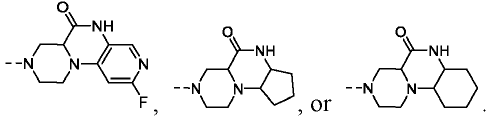

14. The compound of formula I, the *cis-trans* isomer thereof, the enantiomer thereof, the diastereomer thereof, the racemate thereof, the solvate thereof, the hydrate thereof, the pharmaceutically acceptable salt thereof, or the prodrug thereof according to at least one of claims 1 to 13, wherein
the compound of formula I is a compound of formula I-A: 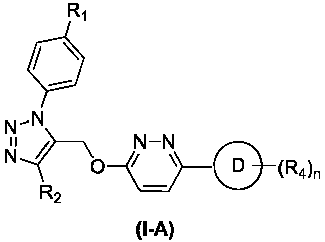
wherein 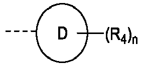 is 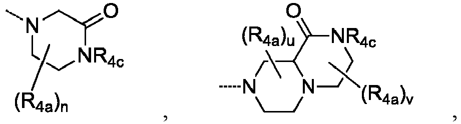 or 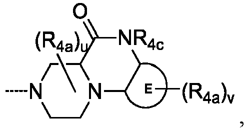
R₁, R₂, R₄ₐ, R_{4c}, u, v, n, and ring E are as defined in at least one of claims 9 to 11.

15. The compound of formula I, the *cis-trans* isomer thereof, the enantiomer thereof, the diastereomer thereof, the racemate thereof, the solvate thereof, the hydrate thereof, the pharmaceutically acceptable salt thereof, or the prodrug thereof according to claim 14, wherein it satisfies one or more than one of the following conditions:
(1) in 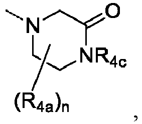 R_{4c} is hydrogen, C₁₋₃ alkyl, C₃₋₆ cycloalkyl, 5- to 10-membered heteroaryl, 5- to 10-membered heteroaryl(C₁₋₃)alkyl, or 3- to 6-membered heterocycloalkyl;
in R_{4c}, the C₁₋₃ alkyl is optionally substituted by 1 to 3 independently selected substituents: halogen, cyano, hydroxyl, amino, C₁₋₃ alkyl, C₁₋₃ alkoxy, C₁₋₆ alkylamino, C₃₋₆ cycloalkyl, 3-to 6-membered heterocycloalkyl, or 3- to 6-membered heterocycloalkyl substituted by 1 to 3 halogens;
in R_{4c}, the 3- to 6-membered heterocycloalkyl is optionally substituted by 1 to 3 C₁₋₃ alkyl groups;
in R_{4c}, the 5- to 10-membered heteroaryl is optionally substituted by 1 to 3 independently selected substituents: C₁₋₃ alkyl or C₁₋₃ haloalkyl;
in R_{4c}, the 5- to 10-membered heteroaryl(C₁₋₃)alkyl is optionally substituted by 1 to 3 independently selected substituents: halogen, cyano, or C₁₋₃ alkoxy;
(2) in 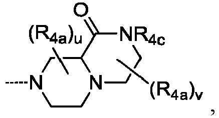 R_{4c} is hydrogen, C₁₋₃ alkyl, C₃₋₆ cycloalkyl, or 3- to 6-membered heterocycloalkyl;
(3) in 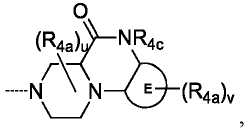 R_{4c} is hydrogen;
(4) in 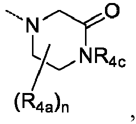 each R₄ₐ is independently hydrogen, oxo, C₁₋₃ alkyl, or COOH, and n is 0, 1, or 2; preferably, n is 0;
(5) in 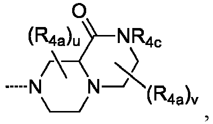 each R₄ₐ is independently hydrogen, oxo, C₁₋₃ alkyl, C₃₋₆ cycloalkyl, phenyl, or 5- to 6-membered heteroaryl;
in R₄ₐ, the C₁₋₃ alkyl is optionally substituted by 1 to 3 C₁₋₃ alkyl groups;
in R₄ₐ, the 5- to 6-membered heteroaryl is optionally substituted by 1 to 3 C₁₋₃ alkyl groups;
in R₄ₐ, the phenyl is optionally substituted by 1 to 3 halogens;
u is 0;
v is 0 or 1;
preferably, R₄ₐ is hydrogen or C₁₋₃ alkyl, and the C₁₋₃ alkyl is optionally substituted by 1 to 3 C₁₋₃ alkyl groups; and
(6) in 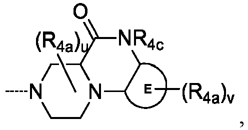 each R₄ₐ is independently hydrogen or halogen; u is 0, and v is 0 or 1; preferably, u is 0, and v is 0.

16. The compound of formula I, the *cis-trans* isomer thereof, the enantiomer thereof, the diastereomer thereof, the racemate thereof, the solvate thereof, the hydrate thereof, the pharmaceutically acceptable salt thereof, or the prodrug thereof according to claim 1, wherein
the compound of formula I is selected from the following compounds:
| **Example** | **Structure** | **Example** | **Structure** |
|---|---|---|---|
| 1 | 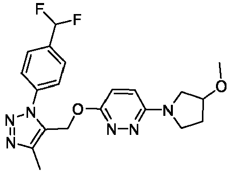 | 109 | 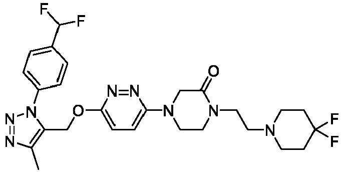 |
| 2 | 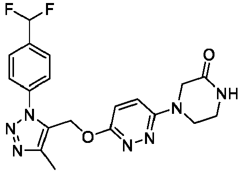 | 110 | 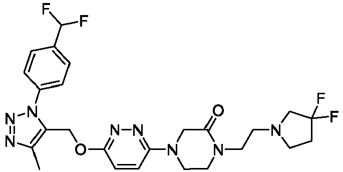 |
| 3 | 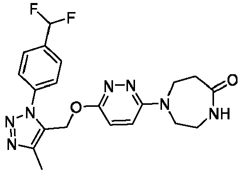 | 111 | 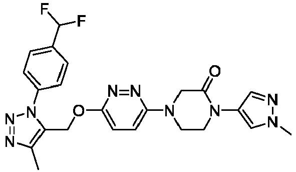 |
| 4 | 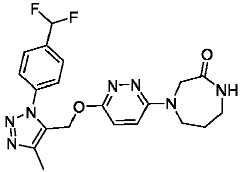 | 112 | 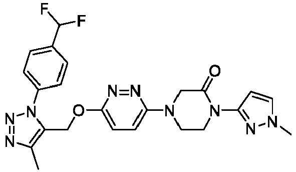 |
| 5 | 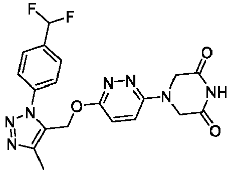 | 113 | 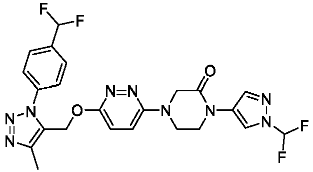 |
| 6 | 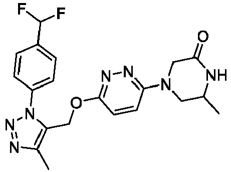 | 114 | 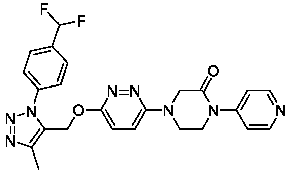 |
| 7 | 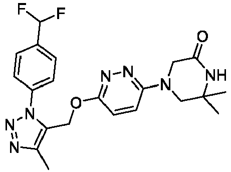 | 115 | 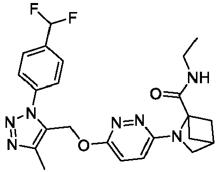 |
| 8 | 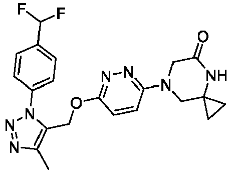 | 116 | 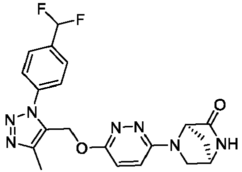 |
| 9 | 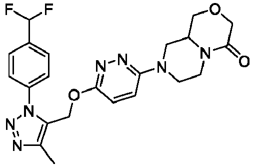 | 117 | 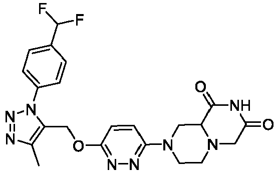 |
| 10 | 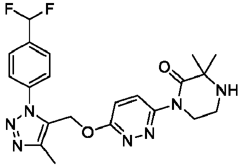 | 118 | 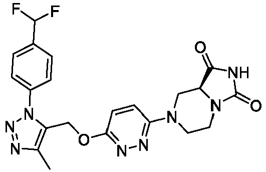 |
| 11 | 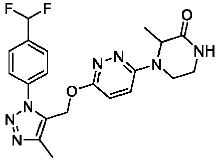 | 119 | 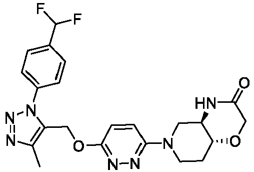 |
| 12 | 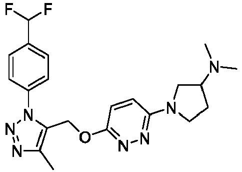 | 120 | 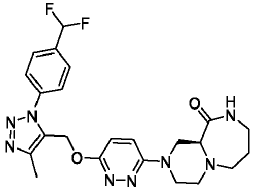 |
| 13 | 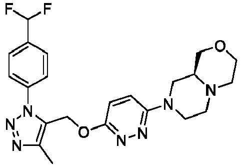 | 121 | 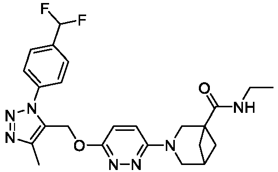 |
| 14 | 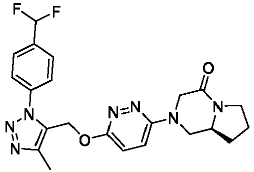 | 122 | 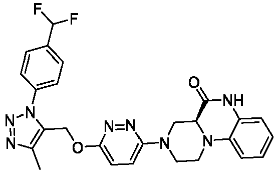 |
| 15 | 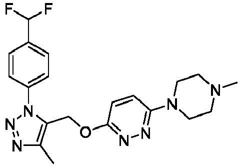 | 123 | 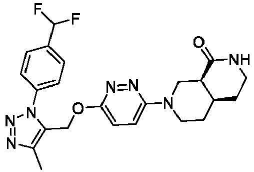 |
| 16 | 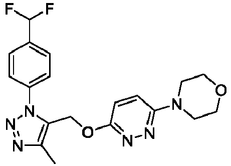 | 124 | 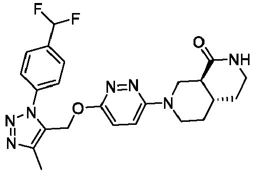 |
| 17 | 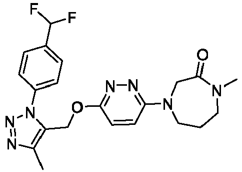 | 125 | 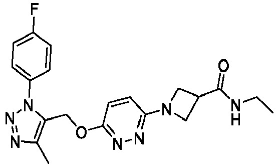 |
| 18 | 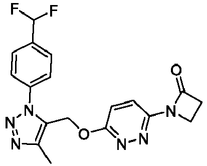 | 126 | 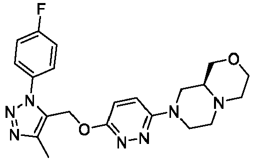 |
| 19 | 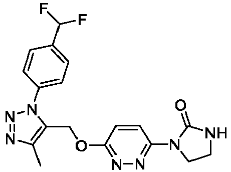 | 127 | 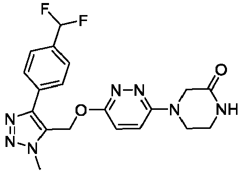 |
| 20 | 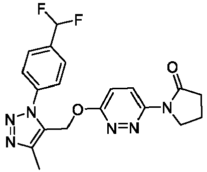 | 128 | 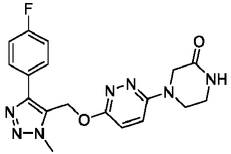 |
| 21 | 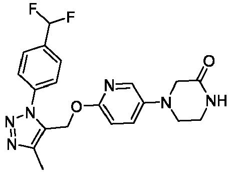 | 129 | 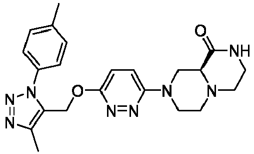 |
| 22 | 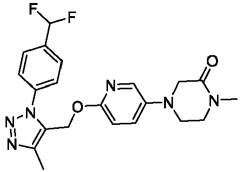 | 130 | 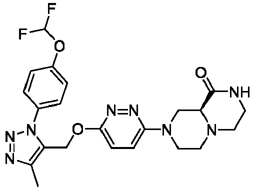 |
| 23 | 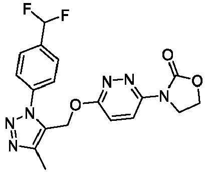 | 131 | 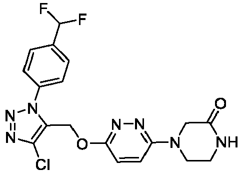 |
| 24 | 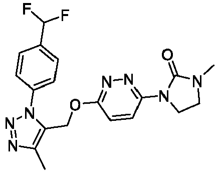 | 132 | 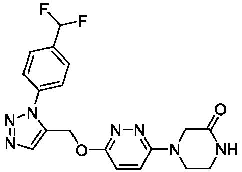 |
| 25 | 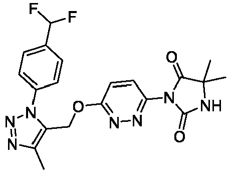 | 133 | 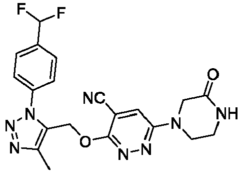 |
| 26 | 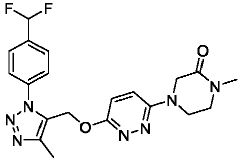 | 134 | 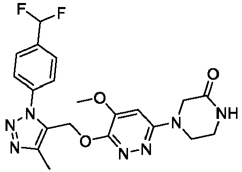 |
| 27 | 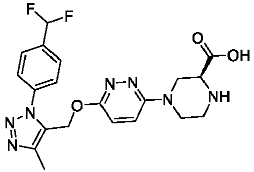 | 135 | 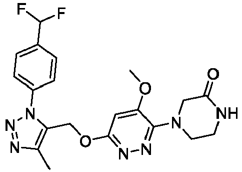 |
| 28 | 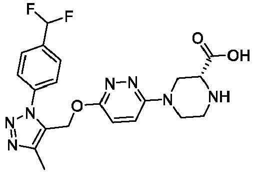 | 136 | 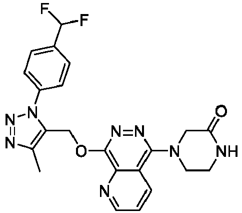 |
| 29 | 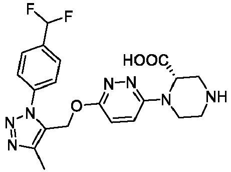 | 137 | 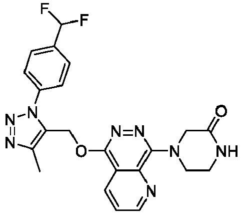 |
| 30 | 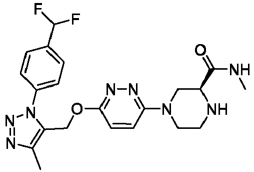 | 138 | 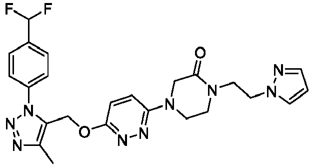 |
| 31 | 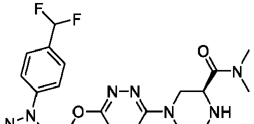 | 139 | 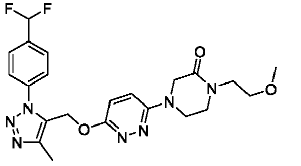 |
| 32 | 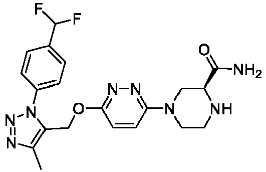 | 140 | 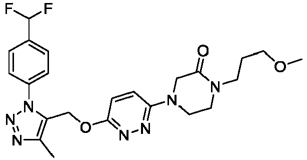 |
| 33 | 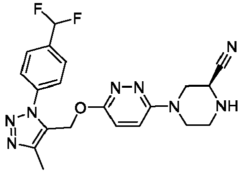 | 141 | 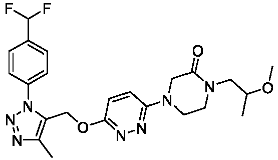 |
| 34 | 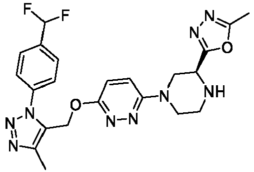 | 142 | 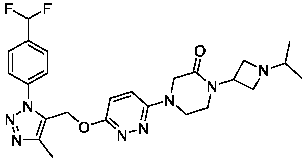 |
| 35 | 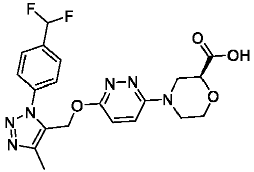 | 143 | 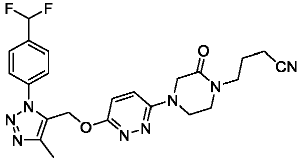 |
| 36 | 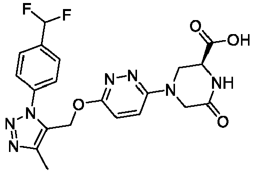 | 144 | 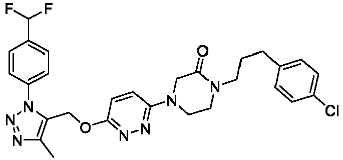 |
| 37 | 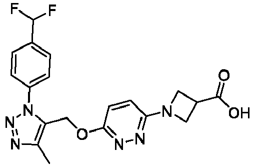 | 145 | 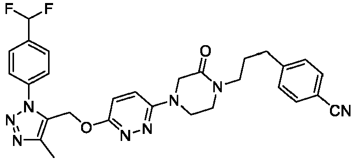 |
| 38 | 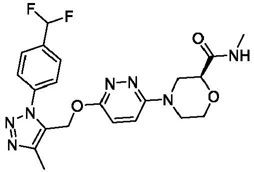 | 146 | 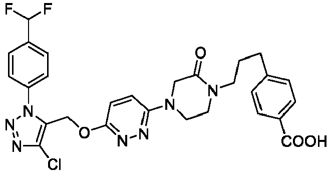 |
| 39 | 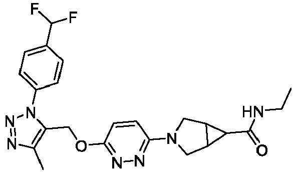 | 147 | 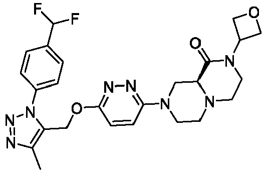 |
| 40 | 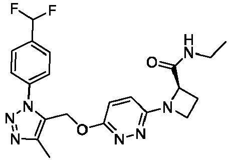 | 148 | 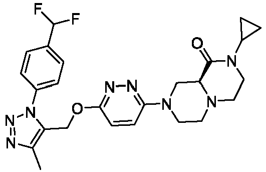 |
| 41 | 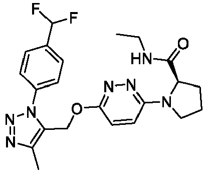 | 149 | 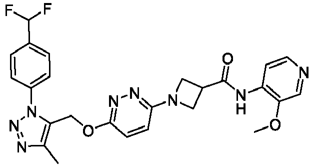 |
| 42 | 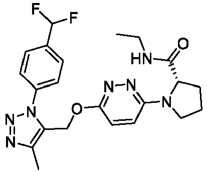 | 150 | 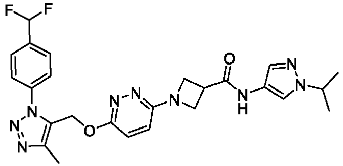 |
| 43 | 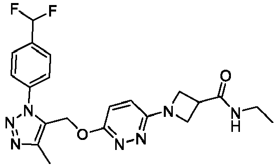 | 151 | 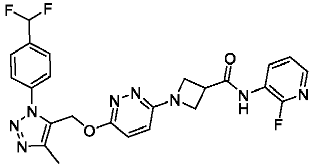 |
| 44 | 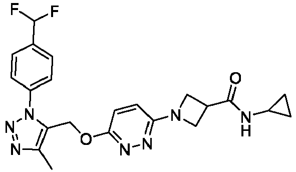 | 152 | 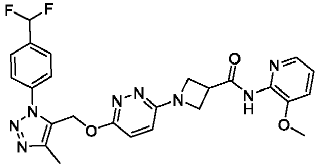 |
| 45 | 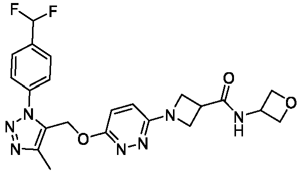 | 153 | 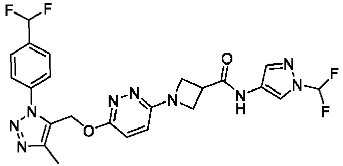 |
| 46 | 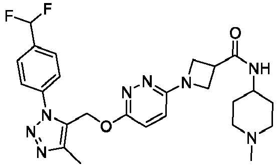 | 154 | 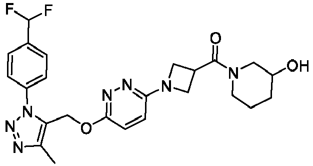 |
| 47 | 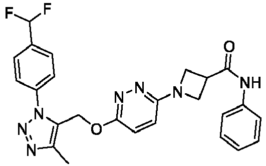 | 155 | 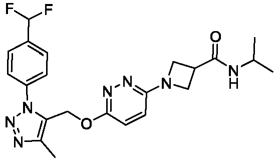 |
| 48 | 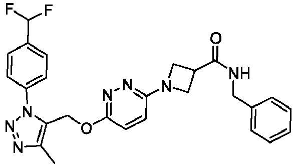 | 156 | 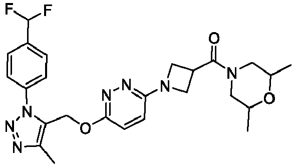 |
| 49 | 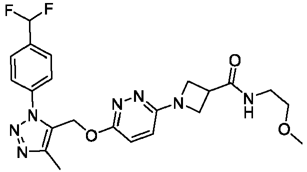 | 157 | 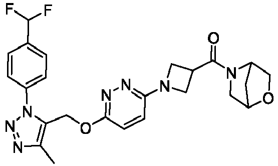 |
| 50 | 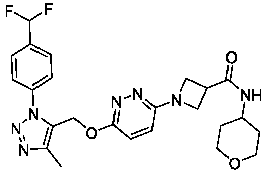 | 158 | 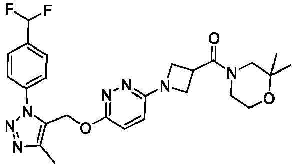 |
| 51 | 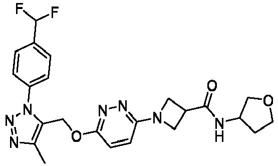 | 159 | 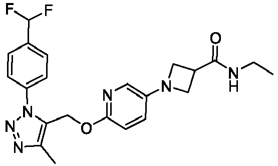 |
| 52 | 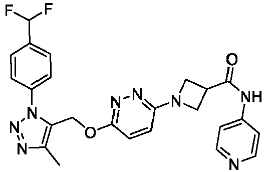 | 160 | 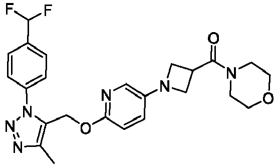 |
| 53 | 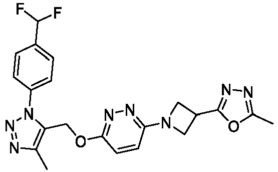 | 161 | 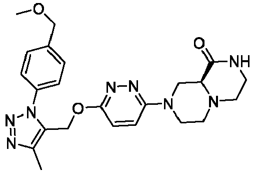 |
| 54 | 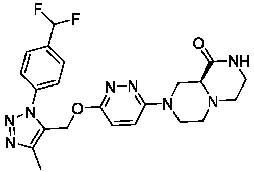 | 162 | 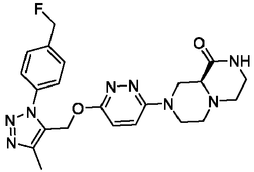 |
| 55 | 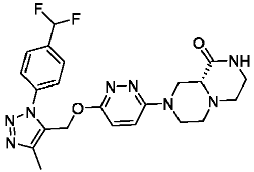 | 163 | 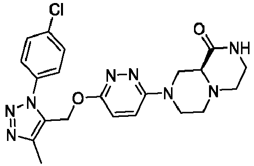 |
| 56 | 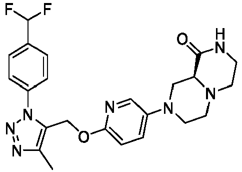 | 164 | 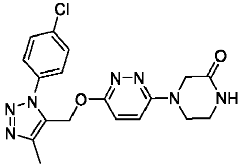 |
| 57 | 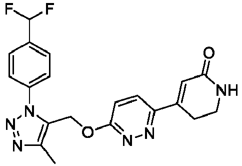 | 165 | 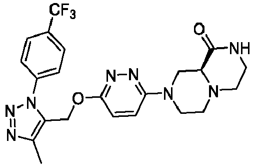 |
| 58 | 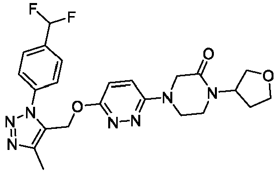 | 166 | 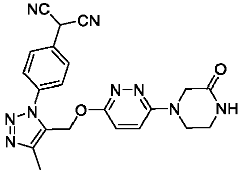 |
| 59 | 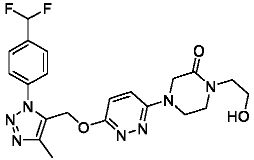 | 167 | 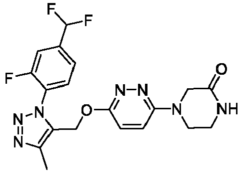 |
| 60 | 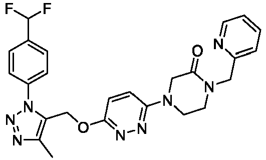 | 168 | 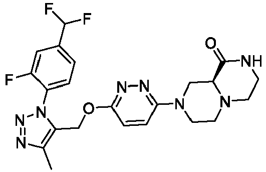 |
| 61 | 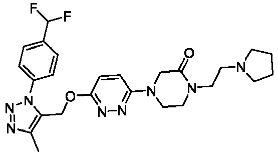 | 169 | 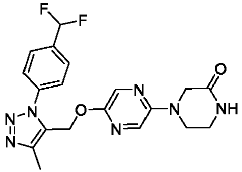 |
| 62 | 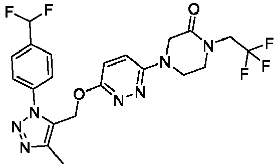 | 170 | 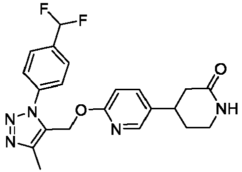 |
| 63 | 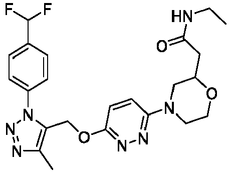 | 171 | 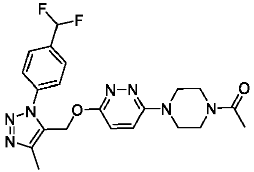 |
| 64 | 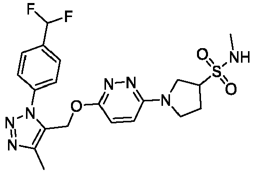 | 172 | 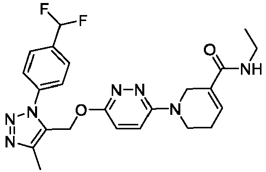 |
| 65 | 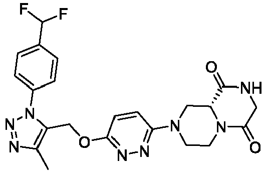 | 173 | 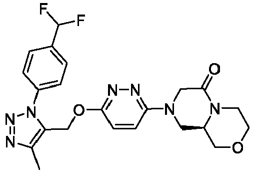 |
| 66 | 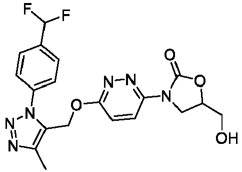 | 174 | 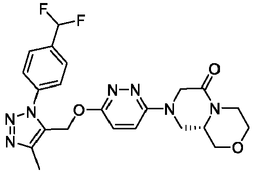 |
| 67 | 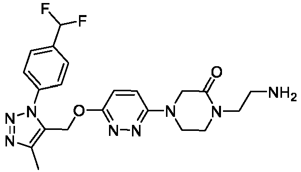 | 175 | 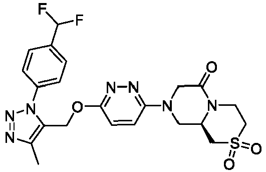 |
| 68 | 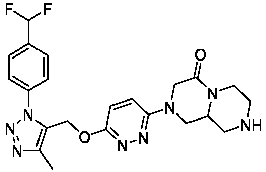 | 176 | 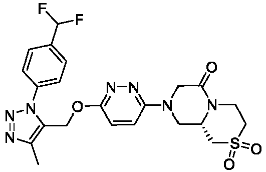 |
| 69 | 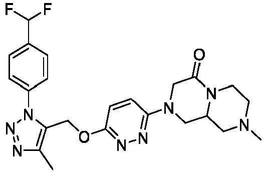 | 177 | 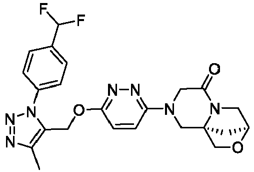 |
| 70 | 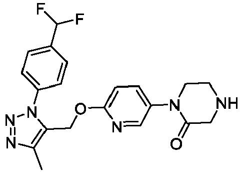 | 178 | 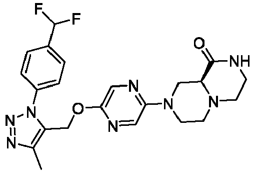 |
| 71 | 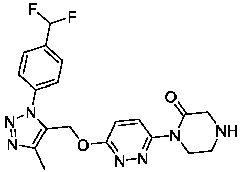 | 179 | 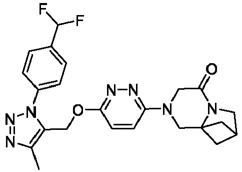 |
| 72 | 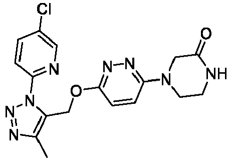 | 180 | 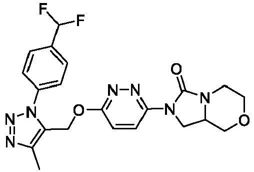 |
| 73 | 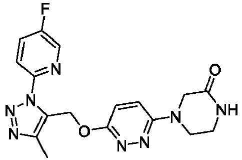 | 181 | 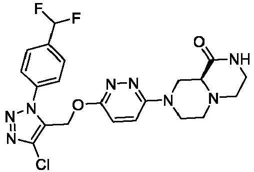 |
| 74 | 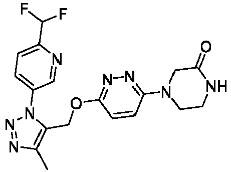 | 182 | 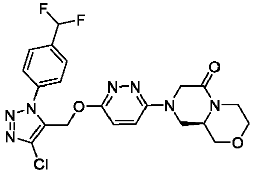 |
| 75 | 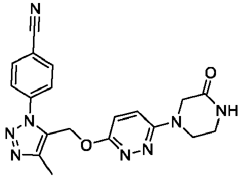 | 183 | 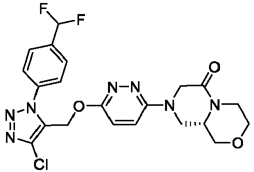 |
| 76 | 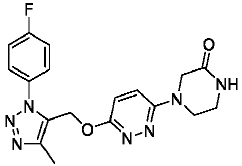 | 184 | 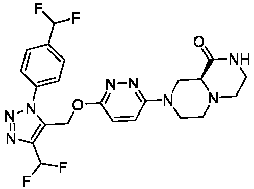 |
| 77 | 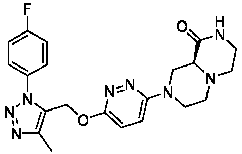 | 185 | 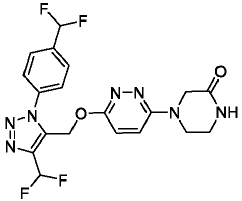 |
| 78 | 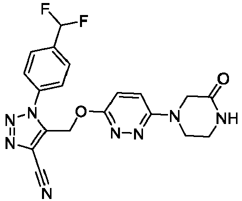 | 186 | 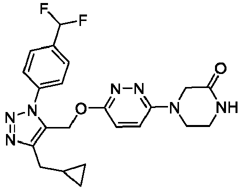 |
| 79 | 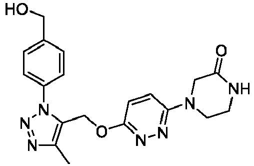 | 187 | 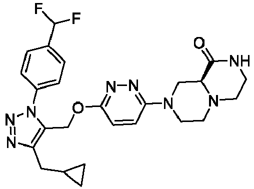 |
| 80 | 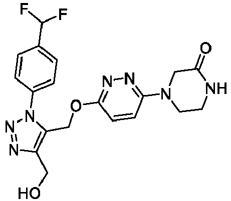 | 188 | 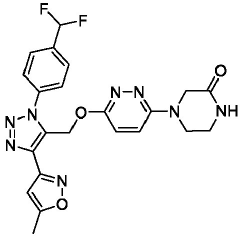 |
| 81 | 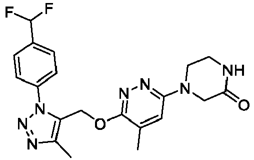 | 189 | 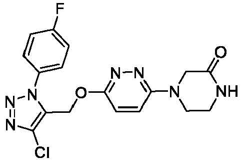 |
| 82 | 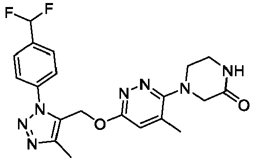 | 190 | 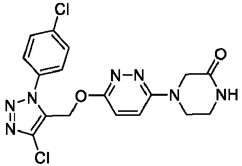 |
| 83 | 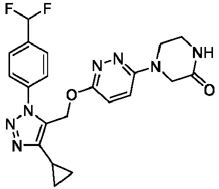 | 191 | 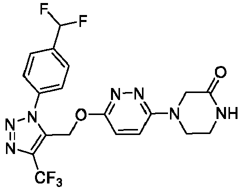 |
| 84 | 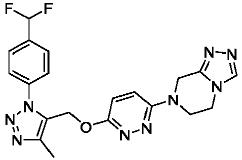 | 192 | 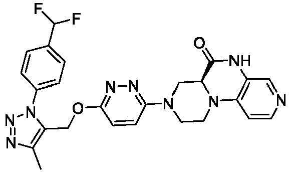 |
| 85 | 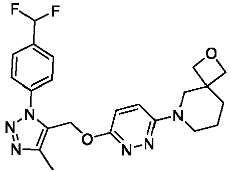 | 193 | 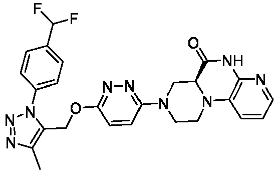 |
| 86 | 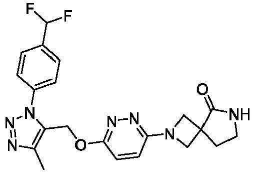 | 194 | 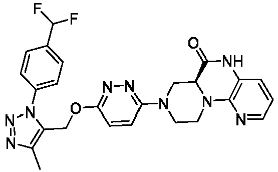 |
| 87 | 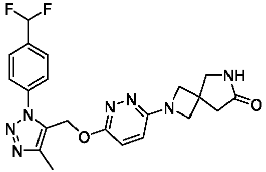 | 195 | 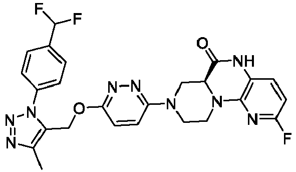 |
| 88 | 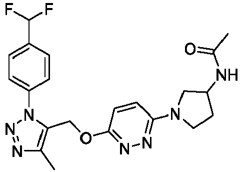 | 196 | 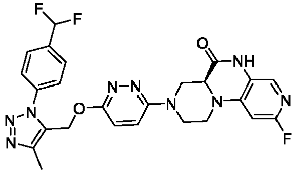 |
| 89 | 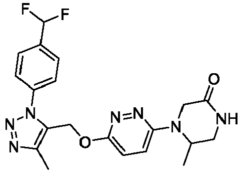 | 197 | 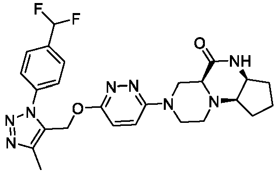 |
| 90 | 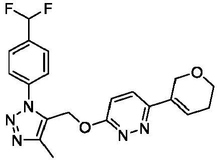 | 198 | 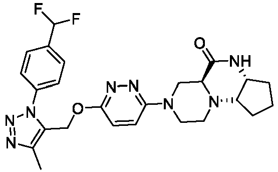 |
| 91 | 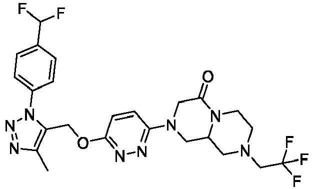 | 199 | 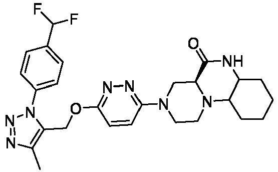 |
| 92 | 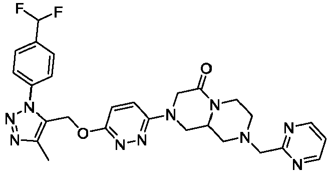 | 200 | 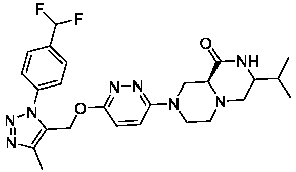 |
| 93 | 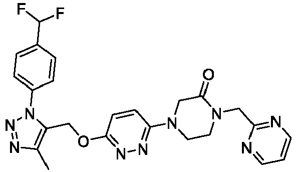 | 201 | 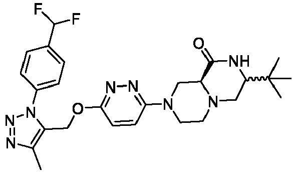 |
| 94 | 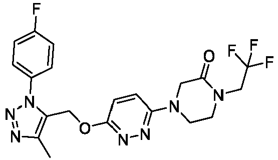 | 202 | 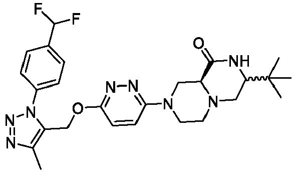 |
| 95 | 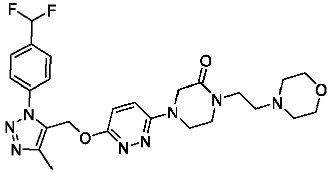 | 203 | 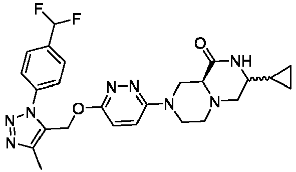 |
| 96 | 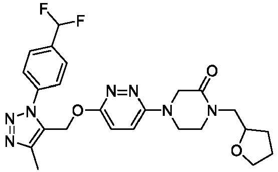 | 204 | 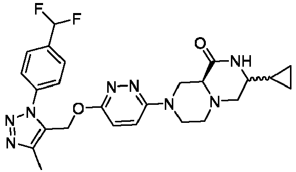 |
| 97 | 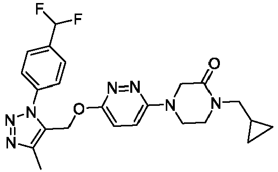 | 205 | 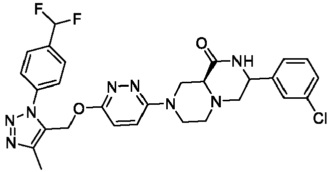 |
| 98 | 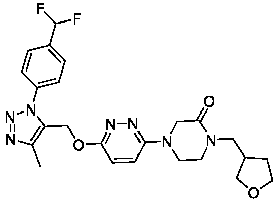 | 206 | 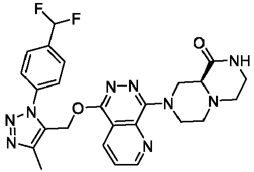 |
| 99 | 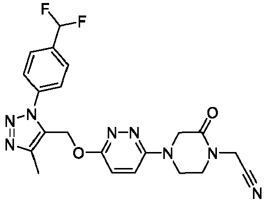 | 207 | 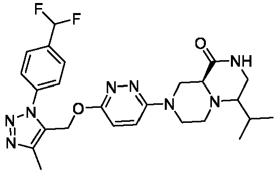 |
| 100 | 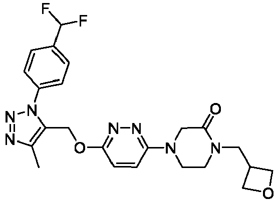 | 208 | 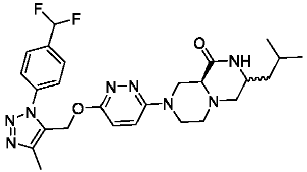 |
| 101 | 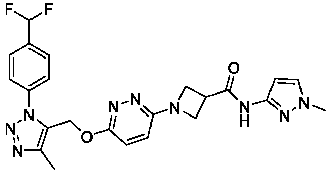 | 209 | 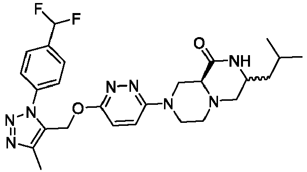 |
| 102 | 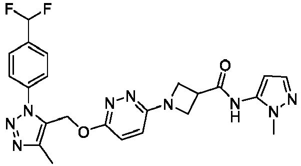 | 210 | 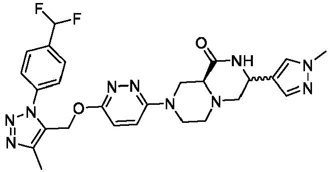 |
| 103 | 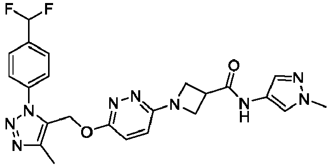 | 211 | 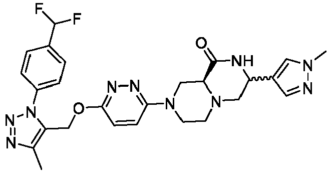 |
| 104 | 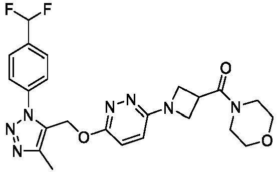 | 212 | 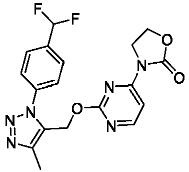 |
| 105 | 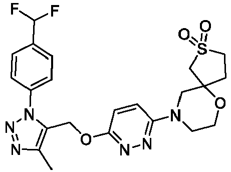 | 213 | 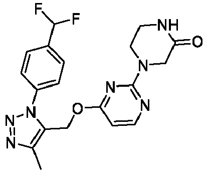 |
| 106 | 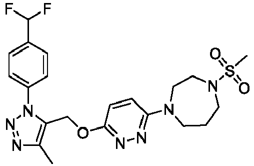 | 214 | 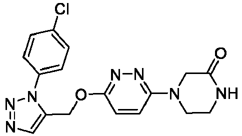 |
| 107 | 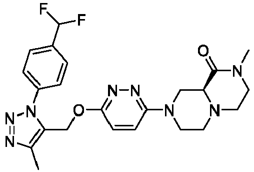 | 215 | 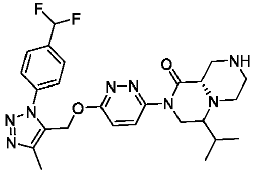 |
| 108 | 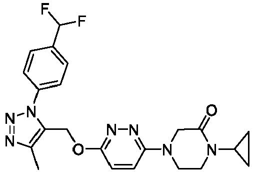 | | |

17. A pharmaceutical composition, comprising the compound of formula I, the *cis-trans* isomer thereof, the enantiomer thereof, the diastereomer thereof, the racemate thereof, the solvate thereof, the hydrate thereof, the pharmaceutically acceptable salt thereof, or the prodrug thereof according to at least one of claims 1 to 16; preferably, the pharmaceutical composition further comprises one or more than one pharmaceutically acceptable carrier, diluent, or excipient.

18. A use of the compound of formula I, the *cis-trans* isomer thereof, the enantiomer thereof, the diastereomer thereof, the racemate thereof, the solvate thereof, the hydrate thereof, the pharmaceutically acceptable salt thereof, or the prodrug thereof according to at least one of claims 1 to 16, or the pharmaceutical composition according to claim 17, the use is selected from:
(1) manufacture of an α5-GABA_{A} receptor modulator;
(2) manufacture of a medicament for treating or preventing a disease related to an α5-GABA_{A} receptor;
(3) manufacture of a medicament for treating or preventing the following diseases: pain, Alzheimer's disease, multi-infarct dementia, and stroke.
